# EUROPEAN PATENT APPLICATION

(11) **EP 4 650 348 A1**
(43) Date of publication of application: **19.11.2025**
(21) Application number: 24741587.0
(22) Date of filing: 12.01.2024
(51) Int. Cl.: C07D 223/10, A61K 31/45, A61K 31/55, A61K 31/551, A61K 31/553, A61P 25/28, A61P 29/00, A61P 35/00, A61P 37/06, C07D 243/04, C07D 267/10, C07D 401/12, C07D 401/14, C07D 403/04, C07D 403/06, C07D 471/10, C07D 487/10, C07D 498/10, C07D 519/00

(54) **CEREBLON E3 LIGASE BINDING COMPOUND, PHARMACEUTICAL COMPOSITION CONTAINING SAME, AND PRODUCTION METHOD THEREFOR**

(30) Priority: 12.01.2023 JP 2023003271
(71) Applicant: Mitsubishi Tanabe Pharma Corporation, Osaka-shi Osaka 541-8505 (JP)
(72) Inventor: SAKAMAKI Shigeki, Osaka-shi, Osaka 541-8505 (JP); NIWA Yasuki, Osaka-shi, Osaka 541-8505 (JP); IKUBO Masaya, Osaka-shi, Osaka 541-8505 (JP); SUGAMA Hiroshi, Osaka-shi, Osaka 541-8505 (JP)
(74) Representative: Hoffmann Eitle
(86) International application number: PCT/JP2024/000578
(87) International publication number: WO 2024/150815

(57) **Abstract**

The problem addressed is to provide a novel compound having cereblon E3 ligase binding ability. Provided is a compound represented by the following Formula (I): 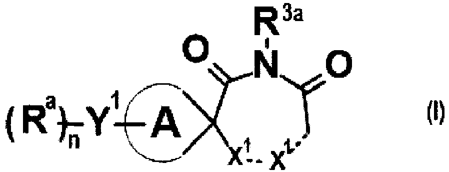 [wherein the symbols are as described in the specification], or the following Formula (II): 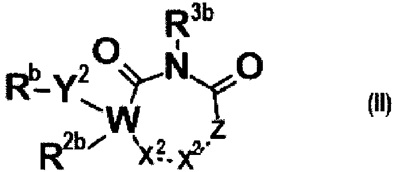 [wherein the symbols are as described in the specification], or the following Formula (III): 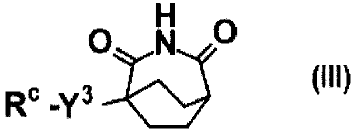 [wherein the symbols are as described in the specification], or the following Formula (IV): 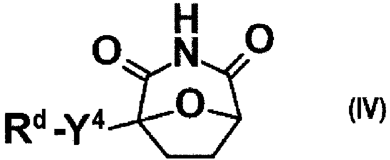 [wherein the symbols are as described in the specification], or a pharmacologically acceptable salt thereof.

## Description

### [Technical Field]

The present invention relates to a cereblon E3 ligase binding compound, a pharmaceutical composition containing same, and a production method therefor.

### [Technical Background]

An ubiquitin-proteasome system is a system in which unnecessary proteins in cells are degraded and removed via ubiquitination of target proteins in an ATP-dependent manner by the proteasome, and plays an important role in cell proliferation, survival, maintenance of homeostasis, and the like. The ubiquitin-proteasome system involves multiple enzymes called ubiquitin-activating enzyme (E1), ubiquitin-conjugating enzyme (E2), and ubiquitin ligase (E3), which function sequentially to achieve ubiquitination of target proteins. In particular, target proteins undergoing so-called polyubiquitination, in which ubiquitin chains are linked via the 48th lysine residue of ubiquitin, are efficiently recognized and degraded by the proteasome. It has been reported that humans have two types of ubiquitin-activating enzymes, about 40 types of ubiquitin-conjugating enzymes, and about 600 types of ubiquitin ligases, and it is known that substrate recognition by ubiquitin ligases is strictly regulated. Ubiquitin ligases include those that function as single entities and those that function by forming complexes, with the latter adopting a complex structure in which a substrate recognition protein binds to a scaffold protein via an adapter protein, and this substrate recognition protein determines a substrate protein to be ubiquitinated. In recent years, it has been reported that by chemically linking a compound that binds to several types of proteins (target-directed ligands) with a compound that binds to a substrate recognition protein, proteins that are not originally substrates of ubiquitin ligase can be induced to degrade, and to date, research has been conducted on selective protein degradation induction (chemical knockdown) via ubiquitin ligase using small molecule compounds.

In recent years, a technology that uses a compound linking a ligand of an E3 ligase with ubiquitin ligase activity and an inhibitor of a target protein to form an artificial complex of the E3 ligase and the target protein in cells, thereby inducing degradation of the target protein by utilizing the ubiquitin-proteasome system, which is an intracellular protein degradation mechanism, has been attracting attention as a novel drug discovery technology (see, for example, Non-Patent Document 1).

CRBN (cereblon) forms a CRL4 (Cullin-Ring Ligase 4) complex with three other proteins, DDB1 (Damage-specific DNA binding protein 1), Cul4A, and Roc1, and functions as a ubiquitin ligase. A role of cereblon as a component of this complex includes targeting proteins for protein hydrolysis (degradation) via a ubiquitin-proteasome pathway (see, for example, Non-Patent Document 2).

Cereblon is closely associated with metabolism and proliferation of tumor cells in addition to normal cells. On the one hand, the presence of cereblon ensures a normal metabolic function of ion channels and a normal physiological function, which are important for maintaining cell growth and proliferation. On the other hand, cereblon is also involved in the onset of many diseases, such as cancer. See, for example, Non-Patent Document 3.

Patent Document 1 discloses a bifunctional compound having, at one end, a CRBN ligand that binds to CRBN (cereblon), which is a substrate recognition protein of ubiquitin ligase, and, at the other end, a moiety that binds to a target protein.

### [Related Art]

### [Patent Documents]

[Patent Document 1] International Publication No. 2015/160845.

### [Non-Patent Documents]

[Non-Patent Document 1] Toure M., Angew Chem Int Ed Engl., 2016, 55: 1966-1973.
[Non-Patent Document 2] Chang et al., Int. J. Biochem. Mol. Biol., 2 (3): 287-94 (2011).
[Non-Patent Document 3] Shi et al., J. Immunol. Res. Article ID 9130608 (2017).

### [Summary of the Invention]

In drug discovery of bifunctional compounds as described in Patent Document 1, a CRBN ligand moiety that binds to CRBN (cereblon) is required to have cereblon E3 ligase binding ability.

The present invention is intended to provide novel compounds having cereblon E3 ligase binding ability.

As a result of examination, it was found that the problem is solved by the following specific means.

A first embodiment of the present invention is a compound represented by the following Formula (I): 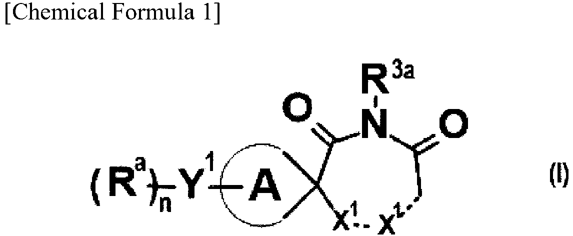 [wherein
a dotted line represents a single bond or a double bond,
two X¹ groups are independently selected from CH and CH₂, or one X¹ is CH₂ and the other X¹ is O, NH, NCH₃, or S,
Ring A is a saturated or partially unsaturated 3- to 8-membered ring that may contain 1 to 3 atoms independently selected from nitrogen, oxygen, and sulfur atoms, and the ring may be substituted with one or more substituents independently selected from a group consisting of a fluorine atom, a methyl group, an ethyl group, and an oxo group,
R^{3a} is a hydrogen atom, a methyl group, -OH, -CH₂OC(O)R'^{a}, -CH₂OP(O)OHOR'^{a}, - CH₂OP(O)R'^{a}₂, or -CH₂OP(O)(OR'^{a})₂,
R'^{a} is a C₁₋₄ alkyl group,
n is 0 or 1,
when n is 0,
Y¹ is an optionally substituted C₁₋₁₀ alkyl group, an optionally substituted C₁₋₁₀ alkoxy group, an optionally substituted C₁₋₁₀ alkylcarbonyl group, or an optionally substituted C₁₋₁₀ alkoxycarbonyl group,
when n is 1,
Y¹ is a single bond, CO, SO₂, CH₂CO, or a C₁₋₄ alkylene group, and
R^{a} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group].
or the following Formula (II): 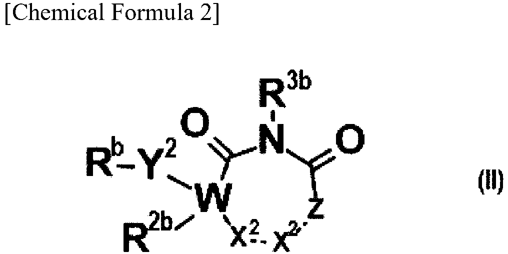
[wherein
a dotted line represents a single bond or a double bond,
two X² groups are independently selected from CH and CH₂, or one X² is CH₂ and the other X² is O, NH, NCH₃, or S,
Z is a single bond or CH₂,
R^{2b} is a hydrogen atom, a fluorine atom, or a methyl group,
R^{3b} is a hydrogen atom, a methyl group, -OH, -CH₂OC(O)R'^{b}, -CH₂OP(O)OHOR'^{b}, - CH₂OP(O)R'^{b}₂, or -CH₂OP(O)(OR'^{b})₂,
R'^{b} is a C₁₋₄ alkyl group,
when Z is CH₂,
W is C or N, when W is N, R^{2b} is absent,
Y² is a single bond, -CR"R"'-, -O-, -NR"-, -NR"C(O)-, -C(O)NR"-, -NR"SO₂-, -SO₂NR"-, - CH₂NR"-, -NR"CH₂-, -C(O)NR''CH₂-, -CH₂NR''C(O)-, -CH₂N(COR")-, -N(COR")CH₂-, - CH₂N(COOR")-, or -N(COOR")CH₂-,
R" and R‴ are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms,
R^{b} is an optionally substituted 6- to 10-membered aryl group (provided that when W is N, a 6-membered aryl group is excluded), an optionally substituted 5- to 7-membered monocyclic heteroaryl group, an optionally substituted 5- to 7-membered monocyclic heterosaturated ring group (provided that when W is N, a tetrahydrofuranyl group is excluded), an optionally substituted and optionally partially saturated 8- to 10-membered bicyclic heteroaryl group (provided that when W is C, the following structures: 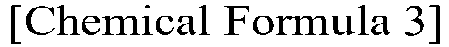 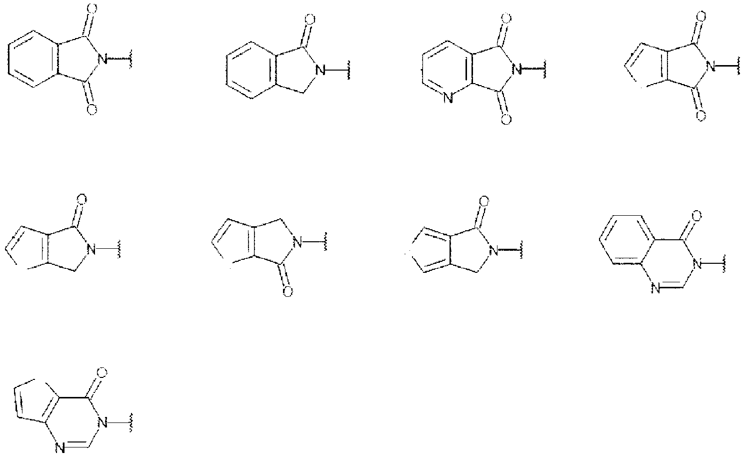
(wherein the ring structures are unsubstituted or substituted with 1 to 5 arbitrary substituents) are excluded), or an optionally substituted 5-4 spiro ring group which may contain 1 to 3 atoms independently selected from nitrogen, oxygen, and sulfur atoms,
when Z is a single bond,
W is C,
Y² is an optionally substituted 5-4 spiro ring divalent group which may contain 1 to 3 atoms independently selected from nitrogen, oxygen, and sulfur atoms, wherein the 5-4 spiro ring is bonded to W at any position of its 5-membered ring, and
R^{b} is an optionally substituted C₁₋₁₀ alkyl group, an optionally substituted C₁₋₁₀ alkylcarbonyl group, an optionally substituted C₁₋₁₀ alkoxy group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or the following Formula (III): 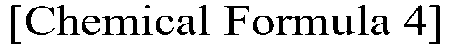 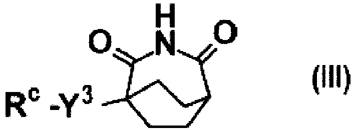
[wherein
Y³ is a single bond or -NH-, and
R^{c} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or the following Formula (IV): 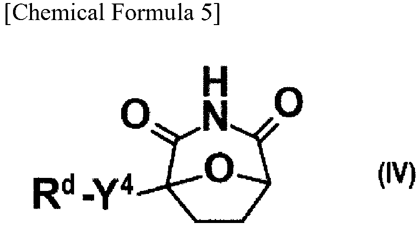
[wherein
Y⁴ is a single bond, and
R^{d} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or a pharmacologically acceptable salt thereof (provided that the following compounds: 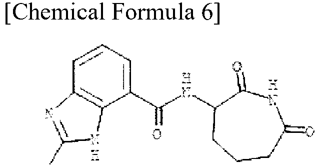 and 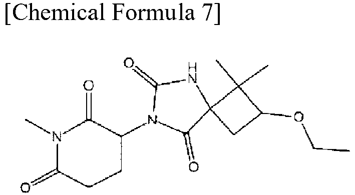
are excluded).

A second embodiment of the present invention is a pharmaceutical composition containing, as an active ingredient, a compound of Formula (I) or Formula (II) substituted with L substituted with TBL, that is, a compound represented by Formula (I-1): 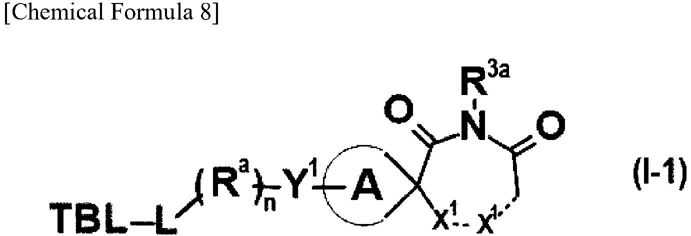
[wherein the symbols have the same meanings as described above, and
Y¹ or R^{a} is substituted with L substituted with TBL],
or Formula (II-1): 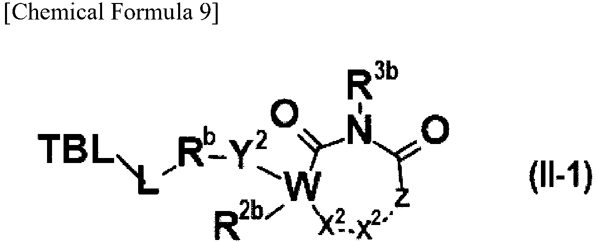
[wherein the symbols have the same meanings as described above, and
R^{b} is substituted with L substituted with TBL],
or Formula (III-1): 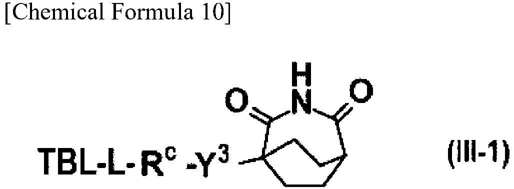
[wherein the symbols have the same meanings as described above, and
R^{c} is substituted with L substituted with TBL],
or Formula (IV-1): 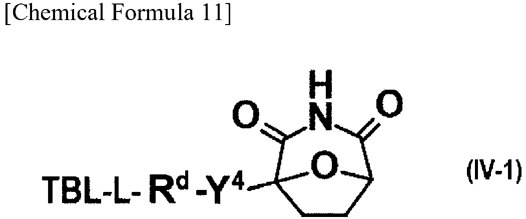
[wherein the symbols have the same meanings as described above, and
R^{d} is substituted with L substituted with TBL]
or a pharmacologically acceptable salt thereof, and a pharmaceutically acceptable carrier.

Another embodiment of the present invention is a method for producing a compound of Formula (I-1), Formula (II-1), Formula (III-1), or Formula (IV-1), or a pharmacologically acceptable salt thereof.

Another embodiment of the present invention is a pharmaceutical containing a compound of the above Formula (I-1), Formula (II-1), Formula (III-1), or Formula (IV-1), or a pharmacologically acceptable salt thereof, as an active ingredient. This pharmaceutical can be an agent for prevention or treatment of diseases caused by dysregulation of protein activity.

Further, another embodiment of the present invention is use of a compound of Formula (I-1), Formula (II-1), Formula (III-1), or Formula (IV-1), or a pharmacologically acceptable salt thereof, for manufacture of an agent for prevention or treatment of a disease caused by dysregulation of protein activity.

Further, another embodiment of the present invention is a method for modulating protein activity of a target protein in a mammal, including administering an effective amount of a compound of Formula (I-1), Formula (II-1), Formula (III-1), or Formula (IV-1), or a pharmacologically acceptable salt thereof, to the mammal.

Further, another embodiment of the present invention is a method for prevention or treatment of diseases caused by dysregulation of protein activity, including administering a prophylactically or therapeutically effective amount of a compound of Formula (I-1), Formula (II-1), Formula (III-1), or Formula (IV-1), or a pharmacologically acceptable salt thereof, to a mammal in need of such administration.

Further, another embodiment of the present invention is a compound of Formula (I-1), Formula (II-1), Formula (III-1), or Formula (IV-1), or a pharmacologically acceptable salt thereof, for use in prevention or treatment of diseases caused by dysregulation of protein activity.

According to an embodiment of the present invention, novel compounds having cereblon E3 ligase binding ability are provided. Cereblon E3 ligase binding compounds containing a linker, and E3 ligase binding compounds containing a linker and a moiety that binds to a target protein, are included in the present embodiment.

E3 ligase binding compounds containing a linker and a moiety that binds to a target protein can exhibit a wide range of pharmacological activities by modulating ubiquitination of a target protein and inducing degradation of the target protein. A pharmaceutical composition containing this E3 ligase binding compound is an embodiment of the present invention and can be useful as a therapeutic agent for diseases caused by dysregulation of protein activity, for example, for diseases such as cancer.

According to another embodiment of the present invention, a method is provided for producing an E3 ligase binding compound containing a linker and a moiety that binds to a target protein, the method including use of a novel compound,.

### [Mode for Carrying Out the Invention]

### [Definition of groups used in the present specification]

Definitions of the groups used in the present specification are described in detail below. Unless otherwise specified, the groups have the following definitions. In the present specification, when indicating the number of carbon atoms constituting a certain group, the notation "C₁-C₆" may be used instead of "1 to 6 carbon atoms." Further, when indicating the number of atoms constituting a certain ring, the notation "3- to 10-membered" may be used instead of "3 to 10 ring-constituting atoms."

When a compound has an acidic functional group and/or a basic functional group within the compound, a salt can be formed. Examples of such salts include metal salts, ammonium salts, salts with organic bases, salts with inorganic acids, salts with organic acids, salts with basic or acidic amino acids, and the like.

In the present specification, examples of "halogen atom" include fluorine, chlorine, bromine, and iodine.

In the present specification, the term "an alkyl group" (including the "alkyl" portion in definitions) refers to a linear or branched alkyl group having 1 to 10 carbon atoms, such as methyl, ethyl, propyl, isopropyl, butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, 2-ethylbutyl, heptyl, octyl, 3-methyloctyl, nonyl, and decyl.

Here, in one embodiment, it may be an alkyl group having 1 to 8 carbon atoms. In another embodiment, it may be an alkyl group having 1 to 6 carbon atoms. In yet another embodiment, it may be an alkyl group having 1 to 4 carbon atoms.

In the present specification, the term "an alkenyl group" (including the "alkenyl" portion in definitions) refers to a linear or branched alkenyl group having 2 to 10 carbon atoms, such as ethenyl, 1-propenyl, 2-propenyl, 2-methyl-1-propenyl, 1-butenyl, 2-butenyl, 3-butenyl, 3-methyl-2-butenyl, 1-pentenyl, 2-pentenyl, 3-pentenyl, 4-pentenyl, 4-methyl-3-pentenyl, 1-hexenyl, 3-hexenyl, and 5-hexenyl.

In the present specification, the term "an alkynyl group" (including the "alkynyl" portion in definitions) refers to a linear or branched alkynyl group having 2 to 10 carbon atoms, such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl, and 4-methyl-2-pentynyl.

In the present specification, the term "an alkylene group" (including the "alkylene" portion in definitions) refers to a linear or branched alkylene group having 1 to 10 carbon atoms, such as - CH₂-, -(CH₂)₂-, -(CH₂)₃-, -(CH₂)₄-, -(CH₂)₅-, -(CH₂)₆-, -CH(CH₃)-, -C(CH₃)₂-, - CH(C₂H₅)-, -CH(C₃H₇)-, -CH(CH(CH₃)₂)-, -(CH(CH₃))₂-, -CH₂-CH(CH₃)-, -CH(CH₃)-CH₂-, -CH₂-CH₂-C(CH₃)₂-, -C(CH₃)₂-CH₂-CH₂-, -CH₂-CH₂-CH₂-C(CH₃)₂-, and - C(CH₃)₂-CH₂-CH₂-CH₂-.

In the present specification, the term "an alkenylene group" (including the "alkenylene" portion in definitions) refers to a linear or branched alkenylene group having 2 to 10 carbon atoms, such as -CH=CH-, -CH₂-CH=CH-, -CH=CH-CH₂-, -C(CH₃)₂-CH=CH-, -CH=CH-C(CH₃)₂-, - CH₂-CH=CH-CH₂-, -CH₂-CH₂-CH=CH-, -CH=CH-CH₂-CH₂-, -CH=CH-CH=CH-, - CH=CH-CH₂-CH₂-CH₂-, and -CH₂-CH₂-CH₂-CH=CH-.

In the present specification, the term "an alkynylene group" (including the "alkynylene" portion in definitions) refers to a linear or branched alkynylene group having 2 to 10 carbon atoms, such as -C≡C-, -CH₂-C≡C-, -C≡C-CH₂-, -C(CH₃)₂-C≡C-, -C≡C-C(CH₃)₂-, -CH₂-C≡C-CH₂-, -CH₂-CH₂-C≡C-, -C≡C-CH₂-CH₂-, -C≡ C-C≡C-, -C≡C-CH₂-CH₂-CH₂-,and -CH₂-CH₂-CH₂-C≡C-.

In the present specification, the term "an alkoxy group" (including the "alkoxy" portion in definitions) refers to a linear or branched alkoxy group having 1 to 10 carbon atoms, such as methoxy, ethoxy, propoxy, isopropoxy, butoxy, isobutoxy, sec-butoxy, tert-butoxy, pentyloxy, and hexyloxy.

Here, in one embodiment, it may be an alkoxy group having 1 to 4 carbon atoms.

In the present specification, the term "an alkylcarbonyl group" refers to a monovalent group in which the above-described alkyl group is bonded to a carbonyl, such as linear or branched alkyl-CO- having 1 to 10 carbon atoms (C₁₋₁₀). Specific examples include acetyl, propionyl, pivaloyl, butanoyl, pentanoyl, hexanoyl, and heptanoyl.

In the present specification, the term "an alkoxycarbonyl group" refers to a (linear or branched alkoxy having 1 to 10 carbon atoms)-carbonyl group, such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, and hexyloxycarbonyl.

In the present specification, the term "a cycloalkyl group" (including the "cycloalkyl" portion in definitions) refers to a saturated or partially unsaturated, monocyclic or fused cycloalkyl group having 3 to 10 carbon atoms, such as cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, and adamantyl. The cycloalkyl group can have one bond at any substitutable position.

Here, in one embodiment, it may be a cycloalkyl group having 3 to 8 carbon atoms. Here, in another embodiment, it may be a cycloalkyl group having 3 to 6 carbon atoms.

In the present specification, the term "a partially unsaturated cycloalkyl group" refers to a cycloalkyl group containing a double bond in part of the ring, such as cyclic groups with one or more double bonds in the above-described saturated cycloalkyl groups. Examples thereof include groups in which one of carbon bonds constituting the ring is a double bond, such as cyclopropene, cyclobutene, cyclopentene, cyclohexene, and cycloheptene.

In the present specification, the term "a cycloalkyl divalent group" (may be referred to as a "cycloalkane-diyl group") (including the "cycloalkyl divalent group" portion in definitions) refers to a divalent group formed when the above-described "cycloalkyl group having 3 to 10 carbon atoms" has an additional bond, such as 1,3-cyclopropanediyl. The cycloalkyl divalent group can have two bonds at any substitutable positions.

Here, in one embodiment, it may be a cycloalkyl divalent group having 3 to 6 carbon atoms.

In the present specification, the term "an aryl group" (including the "aryl" portion in definitions) refers to a monocyclic or fused aryl group having 6 to 14 carbon atoms, such as phenyl, 1-naphthyl, 2-naphthyl, 1-anthryl, 2-anthryl, 9-anthryl, and indenyl. Aryl groups include those in which part of a fused ring is hydrogenated, such as a dihydroindenyl group.

Here, in one embodiment, it may be an aryl group having 6 to 10 carbon atoms (6- to 10-membered). The aryl group can have one bond at any substitutable position.

In the present specification, the term "an aryl divalent group" (including the "aryl divalent group" portion in definitions) (may be referred to as an "arylene group") refers to a divalent group formed when the above-described "aryl group" has an additional bond, which may be an aryl divalent group having 6 to 14 carbon atoms. Here, in one embodiment, it may be an aryl divalent group having 6 to 10 carbon atoms. An example thereof is phenylene. The aryl divalent group may have two bonds at any substitutable positions.

In the present specification, the term "an arylsulfonyl group" (including the "arylsulfonyl group" portion in definitions) refers to the above-described "aryl group"-SO₂- group.

In the present specification, the term "a hetero saturated ring group" (including the "hetero saturated ring" portion in definitions) (may be referred to as a "non-aromatic heterocyclic group") refers to a saturated or partially unsaturated, 3- to 8-membered monocyclic or 9- to 14-membered fused polycyclic aliphatic heterocyclic group containing 1 to 3 heteroatoms independently selected from a group consisting of oxygen, nitrogen, and sulfur, in addition to carbon atoms, as ring-constituting atoms. Here, in one embodiment, it may be a bicyclic or tricyclic aliphatic heterocyclic group. Fused polycyclic hetero saturated ring groups include those in which a cycloalkyl or hetero saturated ring group is fused or bonded via a spiro atom to a single hetero saturated ring. Specific examples include 3- to 8-membered monocyclic hetero saturated ring groups, such as aziridinyl, oxiranyl, thiiranyl, azetidinyl, oxetanyl, thietanyl, tetrahydrothienyl, tetrahydrofuranyl, pyrrolinyl, pyrrolidinyl, imidazolinyl, imidazolidinyl, oxazolinyl, oxazolidinyl, pyrazolinyl, pyrazolidinyl, thiazolinyl, thiazolidinyl, tetrahydroisothiazolyl, tetrahydrooxazolyl, tetrahydroisoxazolyl, piperidinyl, piperazinyl, tetrahydropyridinyl, dihydropyridinyl, dihydrothiopyranyl, tetrahydropyrimidinyl, tetrahydropyridazinyl, dihydropyranyl, tetrahydropyranyl, tetrahydrothiopyranyl, morpholinyl, thiomorpholinyl, azepanyl, diazepanyl, azepinyl, oxepanyl, azocanyl, and diazocanyl; and 9- to 14-membered fused polycyclic (for example, bicyclic or tricyclic) hetero saturated ring groups, such as dihydrobenzofuranyl, dihydrobenzoimidazolyl, dihydrobenzooxazolyl, dihydrobenzothiazolyl, dihydrobenzoisothiazolyl, dihydronaphtho[2,3-b]thienyl, tetrahydroisoquinolyl, tetrahydroquinolyl, 4H-quinolizinyl, indolinyl, isoindolinyl, tetrahydrothieno[2,3-c]pyridinyl, tetrahydrobenzoazepinyl, tetrahydroquinoxalinyl, tetrahydrophenanthridinyl, hexahydrophenothiazinyl, hexahydrophenoxazinyl, tetrahydrop phthalazinyl, tetrahydronaphthyridinyl, tetrahydroquinazolinyl, tetrahydrocinnolinyl, tetrahydrocarbazolyl, tetrahydro-β-carbolinyl, tetrahydroacridinyl, tetrahydrophenazinyl, tetrahydrothioxanthenyl, and octahydroisoquinolyl.

In the present specification, the term "a partially unsaturated hetero saturated ring" refers to an aliphatic heterocyclic ring containing a double bond in part of the ring, such as cyclic groups with one or more double bonds in the above-described saturated hetero saturated ring. Examples thereof include groups in which one of carbon bonds constituting the ring is a double bond, such as azinanyl, diazinanyl, or azepanyl.

The hetero saturated ring group can have one bond at any substitutable position, and may have a bond on either a carbon atom or a nitrogen atom.

In the present specification, the term "a hetero saturated ring divalent group" (including the "hetero saturated ring divalent group" portion in definitions) refers to a divalent group formed when the above-described "hetero saturated ring group" has an additional bond, such as piperazine-diyl. The hetero saturated ring divalent group can have two bonds at any substitutable positions, and may have a bond on either a carbon atom or a nitrogen atom.

In the present specification, the term "a heteroaryl group" (including the "heteroaryl" portion in definitions) (may be referred to as an "aromatic heterocyclic group") refers to, for example, a monocyclic or fused heteroaryl group (aromatic heterocyclic group) containing 1 to 6 identical or different heteroatoms selected from nitrogen, oxygen, and sulfur atoms, in addition to carbon atoms, as ring-constituting atoms, with a total of 5 to 14 ring-constituting atoms. Here, in one embodiment, the heteroaryl group may contain 1 to 4 identical or different heteroatoms selected from nitrogen, oxygen, and sulfur atoms, in addition to carbon atoms, as ring-constituting atoms, with a total of 5 to 10 ring-constituting atoms. The heteroaryl group can have one bond at any substitutable position, and may have a bond on either a carbon atom or a nitrogen atom.

Preferred examples of the "heteroaryl group" ("aromatic heterocyclic group") include 5- to 7-membered monocyclic heteroaryl groups (aromatic heterocyclic groups), such as thienyl, furyl, pyrrolyl, imidazolyl, pyrazolyl, thiazolyl, isothiazolyl, oxazolyl, isoxazolyl, pyridyl, pyrazinyl, pyrimidinyl, pyridazinyl, 1,2,4-oxadiazolyl, 1,3,4-oxadiazolyl, 1,2,4-thiadiazolyl, 1,3,4-thiadiazolyl, triazolyl, tetrazolyl, and triazinyl; and fused polycyclic heteroaryl groups (aromatic heterocyclic groups) having 8 to 14 ring-constituting atoms, such as benzothienyl, benzofuranyl, benzoimidazolyl, benzooxazolyl, benzoisoxazolyl, benzothiazolyl, benzoisothiazolyl, benzotriazolyl, imidazopyridinyl, thienopyridinyl, furopyridinyl, pyrrolopyridinyl, pyrazolopyridinyl, oxazolopyridinyl, thiazolopyridinyl, imidazopyrazinyl, imidazopyrimidinyl, thienopyrimidinyl, furopyrimidinyl, pyrrolopyrimidinyl, pyrazolopyrimidinyl, oxazolopyrimidinyl, thiazolopyrimidinyl, pyrazolotriazinyl, naphtho[2,3-b]thienyl, phenoxathiinyl, indolyl, isoindolyl, 1H-indazolyl, purinyl, isoquinolyl, quinolyl, phthalazinyl, naphthyridinyl, quinoxalinyl, quinazolinyl, cinnolinyl, carbazolyl, β-carbolinyl, phenanthridinyl, acridinyl, phenazinyl, phenothiazinyl, and phenoxazinyl. **In** one embodiment, the fused polycyclic heteroaryl group having 8 to 14 ring-constituting atoms may be bicyclic or tricyclic.

**In** the present specification, the term "a heteroaryl divalent group" (including the "heteroaryl divalent group" portion in definitions) refers to a divalent group formed when the above-described "heteroaryl group" has an additional bond, such as pyridine-diyl. The heteroaryl divalent group can have two bonds at any substitutable positions, and may have a bond on either a carbon atom or a nitrogen atom.

**In** the present specification, the term "a saturated or partially unsaturated 3- to 8-membered ring which may contain 1 to 3 atoms independently selected from nitrogen, oxygen, and sulfur atoms" refers to, among the above-described "cycloalkyl" and "hetero saturated ring" groups, those that contain 0 to 3 heteroatoms in addition to carbon atoms as ring-constituting atoms, have 3 to 8 ring-constituting atoms, and may include a double bond in part of the ring.

**In** the present specification, the term "a 6- to 10-membered aryl group" refers to, among the above-described "aryl groups," those that have 6 to 10 ring-constituting atoms.

**In** the present specification, the term "a 5- to 7-membered monocyclic heteroaryl group" refers to, among the above-described "heteroaryl groups," those that are monocyclic and have 5 to 7 ring-constituting atoms.

In the present specification, the term "a 5- to 7-membered monocyclic hetero saturated ring group" refers to, among the above-described "hetero saturated ring" groups, those that are monocyclic and have 5 to 7 ring-constituting atoms.

In the present specification, the term "a 8- to 10-membered bicyclic heteroaryl group which may be partially saturated" refers to, among the above-described "heteroaryl groups," those that are bicyclic, and have 8 to 10 ring-constituting atoms, and in which a first ring and/or a second ring may be partially saturated. Examples of partially saturated 8- to 10-membered bicyclic heteroaryl groups include dihydrobenzothienyl, dihydrobenzofuranyl, dihydrobenzoimidazolyl, dihydrobenzooxazolyl, benzothiazolyl, benzoisothiazolyl, and the like.

In the present specification, the term "a 5-4 spiro ring group which may contain 1 to 3 atoms independently selected from nitrogen, oxygen, and sulfur atoms" refers to a group in which a 5-membered ring and a 4-membered ring are bonded via a spiro atom, and which contains 0 to 3 heteroatoms in addition to carbon atoms as ring-constituting atoms. Examples of the 5-4 spiro ring portion in the 5-4 spiro ring group which may contain 1 to 3 atoms independently selected from nitrogen, oxygen, and sulfur atoms include, but are not limited to, those shown in the following

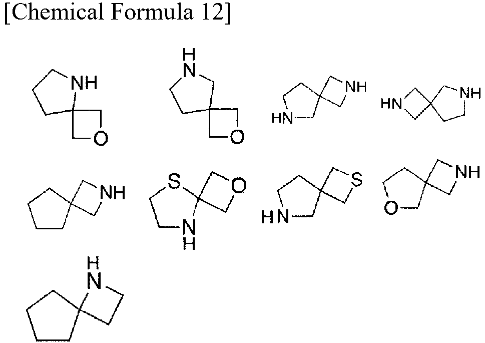

The 5-4 spiro ring group can have one bond at any substitutable position on either the 5-membered ring or the 4-membered ring, and may have a bond on either a carbon atom or a nitrogen atom. The 5-4 spiro ring group may be substituted with 1 to 5 independently selected substituents, and examples of the "substituents" include substituents selected from a [Substituent Group] described below.

In the present specification, the term "a 5-4 spiro ring divalent group which may contain 1 to 3 atoms independently selected from nitrogen, oxygen, and sulfur atoms" refers to a divalent group formed when the above-described "5-4 spiro ring group" has an additional bond. The 5-4 spiro ring divalent group may have two bonds at any substitutable positions, and may have a bond on either a carbon atom or a nitrogen atom. For example, the 5-4 spiro ring divalent group has one bond at any position on the 5-membered ring and one bond at any position on the 4-membered ring. In one embodiment, the bond at any position on the 5-membered ring is bonded to W, and the bond at any position on the 4-membered ring is bonded to R^{b}. In one embodiment, the bond at any position on the 4-membered ring is bonded to W, and the bond at any position on the 5-membered ring is bonded to R^{b}. The 5-4 spiro ring divalent group may be substituted with 1 to 5 independently selected substituents, and examples of the "substituents" include substituents selected from the [Substituent Group] described below. In one embodiment, the substituents may each be a halogen atom, an oxo group, a hydroxy group, an optionally halogenated C₁-C₆ alkoxy group (such as methoxy, chloromethoxy, or trifluoroethoxy), or an optionally halogenated C₁-C₆ alkyl group (such as methyl, chloromethyl, difluoromethyl, trifluoromethyl, ethyl, 2-bromoethyl, or 2,2,2-trifluoroethyl). In one embodiment, the substituents may each be a fluorine atom, a methyl group, an ethyl group, or an oxo group.

In the present specification, the term "a saturated, partially unsaturated, or aromatic 4- to 6-membered ring, which may contain 1 to 3 atoms independently selected from nitrogen, oxygen, and sulfur atoms," represented by Ring B refers to: among the above-described "cycloalkyl" and "heterosaturated ring" groups, those that contain 0 to 3 heteroatoms in addition to carbon atoms as ring-constituting atoms, have 4 to 6 ring-constituting atoms, and may include a double bond in part of the ring; and, among the above-described "aryl" and "heteroaryl" groups, those that contain 0 to 3 heteroatoms in addition to carbon atoms as ring-constituting atoms and have 4 to 6 ring-constituting atoms.

In the present specification, the "substituents" in: "an optionally substituted C₁₋₁₀ alkyl group," "an optionally substituted C₁₋₁₀ alkoxy group," "an optionally substituted C₁₋₁₀ alkylcarbonyl group," and "an optionally substituted C₁₋₁₀ alkoxycarbonyl group" represented by Y¹ when n is 0 in Formula (I); "an optionally substituted 6- to 10-membered aryl group," "an optionally substituted 5- to 7-membered monocyclic heteroaryl group," "an optionally substituted 5- to 7-membered monocyclic heterosaturated ring group," and "an optionally substituted, optionally partially saturated 8- to 10-membered bicyclic heteroaryl group" represented by R^{a} when n is 1 in Formula (I); "an optionally substituted 6- to 10-membered aryl group," "an optionally substituted 5- to 7-membered monocyclic heteroaryl group," "an optionally substituted 5- to 7-membered monocyclic saturated heterocyclic ring group," and "an optionally substituted or partially saturated 8- to 10-membered bicyclic heteroaryl group" represented by R^{b} in Formula (II); "an optionally substituted 6- to 10-membered aryl group," "an optionally substituted 5- to 7-membered monocyclic heteroaryl group," "an optionally substituted 5- to 7-membered monocyclic saturated heterocyclic ring group," and "an optionally substituted or partially saturated 8- to 10-membered bicyclic heteroaryl group" represented by R^{c} in Formula (III); "an optionally substituted 6- to 10-membered aryl group," "an optionally substituted 5- to 7-membered monocyclic heteroaryl group," "an optionally substituted 5- to 7-membered monocyclic saturated heterocyclic ring group," and "an optionally substituted or partially saturated 8- to 10-membered bicyclic heteroaryl group" represented by R^{d} in Formula (IV); and "an optionally substituted group or ring" in definitions regarding other compounds, can be any substituents, and examples thereof include substituents selected from the following [Substituent Group]. In the general synthesis method below, the substituents R^{S1} and R^{S2} are each independently any substituent selected from the [Substituent Group] below.

1 to 5 or 1 to 3 such "substituents" can be present at any substitutable positions, and when the number of substituents is 2 or more, the substituents may be the same or different.

For each group or ring or the like, when a specific description is provided regarding a "substituent" thereof, that description shall take precedence.

### [Substituent Group]

(1) Halogen atom,
(2) Nitro group,
(3) Cyano group,
(4) Oxo group,
(5) Hydroxy group,
(6) C₁-C₆ alkoxy group optionally substituted with one or more substituents (for example, methoxy, chloromethoxy, trifluoroethoxy, tert-butoxycarbonylmethyloxy),
(7) C₃-C₁₀ cycloalkyl-oxy group optionally substituted with one or more substituents (for example, cyclopropyloxy, cyclobutyloxy, optionally substituted with tert-butoxycarbonyl),
(8) C₆-C₁₄ aryloxy group optionally substituted with one or more substituents (for exampl, phenoxy, naphthoxy, each optionally substituted with tert-butoxycarbonyl),
(9) C₇-C₁₆ aralkyloxy group optionally substituted with one or more substituents (for example, benzyloxy optionally substituted with tert-butoxycarbonyl),
(10) C₃-C₁₄ heterosaturated ring-oxy group optionally substituted with one or more substituents (for example, piperazinyloxy optionally substituted with tert-butoxycarbonyl),
(11) C₅-C₁₄ heteroaryl-oxy group optionally substituted with one or more substituents (for example, pyridinyl, triazolyl, each optionally substituted with tert-butoxycarbonyl),
(12) C₁-C₆ alkyl-carbonyloxy group optionally substituted with one or more substituents (for example, acetoxy, propanoyloxy, each optionally substituted with tert-butoxycarbonyl),
(13) C₃-C₁₀ cycloalkyl-carbonyloxy group optionally substituted with one or more substituents (for example, cyclopropylcarbonyloxy, cyclobutylcarbonyloxy, each optionally substituted with tert-butoxycarbonyl),
(14) C₆-C₁₄ aryl-carbonyloxy group optionally substituted with one or more substituents (for example, benzoyloxy, 1-naphthoyloxy, 2-naphthoyloxy, each optionally substituted with tert-butoxycarbonyl),
*(15)* C₁-C₆ alkoxy-carbonyloxy group optionally substituted with one or more substituents (for example, methoxycarbonyloxy, ethoxycarbonyloxy, propoxycarbonyloxy, butoxycarbonyloxy),
(16) C₃-C₁₄ heterosaturated ring-carbonyloxy group optionally substituted with one or more substituents (for example, piperazinylcarbonyloxy optionally substituted with tert-butoxycarbonyl),
(17) C₅-C₁₄ heteroaryl-carbonyloxy group optionally substituted with one or more substituents (for example, pyridinylcarbonyloxy, triazolylcarbonyloxy, each optionally substituted with tert-butoxycarbonyl),
(18) 5- to 14-membered heteroaryl group (aromatic heterocyclic group) optionally substituted with one or more substituents (for example, triazolyl optionally substituted with a (tert-butoxycarbonylamino)methyl group, pyridinyl optionally substituted with a tert-butoxycarbonyl group),
(19) 3- to 14-membered heterosaturated ring group (non-aromatic heterocyclic group) optionally substituted with one or more substituents (for example, piperidinyl, piperazinyl, each optionally substituted with tert-butoxycarbonyl),
(20) Formyl group,
(21) Carboxy group,
(22) C₁-C₆ alkyl-carbonyl group optionally substituted with one or more substituents (for example, acetyl, chloroacetyl, trifluoroacetyl),
(23) C₃-C₁₀ cycloalkyl-carbonyl group optionally substituted with one or more substituents (for example, cyclopropylcarbonyl, cyclobutylcarbonyl),
(24) C₆-C₁₄ aryl-carbonyl group optionally substituted with one or more substituents (for example, benzoyl, 1-naphthoyl, 2-naphthoyl, each optionally substituted with tert-butoxycarbonyl),
(25) C₁-C₆ alkoxy-carbonyl group optionally substituted with one or more substituents (for example, methoxycarbonyl, ethoxycarbonyl, tert-butoxycarbonyl, benzyloxycarbonyl),
(26) C₃-C₁₄ heterosaturated ring-carbonyl group optionally substituted with one or more substituents (for example, piperazinylcarbonyl optionally substituted with tert-butoxycarbonyl),
(27) C₅-C₁₄ heteroaryl-carbonyl group optionally substituted with one or more substituents (for example, pyridinylcarbonyl, triazolylcarbonyl, each optionally substituted with tert-butoxycarbonyl),
(28) Carbamoyl group or mono- or di-alkylcarbamoyl group (for example, N-methylcarbamoyl group),
(29) Amino group,
(30) Mono- or di-C₁-C₆ alkylamino group optionally substituted with one or more substituents (for example, methylamino, ethylamino, propylamino, isopropylamino, butylamino, dimethylamino, diethylamino, dipropylamino, dibutylamino, N-ethyl-N-methylamino, acetylamino),
(31) Mono- or di-C₃-C₁₀ cycloalkyl-amino group optionally substituted with one or more substituents (for example, cyclopropylamino, cyclobutylamino, each optionally substituted with tert-butoxycarbonyl),
(32) Mono- or di-C₆-C₁₄ arylamino group optionally substituted with one or more substituents (for example, phenylamino optionally substituted with tert-butoxycarbonyl),
(33) Mono- or di-C₃-C₁₄ heterosaturated ring amino group optionally substituted with one or more substituents (for example, piperazinylamino optionally substituted with tert-butoxycarbonyl),
(34) Mono- or di-C₅-C₁₄ heteroaryl amino group optionally substituted with one or more substituents (for example, pyridinylamino, triazolylamino, each optionally substituted with tert-butoxycarbonyl),
(35) Formylamino group,
(36) C₁-C₆ alkyl-carbonylamino group optionally substituted with one or more substituents (for example, acetylamino, propanoylamino, butanoylamino),
(37) C₁-C₆ alkoxy-carbonylamino group optionally substituted with one or more substituents (for example, methoxycarbonylamino, ethoxycarbonylamino, propoxycarbonylamino, butoxycarbonylamino, tert-butoxycarbonylamino),
(38) C₃-C₁₀ cycloalkyl-carbonylamino group optionally substituted with one or more substituents (for example, cyclopropylcarbonylamino, cyclobutylcarbonylamino),
(39) C₆-C₁₄ aryl-carbonylamino group optionally substituted with one or more substituents (for example, benzoylamino, 1-naphthoylamino, 2-naphthoylamino),
(40) C₃-C₁₄ heterosaturated ring-carbonylamino group optionally substituted with one or more substituents (for example, piperazinylcarbonylamino optionally substituted with tert-butoxycarbonyl),
(41) C₅-C₁₄ heteroaryl-carbonylamino group optionally substituted with one or more substituents (for example, pyridinylcarbonylamino, triazolylcarbonylamino, each optionally substituted with tert-butoxycarbonyl),
(42) C₁-C₁₀ alkyl group optionally substituted with one or more substituents (for example, methyl, chloromethyl, difluoromethyl, trifluoromethyl, ethyl, 2-bromoethyl, 2,2,2-trifluoroethyl, tert-butoxycarbonylmethyl, tert-butoxycarbonylethyl, tert-butoxycarbonylaminomethyl, piperazinylmethyl optionally substituted with tert-butoxycarbonyl, hexyl optionally substituted with a carboxy group, hexyl optionally substituted with benzyloxycarbonyl),
(43) C₂-C₁₀ alkenyl group optionally substituted with one or more substituents (for example, ethenyl, 1-propenyl, 2-propenyl, 2-methyl-1-propenyl, 1-butenyl, tert-butoxycarbonylethenyl, benzyloxycarbonylheptenyl),
(44) C₂-C₁₀ alkynyl group optionally substituted with one or more substituents (for example, ethynyl, 1-propynyl, 2-propynyl, 1-butynyl, 2-butynyl, 3-butynyl, benzyloxycarbonylheptynyl),
(45) C₃-C₁₀ cycloalkyl group optionally substituted with one or more substituents (for example, cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, adamantyl, cyclopropenyl, cyclobutenyl, cyclopentenyl, cyclohexenyl, cycloheptenyl, cyclooctenyl),
(46) C₆-C₁₄ aryl group optionally substituted with one or more substituents (for example, phenyl, naphthyl, each optionally substituted with a tert-butoxycarbonylamino group).

When each group in the above Substituent Group is substituted, substituents thereof can be any substituents, for example, can be 1 to 5 substituents independently selected from the above [Substituent Group]. When substituents of each group in the above Substituent Group are substituted, substituents thereof can be any substituents, for example, can be 1 to 5 substituents independently selected from the above [Substituent Group].

In the present specification, examples of "substituents" in: "an optionally substituted C₁₋₁₀ alkyl group," "an optionally substituted C₁₋₁₀ alkoxy group," "an optionally substituted C₁₋₁₀ alkylcarbonyl group," and "an optionally substituted C₁₋₁₀ alkoxycarbonyl group" represented by Y¹ when n is 0 in Formula (I); "an optionally substituted 6- to 10-membered aryl group," "an optionally substituted 5- to 7-membered monocyclic heteroaryl group," "an optionally substituted 5- to 7-membered monocyclic saturated heterocyclic ring group," and "an optionally substituted and partially saturated 8- to 10-membered bicyclic heteroaryl group" represented by R^{a} when n is 1 in Formula (I); "an optionally substituted 6- to 10-membered aryl group," "an optionally substituted 5- to 7-membered monocyclic heteroaryl group," "an optionally substituted 5- to 7-membered monocyclic saturated heterocyclic ring group," and "an optionally substituted and partially saturated 8- to 10-membered bicyclic heteroaryl group" represented by R^{b} in Formula (II); "an optionally substituted 6- to 10-membered aryl group," "an optionally substituted 5- to 7-membered monocyclic heteroaryl group," "an optionally substituted 5- to 7-membered monocyclic saturated heterocyclic ring group," and "an optionally substituted and partially saturated 8- to 10-membered bicyclic heteroaryl group" represented by R^{c} in Formula (III); and "an optionally substituted 6- to 10-membered aryl group," "an optionally substituted 5-to 7-membered monocyclic heteroaryl group," "an optionally substituted 5- to 7-membered monocyclic saturated heterocyclic ring group," and "an optionally substituted and partially saturated 8- to 10-membered bicyclic heteroaryl group," also include L having a first terminal group or substituted L. L is a bond or a chemical linker. L having a first terminal group or substituted L, as a "substituent," can be present at any substitutable position in one or more (for example, 1 to 3) instances.

In the present specification, a "chemical linker" can be appropriately determined by a person skilled in the art depending on a selection of a target protein. Details will be described later.

In the present specification, an example of a "substituent" in "substituted L" is TBL.

In the present specification, the term "TBL" refers to a group having a moiety capable of binding, or a moiety that binds, to a target protein. Details will be described later.

[A compound represented by Formula (I), Formula (II), Formula (III), or Formula (IV), or a pharmacologically acceptable salt thereof]

A compound represented by Formula (I), Formula (II), Formula (III), or Formula (IV), or a pharmacologically acceptable salt thereof, which is the first embodiment of the present invention, includes, as an aspect (1), a compound represented by the following Formula (I): 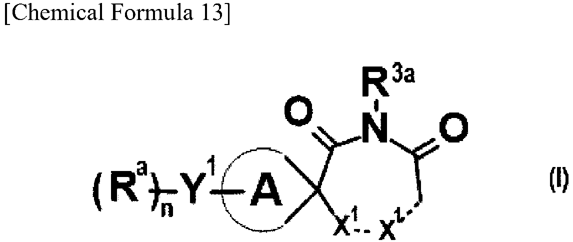 [wherein
a dotted line represents a single bond or a double bond,
two X¹ groups are independently selected from CH and CH₂, or one X¹ is CH₂ and the other X¹ is O, NH, NCH₃, or S,
Ring A is a saturated or partially unsaturated 3- to 8-membered ring that may contain 1 to 3 atoms independently selected from nitrogen, oxygen, and sulfur atoms, and the ring may be substituted with one or more substituents independently selected from a group consisting of a fluorine atom, a methyl group, an ethyl group, and an oxo group,
R^{3a} is a hydrogen atom, a methyl group, -OH, -CH₂OC(O)R'^{a}, -CH₂OP(O)OHOR'^{a}, - CH₂OP(O)R'^{a}₂, or -CH₂OP(O)(OR'^{a})₂,
R'^{a} is a C₁₋₄ alkyl group,
n is 0 or 1,
when n is 0,
Y¹ is an optionally substituted C₁₋₁₀ alkyl group, an optionally substituted C₁₋₁₀ alkoxy group, an optionally substituted C₁₋₁₀ alkylcarbonyl group, or an optionally substituted C₁₋₁₀ alkoxycarbonyl group,
when n is 1,
Y¹ is a single bond, CO, SO₂, CH₂CO, or a C₁₋₄ alkylene group, and
R^{a} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group].
or the following Formula (II): 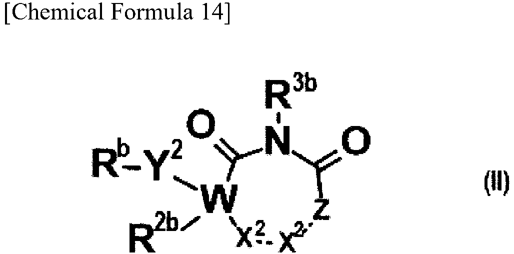
[wherein
a dotted line represents a single bond or a double bond,
two X² groups are independently selected from CH and CH₂, or one X² is CH₂ and the other X² is O, NH, NCH₃, or S,
Z is a single bond or CH₂,
R^{2b} is a hydrogen atom, a fluorine atom, or a methyl group,
R^{3b} is a hydrogen atom, a methyl group, -OH, -CH₂OC(O)R'^{b}, -CH₂OP(O)OHOR'^{b}, - CH₂OP(O)R'^{b}₂, or -CH₂OP(O)(OR'^{b})₂,
R'^{b} is a C₁₋₄ alkyl group,
when Z is CH₂,
W is C or N, when W is N, R^{2b} is absent,
Y² is a single bond, -CR"R"'-, -O-, -NR"-, -NR"C(O)-, -C(O)NR"-, -NR"SO₂-, -SO₂NR"-, - CH₂NR"-, -NR"CH₂-, -C(O)NR''CH₂-, -CH₂NR''C(O)-, -CH₂N(COR")-, -N(COR")CH₂-, - CH₂N(COOR")-, or -N(COOR")CH₂-,
R" and R‴ are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms,
R^{b} is an optionally substituted 6- to 10-membered aryl group (provided that when W is N, a 6-membered aryl group is excluded), an optionally substituted 5- to 7-membered monocyclic heteroaryl group, an optionally substituted 5- to 7-membered monocyclic heterosaturated ring group (provided that when W is N, a tetrahydrofuranyl group is excluded), an optionally substituted and optionally partially saturated 8- to 10-membered bicyclic heteroaryl group (provided that when W is C, the following structures: 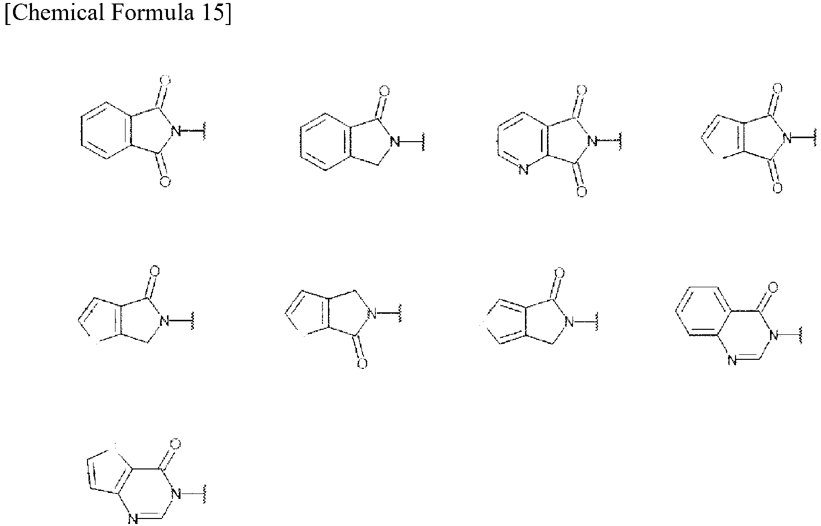
(wherein the ring structures are unsubstituted or substituted with 1 to 5 arbitrary substituents) are excluded), or an optionally substituted 5-4 spiro ring group which may contain 1 to 3 atoms independently selected from nitrogen, oxygen, and sulfur atoms,
when Z is a single bond,
W is C,
Y² is an optionally substituted 5-4 spiro ring divalent group which may contain 1 to 3 atoms independently selected from nitrogen, oxygen, and sulfur atoms, wherein the 5-4 spiro ring is bonded to W at any position of its 5-membered ring, and
R^{b} is an optionally substituted C₁₋₁₀ alkyl group, an optionally substituted C₁₋₁₀ alkylcarbonyl group, an optionally substituted C₁₋₁₀ alkoxy group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or the following Formula (III): 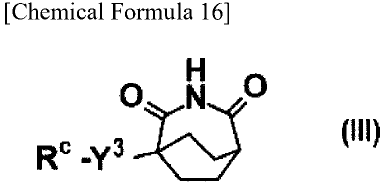
[wherein
Y³ is a single bond or -NH-, and
R^{c} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or the following Formula (IV): 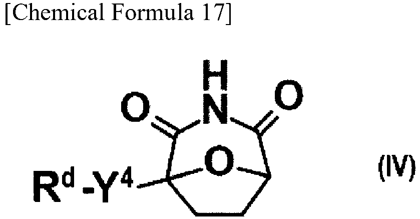
[wherein
Y⁴ is a single bond, and
R^{d} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or a pharmacologically acceptable salt thereof (provided that the following compounds: 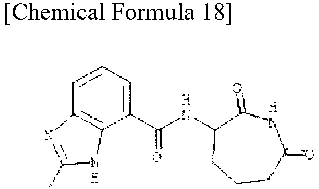 and 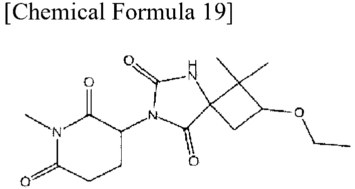
are excluded).

Further, in the above aspect (1), as an aspect (2), the compound or a pharmacologically acceptable salt thereof is provided wherein, the compound represented by the above Formula (I) is a compound represented by the following Formula (IA): 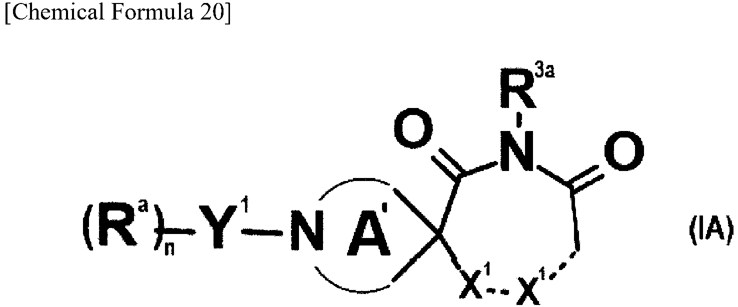 [wherein
a dotted line represents a single bond or a double bond,
two X¹ groups are independently selected from CH and CH₂, or one X¹ is CH₂ and the other X¹ is O, NH, NCH₃, or S,
Ring A' is a 3- to 8-membered monocyclic heterosaturated ring containing a nitrogen atom bonded to Y¹ and optionally containing 1 to 2 atoms independently selected from nitrogen, oxygen, and sulfur atoms, and the ring may be substituted with 1 to 2 substituents independently selected from a group consisting of a fluorine atom, a methyl group, an ethyl group, and an oxo group,
R^{3a} is a hydrogen atom, a methyl group, or -OH,
n is 0 or 1,
when n is 0,
Y¹ is an optionally substituted C₁₋₁₀ alkyl group, an optionally substituted C₁₋₁₀ alkoxy group, an optionally substituted C₁₋₁₀ alkylcarbonyl group, or an optionally substituted C₁₋₁₀ alkoxycarbonyl group,
when n is 1,
Y¹ is a single bond, CO, SO₂, -CH₂CO-, or a C₁₋₄ alkylene group, and
R^{a} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group].

Further, in the above aspect (2), as a preferred aspect (3), the compound or a pharmacologically acceptable salt thereof is provided wherein, in the above Formula (IA),
a dotted line represents a single bond or a double bond,
two X¹ groups are independently selected from CH and CH₂, or one X¹ is CH₂ and the other X¹ is O,
Ring A' is a 3- to 8-membered monocyclic heterosaturated ring containing a nitrogen atom bonded to Y¹ and optionally containing 1 to 2 atoms independently selected from nitrogen, oxygen, and sulfur atoms, and the ring may be substituted with 1 to 2 substituents independently selected from a group consisting of a fluorine atom, a methyl group, an ethyl group, and an oxo group,
R^{3a} is a hydrogen atom,
n is 1,
Y¹ is a single bond, CO, SO₂, -CH₂CO-, or a C₁₋₄ alkylene group, and
R^{a} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

Further, in the above aspect (3), as a preferred aspect (4), the compound or a pharmacologically acceptable salt thereof is provided wherein, in the above Formula (IA),
a dotted line represents a single bond,
two X¹ groups are CH₂,
Ring A' is a 4- to 6-membered monocyclic heterosaturated ring containing a nitrogen atom bonded to Y¹ and optionally containing 1 to 2 atoms independently selected from nitrogen and oxygen atoms, and the ring may be substituted with 1 to 2 substituents independently selected from a group consisting of a fluorine atom, a methyl group, an ethyl group, and an oxo group,
R^{3a} is a hydrogen atom,
n is 1,
Y¹ is a single bond, CO, SO₂, -CH₂CO-, or a C₁₋₄ alkylene group, and
R^{a} is an optionally substituted phenyl group, an optionally substituted naphthyl group, an optionally substituted dihydroindenyl group, an optionally substituted 5- to 6-membered monocyclic heteroaryl group, an optionally substituted 5- to 6-membered monocyclic heterosaturated ring group, or a bicyclic heteroaryl group selected from a group consisting of the following: 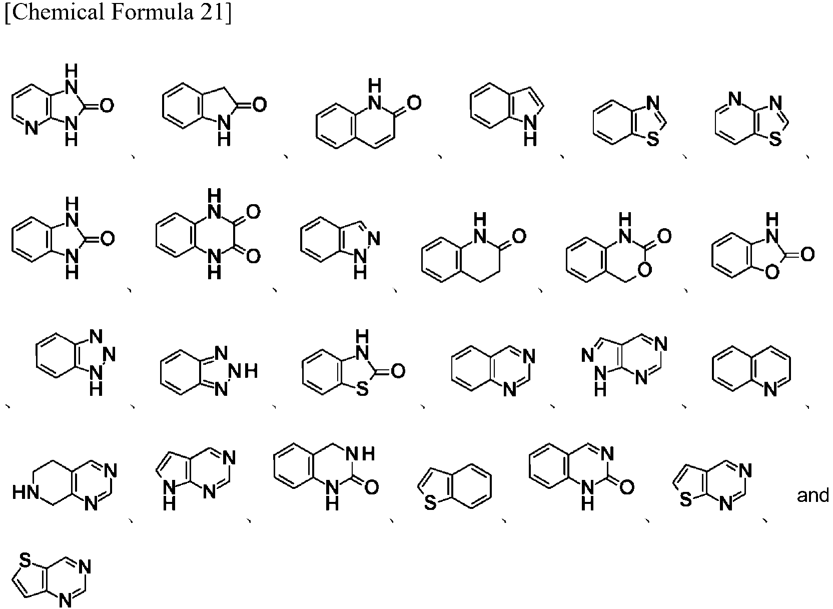
(wherein the bicyclic heteroaryl group may be substituted).

Further, in any one of the above aspects (2) to (4), as an aspect (5), the compound or a pharmacologically acceptable salt thereof is provided wherein, in the above Formula (IA), Ring A' is a 5- to 6-membered monocyclic heterosaturated ring containing a nitrogen atom bonded to Y¹, and the ring may be substituted with 1 to 2 oxo groups.

Further, in the above aspect (5), as a preferred aspect (6), the compound or a pharmacologically acceptable salt thereof is provided wherein, in the above Formula (IA),

Ring A' is a 5-membered monocyclic heterosaturated ring containing a nitrogen atom bonded to Y¹, and the ring may be substituted with 1 to 2 oxo groups.

Further, in any one of the above aspects (2) to (6), as an aspect (7), the compound or a pharmacologically acceptable salt thereof is provided wherein, in the above Formula (IA),
n is 1, and
Y¹ is a single bond or CO.

Further, in any one of the above aspects (3) to (7), as an aspect (8), the compound or a pharmacologically acceptable salt thereof is provided wherein, in the above Formula (IA),
n is 1, and
R^{a} is an optionally substituted phenyl group, an optionally substituted naphthyl group, an optionally substituted pyridinyl group, an optionally substituted pyridazinyl group, an optionally substituted pyrimidinyl group, an optionally substituted pyrazinyl group, an optionally substituted thiazolyl group, an optionally substituted piperidinyl group, an optionally substituted piperazinyl group, or a bicyclic heteroaryl group selected from a group consisting of the following: 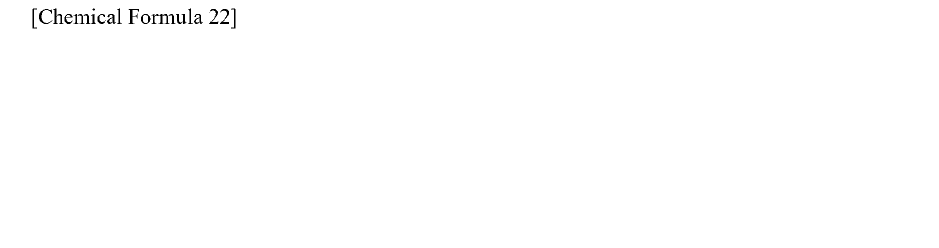 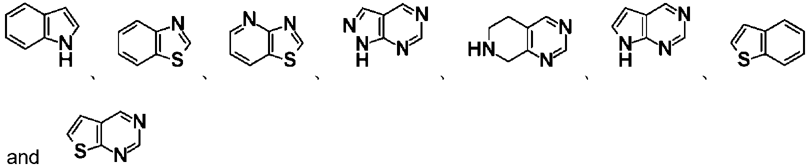
(wherein the bicyclic heteroaryl group may be substituted).

Further, in any one of the above aspects (3) to (8), as an aspect (9), the compound or a pharmacologically acceptable salt thereof is provided wherein, in the above Formula (IA),
n is 1,
R^{a} is an optionally substituted nitrogen-containing heterocycle, and Ra has a bond to Y¹ on a nitrogen atom that is a member of the ring.

Further, in the above aspect (1), as an aspect (10), the compound or a pharmacologically acceptable salt thereof is provided wherein, the compound is a compound represented by the above Formula (II), and in the above Formula (II),
a dotted line represents a single bond or a double bond,
two X² groups are independently selected from CH and CH₂, or one X² is CH₂ and the other X² is O, NH, NCH₃, or S,
Z is a single bond,
R^{2b} is a hydrogen atom, a fluorine atom, or a methyl group,
R^{3b} is a hydrogen atom, a methyl group, or -OH,
W is C,
Y² is an optionally substituted 5-4 spiro ring divalent group which may contain 1 to 3 atoms independently selected from nitrogen, oxygen, and sulfur atoms, wherein the 5-4 spiro ring is bonded to W at any position of its 5-membered ring, and
R^{b} is an optionally substituted C₁₋₁₀ alkyl group, an optionally substituted C₁₋₁₀ alkylcarbonyl group, an optionally substituted C₁₋₁₀ alkoxy group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

Further, in the above aspect (1), as an aspect (11), the compound or a pharmacologically acceptable salt thereof is provided wherein, the compound is a compound represented by the above Formula (II), and in the above Formula (II),
a dotted line represents a single bond or a double bond,
two X² groups are independently selected from CH and CH₂, or one X² is CH₂ and the other X² is O, NH, NCH₃, or S,
Z is CH₂,
R^{2b} is a hydrogen atom, a fluorine atom, or a methyl group,
R^{3b} is a hydrogen atom, a methyl group, or -OH,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R‴ are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted 5-4 spiro ring group which may contain 1 to 3 atoms independently selected from nitrogen, oxygen, and sulfur atoms.

Further, in the above aspect (1), as an aspect (12), the compound or a pharmacologically acceptable salt thereof is provided wherein, the compound is a compound represented by the above Formula (II), and in the above Formula (II),
a dotted line represents a single bond or a double bond,
two X² groups are independently selected from CH and CH₂, or one X² is CH₂ and the other X² is O, NH, NCH₃, or S,
Z is CH₂,
R^{2b} is a hydrogen atom, a fluorine atom, or a methyl group,
R^{3b} is a hydrogen atom, a methyl group, or -OH,
W is N, R^{2b} is absent,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R‴ are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted naphthyl group, an optionally substituted 5- to 7-membered monocyclic heteroaryl group, an optionally substituted 5- to 7-membered monocyclic heterosaturated ring group (provided that a tetrahydrofuranyl group is excluded), an optionally substituted and optionally partially saturated 8- to 10-membered bicyclic heteroaryl group, or an optionally substituted 5-4 spiro ring group which may contain 1 to 3 atoms independently selected from nitrogen, oxygen, and sulfur atoms.

Further, in the above aspect (10), as a preferred aspect (13), the compound or a pharmacologically acceptable salt thereof is provided wherein, in the above Formula (II),
a dotted line represents a single bond or a double bond,
two X² groups are independently selected from CH and CH₂, or one X² is CH₂ and the other X² is O,
Z is a single bond,
R^{2b} is a hydrogen atom, a fluorine atom, or a methyl group,
R^{3b} is a hydrogen atom,
W is C,
Y² is an optionally substituted 5-4 spiro ring divalent group which may contain 1 to 3 atoms independently selected from nitrogen, oxygen, and sulfur atoms, wherein the 5-4 spiro ring is bonded to W at any position of its 5-membered ring, and
R^{b} is an optionally substituted C₁₋₁₀ alkyl group, an optionally substituted C₁₋₁₀ alkylcarbonyl group, an optionally substituted C₁₋₁₀ alkoxy group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

Further, in the above aspect (13), as a preferred aspect (14), the compound or a pharmacologically acceptable salt thereof is provided wherein, in the above Formula (II),
a dotted line represents a single bond,
two X² groups are CH₂,
Z is a single bond,
R^{2b} is a hydrogen atom, or a methyl group,
R^{3b} is a hydrogen atom,
W is C,
Y² is selected from a group consisting of the following: 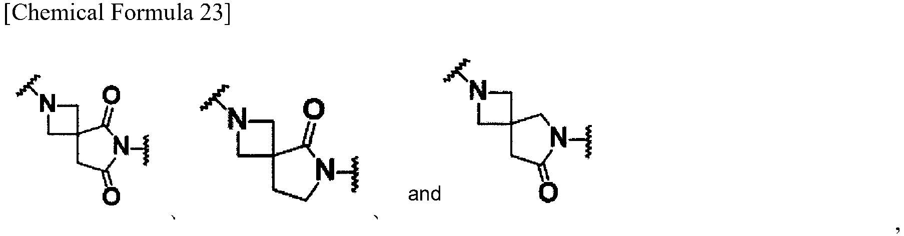
wherein a wavy line represents a point of attachment to W and R^{b}, and
R^{b} is an optionally substituted C₁₋₁₀ alkyl group, an optionally substituted C₁₋₁₀ alkylcarbonyl group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

Further, in the above aspect (14), as a preferred aspect (15), the compound or a pharmacologically acceptable salt thereof is provided wherein, the compound represented by the above Formula (II) is selected from a group consisting of the following: 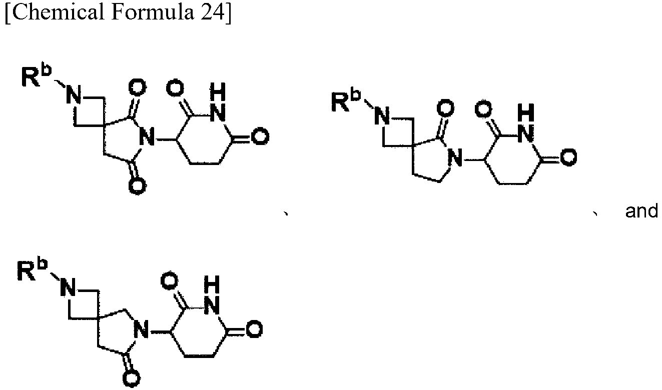 wherein
R^{b} is an optionally substituted C₁₋₁₀ alkyl group, an optionally substituted C₁₋₁₀ alkylcarbonyl group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

Further, in any one of the above aspects (13) to (15), as an aspect (16), the compound or a pharmacologically acceptable salt thereof is provided wherein, in the above Formula (II),
R^{b} is an optionally substituted C₁₋₈ alkyl group, a tert-butoxycarbonyl group, an optionally substituted benzoyl group, an optionally substituted phenyl group, an optionally substituted phenylsulfonyl group, an optionally substituted pyridinyl group, an optionally substituted pyrimidinyl group, an optionally substituted piperidinyl group, or a bicyclic heteroaryl group of the following: 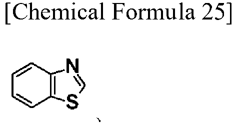 or 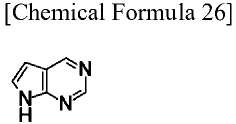
(wherein the bicyclic heteroaryl groups may be substituted).

Further, in any one of the above aspects (13) to (16), as an aspect (17), the compound or a pharmacologically acceptable salt thereof is provided wherein, in the above Formula (II), R^{b} is an optionally substituted nitrogen-containing heterocycle, and R^{b} has a bond to Y² on a nitrogen atom that is a member of the ring.

Further, in the above aspect (11), as a preferred aspect (18), the compound or a pharmacologically acceptable salt thereof is provided wherein, in the above Formula (II),
a dotted line represents a single bond or a double bond,
two X² groups are independently selected from CH and CH₂, or one X² is CH₂ and the other X² is O,
Z is CH₂,
R^{2b} is a hydrogen atom, a fluorine atom, or a methyl group,
R^{3b} is a hydrogen atom,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R‴ are each independently a hydrogen atom or a methyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted 5-4 spiro ring group which may contain 1 to 3 atoms independently selected from nitrogen, oxygen, and sulfur atoms.

Further, in the above aspect (18), as a preferred aspect (19), the compound or a pharmacologically acceptable salt thereof is provided wherein, in the above Formula (II),
a dotted line represents a single bond or a double bond,
two X² groups are CH₂,
Z is CH₂,
R^{2b} is a hydrogen atom, a fluorine atom, or a methyl group,
R^{3b} is a hydrogen atom,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R‴ are each independently a hydrogen atom or a methyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted phenyl group, an optionally substituted naphthyl group, an optionally substituted dihydroindenyl group, an optionally substituted 5- to 6-membered monocyclic heteroaryl group, an optionally substituted 5- to 6-membered monocyclic heterosaturated ring group, or a 5-4 spiro ring group of the following: 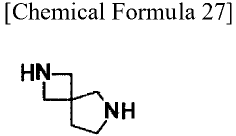
(wherein the 5-4 spiro ring group may be substituted).

Further, in the above aspect (19), as a preferred aspect (20), the compound or a pharmacologically acceptable salt thereof is provided wherein, in the above Formula (II),
R^{2b} is a hydrogen atom or a fluorine atom,
Y² is a single bond, and
R^{b} is an optionally substituted phenyl group, an optionally substituted dihydroindenyl group, an optionally substituted 5- to 6-membered monocyclic heteroaryl group, an optionally substituted 5- to 6-membered monocyclic heterosaturated ring group, or a 5-4 spiro ring group of the following: 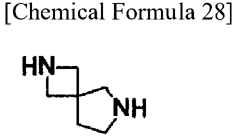
(wherein the 5-4 spiro ring group may be substituted).

Further, in the above aspect (20), as a preferred aspect (21), the compound or a pharmacologically acceptable salt thereof is provided wherein, in the above Formula (II),
R^{b} is an optionally substituted phenyl group, a 5- to 6-membered monocyclic heteroaryl group optionally substituted with an oxo group (wherein the monocyclic heteroaryl group may be further substituted with a substituent other than an oxo group), a 5- to 6-membered monocyclic heterosaturated ring group optionally substituted with an oxo group (wherein the monocyclic heterosaturated ring group may be further substituted with a substituent other than an oxo group), or a 5-4 spiro ring group selected from the following: 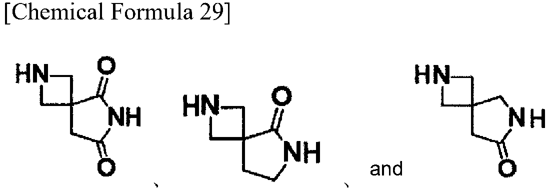
(wherein the 5-4 spiro ring group may be further substituted with a substituent other than an oxo group).

Further, in any one of the above aspects (18) to (21), as an aspect (22), the compound or a pharmacologically acceptable salt thereof is provided wherein, in the above Formula (II),
R^{b} is an optionally substituted phenyl group, an optionally substituted pyridinyl group, an optionally substituted pyridazinyl group, an optionally substituted triazolyl group, an optionally substituted pyrrolidinyl group, an optionally substituted piperazinyl group, or a 5-4 spiro ring group of the following: 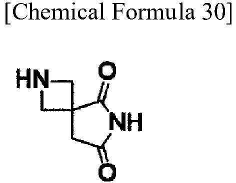
(wherein the 5-4 spiro ring group may be further substituted).

Further, in any one of the above aspects (18) to (22), as an aspect (23), the compound or a pharmacologically acceptable salt thereof is provided wherein, in the above Formula (II),
R^{b} is an optionally substituted nitrogen-containing heterocycle, and R^{b} has a bond to Y² on a nitrogen atom that is a member of the ring.

Further, in the above aspect (12), as a preferred aspect (24), the compound or a pharmacologically acceptable salt thereof is provided wherein, in the above Formula (II),
a dotted line represents a single bond or a double bond,
two X² groups are independently selected from CH and CH₂, or one X² is CH₂ and the other X² is O,
Z is CH₂,
R^{3b} is a hydrogen atom,
W is N, R^{2b} is absent,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R"' are each independently a hydrogen atom or a methyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted naphthyl group, an optionally substituted dihydroindenyl group, an optionally substituted 5- to 7-membered monocyclic heteroaryl group, an optionally substituted 5- to 7-membered monocyclic heterosaturated ring group (provided that a tetrahydrofuranyl group is excluded), an optionally substituted and optionally partially saturated 8- to 10-membered bicyclic heteroaryl group, or an optionally substituted 5-4 spiro ring group which may contain 1 to 3 atoms independently selected from nitrogen, oxygen, and sulfur atoms.

Further, in the above aspect (24), as a preferred aspect (25), the compound or a pharmacologically acceptable salt thereof is provided wherein, in the above Formula (II),
a dotted line represents a single bond or a double bond,
two X² groups are CH₂,
Z is CH₂,
R^{3b} is a hydrogen atom,
W is N, R^{2b} is absent,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R‴ are each independently a hydrogen atom or a methyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted naphthyl group, an optionally substituted dihydroindenyl group, an optionally substituted 5- to 6-membered monocyclic heteroaryl group, an optionally substituted 5- to 6-membered monocyclic heterosaturated ring group (provided that a tetrahydrofuranyl group is excluded), a bicyclic heteroaryl group selected from a group consisting of the following: 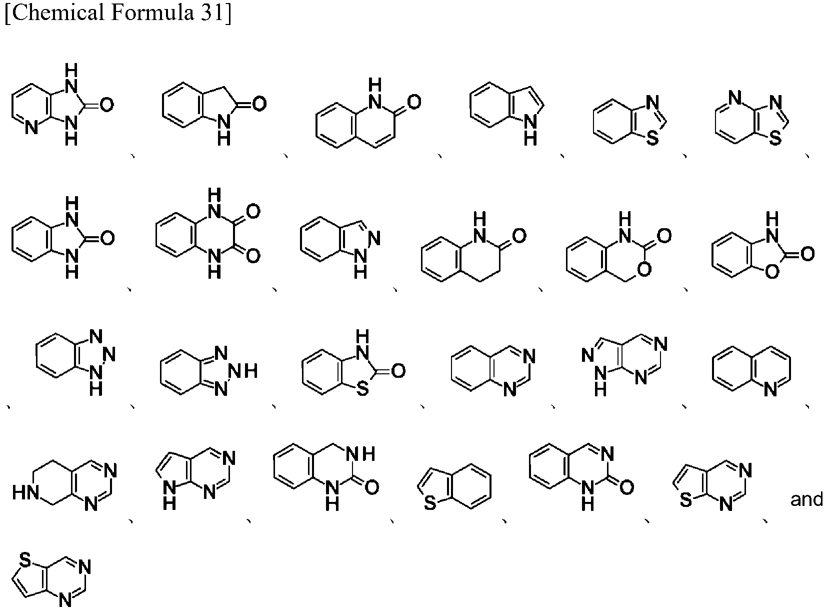
(wherein the bicyclic heteroaryl group may be substituted), or a 5-4 spiro ring group of the following: 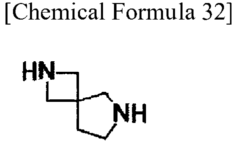
(wherein the 5-4 spiro ring group may be substituted).

Further, in the above aspect (24) or (25), as an aspect (26), the compound or a pharmacologically acceptable salt thereof is provided wherein, in the above Formula (II),
R^{b} is an optionally substituted nitrogen-containing heterocycle, and R^{b} has a bond to Y¹ on a nitrogen atom that is a member of the ring.

Further, in the above aspect (1), as an aspect (27), the compound or a pharmacologically acceptable salt thereof is provided wherein, the compound is a compound represented by the above Formula (II), and in the above Formula (II),
a dotted line represents a single bond or a double bond,
two X² groups are independently selected from CH and CH₂, or one X² is CH₂ and the other X² is O, NH, NCH₃, or S,
R^{2b} is a hydrogen atom, a fluorine atom, or a methyl group,
R^{3b} is a hydrogen atom, a methyl group, or -OH,
Z is CH₂,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R‴ are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is a bicyclic group selected from a group consisting of the following: 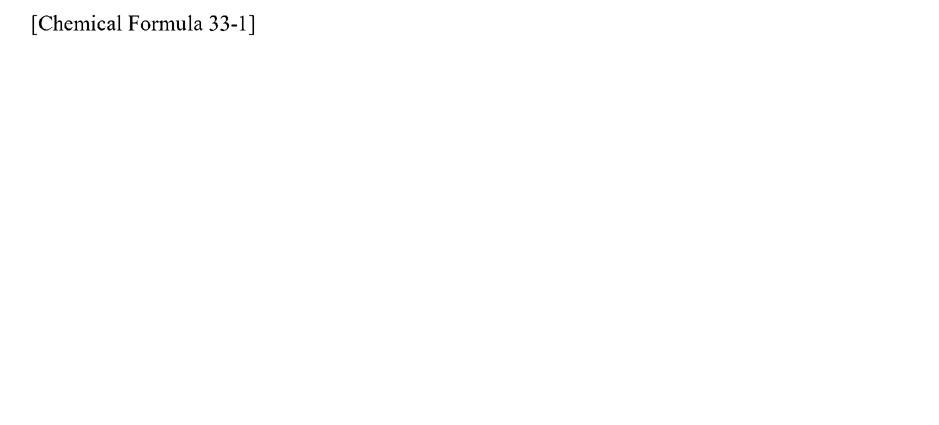 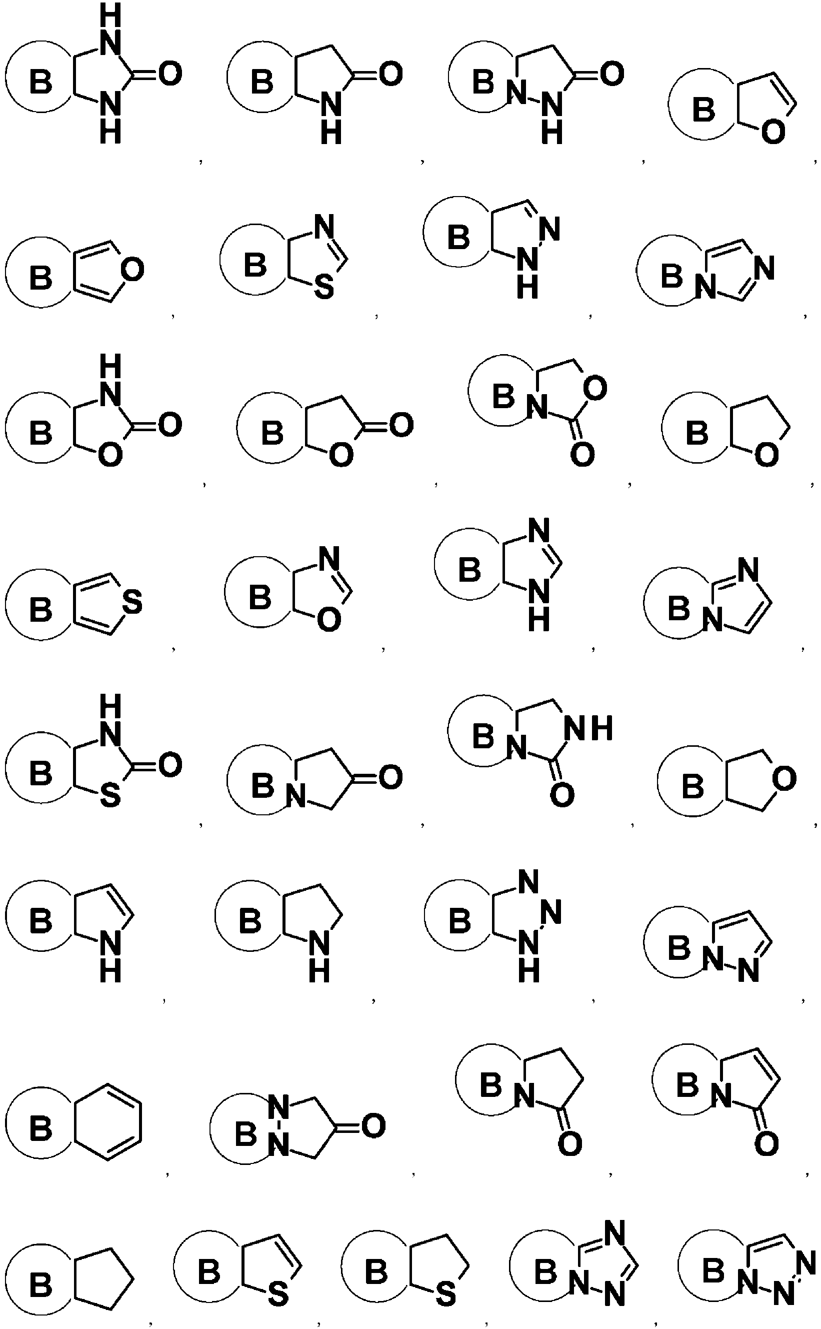 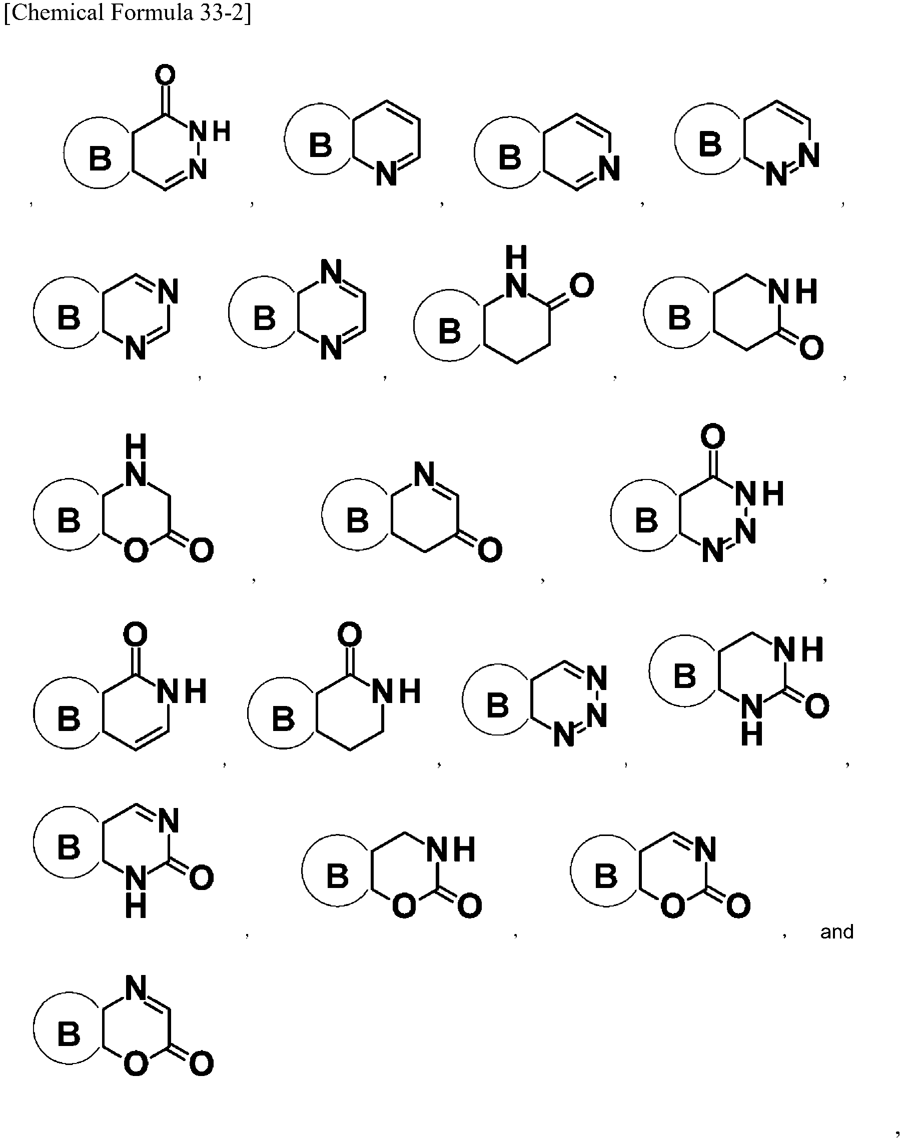
wherein Ring B is a 4- to 6-membered ring that may contain 1 to 3 atoms independently selected from nitrogen, oxygen, and sulfur atoms, and is saturated, partially unsaturated, or aromatic, and the bicyclic group may be substituted.

The bicyclic group can have one bond connecting to Y² at any substitutable position on the B ring or the other ring, and may have a bond on either a carbon atom or a nitrogen atom.

Further, in the above aspect (27), as a preferred aspect (28), the compound or a pharmacologically acceptable salt thereof is provided wherein, in the above Formula (II),
a dotted line represents a single bond,
two X² groups are CH₂,
R^{2b} is a hydrogen atom, a fluorine atom, or a methyl group,
R^{3b} is a hydrogen atom,
Z is CH₂,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R‴ are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is a bicyclic group selected from a group consisting of the following: 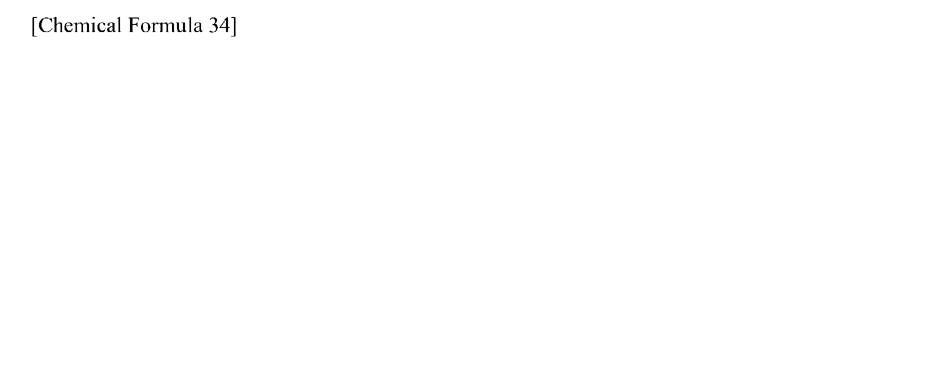 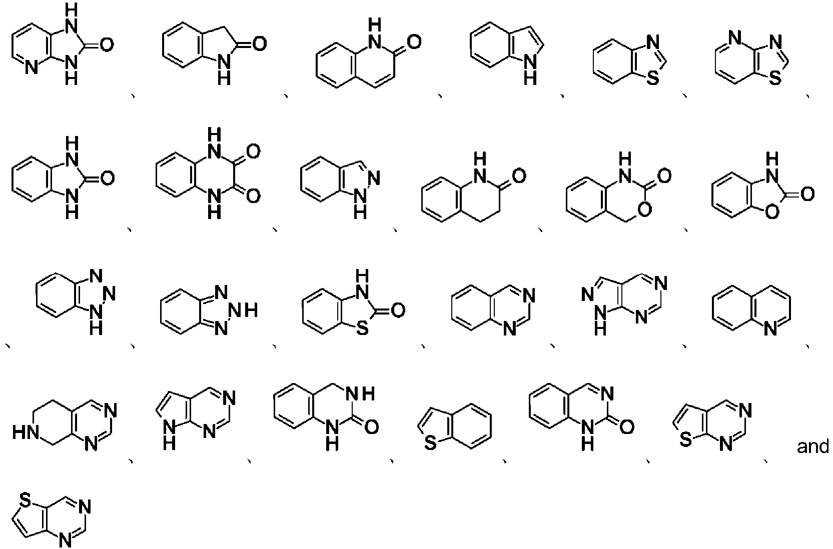
(wherein the bicyclic group may be substituted).

Further, in the above aspect (28), as a preferred aspect (29), the compound or a pharmacologically acceptable salt thereof is provided wherein, in the above Formula (II),
Y² is a single bond, and
R^{2b} is a hydrogen atom.

Further, in any one of the above aspects (27) to (29), as an aspect (30), the compound or a pharmacologically acceptable salt thereof is provided wherein, in the above Formula (II), R^{b} is an optionally substituted nitrogen-containing heterocycle, and R^{b} has a bond to Y² on a nitrogen atom that is a member of the ring.

Further, in the above aspect (1), as an aspect (31), the compound or a pharmacologically acceptable salt thereof is provided wherein, the compound is a compound represented by the above Formula (III), and in the above Formula (III),
R^{c} is an optionally substituted 6- to 10-membered aryl group.

Further, in the above aspect (31), as a preferred aspect (32), the compound or a pharmacologically acceptable salt thereof is provided wherein, in the above Formula (III),
R^{c} is an optionally substituted phenyl group.

Further, in the above aspect (1), as an aspect (33), the compound or a pharmacologically acceptable salt thereof is provided wherein, the compound is a compound represented by the above Formula (IV), and in the above Formula (IV),
R^{d} is an optionally substituted 6- to 10-membered aryl group.

Further, in the above aspect (33), as a preferred aspect (34), the compound or a pharmacologically acceptable salt thereof is provided wherein, in the above Formula (IV),
R^{d} is an optionally substituted phenyl group.

Further, as an aspect (34-2), the compound or a pharmacologically acceptable salt thereof is provided wherein, the compound is selected from a group consisting of the following: 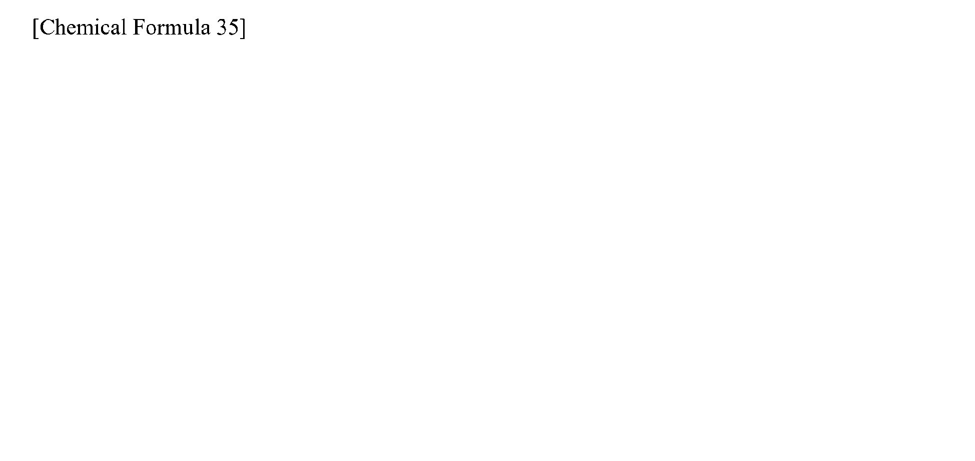 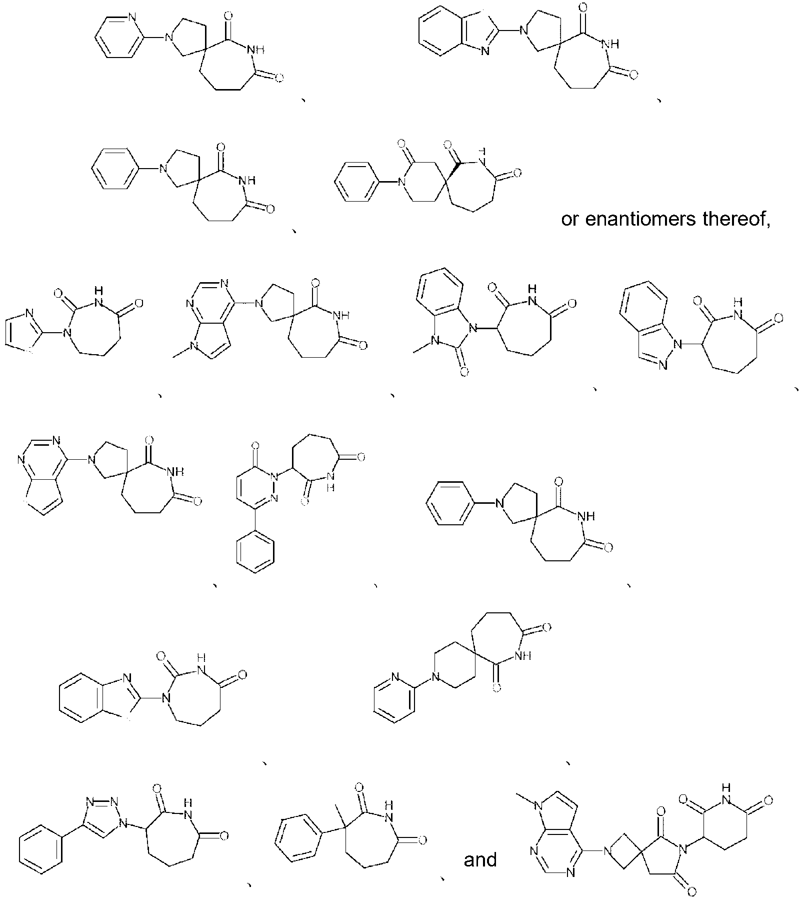 (wherein an aryl group or a heteroaryl group may be substituted).

Further, as an aspect (34-3), the compound or a pharmacologically acceptable salt thereof is provided wherein, the compound is selected from a group consisting of the following:

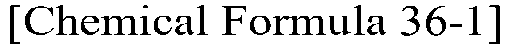

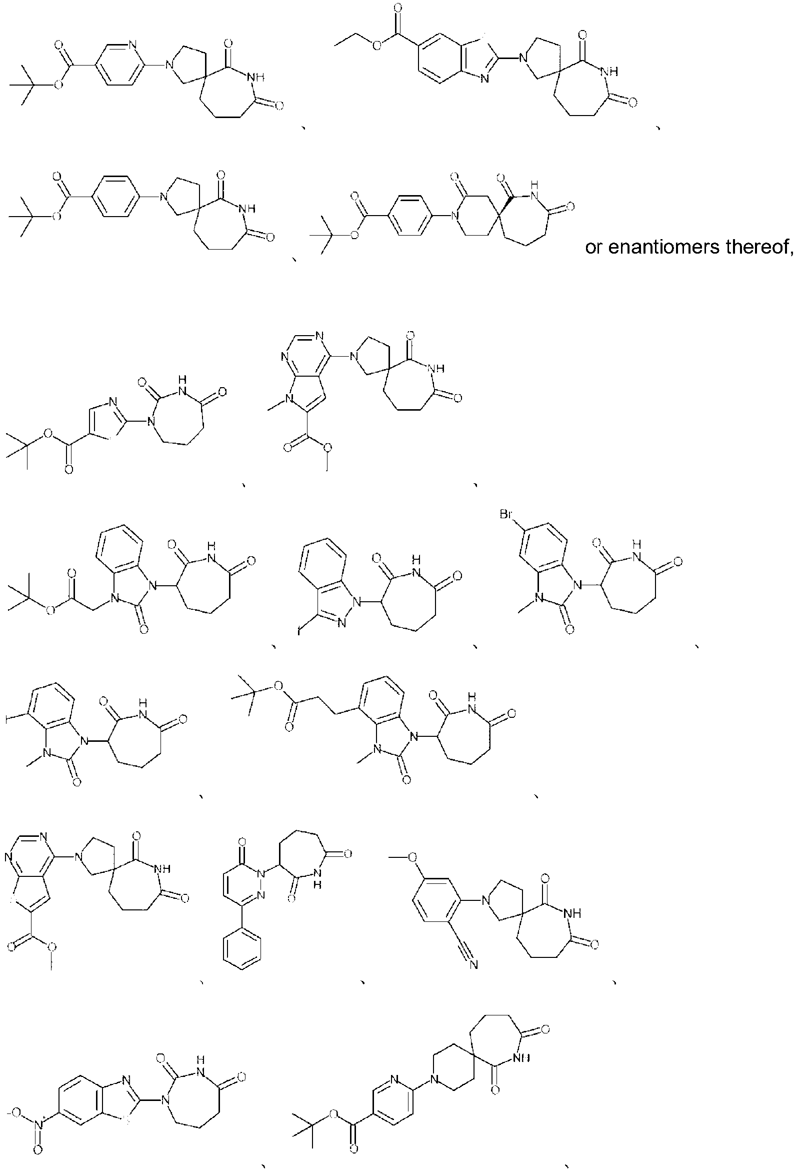

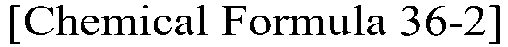

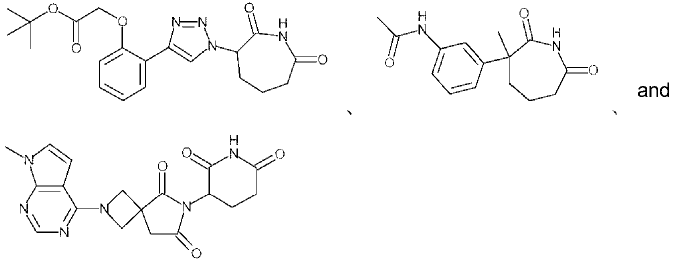

Further, in any one of the above aspects (1) to (34), as an aspect (35), the compound or a pharmacologically acceptable salt thereof is provided wherein,
when n is 0 in Formula (I), each optionally substituted group represented by Y¹ is substituted with L having a first terminal group or with substituted L,
when n is 1 in Formula (I), each optionally substituted group represented by R^{a} is substituted with L having a first terminal group or with substituted L,
in Formula (II), each optionally substituted group represented by R^{b} is substituted with L having a first terminal group or with substituted L,
in Formula (III), each optionally substituted group represented by R^{c} is substituted with L having a first terminal group or with substituted L,
in Formula (IV), each optionally substituted group represented by R^{d} is substituted with L having a first terminal group or with substituted L, and
L is a bond or a chemical linker.

L represents a bond or a chemical linker. However, in the case of L having a first terminal group, L is a chemical linker. In the case of substituted L, L is a bond or a chemical linker. L having a first terminal group or substituted L, as a "substituent," can be present in one or more (for example, 1 to 3) instances at any substitutable position of each optionally substituted group represented by Y¹, R^{a}, R^{b}, R^{c}, or R^{d}.

Further, as an aspect (35-2), the compound or a pharmacologically acceptable salt thereof is provided wherein, the compound according to the aspect (34-2) is further substituted with L having a first terminal group or with substituted L, and L is a bond or a chemical linker. L represents a bond or a chemical linker. However, in the case of L having a first terminal group, L is a chemical linker. In the case of substituted L, L is a bond or a chemical linker. L having a first terminal group or substituted L, as a "substituent," can be present in one or more (for example, 1 to 3) instances at any substitutable position of each aryl group or heteroaryl group in each formula of the compound described in the above aspect (34-2). Substitutable positions include those on ring atoms of aryl groups or heteroaryl groups, as well as on methylene carbon atoms on ring nitrogen atoms of the heteroaryl groups.

Further, as an aspect (35-3), the compound or a pharmacologically acceptable salt thereof is provided wherein, the compound according to the aspect (34-3) is further substituted with L having a first terminal group or with substituted L, and L is a bond or a chemical linker. L represents a bond or a chemical linker. However, in the case of L having a first terminal group, L is a chemical linker. In the case of substituted L, L is a bond or a chemical linker. L having a first terminal group or substituted L, as a "substituent," can be present in one or more (for example, 1 to 3) instances at any substitutable position of each substituted or unsubstituted aryl group or substituted heteroaryl group in each formula of the compound described in the above aspect (34-3). Substitutable positions include those on ring atoms of substituted or unsubstituted aryl groups or substituted heteroaryl groups (including positions currently bearing substituents), as well as any positions on substituents of substituted aryl groups or substituted heteroaryl groups.

Further, in the above aspect (35), (35-2) or (35-3), as an aspect (36), the compound or a pharmacologically acceptable salt thereof is provided wherein,
L is a group represented by -L₁-L₂-L₃-
[wherein
L₁ and L₃ each independently represent
   (1) a bond,
   (2) CR^{L1}R^{L1}',
   (3) O,
   (4) S,
   (5) SO,
   (6) SO₂,
   (7) NR^{L1},
   (8) SO₂NR^{L1},
   (9) NR^{L1}SO₂,
   (10) SONR^{L1},
   (11) NR^{L1}SO,
   (12) CONR^{L1},
   (13) NR^{L1}CO,
   (14) NR^{L1}CONR^{L1}',
   (15) NR^{L1}SO₂NR^{L1}', or
   (16) CO,
in the above formulas, R^{L1} and R^{L1}' each independently represent
   1) a hydrogen atom,
   2) a halogen atom,
   3) -CN,
   4) -NO₂,
   5) -SF₅,
   6) -COOH,
   7) -N(R^{L2}R^{L2}'),
   8) -A^{L2}R^{L2},
   9) an optionally substituted alkyl group,
   10) an optionally substituted cycloalkyl group,
   11) an optionally substituted heterocyclic saturated group,
   12) an optionally substituted aryl group,
   13) an optionally substituted heteroaryl group,
   14) -SO₂R^{L2},
   15) -P(O)(OR^{L2})OR^{L2}',
   16) -C≡CR^{L2},
   17) -C(R^{L2})=C(R^{L2}'R^{L2}"),
   18) -COR^{L2},
   19) -CON(R^{L2}R^{L2}'),
   20) -SO₂N (R^{L2}R^{L2}'),
   21) -N(R^{L2})CON(R^{L2}'R^{L2}"), and
   22) -N(R^{L2})SO₂N(R^{L2}'R^{L2}"),
A^{L2} represents an oxygen atom or a sulfur atom,
R^{L2}, R^{L2}', and R^{L2}" each independently represent
   a) a hydrogen atom,
   b) an optionally substituted C₁₋₈ alkyl group, or
   c) an optionally substituted C₃₋₈ cycloalkyl group,
L₂ represents

   (CH₂)ₚ₁ₐ-O-(CH₂-CH₂-O)ₚ₂ₐ-(CH₂)ₚ₃ₐ,
wherein
p1a and p3a represent an integer from 0 to 10 when an atom directly bonded to adjacent L₁ or L₃ is a carbon atom or a bond, and an integer from 2 to 10 when the atom is other than a carbon atom or a bond, and
p2a represents an integer from 0 to 10, and
some -CH₂-CH₂- groups in (CH₂)ₚ₁ₐ or (CH₂)ₚ₃ₐ of L₂ may be replaced with -CH=CH- or -C≡ C-].

In --L₁-L₂-L₃-, a bond on a side of L₁ and L₃ that is not connected to L₂ is a point of attachment to an optionally substituted group represented by Y¹, R^{a}, R^{b}, R^{c}, or R^{d}, and to a first terminal group or a substituent of substituted L. Each of a bond on the L₁ side and a bond on the L3 side can be a point of attachment to either an optionally substituted group represented by Y¹, R^{a}, R^{b}, R^{c}, or R^{d}, or to a first terminal group or a substituent of substituted L.

Further, in the above aspect (35), (35-2) or (35-3), as another aspect (37), the compound or a pharmacologically acceptable salt thereof is provided wherein,
L is a group represented by -L_{b1}-L_{b2}-L_{b3}-
[wherein
L_{b1} and L_{b3} each independently represent
   (1) a bond,
   (2) CR^{L1}R^{L1}',
   (3) O,
   (4) S,
   (5) SO,
   (6) SO₂,
   (7) NR^{L1},
   (8) SO₂NR^{L1},
   (9) NR^{L1}SO₂,
   (10) SONR^{L1},
   (11) NR^{L1}SO,
   (12) CONR^{L1},
   (13) NR^{L1}CO,
   (14) NR^{L1}CONR^{L1}',
   (15) NR^{L1}SO₂NR^{L1}', or
   (16) CO,
in the above formulas, R^{L1} and R^{L1}' each independently represent
   1) a hydrogen atom,
   2) a halogen atom,
   3) -CN,
   4) -NO₂,
   5) -SF₅,
   6) -COOH,
   7) -N(R^{L2}R^{L2}'),
   8) -A^{L2}R^{L2},
   9) an optionally substituted alkyl group,
   10) an optionally substituted cycloalkyl group,
   11) an optionally substituted heterocyclic saturated group,
   12) an optionally substituted aryl group,
   13) an optionally substituted heteroaryl group,
   14) -SO₂R^{L2},
   15) -P(O)(OR^{L2})OR^{L2}',
   16) -C≡CR^{L2},
   17) -C(R^{L2})=C(R^{L2}'R^{L2}"),
   18) -COR^{L2},
   19) -CON(R^{L2}R^{L2}'),
   20) -SO₂N (R^{L2}R^{L2}'),
   21) -N(R^{L2})CON(R^{L2}'R^{L2}"), and
   22) -N(R^{L2})SO₂N(R^{L2}'R^{L2}"),
A^{L2} represents an oxygen atom or a sulfur atom,
R^{L2}, R^{L2}', and R^{L2}" each independently represent
   a) a hydrogen atom,
   b) an optionally substituted C₁₋₈ alkyl group, or
   c) an optionally substituted C₃₋₈ cycloalkyl group,
L_{b2} represents
   (1) a bond,
   (2) (CH₂)₁₋₁₀,
   (3) (CH₂)₀₋₆-O-(CH₂)₀₋₆,
   (4) (CH₂)₀₋₆-CONH-(CH₂)₀₋₆,
   (5) (CH₂)₀₋₆-NHCO-(CH₂)₀₋₆,
   (6) (CH₂)₀₋₆-NH-(CH₂)₀₋₆,
   (7) (CH₂)₀₋₆-NHSO₂-(CH₂)₀₋₆, or
   (8) (CH₂)₀₋₆-SO₂NH-(CH₂)₀₋₆,
wherein some -CH₂-CH₂- groups in (CH₂)₁₋₁₀ or (CH₂)₀₋₆ in L_{b2} may be replaced with -CH=CH- or *-*C≡C*-,*
provided that a case where L_{b1}, L_{b2}, and L_{b3} are all bonds is excluded].

In --L_{b1}-L_{b2}-L_{b3}-, a bond on a side of L_{b1} and L_{b3} that is not connected to L_{b2} is a point of attachment to an optionally substituted group represented by Y¹, R^{a}, R^{b}, R^{c}, or R^{d}, and to a first terminal group or a substituent of substituted L. Each of a bond on the L_{b1} side and a bond on the L_{b3} side can be a point of attachment to either an optionally substituted group represented by Y¹, R^{a}, R^{b}, R^{c}, or R^{d}, or to a first terminal group or a substituent of substituted L.

Further, in the above aspect (35), (35-2) or (35-3), as another aspect (38), the compound or a pharmacologically acceptable salt thereof is provided wherein,
L is a group represented by the following Formula (V): 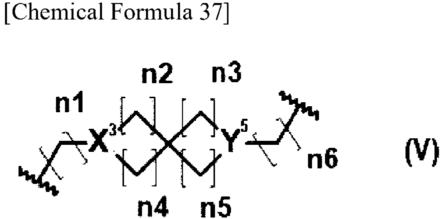
[wherein
n1 and n6 are each independently 0, 1, 2, 3, 4, 5, 6, 7, 8, 9, or 10,
n2, n3, n4, and n5 are each independently 1, 2, 3, or 4, and
X³ and Y⁵ are each independently CH or N].

In Formula (V), each wavy line represents a point of attachment to an optionally substituted group represented by Y¹, R^{a}, R^{b}, R^{c}, or R^{d}, and to a first terminal group or a substituent of substituted L. Each of a wavy line on the X³ side and a wavy line on the Y⁵ side can be a point of attachment to either an optionally substituted group represented by Y¹, R^{a}, R^{b}, R^{c}, or R^{d}, or to a first terminal group or a substituent of substituted L.

Further, in the above aspect (35), (35-2) or (35-3), as another aspect (39), the compound or a pharmacologically acceptable salt thereof is provided wherein,
L is a group selected from a group consisting of:

   -N(R^{L3})-(CH₂)ₘ₁-O(CH₂)ₘ₂-O(CH₂)ₘ₃-O(CH₂)ₘ₄-O(CH₂)ₘ₅-O(CH₂)ₘ₆-OCH₂-,

   -O-(CH₂)ₘ₁-O(CH₂)ₘ₂-O(CH₂)ₘ₃-O(CH₂)ₘ₄-O(CH₂)ₘ₅-O(CH₂)ₘ₆-OCH₂-,

   -O-(CH₂)ₘ₁-O(CH₂)ₘ₂-O(CH₂)ₘ₃-O(CH₂)ₘ₄-O(CH₂)ₘ₅-O(CH₂)ₘ₆-O-,

   -N(R)-(CH₂)ₘ₁-O(CH₂)ₘ₂-O(CH₂)ₘ₃-O(CH₂)ₘ₄-O(CH₂)ₘ₅-O(CH₂)ₘ₆-O-,

   -(CH₂)ₘ₁-O(CH₂)ₘ₂-O(CH₂)ₘ₃-O(CH₂)ₘ₄-O(CH₂)ₘ₅-O(CH₂)ₘ₆-O-,

   -(CH₂)ₘ₁-O(CH₂)ₘ₂-O(CH₂)ₘ₃-O(CH₂)ₘ₄-O(CH₂)ₘ₅-O(CH₂)ₘ₆-OCH₂-, 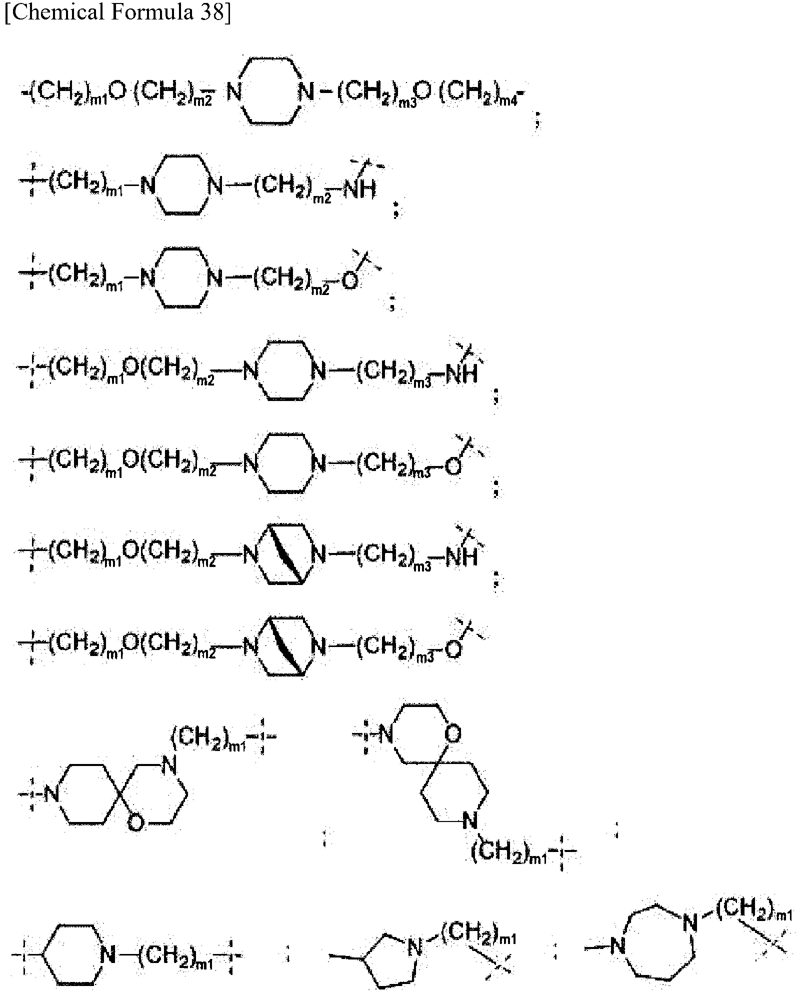 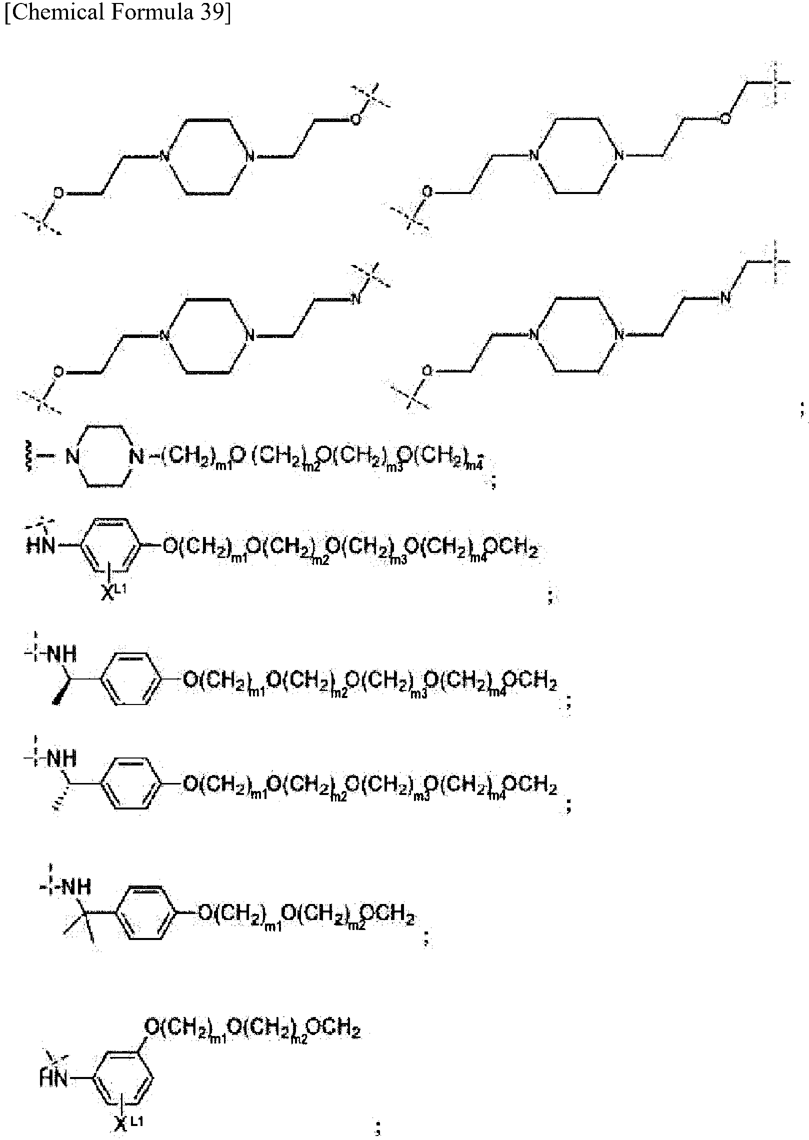 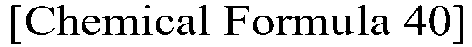 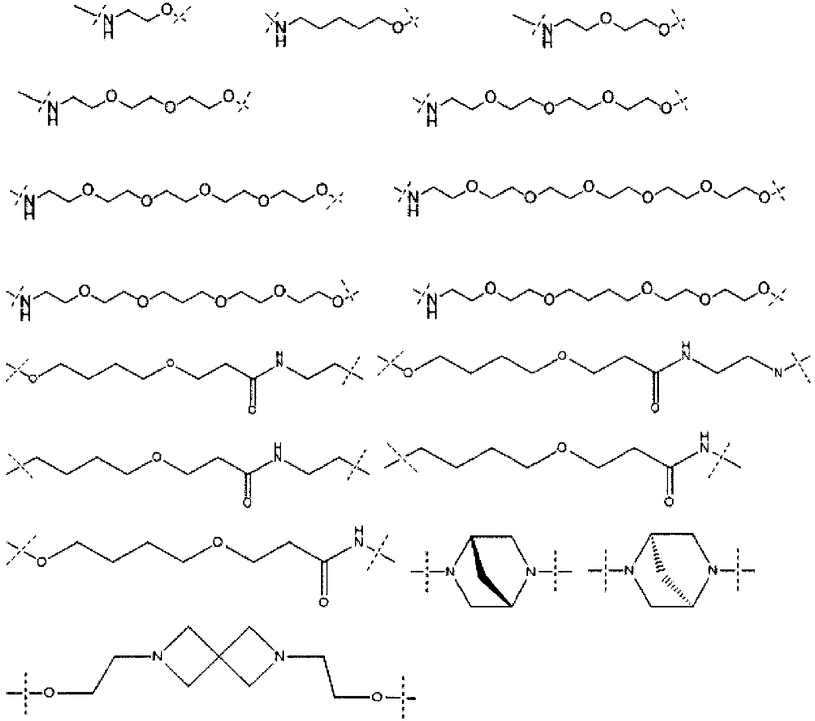 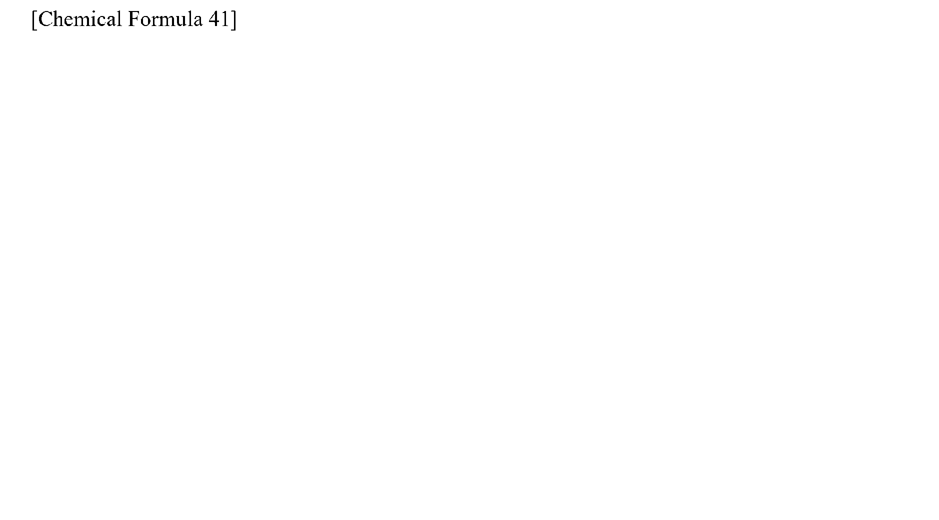 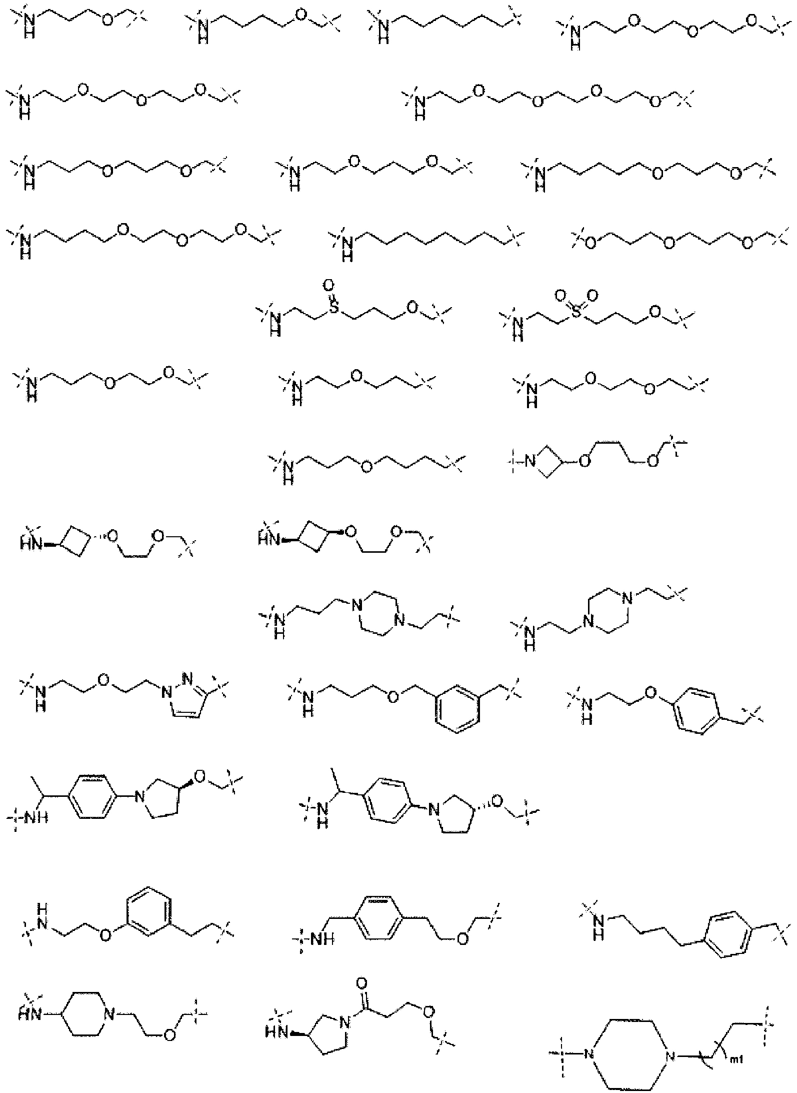 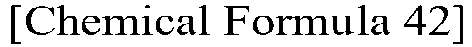 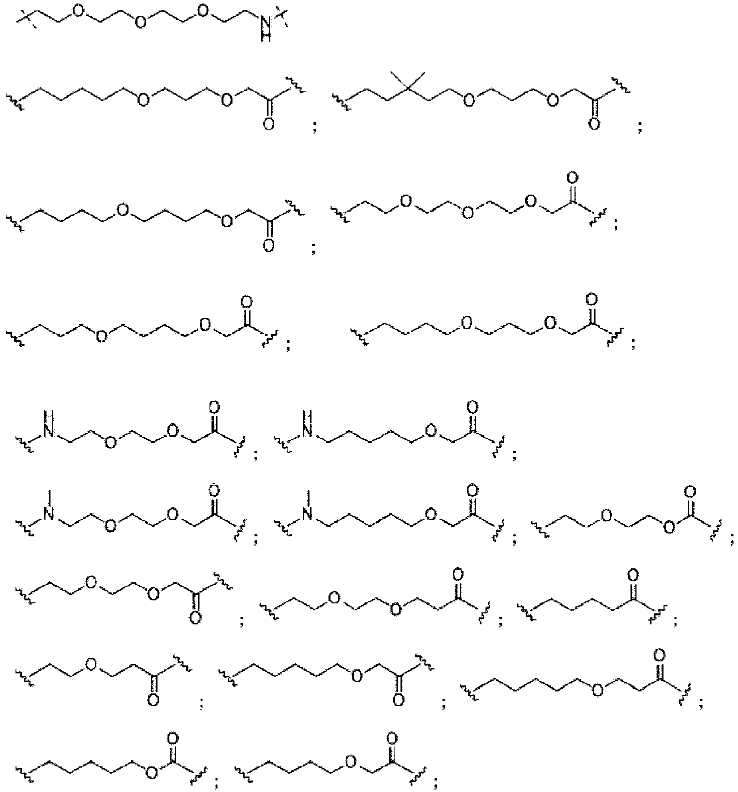 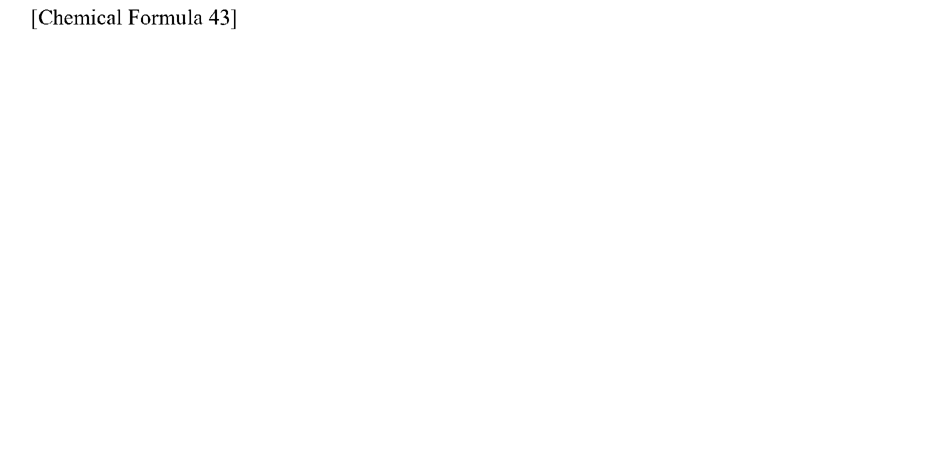 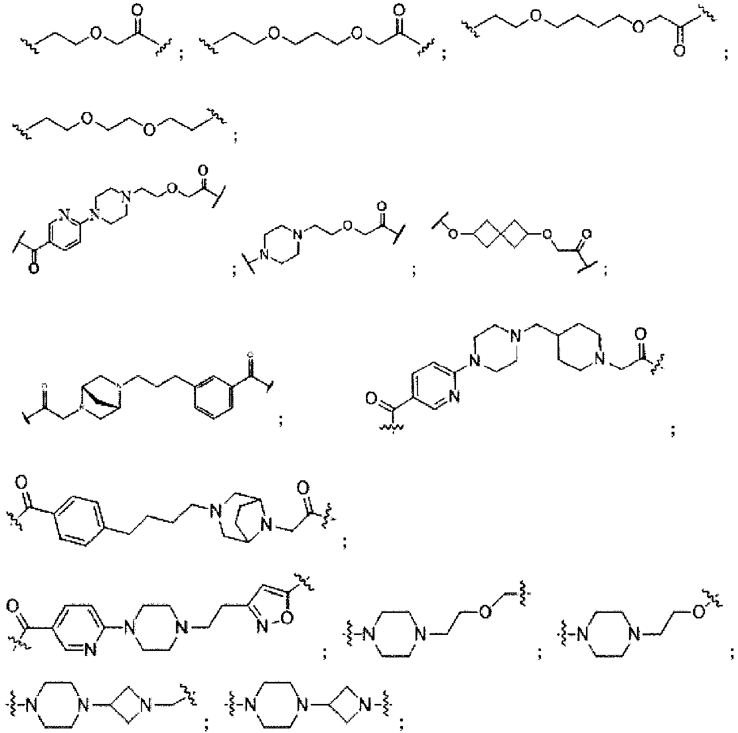 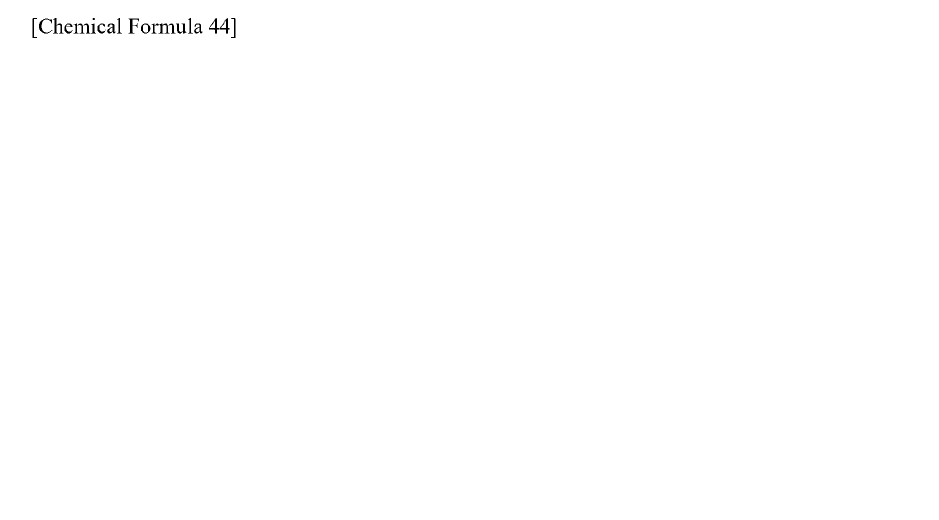 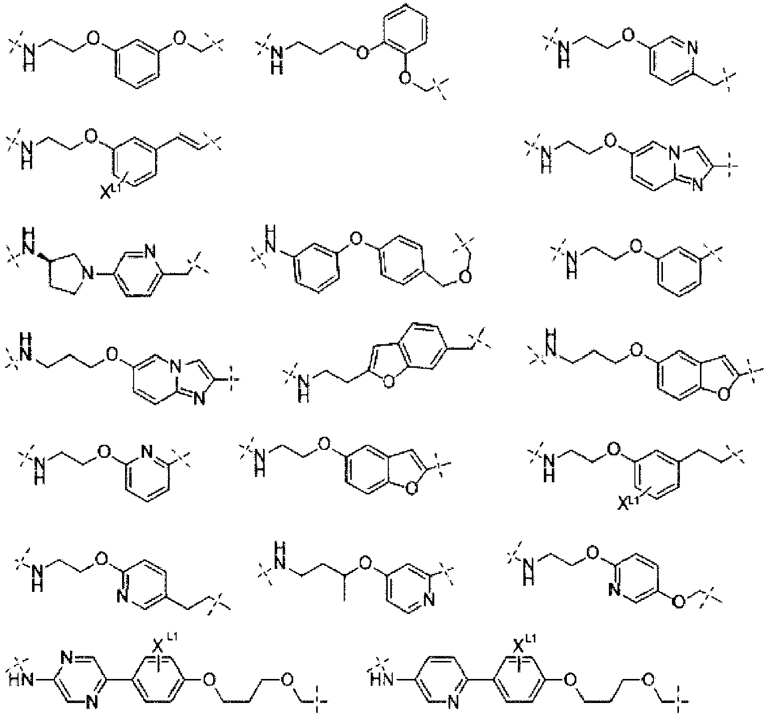 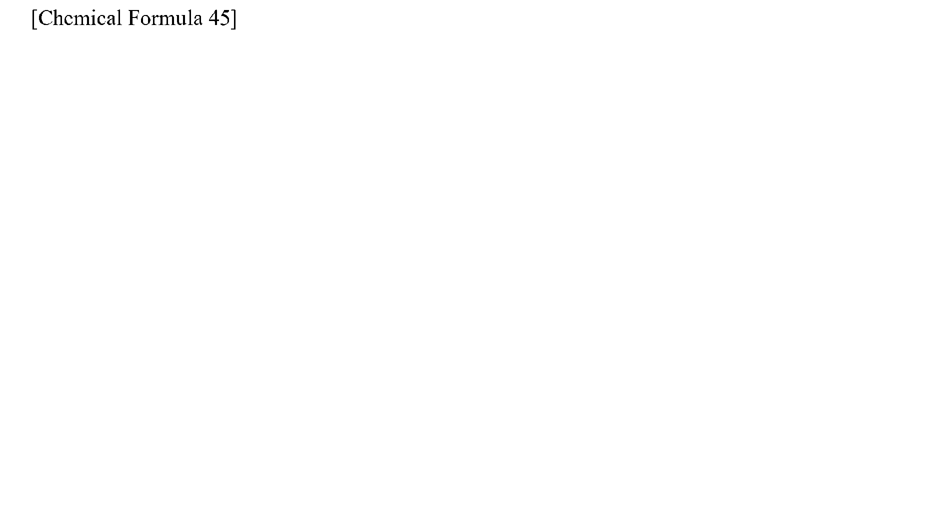 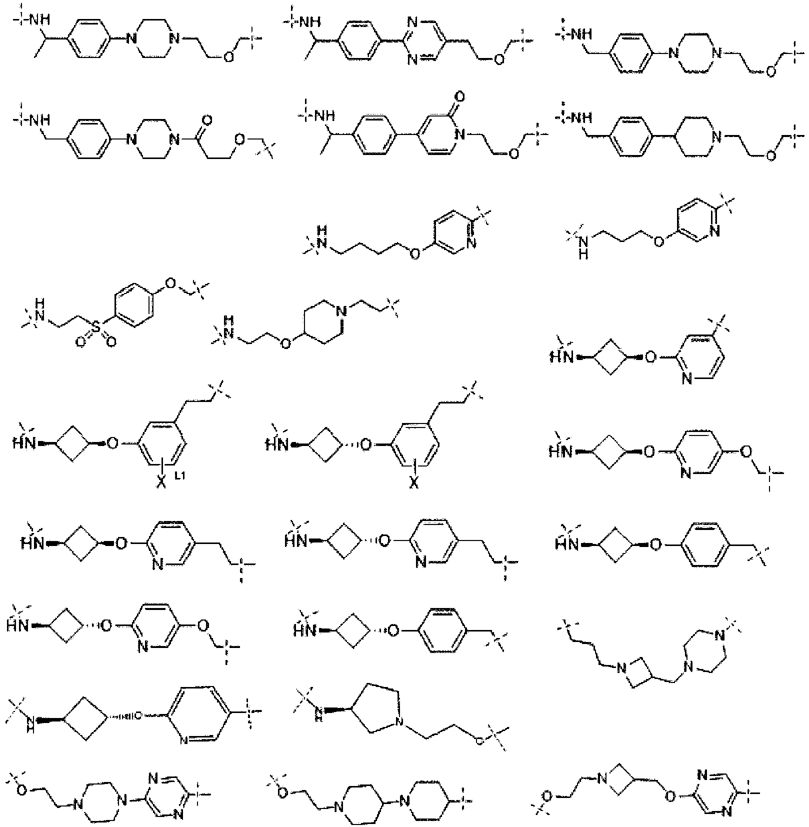 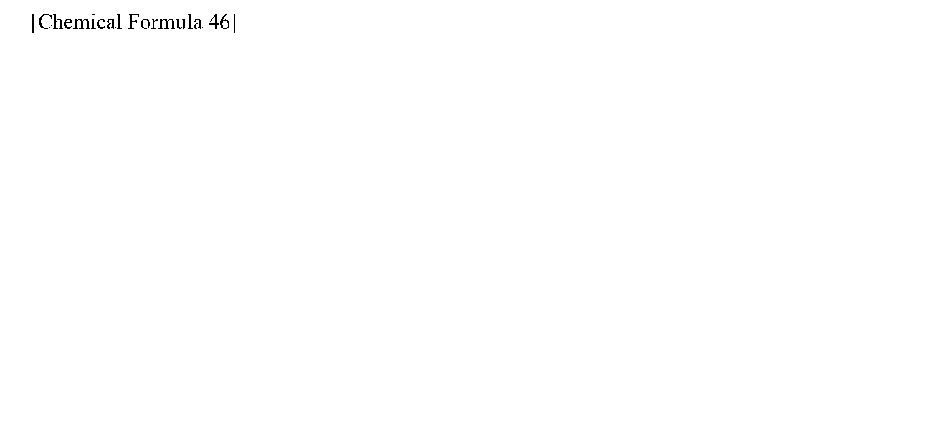 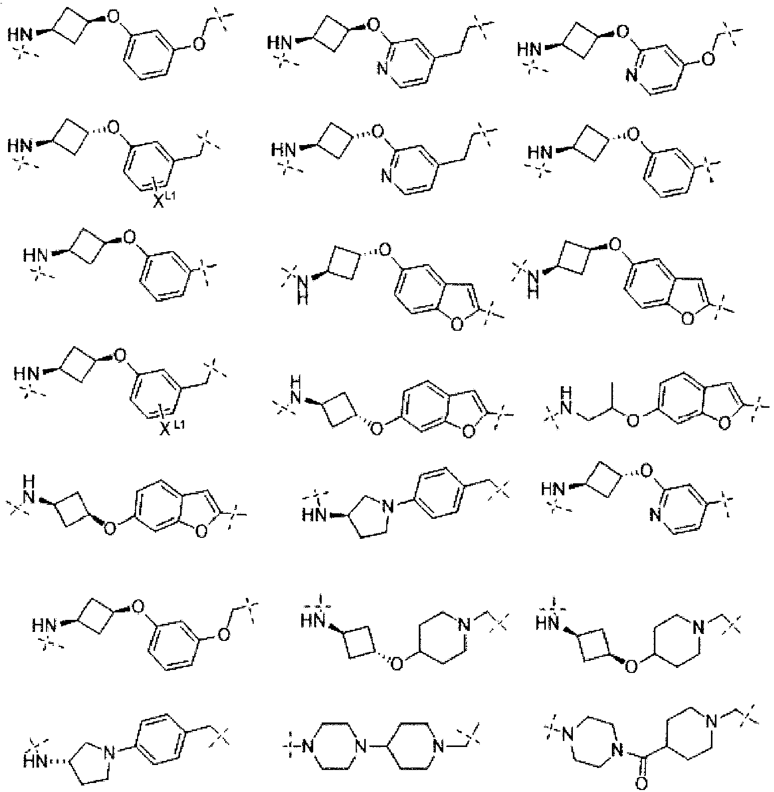 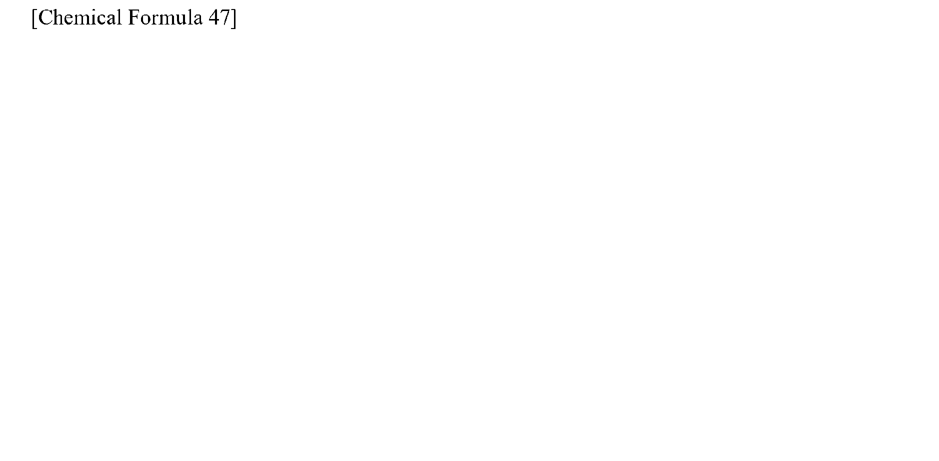 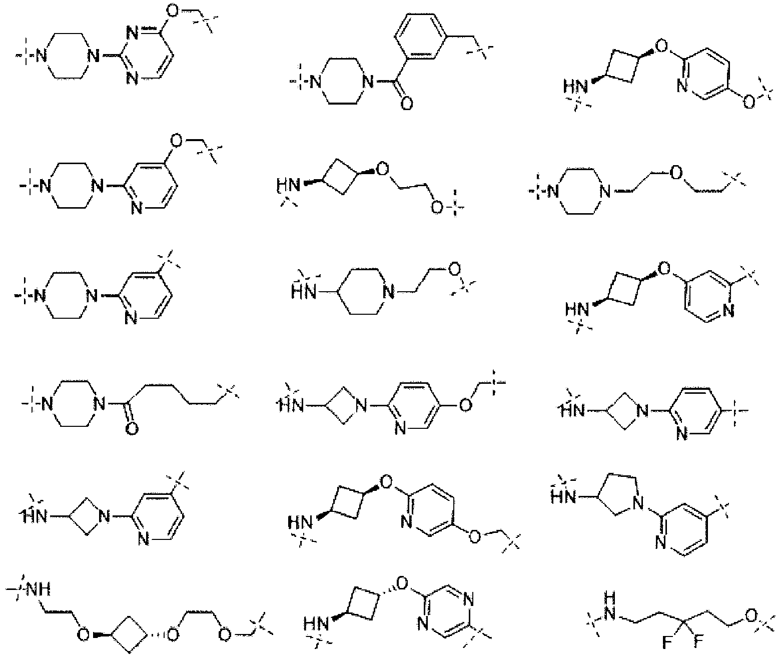
[wherein
m1, m2, m3, m4, m5, and m6 are each independently 0, 1, 2, 3, 4, 5, 6, 7, 8, 9, 10, 11, 12, 13, 14, 15, 16, 17, 18, 19, or 20, provided that when the number is 0, no N-O bond or O-O bond is present,
R^{L3} is H, methyl, or ethyl; and
X^{L1} is H or F].
m1, m2, m3, m4, m5, and m6 are each independently preferably 1, 2, 3, 4, 5, or 6. In the above groups, two termini (may each be indicated by a dashed line or a wavy line) are each a point of attachment to an optionally substituted group represented by Y¹, R^{a}, R^{b}, R^{c}, or R^{d}, and to a first terminal group or a substituent of substituted L. Each terminus can be a point of attachment to either an optionally substituted group represented by Y¹, R^{a}, R^{b}, R^{c}, or R^{d}, or to a first terminal group or a substituent of substituted L.

Further, in the above aspect (35), (35-2) or (35-3), as another aspect (40), the compound or a pharmacologically acceptable salt thereof is provided wherein, L is a group selected from a group consisting of: 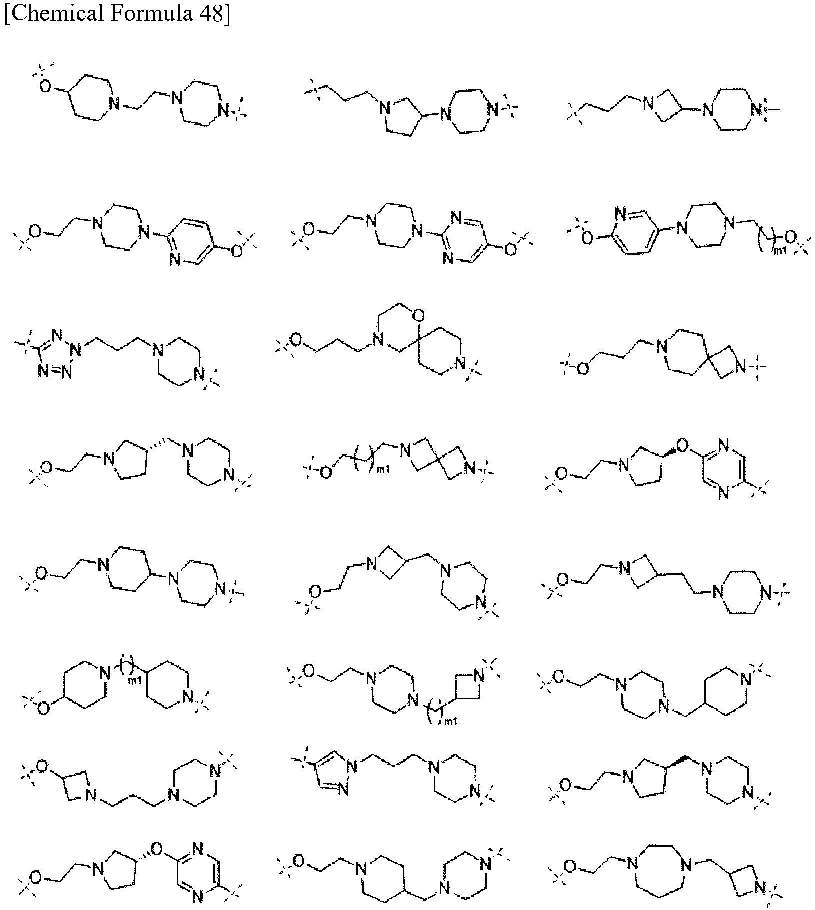 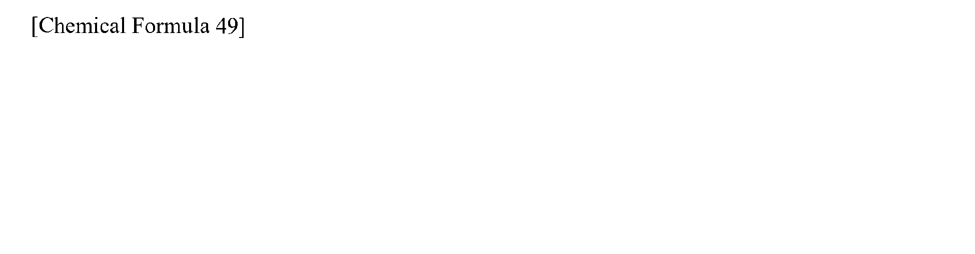 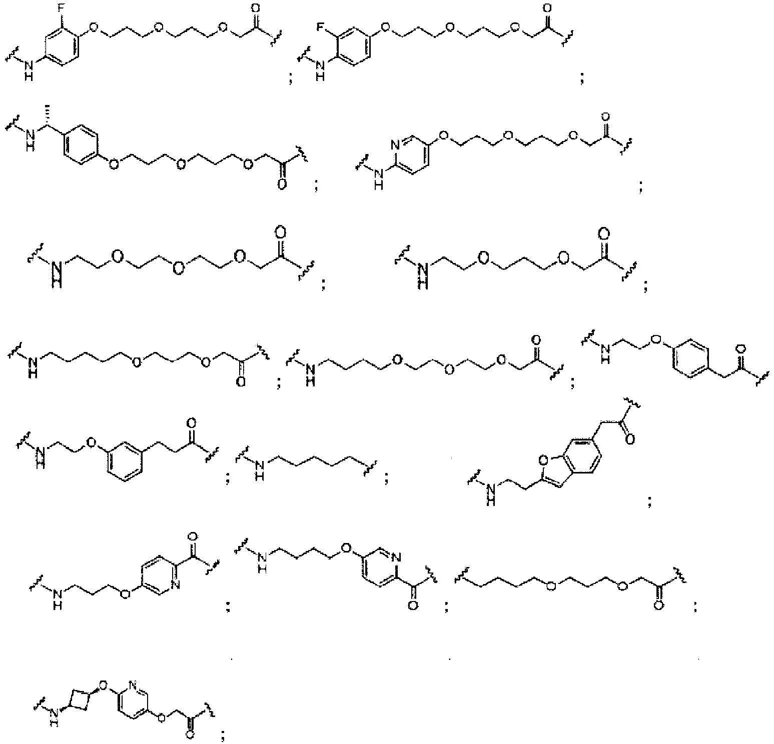 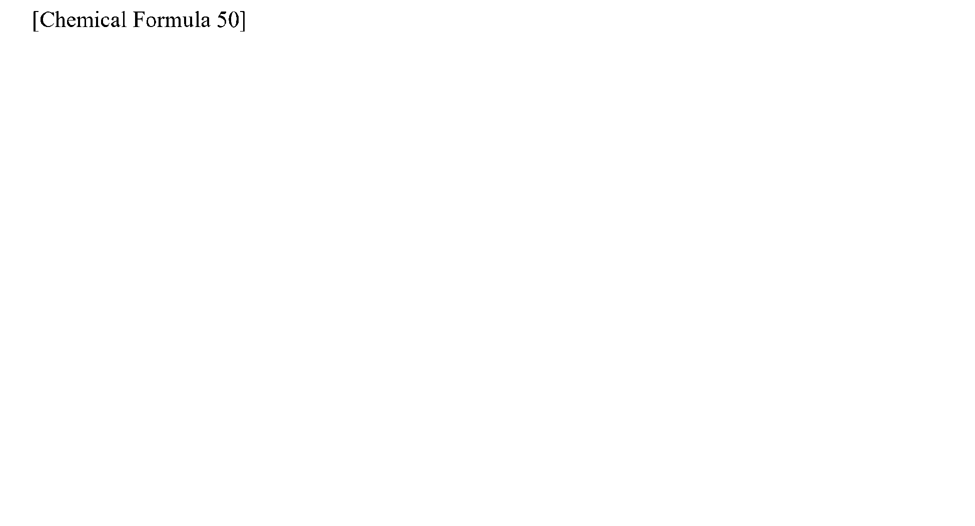 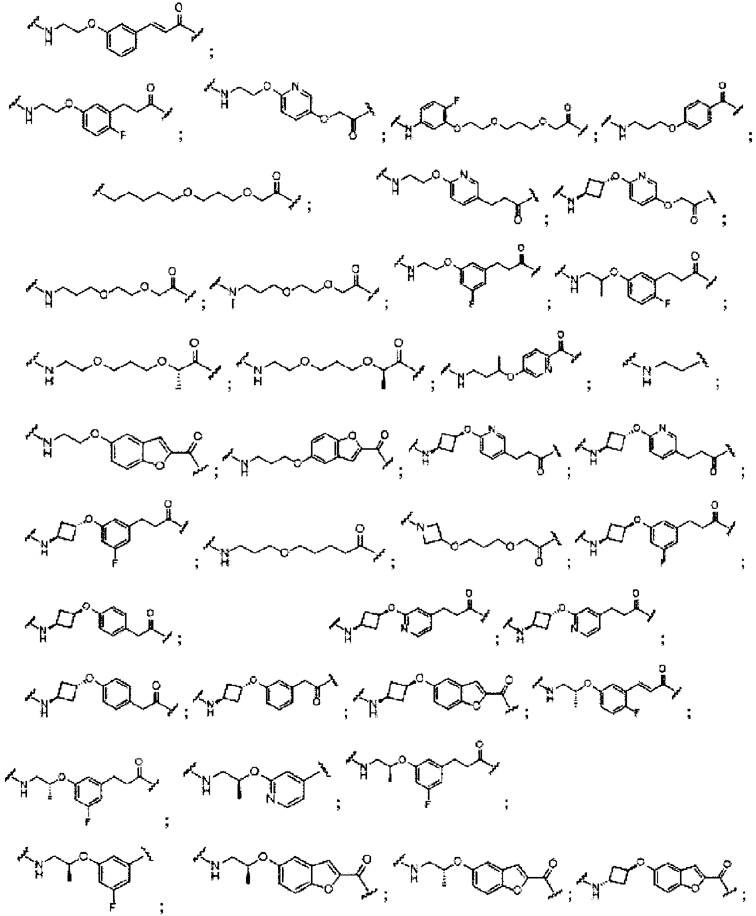 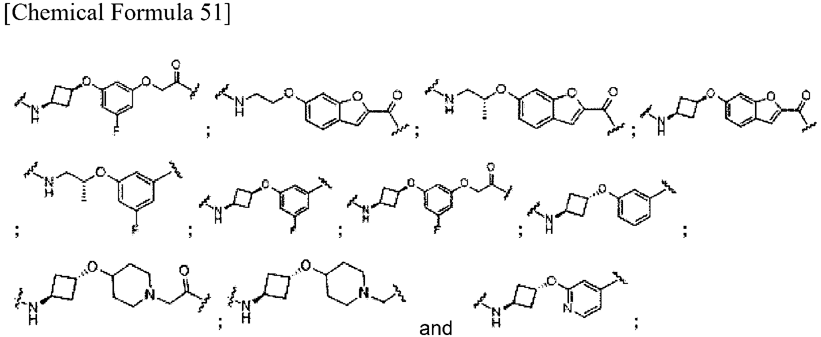 [wherein
Each m1 is independently 0, 1, 2, 3, 4, 5, 6, 7, 8, 9, 10, 11, 12, 13, 14, 15, 16, 17, 18, 19, or 20].

Each m1 is independently preferably 1, 2, 3, 4, 5 or 6. In the above groups, two termini (may each be indicated by a dashed line or a wavy line) are each a point of attachment to an optionally substituted group represented by Y¹, R^{a}, R^{b}, R^{c}, or R^{d}, and to a first terminal group or a substituent of substituted L. Each terminus can be a point of attachment to either an optionally substituted group represented by Y¹, R^{a}, R^{b}, R^{c}, or R^{d}, or to a first terminal group or a substituent of substituted L.

Further, in the above aspect (35), (35-2) or (35-3), as another aspect (41), the compound or a pharmacologically acceptable salt thereof is provided wherein, L is a group selected from a group consisting of: 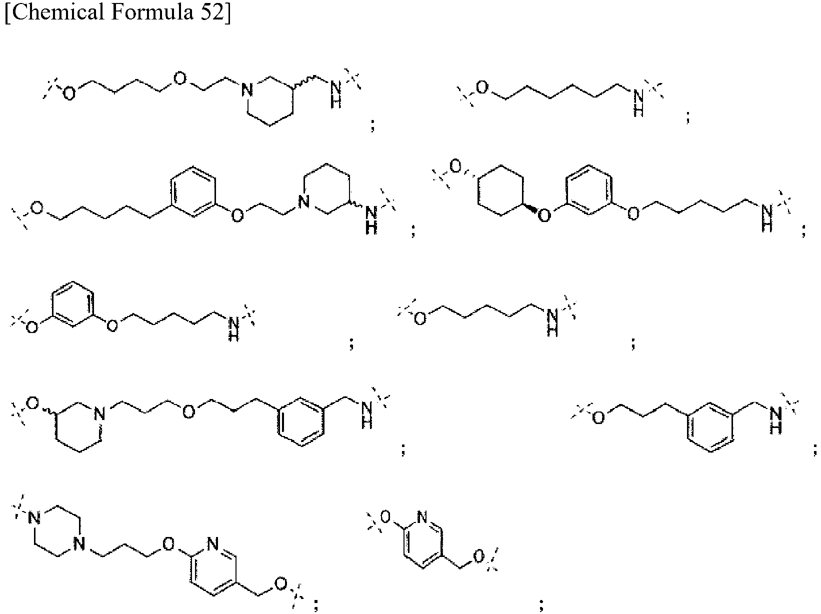 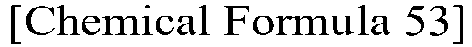 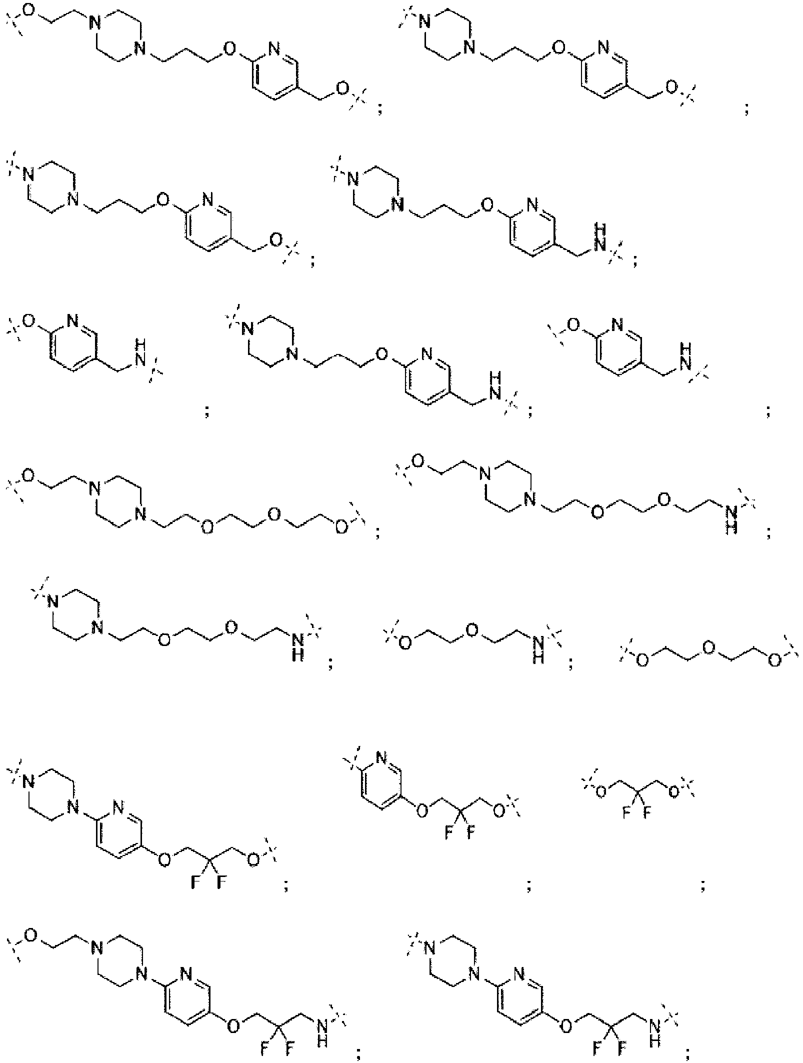 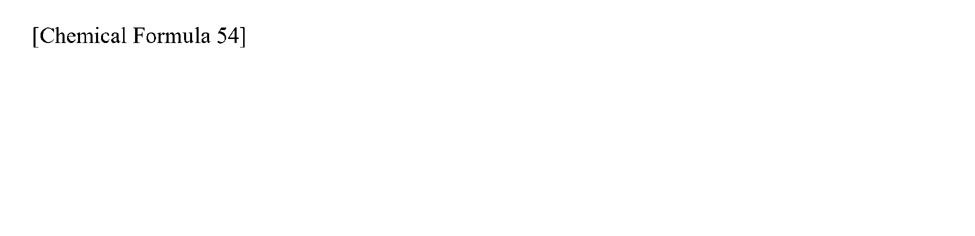 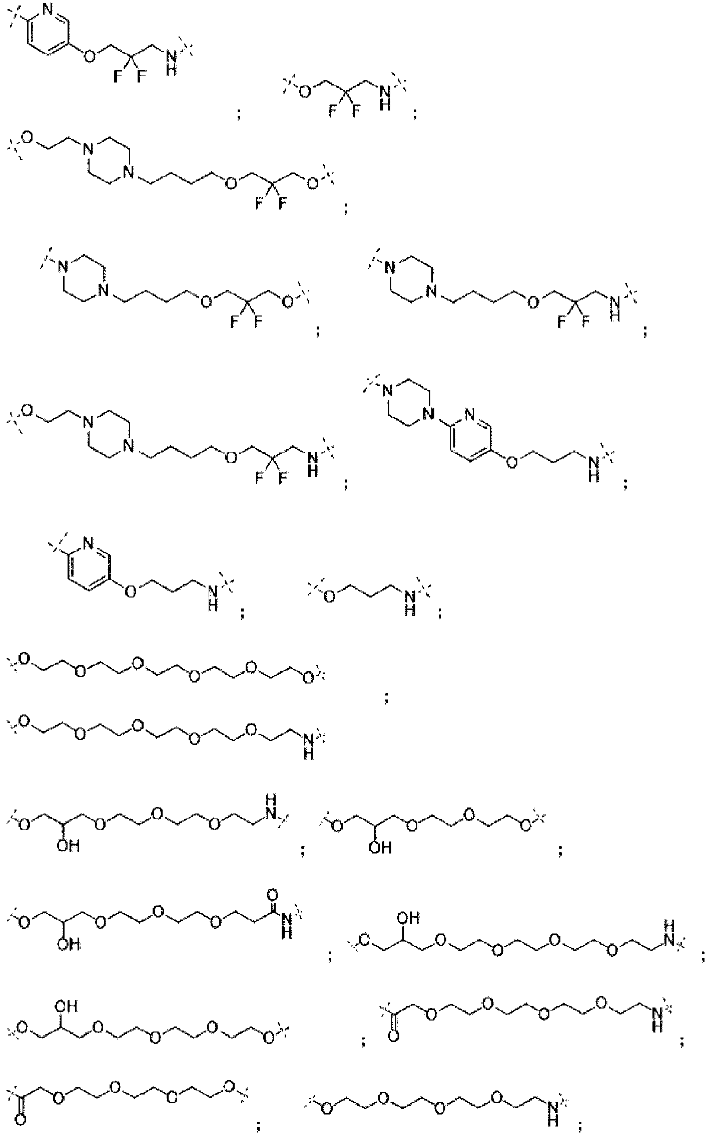 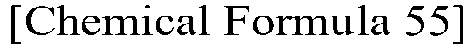 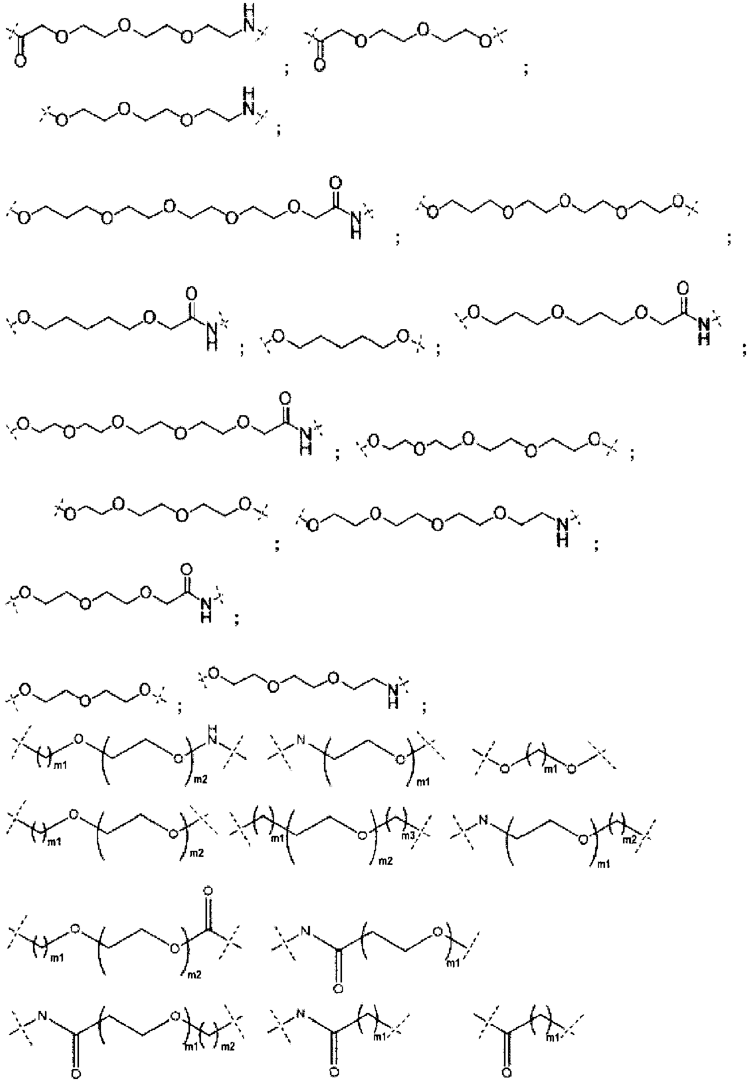 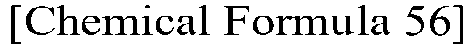 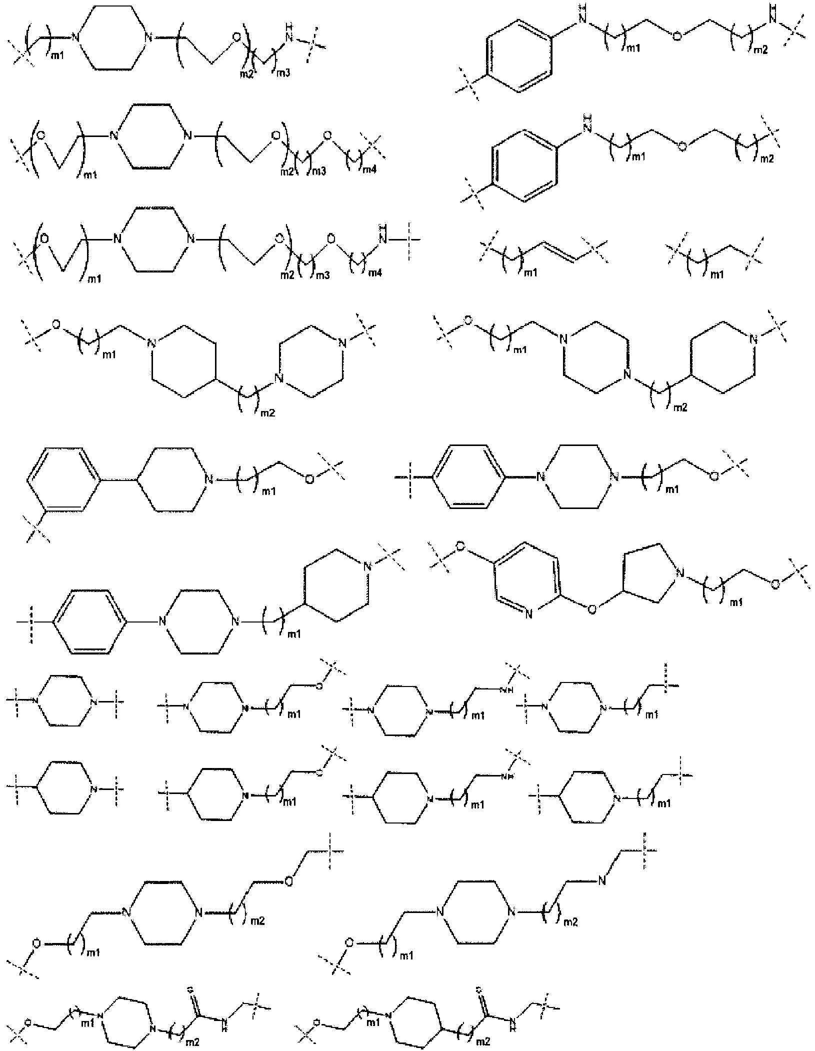 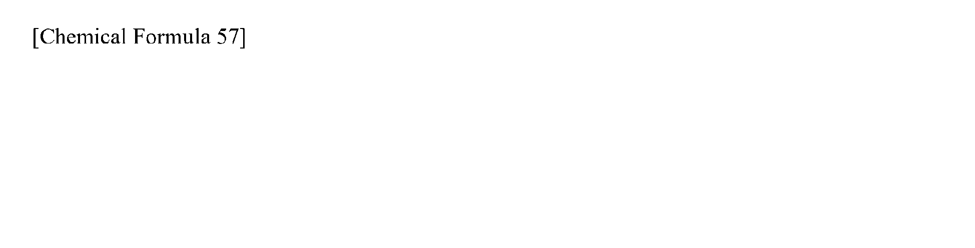 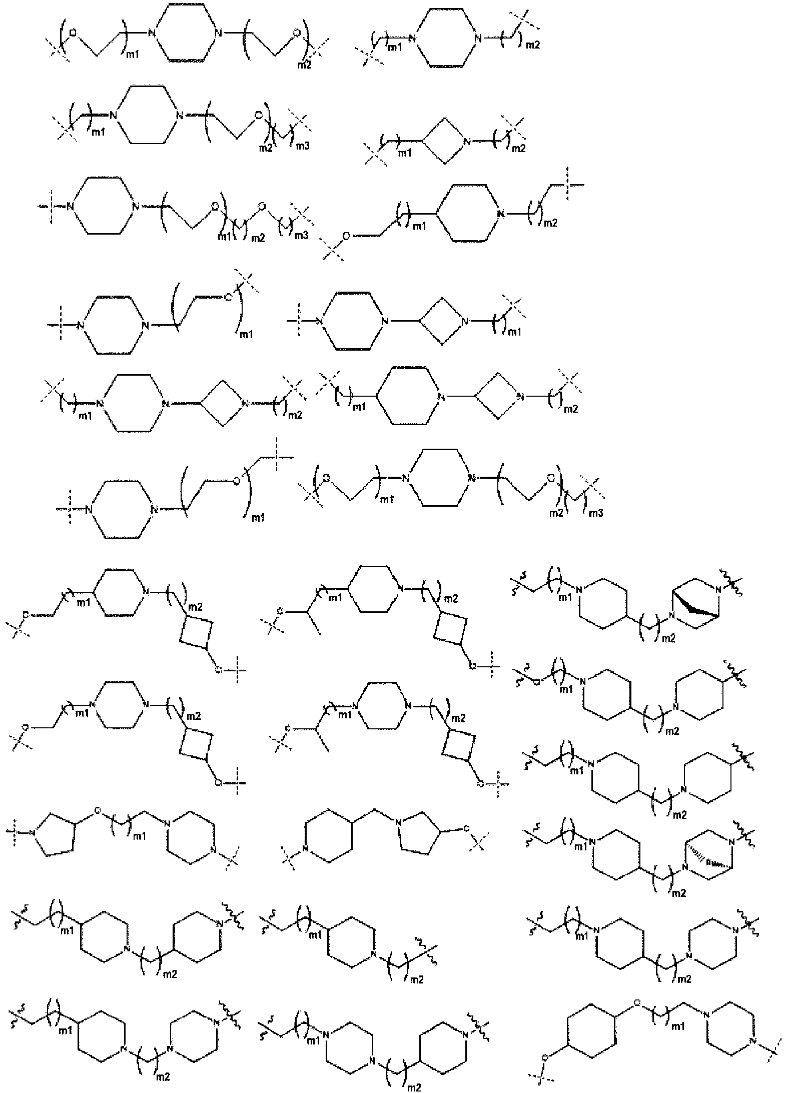 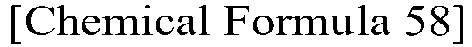 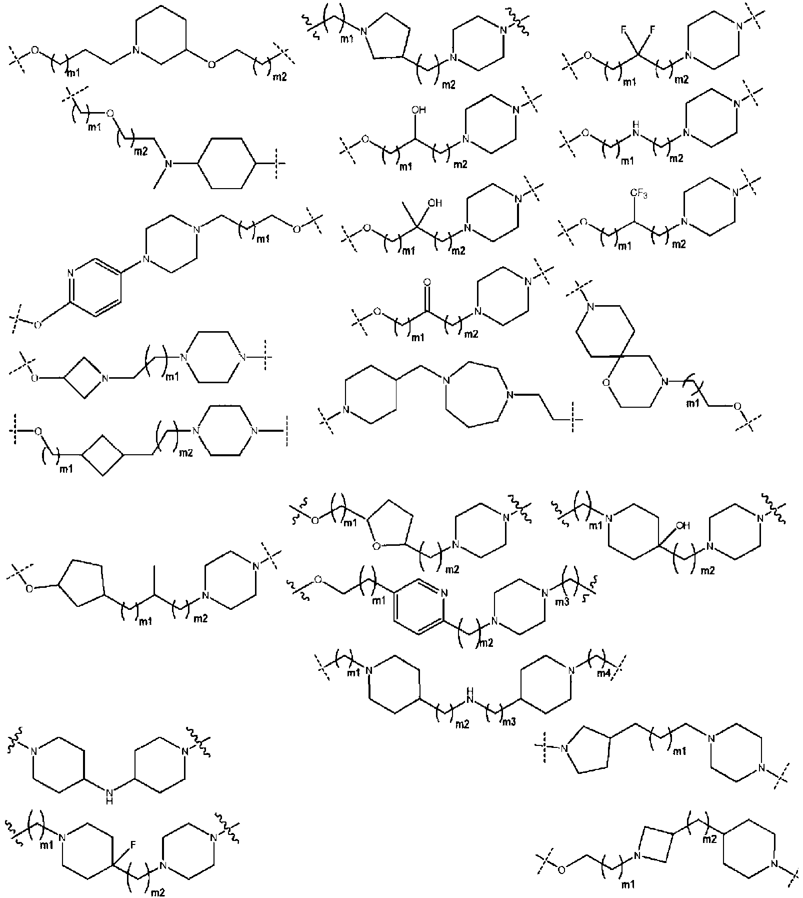 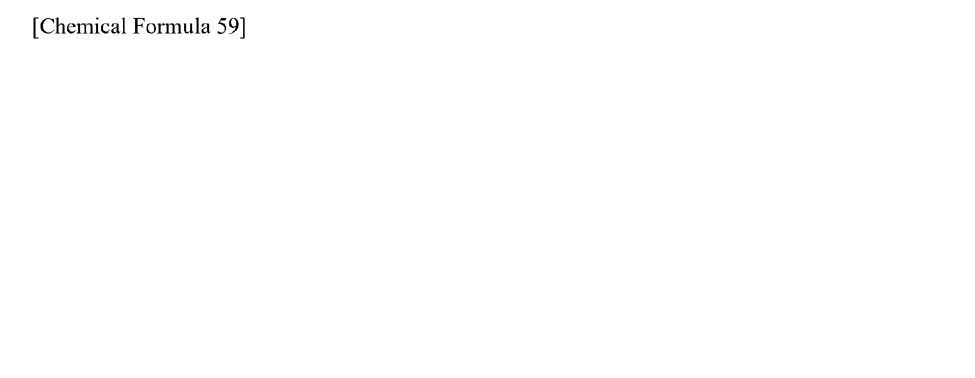 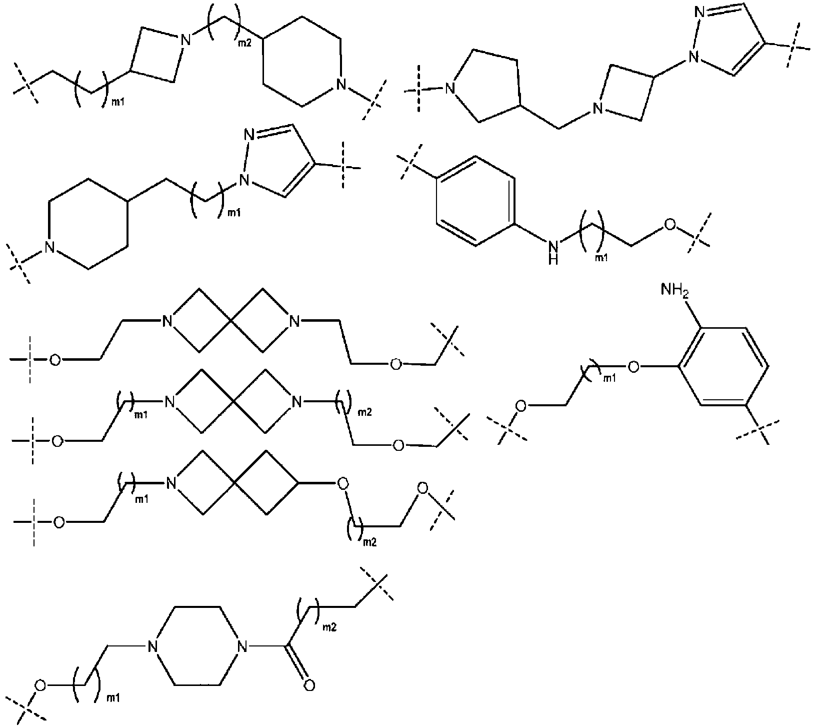 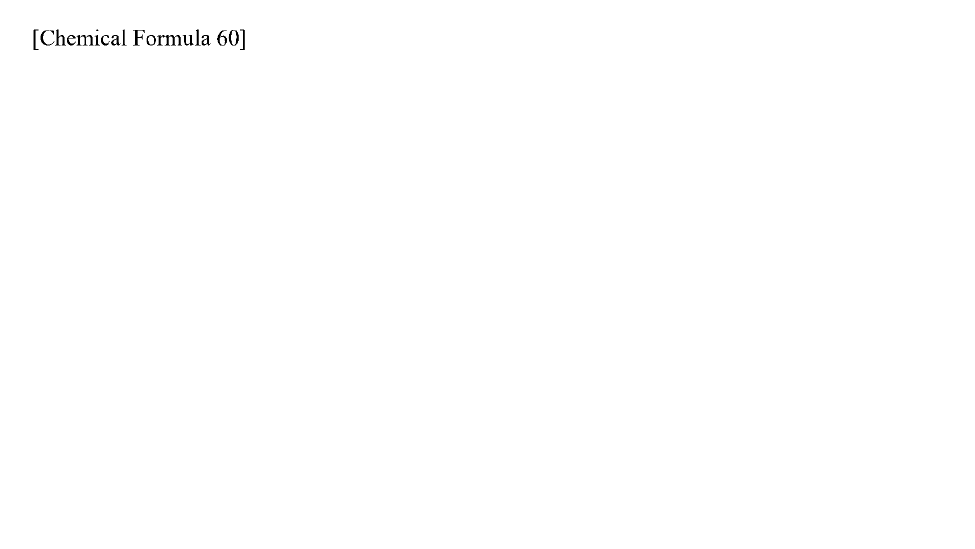 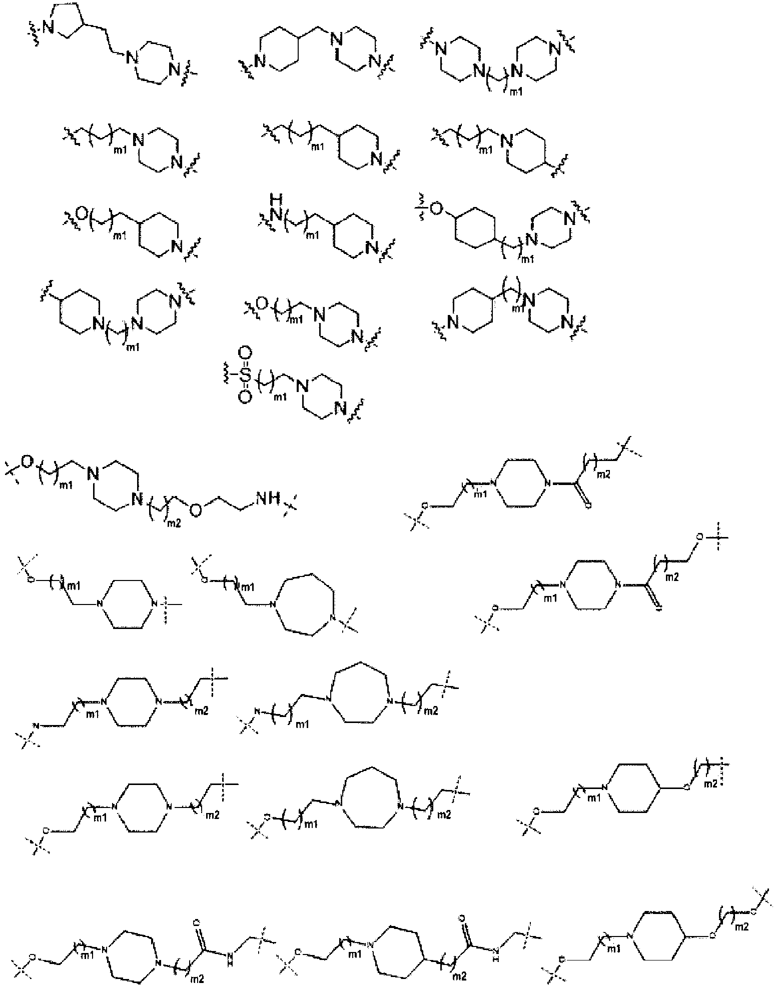 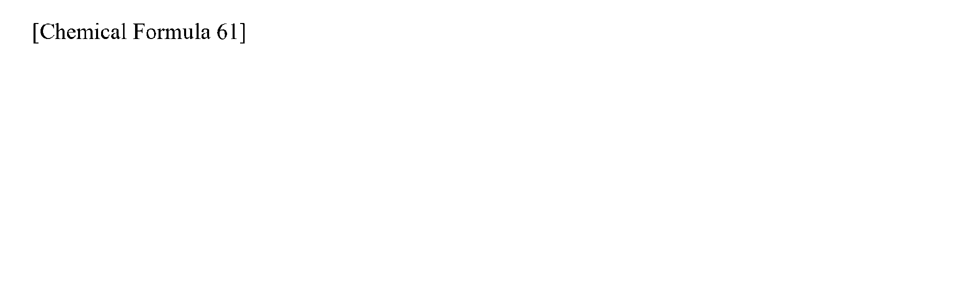 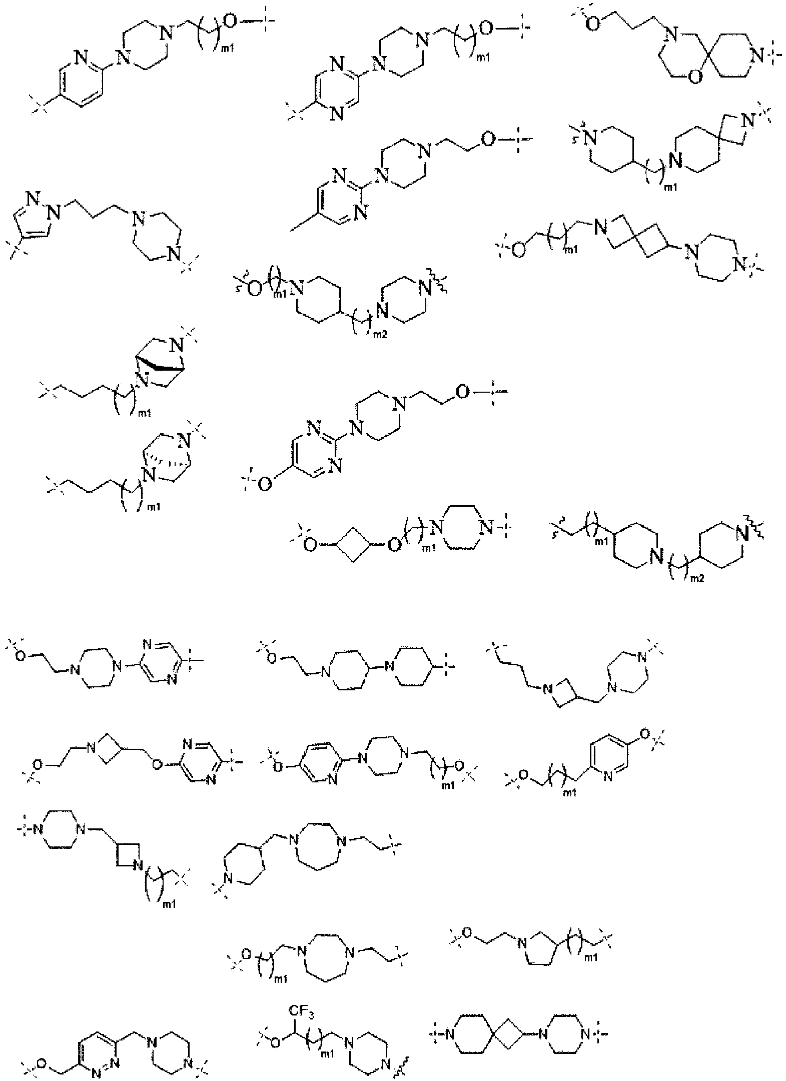 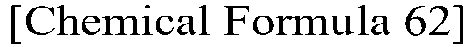 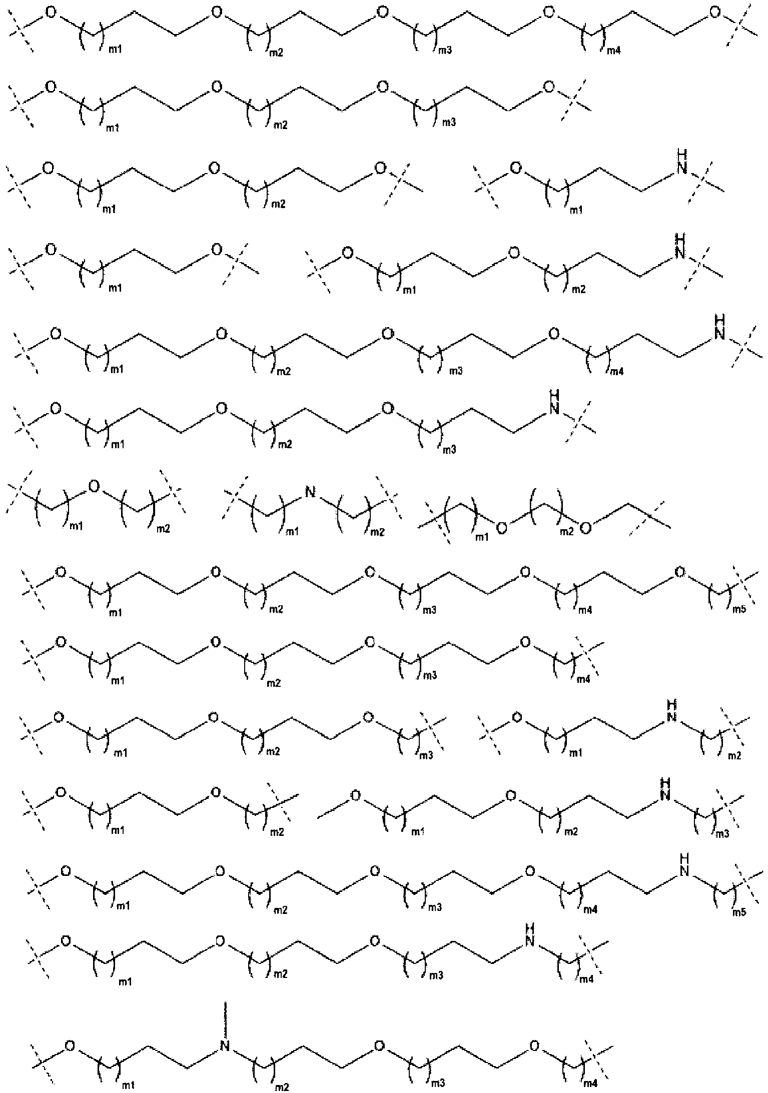 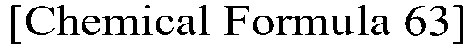 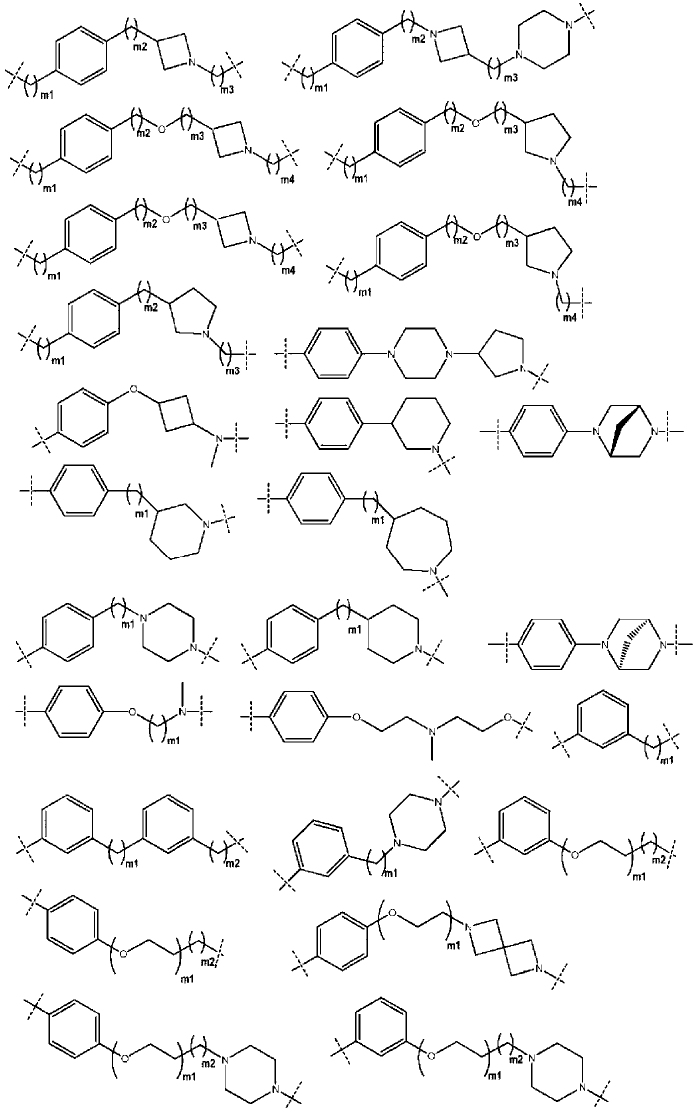 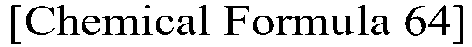 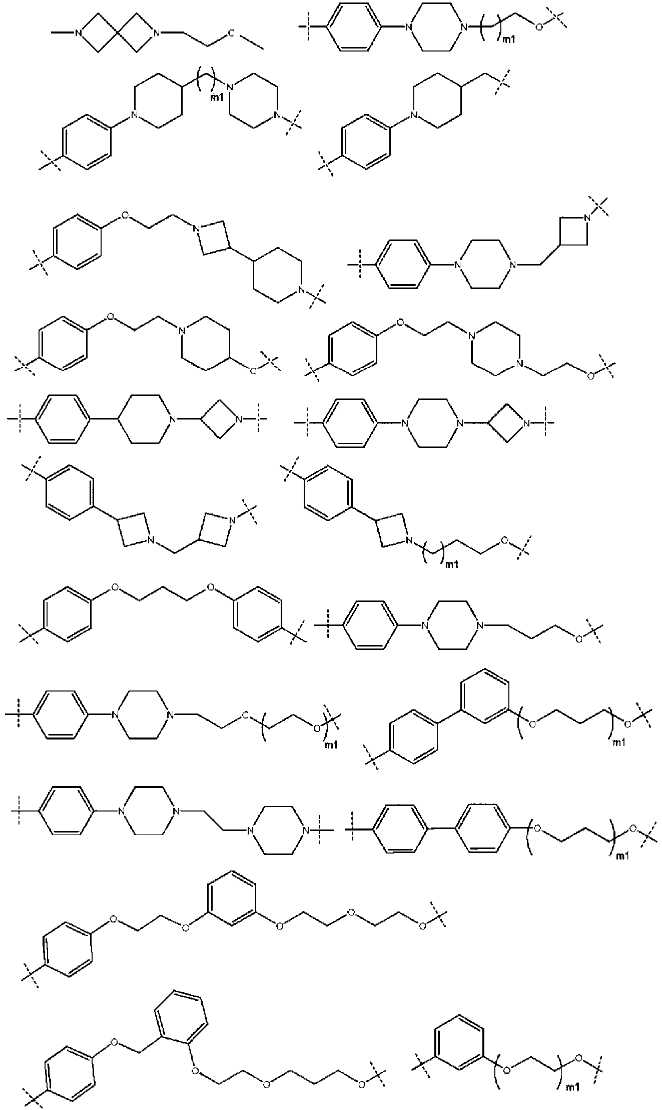 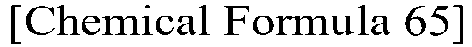 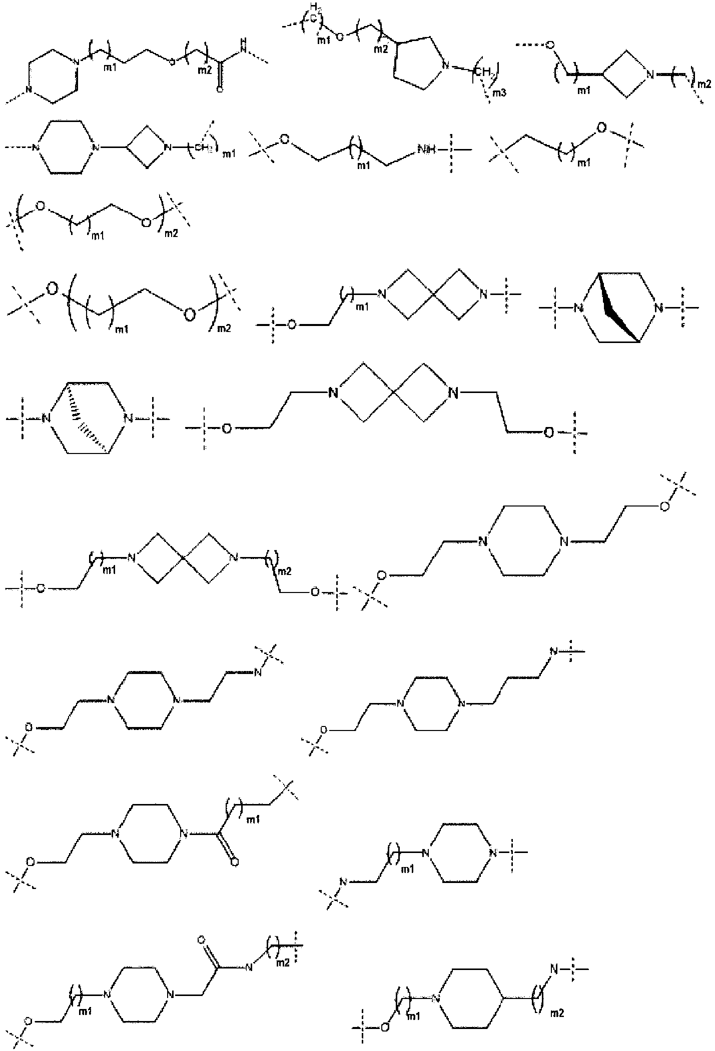 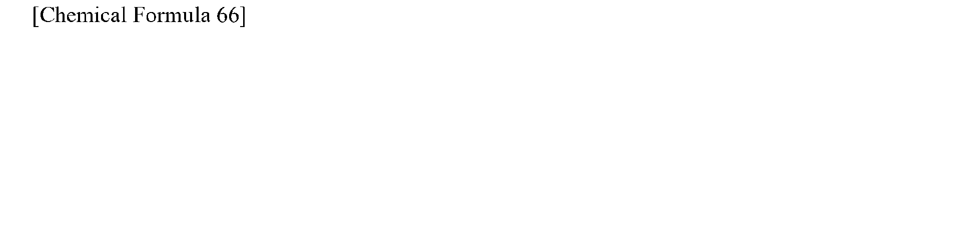 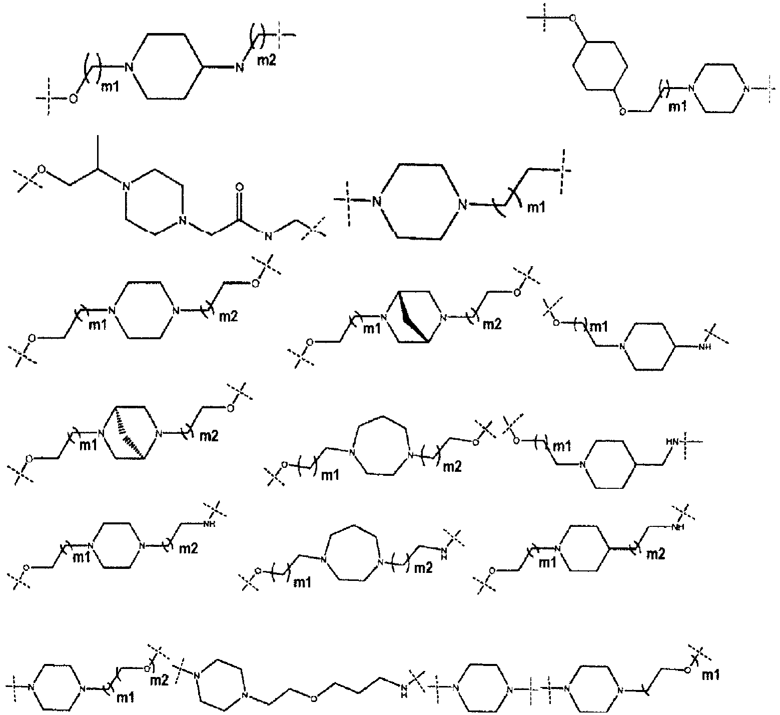 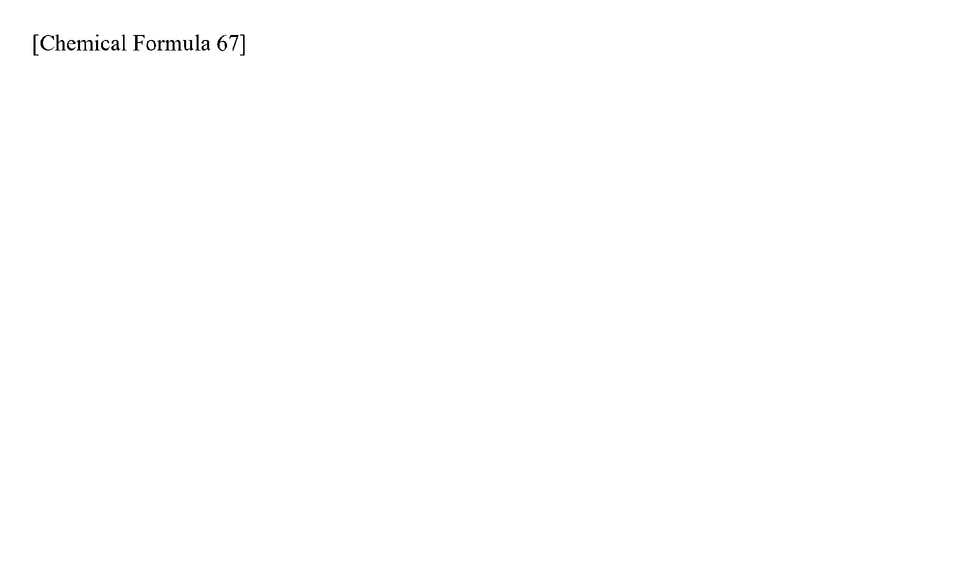 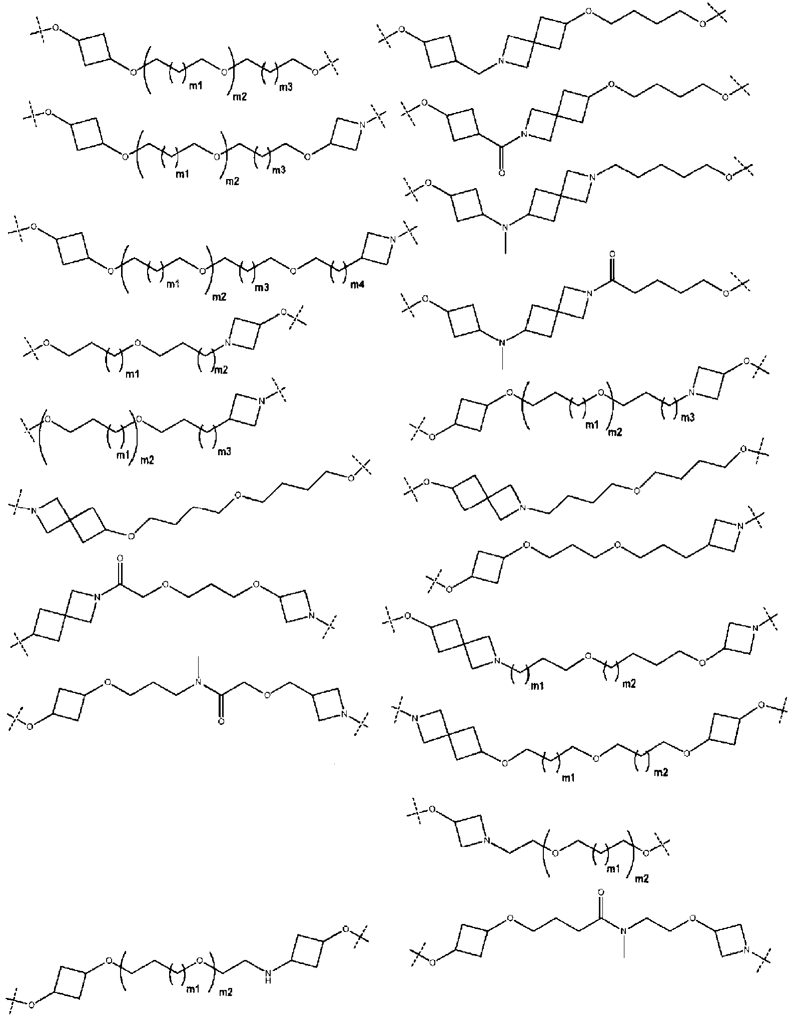 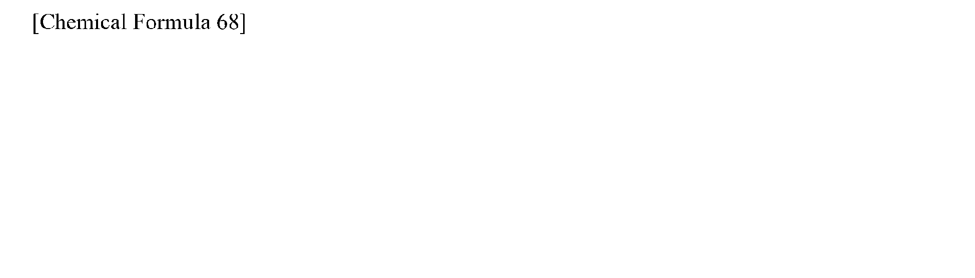 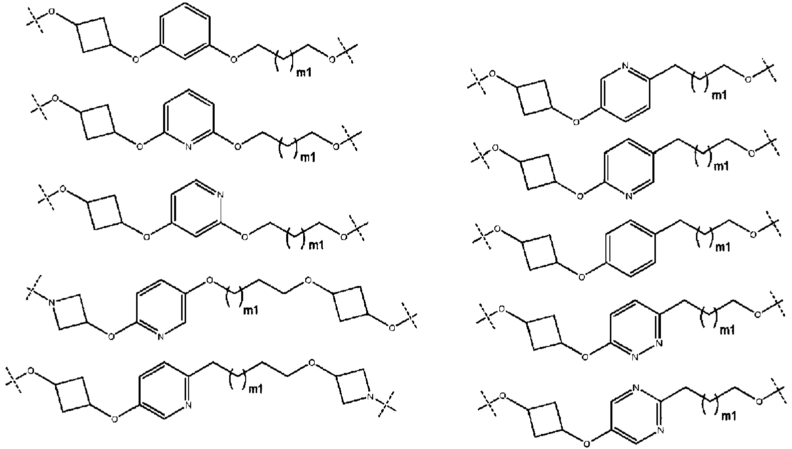 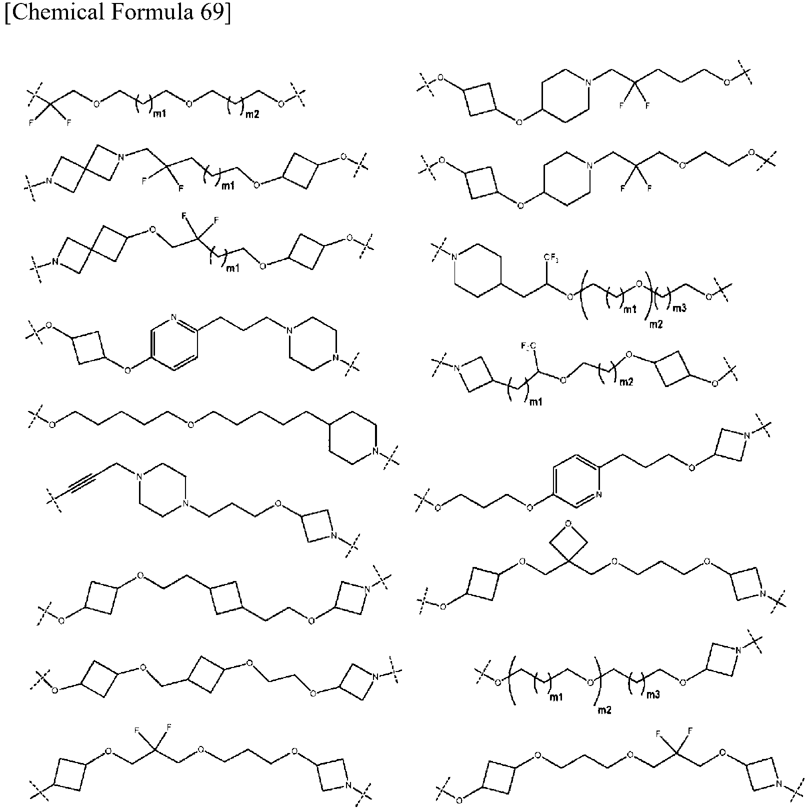 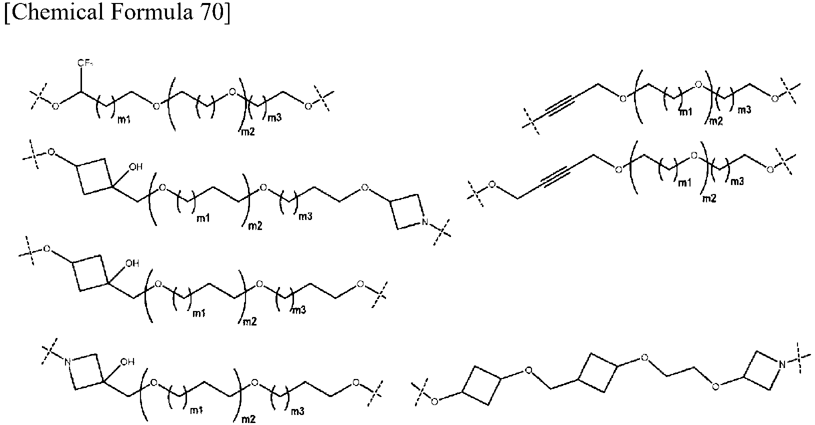 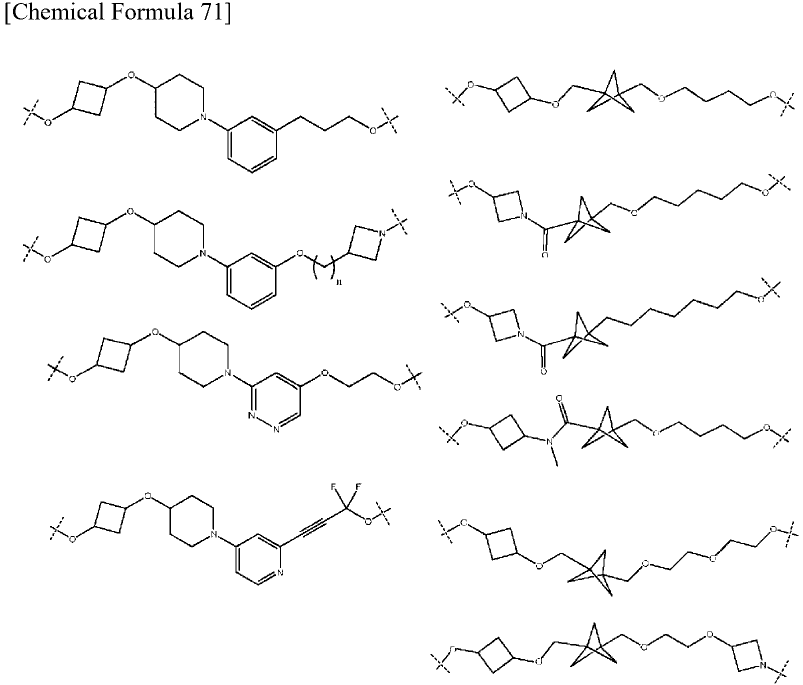 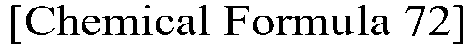 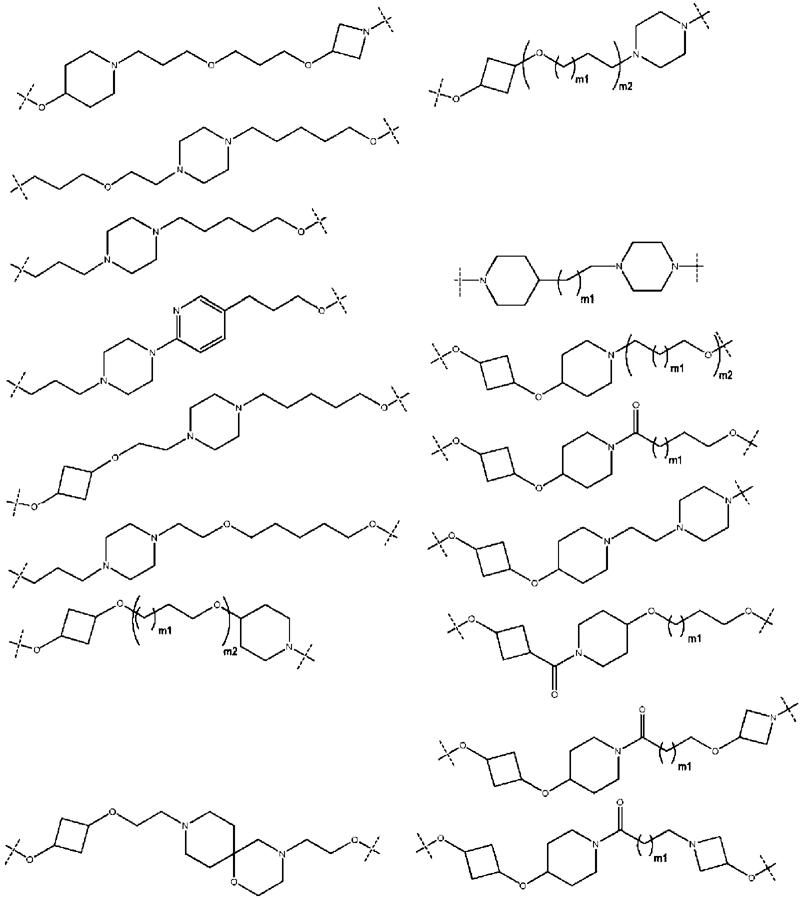 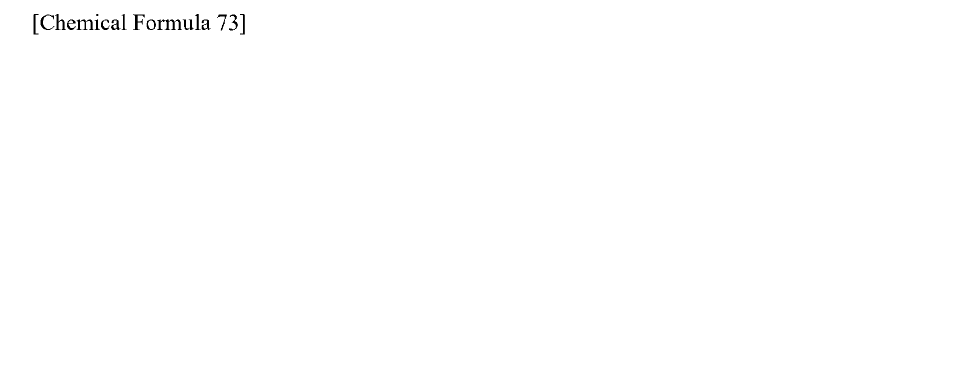 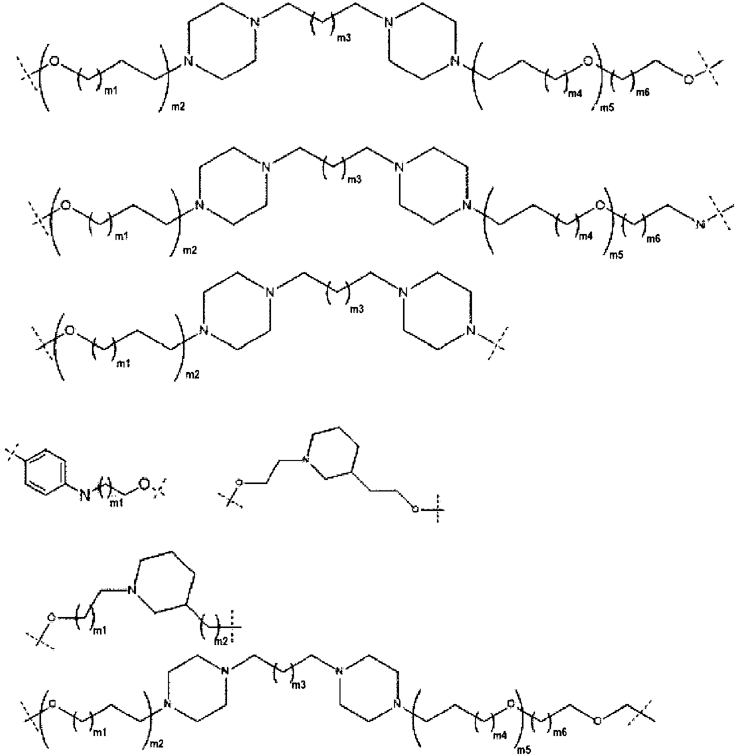 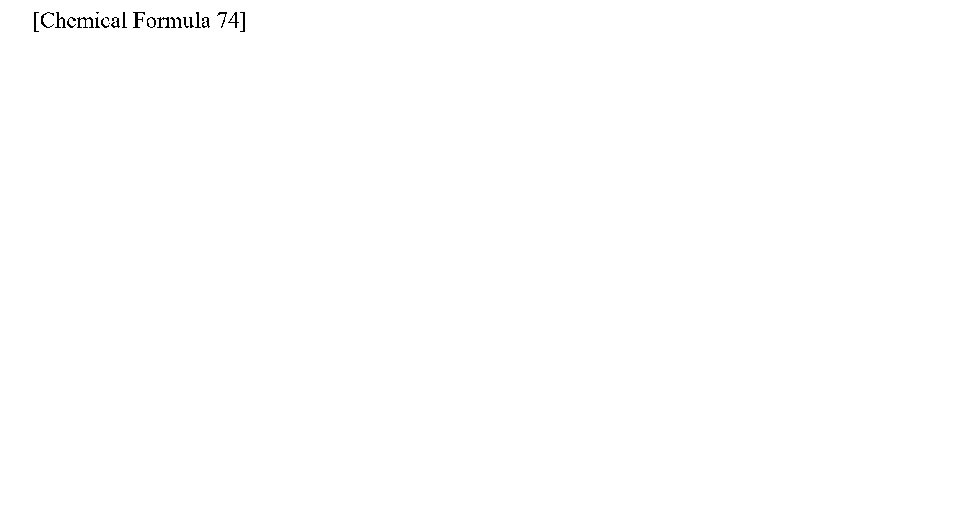 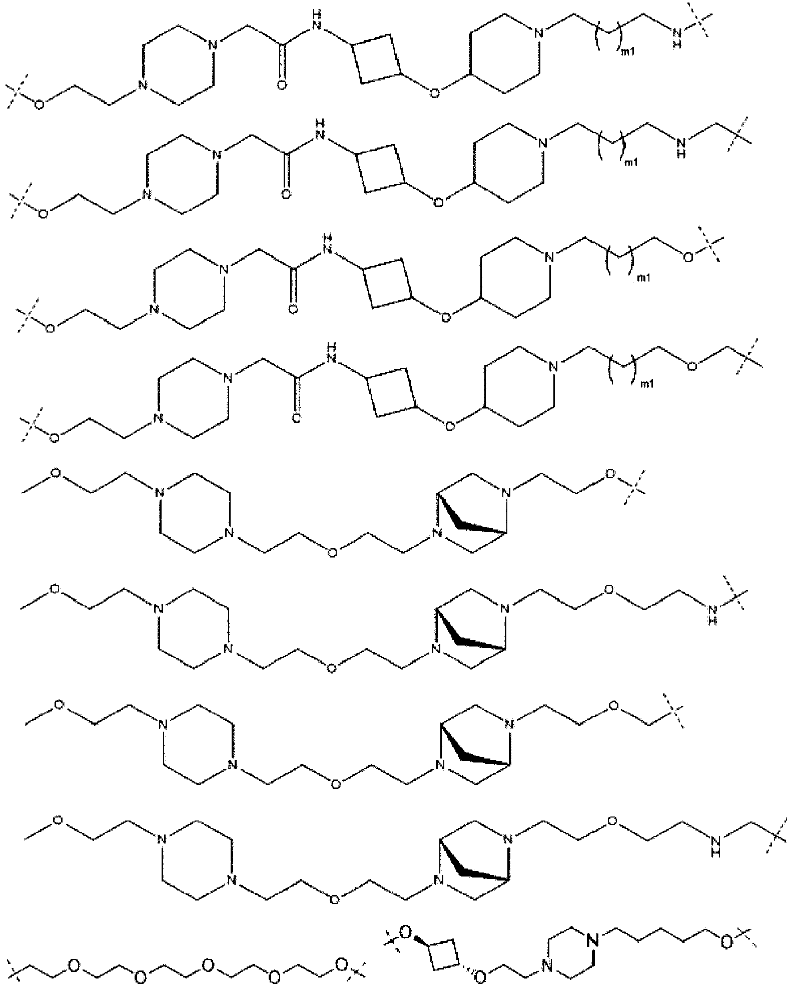 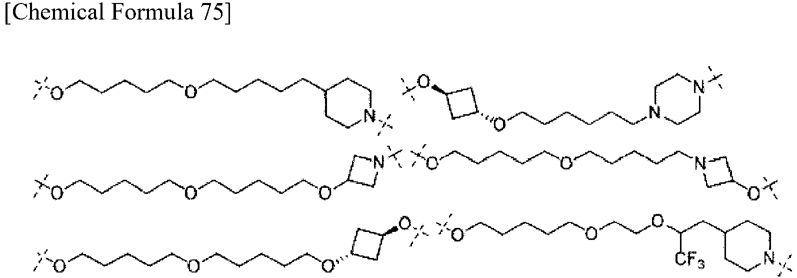 [wherein
m1, m2, m3, m4, m5, and m6 are each independently 0, 1, 2, 3, 4, 5, 6, 7, 8, 9, 10, 11, 12, 13, 14, 15, 16, 17, 18, 19, or 20].
m1, m2, m3, m4, m5, and m6 are each independently preferably 1, 2, 3, 4, 5, or 6. In the above groups, two termini (may each be indicated by a dashed line or a wavy line) are each a point of attachment to an optionally substituted group represented by Y¹, R^{a}, R^{b}, R^{c}, or R^{d}, and to a first terminal group or a substituent of substituted L. Each terminus can be a point of attachment to either an optionally substituted group represented by Y¹, R^{a}, R^{b}, R^{c}, or R^{d}, or to a first terminal group or a substituent of substituted L.

Further, in the above aspect (35), (35-2) or (35-3), as another aspect (42), the compound or a pharmacologically acceptable salt thereof is provided wherein, the linker is a group selected from a group consisting of:

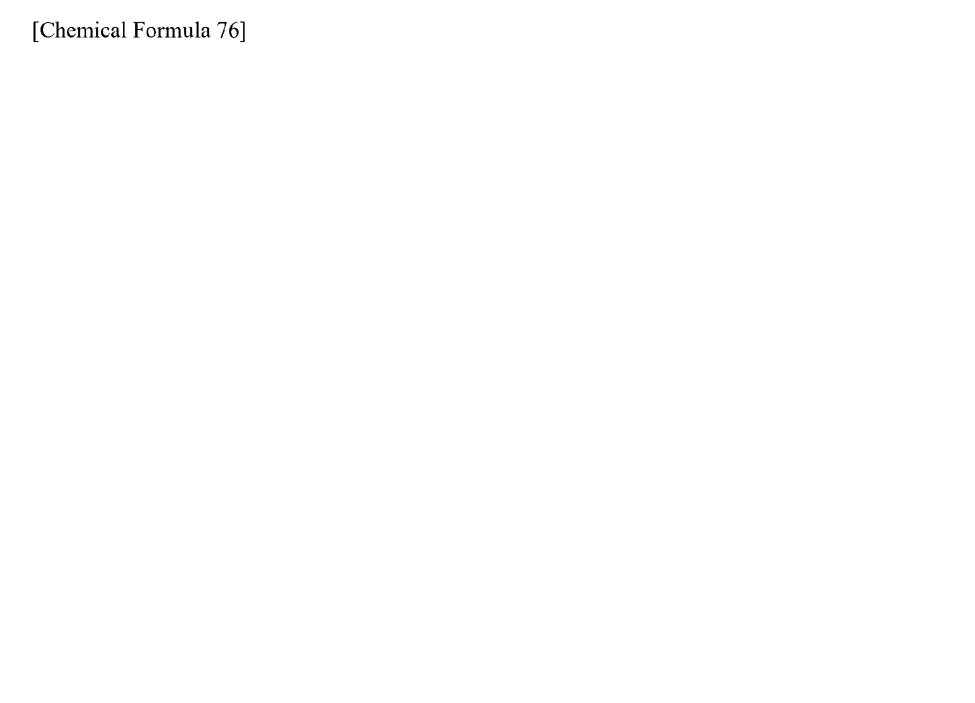

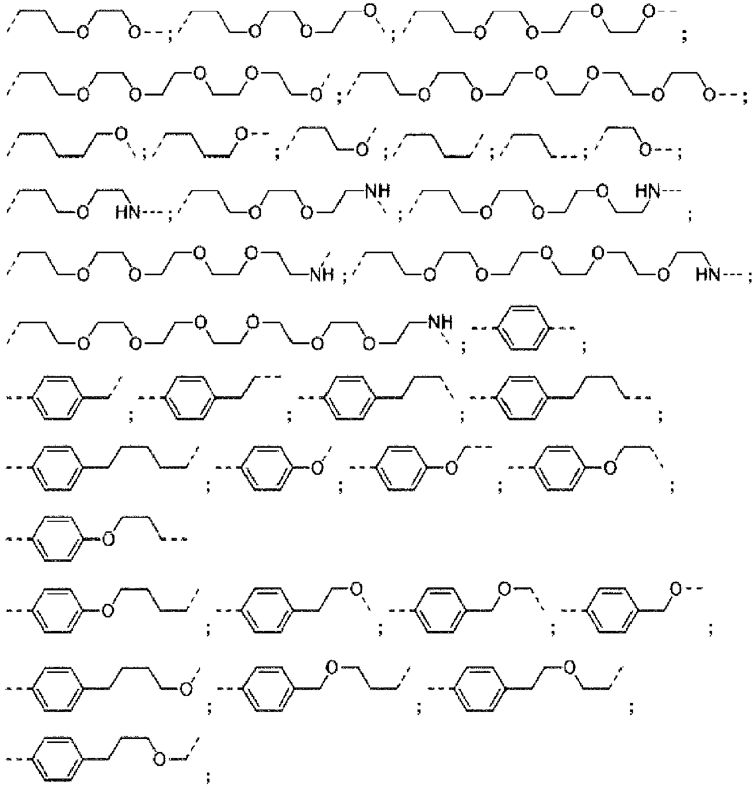

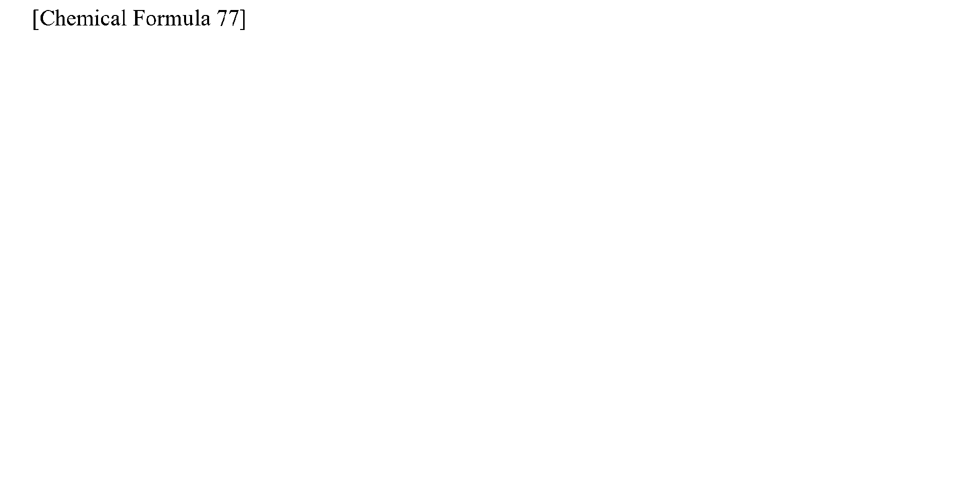

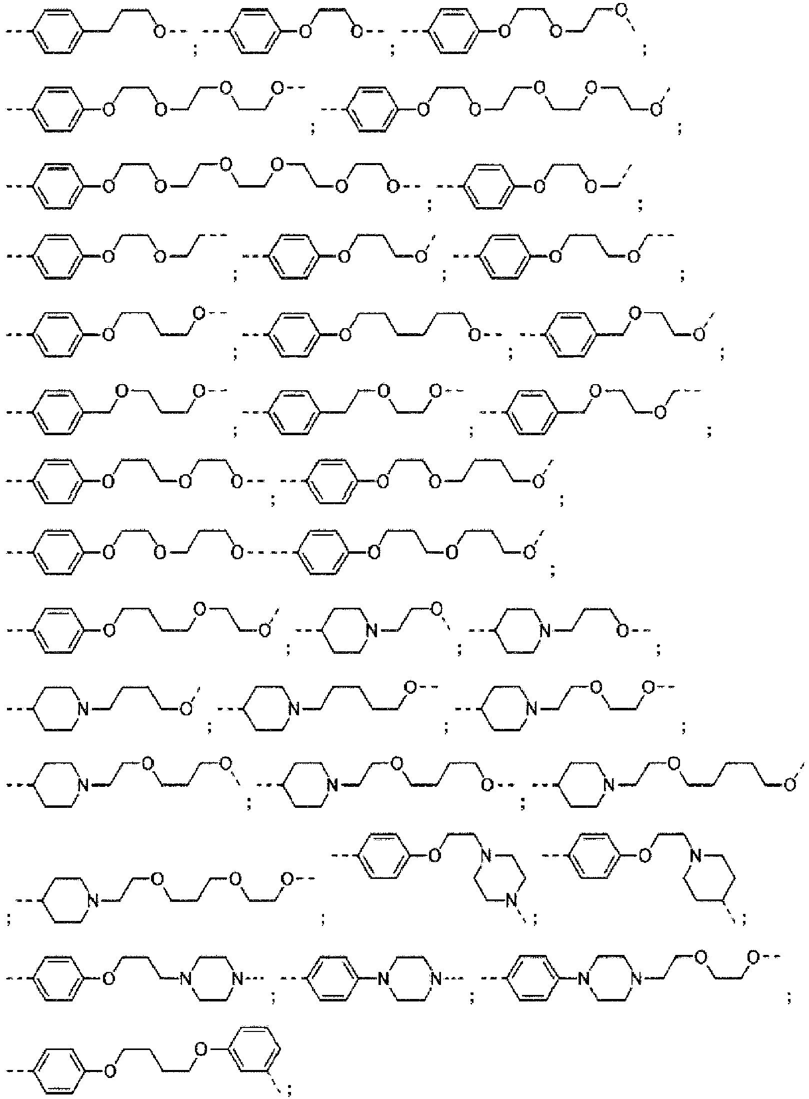

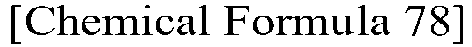

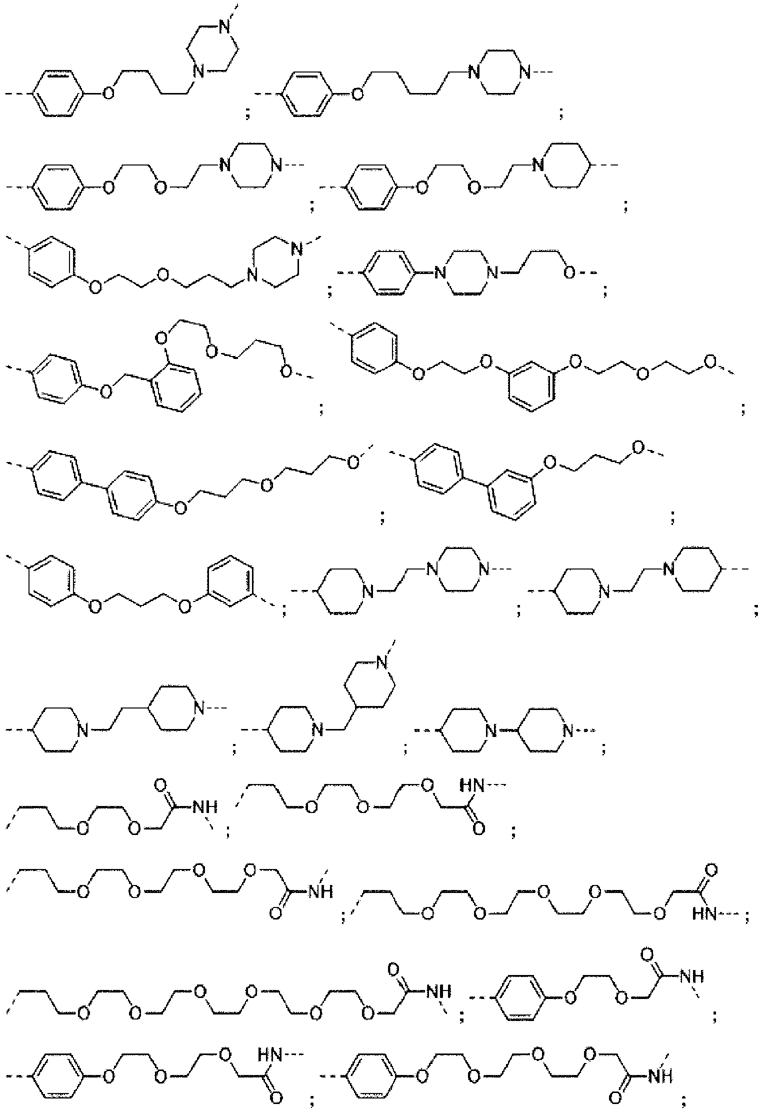

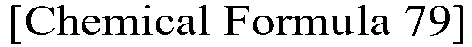

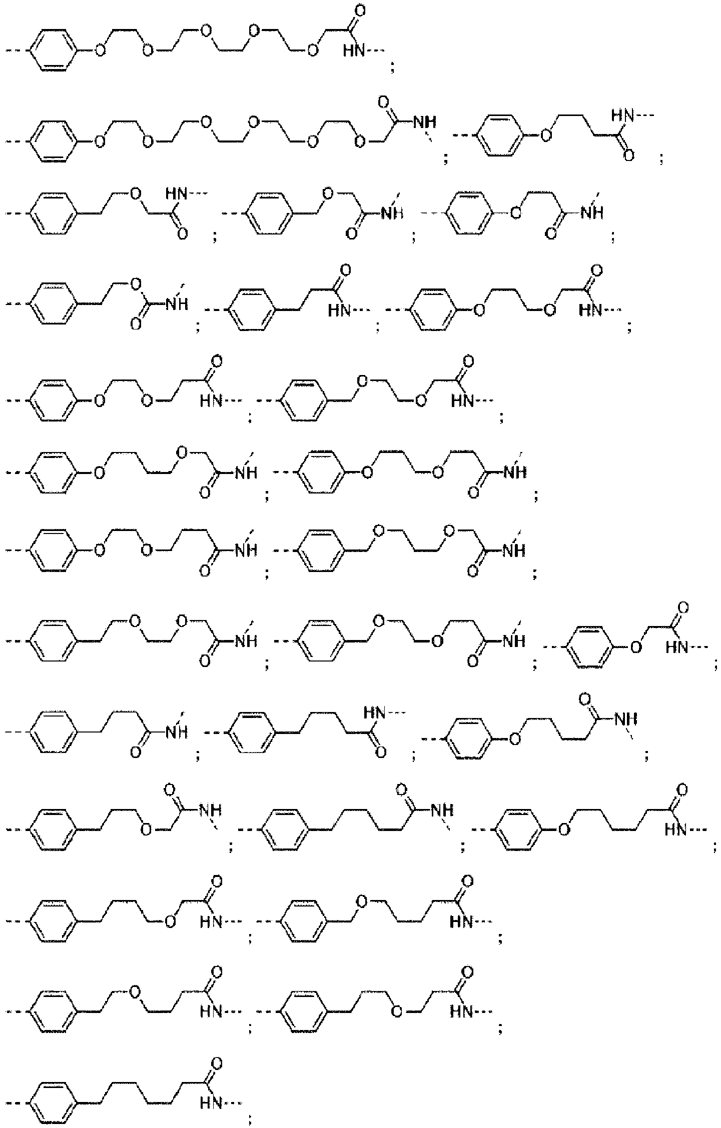

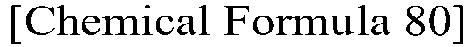

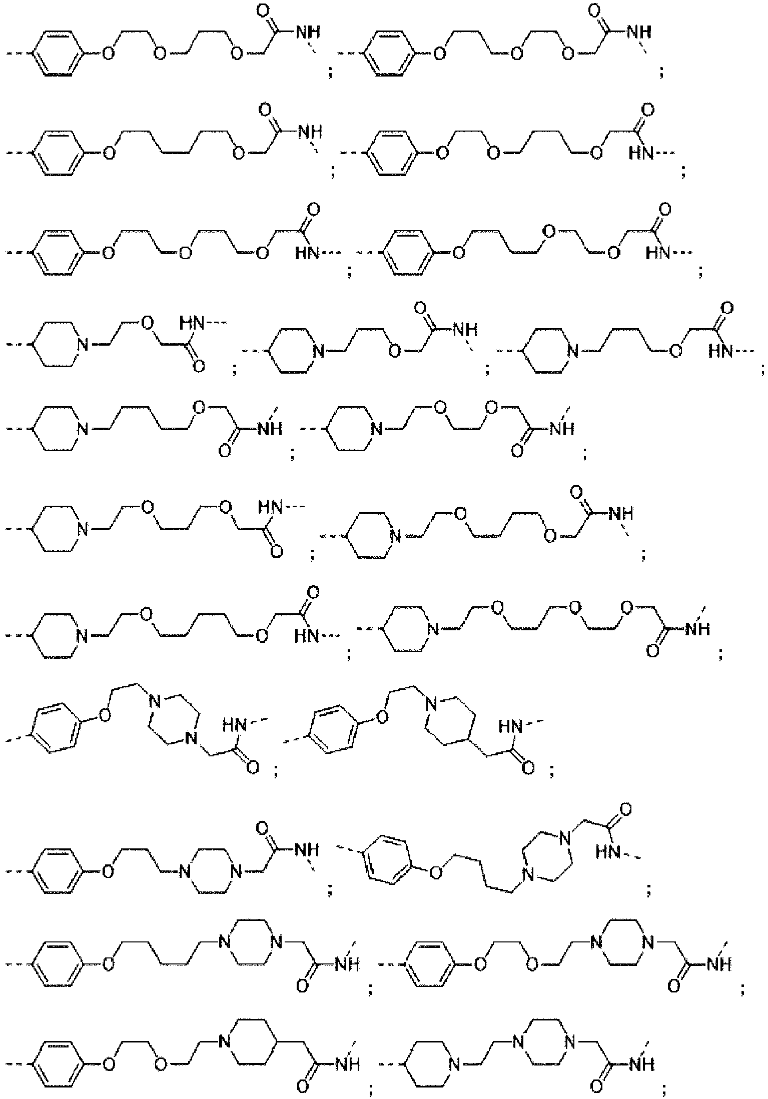

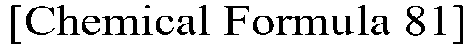

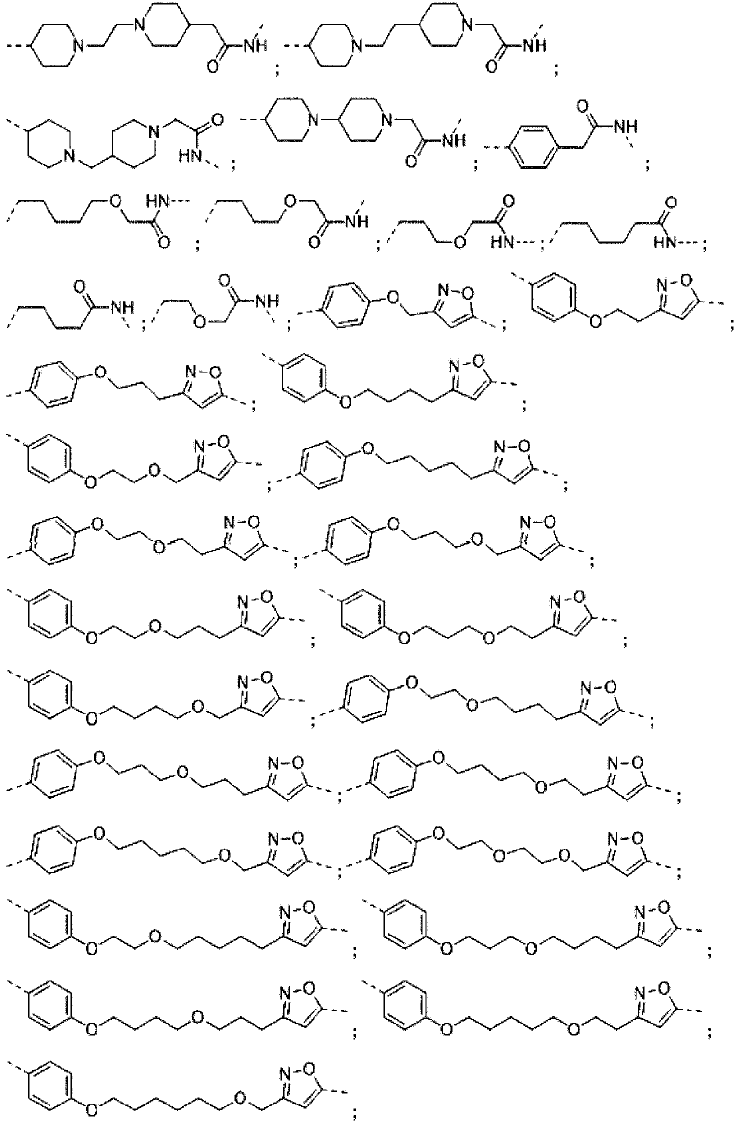

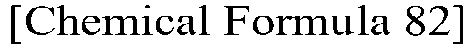

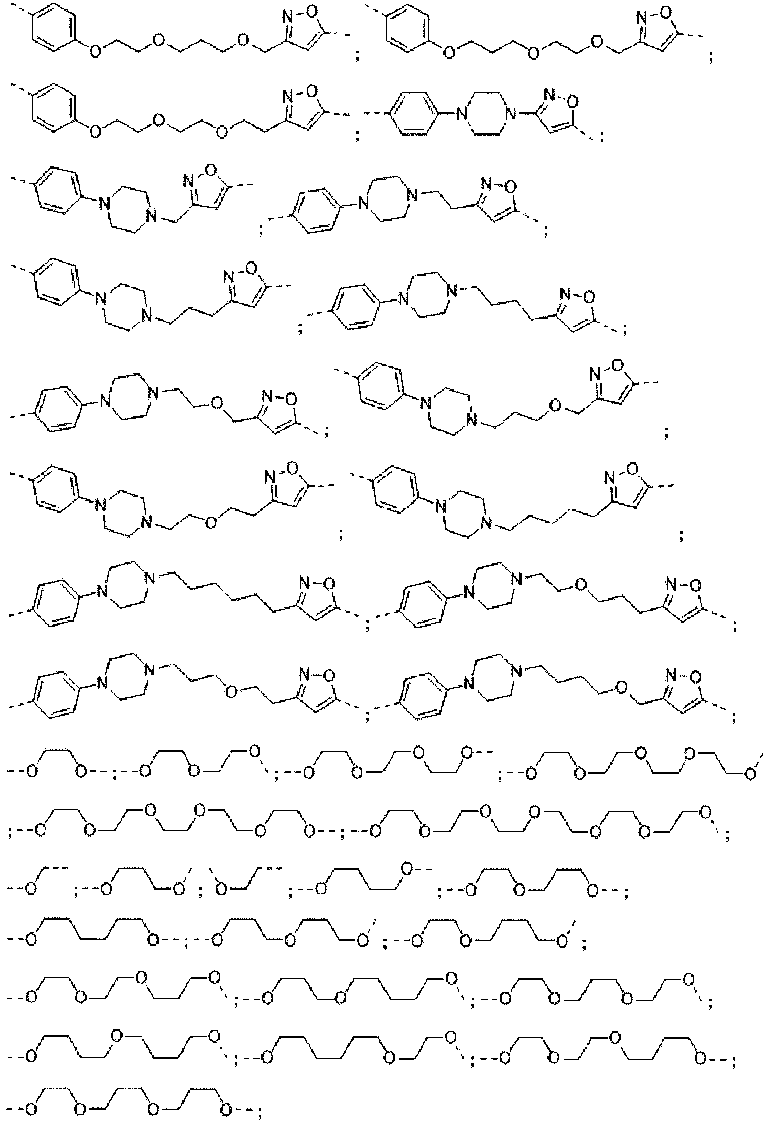

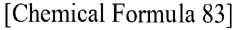

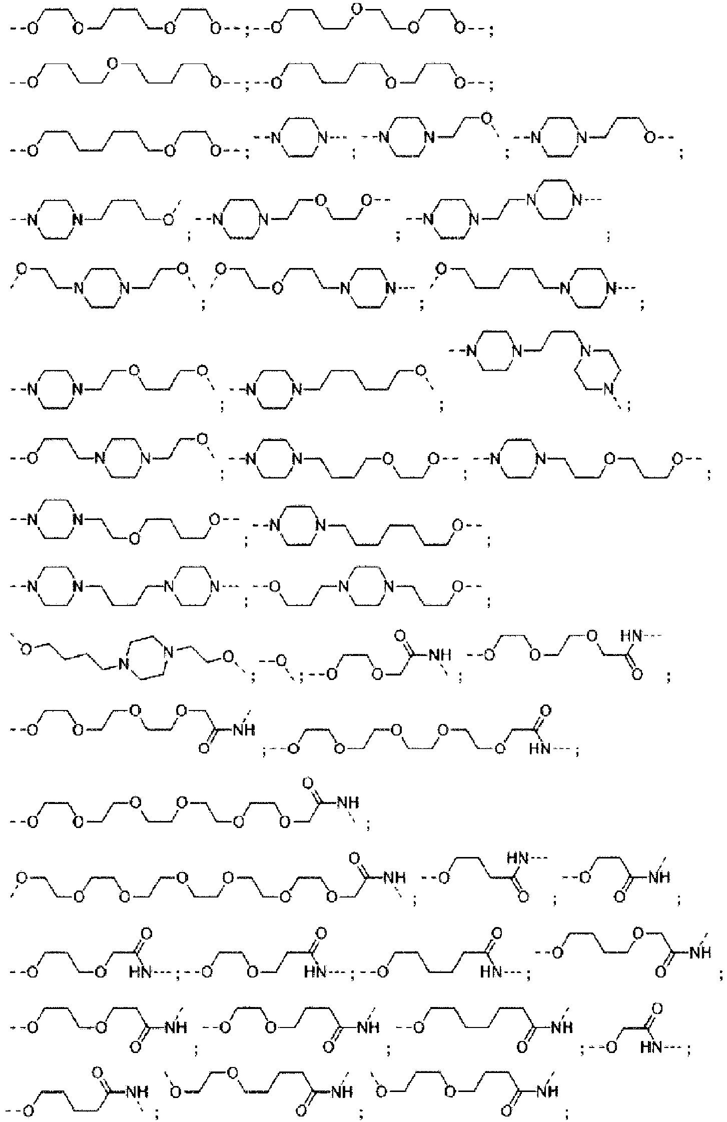

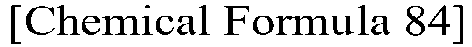

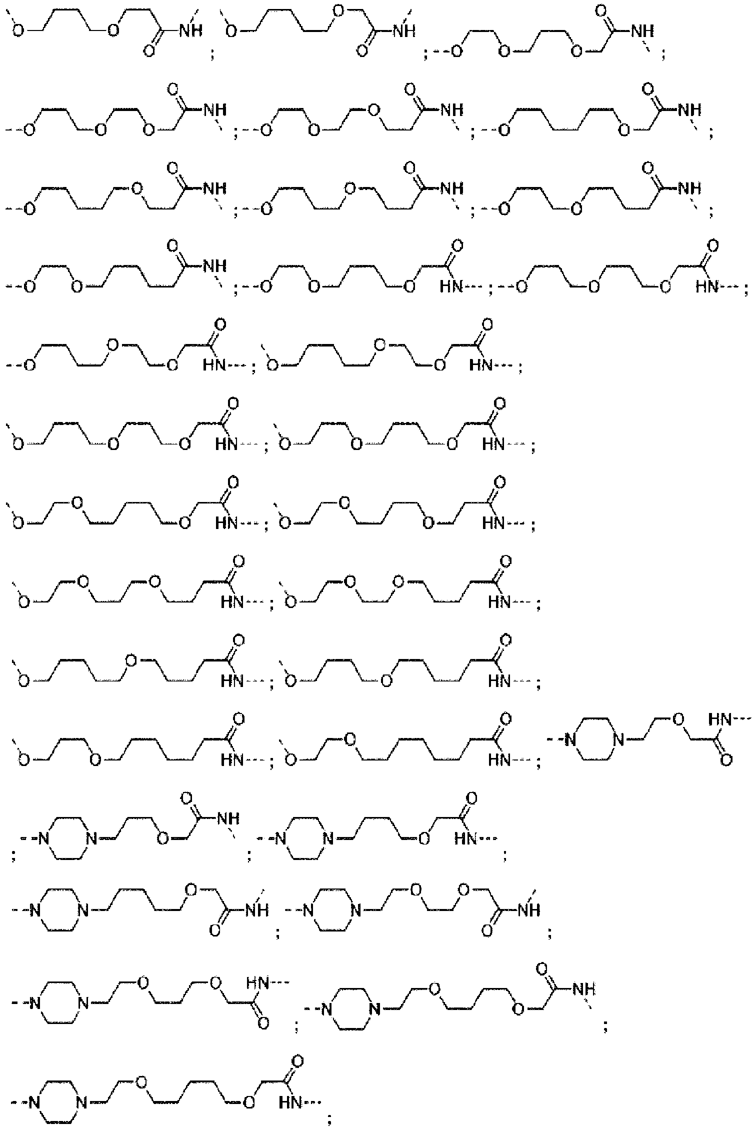

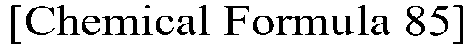

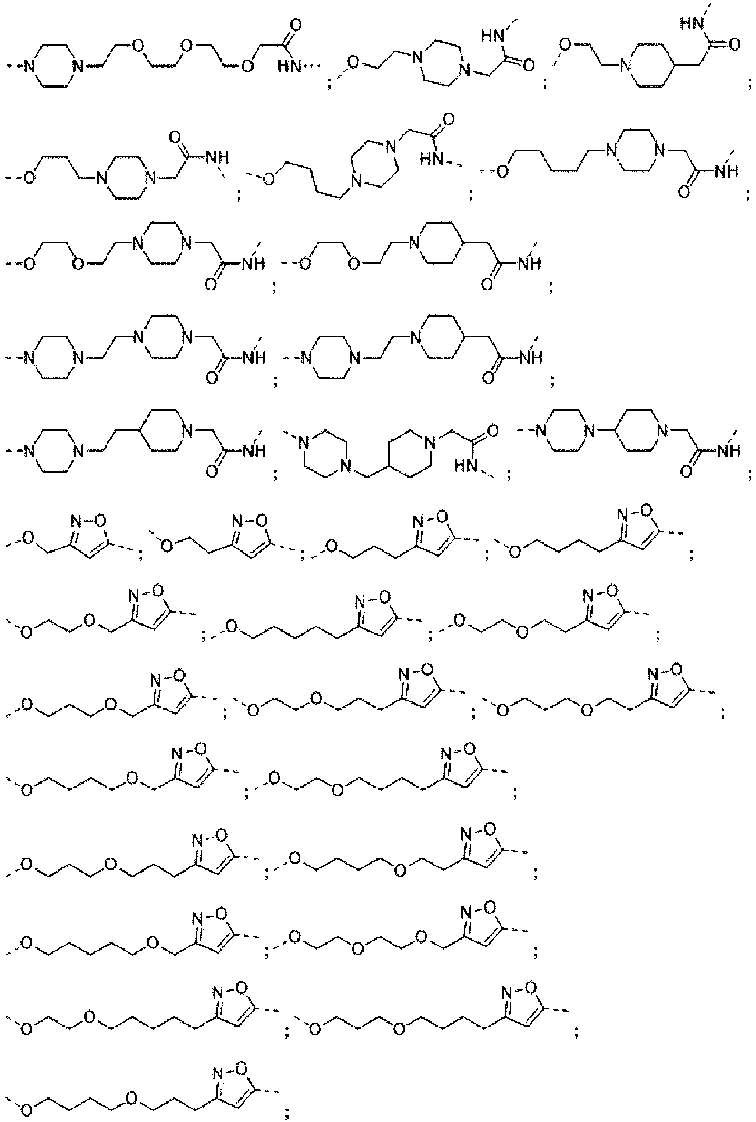

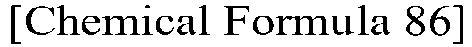

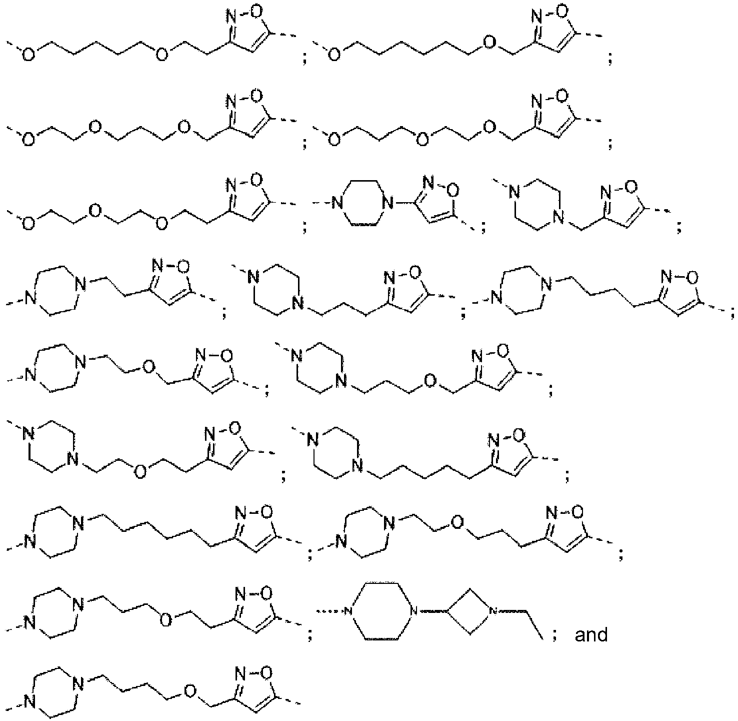

In the above groups, two termini (may each be indicated by a dashed line or a wavy line) are each a point of attachment to an optionally substituted group represented by Y¹, R^{a}, R^{b}, R^{c}, or R^{d}, and to a first terminal group or a substituent of substituted L. Each terminus can be a point of attachment to either an optionally substituted group represented by Y¹, R^{a}, R^{b}, R^{c}, or R^{d}, or to a first terminal group or a substituent of substituted L.

Further, in the above aspect (35), (35-2) or (35-3), as another aspect (43), the compound or a pharmacologically acceptable salt thereof is provided wherein, L is a group selected from a group consisting of: 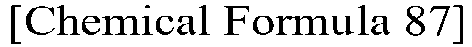 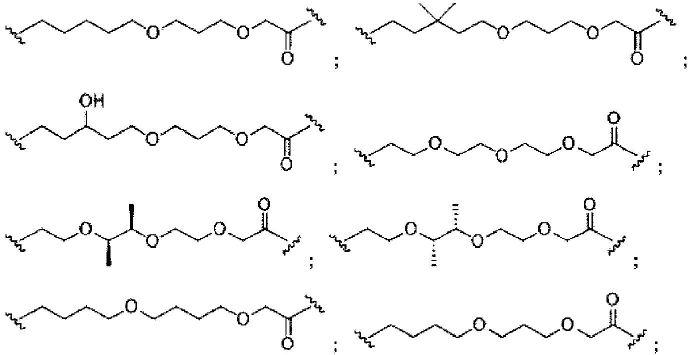 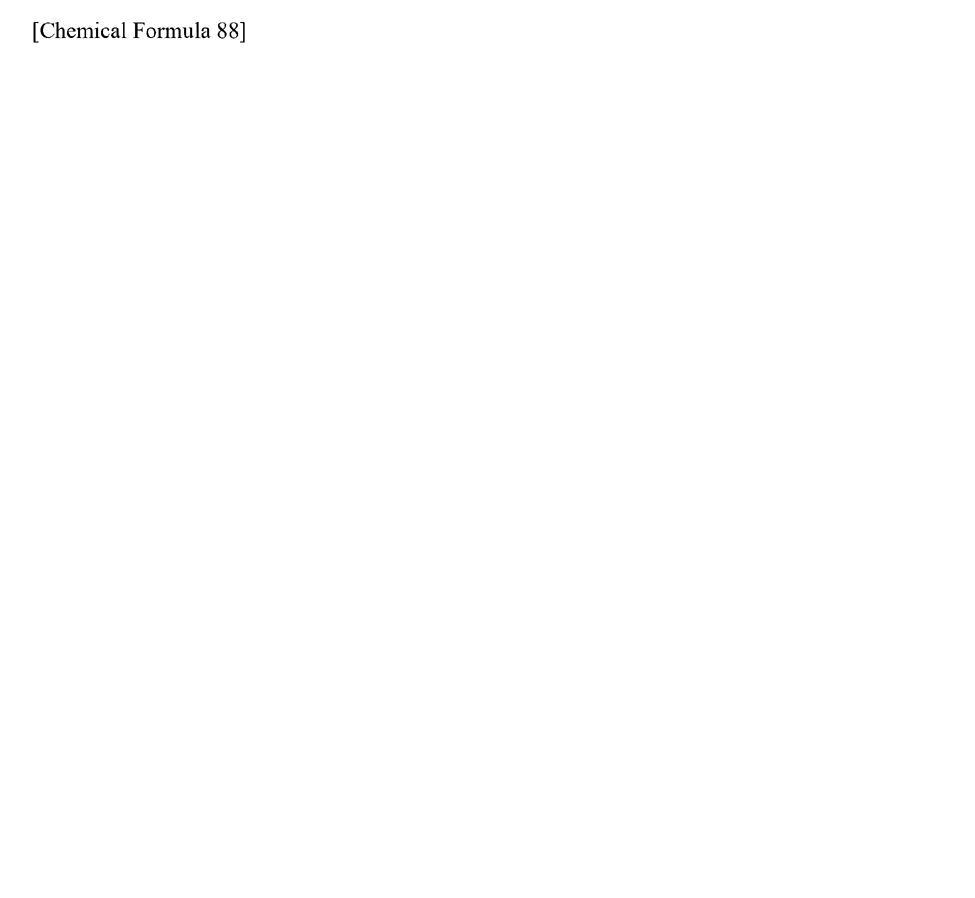 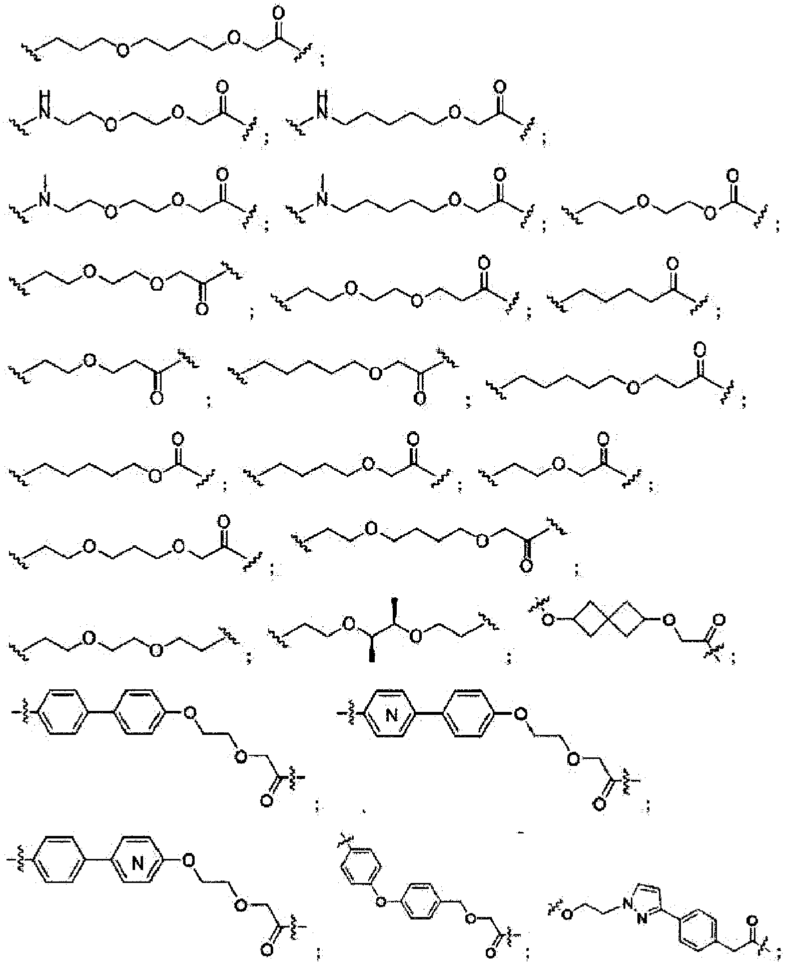 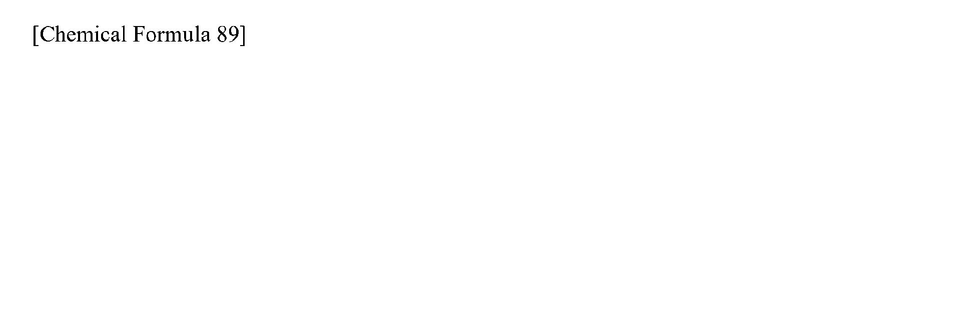 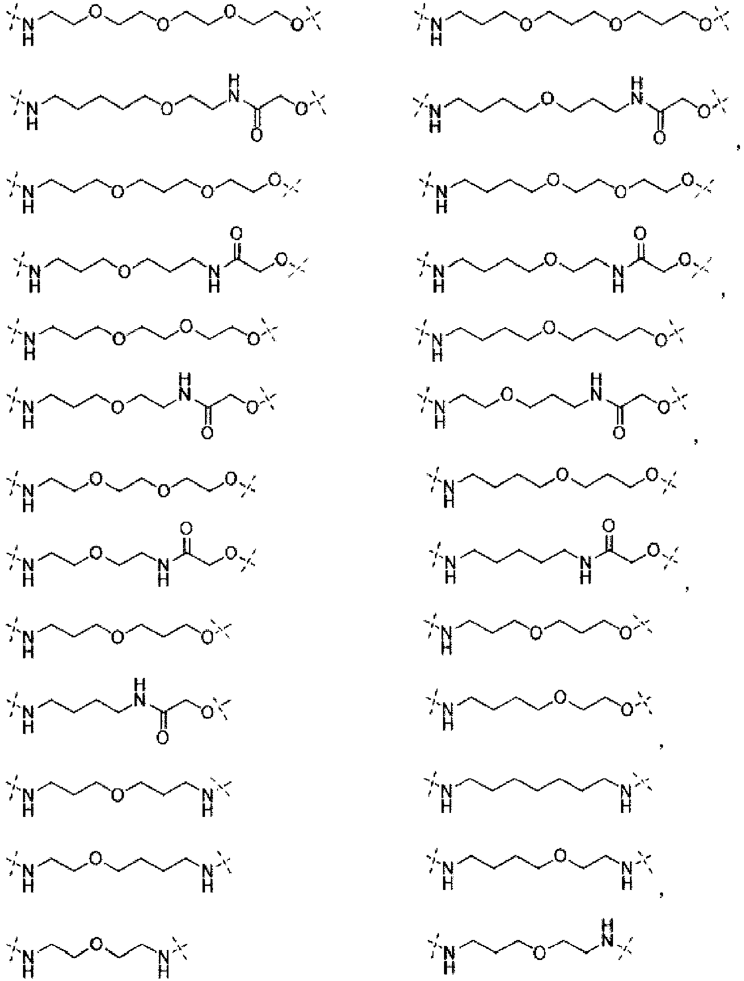 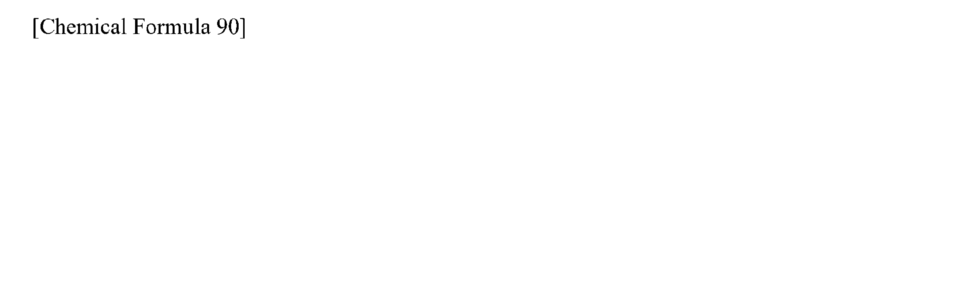 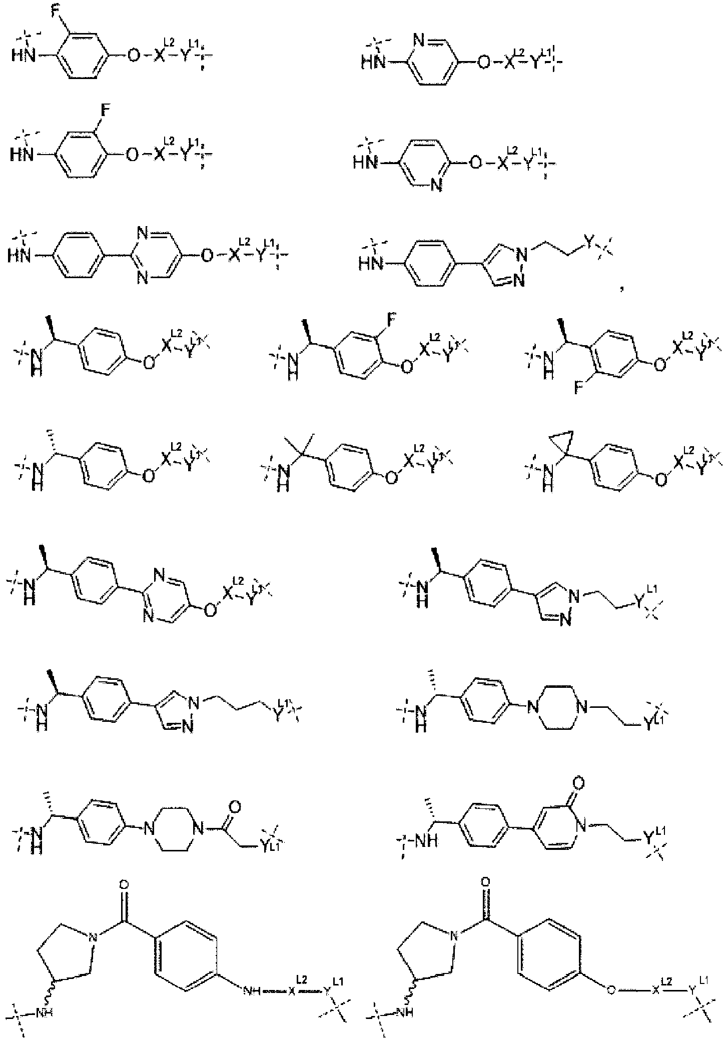 [wherein
X^{L2} is a linear chain containing 2 to 14 atoms, and may contain an oxygen atom, and
Y^{L1} is O, N, S, SO, or SO₂].

In the above groups, two termini (may each be indicated by a dashed line or a wavy line) are each a point of attachment to an optionally substituted group represented by Y¹, R^{a}, R^{b}, R^{c}, or R^{d}, and to a first terminal group or a substituent of substituted L. Each terminus can be a point of attachment to either an optionally substituted group represented by Y¹, R^{a}, R^{b}, R^{c}, or R^{d}, or to a first terminal group or a substituent of substituted L. In the above formula, 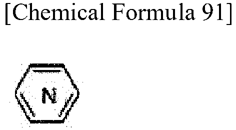 represents a 6-membered heteroaryl ring containing one or more nitrogen atoms.

The first terminal group in L having a first terminal group is a group capable of forming a chemical bond with a TBL compound and is bonded to a linker main chain (may be denoted as "L_{M}"). The linker main chain L_{M} can be, for example, L2 in the above aspect (36), L_{b2} in the above aspect (37), a group represented by Formula (V) in the above aspect (38), or each group in the above aspects (39) to (43). Examples of the first terminal group include: 1) hydrogen atom, 2) halogen atom, 3) -CN, 4) -NO₂, 5) -SF₅, 6) -CO₂H, 7) -N(R^{L2}R^{L2}'), 8) -A^{L2}R^{L2}, 9) optionally substituted alkyl group, 10) optionally substituted cycloalkyl group, 11) optionally substituted heterosaturated ring group, 12) optionally substituted aryl group, 13) optionally substituted heteroaryl group, 14) -SO₂R^{L2}, 15) -P(O)(OR^{L2})OR^{L2}', 16) -C≡CR^{L2}, 17) -C(R^{L2})=C(R^{L2}'R^{L2}"), 18) -COR^{L2}, 19) -CON(R^{L2}R^{L2}'), 20) -SO₂N(R^{L2}R^{L2}'), 21) -N(R^{L2})CON(R^{L2}'R^{L2}"), and 22) - N(R^{L2})SO₂N(R^{L2}'R^{L2}") (wherein, A^{L2} represents an oxygen atom or a sulfur atom, and R^{L2}, R^{L2}', and R^{L2}" each independently represent: a) a hydrogen atom, b) an optionally substituted C₁-C₈ alkyl group, c) an optionally substituted C₃-C₈ cycloalkyl group, or d) an optionally substituted C₁-C₈ alkoxy group (for example, methoxy, ethoxy, tert-butoxy, benzyloxy)).

The meanings of the terms "halogen atom," "optionally substituted alkyl group," "optionally substituted cycloalkyl group," "optionally substituted heterosaturated ring," "optionally substituted aryl group," "optionally substituted heteroaryl group," "optionally substituted C₁-C₈ alkyl group," "optionally substituted C₃-C₈ cycloalkyl group," and "optionally substituted C₁-C₈ alkoxy group" for the groups in the above definitions can be referred to in the [Definitions of the groups used in the present specification] described earlier. Here, examples of "substituents" in an "optionally substituted group" include substituents selected from the [Substituent Group] described in the above [Definitions of the groups used in the present specification]. 1 to 5 or 1 to 3 such "substituents" can be present at any substitutable positions, and when the number of substituents is 2 or more, the substituents may be the same or different.

Further, in any one of the above aspects (36) to (45), as a further aspect (46), the compound or a pharmacologically acceptable salt thereof is provided wherein, the substituted L is substituted with TBL, wherein TBL is a group having a moiety capable of binding to a target protein or a moiety that binds to a target protein. TBL may also be referred to as a target-directed ligand moiety. A person skilled in the art can appropriately select or design a target-directed ligand for use depending on an intended target protein.

When L is a bond and substituted with TBL, TBL is directly bonded to a compound of Formula (I), Formula (II), Formula (III), or Formula (IV). The bond may be a single bond or a double bond.

Further, in the above aspect (44), as an aspect (45), the compound or a pharmacologically acceptable salt thereof is provided wherein, the target protein is selected from a group consisting of proteins related to cancer-related proteins, autoimmune disease-related proteins, inflammatory disease-related proteins, neurodegenerative disease-related proteins, and genetic disease-related proteins.

Further, in the above aspect (45), as an aspect (46), the compound or a pharmacologically acceptable salt thereof is provided wherein, the target protein is a cancer-related protein. Further, in the above aspect (46), as an aspect (47), the compound or a pharmacologically acceptable salt thereof is provided wherein, the cancer-related protein is selected from a group consisting of ABL, AKT, ALK, AR, ARG1, AR-V7, ASH1L, ATM, AURKA, AURORA-A, AuroraA, Bcl2, Bcl-6, BCL9 (β-catenin PPI), Bcl-XL, BCR-ABL, BRAF, BRDs, BRD4, BET (Bromodomain and extraterminal domain) proteins, BRG1/BRM, BRPF1, BTK, CBFβ, CBP, CBP/p300, CDK2, CDK2/5, CDK2/9, CDK4/6, CDK8 (or CDK19), CDK12 (or CDK9), c-KIT, CK1α, CK1α/CDK, CK2, cMet, CRBN, CREBBP, CSF-1R kinase, cyclosporin, DOT1L, EED, EGFR, EGFR/PARP, ENL, EP300 (HAT), ER, ErBb, ERK1/2, ERK1, ERK2, EZH2, FGFR, FGFR2, FGFR3, FGFR4, FKBP, FLT3, FLT3-ITD, Gli1, GSK3β, HDAC, HDAC3, HER3, HMGCR, HPK1, HSP90, IRAK, IRAK4 BTK, ITK, JAK, JAK1,2, JAK2, JAK3, KEAP1, KRas, KRASG12D, LRRK2, LZK, MALT1, MEK, MDM2, mHTT, mTOR, MYB, NF-kB, NR4A1, NTRK1, p38a/d, PARP, PARP1, PBRM1, PD-L1, PDE4, PDGFRa, PIK3CA, PI3K/mTOR, PKCBβ1, PLK1/BRD4, PPAR, PPARγ, PRC2, PTK6, PTPN1/2, RAF, Ras, RET, SHP2, smad3, SMARCA, SMARCA2, SMARCA2/4, SOS1, STAT, STAT3, STK4, Tau, TEAD, TERT, TOP1, TDP1, TRIM24, VEGFR-2, α-tubulin, AURKB, AXL, BRD3, BRD7, BUB1B, CDK12, CDK12 C1039F, CDK17, CHEK1, c-Met, CSNK1A1, DAPK1, DDR2, eIF4E, EPHA1, EPHA2, EPHA3, EPHB2, EPHB3, EPHB4, EPHB6, FKBP12, FLT1, FYN, GCN5, HDAC2, IGF-1R, KRASG12C, LATS1, LCK, LXRA, LYN, MAP3K1, MAP3K11, MAP3K7, MAP4K1, MAP4K3, MAPK10, MAPK9, MAPKAPK2, MCL1, MerTK, MLLT1, MYC, NUAK1, PAK1, PARP2, PARP3, PCAF, PDK1, PRKAA1, PRKAA2, PRKCI, RPS6KA3, RPS6KA4, RPS6KA6, SF3B1, SIRT2, SLC9A2, Src, STK10, STK33, STK40, TAOK2, TAOK3, TGFBR1, TNK1, TTK, TYK2, and YES1.

When compounds of Formula (I), Formula (II), Formula (III), or Formula (IV) contain optical isomers (enantiomers, diastereomers), stereoisomers, positional isomers, or rotational isomers, these are also included as compounds of Formula (I), Formula (II), Formula (III), or Formula (IV), and each can be obtained as a single entity using commonly known synthetic methods or separation methods (for example, concentration, solvent extraction, column chromatography, recrystallization, and the like).

A compound of the above Formula (I), Formula (II), Formula (III), or Formula (IV) may be appropriately converted into a prodrug, and such an embodiment is also included in the scope of the present invention. A prodrug may be a compond that transforms into a compound of the above Formula (I), Formula (II), Formula (III), or Formula (IV) under a physiological condition, as described in "Drug Development," Volume 7, Molecular Design, pages 163 to 198, published by Hirokawa Shoten in 1990.

A compound of Formula (I), Formula (II), Formula (III), or Formula (IV) may be a hydrate, non-hydrate, solvate, or non-solvate. Further, a compound of Formula (I), Formula (II), Formula (III), or Formula (IV) may be a compound labeled or substituted with an isotope (for example, ²H, ³H, ¹¹C, 14C, ¹⁸F, ³⁵S, ¹²⁵I, and the like), and a compound labeled or substituted with an isotope can be used, for example, as a tracer (PET tracer) in positron emission tomography (PET) and can be useful in fields such as medical diagnostics. Deuterium-converted compounds in which ¹H is converted to ^{2H}(D), are also included in compounds of Formula (I), Formula (II), Formula (III), or Formula (IV).

Tautomers are also included in compounds of Formula (I), Formula (II), Formula (III), or Formula (IV).

### [Compound Production Methods]

### (General Synthetic Approach)

A compound of the above Formula (I), Formula (II), Formula (III), or Formula (IV), or an intermediate compound thereof, can be produced using the following Methods A to X27. These methods or steps may be combined with each other. Compound production methods are not limited to these.

In Synthetic Methods A to X27, PG₁ represents a protecting group for a carboxylic acid, PG₂ represents a protecting group for an amine, PG₃ represents a protecting group for a hydroxy group, R^{S1} and R^{S2} each independently represent any substituent, and other symbols have the same meanings as described above. An example of the protecting group represented by PG₁ is an alkyl protecting group. An example of the protecting group represented by PG₂ is a carbamate-based protecting group. An example of the protecting group represented by PG₃ is a silyl-based protecting group. Any substituents represented by R^{S1} and R^{S2} are any substituents selected from the [Substituent Group] described above. Hal is a halogen atom, and a halogen atom refers to a chlorine atom, a bromine atom, or an iodine atom. Ar represents an aryl group or a heteroaryl group.

### Synthetic Method A

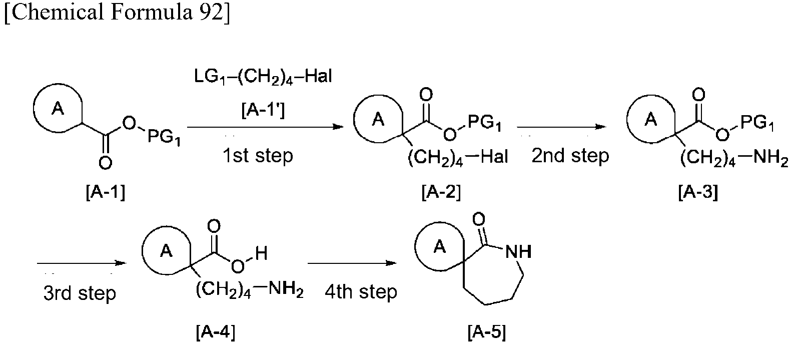

(wherein LG₁ represents a leaving group, Hal is a halogen atom, and a halogen atom refers to a chlorine atom, a bromine atom, or an iodine atom, and other symbols have the same meanings as described above).

A compound of General Formula [A-2] can be obtained by reacting a compound of General Formula [A-1] with a compound of General Formula [A-1'] in the presence of a base, in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include alkali metal amides such as lithium diisopropylamide, sodium amide, and lithium bis(trimethylsilyl)amide; inorganic bases such as sodium hydride; and alkali metal alkoxides such as sodium t-butoxide. Examples of the solvent include tetrahydrofuran, diethyl ether, toluene, and the like.

A compound of General Formula [A-3] can be obtained, for example, using one of the following two methods.

Method 1: A compound of General Formula [A-3] can be obtained by azidating a compound of General Formula [A-2] followed by reduction. The azidation reaction proceeds using an azidating agent, in an appropriate solvent, typically at a temperature ranging from room temperature to the solvent reflux temperature. The reaction time varies depending on starting materials, solvent, and reaction temperature used, but is typically from 0.5 hours to 24 hours. Examples of the azidating agent include sodium azide, diphenylphosphoryl azide, tetra-n-alkylammonium azide, trimethylsilyl azide, and the like. Examples of the solvent include N,N-dimethylformamide, N-methyl-2-pyrrolidone, and the like. The reduction reaction proceeds using a reducing agent, in an appropriate solvent, typically at a temperature ranging from room temperature to the solvent reflux temperature. The reaction time varies depending on starting materials, solvent, and reaction temperature used, but is typically from 0.5 hours to 24 hours. Examples of the reducing agent include phosphorus compounds such as triphenylphosphine. Examples of the solvent include tetrahydrofuran, 1,4-dioxane, methanol, ethanol, water, and mixtures thereof.

Method 2: A compound of General Formula [A-3] can be obtained by iminating a compound of General Formula [A-2] in the presence of a base, followed by deprotection. The imination reaction proceeds using an iminating agent, in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include organic amines such as triethylamine and N,N-diisopropylamine, and inorganic bases such as potassium carbonate. Examples of the iminating agent include bis-(tert-butylcarbonyl)amide, sodium diformylamide, and potassium phthalimide. Examples of the solvent include acetonitrile, tetrahydrofuran, N,N-dimethylformamide, N-methyl-2-pyrrolidone, and the like. The deprotection reaction proceeds using a deprotecting agent, in an appropriate solvent, typically at a temperature ranging from room temperature to the solvent reflux temperature. The reaction time varies depending on starting materials, solvent, and reaction temperature used, but is typically from 0.5 hours to 24 hours. Examples of the deprotecting agent include hydrochloric acid, sodium hydroxide, and hydrazine monohydrate. Examples of the solvent include acetonitrile, tetrahydrofuran, methanol, water, and the like.

A compound of General Formula [A-4] can be obtained by subjecting a compound of General Formula [A-3] to basic or acidic conditions. The reaction proceeds using a base or an acid, in an appropriate solvent, typically at a temperature ranging from room temperature to the solvent reflux temperature. The reaction time varies depending on starting materials, solvent, and reaction time used, but the reaction typically progresses within 1 hour to 48 hours. When a base is used, examples thereof include sodium hydroxide, potassium hydroxide, lithium hydroxide. and the like. When an acid is used, examples thereof include trifluoroacetic acid, hydrochloric acid, and the like. When a base is used, examples of the solvent include methanol, ethanol, tetrahydrofuran, and water. When an acid is used, examples of the solvent include dichloromethane, 1,2-dichloroethane, chloroform, methanol, ethyl acetate, toluene, 1,4-dioxane, water, and the like.

A compound of General Formula [A-5] can be obtained through a condensation reaction of a compound of General Formula [A-4]. The reaction proceeds using a condensing agent in the presence of an appropriate base, in an appropriate solvent, at a temperature ranging from 0 °C to the solvent reflux temperature. The reaction time varies depending on starting materials, solvent, and reaction temperature used, but is typically from 30 minutes to 24 hours. Examples of the condensing agent include 1-ethyl-3-(3-dimethylaminopropyl)carbodiimide hydrochloride (WSC·HCl), 1-[bis(dimethylamino)methylene]-1H-1,2,3-triazolo[4,5-b]pyridinium 3-oxide hexafluorophosphate (HATU), 4-(4,6-dimethoxy[1.3.5]triazin-2-yl)-4-methylmorpholinium chloride hydrate (DMT-MM), 2-chloro-1-methylpyridinium iodide, and the like. Examples of the solvent include methanol, N,N-dimethylformamide, chloroform, dichloromethane, tetrahydrofuran, and the like. Further, the reaction may be promoted by adding 1-hydroxybenzotriazole (HOBt). Examples of the base include triethylamine, N,N-diisopropylethylamine, pyridine, and the like.

### Synthetic Method A1

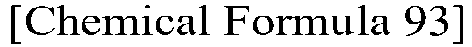

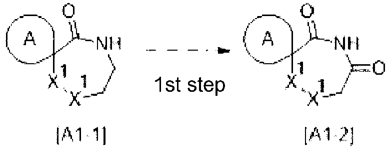

(wherein the symbols have the same meaning as described above)

A compound of General Formula [A1-2] can be obtained through an oxidation reaction of a compound of General Formula [A1-1]. The reaction proceeds using an oxidizing agent, in an appropriate solvent, typically at a temperature ranging from 0 °C to the solvent reflux temperature. The reaction time varies depending on starting materials, solvent, and reaction time used, but the reaction typically progresses within 1 hour to 48 hours. Examples of the oxidizing agent include Dess-Martin periodinane, ruthenium tetroxide, Oxone, sodium periodate, t-butyl hydroperoxide, tetra-n-propylammonium perruthenate, and the like. Examples of the solvent include acetonitrile, tetrahydrofuran, 1,4-dioxane, methylene chloride, 1,2-dichloroethane, dimethyl sulfoxide, water, and mixtures thereof.

### Synthetic Method A'

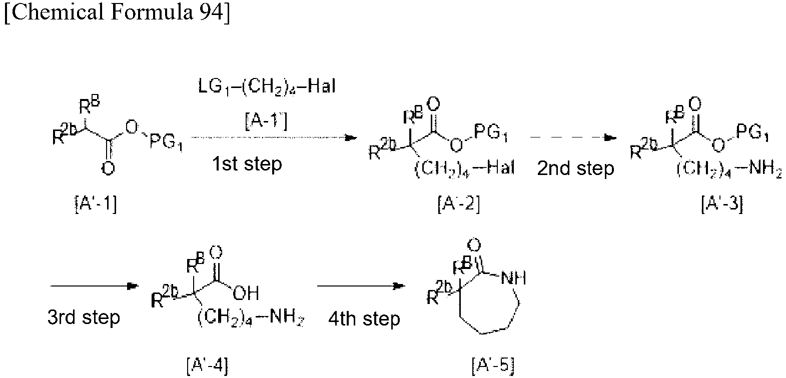

(wherein R^{B} is -Y²-R^{b}, and the other symbols have the same meanings as described above)

A compound of General Formula [A'-2] can be obtained by reacting a compound of General Formula [A'-1] with a compound of General Formula [A'-1']. The reaction temperature and time, as well as the reagents and solvents used, are the same as those in the first step of Synthetic Method A.

A compound of General Formula [A'-3] can be obtained, for example, using one of the following two methods.

Method 1: A compound of General Formula [A'-3] can be obtained by azidating a compound of General Formula [A'-2] followed by reduction. The reaction temperature and time, as well as the reagents and solvents used, are the same as those in the second step of Synthetic Method A.

Method 2: A compound of General Formula [A-3] can be obtained by iminating a compound of General Formula [A-2] in the presence of a base, followed by deprotection. The reaction temperature and time, as well as the reagents and solvents used, are the same as those in the second step of Synthetic Method A.

A compound of General Formula [A'-4] can be obtained by subjecting a compound of General Formula [A'-3] to basic or acidic conditions. The reaction temperature and time, as well as the reagents and solvents used, are the same as those in the third step of Synthetic Method A.

A compound of General Formula [A'-5] can be obtained through a condensation reaction of a compound of General Formula [A'-4]. The reaction temperature and time, as well as the reagents and solvents used, are the same as those in the fourth step of Synthetic Method A.

### Synthetic Method A'1

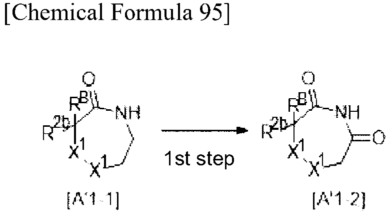

(wherein the symbols have the same meaning as described above)

A compound of General Formula [A'1-2] can be obtained through an oxidation reaction of a compound of General Formula [A'1-1]. The reaction temperature and time, as well as the reagents and solvents used, are the same as those in the first step of Synthetic Method A1.

### Synthetic Method B

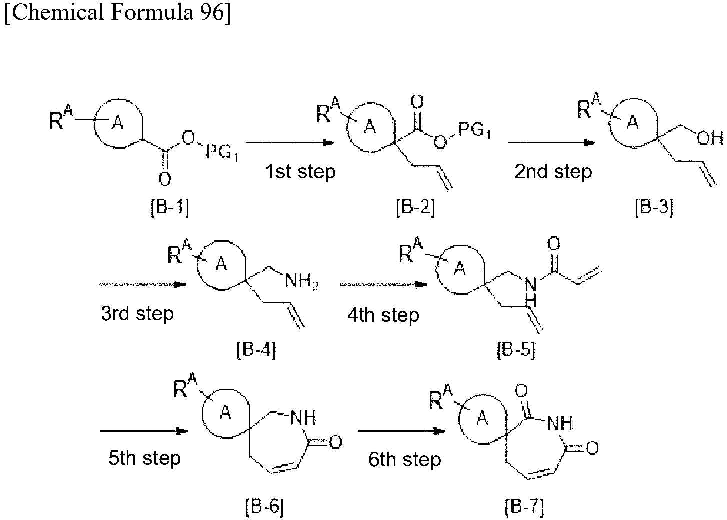

(wherein R^{A} represents -Y¹-(R^{a})ₙ, and the other symbols have the same meanings as described above)

A compound of General Formula [B-2] can be obtained by reacting a compound of General Formula [B-1] with an allylating agent in the presence of a base. The reaction proceeds in an appropriate solvent, typically at a temperature ranging from -78 °C to the solvent reflux temperature. The reaction time varies depending on starting materials and solvent used, but the reaction typically progresses within 10 minutes to 24 hours. Examples of the base include lithium diisopropylamide, sodium hydride, and the like. Examples of the allylating agent include allyl bromide, allyl chloride, and the like. Examples of the solvent include tetrahydrofuran, diethyl ether, and the like.

A compound of General Formula [B-3] can be obtained through a reduction reaction of a compound of General Formula [B-2]. The reaction proceeds using a reducing agent in an appropriate solvent, typically at a temperature ranging from -78 °C to the solvent reflux temperature. The reaction time varies depending on starting materials and solvent used, but the reaction typically progresses within 10 minutes to 24 hours. When a reducing agent is used, examples thereof include lithium aluminum hydride, DIBAL-H, sodium borohydride, and the like. Examples of the solvent include tetrahydrofuran, diethyl ether, methylene chloride, and toluene.

A compound of General Formula [B-4] can be obtained, for example, using one of the following two methods.

Method 1: A compound of General Formula [B-4] can be obtained by converting a compound of General Formula [B-3] into an azide compound using an azidating reagent in the presence of a phosphine derivative (for example, triphenylphosphine or the like) and an activating agent (for example, diisopropyl azodicarboxylate or the like), followed by a reduction reaction using a reducing agent. The conversion reaction to the azide compound proceeds in an appropriate solvent, typically at a temperature ranging from -20 °C to the solvent reflux temperature. The reaction time varies depending on starting materials, solvent, and reaction temperature used, but is typically from 0.5 hours to 24 hours. Examples of the azidating reagent include diphenylphosphoryl azide, and the like. The reduction reaction proceeds using a reducing agent, in an appropriate solvent, typically at a temperature ranging from -78 °C to the solvent reflux temperature. The reaction time varies depending on starting materials and solvent used, but the reaction typically progresses within 10 minutes to 24 hours. Examples of the reducing agent include triphenylphosphine, and the like. Examples of the solvent include 1,4-dioxane, tetrahydrofuran, water, mixtures thereof, and the like.

Method 2: A compound of General Formula [B-4] can be obtained by reacting a compound of General Formula [B-3] with a protecting reagent such as methanesulfonyl chloride, toluenesulfonyl chloride, or trimethylsilyl trifluoromethanesulfonate in the presence of a base, followed by conversion to an azide compound using an azidating reagent, and then subjecting the azide compound to a reduction reaction. The reaction with the protecting reagent proceeds in an appropriate solvent at a temperature ranging from 0 °C to the solvent reflux temperature. The reaction time varies depending on starting materials, solvent, and reaction temperature used, but is typically from 30 minutes to 24 hours. Examples of the base include triethylamine, pyridine, and the like. Examples of the solvent include methylene chloride, chloroform, and the like. Examples of the azidating reagent include sodium azide, diphenylphosphoryl azide, tetra-n-alkylammonium azide, trimethylsilyl azide, and the like. As conditions of the reaction with the azidating reagent, as well as conditions of the reduction reaction, the same conditions, reagents, and solvents as those used in Method 1 can be used.

A compound of General Formula [B-5] can be obtained through an acryloylation reaction of a compound of General Formula [B-4]. The reaction proceeds using acrylic acid chloride in the presence of a base, in an appropriate solvent, typically at a temperature ranging from 0 °C to the solvent reflux temperature. The reaction time varies depending on starting materials, solvent, and reaction temperature used, but is typically from 0.5 hours to 24 hours. Examples of the base include triethylamine, N,N-diisopropylethylamine, pyridine, and the like. Examples of the solvent include dichloromethane, 1,2-dichloroethane, chloroform, pyridine, toluene, and the like.

A compound of General Formula [B-6] can be obtained through a cyclization reaction of a compound of General Formula [B-5]. The reaction proceeds using a catalyst, in an appropriate solvent, typically at a temperature ranging from 0 °C to the solvent reflux temperature. The reaction time varies depending on starting materials, solvent, and reaction temperature used, but is typically from 0.5 hours to 24 hours. Examples of the catalyst include a second-generation Grubbs catalyst. Examples of the solvent include dichloromethane, dimethyl sulfoxide, and mixtures thereof.

A compound of General Formula [B-7] can be obtained through an oxidation reaction of a compound of General Formula [B-6]. The reaction temperature and time, as well as the reagents and solvents used, are the same as those in the first step of Synthetic Method A1.

### Synthetic Method B1

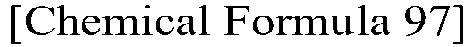

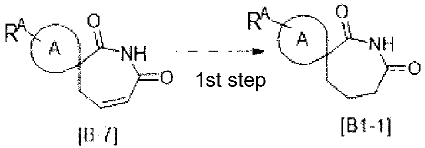

(wherein the symbols have the same meaning as described above)

A compound of General Formula [B1-1] can be obtained by subjecting a compound of General Formula [B-7] to a catalytic reduction reaction under a hydrogen atmosphere. The reaction proceeds using a catalyst, in an appropriate solvent, typically at a temperature ranging from room temperature to the solvent reflux temperature. The reaction time varies depending on starting materials, solvent, and reaction temperature used, but is typically from 0.5 hours to 24 hours. Examples of the catalyst include palladium on carbon, and the like. Examples of the solvent include methanol, ethanol, tetrahydrofuran, mixtures thereof, and the like.

### Synthetic Method B'

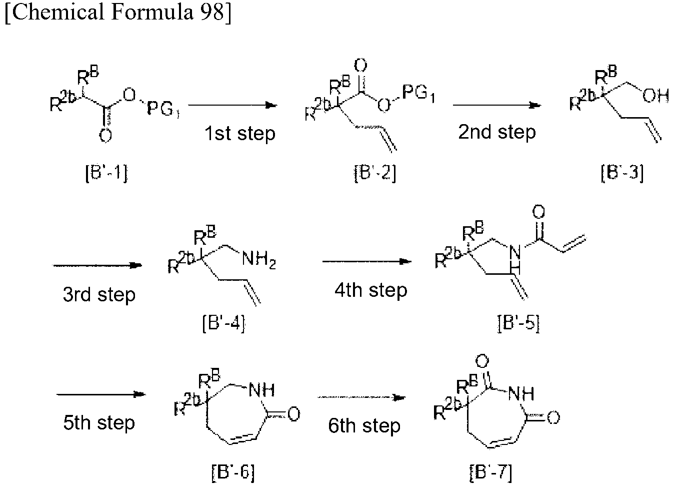

(wherein the symbols have the same meaning as described above)

A compound of General Formula [B'-2] can be obtained by reacting a compound of General Formula [B'-1] with an allylating agent in the presence of a base. The reaction temperature and time, as well as the reagents and solvents used, are the same as those in the first step of Synthetic Method B.

A compound of General Formula [B'-3] can be obtained through a reduction reaction of a compound of General Formula [B'-2]. The reaction temperature and time, as well as the reagents and solvents used, are the same as those in the first step of Synthetic Method B.

A compound of General Formula [B'-4] can be obtained from a compound of General Formula [B'-3]. The reaction temperature and time, as well as the reagents and solvents used, are the same as those in the first step of Synthetic Method B.

A compound of General Formula [B'-5] can be obtained through an acryloylation reaction of a compound of General Formula [B'-4]. The reaction temperature and time, as well as the reagents and solvents used, are the same as those in the fifth step of Synthetic Method B.

A compound of General Formula [B'-6] can be obtained through a cyclization reaction of a compound of General Formula [B'-5]. The reaction temperature and time, as well as the reagents and solvents used, are the same as those in the fifth step of Synthetic Method B.

A compound of General Formula [B'-7] can be obtained through an oxidation reaction of a compound of General Formula [B'-6]. The reaction temperature and time, as well as the reagents and solvents used, are the same as those in the first step of Synthetic Method A1.

### Synthetic Method B'1

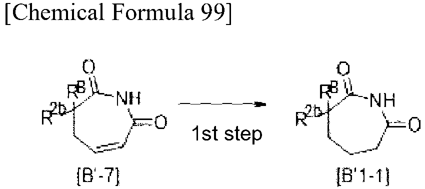

(wherein the symbols have the same meaning as described above)

A compound of General Formula [B'1-1] can be obtained by subjecting a compound of General Formula [B'-7] to a catalytic reduction reaction under a hydrogen atmosphere. The reaction temperature and time, as well as the reagents and solvents used, are the same as those in the first step of Synthetic Method B1.

### Synthetic Method C

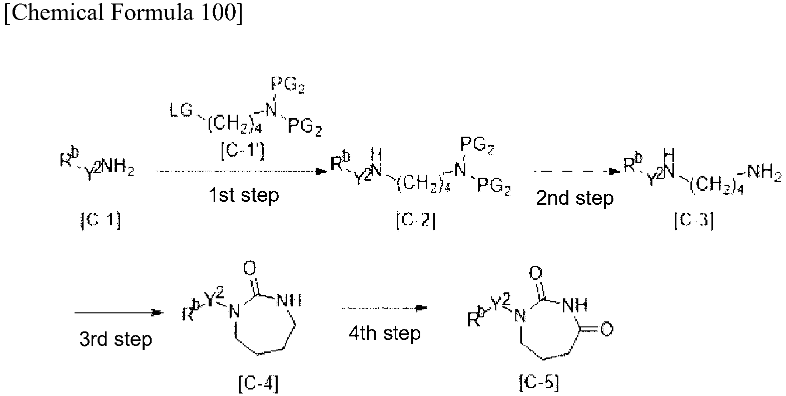

(wherein LG represents a leaving group, and the other symbols have the same meanings as described above)

A compound of General Formula [C-2] can be obtained by reacting a compound of General Formula [C-1] with a compound of General Formula [C-1'] in the presence of a base. The reaction proceeds in an appropriate solvent, typically at a temperature ranging from -78 °C to the solvent reflux temperature. The reaction time varies depending on starting materials and solvent used, but the reaction typically progresses within 10 minutes to 24 hours. Examples of the base include: organic amines such as triethylamine, N,N-diisopropylethylamine, and pyridine; inorganic bases such as sodium hydride, potassium carbonate, and sodium hydroxide; and the like. Examples of the solvent include N,N-dimethylformamide, tetrahydrofuran, acetonitrile, acetone, and the like.

A compound of General Formula [C-3] can be obtained by deprotection of a compound of General Formula [C-2]. The deprotection can be carried out using conventional methods such as reduction, hydrolysis, or acid treatment, depending on the type of protecting group to be removed.

In the case of reduction, the reaction proceeds in an appropriate solvent under a hydrogen atmosphere using a catalyst, typically at a temperature ranging from -78 °C to the solvent reflux temperature. The reaction time varies depending on starting materials and solvent used, but the reaction typically progresses within 10 minutes to 24 hours. Examples of the catalyst include palladium on carbon, platinum, and the like. Examples of the solvent include methanol, ethanol, and the like.

In the case of hydrolysis, the reaction proceeds in an appropriate solvent using a base, typically at a temperature ranging from -78 °C to the solvent reflux temperature. The reaction time varies depending on starting materials and solvent used, but the reaction typically progresses within 10 minutes to 24 hours. Examples of the base include sodium hydroxide, potassium hydroxide, lithium hydroxide, sodium methoxide, sodium ethoxide, hydrazine monohydrate, and the like. Examples of the solvent include tetrahydrofuran, dioxane, methanol, ethanol, water, and the like.

In the case of acid treatment, the reaction can be carried out by treating with an acid in an appropriate solvent. The reaction typically proceeds at a temperature ranging from -78 °C to the solvent reflux temperature. The reaction time varies depending on starting materials and solvent used, but the reaction typically progresses within 10 minutes to 24 hours. Examples of the acid include hydrochloric acid, p-toluenesulfonic acid, methanesulfonic acid, trifluoroacetic acid, and the like. Examples of the solvent include methylene chloride, chloroform, methanol, ethanol, and the like.

A compound of General Formula [C-4] can be obtained by carbonylating a compound of General Formula [C-3] in the presence of a base. The reaction proceeds using a carbonylating agent, in an appropriate solvent, typically at a temperature ranging from -78 °C to the solvent reflux temperature. The reaction time varies depending on starting materials and solvent used, but the reaction typically progresses within 10 minutes to 24 hours. Examples of the base include triethylamine, N,N-diisopropylethylamine, and the like. Examples of the carbonylating agent include 1,1'-carbonyldiimidazole, triphosgene, and the like. Examples of the solvent include tetrahydrofuran, diethyl ether, methylene chloride, and toluene.

A compound of General Formula [C-5] can be obtained through an oxidation reaction of a compound of General Formula [C-4]. The reaction temperature and time, as well as the reagents and solvents used, are the same as those in the first step of Synthetic Method A1.

### Synthetic Method C'

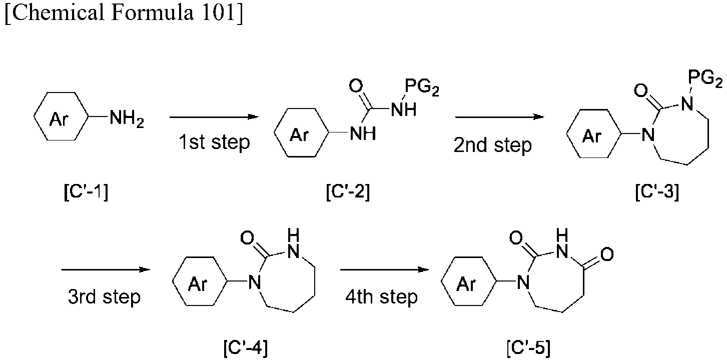

(wherein Ar represents an aryl group or a heteroaryl group, and the other symbols have the same meaning as described above).

A compound of General Formula [C'-2] can be obtained, for example, using one of the following two methods.

Method 1: A compound of General Formula [C'-2] can be obtained by converting a compound of General Formula [C'-1] into a urethane in the presence of a base, followed by reaction with an amine. The urethane formation proceeds using a urethanating agent, in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include pyridine, and the like. Examples of the urethanating agent include (4-methoxyphenyl) chloroformate, and the like. Examples of the solvent include acetonitrile, N,N-dimethylformamide, N-methyl-2-pyrrolidone, and the like.

Method 2: A compound of General Formula [C'-2] can be obtained by reacting a compound of General Formula [C'-1] with an isocyanate in the presence or absence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include triethylamine, pyridine, and the like. Examples of the isocyanate include trimethylsilyl isocyanate, and the like. Examples of the solvent include acetonitrile, N,N-dimethylformamide, N-methyl-2-pyrrolidone, and the like.

A compound of General Formula [C'-3] can be obtained by deprotection of a compound of General Formula [C'-2]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [C'-4] can be obtained by reacting a compound of General Formula [C'-3] with 1,4-dibromobutane in the presence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include potassium carbonate, sodium t-butoxide, and the like. Examples of the solvent include tetrahydrofuran, N,N-dimethylformamide, and the like.

A compound of General Formula [C'-5] can be obtained through an oxidation reaction of a compound of General Formula [C'-4]. The reaction temperature and time, as well as the reagents and solvents used, are the same as those in the first step of Synthetic Method A1.

### Synthetic Method C"

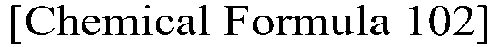

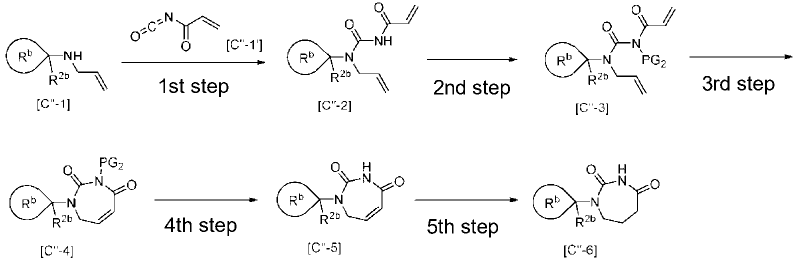

(wherein the symbols have the same meaning as described above)

A compound of General Formula [C" -2] can be obtained through an acylation reaction of a compound of General Formula [C''-1]. It can be obtained by reacting with prop-2-enoyl isocyanate. The reaction proceeds in an appropriate solvent, at a temperature ranging from 0 °C to the solvent reflux temperature. The reaction time varies depending on starting materials, solvent, and reaction temperature used, but is typically from 30 minutes to 24 hours. Examples of the solvent include diethyl ether, tetrahydrofuran, and the like.

A compound of General Formula [C''-3] can be obtained by protecting a compound of General Formula [C''-2]. It can be obtained by reacting with a protecting reagent such as 2-(chloromethoxy)ethyl-trimethylsilane or 4-methoxybenzyl chloride in the presence of a base. The reaction with the protecting reagent proceeds in an appropriate solvent at a temperature ranging from 0 °C to the solvent reflux temperature. The reaction time varies depending on starting materials, solvent, and reaction temperature used, but is typically from 30 minutes to 24 hours. Examples of the base include triethylamine, N,N-diisopropylethylamine, and the like. Examples of the solvent include methylene chloride, chloroform, and the like.

A compound of General Formula [C" -4] can be obtained through a cyclization reaction of a compound of General Formula [C''-3]. The reaction proceeds using a catalyst, in an appropriate solvent, typically at a temperature ranging from 0 °C to the solvent reflux temperature. The reaction time varies depending on starting materials, solvent, and reaction temperature used, but is typically from 0.5 hours to 24 hours. Examples of the catalyst include a second-generation Grubbs catalyst and a second-generation Hoveyda-Grubbs catalyst. Examples of the solvent include dichloromethane, dichloroethane, and mixtures thereof.

A compound of General Formula [C''-5] can be obtained by deprotection of a compound of General Formula [C''-4]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [C''-6] can be obtained by subjecting a compound of General Formula [C''-5] to a catalytic reduction reaction under a hydrogen atmosphere. The reaction temperature and time, as well as the reagents and solvents used, are the same as those in the first step of Synthetic Method B1.

### Synthetic Method C"A

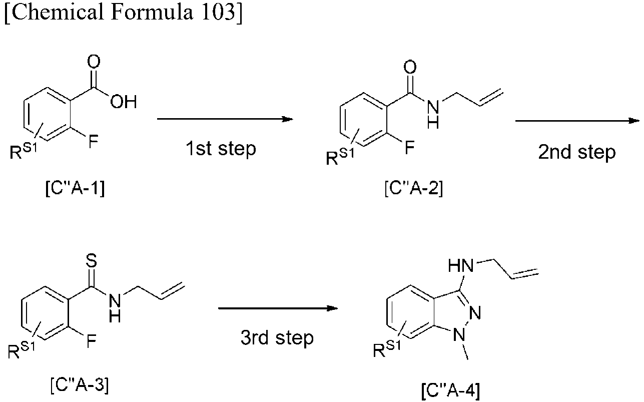

(wherein the symbols have the same meaning as described above)

A compound of General Formula [C"A-2] can be obtained through a condensation reaction of a compound of General Formula [C"A-1] with allylamine hydrochloride. The reaction temperature and time, as well as the reagents and solvents used, are the same as those in the fourth step of Synthetic Method A.

A compound of General Formula [C"A-3] can be obtained through a thioamidation reaction of a compound of General Formula [C''A-2]. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the thioamidation reagent include Lawesson's reagent, and the like. Examples of the solvent include toluene, tetrahydrofuran, and the like.

A compound of General Formula [C''A-4] can be obtained through a condensation cyclization reaction of a compound of General Formula [C"A-3] with methylhydrazine. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the solvent include ethanol, and the like.

### Synthetic Method C''B

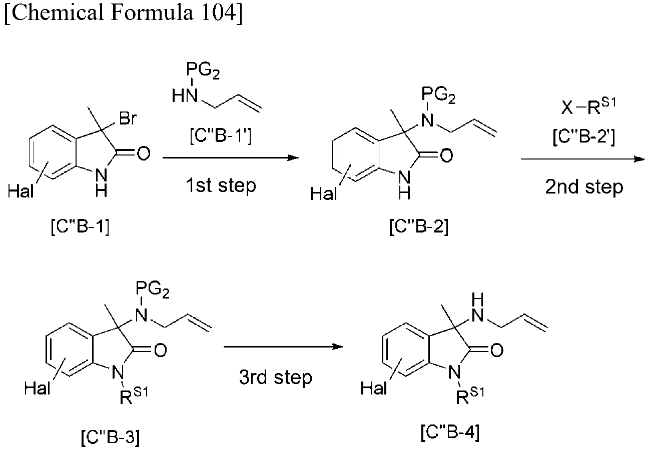

(wherein X is a leaving group, and the other symbols have the same meanings as described above)

A compound of General Formula [C"B-2] can be obtained by reacting a compound of General Formula [C''B-1] with a compound of General Formula [C"B-1']. The reaction proceeds in an appropriate solvent, at a temperature ranging from 0 °C to the solvent reflux temperature. The reaction time varies depending on starting materials, solvent, and reaction temperature used, but is typically from 30 minutes to 24 hours. Examples of the base include triethylamine, N,N-diisopropylethylamine, potassium carbonate, cesium carbonate, and the like. Examples of the solvent include N,N-dimethylformamide, dichloromethane, and the like.

A compound of General Formula [C''B-3] can be derived from a compound of General Formula [C"B-2] and a compound of General Formula [C''B-2'] according to a commonly known (for example, ChemMedChem (2022), 17 (4), e202100512).

A compound of General Formula [C"B-4] can be obtained by deprotection of a compound of General Formula [C''B-3]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

### Synthetic Method C‴

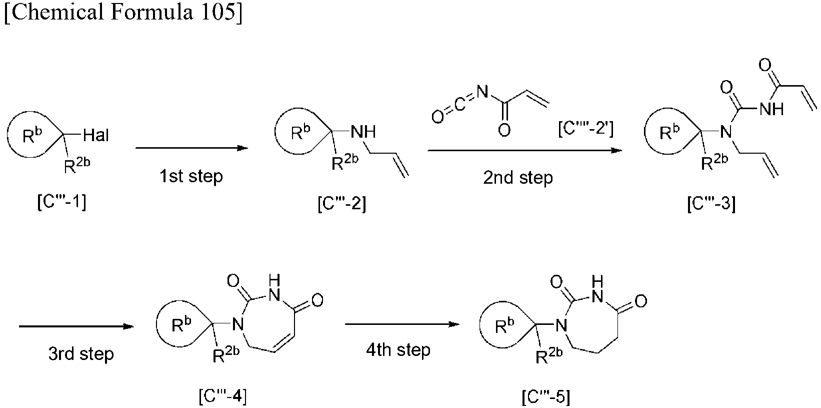

(wherein the symbols have the same meaning as described above)

A compound of General Formula [C"'-2] can be obtained by reacting a compound of General Formula [C‴-1] with allylamine hydrochloride in the presence of a base. The reaction proceeds in an appropriate solvent, at a temperature ranging from 0 °C to the solvent reflux temperature. The reaction time varies depending on starting materials, solvent, and reaction temperature used, but is typically from 30 minutes to 24 hours. Examples of the base include triethylamine, N,N-diisopropylethylamine, potassium carbonate, cesium carbonate, and the like. Examples of the solvent include N,N-dimethylformamide, dichloromethane, and the like.

A compound of General Formula [C‴-3] can be obtained through an acylation reaction of a compound of General Formula [C‴-2]. The reaction temperature and time, as well as the reagents and solvents used, are the same as those in the first step of Synthetic Method C".

A compound of General Formula [C‴-4] can be obtained through a cyclization reaction of a compound of General Formula [C‴-3]. The reaction temperature and time, as well as the reagents and solvents used, are the same as those in the fourth step of Synthetic Method A.

A compound of General Formula [C‴-5] can be obtained by subjecting a compound of General Formula [C‴-4] to a catalytic reduction reaction under a hydrogen atmosphere. The reaction temperature and time, as well as the reagents and solvents used, are the same as those in the first step of Synthetic Method B1.

### Synthetic Method D

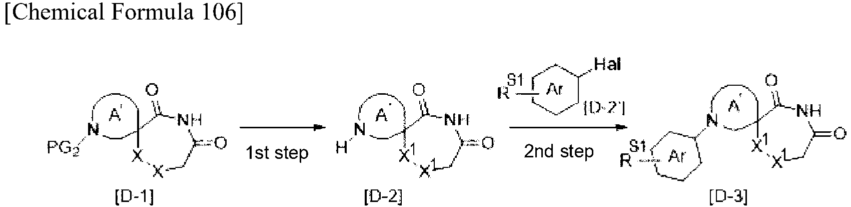

(wherein R^{S1} represents any substituent, and the other symbols have the same meaning as described above).

A compound of General Formula [D-2] can be obtained by deprotection of a compound of General Formula [D-1]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [D-3] can be obtained by reacting a compound of General Formula [D-2] with a compound of General Formula [D-2'] in the presence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include potassium carbonate, sodium t-butoxide, diisopropylethylamine, and the like. Examples of the solvent include tetrahydrofuran, N,N-dimethylformamide, and the like.

### Synthetic Method D1

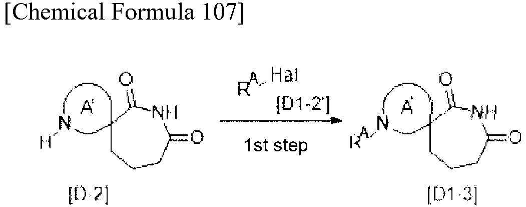

(wherein the symbols have the same meaning as described above)

A compound of General Formula [D1-3] can be obtained by reacting a compound of General Formula [D-2] with a compound of General Formula [D1-2'] in the presence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include potassium carbonate, sodium t-butoxide, diisopropylethylamine, and the like. Examples of the solvent include tetrahydrofuran, N,N-dimethylformamide, and the like.

### Synthetic Method D2

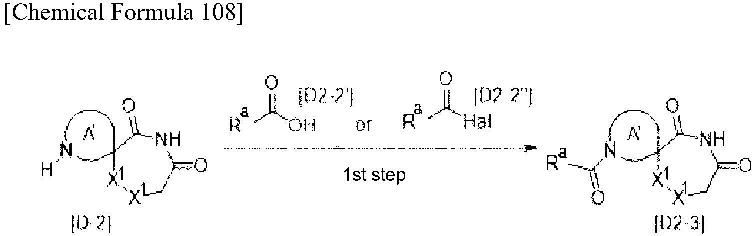

(wherein the symbols have the same meaning as described above)

A compound of General Formula [D2-3] can be obtained, for example, using one of the following two methods.

Method 1: A compound of General Formula [D2-3] can be obtained by reacting a compound of General Formula [D-2] with a compound of General Formula [D2-2'] using a condensing agent. The reaction proceeds in the presence of an appropriate base, in an appropriate solvent, at a temperature ranging from 0 °C to the solvent reflux temperature. The reaction time varies depending on starting materials, solvent, and reaction temperature used, but is typically from 30 minutes to 24 hours. Examples of the condensing agent include 1-ethyl-3-(3-dimethylaminopropyl)carbodiimide hydrochloride (WSC·HCl), 1-[bis(dimethylamino)methylene]-1H-1,2,3-triazolo[4,5-b]pyridinium 3-oxide hexafluorophosphate (HATU), 4-(4,6-dimethoxy[1.3.5]triazin-2-yl)-4-methylmorpholinium chloride hydrate (DMT-MM), 2-chloro-1-methylpyridinium iodide, and the like. Examples of the solvent include methanol, N,N-dimethylformamide, chloroform, dichloromethane, tetrahydrofuran, and the like. Further, the reaction may be promoted by adding 1-hydroxybenzotriazole (HOBt). Examples of the base include triethylamine, N,N-diisopropylethylamine, pyridine, and the like.

Methoc 2: A compound of General Formula [D2-3] can be obtained by reacting a compound of General Formula [D-2] with a compound of General Formula [D2-2''] in the presence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include triethylamine, diisopropylethylamine, and the like. Examples of the solvent include acetonitrile, N,N-dimethylformamide, and the like.

### Synthetic Method D'

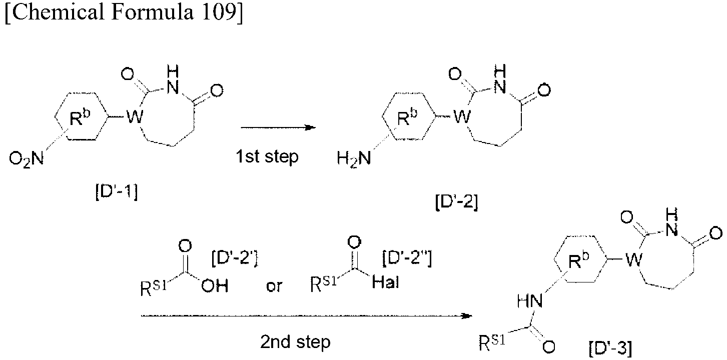

(wherein the symbols have the same meaning as described above)

A compound of General Formula [D'-2] can be obtained by subjecting a compound of General Formula [D'-1] to a reduction reaction under a hydrogen atmosphere in the presence of a catalyst. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the catalyst include palladium on carbon, and the like. Examples of the solvent include tetrahydrofuran, methanol, ethanol, water, mixtures thereof, and the like.

A compound of General Formula [D'-3] can be obtained, for example, using one of the following two methods.

Method 1: A compound of General Formula [D'-3] can be obtained through a condensation reaction of a compound of General Formula [D'-2] with a compound of General Formula [D'-2']. The reaction temperature and time, as well as the reagents and solvents used, are the same as those in the fourth step of Synthetic Method A.

Method 2: A compound of General Formula [D'-3] can be obtained by reacting a compound of General Formula [D'-2] with a compound of General Formula [D'-2"] in the presence of a base. The reaction temperature and time, as well as the reagents and solvents used, are the same as those in the first step of Synthetic Method D2.

### Synthetic Method D''

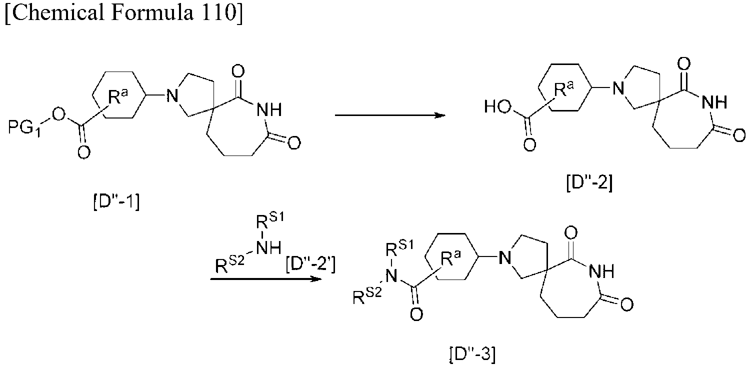

(wherein the symbols have the same meaning as described above)

A compound of General Formula [D"-2] can be obtained by subjecting a compound of General Formula [D''-1] to basic or acidic conditions. The reaction temperature and time, as well as the reagents and solvents used, are the same as those in the third step of Synthetic Method A.

A compound of General Formula [D"-3] can be obtained through a condensation reaction of a compound of General Formula [D''-2] with a compound of General Formula [D"-2']. The reaction temperature and time, as well as the reagents and solvents used, are the same as those in the fourth step of Synthetic Method A.

### Synthetic Method E

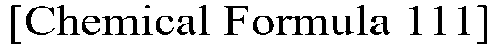

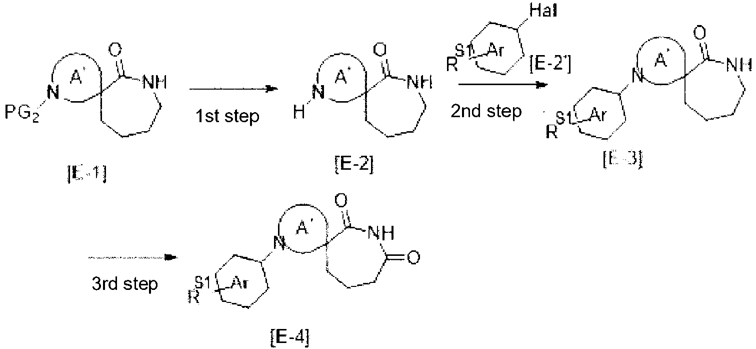

(wherein the symbols have the same meaning as described above)

A compound of General Formula [E-2] can be obtained by deprotection of a compound of General Formula [E-1]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [E-3] can be obtained, for example, using one of the following two methods.

Method 1: A compound of General Formula [E-3] can be obtained by reacting a compound of General Formula [E-2] with a compound of General Formula [E-2'] in the presence of a base. The reaction temperature and time, as well as the reagents and solvents used, are the same as those in the second step of Synthetic Method D.

Method 2: A compound of General Formula [E-3] can be obtained by reacting a compound of General Formula [E-2] with a compound of General Formula [E-2'] in the presence of a palladium catalyst, a phosphine ligand, and a base. The reaction proceeds in an appropriate solvent, typically at a temperature ranging from room temperature to the solvent reflux temperature. The reaction may be accelerated under microwave irradiation. The reaction time varies depending on starting materials, solvent, and reaction temperature used, but is typically from 0.5 hours to 24 hours. Examples of the catalyst include tris(dibenzylideneacetone)dipalladium and the like, and examples of the phosphine ligand include 2-dicyclohexylphosphino-2'-(N,N-dimethylamino)biphenyl and the like. Examples of the base include potassium phosphate and the like. Examples of the solvent include 1,4-dioxane, N,N-dimethylformamide, toluene, and the like.

### Synthetic Method E'

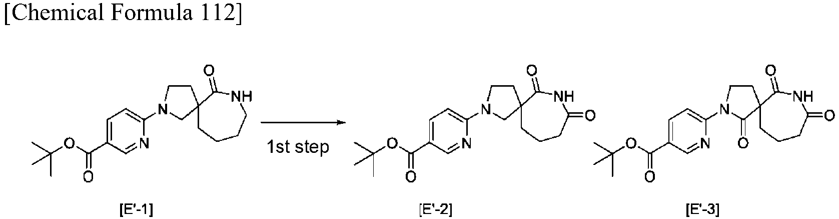

A compound of General Formula [E'-2] and a compound of General Formula [E'-3] can be obtained through an oxidation reaction of a compound of General Formula [E'-1]. The reaction temperature and time, as well as the reagents and solvents used, are the same as those in the first step of Synthetic Method A1.

### Synthetic Method E1

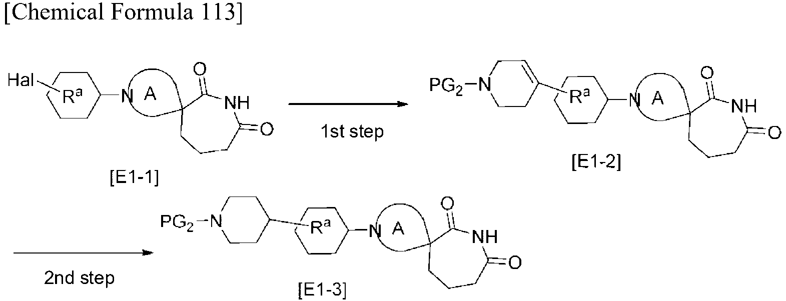

(wherein the symbols have the same meaning as described above)

A compound of General Formula [E1-2] can be derived from a compound of General Formula [E1-1] according to a commonly known method (for example, Eur. J. Med. Chem., (2019), 177, 316).

A compound of General Formula [E1-3] can be obtained by subjecting a compound of General Formula [E1-2] to a catalytic reduction reaction under a hydrogen atmosphere. The reaction temperature and time, as well as the reagents and solvents used, are the same as those in the first step of Synthetic Method B1.

### Synthetic Method E''

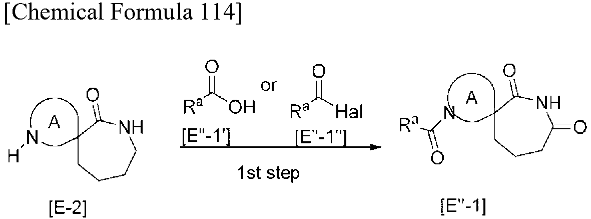

(wherein the symbols have the same meaning as described above)

A compound of General Formula [E"-1] can be obtained, for example, using one of the following two methods.

Method 1: A compound of General Formula [E"-1] can be obtained through a condensation reaction of a compound of General Formula [E-2] with a compound of General Formula [E"-1']. The reaction temperature and time, as well as the reagents and solvents used, are the same as those in Method 1 of the first step of Synthetic Method D2.

Method 2: A compound of General Formula [E"-1] can be obtained through a reaction of a compound of General Formula [E-2] with a compound of General Formula [E"-1"] in the presence of a base. The reaction temperature and time, as well as the reagents and solvents used, are the same as those in Method 2 of the first step of Synthetic Method D2.

### Synthetic Method F

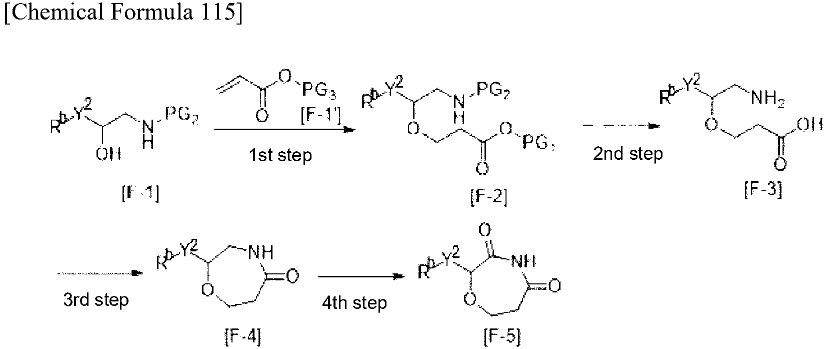

(wherein the symbols have the same meaning as described above)

A compound of General Formula [F-2] can be obtained by reacting a compound of General Formula [F-1] with a compound of General Formula [F-1'] in the presence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include cesium carbonate and the like. Examples of the solvent include t-butyl alcohol, tetrahydrofuran, and the like.

A compound of General Formula [F-3] can be obtained by deprotection of a compound of General Formula [F-2]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [F-4] can be obtained through a condensation reaction of a compound of General Formula [F-3]. The reaction temperature and time, as well as the reagents and solvents used, are the same as those in the fourth step of Synthetic Method A.

A compound of General Formula [F-5] can be obtained through an oxidation reaction of a compound of General Formula [F-4]. The reaction temperature and time, as well as the reagents and solvents used, are the same as those in the first step of Synthetic Method A1.

### Synthetic Method F'

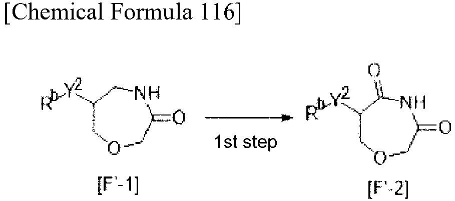

(wherein the symbols have the same meaning as described above)

A compound of General Formula [F'-2] can be obtained through an oxidation reaction of a compound of General Formula [F'-1]. The reaction temperature and time, as well as the reagents and solvents used, are the same as those in the first step of Synthetic Method A1.

### Synthetic Method G

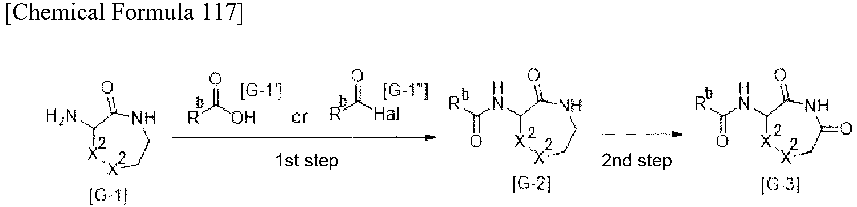

(wherein the symbols have the same meaning as described above)

A compound of General Formula [G-2] can be obtained, for example, using one of the following two methods.

Method 1: A compound of General Formula [G-2] can be obtained through a condensation reaction of a compound of General Formula [G-1] with a compound of General Formula [G-1']. The reaction temperature and time, as well as the reagents and solvents used, are the same as those in Method 1 of the first step of Synthetic Method D2.

Method 2: A compound of General Formula [G-2] can be obtained through a reaction of a compound of General Formula [G-1] with a compound of General Formula [G-1"] in the presence of a base. The reaction temperature and time, as well as the reagents and solvents used, are the same as those in Method 2 of the first step of Synthetic Method D2.

A compound of General Formula [G-3] can be obtained through an oxidation reaction of a compound of General Formula [G-2]. The reaction temperature and time, as well as the reagents and solvents used, are the same as those in the first step of Synthetic Method A1.

### Synthetic Method H

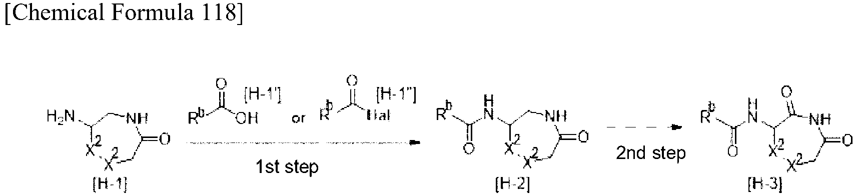

(wherein the symbols have the same meaning as described above)

A compound of General Formula [H-2] can be obtained, for example, using one of the following two methods.

Method 1: A compound of General Formula [H-2] can be obtained through a condensation reaction of a compound of General Formula [H-1] with a compound of General Formula [H-1']. The reaction temperature and time, as well as the reagents and solvents used, are the same as those in Method 1 of the first step of Synthetic Method D2.

Method 2: A compound of General Formula [H-2] can be obtained through a reaction of a compound of General Formula [H-1] with a compound of General Formula [H-1"] in the presence of a base. The reaction temperature and time, as well as the reagents and solvents used, are the same as those in Method 2 of the first step of Synthetic Method D2.

A compound of General Formula [H-3] can be obtained through an oxidation reaction of a compound of General Formula [H-2]. The reaction temperature and time, as well as the reagents and solvents used, are the same as those in the first step of Synthetic Method A1.

### Synthetic Method H'

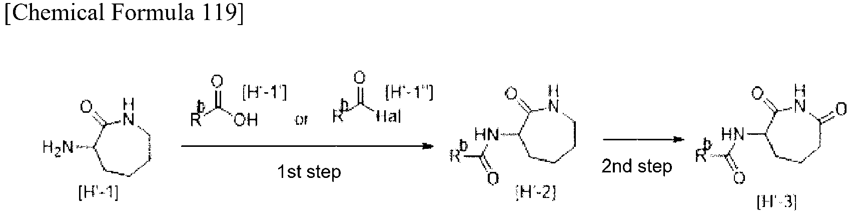

(wherein the symbols have the same meaning as described above)

A compound of General Formula [H'-2] can be obtained, for example, using one of the following two methods.

Method 1: A compound of General Formula [H'-2] can be obtained through a condensation reaction of a compound of General Formula [H'-1] with a compound of General Formula [H'-1']. The reaction temperature and time, as well as the reagents and solvents used, are the same as those in Method 1 of the first step of Synthetic Method D2.

Method 2: A compound of General Formula [H'-2] can be obtained through a reaction of a compound of General Formula [H'-1] with a compound of General Formula [H'-1"] in the presence of a base. The reaction temperature and time, as well as the reagents and solvents used, are the same as those in Method 2 of the first step of Synthetic Method D2.

A compound of General Formula [H'-3] can be obtained through an oxidation reaction of a compound of General Formula [H'-2]. The reaction temperature and time, as well as the reagents and solvents used, are the same as those in the first step of Synthetic Method A1.

### Synthetic Method H"

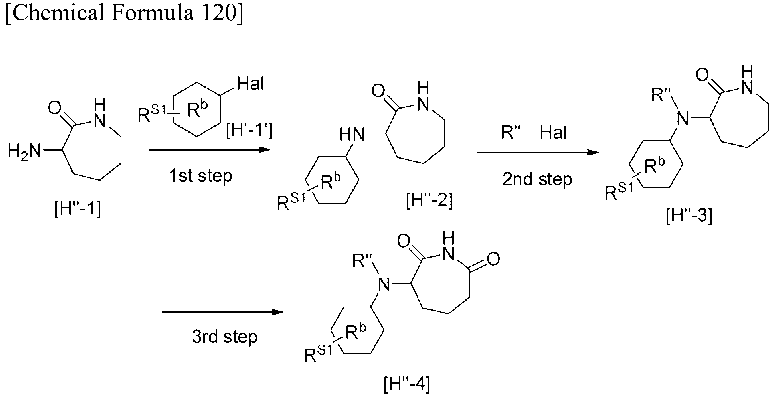

(wherein the symbols have the same meaning as described above)

A compound of General Formula [H"-2] can be obtained through a reaction of a compound of General Formula [H"-1] with a compound of General Formula [H"-1'] in the presence of a base. The reaction temperature and time, as well as the reagents and solvents used, are the same as those in the second step of Synthetic Method D.

A compound of General Formula [H"-3] can be obtained through a reaction of a compound of General Formula [H"-2] with a compound of General Formula [H"-2'] in the presence of a base. The reaction temperature and time, as well as the reagents and solvents used, are the same as those in the first step of Synthetic Method D1.

A compound of General Formula [H"-4] can be obtained through an oxidation reaction of a compound of General Formula [H"-3]. The reaction temperature and time, as well as the reagents and solvents used, are the same as those in the first step of Synthetic Method A1.

### Synthetic Method H‴

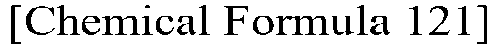

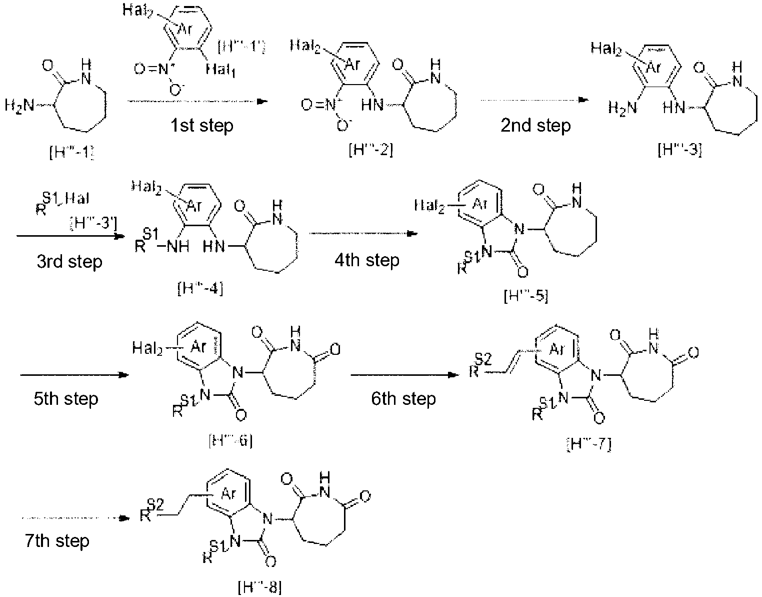

(wherein Hal₁ represents fluorine and chlorine, Hal₂ represents chlorine, bromine, and iodine, and the other symbols have the same meanings as described above)

A compound of General Formula [H‴-2] can be obtained by reacting a compound of General Formula [H‴-1] with a compound of General Formula [H‴-1'] in the presence of a base. The reaction temperature and time, as well as the reagents and solvents used, are the same as those in the second step of Synthetic Method D.

A compound of General Formula [H‴-3] can be obtained by subjecting a compound of General Formula [H"'-2] to a reduction reaction. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the reducing agent include sodium dithionite and the like. Examples of the base include sodium carbonate and the like. Examples of the solvent include tetrahydrofuran, methanol, ethanol, water, mixtures thereof, and the like. A compound of General Formula [H"'-4] can be obtained by reacting a compound of General Formula [H‴-3] with a compound of General Formula [H‴-3'] in the presence of a base. The reaction temperature and time, as well as the reagents and solvents used, are the same as those in the first step of Synthetic Method D1.

A compound of General Formula [H"'-5] can be obtained by carbonylating a compound of General Formula [H‴-4] in the presence of a base. The reaction temperature and time, as well as the reagents and solvents used, are the same as those in the third step of Synthetic Method C. A compound of General Formula [H‴-6] can be obtained through an oxidation reaction of a compound of General Formula [H"'-5]. The reaction temperature and time, as well as the reagents and solvents used, are the same as those in the first step of Synthetic Method A1.

A compound of General Formula [H‴-7] can be obtained by alkenylating a compound of General Formula [H"'-6] in the presence of a palladium catalyst, a phosphine ligand, and a base. The reaction proceeds in an appropriate solvent, typically at a temperature ranging from room temperature to the solvent reflux temperature. The reaction may be accelerated under microwave irradiation. The reaction time varies depending on starting materials, solvent, and reaction temperature used, but is typically from 0.5 hours to 24 hours. Examples of the catalyst include palladium acetate and the like, and examples of the phosphine ligand include tri(o-tolyl)phosphine and the like. Examples of the base include sodium tert-butoxide and the like. Examples of the solvent include N,N-dimethylformamide, 1,4-dioxane, toluene, and the like.

A compound of General Formula [H‴-8] can be obtained by subjecting a compound of General Formula [H‴-7] to a reduction reaction under a hydrogen atmosphere in the presence of a catalyst. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the catalyst include palladium on carbon, and the like. Examples of the solvent include tetrahydrofuran, methanol, ethanol, water, mixtures thereof, and the like.

### Synthetic Method I

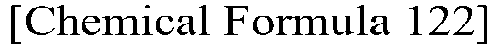

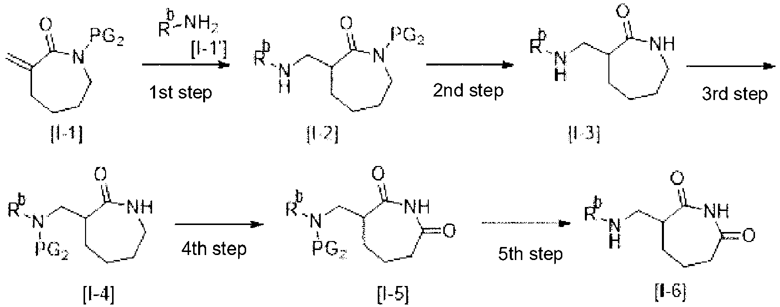

### (wherein the symbols have the same meaning as described above)

A compound of General Formula [I-2] can be obtained by reacting a compound of General Formula [I-1] with a compound of General Formula [I-2] in the presence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include triethylamine, diisopropylethylamine, and the like. Examples of the solvent include acetonitrile, tetrahydrofuran, N,N-dimethylformamide, and the like.

A compound of General Formula [I-3] can be obtained by deprotection of a compound of General Formula [I-2]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [I-4] can be obtained by introducing a protecting group to a functional group of a compound of General Formula [I-3] with reference to a known method (for example, PROTECTIVE GROUPS IN ORGANIC SYNTHESIS by Theodora W. Greene and Peter G. M. Wuts, or the like).

A compound of General Formula [I-5] can be obtained through an oxidation reaction of a compound of General Formula [I-4]. The reaction temperature and time, as well as the reagents and solvents used, are the same as those in the first step of Synthetic Method A1.

A compound of General Formula [I-6] can be obtained by deprotection of a compound of General Formula [I-5]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

### Synthetic Method I'

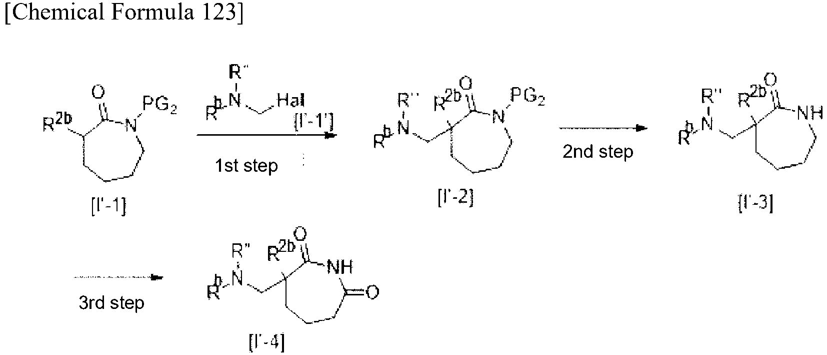

(wherein the symbols have the same meaning as described above)

A compound of General Formula [I'-2] can be obtained through a reaction of a compound of General Formula [I'-1] with a compound of General Formula [I'-1'] in the presence of a base. The reaction temperature and time, as well as the reagents and solvents used, are the same as those in the first step of Synthetic Method A.

A compound of General Formula [I'-3] can be obtained by deprotection of a compound of General Formula [I'-2]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [I'-4] can be obtained through an oxidation reaction of a compound of General Formula [I'-3]. The reaction temperature and time, as well as the reagents and solvents used, are the same as those in the first step of Synthetic Method A1.

### Synthetic Method I"

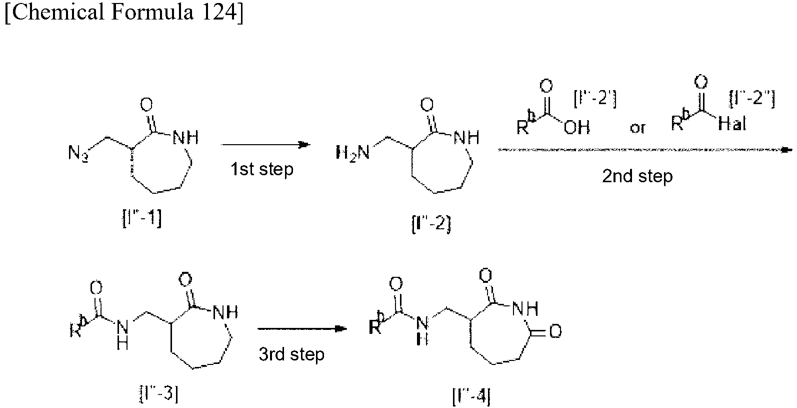

(wherein the symbols have the same meaning as described above)

A compound of General Formula [I"-2] can be obtained through a reduction reaction of a compound of General Formula [I"-1]. The reduction reaction proceeds using a reducing agent, in an appropriate solvent, typically at a temperature ranging from room temperature to the solvent reflux temperature. The reaction time varies depending on starting materials, solvent, and reaction temperature used, but is typically from 0.5 hours to 24 hours. Examples of the reducing agent include phosphorus compounds such as triphenylphosphine. Examples of the solvent include tetrahydrofuran, 1,4-dioxane, methanol, ethanol, water, and mixtures thereof.

A compound of General Formula [I''-3] can be obtained, for example, using one of the following two methods.

Method 1: A compound of General Formula [I"-3] can be obtained through a condensation reaction of a compound of General Formula [I"-2] with a compound of General Formula [I"-2']. The reaction temperature and time, as well as the reagents and solvents used, are the same as those in Method 1 of the first step of Synthetic Method D2.

Method 2: A compound of General Formula [I''-3] can be obtained through a reaction of a compound of General Formula [I"-2] with a compound of General Formula [I"-2"] in the presence of a base. The reaction temperature and time, as well as the reagents and solvents used, are the same as those in Method 2 of the first step of Synthetic Method D2.

A compound of General Formula [I"-4] can be obtained through an oxidation reaction of a compound of General Formula [I"-3]. The reaction temperature and time, as well as the reagents and solvents used, are the same as those in the first step of Synthetic Method A1.

### Synthetic Method J

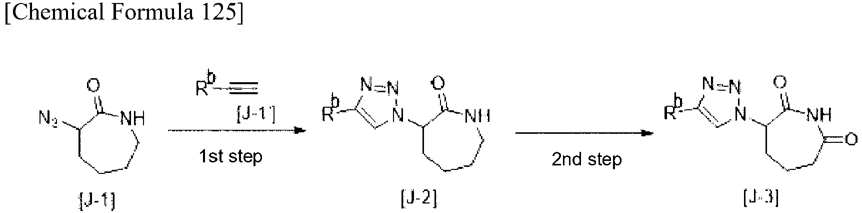

(wherein the symbols have the same meaning as described above)

A compound of General Formula [J-2] can be obtained by reacting a compound of General Formula [J-1] with a compound of General Formula [J-1'] in the presence of an additive. The reaction proceeds using a catalyst, in an appropriate solvent, typically at a temperature ranging from room temperature to the solvent reflux temperature. The reaction time varies depending on starting materials, solvent, and reaction temperature used, but is typically from 0.5 hours to 24 hours. Examples of the catalyst include copper(II) sulfate pentahydrate and the like. Examples of the additive include sodium ascorbate and the like. Examples of the solvent include tetrahydrofuran and the like.

A compound of General Formula [J-3] can be obtained through an oxidation reaction of a compound of General Formula [J-2]. The reaction temperature and time, as well as the reagents and solvents used, are the same as those in the first step of Synthetic Method A1.

### Synthetic Method K

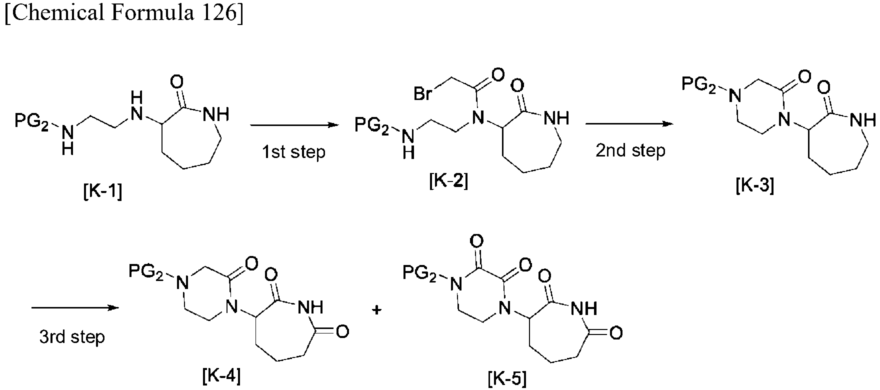

(wherein the symbols have the same meaning as described above)

A compound of General Formula [K-2] can be obtained through a bromoacetylation reaction of a compound of General Formula [K-1]. The reaction proceeds using bromoacetyl bromide in the presence of a base, in an appropriate solvent, typically at a temperature ranging from 0 °C to the solvent reflux temperature. The reaction time varies depending on starting materials, solvent, and reaction temperature used, but is typically from 0.5 hours to 24 hours. Examples of the base include triethylamine, N,N-diisopropylethylamine, pyridine, and the like. Examples of the solvent include dichloromethane, 1,2-dichloroethane, chloroform, pyridine, toluene, and the like.

A compound of General Formula [K-3] can be obtained by reacting a compound of General Formula [K-2] in the presence of a base. The reaction temperature and time, as well as the reagents and solvents used, are the same as those in the first step of Synthetic Method D1.

A compound of General Formula [K-4] and a compound of General Formula [K-5] can be obtained through an oxidation reaction of a compound of General Formula [K-3]. The reaction temperature and time, as well as the reagents and solvents used, are the same as those in the first step of Synthetic Method A1.

### Synthetic Method K'

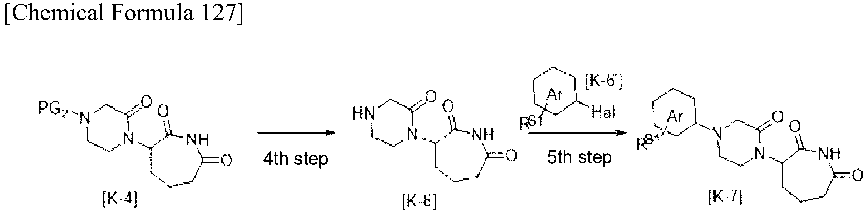

(wherein the symbols have the same meaning as described above)

A compound of General Formula [K-6] can be obtained by deprotection of a compound of General Formula K-4]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [K-7] can be obtained by reacting a compound of General Formula [K-6] with a compound of General Formula [K-6'] in the presence of a base. The reaction temperature and time, as well as the reagents and solvents used, are the same as those in the second step of Synthetic Method D.

### Synthetic Method K"

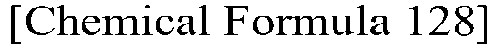

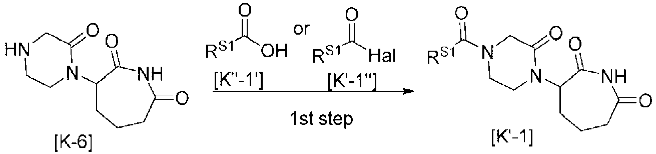

(wherein the symbols have the same meaning as described above)

A compound of General Formula [K'-1] can be obtained, for example, using one of the following two methods.

Method 1: A compound of General Formula [K'-1] can be obtained through a condensation reaction of a compound of General Formula [K-6] with a compound of General Formula [K'-1']. The reaction temperature and time, as well as the reagents and solvents used, are the same as those in Method 1 of the first step of Synthetic Method D2.

Method 2: A compound of General Formula [K'-1] can be obtained through a reaction of a compound of General Formula [K-6] with a compound of General Formula [K'-1"] in the presence of a base. The reaction temperature and time, as well as the reagents and solvents used, are the same as those in Method 2 of the first step of Synthetic Method D2.

### Synthetic Method L

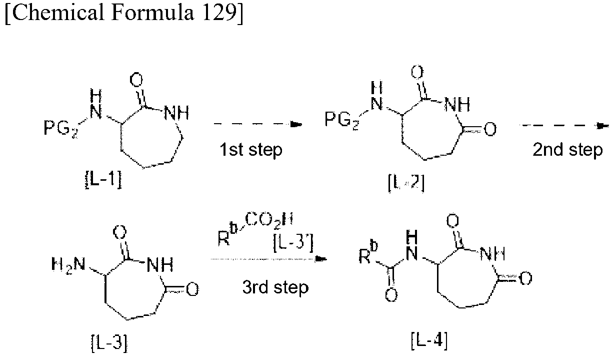

(wherein the symbols have the same meaning as described above)

A compound of General Formula [L-2] can be obtained through an oxidation reaction of a compound of General Formula [L-1]. The reaction temperature and time, as well as the reagents and solvents used, are the same as those in the first step of Synthetic Method A1.

A compound of General Formula [L-3] can be obtained by deprotection of a compound of General Formula [L-2]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [L-4] can be obtained through a condensation reaction of a compound of General Formula [L-3] with a compound of General Formula [L-3']. The reaction temperature and time, as well as the reagents and solvents used, are the same as those in the fourth step of Synthetic Method A.

### Synthetic Method M

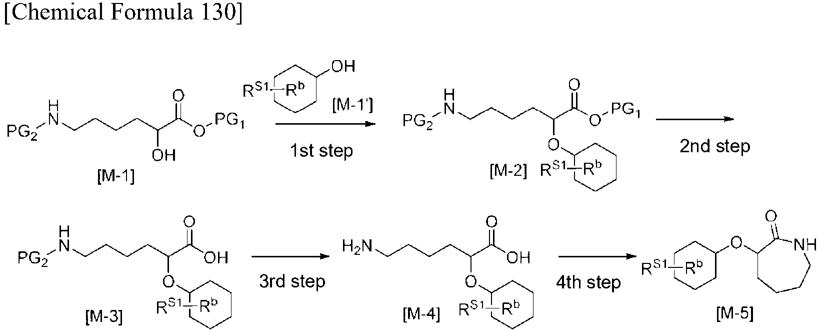

(wherein the symbols have the same meaning as described above)

A compound of General Formula [M-2] can be obtained by reacting a compound of General Formula [M-1] with a compound of General Formula [M-1'] in the presence of a phosphine derivative (for example, triphenylphosphine or the like) and an activating agent (for example, diisopropyl azodicarboxylate or the like). The reaction proceeds in an appropriate solvent, typically at a temperature ranging from -20 °C to the solvent reflux temperature. The reaction time varies depending on starting materials, solvent, and reaction temperature used, but is typically from 0.5 hours to 24 hours. Examples of the solvent include 1,4-dioxane, tetrahydrofuran, toluene, and the like.

A compound of General Formula [M-3] can be obtained by subjecting a compound of General Formula [M-2] to basic conditions. The reaction proceeds using a base, in an appropriate solvent, typically at a temperature ranging from room temperature to the solvent reflux temperature. The reaction time varies depending on starting materials, solvent, and reaction time used, but the reaction typically progresses within 1 hour to 48 hours. Examples of the base include sodium hydroxide, potassium hydroxide, lithium hydroxide, and the like, and examples of the solvent include methanol, ethanol, tetrahydrofuran, and water.

A compound of General Formula [M-4] can be obtained by subjecting a compound of General Formula [M-3] to acidic conditions. The reaction proceeds using an acid, in an appropriate solvent, typically at a temperature ranging from room temperature to the solvent reflux temperature. The reaction time varies depending on starting materials, solvent, and reaction time used, but the reaction typically progresses within 1 hour to 48 hours. Examples of the acid include trifluoroacetic acid, hydrochloric acid, and the like, and examples of the solvent include dichloromethane, 1,2-dichloroethane, chloroform, methanol, ethyl acetate, toluene, 1,4-dioxane, and water.

A compound of General Formula [M-5] can be obtained through a condensation reaction of a compound of General Formula [M-4]. The reaction temperature and time, as well as the reagents and solvents used, are the same as those in the fourth step of Synthetic Method A.

### Synthetic Method N

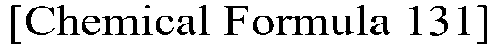

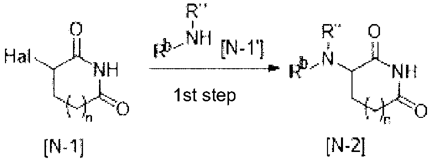

(wherein n is 1 or 2, and the other symbols have the same meanings as described above)

A compound of General Formula [N-2] can be obtained by reacting a compound of General Formula [N-1] with a compound of General Formula [N-1'] in the presence of a base. The reaction temperature and time, as well as the reagents and solvents used, are the same as those in the first step of Synthetic Method D1.

### Synthetic Method N'

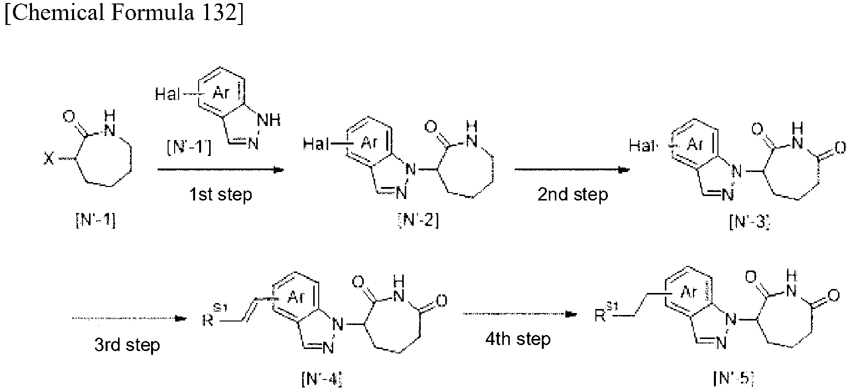

(wherein the symbols have the same meaning as described above)

A compound of General Formula [N'-2] can be obtained by reacting a compound of General Formula [N'-1] with a compound of General Formula [N'-1'] in the presence of a base. The reaction temperature and time, as well as the reagents and solvents used, are the same as those in the first step of Synthetic Method D1.

A compound of General Formula [N'-3] can be obtained through an oxidation reaction of a compound of General Formula [N'-2]. The reaction temperature and time, as well as the reagents and solvents used, are the same as those in the first step of Synthetic Method A1.

A compound of General Formula [N'-4] can be obtained by alkenylating a compound of General Formula [N'-3] in the presence of a palladium catalyst, a phosphine ligand, and a base. The reaction proceeds in an appropriate solvent, typically at a temperature ranging from room temperature to the solvent reflux temperature. The reaction may be accelerated under microwave irradiation. The reaction time varies depending on starting materials, solvent, and reaction temperature used, but is typically from 0.5 hours to 24 hours. Examples of the catalyst include palladium acetate and the like, and examples of the phosphine ligand include tri(o-tolyl)phosphine and the like. Examples of the base include sodium tert-butoxide and the like. Examples of the solvent include N,N-dimethylformamide, 1,4-dioxane, toluene, and the like.

A compound of General Formula [N'-5] can be obtained by subjecting a compound of General Formula [N'-4] to a reduction reaction under a hydrogen atmosphere in the presence of a catalyst. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the catalyst include palladium on carbon, and the like. Examples of the solvent include tetrahydrofuran, methanol, ethanol, water, mixtures thereof, and the like.

### Synthetic Method N"

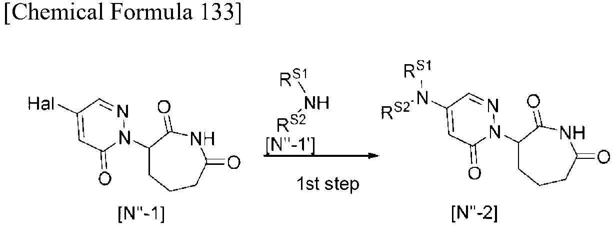

(wherein the symbols have the same meaning as described above)

A compound of General Formula [N"-2] can be obtained by reacting a compound of General Formula [N"-1] with a compound of General Formula [N"-1'] in the presence of a base. The reaction temperature and time, as well as the reagents and solvents used, are the same as those in the first step of Synthetic Method D1.

### Synthetic Method O

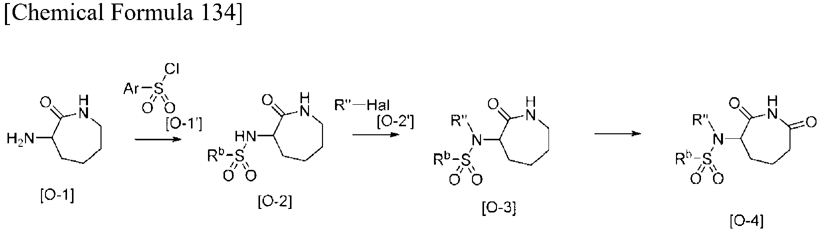

(wherein the symbols have the same meaning as described above)

A compound of General Formula [O-2] can be obtained by reacting a compound of General Formula [O-1] with a compound of General Formula [O-1'] in the presence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include triethylamine, diisopropylethylamine, pyridine, and the like. Examples of the solvent include methylene chloride, chloroform, and the like.

A compound of General Formula [O-3] can be obtained by reacting a compound of General Formula [O-2] with a compound of General Formula [O-2'] in the presence of a base. The reaction temperature and time, as well as the reagents and solvents used, are the same as those in the first step of Synthetic Method D1.

A compound of General Formula [O-4] can be obtained through an oxidation reaction of a compound of General Formula [O-3]. The reaction temperature and time, as well as the reagents and solvents used, are the same as those in the first step of Synthetic Method A1.

### Synthetic Method O'

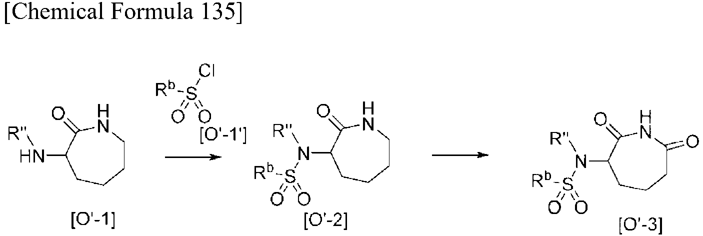

(wherein the symbols have the same meaning as described above)

A compound of General Formula [O'-2] can be obtained by reacting a compound of General Formula [O'-1] with a compound of General Formula [O'-1'] in the presence of a base. The reaction temperature and time, as well as the reagents and solvents used, are the same as those in the first step of Synthetic Method O.

A compound of General Formula [O'-3] can be obtained through an oxidation reaction of a compound of General Formula [O'-2]. The reaction temperature and time, as well as the reagents and solvents used, are the same as those in the first step of Synthetic Method A1.

### Synthetic Method P

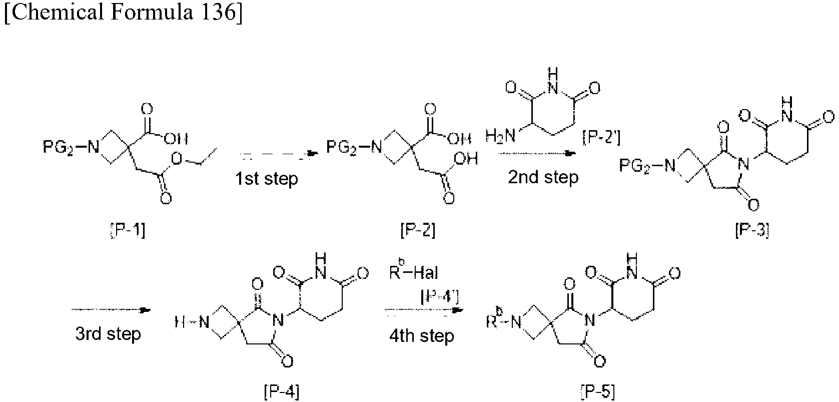

(wherein the symbols have the same meaning as described above)

A compound of General Formula [P-2] can be obtained by subjecting a compound of General Formula [P-1] to basic or acidic conditions. The reaction temperature and time, as well as the reagents and solvents used, are the same as those in the third step of Synthetic Method A.

A compound of General Formula [P-3] can be obtained by converting a compound of General Formula [P-2] into an acid anhydride using a dehydration reagent, followed by reacting it with a compound of General Formula [P-2'] in an appropriate solvent, typically at a temperature ranging from room temperature to the solvent reflux temperature. The reaction time varies depending on starting materials, solvent, and reaction temperature used, but is typically from 0.5 hours to 24 hours. Examples of the dehydration reagent include acetic anhydride, trifluoroacetic anhydride, and the like. Examples of the solvent include pyridine and the like.

A compound of General Formula [P-4] can be obtained through a deprotection reaction of a compound of General Formula [P-3]. The deprotection can be carried out using conventional methods such as reduction, hydrolysis, or acid treatment, depending on the type of protecting group to be removed. The reaction temperature and time, as well as the reagentsents and solvents used, are the same as those in the first step of Synthetic Method D.

A compound of General Formula [P-5] can be obtained by reacting a compound of General Formula [P-4] with a compound of General Formula [P-4'] in the presence of a base. The reaction temperature and time, as well as the reagents and solvents used, are the same as those in the first step of Synthetic Method D1.

### Synthetic Method P'

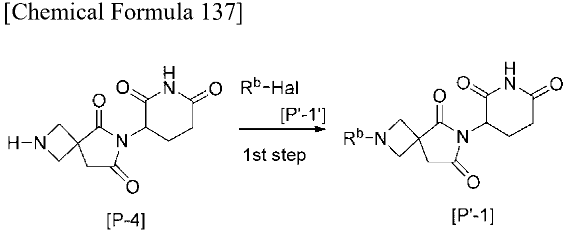

(wherein the symbols have the same meaning as described above)

A compound of General Formula [P'-1] can be obtained by reacting a compound of General Formula [P-4] with a compound of General Formula [P'-1'] in the presence of a base. The reaction temperature and time, as well as the reagents and solvents used, are the same as those in the first step of Synthetic Method D1.

### Synthetic Method P"

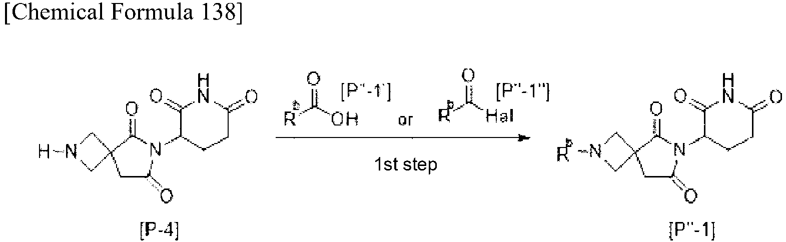

(wherein the symbols have the same meaning as described above)

A compound of General Formula [P"-1] can be obtained, for example, using one of the following two methods.

Method 1: A compound of General Formula [P"-1] can be obtained through a condensation reaction of a compound of General Formula [P-4] with a compound of General Formula [P"-1']. The reaction temperature and time, as well as the reagents and solvents used, are the same as those in Method 1 of the first step of Synthetic Method D2.

Method 2: A compound of General Formula [P"-] can be obtained through a reaction of a compound of General Formula [P-4] with a compound of General Formula [P"-1"] in the presence of a base. The reaction temperature and time, as well as the reagents and solvents used, are the same as those in Method 2 of the first step of Synthetic Method D2.

### Synthetic Method P1

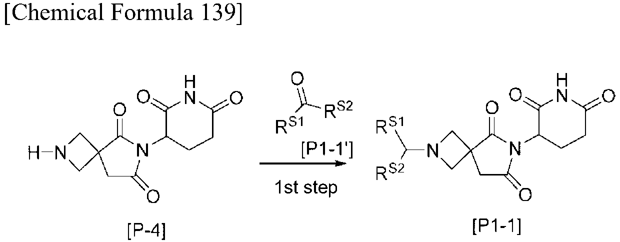

(wherein the symbols have the same meaning as described above)

A compound of General Formula [P1-1] can be obtained by reacting a compound of General Formula [P-4] with a compound of General Formula [P1-1"] in the presence of a reducing agent. The reaction temperature and time, as well as the reagents and solvents used, are the same as those in the first step of Synthetic Method X12.

### Synthetic Method P2

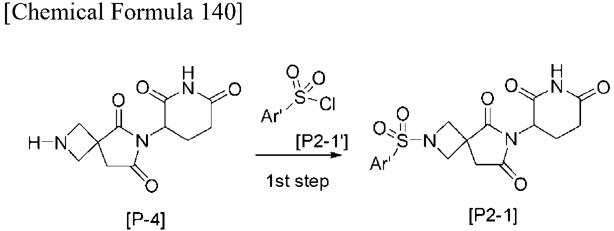

(wherein Ar' represents an aryl group, and the other symbols have the same meanings as described above)

A compound of General Formula [P2-1] can be obtained by reacting a compound of General Formula [P-4] with a compound of General Formula [P2-1'] in the presence of a base. The reaction temperature and time, as well as the reagents and solvents used, are the same as those in the first step of Synthetic Method O.

### Synthetic Method Q

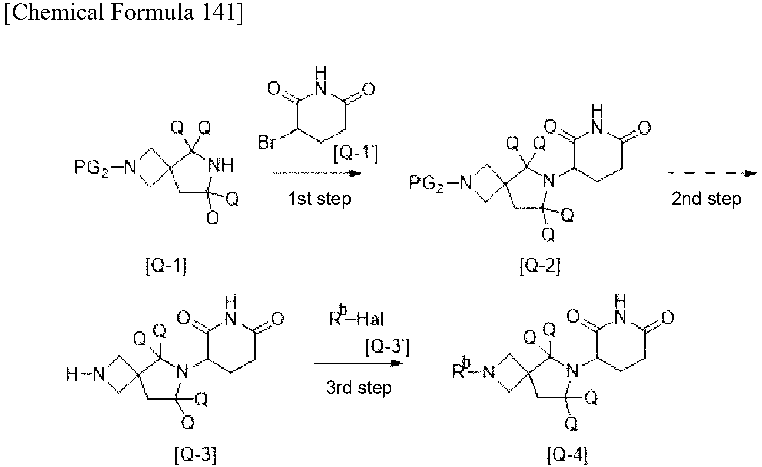

(wherein Q represents a hydrogen atom or any substituent, and the other symbols have the same meanings as described above. The any substituent is selected from the [Substituent Group] described above)

A compound of General Formula [Q-2] can be obtained by reacting a compound of General Formula [Q-1] with a compound of General Formula [Q-1'] in the presence of a base. The reaction temperature and time, as well as the reagents and solvents used, are the same as those in the first step of Synthetic Method D1.

A compound of General Formula [Q-3] can be obtained through a deprotection reaction of a compound of General Formula [Q-2]. The deprotection can be carried out using conventional methods such as reduction, or acid treatment, depending on the type of protecting group to be removed. The reaction temperature and time used in each treatment, as well as the reagents and solvents used, are the same as those in the first step of Synthesis Method D.

A compound of General Formula [Q-4] can be obtained by reacting a compound of General Formula [Q-3] with a compound of General Formula [Q-3'] in the presence of a base. The reaction temperature and time, as well as the reagents and solvents used, are the same as those in the second step of Synthetic Method D.

### Synthetic Method R

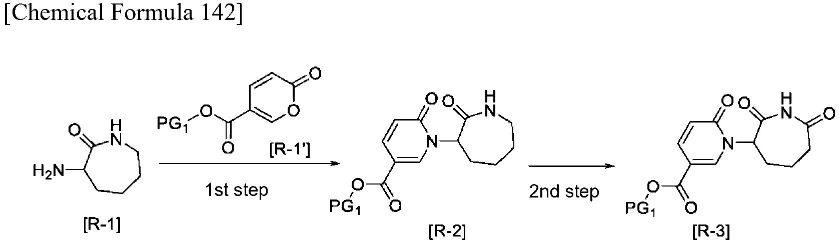

(wherein the symbols have the same meaning as described above)

A compound of General Formula [R-2] can be obtained by reacting a compound of General Formula [R-1] with a compound of General Formula [R-1']. The reaction proceeds in an appropriate solvent, typically at a temperature ranging from room temperature to the solvent reflux temperature. The reaction time varies depending on starting materials and solvent used, but the reaction typically progresses within 10 minutes to 24 hours. Examples of the solvent include pyridine and the like.

A compound of General Formula [R-3] can be obtained through an oxidation reaction of a compound of General Formula [R-2]. The reaction temperature and time, as well as the reagents and solvents used, are the same as those in the first step of Synthetic Method A1.

### Synthetic Method S

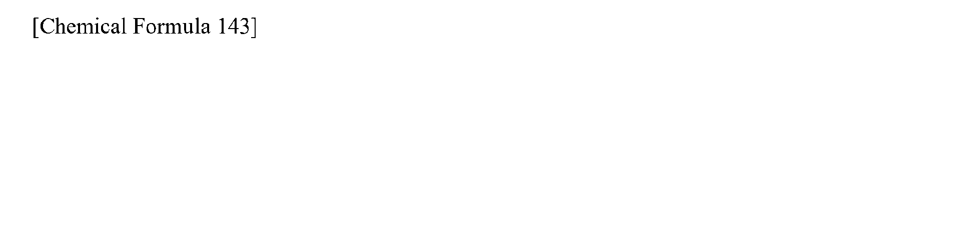

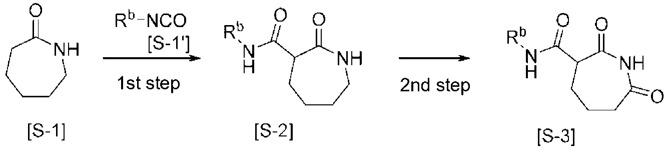

(wherein the symbols have the same meaning as described above)

A compound of General Formula [S-2] can be obtained by reacting a compound of General Formula [S-1] with a compound of General Formula [S-1'] in the presence of a base. The reaction proceeds in an appropriate solvent, typically at a temperature ranging from -78 °C to the solvent reflux temperature. The reaction time varies depending on starting materials and solvent used, but the reaction typically progresses within 10 minutes to 24 hours. Examples of the base include lithium bis(trimethylsilyl)amide, sodium hydride, lithium diisopropylamide, and the like. Examples of the solvent include tetrahydrofuran and the like.

A compound of General Formula [S-3] can be obtained through an oxidation reaction of a compound of General Formula [S-2]. The reaction temperature and time, as well as the reagents and solvents used, are the same as those in the first step of Synthetic Method A1.

### Synthetic Method T

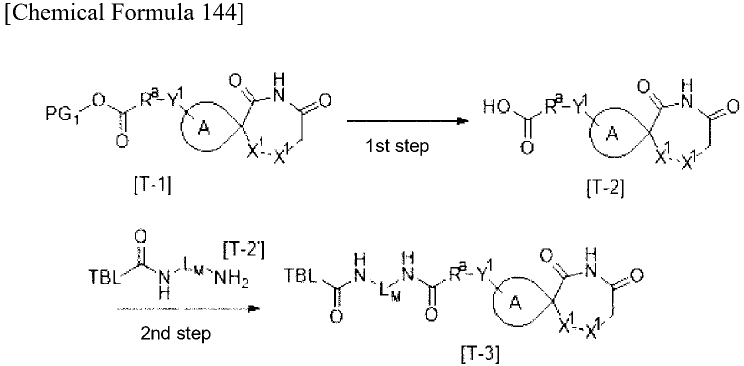

(wherein L_{M} represents a linker main chain, TBL represents a group having a moiety capable of binding to a target protein or a moiety that binds to a target protein, and the other symbols have the same meanings as described above)

A compound of General Formula [T-2] can be obtained by subjecting a compound of General Formula [T-1] to basic or acidic conditions. The reaction temperature and time, as well as the reagents and solvents used, are the same as those in the third step of Synthetic Method A.

A compound of General Formula [T-3] can be obtained through a condensation reaction of a compound of General Formula [T-2] with a compound of General Formula [T-2']. The reaction temperature and time, as well as the reagents and solvents used, are the same as those in the fourth step of Synthetic Method A.

### Synthetic Method T'

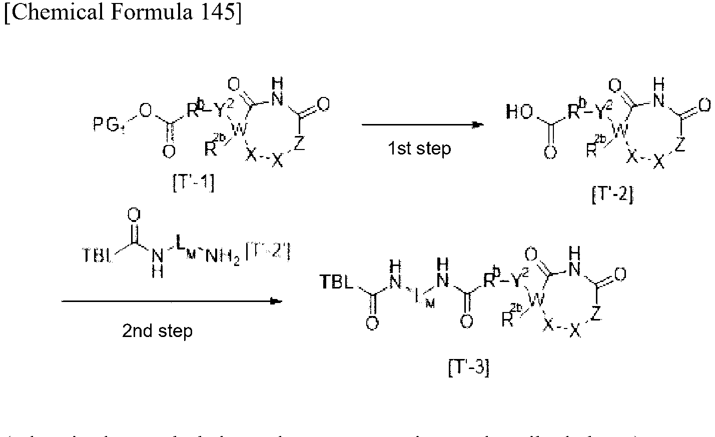

(wherein the symbols have the same meaning as described above)

A compound of General Formula [T'-2] can be obtained by subjecting a compound of General Formula [T'-1] to basic or acidic conditions. The reaction temperature and time, as well as the reagents and solvents used, are the same as those in the third step of Synthetic Method A.

A compound of General Formula [T'-3] can be obtained through a condensation reaction of a compound of General Formula [T'-2] with a compound of General Formula [T'-2']. The reaction temperature and time, as well as the reagents and solvents used, are the same as those in the fourth step of Synthetic Method A.

### Synthetic Method U

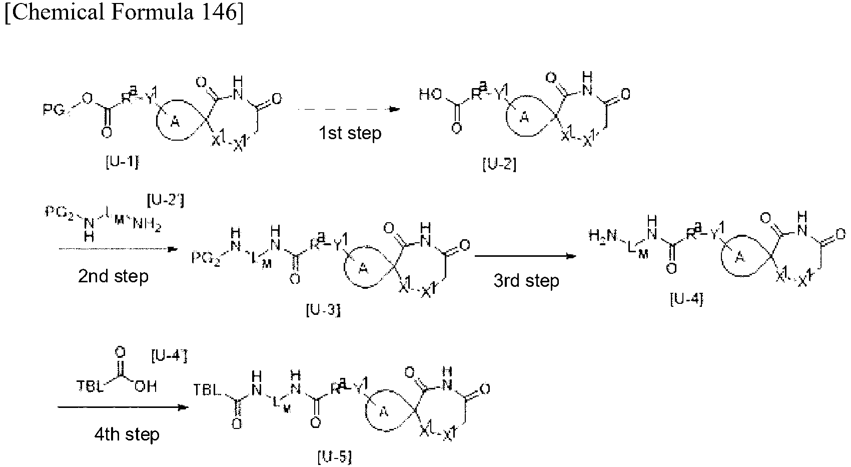

(wherein the symbols have the same meaning as described above)

A compound of General Formula [U-2] can be obtained by subjecting a compound of General Formula [U-1] to basic or acidic conditions. The reaction temperature and time, as well as the reagents and solvents used, are the same as those in the third step of Synthetic Method A.

A compound of General Formula [U-3] can be obtained through a condensation reaction of a compound of General Formula [U-2] with a compound of General Formula [U-2']. The reaction temperature and time, as well as the reagents and solvents used, are the same as those in the fourth step of Synthetic Method A.

A compound of General Formula [U-4] can be obtained through a deprotection reaction of a compound of General Formula [U-3]. The deprotection can be carried out using conventional methods such as reduction, hydrolysis, or acid treatment, depending on the type of protecting group to be removed. The reaction temperature and time, as well as the reagentsents and solvents used, are the same as those in the first step of Synthetic Method D.

A compound of General Formula [U-5] can be obtained through a condensation reaction of a compound of General Formula [U-4] with a compound of General Formula [U-4']. The reaction temperature and time, as well as the reagents and solvents used, are the same as those in the fourth step of Synthetic Method A.

### Synthetic Method U'

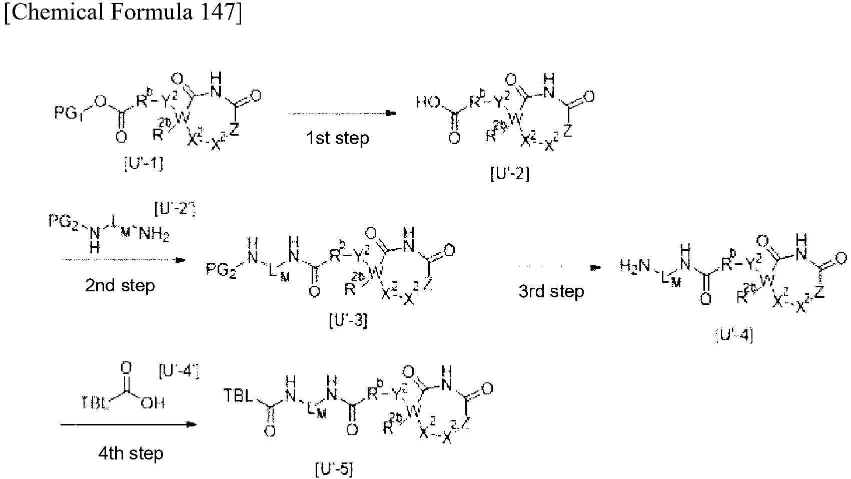

(wherein the symbols have the same meaning as described above)

A compound of General Formula [U'-2] can be obtained by subjecting a compound of General Formula [U'-1] to basic or acidic conditions. The reaction temperature and time, as well as the reagents and solvents used, are the same as those in the third step of Synthetic Method A.

A compound of General Formula [U'-3] can be obtained through a condensation reaction of a compound of General Formula [U'-2] with a compound of General Formula [U'-2']. The reaction temperature and time, as well as the reagents and solvents used, are the same as those in the fourth step of Synthetic Method A.

A compound of General Formula [U'-4] can be obtained through a deprotection reaction of a compound of General Formula [U'-3]. The deprotection can be carried out using conventional methods such as reduction, hydrolysis, or acid treatment, depending on the type of protecting group to be removed. The reaction temperature and time, as well as the reagentsents and solvents used, are the same as those in the first step of Synthetic Method D.

A compound of General Formula [U'-5] can be obtained through a condensation reaction of a compound of General Formula [U'-4] with a compound of General Formula [U'-4']. The reaction temperature and time, as well as the reagents and solvents used, are the same as those in the fourth step of Synthetic Method A.

### Synthetic Method V

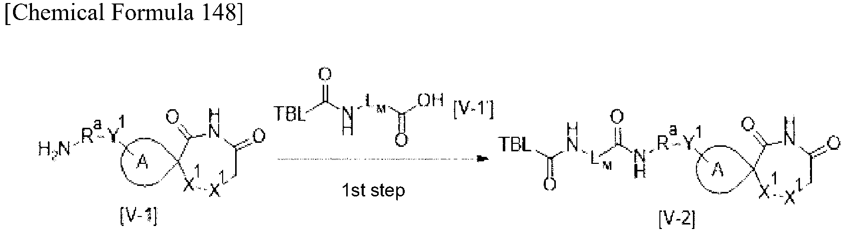

(wherein the symbols have the same meaning as described above)

A compound of General Formula [V-2] can be obtained through a condensation reaction of a compound of General Formula [V-1] with a compound of General Formula [V-1']. The reaction temperature and time, as well as the reagents and solvents used, are the same as those in the fourth step of Synthetic Method A.

### Synthetic Method V'

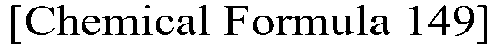

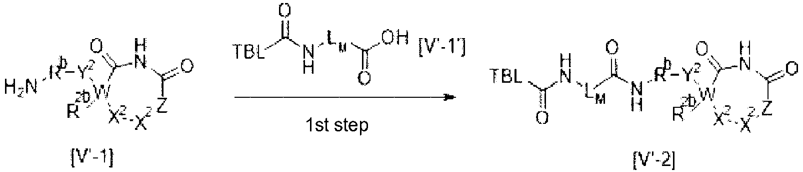

(wherein the symbols have the same meaning as described above)

A compound of General Formula [V'-2] can be obtained through a condensation reaction of a compound of General Formula [V'-1] with a compound of General Formula [V'-1']. The reaction temperature and time, as well as the reagents and solvents used, are the same as those in the fourth step of Synthetic Method A.

### Synthetic Method V"

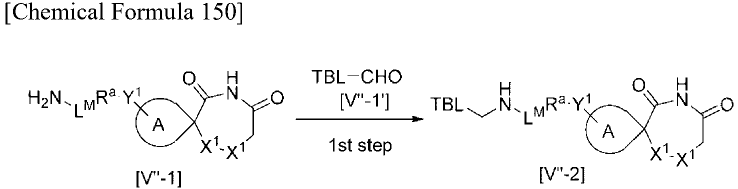

(wherein the symbols have the same meaning as described above)

A compound of General Formula [V"-2] can be obtained by reacting a compound of General Formula [V"-1] with a compound of General Formula [V"-1'] in the presence of a reducing agent. The reaction temperature and time, as well as the reagents and solvents used, are the same as those in the first step of Synthetic Method X12.

### Synthetic Method V‴

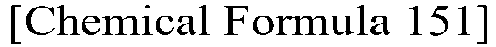

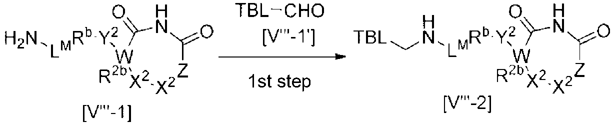

(wherein the symbols have the same meaning as described above)

A compound of General Formula [V‴-2] can be obtained by reacting a compound of General Formula [V‴-1] with a compound of General Formula [V‴-1'] in the presence of a reducing agent. The reaction temperature and time, as well as the reagents and solvents used, are the same as those in the first step of Synthetic Method X12.

### Synthetic Method W

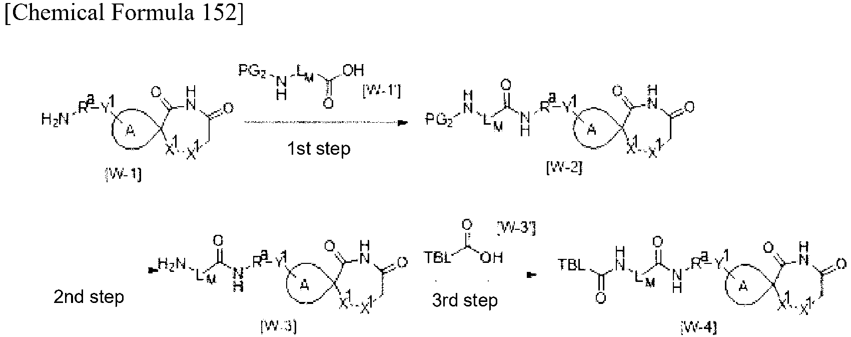

(wherein the symbols have the same meaning as described above)

A compound of General Formula [W-2] can be obtained through a condensation reaction of a compound of General Formula [W-1] with a compound of General Formula [W-1']. The reaction temperature and time, as well as the reagents and solvents used, are the same as those in the fourth step of Synthetic Method A.

A compound of General Formula [W-3] can be obtained through a deprotection reaction of a compound of General Formula [W-2]. The deprotection can be carried out using conventional methods such as reduction, hydrolysis, or acid treatment, depending on the type of protecting group to be removed. The reaction temperature and time, as well as the reagentsents and solvents used, are the same as those in the first step of Synthetic Method D.

A compound of General Formula [W-4] can be obtained through a condensation reaction of a compound of General Formula [W-3] with a compound of General Formula [W-3']. The reaction temperature and time, as well as the reagents and solvents used, are the same as those in the fourth step of Synthetic Method A.

### Synthetic Method W'

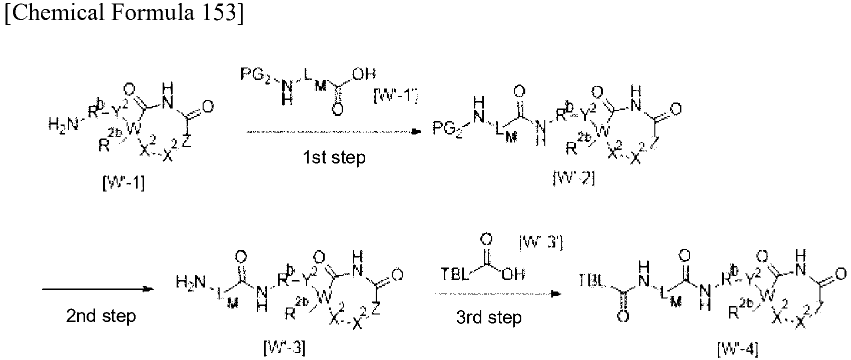

(wherein the symbols have the same meaning as described above)

A compound of General Formula [W'-2] can be obtained through a condensation reaction of a compound of General Formula [W'-1] with a compound of General Formula [W'-1']. The reaction temperature and time, as well as the reagents and solvents used, are the same as those in the fourth step of Synthetic Method A.

A compound of General Formula [W'-3] can be obtained through a deprotection reaction of a compound of General Formula [W'-2]. The deprotection can be carried out using conventional methods such as reduction, hydrolysis, or acid treatment, depending on the type of protecting group to be removed. The reaction temperature and time, as well as the reagentsents and solvents used, are the same as those in the first step of Synthetic Method D.

A compound of General Formula [W'-4] can be obtained through a condensation reaction of a compound of General Formula [W'-3] with a compound of General Formula [W'-3']. The reaction temperature and time, as well as the reagents and solvents used, are the same as those in the fourth step of Synthetic Method A.

### Synthetic Method X1

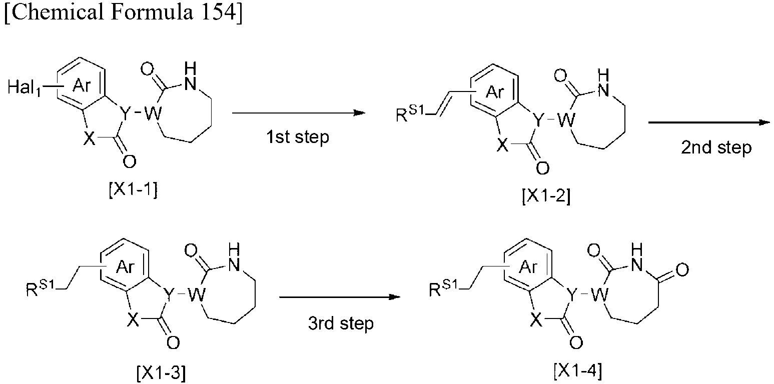

(wherein X represents an optionally substituted nitrogen atom, sulfur atom, or oxygen atom, Y represents an optionally substituted carbon atom or nitrogen atom, Hal₁ represents chlorine, bromine, or iodine, and the other symbols have the same meanings as described above)

A compound of General Formula [X1-2] can be obtained by alkenylating a compound of General Formula [X1-1] in the presence of a palladium catalyst, a phosphine ligand, and a base. The reaction temperature and time, as well as the reagents and solvents used, are the same as those in the sixth step of Synthetic Method H‴.

A compound of General Formula [X1-3] can be obtained by subjecting a compound of General Formula X1-2] to a reduction reaction under a hydrogen atmosphere in the presence of a catalyst.

The reaction temperature and time, as well as the reagents and solvents used, are the same as those in the seventh step of Synthetic Method H‴.

A compound of General Formula [X1-4] can be obtained through an oxidation reaction of a compound of General Formula [X1-3]. The reaction temperature and time, as well as the reagents and solvents used, are the same as those in the first step of Synthetic Method A1.

### Synthetic Method X2

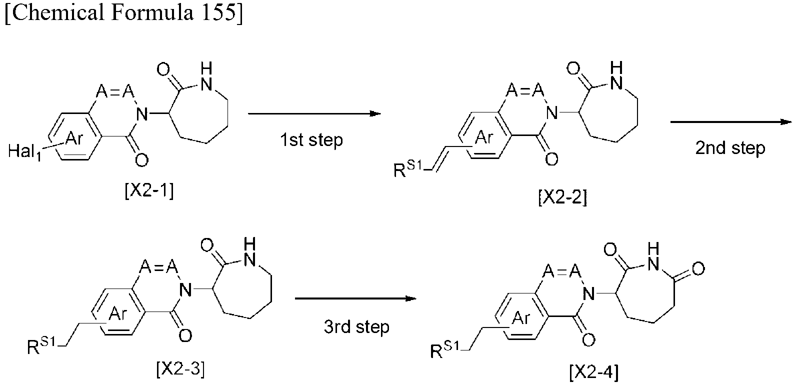

(wherein A represents an optionally substituted carbon atom or nitrogen atom, Hal₁ represents chlorine, bromine, or iodine, and the other symbols have the same meanings as described above)

A compound of General Formula [X2-2] can be obtained by alkenylating a compound of General Formula [X2-1] in the presence of a palladium catalyst, a phosphine ligand, and a base. The reaction temperature and time, as well as the reagents and solvents used, are the same as those in the sixth step of Synthetic Method H‴.

A compound of General Formula [X2-3] can be obtained by subjecting a compound of General Formula X2-2] to a reduction reaction under a hydrogen atmosphere in the presence of a catalyst. The reaction temperature and time, as well as the reagents and solvents used, are the same as those in the seventh step of Synthetic Method H‴.

A compound of General Formula [X2-4] can be obtained through an oxidation reaction of a compound of General Formula [X2-3]. The reaction temperature and time, as well as the reagents and solvents used, are the same as those in the first step of Synthetic Method A1.

### Synthetic Method X3

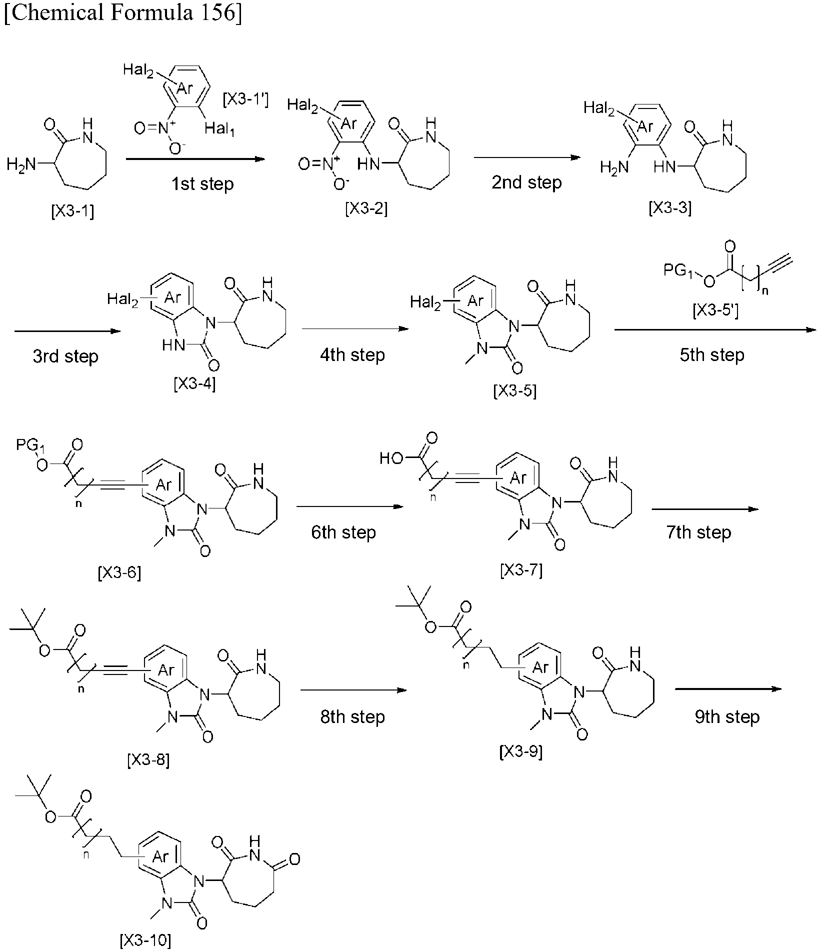

(wherein Hal₂ represents chlorine, bromine, or iodine, n is 0, 1, 2, 3, 4, 5, or 6, and the other symbols have the same meaning as described above).

A compound of General Formula [X3-2] can be obtained by reacting a compound of General Formula [X3-1] with a compound of General Formula [X3-1'] in the presence of a base. The reaction temperature and time, as well as the reagents and solvents used, are the same as those in the second step of Synthetic Method H‴.

A compound of General Formula [X3-3] can be obtained by subjecting a compound of General Formula [X3-2] to a reduction reaction. The reaction temperature and time, as well as the reagents and solvents used, are the same as those in the second step of Synthetic Method D.

A compound of General Formula [X3-4] can be obtained by carbonylating a compound of General Formula [X3-3] in the presence of a base. The reaction temperature and time, as well as the reagents and solvents used, are the same as those in the third step of Synthetic Method C.

A compound of General Formula [X3-5] can be obtained by methylating a compound of General Formula [X3-4]. The reaction proceeds in an appropriate solvent, at a temperature ranging from 0 °C to the solvent reflux temperature. The reaction time varies depending on starting materials, solvent, and reaction temperature used, but is typically from 30 minutes to 24 hours. Examples of the methylating agent include methyl iodide, and the like. Examples of the base include potassium carbonate, and the like. Examples of the solvent include N,N-dimethylformamide, and the like.

A compound of General Formula [X3-6] can be obtained by alkynylating a compound of General Formula [X3-5] with a compound of General Formula [X3-5'] in the presence of a palladium catalyst, a copper catalyst, and a base. The reaction proceeds in an appropriate solvent, at a temperature ranging from 0 °C to the solvent reflux temperature. The reaction time varies depending on starting materials, solvent, and reaction temperature used, but is typically from 30 minutes to 24 hours. Examples of the palladium catalyst include bis(triphenylphosphine)palladium(II) dichloride and the like. Examples of the copper catalyst include copper(I) iodide and the like. Examples of the base include N,N-diisopropylethylamine and the like. Examples of the solvent include tetrahydrofuran and the like.

A compound of General Formula [X3-7] can be obtained by hydrolyzing a compound of General Formula [X3-6]. The reaction proceeds using a base, in an appropriate solvent, typically at a temperature ranging from room temperature to the solvent reflux temperature. The reaction time varies depending on starting materials, solvent, and reaction time used, but the reaction typically progresses within 1 hour to 48 hours. Examples of the base include sodium hydroxide, potassium hydroxide, lithium hydroxide, and the like. Examples of the solvent include methanol, ethanol, tetrahydrofuran, water, and mixtures thereof.

A compound of General Formula [X3-8] can be obtained by tert-butyl esterification of a compound of General Formula [X3-7]. The reaction proceeds in an appropriate solvent, at a temperature ranging from 0 °C to the solvent reflux temperature. The reaction time varies depending on starting materials, solvent, and reaction temperature used, but is typically from 30 minutes to 72 hours. Examples of the tert-butylating reagent include 2-tert-butyl-1,3-diisopropylisourea and the like. Examples of the solvent include dichloromethane and the like.

A compound of General Formula [X3-9] can be obtained by subjecting a compound of General Formula [X3-8] to a reduction reaction under a hydrogen atmosphere in the presence of a catalyst. The reaction temperature and time, as well as the reagents and solvents used, are the same as those in the seventh step of Synthetic Method H‴.

A compound of General Formula [X3-10] can be obtained through an oxidation reaction of a compound of General Formula [X3-9]. The reaction temperature and time, as well as the reagents and solvents used, are the same as those in the first step of Synthetic Method A1.

### Synthetic Method X3'

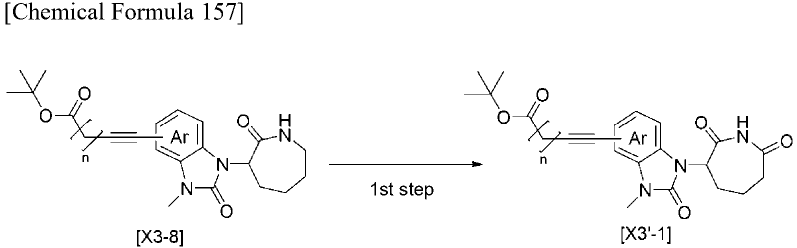

(wherein n is 0, 1, 2, 3, 4, 5, or 6, and the other symbols have the same meaning as described above).

A compound of General Formula [X3'-1] can be obtained through an oxidation reaction of a compound of General Formula [X3-8]. The reaction temperature and time, as well as the reagents and solvents used, are the same as those in the first step of Synthetic Method A1.

### Synthetic Method X3"

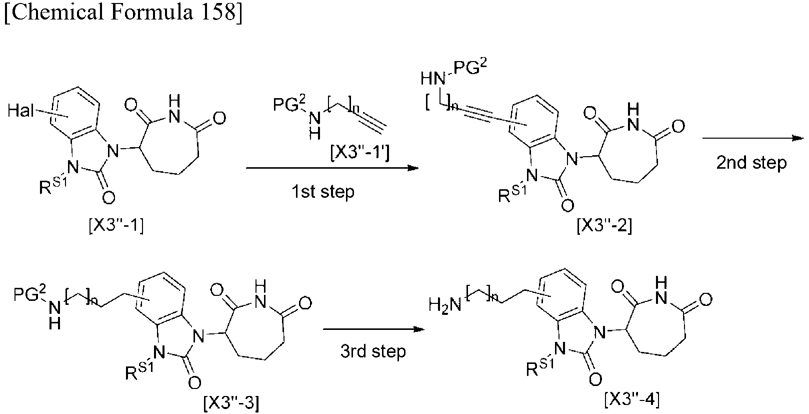

(wherein n is 0, 1, 2, 3, 4, 5, or 6, and the other symbols have the same meaning as described above).

A compound of General Formula [X3"-2] can be obtained by alkynylating a compound of General Formula [X3"-1] with a compound of General Formula [X3"-1'] in the presence of a palladium catalyst, a copper catalyst, and a base. The reaction temperature and time, as well as the reagents and solvents used, are the same as those in the fifth step of Synthetic Method X3.

A compound of General Formula [X3"-3] can be obtained by subjecting a compound of General Formula [X3"-2] to a reduction reaction under a hydrogen atmosphere in the presence of a catalyst. The reaction temperature and time, as well as the reagents and solvents used, are the same as those in the seventh step of Synthetic Method H‴.

A compound of General Formula [X3"-4] can be obtained by deprotection of a compound of General Formula [X3"-3]. The deprotection can be carried out using conventional methods such as reduction, hydrolysis, or acid treatment, depending on the type of protecting group to be removed. The reaction temperature and time, as well as the reagentsents and solvents used, are the same as those in the first step of Synthetic Method D.

### Synthetic Method X3‴

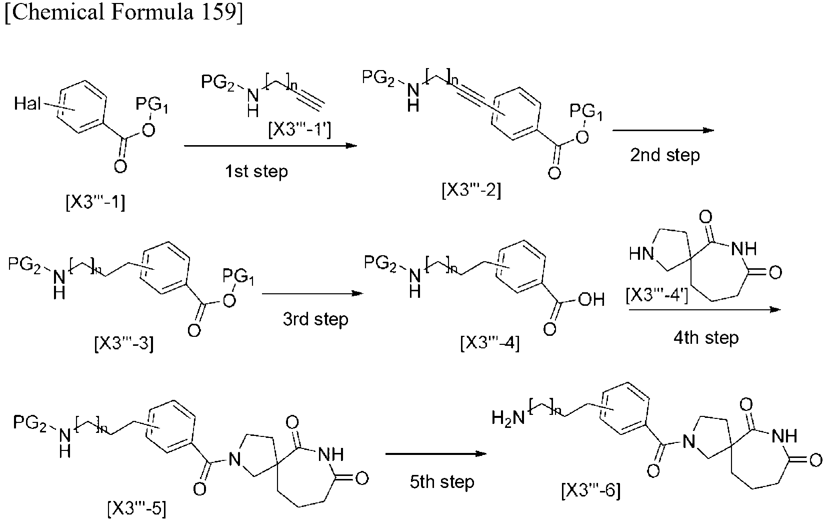

(wherein n is 0, 1, 2, 3, 4, 5, or 6, and the other symbols have the same meaning as described above).

A compound of General Formula [X3‴-2] can be obtained by alkynylating a compound of General Formula [X3‴-1] with a compound of General Formula [X3‴-1'] in the presence of a palladium catalyst, a copper catalyst, and a base. The reaction temperature and time, as well as the reagents and solvents used, are the same as those in the fifth step of Synthetic Method X3.

A compound of General Formula [X3‴-3] can be obtained by subjecting a compound of General Formula [X3‴-2] to a reduction reaction under a hydrogen atmosphere in the presence of a catalyst. The reaction temperature and time, as well as the reagents and solvents used, are the same as those in the seventh step of Synthetic Method H‴.

A compound of General Formula [X3‴-4] can be obtained by deprotection of a compound of General Formula [X3"'-3]. The deprotection can be carried out using conventional methods such as reduction, hydrolysis, or acid treatment, depending on the type of protecting group to be removed. The reaction temperature and time, as well as the reagentsents and solvents used, are the same as those in the first step of Synthetic Method D.

A compound of General Formula [X3‴-5] can be obtained through a condensation reaction of a compound of General Formula [X3‴-4] with a compound of General Formula [X3‴-4']. The reaction temperature and time, as well as the reagents and solvents used, are the same as those in Method 1 of the first step of Synthetic Method D2.

A compound of General Formula [X3‴-6] can be obtained by deprotection of a compound of General Formula [X3‴-5]. The deprotection can be carried out using conventional methods such as reduction, hydrolysis, or acid treatment, depending on the type of protecting group to be removed. The reaction temperature and time, as well as the reagentsents and solvents used, are the same as those in the first step of Synthetic Method D.

### Synthetic Method X4

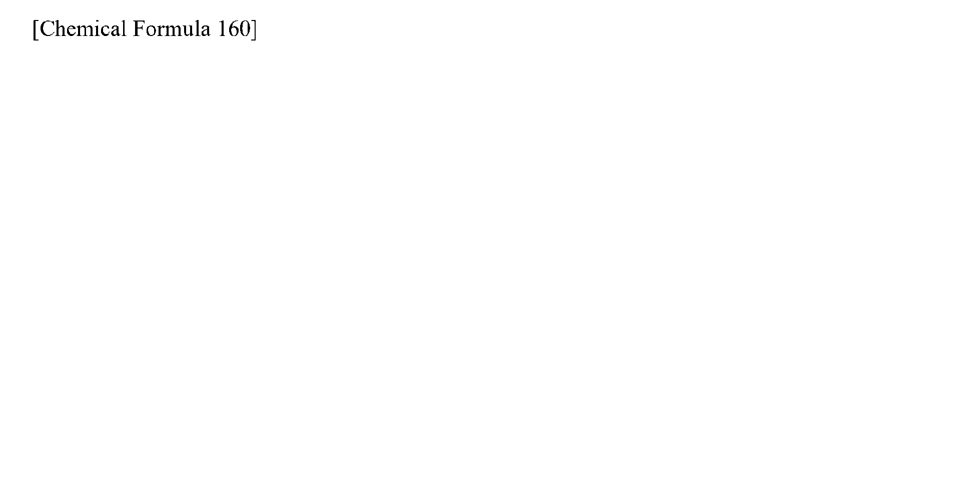

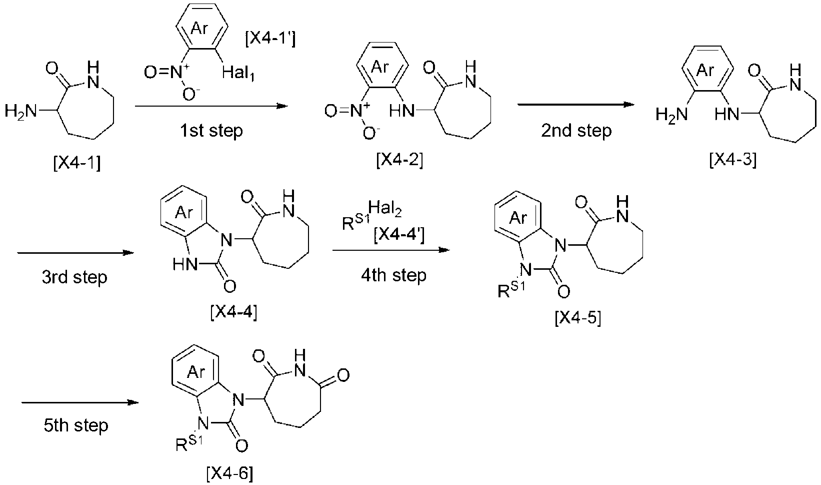

(wherein Hal₁ represents fluorine or chlorine, Hal₂ represents chlorine, bromine, or iodine, and the other symbols have the same meanings as described above)

A compound of General Formula [X4-2] can be obtained by reacting a compound of General Formula [X4-1] with a compound of General Formula [X4-1'] in the presence of a base. The reaction temperature and time, as well as the reagents and solvents used, are the same as those in the second step of Synthetic Method H‴.

A compound of General Formula [X4-3] can be obtained by subjecting a compound of General Formula [X4-2] to a reduction reaction. The reaction temperature and time, as well as the reagents and solvents used, are the same as those in the second step of Synthetic Method D.

A compound of General Formula [X4-4] can be obtained by carbonylating a compound of General Formula [X4-3] in the presence of a base. The reaction temperature and time, as well as the reagents and solvents used, are the same as those in the third step of Synthetic Method C.

A compound of General Formula [X4-5] can be obtained by reacting a compound of General Formula [X4-4] with a compound of General Formula [X4-4'] in the presence of a base. The reaction temperature and time, as well as the reagents and solvents used, are the same as those in the first step of Synthetic Method D1.

A compound of General Formula [X4-6] can be obtained through an oxidation reaction of a compound of General Formula [X4-5]. The reaction temperature and time, as well as the reagents and solvents used, are the same as those in the first step of Synthetic Method A1.

### Synthetic Method X5

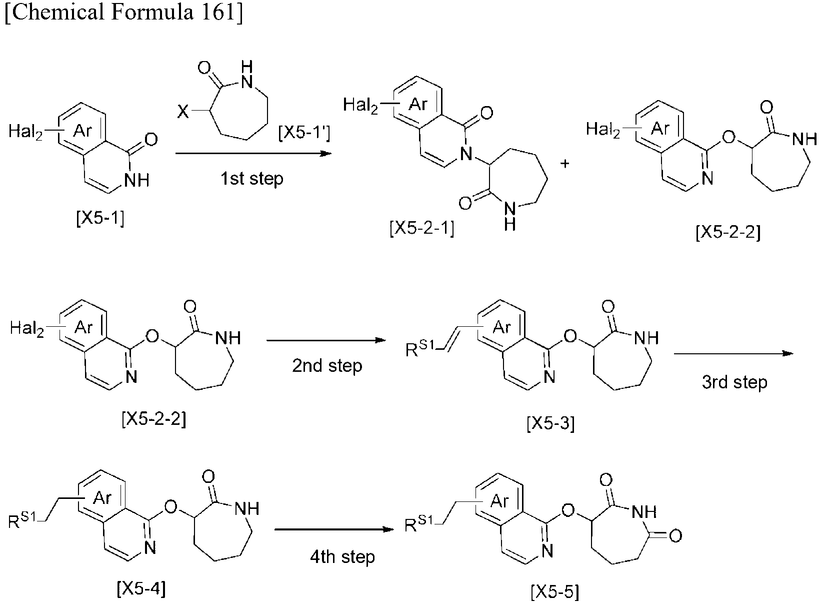

(wherein Hal₂ represents fluorine or chlorine, and the other symbols have the same meaning as described above).

A compound of General Formula [X5-2-1] and a compound of General Formula [X5-2-2] can be obtained by reacting a compound of General Formula [X5-1] with a compound of General Formula [X5-1'] in the presence of a base. The reaction temperature and time, as well as the reagents and solvents used, are the same as those in the first step of Synthetic Method D1.

A compound of General Formula [X5-3] can be obtained by alkenylating a compound of General Formula [X5-2-2] in the presence of a palladium catalyst, a phosphine ligand, and a base. The reaction temperature and time, as well as the reagents and solvents used, are the same as those in the sixth step of Synthetic Method H‴.

A compound of General Formula [X5-4] can be obtained by subjecting a compound of General Formula X5-3] to a reduction reaction under a hydrogen atmosphere in the presence of a catalyst. The reaction temperature and time, as well as the reagents and solvents used, are the same as those in the seventh step of Synthetic Method H‴.

A compound of General Formula [X5-5] can be obtained through an oxidation reaction of a compound of General Formula [X5-4]. The reaction temperature and time, as well as the reagents and solvents used, are the same as those in the first step of Synthetic Method A1.

### Synthetic Method X6

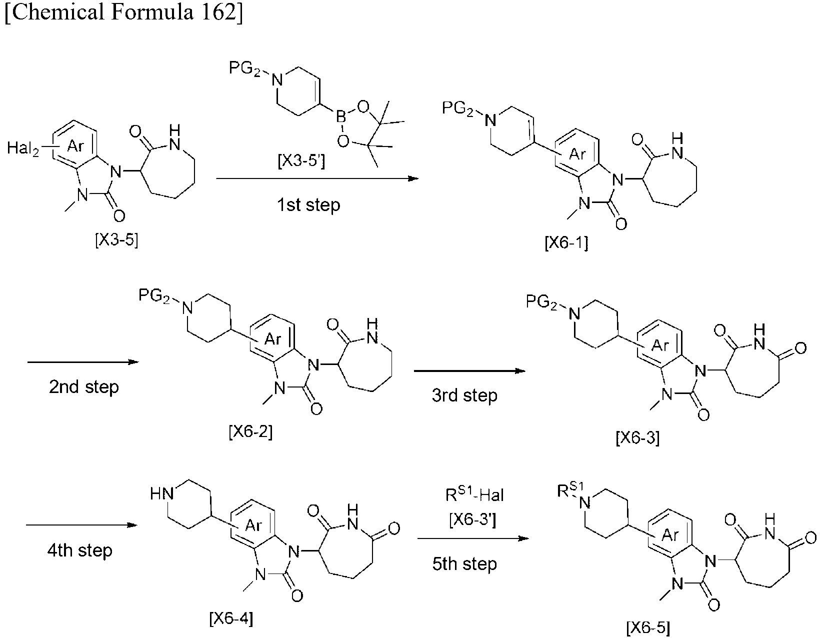

(wherein Hal₂ represents fluorine or chlorine, and the other symbols have the same meanings as described above)

A compound of General Formula [X6-1] can be obtained by alkenylating a compound of General Formula [X3-5] with a compound of General Formula [X3-5'] in the presence of a palladium catalyst and a base. The reaction proceeds in an appropriate solvent, at a temperature ranging from 0 °C to the solvent reflux temperature. The reaction time varies depending on starting materials, solvent, and reaction temperature used, but is typically from 30 minutes to 24 hours. Examples of the palladium catalyst include tetrakis(triphenylphosphine)palladium and the like. Examples of the base include potassium carbonate, and the like. Examples of the solvent include water, 1,4-dioxane, and mixtures thereof, and the like.

A compound of General Formula [X6-2] can be obtained by subjecting a compound of General Formula [X6-1] to a reduction reaction under a hydrogen atmosphere in the presence of a catalyst. The reaction temperature and time, as well as the reagents and solvents used, are the same as those in the seventh step of Synthetic Method H‴.

A compound of General Formula [X6-3] can be obtained through an oxidation reaction of a compound of General Formula [X6-2]. The reaction temperature and time, as well as the reagents and solvents used, are the same as those in the first step of Synthetic Method A1.

A compound of General Formula [X6-4] can be obtained by deprotection of a compound of General Formula [X6-3]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [X6-5] can be obtained by reacting a compound of General Formula [X6-4] with a compound of General Formula [X6-4'] in the presence of a base. The reaction temperature and time, as well as the reagents and solvents used, are the same as those in the first step of Synthetic Method D1.

### Synthetic Method X7

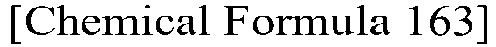

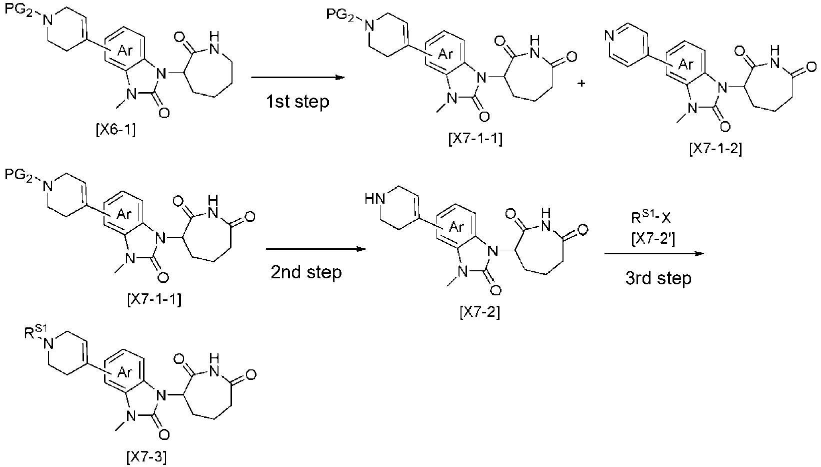

(wherein X is a leaving group, and the other symbols have the same meanings as described above)

A compound of General Formula [X7-1-1] and a compound of General Formula [X7-1-2] can be obtained through an oxidation reaction of a compound of General Formula [X6-1]. The reaction temperature and time, as well as the reagents and solvents used, are the same as those in the first step of Synthetic Method A1.

A compound of General Formula [X7-2] can be obtained by deprotection of a compound of General Formula [X7-1-1]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [X7-3] can be obtained by reacting a compound of General Formula [X7-2] with a compound of General Formula [X7-2'] in the presence of a base. The reaction temperature and time, as well as the reagents and solvents used, are the same as those in the first step of Synthetic Method D1.

### Synthetic Method X8

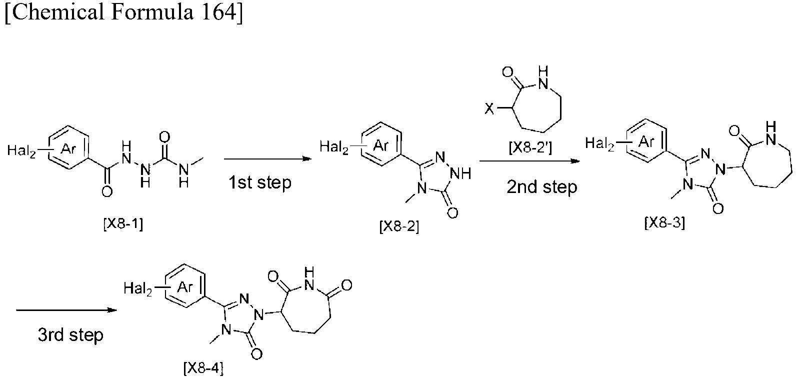

(wherein Hal₂ represents fluorine or chlorine, X is a leaving group, and the other symbols have the same meanings as described above)

A compound of General Formula [X8-2] can be obtained by subjecting a compound of General Formula [X8-1] to basic conditions. The reaction proceeds in an appropriate solvent, at a temperature ranging from 0 °C to the solvent reflux temperature. The reaction time varies depending on starting materials, solvent, and reaction temperature used, but is typically from 30 minutes to 24 hours. Examples of the base include sodium hydroxide and the like. Examples of the solvent include water and the like.

A compound of General Formula [X8-3] can be obtained by reacting a compound of General Formula [X8-2] with a compound of General Formula [X8-2'] in the presence of a base. The reaction temperature and time, as well as the reagents and solvents used, are the same as those in the first step of Synthetic Method D1.

A compound of General Formula [X8-4] can be obtained through an oxidation reaction of a compound of General Formula [X8-3]. The reaction temperature and time, as well as the reagents and solvents used, are the same as those in the first step of Synthetic Method A1.

### Synthetic Method X9

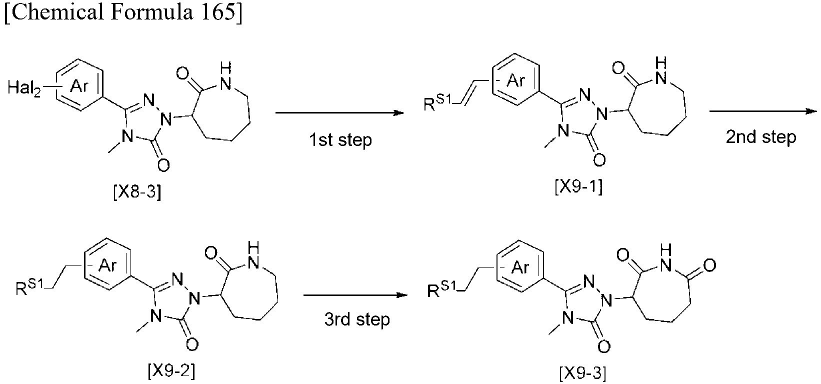

(wherein Hal₂ represents fluorine or chlorine, and the other symbols have the same meanings as described above)

A compound of General Formula [X9-1] can be obtained by alkenylating a compound of General Formula [X8-3] in the presence of a palladium catalyst, a phosphine ligand, and a base. The reaction temperature and time, as well as the reagents and solvents used, are the same as those in the sixth step of Synthetic Method H‴.

A compound of General Formula [X9-2] can be obtained by subjecting a compound of General Formula [X9-1] to a reduction reaction under a hydrogen atmosphere in the presence of a catalyst. The reaction temperature and time, as well as the reagents and solvents used, are the same as those in the seventh step of Synthetic Method H‴.

A compound of General Formula [X9-3] can be obtained through an oxidation reaction of a compound of General Formula [X9-2]. The reaction temperature and time, as well as the reagents and solvents used, are the same as those in the first step of Synthetic Method A1.

### Synthetic Method X10

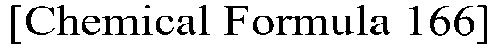

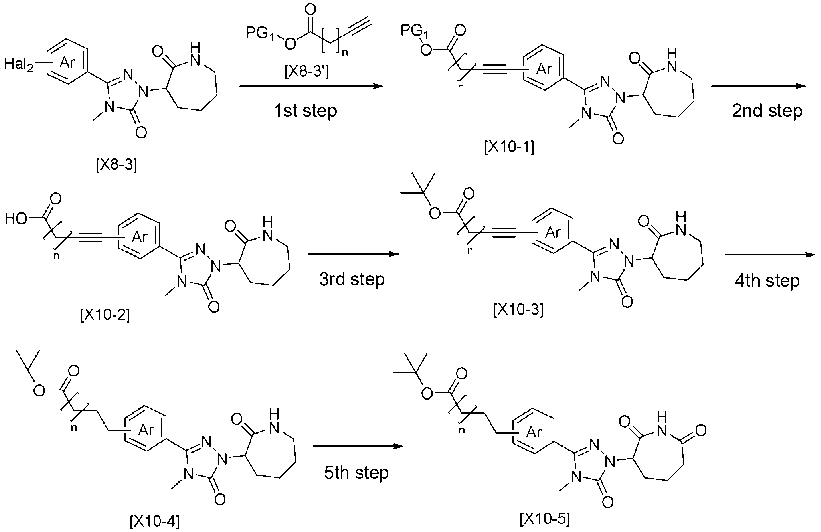

(wherein Hal₂ represents fluorine or chlorine, and n is 0, 1, 2, 3, 4, 5, or 6, and the other symbols have the same meaning as described above).

A compound of General Formula [X10-1] can be obtained by alkynylating a compound of General Formula [X8-3] with a compound of General Formula [X8-3'] in the presence of a palladium catalyst, a copper catalyst, and a base. The reaction temperature and time, as well as the reagents and solvents used, are the same as those in the fifth step of Synthetic Method X3.

A compound of General Formula [X10-2] can be obtained by hydrolyzing a compound of General Formula [X10-1]. The reaction temperature and time, as well as the reagents and solvents used, are the same as those in the sixth step of Synthetic Method X3.

A compound of General Formula [X10-3] can be obtained by tert-butyl esterification of a compound of General Formula [X10-2]. The reaction temperature and time, as well as the reagents and solvents used, are the same as those in the seventh step of Synthetic Method X3.

A compound of General Formula [X10-4] can be obtained by subjecting a compound of General Formula X10-3] to a reduction reaction under a hydrogen atmosphere in the presence of a catalyst. The reaction temperature and time, as well as the reagents and solvents used, are the same as those in the seventh step of Synthetic Method H‴.

A compound of General Formula [X10-5] can be obtained through an oxidation reaction of a compound of General Formula [X10-4]. The reaction temperature and time, as well as the reagents and solvents used, are the same as those in the first step of Synthetic Method A1.

### Synthetic Method X11

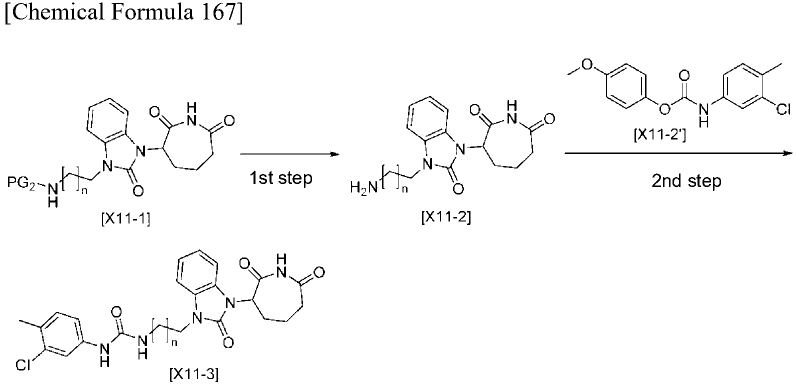

(wherein n is 0, 1, 2, 3, 4, 5, or 6, and the other symbols have the same meaning as described above).

A compound of General Formula [X11-2] can be obtained by deprotection of a compound of General Formula [X11-1]. The deprotection can be carried out using conventional methods such as reduction, hydrolysis, or acid treatment, depending on the type of protecting group to be removed. The reaction temperature and time, as well as the reagents and solvents used, are the same as those in the second step of Synthetic Method C.

A compound of General Formula [X11-3] can be obtained by reacting a compound of General Formula [X11-2] with a compound of General Formula [X11-2'] in the presence of a base. The reaction temperature and time, as well as the reagents and solvents used, are the same as those in the first step of Synthetic Method D1.

### Synthetic Method X12

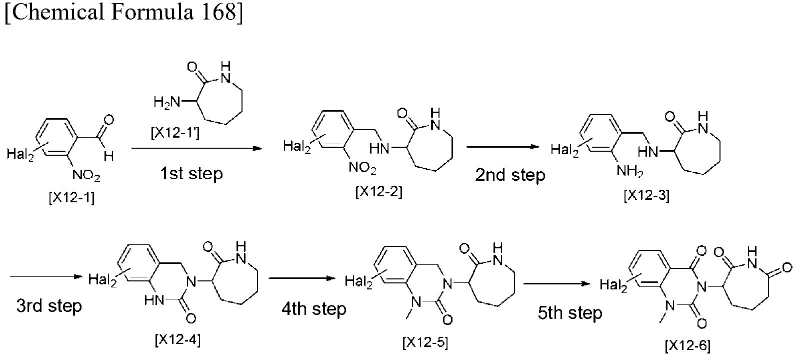

(wherein Hal₂ represents fluorine or chlorine, and the other symbols have the same meanings as described above)

A compound of General Formula [X12-2] can be obtained by reacting a compound of General Formula [X12-1] with a compound of General Formula [X12-1'] in the presence of a reducing agent. The reaction proceeds in an appropriate solvent, at a temperature ranging from 0 °C to the solvent reflux temperature. The reaction time varies depending on starting materials, solvent, and reaction temperature used, but is typically from 30 minutes to 72 hours. Examples of the reducing agent include sodium triacetoxyborohydride and the like. Examples of the solvent include dichloromethane and the like.

A compound of General Formula [X12-3] can be obtained by subjecting a compound of General Formula [X12-2] to a reduction reaction. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the reducing agent include tin(II) chloride and the like. Examples of the solvent include ethanol, and the like.

A compound of General Formula [X12-4] can be obtained by carbonylating a compound of General Formula [X12-3] in the presence of a base. The reaction temperature and time, as well as the reagents and solvents used, are the same as those in the third step of Synthetic Method C.

A compound of General Formula [X12-5] can be obtained by methylating a compound of General Formula [X12-4]. The reaction temperature and time, as well as the reagents and solvents used, are the same as those in the fourth step of Synthetic Method X3.

A compound of General Formula [X12-6] can be obtained through an oxidation reaction of a compound of General Formula [X12-5]. The reaction temperature and time, as well as the reagents and solvents used, are the same as those in the first step of Synthetic Method A1.

### Synthetic Method X13

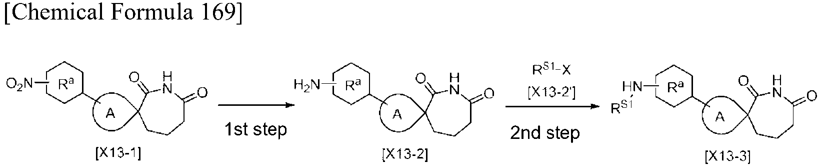

(wherein X is a leaving group, and the other symbols have the same meanings as described above)

A compound of General Formula [X13-2] can be obtained by subjecting a compound of General Formula [X13-1] to a reduction reaction under a hydrogen atmosphere in the presence of a catalyst. The reaction temperature and time, as well as the reagents and solvents used, are the same as those in the second step of Synthetic Method D'.

A compound of General Formula [X13-3] can be obtained by reacting a compound of General Formula [X13-2] with a compound of General Formula [X13-2'] in the presence of a base. The reaction temperature and time, as well as the reagents and solvents used, are the same as those in the first step of Synthetic Method D1.

### Synthetic Method X14

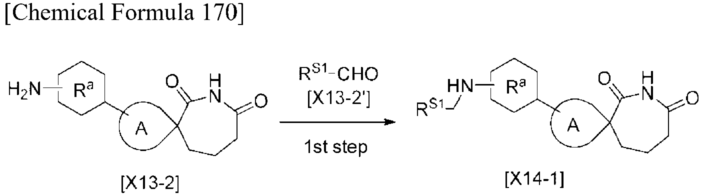

(wherein the symbols have the same meaning as described above)

A compound of General Formula [X14-1] can be obtained by reacting a compound of General Formula [X13-2] with a compound of General Formula [X13-2'] in the presence of a reducing agent. The reaction temperature and time, as well as the reagents and solvents used, are the same as those in the first step of Synthetic Method X12.

### Synthetic Method X15

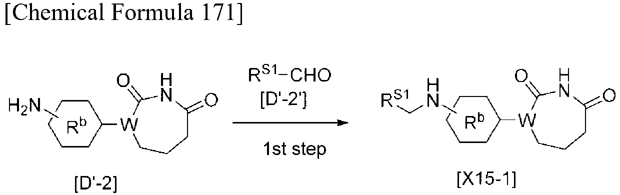

(wherein the symbols have the same meaning as described above)

A compound of General Formula [X15-1] can be obtained by reacting a compound of General Formula [D'-2] with a compound of General Formula [D'-2'] in the presence of a reducing agent. The reaction temperature and time, as well as the reagents and solvents used, are the same as those in the first step of Synthetic Method X12.

### Synthetic Method X16

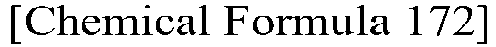

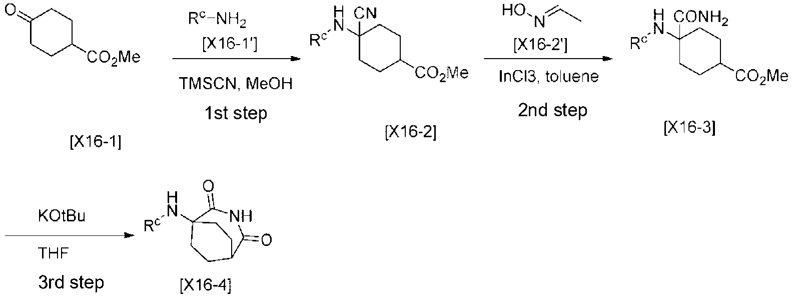

(wherein the symbols have the same meaning as described above)

A compound of General Formula [X16-2] can be obtained by reacting a compound of General Formula [X16-1] and a compound of General Formula [X16-1'] with a cyanation reagent. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the cyanation reagent include cyanotrimethylsilane and the like. Examples of the solvent include methanol and the like.

A compound of General Formula [X16-3] can be obtained by reacting a compound of General Formula [X16-2] and a compound of General Formula [X16-2'] with acetaldoxime in the presence of a catalyst. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the catalyst include indium trichloride and the like. Examples of the solvent include toluene and the like.

A compound of General Formula [X16-4] can be obtained by reacting a compound of General Formula [X16-3] in the presence of a base. The reaction proceeds in an appropriate solvent, typically at a temperature ranging from under ice-cooling to the solvent reflux temperature. The reaction time varies depending on starting materials, solvent, and reaction temperature used, but is typically from 0.5 hours to 24 hours. Examples of the base include potassium tert-butoxide and the like. Examples of the solvent include tetrahydrofuran and the like.

### Synthetic Method X17

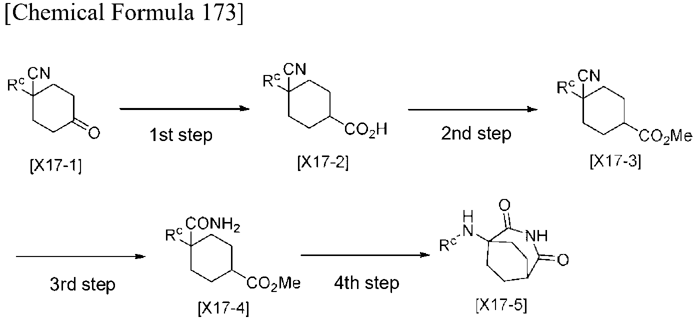

(wherein the symbols have the same meaning as described above)

A compound of General Formula [X17-2] can be derived from a compound of General Formula [X17-1] according to a commonly known method (for example, J. Org. Chem. 2002, 67, 5440).

A compound of General Formula [X17-3] can be derived from a compound of General Formula [X17-2] according to a commonly known method (for example, Org. Lett. 2021, 23(5), 1566).

A compound of General Formula [X17-4] can be obtained by reacting a compound of General Formula [X17-3]. The reaction temperature and time, as well as the reagents and solvents used, are the same as those in the second step of Synthetic Method X16 .

A compound of General Formula [X17-5] can be obtained by reacting a compound of General Formula [X17-4]. The reaction temperature and time, as well as the reagents and solvents used, are the same as those in the third step of Synthetic Method X16 .

### Synthetic Method X18

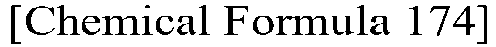

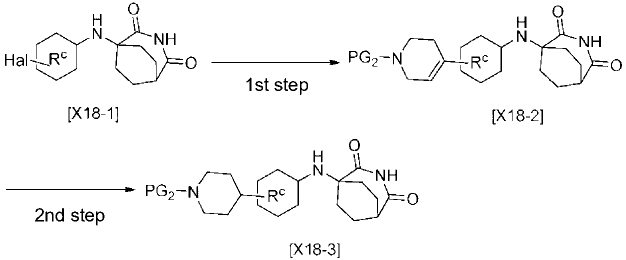

(wherein the symbols have the same meaning as described above)

A compound of General Formula [X18-2] can be derived from a compound of General Formula [X18-1] according to a commonly known method (for example, European Journal of Medicinal Chemistry (2019), 177, 316-337).

A compound of General Formula [X18-3] can be obtained by subjecting a compound of General Formula [X18-2] to a catalytic reduction reaction under a hydrogen atmosphere. The reaction temperature and time, as well as the reagents and solvents used, are the same as those in the first step of Synthetic Method B1.

### Synthetic Method X19

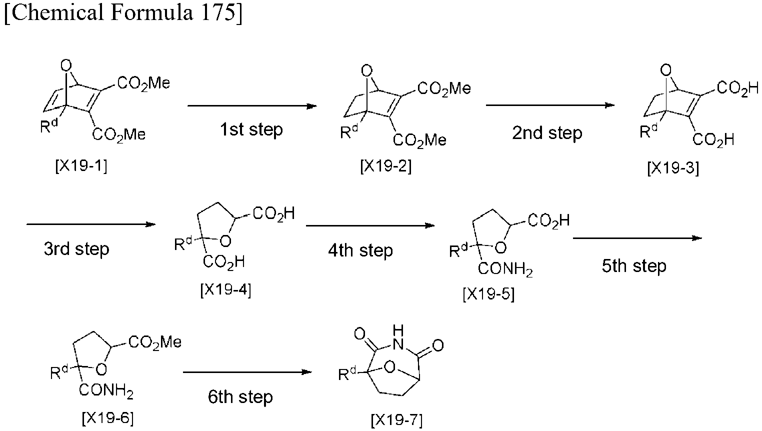

### (wherein the symbols have the same meaning as described above)

A compound of General Formula [X19-2] can be obtained by subjecting a compound of General Formula [X19-1] to a catalytic reduction reaction under a hydrogen atmosphere. The reaction temperature and time, as well as the reagents and solvents used, are the same as those in the first step of Synthetic Method B1.

A compound of General Formula [X19-3] can be obtained by subjecting a compound of General Formula [X19-2] to basic or acidic conditions. The reaction temperature and time, as well as the reagents and solvents used, are the same as those in the third step of Synthetic Method A.

A compound of General Formula [X19-4] can be derived from a compound of General Formula [X19-3] according to a commonly known method (for example, Heterocycles (1993), 35 (1), 129-34).

A compound of General Formula [X19-5] can be obtained through a dehydration reaction of a compound of General Formula [X19-4] followed by a reaction with ammonia. The reaction temperature and time, as well as the reagents and solvents used, are the same as those in the second step of Synthetic Method P.

A compound of General Formula [X19-6] can be derived from a compound of General Formula [X19-5] according to a commonly known method (for example, Organic Letters (2021), 23 (5), 1566-1571).

A compound of General Formula [X19-7] can be obtained by reacting a compound of General Formula [X19-6] in the presence of a base. The reaction temperature and time, as well as the reagents and solvents used, are the same as those in the third step of Synthetic Method X16 .

### Synthetic Method X20

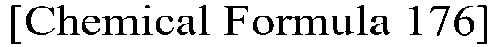

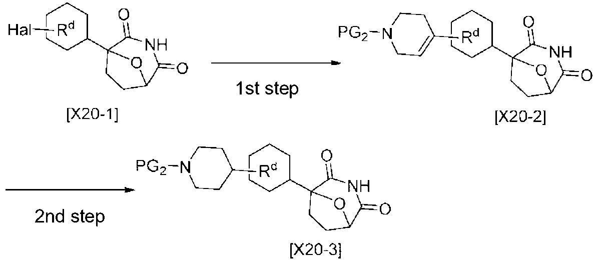

(wherein the symbols have the same meaning as described above)

A compound of General Formula [X20-2] can be derived from a compound of General Formula [X20-1] according to a commonly known method (for example, European Journal of Medicinal Chemistry (2019), 177, 316-337).

A compound of General Formula [X20-3] can be obtained by subjecting a compound of General Formula [X20-2] to a catalytic reduction reaction under a hydrogen atmosphere. The reaction temperature and time, as well as the reagents and solvents used, are the same as those in the first step of Synthetic Method B1.

### Synthetic Method X20'

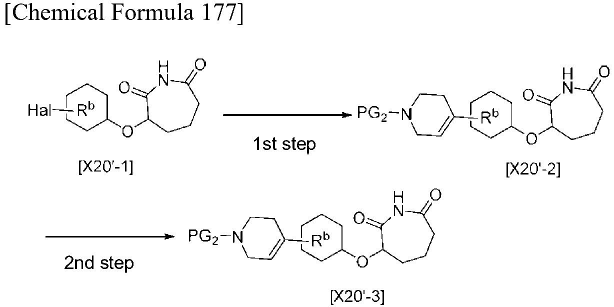

(wherein the symbols have the same meaning as described above)

A compound of General Formula [X20'-2] can be derived from a compound of General Formula [X20'-1] according to a commonly known method (for example, European Journal of Medicinal Chemistry (2019), 177, 316-337).

A compound of General Formula [X20 '-3] can be obtained by subjecting a compound of General Formula [X20 '-2] to a catalytic reduction reaction under a hydrogen atmosphere. The reaction temperature and time, as well as the reagents and solvents used, are the same as those in the first step of Synthetic Method B1.

### Synthetic Method X21

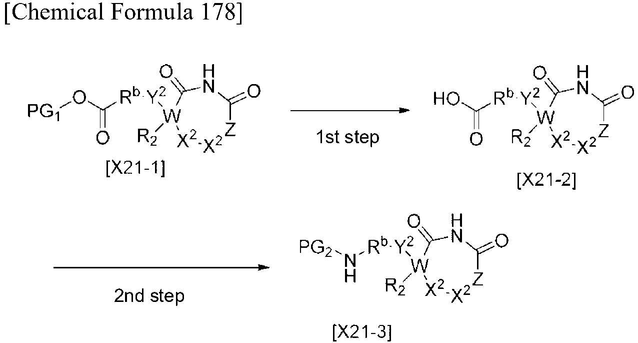

(wherein the symbols have the same meaning as described above)

A compound of General Formula [X21-2] can be obtained by subjecting a compound of General Formula [X21-1] to basic or acidic conditions. The reaction temperature and time, as well as the reagents and solvents used, are the same as those in the third step of Synthetic Method A.

A compound of General Formula [X21-3] can be derived from a compound of General Formula [X21-2] according to a commonly known method (for example, Tetrahedron: Asymmetry (1997), 8 (6), 883-887).

### Synthetic Method X22

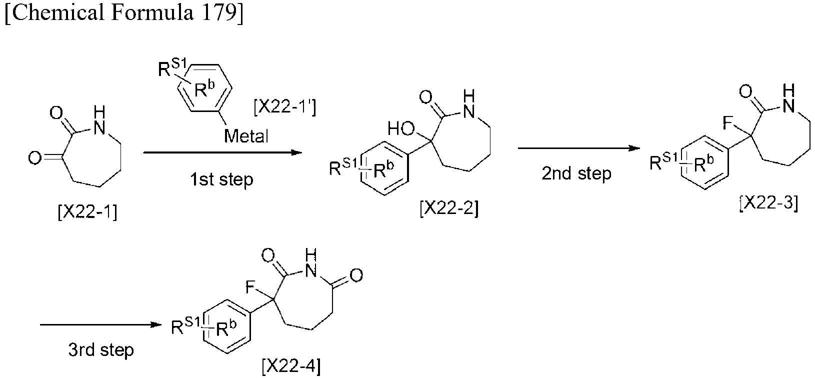

(wherein Metal is magnesium or lithium, and the other symbols have the same meanings as described above)

A compound of General Formula [X22-2] can be obtained by reacting a compound of General Formula [X22-1] with a compound of General Formula [X22-1']. The reaction proceeds in an appropriate solvent, typically at a temperature ranging from -78 °C to room temperature. The reaction time varies depending on starting materials, solvent, and reaction temperature used, but is typically from 0.5 hours to 24 hours. Examples of the solvent include tetrahydrofuran and the like.

A compound of General Formula [X22-3] can be derived from a compound of General Formula [X22-2] according to a commonly known method (for example, Journal of Fluorine Chemistry (2023), 268, 110134).

A compound of General Formula [X22-4] can be obtained through an oxidation reaction of a compound of General Formula [X22-3]. The reaction temperature and time, as well as the reagents and solvents used, are the same as those in the first step of Synthetic Method A1.

### Synthetic Method X22'

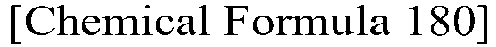

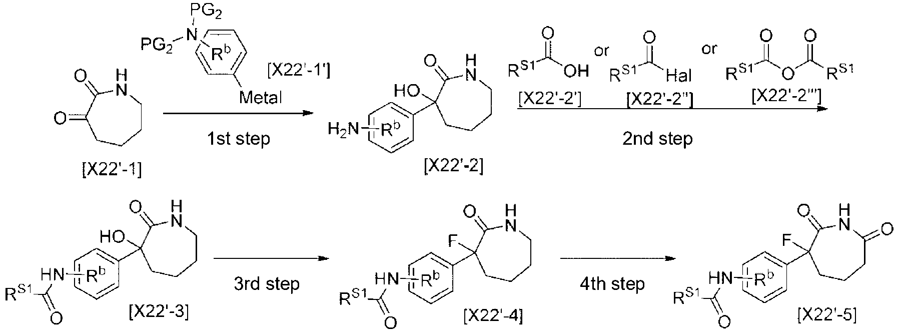

(wherein Metal is magnesium or lithium, and the other symbols have the same meanings as described above)

A compound of General Formula [X22'-2] can be obtained by reacting a compound of General Formula [X22'-1] with a compound of General Formula [X22'-1']. The reaction temperature and time, as well as the reagents and solvents used, are the same as those in the first step of Synthetic Method X22.

A compound of General Formula [X22'-3] can be obtained using a compound of General Formula [X22'-2'] or General Formula [X22'-2''] using a method similar to the first step of Synthetic Method D. Alternatively, it can be obtained by reacting a compound of General Formula [X22-2‴] in the presence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include triethylamine, diisopropylethylamine, and the like. Examples of the solvent include acetonitrile, N,N-dimethylformamide, and the like.

A compound of General Formula [X22'-4] can be derived from a compound of General Formula [X22-2] according to a commonly known method (for example, Journal of Fluorine Chemistry (2023), 268, 110134).

A compound of General Formula [X22'-5] can be obtained through an oxidation reaction of a compound of General Formula [X22'-4]. The reaction temperature and time, as well as the reagents and solvents used, are the same as those in the first step of Synthetic Method A1.

### Synthetic Method X23

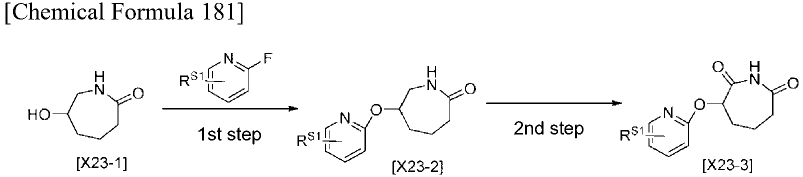

(wherein the symbols have the same meaning as described above)

A compound of General Formula [X23-2] can be obtained by reacting a compound of General Formula [X23-1] with a 2-fluoropyridine derivative in the presence of a base. The reaction proceeds in an appropriate solvent, typically at a temperature ranging from room temperature to the solvent reflux temperature. The reaction time varies depending on starting materials, solvent, and reaction temperature used, but is typically from 0.5 hours to 24 hours. Examples of the base include potassium tert-butoxide and the like. Examples of the solvent include acetonitrile, N,N-dimethylformamide, and the like.

A compound of General Formula [X23-3] can be obtained through an oxidation reaction of a compound of General Formula [X23-2]. The reaction temperature and time, as well as the reagents and solvents used, are the same as those in the first step of Synthetic Method A1.

### Synthetic Method X24

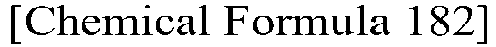

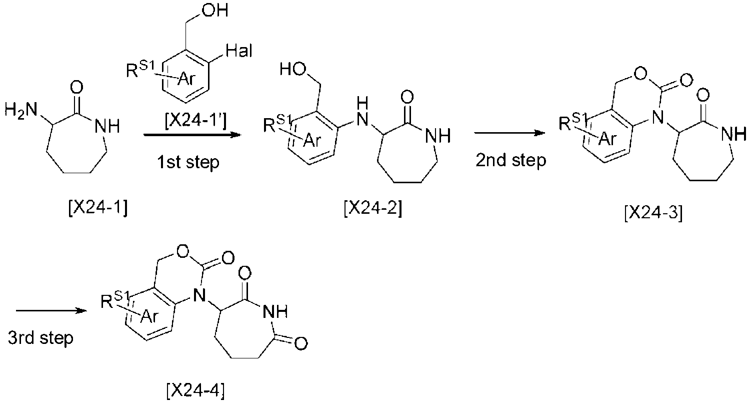

(wherein the symbols have the same meaning as described above)

A compound of General Formula [X24-2] can be obtained by reacting a compound of General Formula [X24-1] with a compound of General Formula [X24-1'] in the presence of a base. The reaction temperature and time, as well as the reagents and solvents used, are the same as those in the first step of Synthetic Method D1.

A compound of General Formula [X24-3] can be obtained by carbonylating a compound of General Formula [X24-2] in the presence of a base. The reaction temperature and time, as well as the reagents and solvents used, are the same as those in the third step of Synthetic Method C. A compound of General Formula [X24-4] can be obtained through an oxidation reaction of a compound of General Formula [X24-3]. The reaction temperature and time, as well as the reagents and solvents used, are the same as those in the first step of Synthetic Method A1.

### Synthetic Method X25

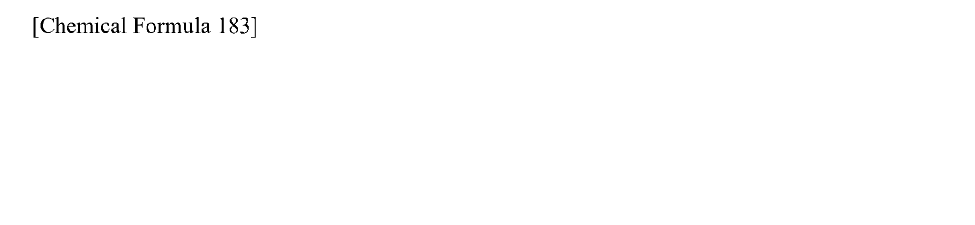

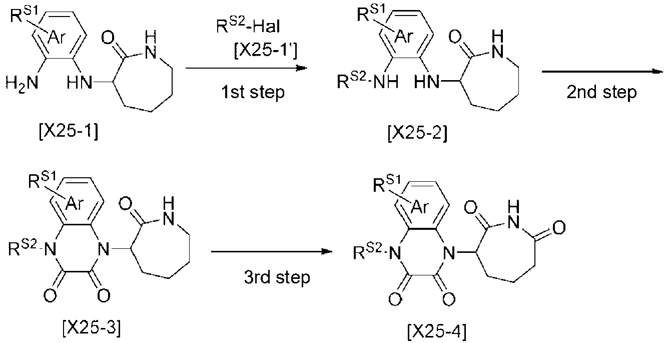

(wherein the symbols have the same meaning as described above)

A compound of General Formula [X25-2] can be obtained by reacting a compound of General Formula [X25-1] with a compound of General Formula [X25-1'] in the presence of a base. The reaction temperature and time, as well as the reagents and solvents used, are the same as those in the first step of Synthetic Method D1.

A compound of General Formula [X25-3] can be derived from a compound of General Formula [X25-2] according to a commonly known method (for example, US20220119356).

A compound of General Formula [X25-4] can be obtained through an oxidation reaction of a compound of General Formula [X25-3]. The reaction temperature and time, as well as the reagents and solvents used, are the same as those in the first step of Synthetic Method A1.

### Synthetic Method X26

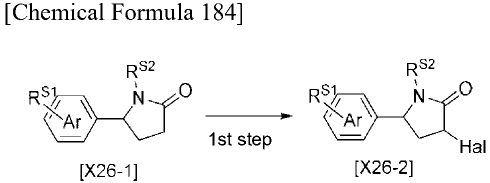

(wherein the symbols have the same meaning as described above)

A compound of General Formula [X26-2] can be derived from a compound of General Formula [X26-1] according to a commonly known method (for example, Journal of Physical Chemistry A (2015), 119 (10), 2111-2121).

### Synthetic Method X26'

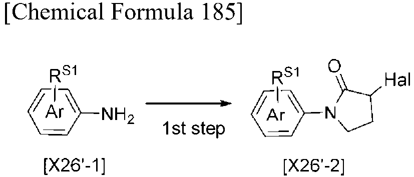

(wherein the symbols have the same meaning as described above)

A compound of General Formula [X26'-2] can be derived from a compound of General Formula [X26'-1] according to a commonly known method (for example, Journal of Medicinal Chemistry (2015), 58 (6), 2809-2820).

### Synthetic Method X27

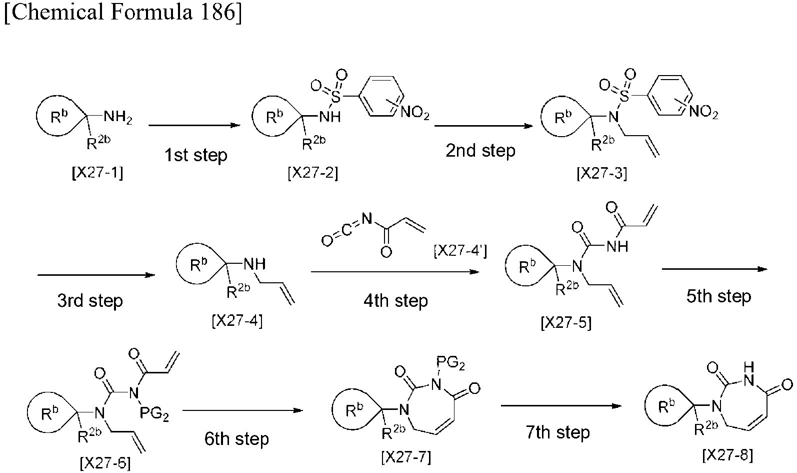

(wherein the symbols have the same meaning as described above)

A compound of General Formula [X27-2] can be derived from a compound of General Formula [X27-1] according to a commonly known method (for example, the method described in US5190975).

A compound of General Formula [X27-3] can be derived from a compound of General Formula [X27-2] according to a commonly known method (for example, the method described in WO2021/175848).

A compound of General Formula [X27-4] can be derived from a compound of General Formula [X27-3] according to a commonly known method (for example, the method described in WO2021/175848).

A compound of General Formula [X27-8] can be derived from a compound of General Formula [X27-4] according to the synthetic method for preparing a compound of General Formula [C" -6] from a compound of General Formula [C"-1] in Synthetic Method C".

A method for producing, among the above compounds, particularly a compound of Formula (I), Formula (II), Formula (III), or Formula (IV), substituted with L substituted with TBL, that is, a compound represented by the following Formula (I-1): 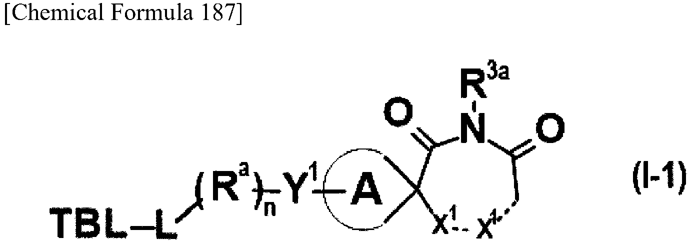
[wherein the symbols have the same meanings as described above,
Y¹ or R^{a} is substituted with L substituted with TBL],
or Formula (II-1): 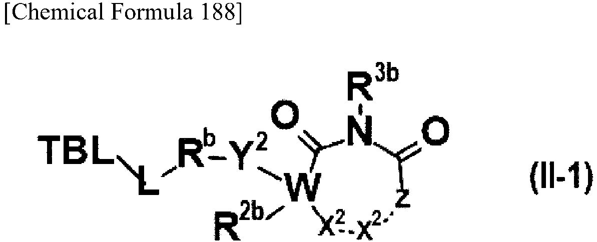
[wherein the symbols have the same meanings as described above,
R^{b} is substituted with L substituted with TBL],
or Formula (III-1): 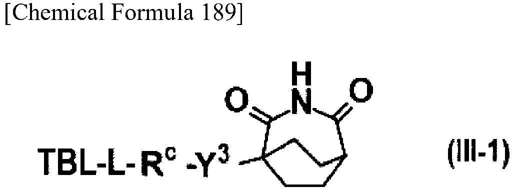
[wherein the symbols have the same meanings as described above,
R^{c} is substituted with L substituted with TBL],
or Formula (IV-1): 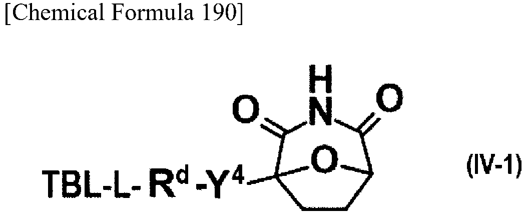
[wherein the symbols have the same meanings as described above,
R^{d} is substituted with L substituted with TBL]
or a pharmacologically acceptable salt thereof, constitutes a third embodiment of the present invention. Examples of methods for producing a compound represented by Formula (I-1), Formula (II-1), Formula (III-1), or Formula (IV-1), or a pharmacologically acceptable salt thereof, include the following first production method and second production method.

That is, the first production method of a compound represented by Formula (I-1), Formula (II-1), Formula (III-1), or Formula (IV-1), or a pharmaceutically acceptable salt thereof, is, as an aspect (52), a method for producing a compound represented by the following Formula (I-1): 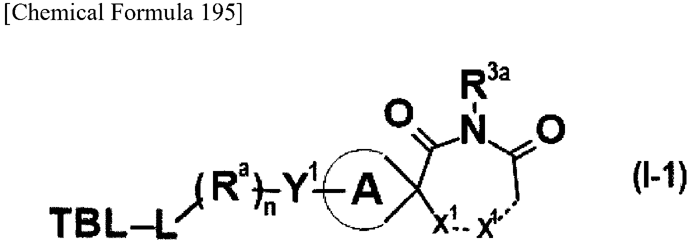
[wherein the symbols have the same meanings as described above,
Y¹ or R^{a} is substituted with L substituted with TBL],
or Formula (II-1): 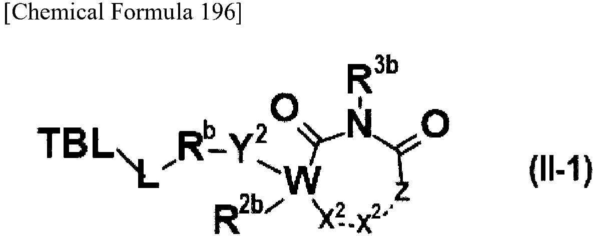
[wherein the symbols have the same meanings as described above,
R^{b} is substituted with L substituted with TBL],
or Formula (III-1): 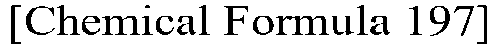 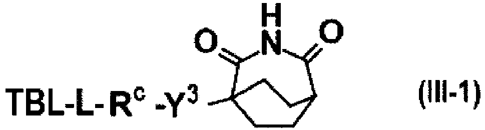
[wherein the symbols have the same meanings as described above,
R^{c} is substituted with L substituted with TBL],
or Formula (IV-1): 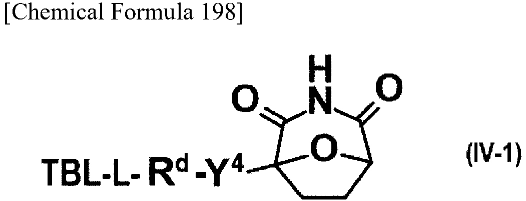
[wherein the symbols have the same meanings as described above,
R^{d} is substituted with L substituted with TBL]
or a pharmacologically acceptable salt thereof, the method comprising:
reacting a compound represented by the following Formula (I'): 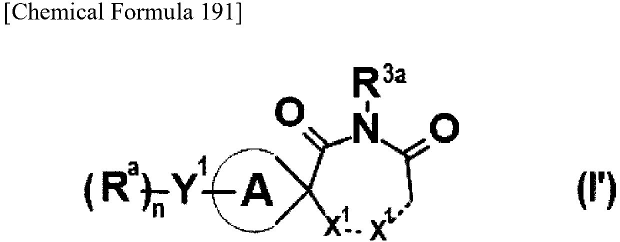
[wherein dotted lines, Ring A, X¹, Y¹, R^{3a}, R^{a}, and n have the same meanings as defined in Formula (I) in the above aspect (1), provided that Y¹ or R^{a} is not substituted with L having a first terminal group or with substituted L],
or the following Formula (II'): 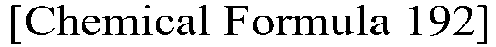 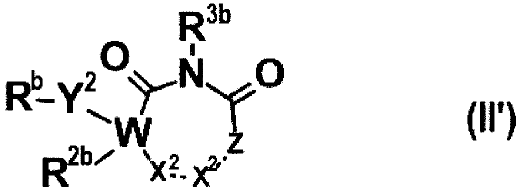
[wherein dotted lines, X², Y², Z, W, R^{b}, R^{2b} and R^{3b} have the same meanings as defined in Formula (II) in the above aspect (1), provided that R^{b} is not substituted with L having a first terminal group or with substituted L],
or the following Formula (III'): 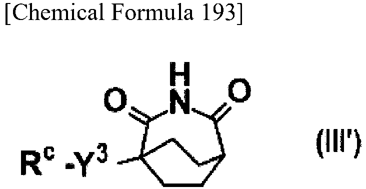
[wherein
Y³ and R^{c} have the same meanings as defined in Formula (III) in the above aspect (1), provided that R^{c} is not substituted with L having a first terminal group or with substituted L],
or the following Formula (IV'): 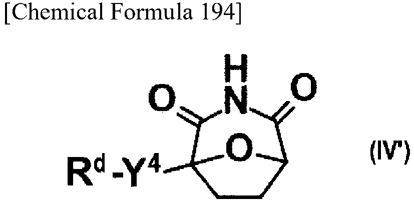
[wherein
Y⁴ and R^{d} have the same meanings as defined in Formula (IV) in the above aspect (1), provided that R^{d} is not substituted with L having a first terminal group or with substituted L],
with a TBL-L compound
[wherein
the TBL-L compound is a compound in which L having a second terminal group is bonded to TBL,
L has the same meaning as defined in the above aspect (35), and
TBL has the same meaning as defined in the above aspect (44)]
in a solvent, in the presence or absence of a base.

In a TBL-L compound, L having a second terminal group may be present in one or more (for example, 1 to 3) instances at any substitutable position of TBL.

The second terminal group in a compound formed by bonding L having a second terminal group to TBL is a group capable of forming a chemical bond by reacting with a compound of Formula (I'), Formula (II'), Formula (III'), or Formula (IV'), or more specifically, with a group, substituent, or ring-constituting atom present in Y¹, Ra, Rb, Rc, and Rd, and is bonded to a linker main chain (may be denoted as "L_{M}"). The linker main chain L_{M} can be, for example, L₂ in the above aspect (36), L_{b2} in the above aspect (37), a group represented by Formula (V) in the above aspect (38), or each group in the above aspects (39) to (43). Examples of the second terminal group include: 1) hydrogen atom, 2) halogen atom, 3) -CN, 4) -NO₂, 5) -SF₅, 6) -CO₂H, 7) - N(R^{L2}R^{L2}'), 8) -A^{L2}R^{L2}, 9) optionally substituted alkyl group, 10) optionally substituted cycloalkyl group, 11) optionally substituted hetero-saturated ring group, 12) optionally substituted aryl group, 13) optionally substituted heteroaryl group, 14) -SO₂R^{L2}, 15) - P(O)(OR^{L2})OR^{L2}', 16) -C≡CR^{L2}, 17) -C(R^{L2})=C(R^{L2}'R^{L2}"), 18) -COR^{L2}, 19) -CON(R^{L2}R^{L2}'), 20) -SO₂N(R^{L2}R^{L2}'), 21) -N(R^{L2})CON(R^{L2}'R^{L2}"), or 22) -N(R^{L2})SO₂N(R^{L2}'R^{L2}") (wherein, A^{L2} represents an oxygen atom or a sulfur atom, and R^{L2}, R^{L2}', and R^{L2}" each independently represent: a) a hydrogen atom, b) an optionally substituted C₁-C₈ alkyl group, c) an optionally substituted C₃-C₈ cycloalkyl group, or d) an ptionally substituted C₁-C₈ alkoxy group (for example, methoxy, ethoxy, tert-butoxy, benzyloxy)). The second terminal group can be appropriately selected depending on the type of group, substituent, or ring-constituting atom that is to be reacted and is present in Y¹, R^{a}, R^{b}, R^{c}, or R^{d} of a compound of Formula (I'), Formula (II'), Formula (III'), or Formula (IV').

The above first production method corresponds to, for example, the second step of General Synthetic Method T, the second step of Synthetic Method T', the first step of Synthetic Method V, and the first step of Synthetic Method V'.

In the step of reacting a compound of Formula (I'), Formula (II'), Formula (III'), or Formula (IV') with a TBL-L compound in the above production method, examples of the solvent include, but are not limited to, alcohols such as methanol; amides such as N,N-dimethylformamide; chlorine-based solvents such as chloroform and dichloromethane; and ethers such as tetrahydrofuran. Examples of the base include, but are not limited to, amines such as triethylamine, N,N-diisopropylethylamine, and pyridine. In the above step, a condensing agent may be used, and examples of the condensing agent include, but are not limited to, 1-ethyl-3-(3-dimethylaminopropyl)carbodiimide hydrochloride (WSC·HCl), 1-[bis(dimethylamino)methylene]-1H-1,2,3-triazolo[4,5-b]pyridinium 3-oxide hexafluorophosphate (HATU), 4-(4,6-dimethoxy[1.3.5]triazin-2-yl)-4-methylmorpholinium chloride hydrate (DMT-MM), and 2-chloro-1-methylpyridinium iodide.

The second production method of a compound represented by Formula (I-1), Formula (II-1), Formula (III-1), or Formula (IV-1), or a pharmaceutically acceptable salt thereof, is, as an aspect (53), a method for producing a compound represented by the following Formula (I-1): 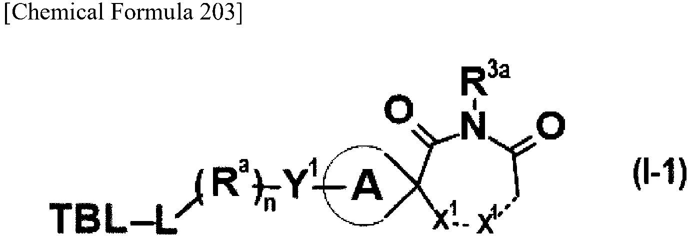
[wherein the symbols have the same meanings as described above,
Y¹ or R^{a} is substituted with L substituted with TBL],
or Formula (II-1): 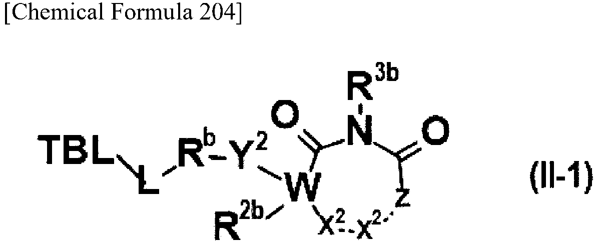
[wherein the symbols have the same meanings as described above,
R^{b} is substituted with L substituted with TBL],
or Formula (III-1): 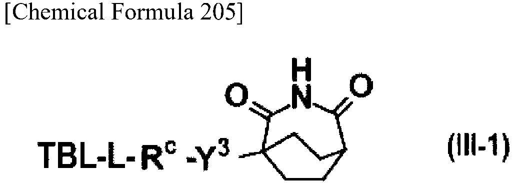
[wherein the symbols have the same meanings as described above,
R^{c} is substituted with L substituted with TBL],
or Formula (IV-1): 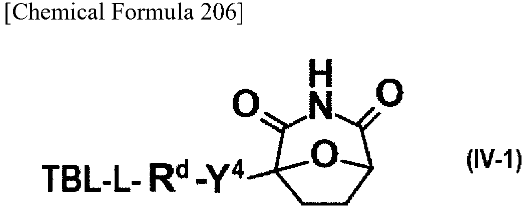
[wherein the symbols have the same meanings as described above,
R^{d} is substituted with L substituted with TBL]
or a pharmacologically acceptable salt thereof, the method comprising:
   reacting a compound represented by the following Formula (I"): 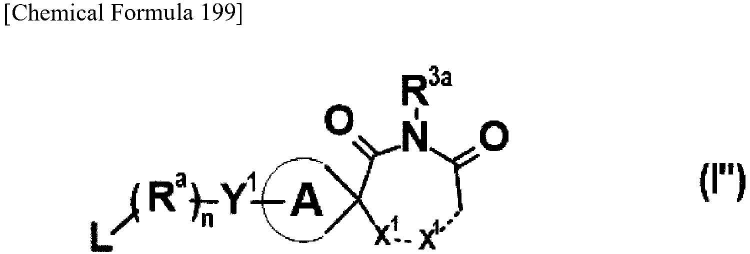
   [wherein dotted lines, Ring A, X¹, Y¹, R^{3a}, R^{a} and n have the same meanings as defined in Formula (I) in the above aspect (1),
   Y¹ or R^{a} is substituted with L having a first terminal group,
   L has the same meaning as defined in the above aspect (35)],
   or the following Formula (II"): 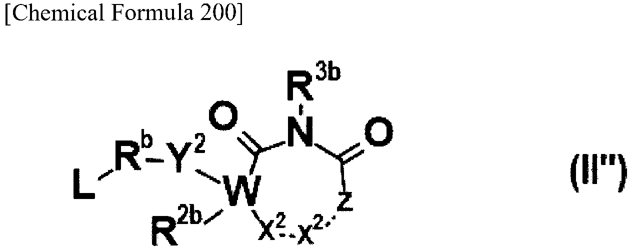
   [wherein dotted lines, X², Y², Z, W, R^{b}, R^{2b} and R^{3b} have the same meanings as defined in Formula (II) in the above aspect (1),
   R^{b} is substituted with L having a first terminal group,
   L has the same meaning as defined in the above aspect (35)],
   or the following Formula (III"): 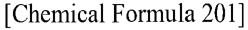 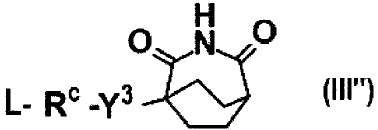
   [wherein Y³ and R^{c} have the same meanings as defined as in Formula (III) in Claim 1,
   R^{c} is substituted with L having a first terminal group,
   L has the same meaning as defined in the above aspect (35)],
   or the following Formula (IV"): 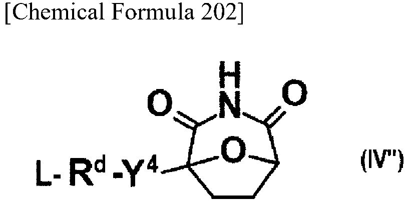
   [wherein Y⁴ and R^{d} have the same meanings as defined as in Formula (IV) in Claim 1,
   R^{d} is substituted with L having a first terminal group,
   L has the same meaning as defined in the above aspect (35)],
   with a TBL compound
   [wherein, the TBL compound is a compound in which TBL is bonded to a monovalent chemical group, and TBL has the same meaning as defined in the above aspect (46)]
   in a solvent, in the presence or absence of a base.

In Formula (I"), Formula (II"), Formula (III"), or Formula (IV"), L having a first terminal group may be present in one or more (for example, 1 to 3) instances at any substitutable position of each optionally substituted group represented by Y¹, R^{a}, R^{b}, R^{c}, or R^{d}.

The monovalent chemical group in the TBL compound is a group capable of forming a chemical bond by reacting with the first terminal group of L having a first terminal group. Examples of the monovalent chemical group include: 1) hydrogen atom, 2) halogen atom, 3) -CN, 4) -NO₂, 5) -SF₅, 6) -CO₂H, 7) -N(R^{L2}R^{L2}'), 8) -A^{L2}R^{L2}, 9) optionally substituted alkyl group, 10) optionally substituted cycloalkyl group, 11) optionally substituted heterosaturated ring group, 12) optionally substituted aryl group, 13) optionally substituted heteroaryl group, 14) -SO₂R^{L2}, 15) - P(O)(OR^{L2})OR^{L2}', 16) -C≡CR^{L2}, 17) -C(R^{L2})=C(R^{L2}'R^{L2}"), 18) -COR^{L2}, 19) -CON(R^{L2}R^{L2}'), 20) -SO₂N(R^{L2}R^{L2}'), 21) -N(R^{L2})CON(R^{L2}'R^{L2}"), and 22) -N(R^{L2})SO₂N(R^{L2}'R^{L2}") (wherein, A^{L2} represents an oxygen atom or a sulfur atom, and R^{L2}, R^{L2}', and R^{L2}" each independently represent: a) a hydrogen atom, b) an optionally substituted C₁-C₈ alkyl group, c) an optionally substituted C₃-C₈ cycloalkyl group, or d) an optionally substituted C₁-C₈ alkoxy group (for example, methoxy, ethoxy, tert-butoxy, benzyloxy)). The first terminal group of L having a first terminal group has the same meaning as described above.

The above second production method corresponds to, for example, the fourth step of General Synthetic Method U, the fourth step of Synthetic Method U', the third step of Synthetic Method W, and the third step of Synthetic Method W'.

In the step of reacting a compound of Formula (I"), Formula (II"), Formula (III"), or Formula (IV") with a TBL compound in the above second production method, examples of the solvent include, but are not limited to, alcohols such as methanol; amides such as N,N-dimethylformamide; chlorine-based solvents such as chloroform and dichloromethane; and ethers such as tetrahydrofuran. Examples of the base include, but are not limited to, amines such as triethylamine, N,N-diisopropylethylamine, and pyridine. In the above step, a condensing agent may be used, and examples of the condensing agent include, but are not limited to, 1-ethyl-3-(3-dimethylaminopropyl)carbodiimide hydrochloride (WSC·HCl), 1-[bis(dimethylamino)methylene]-1H-1,2,3-triazolo[4,5-b]pyridinium 3-oxide hexafluorophosphate (HATU), 4-(4,6-dimethoxy[1.3.5]triazin-2-yl)-4-methylmorpholinium chloride hydrate (DMT-MM), and 2-chloro-1-methylpyridinium iodide.

In the present specification, a starting compound, before a chemical linker reacts with a TBL compound and a compound of Formula (I'), Formula (II'), Formula (III') or Formula (IV'), more specifically with a group, substituent or ring-constituent atom present in Y¹, Ra, Rb, Rc or R^{d}, is referred to as a linker compound. The linker compound has a structure of first terminal group-linker main chain L_{M}-second terminal group or second terminal group-linker main chain L_{M}-first terminal group. The first terminal group is a group capable of forming a chemical bond with a TBL compound. The second terminal group is a group capable of forming a chemical bond by reacting with a compound of Formula (I'), Formula (II'), Formula (III'), or Formula (IV'), or more specifically, with a group, substituent, or ring-constituting atom present in Y¹, R^{a}, R^{b}, R^{c}, or R^{d}. Examples of the first terminal group and the second terminal group include: 1) hydrogen atom, 2) halogen atom, 3) -CN, 4) -NO₂, 5) -SF₅, 6) -CO₂H, 7) -N(R^{L2}R^{L2}'), 8) -A^{L2}R^{L2}, 9) optionally substituted alkyl group, 10) optionally substituted cycloalkyl group, 11) optionally substituted heterosaturated ring group, 12) optionally substituted aryl group, 13) optionally substituted heteroaryl group, 14) -SO₂R^{L2}, 15) -P(O)(OR^{L2})OR^{L2}', 16) -C≡CR^{L2}, 17) - C(R^{L2})=C(R^{L2}'R^{L2}"), 18) -COR^{L2}, 19) -CON(R^{L2}R^{L2}'), 20) -SO₂N(R^{L2}R^{L2}'), 21) - N(R^{L2})CON(R^{L2}'R^{L2}"), and 22) -N(R^{L2})SO₂N(R^{L2}'R^{L2}") (wherein, A^{L2} represents an oxygen atom or a sulfur atom, and R^{L2}, R^{L2}', and R^{L2}" each independently represent: a) a hydrogen atom, b) an optionally substituted C₁-C₈ alkyl group, c) an optionally substituted C₃-C₈ cycloalkyl group, or d) an optionally substituted C₁-C₈ alkoxy group (for example, methoxy, ethoxy, tert-butoxy, benzyloxy)). The linker compound may have a protecting group for a functional group.

A TBL-L compound can be produced using a method that includes reacting a linker compound with a TBL compound [wherein the TBL compound is a compound in which a monovalent chemical group is bonded to TBL, and TBL has the same meaning as defined in the above aspect (46)] in a solvent, in the presence or absence of a base.

Examples of the solvent include, but are not limited to, alcohols such as methanol; amides such as N,N-dimethylformamide; chlorine-based solvents such as chloroform and dichloromethane; and ethers such as tetrahydrofuran. Examples of the base include, but are not limited to, amines such as triethylamine, N,N-diisopropylethylamine, and pyridine. In the above step, a condensing agent may be used, and examples of the condensing agent include, but are not limited to, 1-ethyl-3-(3-dimethylaminopropyl)carbodiimide hydrochloride (WSC·HCl), 1-[bis(dimethylamino)methylene]-1H-1,2,3-triazolo[4,5-b]pyridinium 3-oxide hexafluorophosphate (HATU), 4-(4,6-dimethoxy[1.3.5]triazin-2-yl)-4-methylmorpholinium chloride hydrate (DMT-MM), and 2-chloro-1-methylpyridinium iodide.

The first terminal group of the linker compound can react at one or more (for example, 1 to 3) any substitutable positions in the TBL compound.

A compound of Formula (I"), Formula (II"), Formula (III"), or Formula (IV") can be produced using a method that includes reacting a compound of Formula (I'), Formula (II'), Formula (III'), or Formula (IV') with a linker compound in a solvent, in the presence or absence of a base. Examples of the solvent include, but are not limited to, alcohols such as methanol; amides such as N,N-dimethylformamide; chlorine-based solvents such as chloroform and dichloromethane; and ethers such as tetrahydrofuran. Examples of the base include, but are not limited to, amines such as triethylamine, N,N-diisopropylethylamine, and pyridine. In the above step, a condensing agent may be used, and examples of the condensing agent include, but are not limited to, 1-ethyl-3-(3-dimethylaminopropyl)carbodiimide hydrochloride (WSC·HCl), 1-[bis(dimethylamino)methylene]-1H-1,2,3-triazolo[4,5-b]pyridinium 3-oxide hexafluorophosphate (HATU), 4-(4,6-dimethoxy[1.3.5]triazin-2-yl)-4-methylmorpholinium chloride hydrate (DMT-MM), and 2-chloro-1-methylpyridinium iodide.

The second terminal group of the linker compound can react at one or more (for example, 1 to 3) any substitutable positions of each optionally substituted group represented by Y¹, R^{a}, R^{b}, R^{c}, or R^{d} in Formula (I'), Formula (II'), Formula (III'), or Formula (IV').

The starting compounds in the above methods can be produced using known methods and/or methods similar to those described in examples to be described later.

Introduction of a protecting group to a functional group and removal of a protecting group of a functional group can be carried out with reference to a known method (such as PROTECTIVE GROUPS in ORGANIC SYNTHESIS (by Theodora W. Greene, Peter G. M. Wuts)).

Further, the compounds of the present embodiment, and intermediate compounds, produced using the above methods can be structurally converted into other target compounds or intermediates using methods described in the examples below and/or known methods or combinations thereof.

A compound represented by Formula (I), Formula (II), Formula (III), or Formula (IV) produced using the methods described above can be purified to any desired purity by conventional purification means, such as concentration, extraction, chromatography, reprecipitation, or recrystallization. Further, when necessary, the compound can be converted into a pharmacologically acceptable salt by treatment with an acid or a base in an appropriate solvent (such as water, alcohol, or ether). Further, an obtained compound of the present invention or a pharmacologically acceptable salt thereof can be converted into a hydrate or solvate by treatment with water, an aqueous solvent, or other solvents.

The compounds of the above embodiment of the present invention or pharmacologically acceptable salts thereof include racemic compounds, stereoisomers, and mixtures of these compounds, as well as isotopically labeled and radiolabeled compounds. Such isomers can be isolated using standard separation techniques, including fractional crystallization and chiral column chromatography. Further, the compounds of the above embodiment of the present invention include enantiomers or diastereomers. Diastereomer mixtures can be separated into their individual diastereomers based on their physical/chemical differences using methods well-known in the art, such as chromatography and/or fractional crystallization. Enantiomers can be separated by chiral column chromatography or by reacting an enantiomeric compound with an appropriate optically active compound to convert it into a diastereomer mixture, separating each diastereomer, and then converting the individual diastereomers into corresponding enantiomers. The compounds of the above embodiment of the present invention may be any isomer, including diastereomers, enantiomers, and mixtures thereof.

The compounds of Formula (I), Formula (II), Formula (III), or Formula (IV) of the above embodiment, or pharmacologically acceptable salts thereof, can bind to cereblon E3 ligase. The cereblon E3 ligase binding ability can be evaluated, for example, using a surface plasmon resonance (SPR) method described in the examples below. Additionally, it can be evaluated using a commonly known method described in, for example, WO2018/237026, or the like.

In particular, among the above compounds, compounds of Formula (I), Formula (II), Formula (III), or Formula (IV) substituted with L substituted with TBL, that is, compounds of Formula (I-1), Formula (II-1), Formula (III-1), or Formula (IV-1), possess not only cereblon E3 ligase binding ability but also target protein degradation-inducing ability. Therefore, these componds exhibit low toxicity (for example, acute toxicity, chronic toxicity, genotoxicity, reproductive toxicity, cardiotoxicity, carcinogenicity) and can be used as pharmaceuticals for preventing or treating diseases caused by dysregulation of protein activity in mammals (for example, mice, rats, hamsters, rabbits, cats, dogs, cows, sheep, monkeys, humans, and the like). The target protein degradation-inducing ability can be evaluated, for example, using a commonly known method described in WO2017/079267 or the like. For example, for IRAK4 protein, Estrogen Receptor protein, and BRD4 protein, it can be evaluated using a method described in the examples below.

In one aspect, when a compound of Formula (I), Formula (II), Formula (III), or Formula (IV), or a pharmacologically acceptable salt thereof, is an optically active compound, such an optically active compound exhibits higher stability against racemization. The compounds of Formula (I), Formula (II), Formula (III), or Formula (IV), or their pharmacologically acceptable salts, have a seven-membered imide ring moiety in their structures (provided that, in Formula (II), Z is CH₂), and are considered to have higher chemical stability compared to optically active compounds with a corresponding six-membered imide ring moiety. The high stability against racemization enables the use of a single enantiomer of the compounds of the present embodiment.

Further, in one aspect, it has been found that compounds of Formula (I), Formula (II), Formula (III), or Formula (IV), or their pharmacologically acceptable salts, do not induce degradation of IKZF1 protein and/or SALL4 protein. Since IKZF1 protein regulates differentiation of blood cells, degradation of IKZF1 protein may cause side effects. Degradation of SALL4 protein is considered to be associated with teratogenicity. Not inducing the degradation of these proteins can reduce side effects different from desired pharmacological effects when the compounds of the present embodiment are used as pharmaceuticals for preventing or treating diseases caused by dysregulation of protein activity. Confirmation of not inducing degradation of specific proteins can be evaluated using a commonly known method described, for example, in WO2017/079267 or the like. For example, for IKZF1 protein and SALL4 protein, the evaluation can be performed using the methods described in the examples below.

### [Pharmaceutical Composition]

A pharmaceutical composition, which is a second embodiment of the present invention, contains, as an active ingredient, a compound of Formula (I), Formula (II), Formula (III), or Formula (IV) substituted with L substituted with TBL as described in any of the above aspects (44) to (47) and other aspects, that is, a compound of Formula (I-1), Formula (II-1), Formula (III-1), or Formula (IV-1), or a pharmacologically acceptable salt thereof, and a pharmaceutically acceptable carrier. This pharmaceutical composition can be used for preventing or treating diseases caused by dysregulation of protein activity. Examples of diseases caused by dysregulation of protein activity include cancer, autoimmune diseases, inflammatory diseases, neurodegenerative diseases, and genetic diseases.

In one aspect, the above pharmaceutical composition can be used for preventing or treating diseases caused by dysregulation of protein activity, selected from cancer, autoimmune diseases, inflammatory diseases, neurodegenerative diseases, and genetic diseases. In one aspect, the above pharmaceutical composition can be used for preventing or treating cancer.

In one aspect, the cancer is related to a cancer-related protein selected from a group consisting of ABL, AKT, ALK, AR, ARG1, AR-V7, ASH1L, ATM, AURKA, AURORA-A, AuroraA, Bcl2, Bcl-6, BCL9 (β-catenin PPI), Bcl-XL, BCR-ABL, BRAF, BRDs, BRD4, BET (Bromodomain and extraterminal domain) proteins, BRG1/BRM, BRPF1, BTK, CBFβ, CBP, CBP/p300, CDK2, CDK2/5, CDK2/9, CDK4/6, CDK8 (or CDK19), CDK12 (or CDK9), c-KIT, CK1α, CK1α/CDK, CK2, cMet, CRBN, CREBBP, CSF-1R kinase, cyclosporin, DOT1L, EED, EGFR, EGFR/PARP, ENL, EP300 (HAT), ER, ErBb, ERK1/2, ERK1, ERK2, EZH2, FGFR, FGFR2, FGFR3, FGFR4, FKBP, FLT3, FLT3-ITD, Gli1, GSK3β, HDAC, HDAC3, HER3, HMGCR, HPK1, HSP90, IRAK, IRAK4 BTK, ITK, JAK, JAK1,2, JAK2, JAK3, KEAP1, KRas, KRASG12D, LRRK2, LZK, MALT1, MEK, MDM2, mHTT, mTOR, MYB, NF-kB, NR4A1, NTRK1, p38a/d, PARP, PARP1, PBRM1, PD-L1, PDE4, PDGFRa, PIK3CA, PI3K/mTOR, PKCBβ1, PLK1/BRD4, PPAR, PPARγ, PRC2, PTK6, PTPN1/2, RAF, Ras, RET, SHP2, smad3, SMARCA, SMARCA2, SMARCA2/4, SOS1, STAT, STAT3, STK4, Tau, TEAD, TERT, TOP1, TDP1, TRIM24, VEGFR-2, α-tubulin, AURKB, AXL, BRD3, BRD7, BUB1B, CDK12, CDK12 C1039F, CDK17, CHEK1, c-Met, CSNK1A1, DAPK1, DDR2, eIF4E, EPHA1, EPHA2, EPHA3, EPHB2, EPHB3, EPHB4, EPHB6, FKBP12, FLT1, FYN, GCN5, HDAC2, IGF-1R, KRASG12C, LATS1, LCK, LXRA, LYN, MAP3K1, MAP3K11, MAP3K7, MAP4K1, MAP4K3, MAPK10, MAPK9, MAPKAPK2, MCL1, MerTK, MLLT1, MYC, NUAK1, PAK1, PARP2, PARP3, PCAF, PDK1, PRKAA1, PRKAA2, PRKCI, RPS6KA3, RPS6KA4, RPS6KA6, SF3B1, SIRT2, SLC9A2, Src, STK10, STK33, STK40, TAOK2, TAOK3, TGFBR1, TNK1, TTK, TYK2, and YES1.

In the present specification, "prevention" includes prevention of the onset of a disease (the entire pathological condition or one or more pathological condition) and delay of the onset of the disease. The term "preventive effective amount" refers to a dose of a compound of Formula (I-1), Formula (II-1), Formula (III-1), or Formula (IV-1) sufficient to achieve such a purpose.

In the present specification, "treatment" includes cure of a disease (the entire pathological condition or one or more pathological conditions), improvement of the disease, and suppression of progression of the severity of the disease. The term "therapeutic effective amount" refers to a dose of a compound of Formula (I-1), Formula (II-1), Formula (III-1), or Formula (IV-1) sufficient to achieve such a purpose.

Examples of the form of the pharmaceutical composition include tablets (including sugar-coated tablets, film-coated tablets, sublingual tablets, orally disintegrating tablets, buccal tablets, and the like), pills, powders, granules, capsules (including soft capsules and microcapsules), syrups, solutions, emulsions, suspensions, controlled-release formulations (for example, immediate-release formulations, sustained-release formulations, sustained-release microcapsules), aerosols, films (for example, orally disintegrating films, oral mucosal adhesive films), injectables (for example, subcutaneous injectables, intravenous injectables (for example, bolus), intramuscular injectables, intraperitoneal injectables), infusions, transdermal absorption formulations, ointments, lotions, patches, suppositories (for example, rectal suppositories, vaginal suppositories), pellets, nasal preparations, pulmonary preparations (inhalants), eye drops, and the like.

In the present specification, as the "pharmaceutically acceptable carrier," various carriers commonly used in the field of pharmaceutical formulation technology can be used.

Specific examples of the "pharmaceutically acceptable carrier" include, in solid formulations, excipients (for example, lactose, sucrose, D-mannitol, starch, corn starch, crystalline cellulose, light anhydrous silicic acid, and the like), lubricants (for example, magnesium stearate, talc, colloidal silica, and the like), binders (for example, crystalline cellulose, sucrose, D-mannitol, dextrin, hydroxypropyl cellulose, hydroxypropyl methylcellulose, polyvinylpyrrolidone, starch, sucrose, gelatin, methylcellulose, sodium carboxymethylcellulose, and the like), and disintegrants (for example, starch, carboxymethylcellulose, calcium carboxymethylcellulose, sodium carboxymethyl starch, L-hydroxypropyl cellulose, and the like).

The examples include, in liquid formulations, solvents (for example, water for injection, isotonic saline, alcohol, propylene glycol, macrogol, sesame oil, and the like), solubilizing agents (for example, polyethylene glycol, propylene glycol, D-mannitol, benzyl benzoate, ethanol, triethanolamine, sodium carbonate, sodium citrate, and the like), suspending agents (for example, surfactants such as stearyl triethanolamine, sodium lauryl sulfate, lauryl aminopropionic acid, lecithin, benzalkonium chloride, glyceryl monostearate; hydrophilic polymers such as polyvinyl alcohol, polyvinylpyrrolidone, sodium carboxymethylcellulose, methylcellulose, hydroxymethylcellulose, hydroxypropyl cellulose, and the like), tonicity agents (for example, glucose, D-sorbitol, sodium chloride, glycerin, D-mannitol, and the like), buffering agents (for example, buffers such as phosphate and citrate, and the like), and soothing agents (for example, benzyl alcohol, and the like).

When necessary, formulation additives such as preservatives (for example, paraben esters, chlorobutanol, benzyl alcohol, sorbic acid, and the like), antioxidants (for example, sulfites, ascorbic acid, α-tocopherol, and the like), coloring agents, sweeteners, and the like may further be added.

The pharmaceutical composition of the present embodiment varies depending on the dosage form, administration method, carrier, and the like, but can be produced by adding the compound of the first embodiment described above in an amount typically ranging from 0.01 to 99% (w/w), preferably from 0.1 to 85% (w/w), relative to the total amount of the formulation. The pharmaceutical composition, depending on a form thereof, can be produced using a commonly used method in the field of pharmaceutical formulation technology. The pharmaceutical composition of the present embodiment may be formed into a sustained-release formulation containing the active ingredient.

Further, another embodiment of the present invention is a pharmaceutical containing a compound of the above Formula (I-1), Formula (II-1), Formula (III-1), or Formula (IV-1), or a pharmacologically acceptable salt thereof, as an active ingredient. This pharmaceutical can be an agent for prevention or treatment of diseases caused by dysregulation of protein activity. Further, another embodiment of the present invention is use of a compound of Formula (I-1), Formula (II-1), Formula (III-1), or Formula (IV-1), or a pharmacologically acceptable salt thereof, for manufacture of an agent for prevention or treatment of a disease caused by dysregulation of protein activity.

Further, another embodiment of the present invention is a method for modulating protein activity of a target protein in a mammal, including administering an effective amount of a compound of Formula (I-1), Formula (II-1), Formula (III-1), or Formula (IV-1), or a pharmacologically acceptable salt thereof, to the mammal.

Further, another embodiment of the present invention is a method for prevention or treatment of diseases caused by dysregulation of protein activity, including administering a prophylactically or therapeutically effective amount of a compound of Formula (I-1), Formula (II-1), Formula (III-1), or Formula (IV-1), or a pharmacologically acceptable salt thereof, to a mammal in need of such administration.

Further, another embodiment of the present invention is a compound of Formula (I-1), Formula (II-1), Formula (III-1), or Formula (IV-1), or a pharmacologically acceptable salt thereof, for use in prevention or treatment of diseases caused by dysregulation of protein activity.

### (Subject to be administered)

The compounds of Formula (I-1), Formula (II-1), Formula (III-1), or Formula (IV-1), or pharmaceutical compositions containing them, are expected to have low toxicity and minimal side effects, while also possessing excellent properties as pharmaceuticals. Therefore, the compounds of the first embodiment or the pharmaceutical compositions of the second embodiment can be safely administered to mammals (particularly humans).

### (Route of administration)

The compounds of Formula (I-1), Formula (II-1), Formula (III-1), or Formula (IV-1), or pharmaceutical compositions containing them, can be administered orally or parenterally (for example, intravenously, intramuscularly, subcutaneously, intraorganically, intranasally, intradermally, intraocularly, intracerebrally, rectally, vaginally, intraperitoneally, or directly to the lesion).

### (Dose)

The dose of the compounds of Formula (I-1), Formula (II-1), Formula (III-1), or Formula (IV-1), or pharmaceutical compositions containing them, varies depending on the subject, the administration route, and the age or condition of the subject to be administered, but is not particularly limited. For example, the dose per administration as an active ingredient is 1 to 1000 mg for oral administration and 0.1 to 1000 mg for parenteral administration.

### (Use as prodrug)

A compound of Formula (I-1), Formula (II-1), Formula (III-1), or Formula (IV-1) can also be used in the form of a prodrug. The prodrug of a compound of Formula (I-1), Formula (II-1), Formula (III-1), or Formula (IV-1) refers to a compound that is converted into a compound of Formula (I-1), Formula (II-1), Formula (III-1), or Formula (IV-1) through a reaction by an enzyme, gastric acid, or the like under physiological conditions in vivo; that is, a compound that undergoes enzymatic oxidation, reduction, hydrolysis, or the like to change into a compound of Formula (I-1), Formula (II-1), Formula (III-1), or Formula (IV-1), or a compound that undergoes hydrolysis or the like by gastric acid or the like to change into a compound of Formula (I-1), Formula (II-1), Formula (III-1), or Formula (IV-1). Examples of the prodrug of a compound of Formula (I-1), Formula (II-1), Formula (III-1), or Formula (IV-1) include: a compound in which an amino group of the compound of Formula (I-1), Formula (II-1), Formula (III-1), or Formula (IV-1) is acylated, alkylated, or phosphorylated [for example, a compound in which an amino group of the compound of Formula (I-1), Formula (II-1), Formula (III-1), or Formula (IV-1) is eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated]; a compound in which a hydroxyl group of the compound of Formula (I-1), Formula (II-1), Formula (III-1), or Formula (IV-1) is acylated, alkylated, phosphorylated, or boronated [for example, a compound in which a hydroxyl group of the compound of Formula (I-1), Formula (II-1), Formula (III-1), or Formula (IV-1) is acetylated, palmitoylated, propanoylated, pivaloylated, succinylated, fumarylated, alanylated, or dimethylaminomethylcarbonylated]; or a compound in which a carboxyl group of the compound of Formula (I-1), Formula (II-1), Formula (III-1), or Formula (IV-1) is esterified or amidated [for example, a compound in which a carboxyl group of the compound of Formula (I-1), Formula (II-1), Formula (III-1), or Formula (IV-1) is ethyl-esterified, phenyl-esterified, carboxymethyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolen-4-yl)methyl-esterified, cyclohexyloxycarbonylethyl-esterified, or methylamidated]. These compounds can be produced from compounds of Formula (I-1), Formula (II-1), Formula (III-1), or Formula (IV-1) using commonly known methods.

Further, a prodrug of a compound of Formula (I-1), Formula (II-1), Formula (III-1), or Formula (IV-1) may be one that changes into the compound of Formula (I-1), Formula (II-1), Formula (III-1), or Formula (IV-1) under physiological conditions, as described in "Drug Development," Volume 7, Molecular Design, pages 163 to 198, published by Hirokawa Shoten in 1990.

### (Use in combination with other drugs)

The compounds of Formula (I-1), Formula (II-1), Formula (III-1), or Formula (IV-1) have extremely low toxicity, as described above, and can be used in combination with other drugs for the prevention or treatment of target diseases, and superior preventive and/or therapeutic effects through such combinations can be expected. Further, it can be expected that such combination therapy reduces a dose of the other drugs and thus reduce side effects associated with the other drugs.

A drug that can be used in combination with a compound of Formula (I-1), Formula (II-1), Formula (III-1), or Formula (IV-1) (hereinafter referred to as a concomitant drug) can be appropriately selected based on factors such as the type of disease and the severity of symptoms in the patient.

The administration form of a concomitant drug is not particularly limited, and at the time of administration, a compound of Formula (I-1), Formula (II-1), Formula (III-1), or Formula (IV-1) can be combined with a concomitant drug. For example, the following administration forms can be used: (1) administration of a formulation containing both a compound of Formula (I-1), Formula (II-1), Formula (III-1), or Formula (IV-1) and a concomitant drug in combination; (2) simultaneous or separate administration via the same administration route of two separate formulations, one containing a compound of Formula (I-1), Formula (II-1), Formula (III-1), or Formula (IV-1) and the other containing a concomitant drug; and (3) simultaneous or separate administration via different administration routes of two separate formulations, one containing a compound of Formula (I-1), Formula (II-1), Formula (III-1), or Formula (IV-1) and the other containing a concomitant drug. A preferred form can be appropriately selected according to an actual situation in clinical practice.

A formulation containing a combination of a compound of Formula (I-1), Formula (II-1), Formula (III-1), or Formula (IV-1) and a concomitant drug can be appropriately manufactured by a person skilled in the art in accordance with the pharmaceutical composition containing the compound of the present invention described above.

The dosage of a concomitant drug can be appropriately selected based on a clinically used dosage. Further, a combination ratio of a compound of Formula (I-1), Formula (II-1), Formula (III-1), or Formula (IV-1) with a concomitant drug can be appropriately selected depending on the disease or symptoms of the subject, the administration route, the type of the concomitant drug to be used, and the like. Typically, the combination ratio can be appropriately determined according to an actual situation in clinical practice based on a general clinical dosage of the concomitant drug to be used.

### [Examples]

Hereinafter, the present invention will be described in more detail with reference to reference examples and examples related to the synthesis of the compounds of Formula (I), Formula (II), Formula (III), or Formula (IV), as well as test examples related to the activity of the compounds of Formula (I), Formula (II), Formula (III), or Formula (IV). However, these are merely illustrative, and the present invention is not limited thereto.

### Reference Example and Example 1

### (1-1) tert-Butyl N-[[1-(2-oxoazepan-3-yl)triazol-4-yl]methyl]carbamate (Reference Example Compound 1)

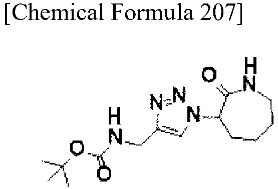

To a solution of tert-butyl 2-propynylcarbamate (111 mg) and 3-azidoazepan-2-one (100 mg) in tetrahydrofuran (3 mL), an aqueous solution (0.5 mL) of sodium ascorbate (64.3 mg) and copper(II) sulfate pentahydrate (16.2 mg) was added, and the mixture was stirred at room temperature for 5 hours. The solution was loaded onto an inject column and purified by silica gel column chromatography (chloroform:methanol = 100:0 to 95:5) to obtain the title compound (200 mg) as a white solid. MS (ESI) m/z: 310.0 (M+H)⁺.

### (1-2) tert-Butyl N-[[1-(2,7-dioxoazepan-3-yl)triazol-4-yl]methyl]carbamate (Example Compound 1)

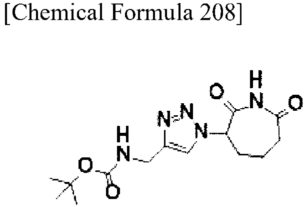

Reference Example Compound 1 (200 mg), Dess-Martin periodinane (823 mg), water (0.0350 mL), and dimethyl sulfoxide (0.5 mL) were heated under reflux in acetonitrile (5 mL) for 14 hours. Saturated aqueous sodium bicarbonate and 1M aqueous sodium thiosulfate were added to the reaction mixture, followed by stirring, and the mixture was extracted twice with ethyl acetate. The organic layer was washed with water and then concentrated under reduced pressure. The residue was purified by silica gel column chromatography (hexane:ethyl acetate = 50:50 to 0:100) to obtain the title compound (89.6 mg) as a white solid. MS (ESI) m/z: 324.0 (M+H)⁺

### Examples 2 - 4:

The corresponding starting compounds were processed in the same manner as in Reference Example and Example 1 to obtain the compounds listed in Table 1 below.

**[Table 1]**

| Example | Structural formula | Physical properties etc. |
|---|---|---|
| 2 | 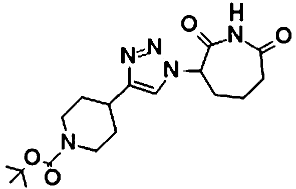 tert-Butyl 4-[1-(2,7-dioxoazepan-3-yl)triazol-4-yl]piperidine-1-carboxylate | White solid |
| | | MS (ESI) m/z: 378.0 (M+H)⁺ |
| 3 | 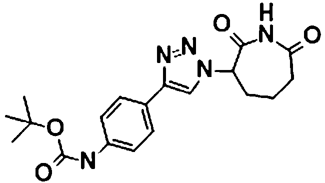 tert-Butyl N-[4-[1-(2,7-dioxoazepan-3-yl)triazol-4-yl]phenyl]carbamate | White solid |
| | | MS (ESI) m/z: 386.0 (M+H)⁺ |
| 4 | 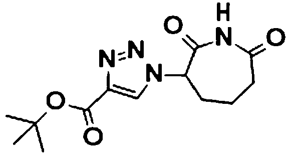 tert-Butyl 1-(2,7-dioxoazepan-3-yl)triazol-4-carboxylate | White solid |
| | | MS (ESI) m/z: 295.0 (M+H)⁺ |

### Reference Example and Example 5

### (5-1) tert-Butyl N-[3-[1-(2-oxoazepan-3-yl)triazol-4-yl]phenyl]carbamate (Reference Example Compound 5)

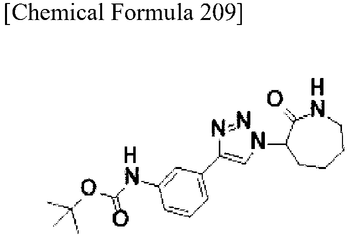

3-Ethynylaniline (91.0 mg) and di-tert-butyl dicarbonate (203 mg) were heated under reflux in tetrahydrofuran (3 mL) for 3 hours. After cooling to room temperature, an aqueous solution (0.5 mL) of 3-azidoazepan-2-one (100 mg), sodium ascorbate (64.3 mg), and copper(II) sulfate pentahydrate (16.2 mg) was added, and the mixture was stirred at room temperature for 1 hour. Water and chloroform were added to the reaction mixture, followed by stirring. The aqueous layer was removed using a Phase Separator (registered trademark), and the organic layer was concentrated under reduced pressure. The residue was purified by silica gel column chromatography (chloroform:methanol = 100:0 to 95:5) to obtain the title compound (254 mg) as a white solid. MS (ESI) m/z: 372.0 (M+H)⁺.

### (5-2) tert-Butyl N-[3-[1-(2,7-dioxoazepan-3-yl)triazol-4-yl]phenyl]carbamate (Example Compound 5)

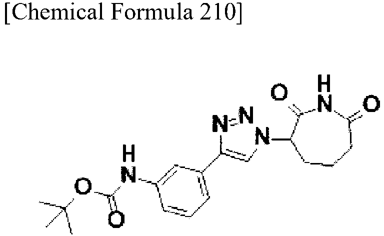

Using Reference Example Compound 5 instead of Reference Example Compound 1 in Reference Example and Example 1, the same reaction and processing as in (1-2) were performed to obtain the title compound as a beige solid. MS (ESI) m/z: 386.0 (M+H)⁺.

### Example 6

### (6-1) tert-Butyl N-[2-[1-(2,7-dioxoazepan-3-yl)triazol-4-yl]phenyl]carbamate (Example Compound 6)

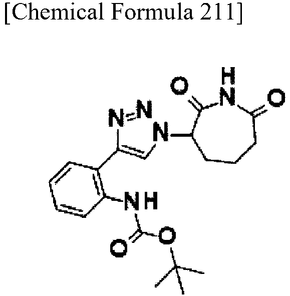

Using 2-ethynylaniline instead of 3-ethynylaniline in Example 5, the same reactions and processing as in (5-1) and (5-2) were performed to obtain the title compound as a white solid. MS (ESI) m/z: 386.2 (M+H)⁺.

### Reference Example and Example 7

### (7-1) 4-Chloro-N-(2-oxoazepan-3-yl)benzenesulfonamide (Reference Example Compound 7-1)

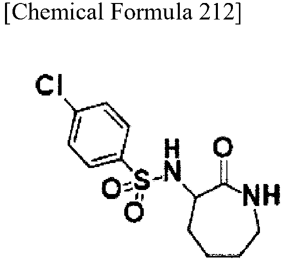

DL-α-Amino-ε-caprolactam (500 mg), 4-chlorobenzenesulfonyl chloride (906 mg), and pyridine (1.26 mL) were stirred in tetrahydrofuran (8 mL) at room temperature for 3 hours and at 50 °C for 5 hours. Water was added to the reaction mixture and the mixture was stirred, followed by addition of 1 M hydrochloric acid and further stirring, and the mixture was extracted twice with ethyl acetate. The organic layer was washed twice with water and then concentrated under reduced pressure to obtain the title compound (480 mg) as a white solid. MS (ESI) m/z: 302.8/304.7 (M+H)⁺.

### (7-2) 4-Chloro-N-methyl-N-(2-oxoazepan-3-yl)benzenesulfonamide (Reference Example Compound 7-2)

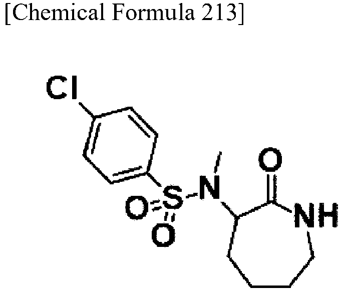

To a solution of Reference Example Compound 7-1 (246 mg) in N,N-dimethylformamide (5 mL), cesium carbonate (397 mg) and methyl iodide (0.0607 mL) were added, and the mixture was stirred at room temperature for 4 hours. Water was added to the reaction mixture, followed by stirring, and the mixture was extracted twice with ethyl acetate. The organic layer was washed twice with water and then concentrated under reduced pressure. The residue was purified by silica gel column chromatography (chloroform:methanol = 100:0 to 95:5) to obtain the title compound (233 mg) as a white solid. MS (ESI) m/z: 317.0/318.8 (M+H)⁺.

### (7-3) 4-Chloro-N-(2,7-dioxoazepan-3-yl)-N-methylbenzenesulfonamide (Example Compound 7)

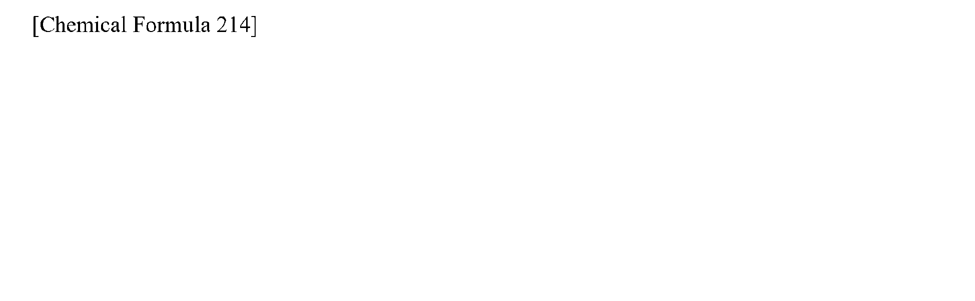

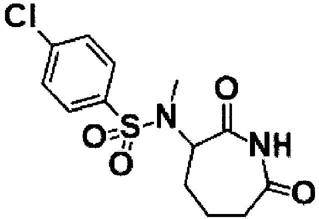

Reference Example Compound 7-2 (233 mg), Dess-Martin periodinane (935 mg), water (0.0397 mL), and dimethyl sulfoxide (0.5 mL) were heated under reflux in acetonitrile (5 mL) for 6 hours. After cooling to room temperature, saturated aqueous sodium bicarbonate and 1M aqueous sodium thiosulfate were added to the reaction mixture, followed by stirring, and the mixture was extracted twice with ethyl acetate. The organic layer was washed twice with water and then concentrated under reduced pressure. The residue was purified by silica gel column chromatography (chloroform:methanol = 100:0 to 95:5) to obtain the title compound (183 mg) as a white solid. MS (ESI) m/z: 330.8/332.7 (M+H)⁺.

### Example 8

### (8-1) 4-Chloro-N-(2,7-dioxoazepan-3-yl)benzenesulfonamide (Example Compound 8)

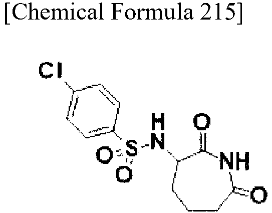

Using Reference Example Compound 7-1 instead of Example Compound 7-2 in Example 7, the same reaction and processing as in (7-3) were performed to obtain the title compound as a white solid. MS (ESI) m/z: 316.8/318.7 (M+H)⁺.

### Example 9

### (9-1) 3-Chloro-N-(2,7-dioxoazepan-3-yl)benzenesulfonamide (Example Compound 9)

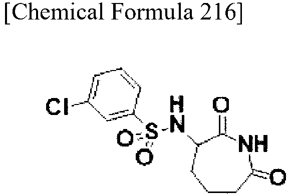

Using 3-chlorobenzenesulfonyl chloride instead of 4-chlorobenzenesulfonyl chloride in Example 7, the same reactions and processing as in (7-1) and (7-3) were performed to obtain the title compound as a white solid. MS (ESI) m/z: 316.9/318.8 (M+H)⁺.

### Example 10

### (10-1) 3-Chloro-N-(2,7-dioxoazepan-3-yl)-N-methylbenzenesulfonamide (Example Compound 10)

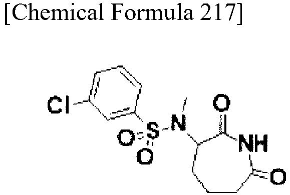

Using 3-chlorobenzenesulfonyl chloride instead of 4-chlorobenzenesulfonyl chloride in Example 7, the same reactions and processing as in (7-1) to (7-3) were performed to obtain the title compound as a white solid. MS (ESI) m/z: 331.1/333.1 (M+H)⁺.

### Example 11

### (11-1) Methyl 3-[(2,7-dioxoazepan-3-yl)-methylsulfamoyl]benzoate (Example Compound 11)

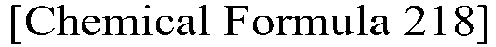

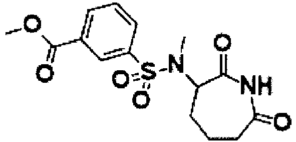

Using 3-(methylamino)azepan-2-one instead of DL-α-amino-ε-caprolactam and methyl 3-(chlorosulfonyl)benzoate instead of 4-chlorobenzenesulfonyl chloride in Example 7, the same reactions and processing as in (7-1) and (7-3) were performed to obtain the title compound as a white solid. MS (ESI) m/z: 355.0 (M+H)⁺.

### Example 12

### (12-1) tert-Butyl 10,12-dioxo-8-oxa-2,11-diazaspiro[5.6]dodecane-2-carboxylate (Example Compound 12-1)

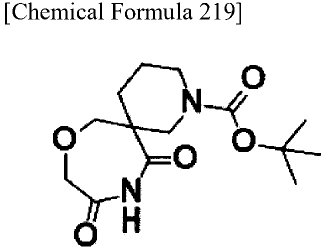

tert-Butyl 10-oxo-8-oxa-2,11-diazaspiro[5.6]dodecane-2-carboxylate (250 mg), Dess-Martin periodinane (1118 mg), water (0.0475 mL), and dimethyl sulfoxide (0.5 mL) were heated under reflux in acetonitrile (5 mL) for 6 hours. Saturated aqueous sodium bicarbonate and 1M aqueous sodium thiosulfate were added to the reaction mixture, followed by stirring, and the mixture was extracted twice with ethyl acetate. The organic layer was washed twice with water and then concentrated under reduced pressure. The residue was purified by silica gel column chromatography (hexane:ethyl acetate = 70:30 to 30:70) to obtain the title compound (205 mg) as a white solid. MS (ESI) m/z: 297.1 (M-H)⁻.

### (12-2) 2-Benzoyl-8-oxa-2,11-diazaspiro[5.6]dodecane-10,12-dione (Example Compound 12-2)

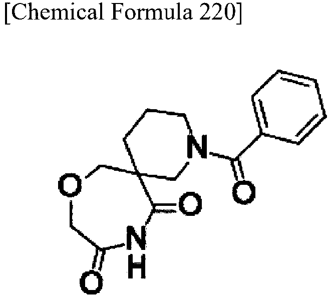

To a solution of Example Compound 12-1 (30.0 mg) in chloroform (1 mL), trifluoroacetic acid (1 mL) was added, and the mixture was stirred at room temperature for 2 hours. The reaction mixture was concentrated under reduced pressure, and azeotropic distillation with toluene was performed twice. The residue, benzoyl chloride (0.0130 mL), and pyridine (0.0243 mL) were stirred in tetrahydrofuran (3 mL) at room temperature for 3 hours. Water and chloroform were added to the reaction mixture, followed by stirring. The aqueous layer was removed using a phase separator, and the organic layer was concentrated under reduced pressure. The residue was purified by silica gel column chromatography (chloroform:methanol = 100:0 to 95:5) to obtain the title compound (13.8 mg) as a white solid. MS (ESI) m/z: 302.9 (M+H)⁺.

### Examples 13 - 14:

The corresponding starting compounds were processed in the same manner as in Example 12 to obtain the compounds listed in Table 2 below.

**[Table 2]**

| Example | Structural formula | Physical properties etc. |
|---|---|---|
| 13-1 | 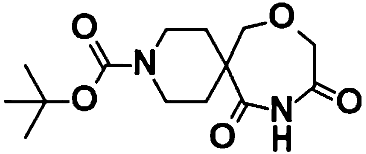 tert-Butyl 10,12-dioxo-8-oxa-3,11-diazaspiro[5.6]dodecane-3-carboxylate | White solid |
| | | MS (ESI) m/z: 297.2 (M-H)⁻ |
| 13-2 | 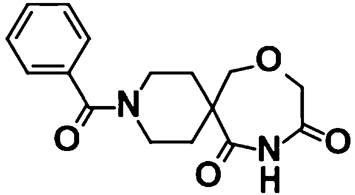 3-benzoyl-8-oxa-3,11-diazaspiro[5. 6]dodecane-10, 2-dione | White solid |
| | | MS (ESI) m/z: 302.9 (M+H)⁺ |
| 14 | 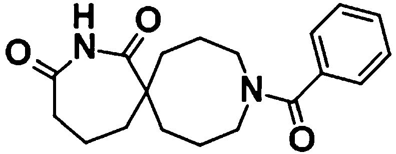 11-Benzoyl-2,11-diazaspiro[6. 7]tetradecane-1,3-dione | Pale yellow solid |
| | | MS (ESI) m/z: 329.0 (M+H)⁺ |

### Example 15

### (15-1) tert-Butyl 6-(10,12-dioxo-8-oxa-2,11-diazaspiro[5.6]dodecan-2-yl)pyridine-3-carboxylate (Example Compound 15)

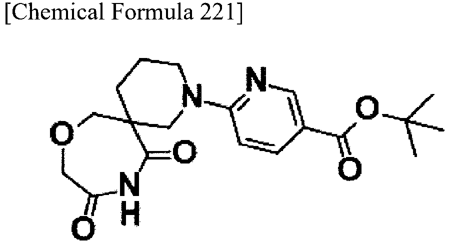

To a solution of Example Compound 12-1 (40.0 mg) in chloroform (1 mL), trifluoroacetic acid (1 mL) was added, and the mixture was stirred at room temperature for 1 hour. The reaction mixture was concentrated under reduced pressure, and azeotropic distillation with toluene was performed twice. The residue, tert-butyl 6-fluoropyridine-3-carboxylate (29.1 mg), and N,N-diisopropylethylamine (0.0696 mL) were stirred in N,N-dimethylformamide (3 mL) at 50 °C for 1 hour and at 80 °C for 3 hours. The reaction mixture was concentrated under reduced pressure, and the residue was purified by silica gel column chromatography (hexane:ethyl acetate = 99:1 to 50:50) to obtain the title compound (23.7 mg) as a white solid. MS (ESI) m/z: 376.1 (M+H)⁺.

### Example 16

### (16-1) tert-Butyl 6-(10,12-dioxo-8-oxa-3,11-diazaspiro[5 .6]dodecan-3-yl)pyridine-3-carboxylate (Example Compound 16)

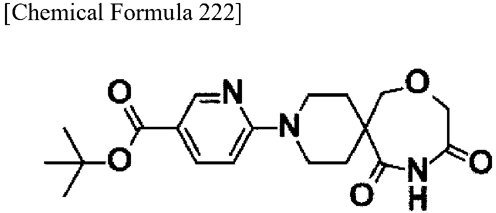

Using Example Compound 13-1 instead of Example Compound 12-1 in Example 15, the same reaction and processing as in (15-1) were performed to obtain the title compound as a white solid. MS (ESI) m/z: 376.1 (M+H)⁺.

### Example 17

### (17-1) 3-Fluoro-3-phenylazepane-2,7-dione (Example Compound 17)

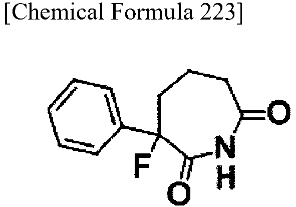

Using 3-fluoro-3-phenylazepan-2-one instead of tert-butyl 10-oxo-8-oxa-2,11-diazaspiro[5.6]dodecane-2-carboxylate in Example 12, the same reaction and processing as in (12-1) were performed to obtain Example Compound 17 as a pale yellow solid. MS (ESI) m/z: 222.1 (M+H)⁺, 201.9 (M+H)⁺.

### Reference Example and Example 18

### (18-1) tert-Butyl 3-[2-[(2-methylpropan-2-yl)oxycarbonylamino]-1-phenylethoxy]propanoate (Reference Example Compound 18-1)

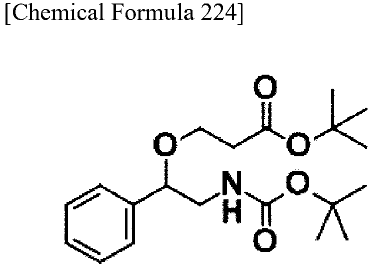

tert-Butyl N-(2-hydroxy-2-phenyl-ethyl)carbamate (594 mg), tert-butyl acrylate (7.30 mL), and cesium carbonate (816 mg) were stirred in tert-butyl alcohol (12 mL) at room temperature for 14 hours. The reaction mixture was concentrated under reduced pressure, water and ethyl acetate were added, followed by stirring, and the mixture was extracted twice with ethyl acetate. The organic layer was concentrated under reduced pressure, and the residue was purified by silica gel column chromatography (hexane:ethyl acetate = 99:1 to 70:30) to obtain the title compound (926 mg) as a colorless viscous material. MS (ESI) m/z: 266.6 (M+H-Boc)⁺.

### (18-2) 2-Phenyl-1,4-oxazepan-5-one (Reference Example Compound 18-2)

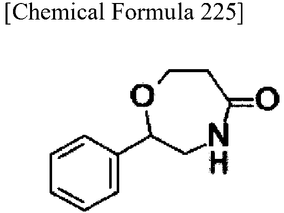

Reference Example Compound 18-1 (346 mg) was stirred in 4M hydrogen chloride/dioxane (5 mL) at room temperature for 2 hours. The reaction mixture was concentrated under reduced pressure. The residue, O-(7-azabenzotriazol-1-yl)-N,N,N',N'-tetramethyluronium hexafluorophosphate (540 mg), and N,N-diisopropylethylamine (0.819 mL) were stirred in N,N-dimethylformamide (5 mL) at room temperature for 2 hours. Water was added to the reaction mixture, followed by stirring, and the mixture was extracted twice with ethyl acetate. The organic layer was washed twice with water and then concentrated under reduced pressure. The residue was purified by silica gel column chromatography (chloroform:methanol = 100:0 to 95:5) to obtain the title compound (93.3 mg) as a pale yellow solid. MS (ESI) m/z: 192.1 (M+H)⁺.

### (18-3) 2-Phenyl-1,4-oxazepane-3,5-dione (Example Compound 18)

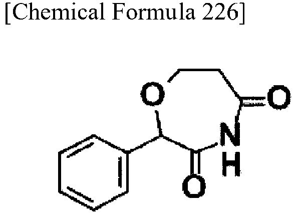

Using Reference Example Compound 18-2 instead of tert-butyl 10-oxo-8-oxa-2,11-diazaspiro[5.6]dodecane-2-carboxylate in Example 12, the same reaction and processing as in (12-1) were performed to obtain the title compound as a white solid. MS (ESI) m/z: 206.0 (M+H)⁺.

### Example 19

### (19-1) 2-(3-Bromophenyl)-1,4-oxazepane-3,5-dione (Example Compound 19)

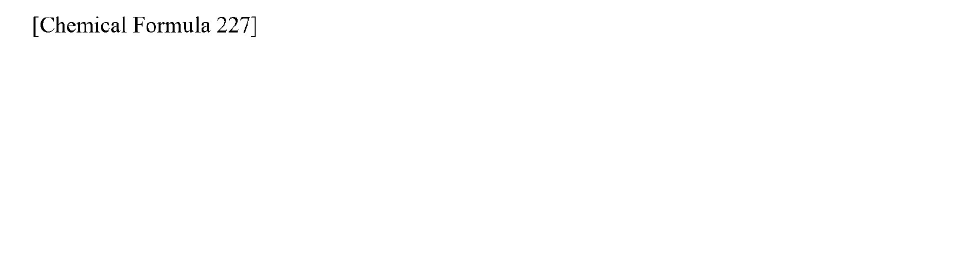

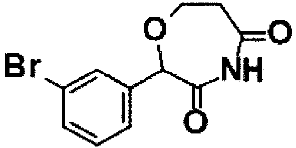

Using tert-butyl N-[2-(3-bromophenyl)-2-hydroxy-ethyl]carbamate instead of tert-butyl N-(2-hydroxy-2-phenyl-ethyl)carbamate in Reference Example and Example 18, the same reactions and processing as in (18-1) to (18-3) were performed to obtain the title compound as a white solid. MS (ESI) m/z: 282.0/284.0 (M-H)⁻.

### Reference Example and Example 20

### (20-1) N-Methyl-N-(2-oxoazepan-3-yl)-3-phenylbenzamide (Reference Example Compound 20)

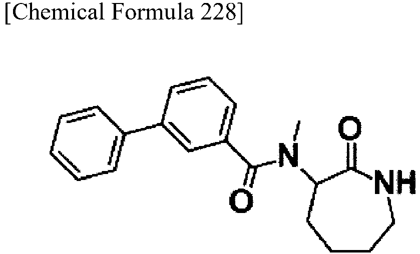

3-(Methylamino)azepan-2-one (35.5 mg), 3-biphenylcarboxylic acid (54.5 mg), O-(7-azabenzotriazol-1-yl)-N,N,N',N'-tetramethyluronium hexafluorophosphate (143 mg), and N,N-diisopropylethylamine (0.216 mL) were stirred in N,N-dimethylformamide (3 mL) at room temperature for 4 hours. Water was added to the reaction mixture, followed by stirring, and the resulting solid was filtered and washed with water. Drying under reduced pressure yielded the title compound (66.6 mg) as a white solid. MS (ESI) m/z: 323.1 (M+H)⁺.

### (20-2) N-(2,7-Dioxoazepan-3-yl)-N-methyl-3-phenylbenzamide (Example Compound 20)

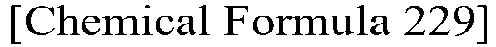

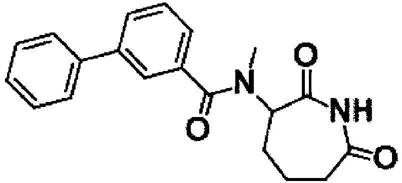

Using Reference Example Compound 20 instead of Reference Example Compound 7-2 in Reference Example and Example 7, the same reaction and processing as in (7-3) were performed to obtain the title compound as a white solid. MS (ESI) m/z: 337.0 (M+H)⁺.

### Examples 21 - 22:

The corresponding starting compounds were processed in the same manner as in Example 20 to obtain the compounds listed in Table 3 below.

**[Table 3]**

| Example | Structural formula | Physical properties etc. |
|---|---|---|
| 21 | 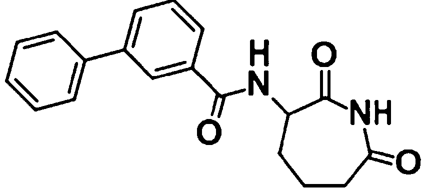 N-(2,7-dioxoazepan-3-yl)-3-phenylbenzamide | White solid |
| | | MS (ESI) m/z: 323.0 (M+H)⁺ |
| 22 | 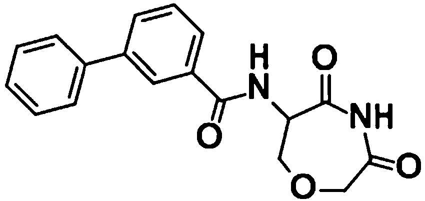 N-(3,5-dioxo-1,4-oxazepan-6-yl)-3-phenylbenzamide | White solid |
| | | MS (ESI) m/z: 325.0 (M+H)⁺ |

### Reference Example 23

### (23-1) N-(7-Oxoazepan-3-yl)-3-phenylbenzamide (Reference Example Compound 23)

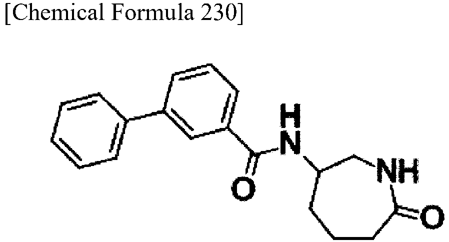

Using 6-aminoazepan-2-one hydrochloride instead of 3-(methylamino)azepan-2-one in Reference Example and Example 20, the same reaction and processing as in (20-1) were performed to obtain the title compound as a white solid. MS (ESI) m/z: 309.0 (M+H)⁺.

### Reference Example and Example 24

### (24-1) tert-Butyl 3-[(4-fluoroanilino)methyl]-2-oxoazepan-1-carboxylate (Reference Example Compound 24-1)

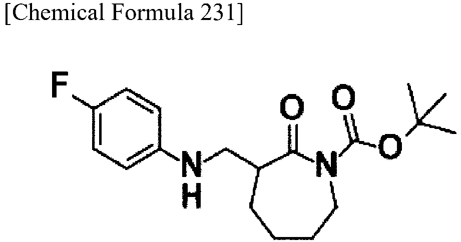

tert-Butyl 3-methylene-2-oxoazepan-1-carboxylate (174 mg), 4-fluoroaniline (0.0813 mL), and triethylamine (0.429 mL) were heated under reflux in acetonitrile (5 mL) for 14 hours. The reaction mixture was concentrated under reduced pressure, and the residue was purified by silica gel column chromatography (hexane:ethyl acetate = 90:10 to 50:50) to obtain the title compound (125 mg) as a pale yellow viscous material. MS (ESI) m/z: 237.0 (M-Boc+H)⁺.

### (24-2) tert-Butyl N-(4-fluorophenyl)-N-[(2-oxoazepan-3-yl)methyl]carbamate (Reference Example Compound 24-2)

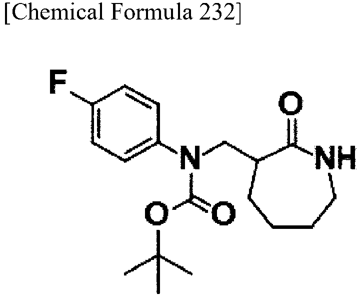

To a solution of Reference Example Compound 24-1 (125 mg) in chloroform (1 mL), trifluoroacetic acid (1 mL) was added, and the mixture was stirred at room temperature for 1 hour. The reaction mixture was concentrated under reduced pressure, and azeotropic distillation with toluene was performed twice. To a solution of the residue in 1,4-dioxane (4 mL), potassium carbonate (102 mg) and di-tert-butyl dicarbonate (97.4 mg) were added, and the mixture was stirred at room temperature for 6 hours. The reaction mixture was concentrated under reduced pressure, and the residue was purified by silica gel column chromatography (hexane:ethyl acetate = 70:30 to 0:100) to obtain the title compound (60.7 mg) as a white solid. MS (ESI) m/z: 337.1 (M+H)⁺.

### (24-3) tert-Butyl N-[(2,7-dioxoazepan-3-yl)methyl]-N-(4-fluorophenyl)carbamate (Example Compound 24)

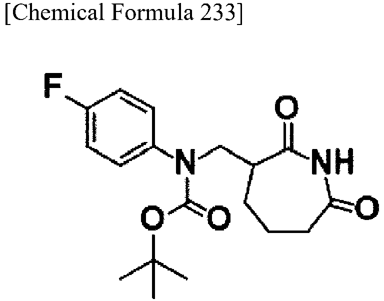

Using Reference Example Compound 24-2 instead of Reference Example Compound 7-2 in Reference Example and Example 7, the same reaction and processing as in (7-3) were performed to obtain the title compound as a white solid. MS (ESI) m/z: 251.0 (M+H-Boc)⁺.

### Example 25

### (25-1) 3-[(4-Fluoroanilino)methyl]azepane-2,7-dione (Example Compound 25)

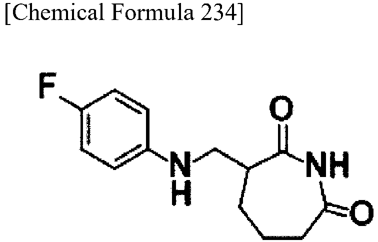

To a solution of Example Compound 24 (39.2 mg) in chloroform (1 mL), trifluoroacetic acid (1 mL) was added, and the mixture was stirred at room temperature for 2 hours. The reaction mixture was concentrated under reduced pressure, and azeotropic distillation with toluene was performed twice. Saturated aqueous sodium bicarbonate and chloroform were added, followed by stirring. The aqueous layer was removed using a Phase Separator, and the organic layer was concentrated under reduced pressure to obtain the title compound (25.9 mg) as a white solid. MS (ESI) m/z: 250.9 (M+H)⁺.

### Reference Example and Example 26

### (26-1) Methyl 6-oxo-1-(2-oxoazepan-3-yl)pyridine-3-carboxylate (Reference Example Compound 26)

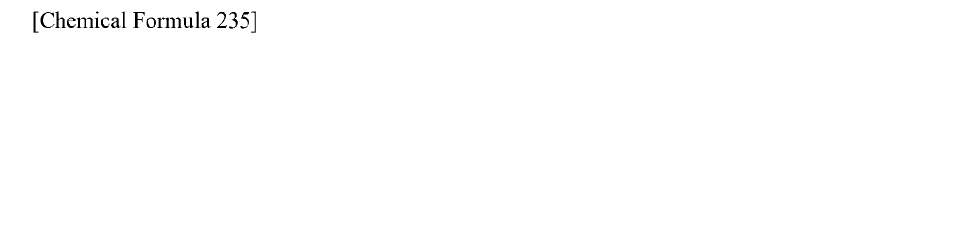

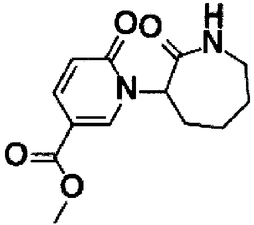

DL-α-Amino-ε-caprolactam (100 mg) and methyl coumalinate (132 mg) were stirred in pyridine (2 mL) at 80 °C for 5 hours. The reaction mixture was concentrated under reduced pressure, and the residue was purified by NH silica gel column chromatography (chloroform:methanol = 100:0 to 95:5) and silica gel column chromatography (ethyl acetate:methanol = 100:0 to 95:5) to obtain the title compound (46.6 mg) as a pale yellow solid. MS (ESI) m/z: 264.9 (M+H)⁺.

### (26-2) Methyl 1-(2,7-dioxoazepan-3-yl)-6-oxopyridine-3-carboxylate (Example Compound 26)

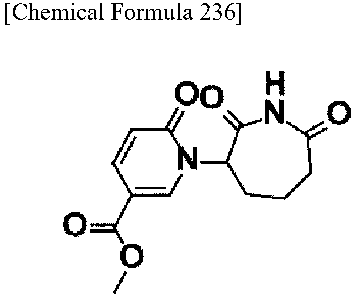

Using Reference Example Compound 26-1 instead of Reference Example Compound 7-2 in Reference Example and Example 7, the same reaction and processing as in (7-3) were performed to obtain the title compound as a yellow solid. MS (ESI) m/z: 278.9 (M+H)⁺.

### Example 27

### (27-1) tert-Butyl N-[[4-[(2,7-dioxoazepan-3-yl)-methylsulfamoyl]phenyl]methyl]carbamate (Example Compound 27)

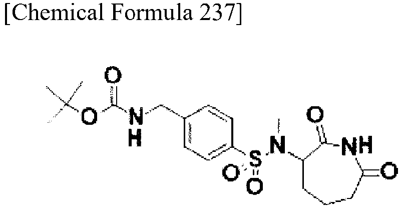

Using 3-(methylamino)azepan-2-one instead of DL-α-amino-ε-caprolactam and tert-butyl N-[(4-chlorosulfonylphenyl)methyl]carbamate instead of 4-chlorobenzenesulfonyl chloride in Reference Example and Example 7, the same reactions and processing as in (7-1) and (7-3) were performed to obtain the title compound as a white solid. MS (ESI) m/z: 424.2 (M-H)⁻.

### Reference Example and Example 28

### (28-1) N-(4-Fluorophenyl)-2-oxoazepane-3-carboxamide (Reference Example Compound 28)

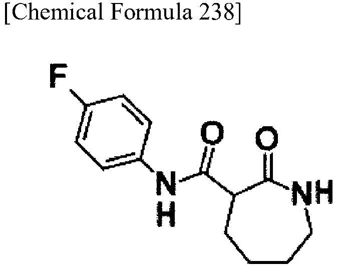

Under a nitrogen stream, a solution of tert-butyl 2-oxoazepan-1-carboxylate (500 mg) in THF (10 mL) was cooled to -78 °C, and lithium bis(trimethylsilyl)amide (about 26% tetrahydrofuran solution) (2.20 mL) was added dropwise, followed by stirring at the same temperature for 30 minutes. 4-Fluorophenyl isocyanate (0.290 mL) was added, and the mixture was stirred for 5 hours while warming to room temperature. Saturated aqueous ammonium chloride was added to the reaction mixture, followed by stirring, and the mixture was extracted twice with ethyl acetate. The organic layer was concentrated under reduced pressure, and the residue was purified by silica gel column chromatography (chloroform:methanol = 100:0 to 95:5) and NH silica gel column chromatography (chloroform:methanol = 100:0 to 95:5) to obtain a yellow viscous material (60.0 mg). The obtained viscous material was dissolved in chloroform (1 mL), trifluoroacetic acid (1 mL) was added, and the mixture was stirred at room temperature for 5 hours. The reaction mixture was concentrated under reduced pressure, and azeotropic distillation with toluene was performed twice. The residue was purified by silica gel column chromatography (chloroform:methanol = 100:0 to 95:5) to obtain the title compound (14.2 mg) as a pale yellow solid. MS (ESI) m/z: 250.9 (M+H)⁺.

### (28-2) N-(4-Fluorophenyl)-2,7-dioxoazepane-3-carboxamide (Example Compound 28)

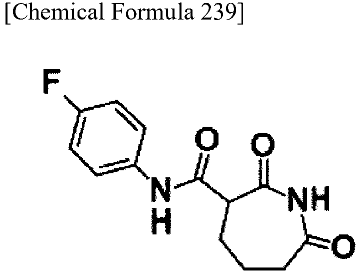

Using Reference Example Compound 28 instead of Reference Example Compound 7-2 in Reference Example and Example 7, the same reaction and processing as in (7-3) were performed to obtain the title compound as a white solid. MS (ESI) m/z: 265.0 (M+H)⁺.

### Reference Example and Example 29

### (29-1) tert-Butyl N-[2-[(2-oxoazepan-3-yl)amino]ethyl]carbamate (Reference Example Compound 29-1)

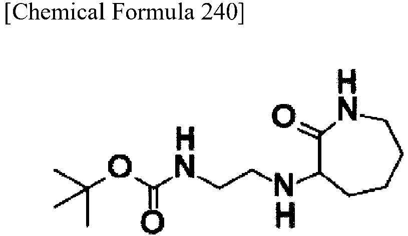

To a solution of DL-α-amino-ε-caprolactam (1000 mg) and N-Boc-2-aminoacetaldehyde (1304 mg) in dichloromethane (10 mL), sodium triacetoxyborohydride (2480 mg) was added, and the mixture was stirred at room temperature for 2 hours. Saturated aqueous sodium bicarbonate and chloroform were added to the reaction mixture, followed by stirring. The aqueous layer was removed using a phase separator, and the organic layer was concentrated under reduced pressure. The residue was purified by silica gel column chromatography (chloroform:methanol = 100:0 to 90: 10) to obtain the title compound (2036 mg) as a colorless viscous material. MS (ESI) m/z: 272.0 (M+H)⁺.

### (29-2) tert-Butyl N-[2-[(2-bromoacetyl)-(2-oxoazepan-3-yl)amino]ethyl]carbamate (Reference Example Compound 29-2)

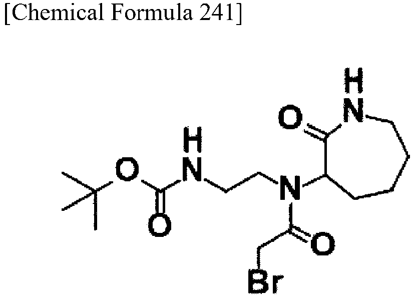

A solution of Reference Example Compound 29-1 (2036 mg) in dichloromethane (10 mL) was cooled to 0 °C, and triethylamine (1.63 mL) followed by bromoacetyl bromide (0.743 mL) was added dropwise. The mixture was stirred for 3 hours while warming to room temperature. Saturated aqueous sodium bicarbonate and chloroform were added to the reaction mixture, followed by stirring. The aqueous layer was removed using a phase separator, and the organic layer was concentrated under reduced pressure. The residue was purified by silica gel column chromatography (chloroform:methanol = 100:0 to 95:5) to obtain the title compound (1253 mg) as a pale yellow solid. MS (ESI) m/z: 391.9/393.8 (M+H)⁺.

### (29-3) tert-Butyl 3-oxo-4-(2-oxoazepan-3-yl)piperazine-1-carboxylate (Reference Example Compound 29-3)

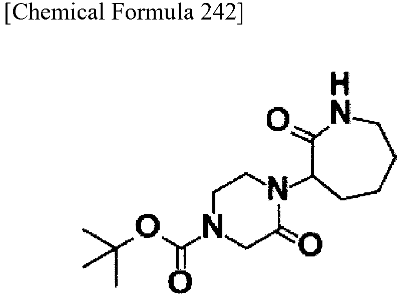

Reference Example Compound 29-2 (605 mg) and cesium carbonate (1005 mg) were stirred in N,N-dimethylformamide (15 mL) at 50 °C for 7 hours. Water was added to the reaction mixture, followed by stirring, and the mixture was extracted twice with ethyl acetate. The organic layer was washed twice with water and then concentrated under reduced pressure. The residue was purified by silica gel column chromatography (chloroform:methanol = 100:0 to 95:5) and silica gel column chromatography (ethyl acetate:methanol = 100:0 to 95:5) to obtain the title compound (99.2 mg) as a white solid. MS (ESI) m/z: 312.0 (M+H)⁺.

### (29-4) tert-Butyl 4-(2,7-dioxoazepan-3-yl)-3-oxopiperazine-1-carboxylate, tert-Butyl 4-(2,7-dioxoazepan-3-yl)-2,3-dioxopiperazine-1-carboxylate (Example Compounds 29-1 and 29-2)

### Example Compound 29-1

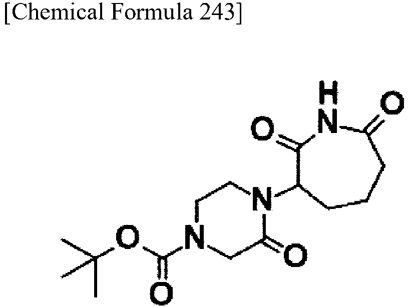

### Example Compound 29-2

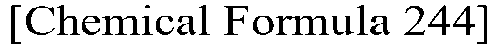

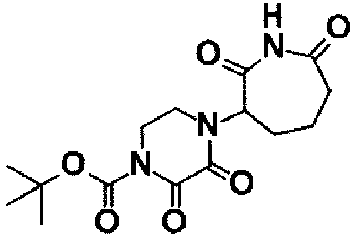

Using Reference Example Compound 29-3 instead of Reference Example Compound 7-2 in Reference Example and Example 7, the same reaction and processing as in (7-3) were performed to obtain Example Compound 29-1 and Example Compound 29-2, respectively, as white solids.
Example Compound 29-1: MS (ESI) m/z: 324.1 (M-H)⁻,
Example Compound 29-2: MS (ESI) m/z: 338.1 (M-H)⁻.

### Example 30

### (30-1) tert-Butyl 6-[4-(2,7-dioxoazepan-3-yl)-3-oxopiperazin-1-yl]pyridine-3-carboxylate (Example Compound 30)

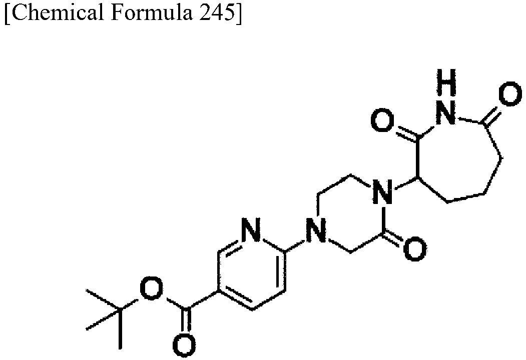

Using Example Compound 29-1 instead of Example Compound 12-1 in Example 15, the same reaction and processing as in (15-1) were performed to obtain the title compound as a white solid. MS (ESI) m/z: 403.1 (M+H)⁺.

### Reference Example and Example 31

### (31-1) 2-[(1-Benzoyl-3-methyl-2-oxoazepan-3-yl)methyl]isoindole-1,3-dione (Reference Example Compound 31-1)

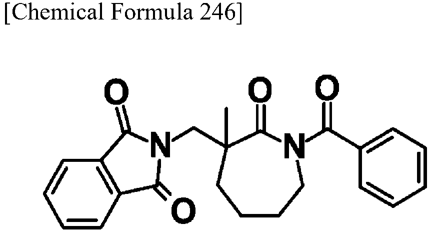

A solution of 1-benzoyl-3-methylazepan-2-one (208 mg) in THF (5 mL) was cooled to 0 °C, and lithium bis(trimethylsilyl)amide (about 26% tetrahydrofuran solution) (0.760 mL) was added dropwise, followed by stirring at the same temperature for 30 minutes. N-(Bromomethyl)phthalimide (238 mg) was added, and the mixture was stirred for 4 hours while warming to room temperature. Saturated aqueous ammonium chloride was added to the reaction mixture, followed by stirring, and the mixture was extracted twice with ethyl acetate. The organic layer was washed twice with water and then concentrated under reduced pressure. The residue was purified by silica gel column chromatography (hexane:ethyl acetate = 99:1 to 50:50) to obtain the title compound (110 mg) as a white solid. MS (ESI) m/z: 391.0 (M+H)⁺.

### (31-2) 2-[(3-Methyl-2-oxoazepan-3-yl)methyl]isoindole-1,3-dione (Reference Example Compound 31-2)

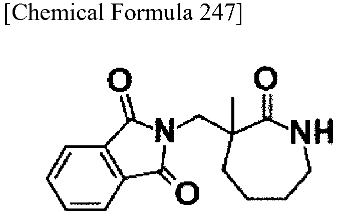

To a mixed solution of Reference Example Compound 31-1 (110 mg) in tetrahydrofuran (2 mL) and methanol (2 mL), 4M aqueous lithium hydroxide (0.141 mL) was added, and the mixture was stirred at room temperature for 6 hours. The mixture was neutralized with 1M hydrochloric acid, and the reaction mixture was concentrated under reduced pressure. The residue, O-(7-azabenzotriazol-1-yl)-N,N,N',N'-tetramethyluronium hexafluorophosphate (129 mg), and N,N-diisopropylethylamine (0.146 mL) were stirred in N,N-dimethylformamide (3 mL) at room temperature for 2 hours and at 50 °C for 6 hours. Water was added to the reaction mixture, followed by stirring, and the mixture was extracted twice with ethyl acetate. The organic layer was washed twice with water and then concentrated under reduced pressure. The residue was purified by silica gel column chromatography (chloroform:methanol = 100:0 to 95:5) and NH silica gel column chromatography (hexane:ethyl acetate = 50:50 to 0:100) to obtain the title compound (31.1 mg) as a white solid. MS (ESI) m/z: 287.0 (M+H)⁺.

### (31-3) 2-[(3-Methyl-2,7-dioxoazepan-3-yl)methyl]isoindole-1,3-dione (Example Compound 31)

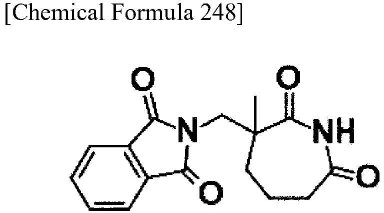

Using Reference Example Compound 31-2 instead of Reference Example Compound 7-2 in Reference Example and Example 7, the same reaction and processing as in (7-3) were performed to obtain the title compound as a beige solid. MS (ESI) m/z: 301.2 (M+H)⁺.

### Example 32

### (32-1) tert-Butyl 2-(6,8-dioxo-2,7-diazaspiro[4.6]undecan-2-yl)pyrimidine-5-carboxylate (Example Compound 32)

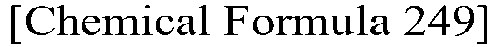

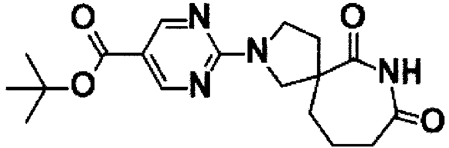

To a solution of tert-butyl 6,8-dioxo-2,7-diazaspiro[4.6]undecane-2-carboxylate (28.0 mg) in chloroform (1 mL), trifluoroacetic acid (1 mL) was added, and the mixture was stirred at room temperature for 1 hour. The reaction mixture was concentrated under reduced pressure, and azeotropic distillation with toluene was performed twice. The residue, tert-butyl 2-chloropyrimidine-5-carboxylate (23.4 mg), and N,N-diisopropylethylamine (0.0858 mL) were stirred in N,N-dimethylformamide (2 mL) at 80 °C for 3 hours. The reaction mixture was concentrated under reduced pressure, and the residue was purified by silica gel column chromatography (hexane:ethyl acetate = 70:30 to 0:100) to obtain the title compound (33.5 mg) as a white solid. MS (ESI) m/z: 361.1 (M+H)⁺.

### Examples 33 - 43:

The corresponding starting compounds were processed in the same manner as in Example 32 to obtain the compounds listed in Table 4 below.

**[Table 4]**

| Example | Structural formula | Physical properties etc. |
|---|---|---|
| 33 | 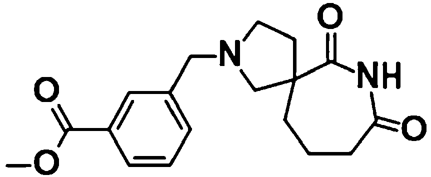 Methyl 3-[(6,8-dioxo-2,7-diazaspiro[4. 6]undecan-2-yl)methyl]benzoate | White solid |
| | | MS (ESI) m/z: 331.0 (M+H)⁺ |
| 34 | 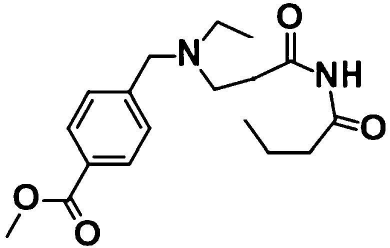 Methyl 4-[(6,8-dioxo-2,7-diazaspiro[4.6]undecan-2-yl)methyl]benzoate | Colorless viscous material |
| | | MS (ESI) m/z: 331.0 (M+H)⁺ |
| 35 | 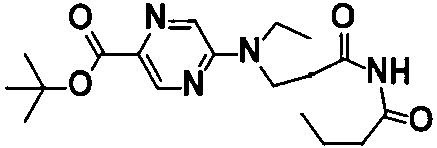 tert-Butyl 5-(6,8-dioxo-2,7-diazaspiro[4.6]undecan-2-yl)pyrazine-2-carboxylate | White solid |
| | | MS (ESI) m/z: 361.1 (M+H)⁺ |
| 36 | 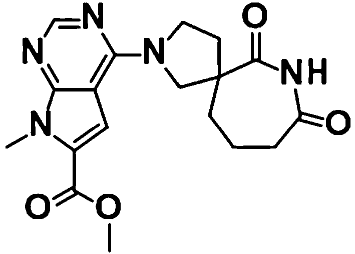 Methyl 4-(6,8-dioxo-2,7-diazaspiro[4.6]undecan-2-yl)-7-methyl-pyrrolo[2,3-d]pyrimidine-6-carboxylate | White solid |
| | | MS (ESI) m/z: 372.0 (M+H)⁺ |
| 37 | 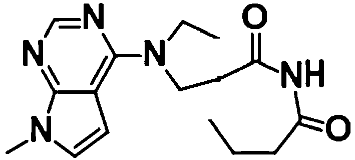 2-(7-methylpyrrolo[2,3-d]pyrimidin-4-yl)-2,7-diazaspiro[4.6]undecane-6,8-dione | White solid |
| | | MS (ESI) m/z: 314.0 (M+H)⁺ |
| 38 | 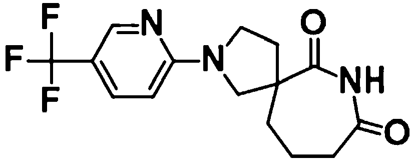 2-[5-(trifluoromethyl)-2-pyridyl]-2,7-diazaspiro[4.6]undecane-6,8-dione | Pale yellow viscous material |
| | | MS (ESI) m/z: 328.0 (M+H)⁺ |
| 39 | 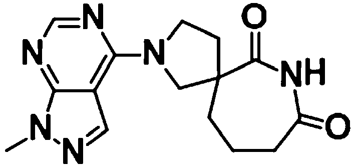 2-(1-methylpyrazolo[3,4-d]pyrimidin-4-yl)-2,7-diazaspiro[4.6]undecane-6,8-dione | Beige solid |
| | | MS (ESI) m/z: 315.0 (M+H)⁺ |
| 40 | 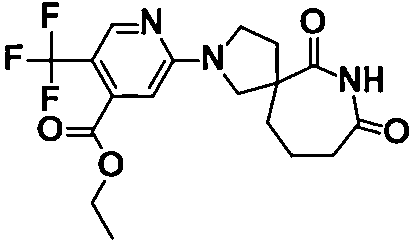 Ethyl 2-(6,8-dioxo-2,7-diazaspiro[4.6]undecan-2-yl)-5-(trifluoromethyl)pyridine-4-carboxylate | Pale yellow solid |
| | | MS (ESI) m/z: 400.2 (M+H)⁺ |
| 41 | 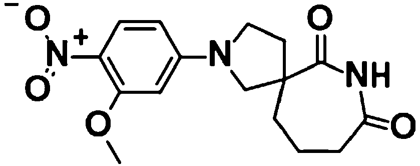 2-(3-Methoxy-4-nitrophenyl)-2,7-diazaspiro[4.6]undecane-6,8-dione | Yellow solid |
| | | MS (ESI) m/z: 334.0 (M+H)⁺ |
| 42 | 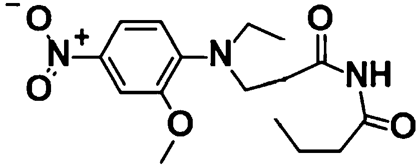 2-(2-Methoxy-4-nitrophenyl)-2,7-diazaspiro[4.6]undecane-6,8-dione | Orange solid |
| | | MS (ESI) m/z: 334.0 (M+H)⁺ |
| 43 | 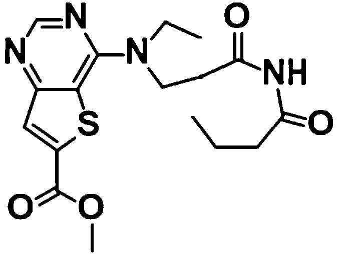 Methyl 4-(6,8-dioxo-2,7-diazaspiro[4.6]undecan-2-yl)thieno[3,2-d]pyrimidine-6-carboxylate | Pale yellow solid |
| | | MS (ESI) m/z: 375.2 (M+H)⁺ |

### Example 44

### (44-1) tert-Butyl 6-(2,6-dioxopiperidin-3-yl)-5,7-dioxo-2,6-diazaspiro[3.4]octane-2-carboxylate (Example Compound 44-1)

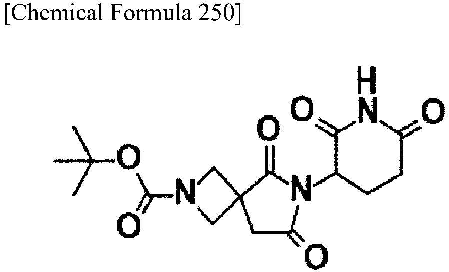

A solution of 1-Boc-3-(2-methoxy-2-oxoethyl)azetidine-3-carboxylic acid (9 g) and lithium hydroxide hydrate (3.45 g) in tetrahydrofuran (30 mL) and water (60 mL) was stirred at room temperature for 16 hours. 1M hydrochloric acid was added to adjust the pH to 4, and the mixture was extracted twice with ethyl acetate. The organic layer was concentrated under reduced pressure to obtain a white solid (7 g). The obtained white solid (3 g) was stirred in acetic anhydride (20 mL) at 120 °C for 30 minutes. The reaction mixture was concentrated under reduced pressure, and pyridine (20 mL) and 3-aminopiperidine-2,6-dione (1.48 g) were added, followed by stirring at 140 °C for 16 hours. The reaction mixture was concentrated under reduced pressure, and the residue was washed by suspension in methanol-water to obtain the title compound (1.03 g) as a white solid. MS (ESI) m/z: 252.0 (M+H-Boc)⁺.

### (44-2) 2-Benzoyl-6-(2,6-dioxopiperidin-3-yl)-2,6-diazaspiro[3.4]octane-5,7-dione (Example Compound 44-2)

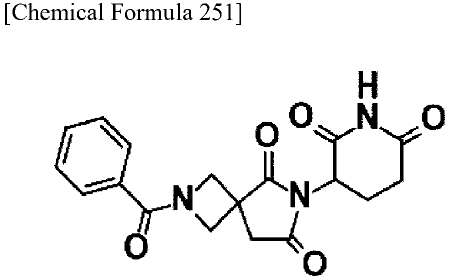

1) To a solution of Example Compound 44-1 (50.0 mg) in chloroform (1 mL), trifluoroacetic acid (1 mL) was added, and the mixture was stirred at room temperature for 2 hours. The reaction mixture was concentrated under reduced pressure, and azeotropic distillation with toluene was performed twice. The residue, benzoyl chloride (0.0165 mL), and pyridine (0.0309 mL) were stirred in tetrahydrofuran (3 mL) at room temperature for 3 hours. Saturated aqueous sodium bicarbonate was added to the reaction mixture, followed by stirring, and the mixture was extracted twice with ethyl acetate. The organic layer was concentrated under reduced pressure, and the residue was purified by silica gel column chromatography (chloroform:methanol = 100:0 to 95:5) to obtain the title compound (46.0 mg) as a white solid. MS (ESI) m/z: 355.9 (M+H)⁺.

### Example 45

### (45-1) tert-Butyl 6-[6-(2,6-dioxopiperidin-3-yl)-5,7-dioxo-2,6-diazaspiro[3.4]octan-2-yl]pyridine-3-carboxylate (Example Compound 45)

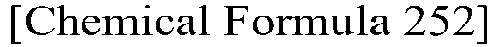

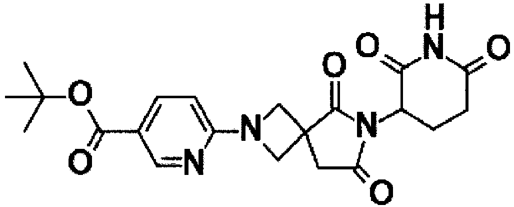

To a solution of Example Compound 44-1 (100 mg) in chloroform (1 mL), trifluoroacetic acid (1 mL) was added, and the mixture was stirred at room temperature for 1 hour. The reaction mixture was concentrated under reduced pressure, and azeotropic distillation with toluene was performed twice. The residue, tert-butyl 6-fluoropyridine-3-carboxylate (55.6 mg), and N,N-diisopropylethylamine (0.133 mL) were stirred in N,N-dimethylformamide (3 mL) at 50 °C for 4 hours. The reaction mixture was concentrated under reduced pressure, and the residue was purified by silica gel column chromatography (chloroform:methanol = 100:0 to 95:5) to obtain the title compound (112 mg) as a white solid. MS (ESI) m/z: 429.1 (M+H)⁺.

### Examples 46 - 54:

The corresponding starting compounds were processed in the same manner as in Example 45 to obtain the compounds listed in Table 5 below.

**[Table 5]**

| Example | Structural formula | Physical properties etc. |
|---|---|---|
| 46 | 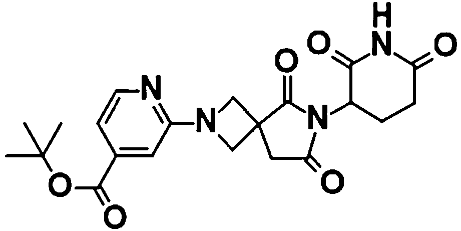 tert-Butyl 2-[6-(2, 6-dioxopiperidin-3-yl)-5,7-dioxo-2,6-diazaspiro[3. 4]octan-2-yl]pyridine-4-carboxylate | White solid |
| | | MS (ESI) m/z: 429.1 (M+H)⁺ |
| 47 | 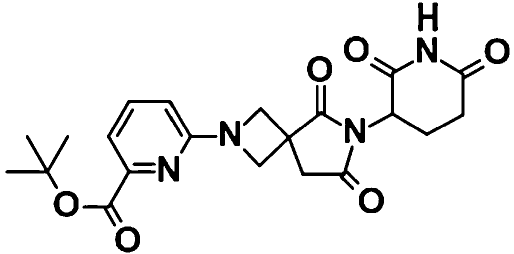 tert-Butyl 6-[6-(2, 6-dioxopiperidin-3-yl)-5,7-dioxo-2,6-diazaspiro[3. 4]octan-2-yl]pyridine-2-carboxylate | White solid |
| | | MS (ESI) m/z: 429.1 (M+H)⁺ |
| 48 | 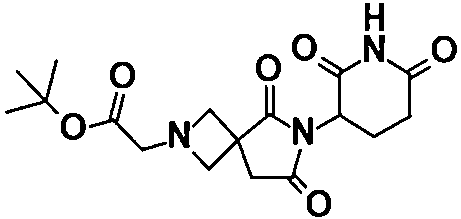 tert-Butyl 2-[6-(2, 6-dioxopiperidin-3-yl)-5, 7-dioxo-2,6-diazaspiro[3. 4]octan-2-yl]acetate | White solid |
| | | MS (ESI) m/z: 366.0 (M+H)⁺ |
| 49 | 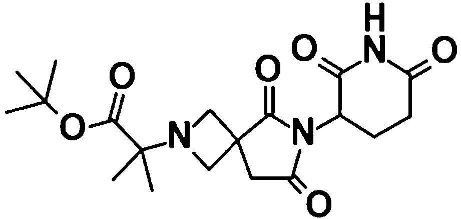 tert-Butyl 2-[6-(2, 6-dioxopiperidin-3-yl)-5, 7-dioxo-2,6-diazaspiro[3. 4]octan-2-yl]-2-methylpropanoate | White solid |
| | | MS (ESI) m/z: 394. 1 (M+H)⁺ |
| 50 | 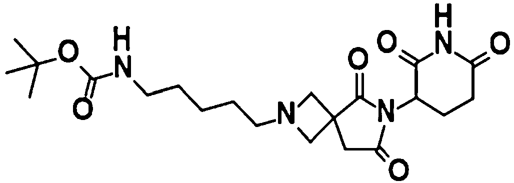 tert-Butyl N-[5-[6-(2, 6-dioxopiperidin-3-yl)-5, 7-dioxo-2,6-diazaspiro[3. 4]octan-2-yl]pentyl]carbamate | White solid |
| | | MS (ESI) m/z: 437. 1 (M+H)⁺ |
| 51 | 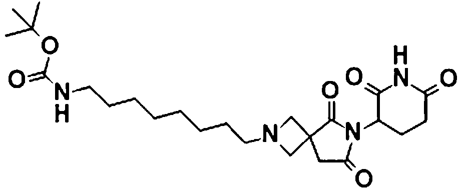 tert-Butyl N-[8-[6-(2, 6-dioxopiperidin-3-yl)-5, 7-dioxo-2,6-diazaspiro[3. 4]octan-2-yl]octyl]carbamate | White solid |
| | | MS (ESI) m/z: 479.2 (M+H)⁺ |
| 52 | 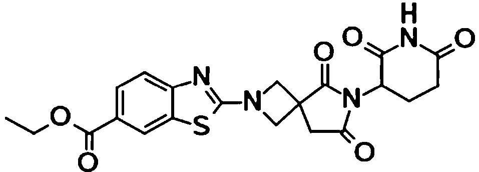 Ethyl 2-[6-(2, 6-dioxopiperidin-3-yl)-5,7-dioxo-2,6-diazaspiro[3. 4]octan-2-yl]-1, 3-benzothiazole-6-carboxylate | Beige solid |
| | | MS (ESI) m/z: 457.0 (M+H)⁺ |
| 53 | 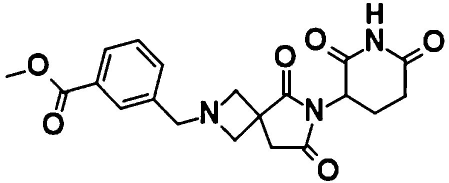 Methyl 3-[[6-(2, 6-dioxopiperidin-3-yl)-5,7-dioxo-2,6-diazaspiro[3. 4]octan-2-yl]methyl]benzoate | White solid |
| | | MS (ESI) m/z: 400.0 (M+H)⁺ |
| 54 | 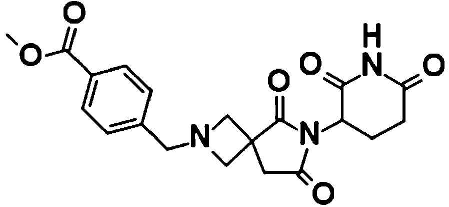 Methyl 4-[[6-(2, 6-dioxopiperidin-3-yl)-5,7-dioxo-2,6-diazaspiro[3. 4]octan-2-yl]methyl]benzoate | White solid |
| | | MS (ESI) m/z: 400.0 (M+H)⁺ |

### Example 55

### (55-1) N-[2-[6-(2,6-Dioxopiperidin-3-yl)-5,7-dioxo-2,6-diazaspiro[3.4]octan-2-yl]-2-oxoethyl]benzamide (Example Compound 55)

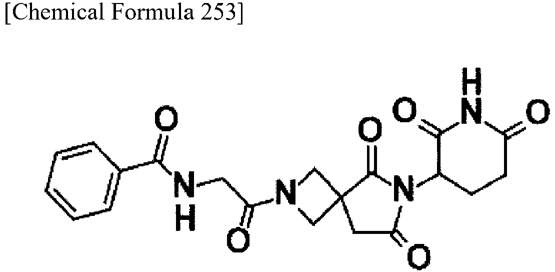

To a solution of Example Compound 44-1 (50.0 mg) in chloroform (1 mL), trifluoroacetic acid (1 mL) was added, and the mixture was stirred at room temperature for 2 hours. The reaction mixture was concentrated under reduced pressure, and azeotropic distillation with toluene was performed twice. The residue, hippuric acid (25.2 mg), HATU (73.0 mg), and N,N-diisopropylethylamine (0.111 mL) were stirred in N,N-dimethylformamide (3 mL) at room temperature for 3 hours. Water was added to the reaction mixture, followed by stirring, and the mixture was extracted twice with ethyl acetate. The organic layer was washed twice with water and then concentrated under reduced pressure. The residue was purified by silica gel column chromatography (chloroform:methanol = 100:0 to 90:10) to obtain the title compound (10.9 mg) as a pale yellow solid. MS (ESI) m/z: 413.0 (M+H)⁺.

### Reference Example and Example 56

### (56-1) tert-Butyl 6-(1-oxo-2,11-diazaspiro[6.7]tetradecan-11-yl)pyridine-3-carboxylate (Reference Example Compound 56)

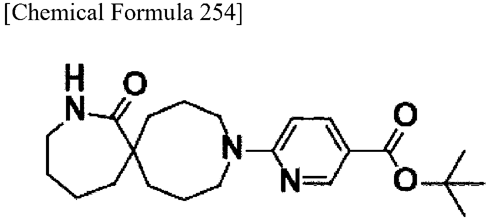

Using tert-butyl 1-oxo-2,11-diazaspiro[6.7]tetradecane-11-carboxylate instead of Example Compound 12-1 in Example 15, the same reaction and processing as in (15-1) were performed to obtain the title compound as a white solid. MS (ESI) m/z: 388.1 (M+H)⁺.

### (56-2) tert-Butyl 6-(1,3-dioxo-2,11-diazaspiro[6.7]tetradecan-11-yl)pyridine-3-carboxylate (Example Compound 56)

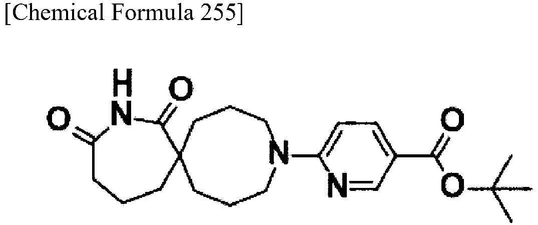

Using Reference Example Compound 56 instead of tert-butyl 10-oxo-8-oxa-2,11-diazaspiro[5.6]dodecane-2-carboxylate in Example 12, the same reaction and processing as in (12-1) were performed to obtain the title compound as a beige solid. MS (ESI) m/z: 402.1 (M+H)⁺.

### Reference Example and Example 57

### (57-1) N-[(2-Oxoazepan-3-yl)methyl]benzamide (Reference Example Compound 57)

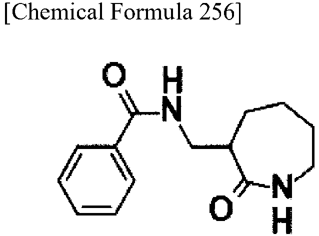

3-(Azidomethyl)azepan-2-one (0.5 M tert-butyl methyl ether solution) (1.00 mL), triphenylphosphine (170 mg), and water (0.5 mL) were stirred in tetrahydrofuran (5 mL) at 50 °C for 2 hours. The reaction mixture was concentrated under reduced pressure, and azeotropic distillation with toluene was performed twice. The residue was dissolved in tetrahydrofuran (5 mL), and pyridine (0.160 mL) and benzoyl chloride (0.0640 mL) were added, followed by stirring at room temperature for 3 hours. The reaction mixture was concentrated under reduced pressure, and the residue was purified by silica gel column chromatography (chloroform:methanol = 100:0 to 95:5) and silica gel column chromatography (ethyl acetate:methanol = 100:0 to 95:5) to obtain the title compound (36.9 mg) as a white solid. MS (ESI) m/z: 247.0 (M+H)⁺.

### (57-2) N-[(2,7-Dioxoazepan-3-yl)methyl]benzamide (Example Compound 57)

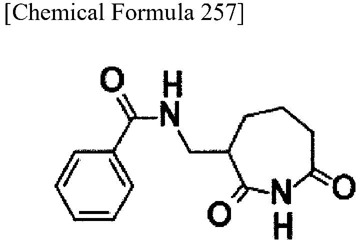

Using Reference Example Compound 57 instead of tert-butyl 10-oxo-8-oxa-2,11-diazaspiro[5.6]dodecane-2-carboxylate in Example 12, the same reaction and processing as in (12-1) were performed to obtain the title compound as a colorless viscous material. MS (ESI) m/z: 261.0 (M+H)⁺.

### Example 58

### (58-1) tert-Butyl 6-(2,6-dioxo-3-piperidyl)-5-oxo-2,6-diazaspiro[3.4]octane-2-carboxylate (Example Compound 58-1)

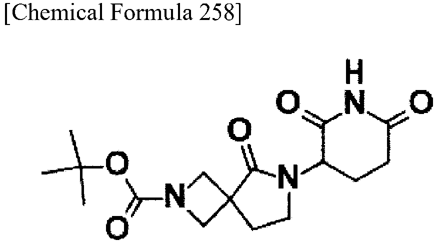

A suspension of sodium hydride (194 mg) in N,N-dimethylformamide (5 mL) was cooled to 0 °C, and tert-butyl 5-oxo-2,6-diazaspiro[3.4]octane-2-carboxylate (500 mg) was added, followed by stirring at the same temperature for 10 minutes. 3-Bromopiperidine-2,6-dione (424 mg) was added, and the mixture was warmed to room temperature and stirred for 2 hours. Water was added to the reaction mixture, followed by stirring, and the mixture was extracted three times with ethyl acetate. The organic layer was washed twice with water and then concentrated under reduced pressure. The residue was purified by silica gel column chromatography (chloroform:methanol = 100:0 to 95:5) to obtain the title compound (316 mg) as a pale yellow solid. MS (ESI) m/z: 338.0 (M+H)⁺.

### (58-2) Ethyl 2-[6-(2,6-dioxo-3-piperidyl)-5-oxo-2,6-diazaspiro[3.4]octan-2-yl]-1,3-benzothiazole-6-carboxylate (Example Compound 58-2)

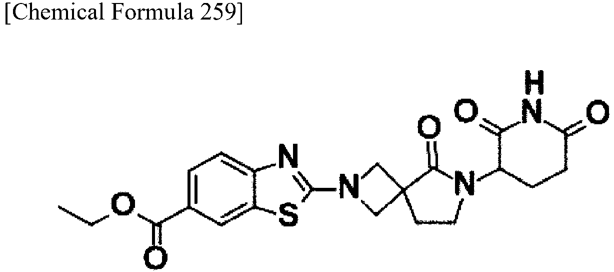

Using Example Compound 58-1 instead of Example Compound 44-1 and ethyl 2-chloro-1,3-benzothiazole-6-carboxylate instead of tert-butyl 6-fluoropyridine-3-carboxylate in Example 45, the same reaction and processing as in (45-1) were performed to obtain the title compound as a pale yellow solid. MS (ESI) m/z: 443.0 (M+H)⁺.

### Example 59

### (59-1) Ethyl 2-[6-(2,6-dioxo-3-piperidyl)-7-oxo-2,6-diazaspiro[3.4]octan-2-yl]-1,3-benzothiazole-6-carboxylate (Example Compound 59)

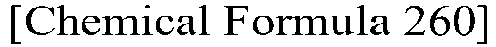

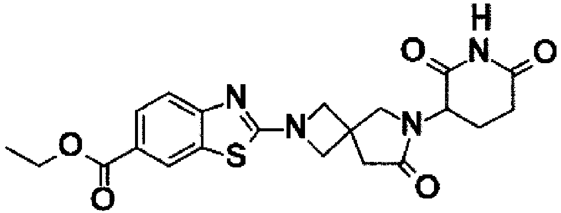

Using tert-butyl 7-oxo-2,6-diazaspiro[3.4]octane-2-carboxylate instead of tert-butyl 5-oxo-2,6-diazaspiro[3.4]octane-2-carboxylate in Example 58, the same reactions and processing as in (58-1) and (58-2) were performed to obtain the title compound as a pale yellow solid. MS (ESI) m/z: 443.0 (M+H)⁺.

### Reference Example and Example 60

### (60-1) 3-O-Benzyl 1-O-methyl 2-[3-[(2-methylpropan-2-yl)oxycarbonyl]phenyl]propanedioate (Reference Example Compound 60-1)

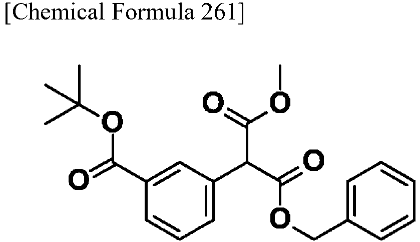

To a solution of benzyl methyl malonate (7.13 g) in tetrahydrofuran (30 mL), sodium hydride (60% oil suspension, 0.82 g) was added at room temperature, and the mixture was stirred at room temperature for 2 minutes. A solution of tert-butyl 3-bromobenzoate (8.00 g) in tetrahydrofuran (60 mL) and bis(tri-tert-butylphosphine)palladium(II) (477 mg) were added thereto, and the mixture was heated and stirred at 80 °C overnight. The reaction mixture was cooled, saturated aqueous ammonium chloride was added, and the mixture was extracted with ethyl acetate. The organic layer was washed with saturated brine and dried over anhydrous sodium sulfate, and then the drying agent was removed by filtration, and the filtrate was concentrated under reduced pressure. The residue was purified by silica gel column chromatography (hexane:ethyl acetate = 100:0 to 90:10) to obtain the title compound (9.26 g) as a colorless oil. MS (ESI) m/z: 383.2 (M-H)⁻.

### (60-2) tert-Butyl 3-(2-methoxy-2-oxoethyl)benzoate (Reference Example Compound 60-2)

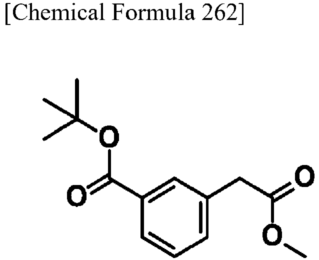

To a solution of 3-O-benzyl 1-O-methyl 2-[3-[(2-methylpropan-2-yl)oxycarbonyl]phenyl]propanedioate (4.00 g) in methanol (50 mL), 5% palladium on carbon (200 mg) was added, and the mixture was stirred under a hydrogen atmosphere at room temperature for 24 hours. The insoluble material in the reaction mixture was filtered off using Celite, and then the filtrate was concentrated under reduced pressure. The residue was dissolved in methanol (50 mL) and heated and stirred for 2 hours. The reaction mixture was concentrated under reduced pressure, and the residue was purified by silica gel column chromatography (hexane:ethyl acetate = 95:5 to 80:20) to obtain the title compound (1.99 g) as a colorless oil.

### (60-3) tert-Butyl 3-(6-chloro-1-methoxy-1-oxohexan-2-yl)benzoate (Reference Example Compound 60-3)

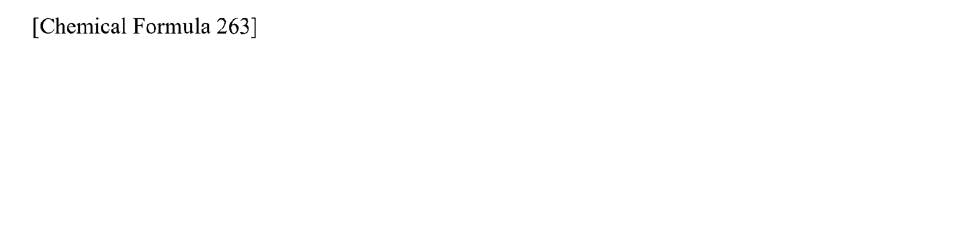

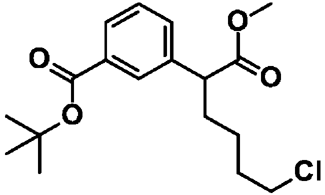

To a suspension of sodium hydride (60% oil suspension, 220 mg) in N,N-dimethylformamide (10 mL), a solution of tert-butyl 3-(2-methoxy-2-oxoethyl)benzoate (1.06 g) in N,N-dimethylformamide (8 mL) was added dropwise under ice-cooling, and the mixture was stirred at 0 °C for 15 minutes, and then at room temperature for 30 minutes. The reaction mixture was cooled again in an ice bath, and a solution of 1-bromo-4-chlorobutane (0.94 g) in N,N-dimethylformamide was added, followed by stirring at room temperature for 1 hour. Aqueous ammonium chloride was added to the reaction mixture, and the mixture was extracted with ethyl acetate. The organic layer was washed with saturated brine and dried over anhydrous sodium sulfate, and then the drying agent was removed by filtration, and the filtrate was concentrated under reduced pressure. The residue was purified by silica gel column chromatography (hexane:ethyl acetate = 90:10 to 70:30) to obtain the title compound (0.58 g) as a colorless oil. MS (ESI) m/z: 282.9/284.8 (M-tBu) -.

### (60-4) tert-Butyl 3-(6-azido-1-methoxy-1-oxohexan-2-yl)benzoate (Reference Example Compound 60-4)

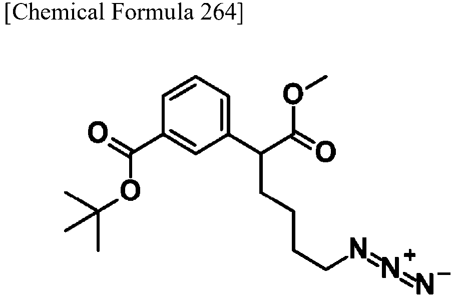

To a solution of tert-butyl 3-(6-chloro-1-methoxy-1-oxohexan-2-yl)benzoate (588 mg) in N,N-dimethylformamide (8 mL), tetrabutylammonium azide (672 mg) was added, and the mixture was stirred at 80 °C for 2 hours. The reaction mixture was cooled, water was added, and the mixture was extracted with hexane-diethyl ether (1:1). The organic layer was washed with saturated brine, and dried over anhydrous sodium sulfate, and then the drying agent was removed by filtration, and the filtrate was concentrated under reduced pressure to obtain the title compound (585 mg) as a pale yellow oil.

### (60-5) tert-Butyl 3-(6-amino-1-methoxy-1-oxohexan-2-yl)benzoate (Reference Example Compound 60-5)

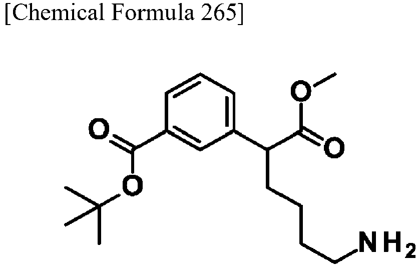

To a solution of tert-butyl 3-(6-azido-1-methoxy-1-oxohexan-2-yl)benzoate (585 mg) in tetrahydrofuran (10 mL), triphenylphosphine (664 mg) and purified water (1 mL) were added, and the mixture was stirred at 80 °C for 2 hours. The reaction mixture was cooled and concentrated under reduced pressure. The residue was purified by NH silica gel column chromatography (ethyl acetate:methanol = 100:0 to 95:5) to obtain the title compound (657 mg) as a colorless oil. MS (ESI) m/z: 322.2 (M+H)⁺

### (60-6) tert-Butyl 3-(2-oxoazepan-3-yl)benzoate (Reference Example Compound 60-6)

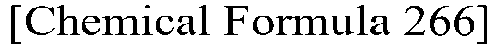

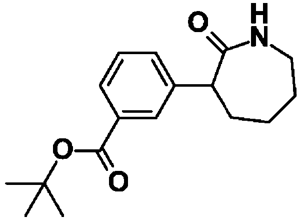

To a solution of tert-butyl 3-(6-amino-1-methoxy-1-oxohexan-2-yl)benzoate (657 mg) in ethanol (10 mL), 2 mol/L aqueous sodium hydroxide (1.09 mL) was added, and the mixture was stirred at room temperature for 1.5 hours. An additional 2 mol/L aqueous sodium hydroxide (0.73 mL) was added, followed by stirring at room temperature for 1 hour, and then the reaction mixture was concentrated under reduced pressure. The resulting residue was mixed with N,N-dimethylformamide (10 mL), and HATU (828 mg) and N,N-diisopropylethylamine (0.75 mL) were added, followed by stirring at room temperature overnight. Water was added to the reaction mixture, which was then extracted with ethyl acetate. The organic layer was washed with saturated brine and then concentrated under reduced pressure. The residue was purified by silica gel column chromatography (chloroform:ethyl acetate = 90:10 to 40:60) to obtain the title compound (196 mg) as a colorless powder. MS (ESI) m/z: 290.1 (M+H)⁺

### (60-7) tert-Butyl 3-(2,7-dioxoazepan-3-yl)benzoate (Example compound 60)

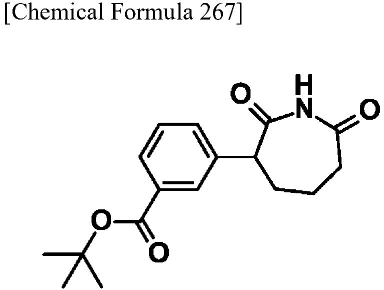

Using Reference Example Compound 60-6 instead of Reference Example Compound 7-2 in Reference Example and Example 7, the same reaction and processing as in (7-3) were performed to obtain the title compound as a colorless powder. MS (ESI) m/z: 302.1 (M-H)⁻.

### Reference Example and Example 61

### (61-1) Ethyl 6-bromo-2-(4-nitrophenyl)hexanoate (Reference Example Compound 61-1)

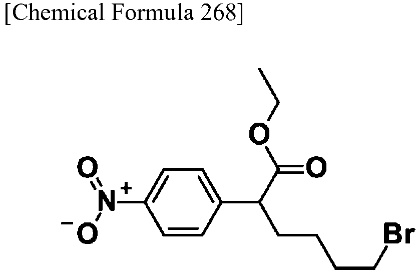

Using ethyl 4-nitrophenylacetate instead of Reference Example Compound 60-2 and 1,4-dibromobutane instead of 1-bromo-4-chlorobutane in Reference Example and Example 60, the same reaction and processing as in (60-3) were performed to obtain the title compound as a colorless oil.

### (61-2) Ethyl 6-azido-2-(4-nitrophenyl)hexanoate (Reference Example Compound 61-2)

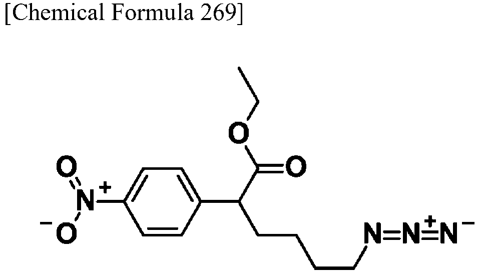

Using Reference Example Compound 61-1 instead of Reference Example Compound 60-3 in Reference Example and Example 60, the same reaction and processing as in (60-4) were performed to obtain the title compound as a yellow oil. MS (ESI) m/z: 305.1 (M-H)⁻.

### (61-3) Ethyl 6-amino-2-(4-nitrophenyl)hexanoate (Reference Example Compound 61-3)

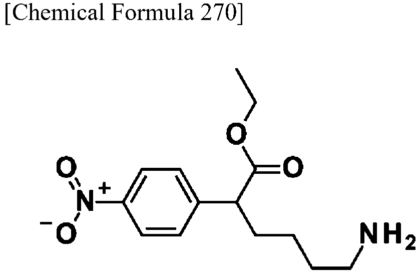

Using Reference Example Compound 61-2 instead of Reference Example Compound 60-4 in Reference Example and Example 60, the same reaction and processing as in (60-5) were performed to obtain the title compound as a yellow oil. MS (ESI) m/z: 281.2 (M+H)⁺.

### (61-4) 3-(4-Nitrophenyl)azepan-2-one (Reference Example Compound 61-4)

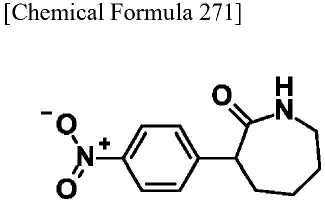

Using Reference Example Compound 61-3 instead of Reference Example Compound 60-5 in Reference Example and Example 60, the same reaction and processing as in (60-6) were performed to obtain the title compound as a yellow powder. MS (ESI) m/z: 235.1 (M+H)⁺.

### (61-5) 3-(4-Nitrophenyl)azepane-2,7-dione (Example Compound 61)

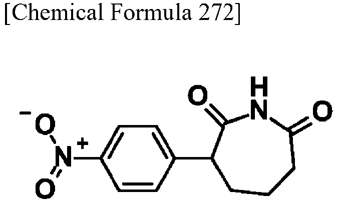

Using Reference Example Compound 61-4 instead of Reference Example Compound 7-2 in Reference Example and Example 7, the same reaction and processing as in (7-3) were performed to obtain the title compound as a pale pink powder. MS (ESI) m/z: 249.1 (M+H)⁺.

### Reference Example and Example 62

### (62-1) Methyl 6-chloro-2-(3-nitrophenyl)hexanoate (Reference Example Compound 62-1)

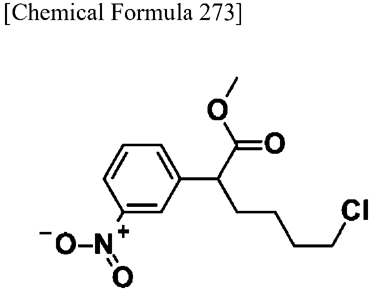

Using methyl 3-nitrophenylacetate instead of Reference Example Compound 60-2 in Reference Example and Example 60, the same reaction and processing as in (60-3) were performed to obtain the title compound as a pale yellow oil.

### (62-2) Methyl 6-azido-2-(3-nitrophenyl)hexanoate (Reference Example Compound 62-2)

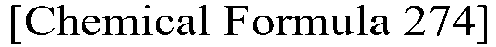

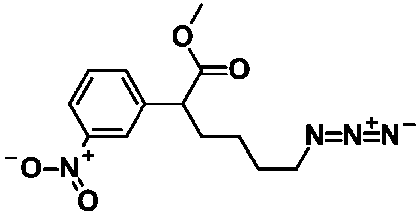

Using Reference Example Compound 62-1 instead of Reference Example Compound 60-3 in Reference Example and Example 60, the same reaction and processing as in (60-4) were performed to obtain the title compound as a yellow oil. MS (ESI) m/z: 291.0 (M-H)⁻.

### (62-3) Methyl 6-amino-2-(3-nitrophenyl)hexanoate (Reference Example Compound 62-3)

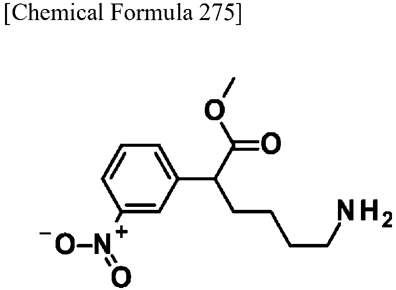

Using Reference Example Compound 62-2 instead of Reference Example Compound 60-4 in Reference Example and Example 60, the same reaction and processing as in (60-5) were performed to obtain the title compound as a yellow oil. MS (ESI) m/z: 267.1 (M+H)⁺.

### (62-4) 3-(3-Nitrophenyl)azepan-2-one (Reference Example Compound 62-4)

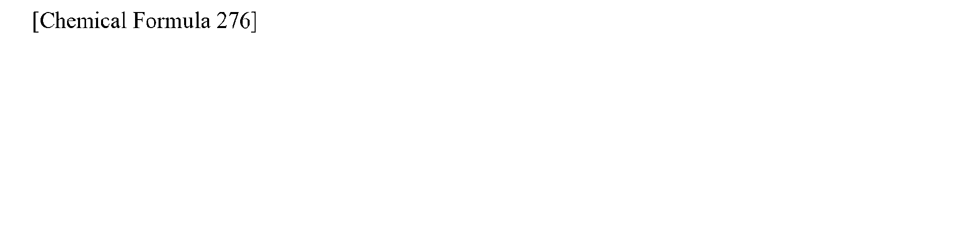

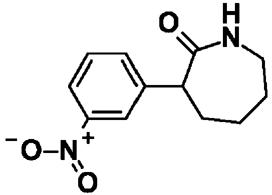

Using Reference Example Compound 62-3 instead of Reference Example Compound 60-5 in Reference Example and Example 60, the same reaction and processing as in (60-6) were performed to obtain the title compound as a yellow powder. MS (ESI) m/z: 235.1 (M+H)⁺.

### (62-5) 3-(3-Nitrophenyl)azepane-2,7-dione (Example Compound 62)

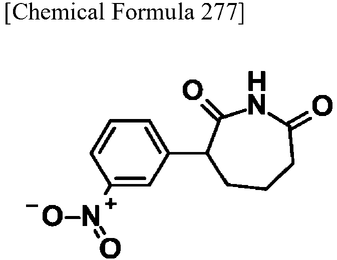

Using Reference Example Compound 62-4 instead of Reference Example Compound 7-2 in Reference Example and Example 7, the same reaction and processing as in (7-3) were performed to obtain the title compound as a pale pink powder. MS (ESI) m/z: 249.0 (M+H)⁺.

### Reference Example and Example 63

### (63-1) Methyl 6-chloro-2-(2-chloro-5-nitrophenyl)hexanoate (Reference Example Compound 63-1)

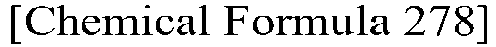

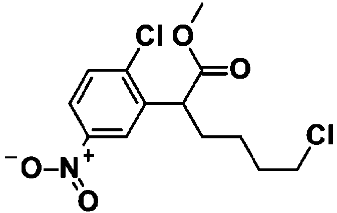

Using methyl 2-chloro-5-nitrophenylacetate instead of Reference Example Compound 60-2 in Reference Example and Example 60, the same reaction and processing as in (60-3) were performed to obtain the title compound as a pale yellow oil.

### (63-2) Methyl 6-azido-2-(2-chloro-5-nitrophenyl)hexanoate (Reference Example Compound 63-2)

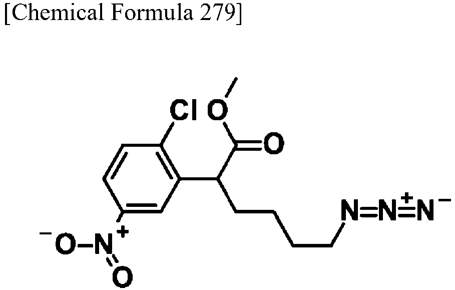

Using Reference Example Compound 63-1 instead of Reference Example Compound 60-3 in Reference Example and Example 60, the same reaction and processing as in (60-4) were performed to obtain the title compound as a yellow oil.

### (63-3) Methyl 6-amino-2-(2-chloro-5-nitrophenyl)hexanoate (Reference Example Compound 63-3)

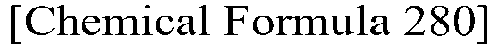

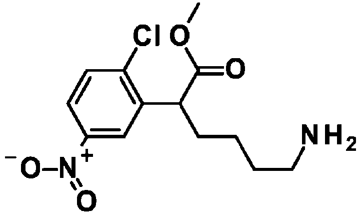

Using Reference Example Compound 63-2 instead of Reference Example Compound 60-4 in Reference Example and Example 60, the same reaction and processing as in (60-5) were performed to obtain the title compound as a yellow oil. MS (ESI) m/z: 301.2/303.2 (M+H)⁺.

### (63-4) 3-(2-Chloro-5-nitrophenyl)azepan-2-one (Reference Example Compound 63-4)

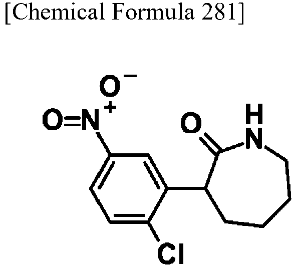

Using Reference Example Compound 63-3 instead of Reference Example Compound 60-5 in Reference Example and Example 60, the same reaction and processing as in (60-6) were performed to obtain the title compound as a yellow powder. MS (ESI) m/z: 269.1/271.1 (M+H)⁺.

### (63-5) 3-(2-Chloro-5-nitrophenyl)azepane-2,7-dione (Example Compound 63)

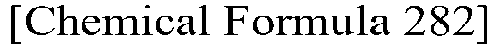

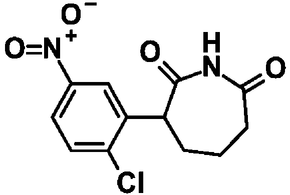

Using Reference Example Compound 63-4 instead of Reference Example Compound 7-2 in Reference Example and Example 7, the same reaction and processing as in (7-3) were performed to obtain the title compound as a pale pink powder. MS (ESI) m/z: 283.1/285.1 (M+H)⁺.

### Example 64

### 3-(5-Amino-2-chlorophenyl)azepane-2,7-dione (Example Compound 64)

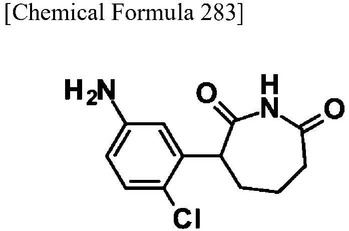

3-(2-Chloro-5-nitrophenyl)azepane-2,7-dione (40 mg) was dissolved in methanol (4 mL) and tetrahydrofuran (4 mL), and palladium/Fibroin (10 mg) was added. The atmosphere was replaced with hydrogen, and the mixture was stirred at room temperature overnight. The reaction mixture was filtered through Celite, and the filtrate was concentrated to obtain 3-(5-amino-2-chlorophenyl)azepane-2,7-dione.

### Example 65

### N-[4-Chloro-3-(2,7-dioxoazepan-3-yl)phenyl]acetamide (Example Compound 65)

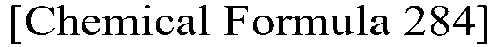

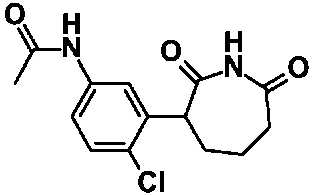

Using Example Compound 64 instead of Example Compound 70 in Example 71, the same reaction and processing as in Example 71 were performed to obtain the title compound as a yellow oil. MS (ESI) m/z: 295.1/297.1 (M+H)⁺.

### Reference Example and Example 66

### (66-1) Methyl 2-(2-nitrophenoxy)-6-(phenylmethoxycarbonylamino)hexanoate (Reference Example Compound 66-1)

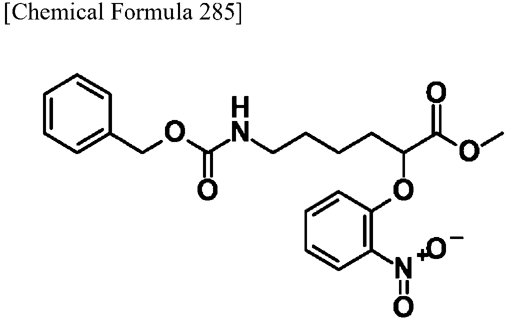

To a solution of methyl 2-(2-hydroxy)-6-(phenylmethoxycarbonylamino)hexanoate (1 g), 2-nitrophenol (612 mg), and triphenylphosphine (1.33 g) in tetrahydrofuran (30 mL), DIAD (1.9 mol/L, 2.7 mL) was added under ice-cooling, and the mixture was stirred at 0 °C for 1 hour and at room temperature for 2 hours. The reaction mixture was diluted with ethyl acetate, washed sequentially with 1N hydrochloric acid, saturated aqueous sodium bicarbonate, and saturated brine, and dried over anhydrous sodium sulfate, and then the drying agent was removed by filtration, and the filtrate was concentrated under reduced pressure. The residue was purified by silica gel column chromatography (hexane:ethyl acetate = 90:10 to 40:60) to obtain the title compound (1.41 g) as a colorless oil. MS (ESI) m/z: 417.2 (M+H)⁺.

### (66-2) 2-(2-Nitrophenoxy)-6-(phenylmethoxycarbonylamino)hexanoic acid (Reference Example Compound 66-2)

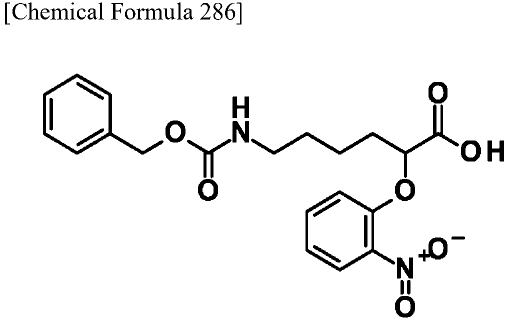

To a solution of methyl 2-(2-nitrophenoxy)-6-(phenylmethoxycarbonylamino)hexanoate (1.41 g) in methanol (30 mL), 2 mol/L aqueous sodium hydroxide (4.06 mL) was added, and the mixture was stirred at room temperature for 1.5 hours. An additional 2 mol/L aqueous sodium hydroxide (0.73 mL) was added, and after stirring at room temperature for 2 hours, 2N hydrochloric acid was added to acidify the reaction mixture. Then, the solvent was evaporated, and the mixture was extracted with ethyl acetate. The organic layer was washed with saturated brine, and dried over anhydrous sodium sulfate, and then, the drying agent was removed by filtration, and the filtrate was concentrated under reduced pressure to obtain the title compound (1.25 g) as a yellow viscous material. MS (ESI) m/z: 401.1 [M-H]⁻

### (66-3) 6-Amino-2-(2-nitrophenoxy)hexanoic acid hydrochloride (Reference Example Compound 66-3)

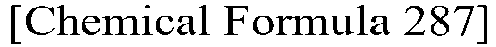

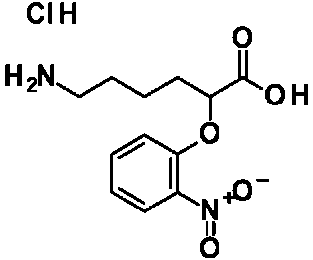

2-(2-Nitrophenoxy)-6-(phenylmethoxycarbonylamino)hexanoic acid (1.25 g) was mixed with 6N hydrochloric acid (10 mL) and heated with stirring at 110 °C for 4 hours. The reaction mixture was concentrated and azeotroped with toluene to obtain the title compound (830 mg) as a light brown solid. MS (ESI) m/z: 269.0 (M+H)⁺

### (66-4) 3-(2-Nitrophenoxy)azepan-2-one (Reference Example Compound 66-4)

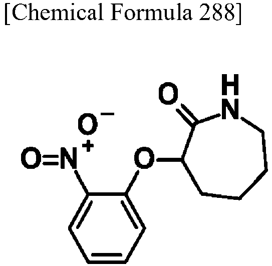

6-Amino-2-(2-nitrophenoxy)hexanoic acid hydrochloride was mixed with N,N-dimethylformamide (15 mL), and HATU (1.4 g) and N,N-diisopropylethylamine (1.4 mL) were added, followed by stirring at room temperature overnight. Water was added to the reaction mixture, which was then extracted with ethyl acetate. The organic layer was washed with saturated brine and then concentrated under reduced pressure. The residue was purified by silica gel column chromatography (ethyl acetate:methanol = 100:0 to 95:5) to obtain the title compound (418 mg) as a light brown powder. MS (ESI) m/z: 251.0 (M+H)⁺.

### (66-5) 3-(2-Nitrophenoxy)azepane-2,7-dione (Example Compound 66-1)

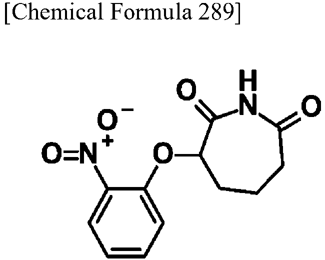

Using Reference Example Compound 66-4 instead of Reference Example Compound 1 in Reference Example and Example 1, the same reaction and processing as in (1-2) were performed to obtain the title compound as a colorless powder. MS (ESI) m/z: 265.0 (M+H)⁺.

### (66-6) 3-(2-Aminophenoxy)azepane-2,7-dione (Example Compound 66-2)

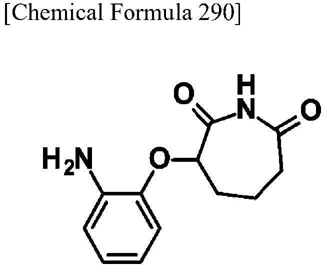

3-(2-Nitrophenoxy)azepane-2,7-dione (20 mg) was dissolved in methanol (2 mL), and palladium on carbon (5 mg) was added. The atmosphere was replaced with hydrogen, and the mixture was stirred at room temperature for 6 hours. The reaction mixture was filtered through Celite, and the filtrate was concentrated to obtain the title compound (15 mg) as a colorless powder. MS (ESI) m/z: 233.0 (M-H)⁻.

### Reference Example and Example 67

### Reference Example 67

### (67-1) Methyl 2-fluoro-3-(2-tert-butoxy-2-oxoethoxy)benzoate (Reference Example Compound 67-1)

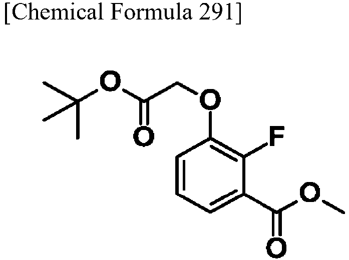

Methyl 2-fluoro-3-hydroxybenzoate (3.00 g), tert-butyl 2-bromoacetate (3.78 g), and potassium carbonate (3.66 g) were suspended in N,N-dimethylformamide (30 mL) and stirred at room temperature for 17 hours. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 95:5 to 80:20) to obtain the title compound (5.01 g) as a colorless viscous material. MS (ESI) m/z: 229.1 (M-tBu+H)⁺

### (67-2) 2-Fluoro-3-[2-[(2-methylpropan-2-yl)oxy]-2-oxoethoxy]benzoic acid (Reference Example Compound 67-2)

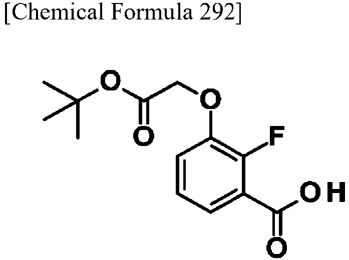

Methyl 2-fluoro-3-(2-tert-butoxy-2-oxoethoxy)benzoate (1.00 g) was dissolved in pyridine (15 mL), and lithium iodide (3.77 g) was added. The mixture was stirred at 110 °C for 7 hours. 1N aqueous hydrochloric acid was added to the reaction mixture, and after extraction with ethyl acetate, washing with 1N aqueous hydrochloric acid and saturated brine was performed, followed by drying over anhydrous sodium sulfate, and then the solvent was evaporated under reduced pressure. The residue was purified by silica gel column chromatography (chloroform:methanol = 100:0 to 92:8) to obtain the title compound (273 mg) as a colorless powder. MS (ESI) m/z: 269.2 (M-H)⁻.

### (67-3) tert-Butyl 2-[2-fluoro-3-[(2-oxoazepan-3-yl)carbamoyl]phenoxy]acetate (Reference Example Compound 67-3)

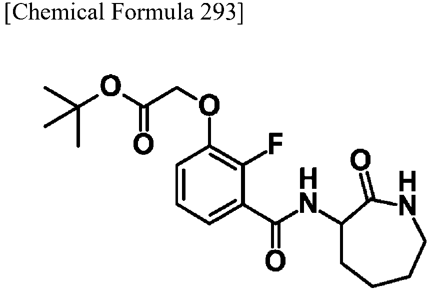

3-Aminoazepan-2-one (303 mg), 3-(2-tert-butoxy-2-oxo-ethoxy)-2-fluorobenzoic acid (500 mg), O-(7-azabenzotriazol-1-yl)-N,N,N',N'-tetramethyluronium hexafluorophosphate (900 mg), and N,N-diisopropylethylamine (305 mg) were dissolved in N,N-dimethylformamide (5 mL) and stirred at room temperature for 2 hours. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 80:20 to 0:100) to obtain the title compound (674 mg) as a colorless powder. MS (ESI) m/z: 381.2 (M+H)⁺

### Example 67

### (67-4) tert-Butyl 2-[3-[(2,7-dioxoazepan-3-yl)carbamoyl]-2-fluorophenoxy]acetate (Example Compound 67)

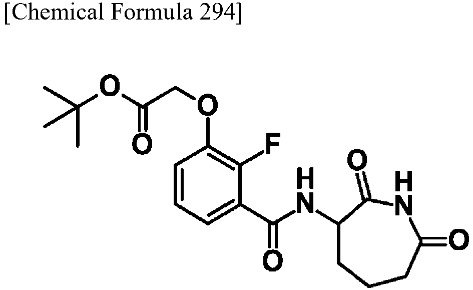

tert-Butyl 2-[2-fluoro-3-[(2-oxoazepan-3-yl)carbamoyl]phenoxy]acetate (670 mg) was dissolved in acetonitrile (20 mL), and water (63 mg) and Dess-Martin periodinane (2310 mg) were added. The mixture was stirred at an external temperature of 80 °C for 3 hours. An aqueous solution of 1N sodium thiosulfate and a saturated aqueous solution of sodium bicarbonate were added to the reaction mixture, followed by stirring for 5 minutes and extraction with ethyl acetate. The organic layer was washed with saturated brine, and dried over anhydrous sodium sulfate, and then the solvent was evaporated under reduced pressure. The residue was purified by silica gel column chromatography (hexane:ethyl acetate = 80:20 to 40:60 to 35:65). After suspension washing with an ethyl acetate-diisopropyl ether mixed solvent, the mixture was filtered and dried under reduced pressure to obtain the titled compound (410 mg) as a colorless powder MS (ESI) m/z: 395.2 (M+H)⁺.

### Reference Example and Example 68

### Reference Example 68

### (68-1) Benzyl 2-methoxy-3-(2-tert-butoxy-2-oxoethoxy)benzoate (Reference Example Compound 68-1)

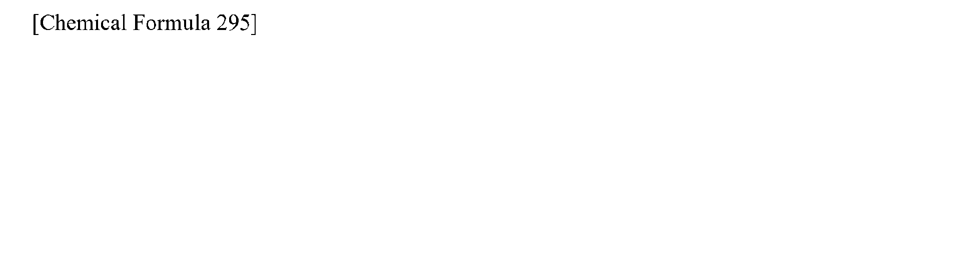

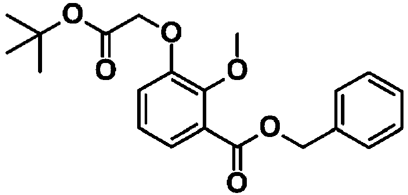

3-Hydroxy-2-methoxybenzoic acid (615 mg) was dissolved in N,N-dimethylformamide (6 mL), and potassium carbonate (760 mg) and benzyl bromide (630 mg) were added. The mixture was stirred at room temperature for 18 hours. tert-Butyl 2-bromoacetate (715 mg) and potassium carbonate (760 mg) were added to the reaction mixture, and the mixture was stirred at room temperature for 5 hours. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 95:5 to 84:16) to obtain the title compound (913 mg) as a colorless viscous material. MS (ESI) m/z: 317.1 (M-tBu+H)⁺

### (68-2) 2-Methoxy-3-(2-tert-butoxy-2-oxoethoxy)benzoic acid (Reference Example Compound 68-2)

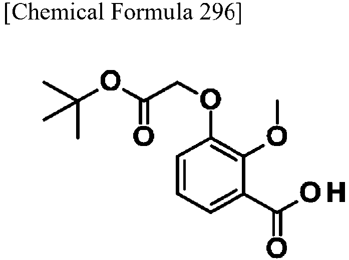

Benzyl 2-methoxy-3-(2-tert-butoxy-2-oxoethoxy)benzoate (910 mg) was dissolved in ethanol (10 mL), and palladium on carbon (50 mg) was added. The atmosphere was replaced with hydrogen, and the mixture was stirred at room temperature for 2 hours. The reaction mixture was filtered through Celite and washed with chloroform. The filtrate was evaporated under reduced pressure to obtain the title compound (730 mg) as a colorless viscous material. MS (ESI) m/z: 281.2 (M-H)⁻.

### Example 68

### (68-3) tert-Butyl 2-[3-[(2,7-dioxoazepan-3-yl)carbamoyl]-2-methoxyphenoxy]acetate (Example Compound 68)

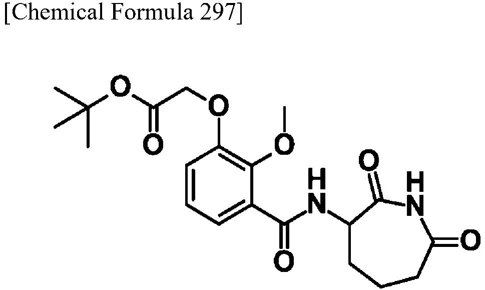

tert-Butyl N-(2,7-dioxoazepan-3-yl)carbamate (100 mg) was dissolved in dichloromethane (1 mL), and trifluoroacetic acid (1 mL) was added, followed by stirring at room temperature for 2 hours. The reaction mixture was concentrated, and the resulting residue was azeotroped with toluene to obtain 3-aminoazepane-2,4-dione trifluoroacetate as a brown viscous material. 3-Aminoazepane-2,4-dione trifluoroacetate, 2-methoxy-3-(2-tert-butoxy-2-oxoethoxy)benzoic acid (115 mg), O-(7-azabenzotriazol-1-yl)-N,N,N',N'-tetramethyluronium hexafluorophosphate (233 mg), and N,N-diisopropylethylamine (166 mg) were dissolved in N,N-dimethylformamide (2 mL) and stirred at room temperature for 20 hours. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 70:30 to 35:65) to obtain the title compound (30 mg) as a colorless powder. MS (ESI) m/z: 407.2 (M+H)⁺.

### Example and Reference Example 69

### Reference Example 69

### (69-1) tert-Butyl 2-(6-((2-oxoazepan-3-yl)carbamoyl)pyridin-2-yl)oxyacetate (Reference Example Compound 69)

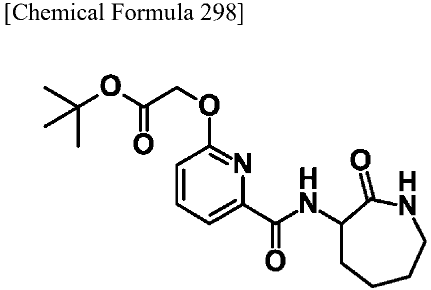

Using 6-(2-((2-methylpropan-2-yl)oxy)-2-oxoethoxy)pyridine-2-carboxylic acid instead of 3-(2-tert-butoxy-2-oxo-ethoxy)-2-fluorobenzoic acid in Reference Example 67-3, the same reaction and processing were performed to obtain the title compound (674 mg) as a colorless powder. MS (ESI) m/z: 308.1 (M+H)⁺

### Example 69

### (69-2) tert-Butyl 2-(6-((2,7-dioxoazepan-3-yl)carbamoyl)pyridin-2-yl)oxyacetate (Example Compound 69)

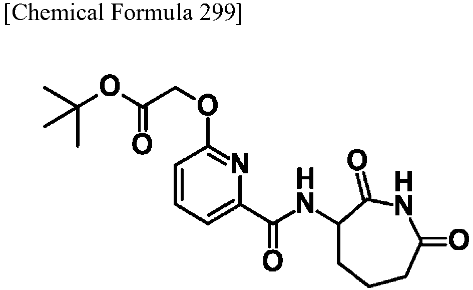

Using tert-butyl 2-(6-((2-oxoazepan-3-yl)carbamoyl)pyridin-2-yl)oxyacetate instead of tert-butyl 2-[2-fluoro-3-[(2-oxoazepan-3-yl)carbamoyl]phenoxy]acetate in Example 67, the same reaction and processing were performed to obtain the title compound (62 mg) as a colorless powder. MS (ESI) m/z: 322.1 (M-tBu+H)⁺

### Example 70

### 3-(3-Aminophenyl)azepane-2,7-dione (Example Compound 70)

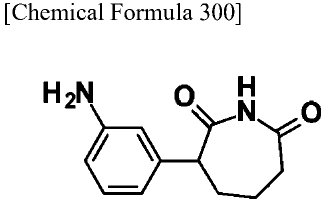

3-(3-Nitrophenyl)azepane-2,7-dione (150 mg) was dissolved in methanol (2 mL), and palladium on carbon (10 mg) was added. The atmosphere was replaced with hydrogen, and the mixture was stirred at room temperature for 6 hours. The reaction mixture was filtered through Celite, and the filtrate was concentrated. The resulting residue was purified by silica gel column chromatography (chloroform:methanol = 100:0 to 96:4) to obtain the title compound (13 mg) as a pale yellow powder. MS (ESI) m/z: 219.2 (M+H)⁺.

### Example 71

### N-[3-(2,7-Dioxoazepan-3-yl)phenyl]acetamide (Example Compound 71)

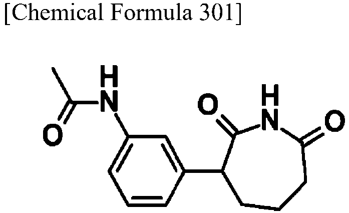

3-(3-Aminophenyl)azepane-2,7-dione (21 mg) was dissolved in dichloromethane (1 mL). Triethylamine (20 mg) and acetyl chloride (11 mg) were added, and the mixture was stirred at room temperature for 0.5 hours. The residue was purified by silica gel column chromatography (chloroform:methanol = 100:0 to 95:5) to obtain the title compound (13 mg) as a colorless powder. MS (ESI) m/z: 261.2 (M+H)⁺.

### Example 72

### 2-(6-Nitro-1,3-benzothiazol-2-yl)-2,7-diazaspiro[4.6]undecane-6,8-dione (Example Compound 72)

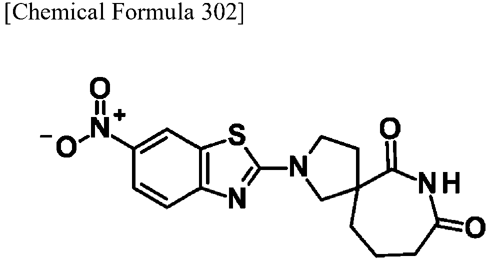

2,7-Diazaspiro[4.6]undecan-6-one:2,2,2-trifluoroacetate (21 mg), 2-chloro-6-nitro-1,3-benzothiazole (16 mg), and N,N-diisopropylethylamine (46 mg) were dissolved in N,N-dimethylformamide (1 mL) and stirred at 80 °C for 1 hour. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 50:50 to 0:100) to obtain the title compound (23 mg) as a yellow powder. MS (ESI) m/z: 361.1 (M+H)⁺.

### Examples 73 - 84:

The corresponding starting compounds were processed in the same manner as in Example 72 to obtain the compounds listed in Table 6 below.

**[Table 6]**

| Example | Structural formula | Physical properties etc. |
|---|---|---|
| 73 | 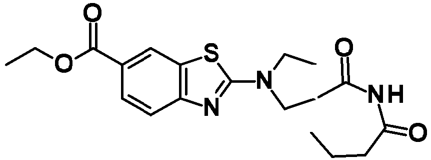 Ethyl 2-(6, 8-dioxo-2, 7-diazaspiro[4. 6]undecane-2-yl)-1,3-benzothiazole-6-carboxylate | Colorless powder |
| | | MS (ESI) m/z: 388.2 (M+H)⁺ |
| 74 | 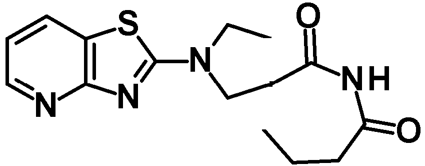 2-([1,3]thiazolo[4,5-b]pyridin-2-yl)-2,7-diazaspiro[4. 6]undecane-6, 8-dione | Colorless powder |
| | | MS (ESI) m/z: 317.2 (M+H)⁺ |
| 75 | 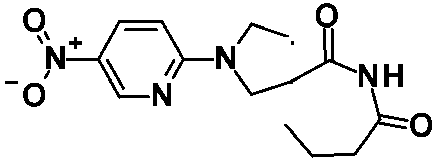 2-(5-nitro-2-pyridyl)-2,7-diazaspiro[4. 6]undecane-6, 8-dione | Yellow powder |
| | | MS (ESI) m/z: 305.1 (M+H)⁺ |
| 76 | 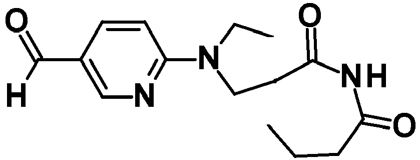 6-(6, 8-Dioxo-2, 7-diazaspiro[4. 6]undecane-2-yl)pyridine-3-carbaldehyde | Pale yellow powder |
| | | MS (ESI) m/z: 288.2 (M+H)⁺ |
| 77 | 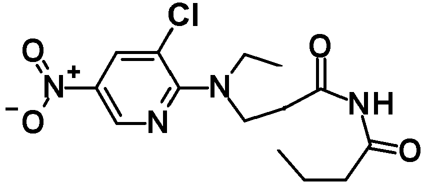 2-(3-chloro-5-nitro-2-pyridyl)-2,7-diazaspiro[4. 6]undecane-6, 8-dione | Pale yellow powder |
| | | MS (ESI) m/z: 339.0/340.9 (M+H)⁺ |
| 78 | 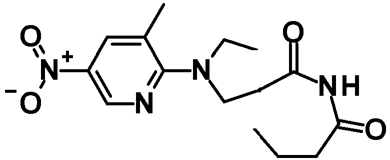 2-(3-Methyl-5-nitro-2-pyridyl)-2,7-diazaspiro[4. 6]undecane-6, 8-dione | Pale yellow powder |
| | | MS (ESI) m/z: 339.0/340.9 (M+H)⁺ |
| 79 | 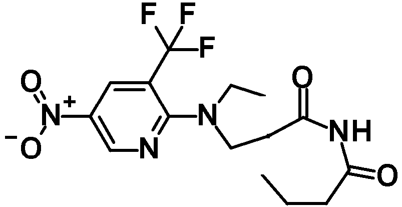 2-(5-nitro-3-(trifluoromethyl)-2-pyridyl)-2,7-diazaspiro[4. 6]undecane-6, 8-dione | Colorless powder |
| | | MS (ESI) m/z: 373.2 (M+H)⁺ |
| 80 | 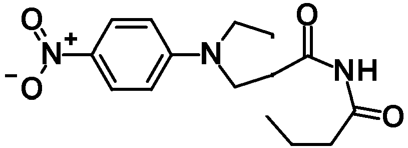 2-(4-Nitrophenyl)-2, 7-diazaspiro[4. 6]undecane-6, 8-dione | Yellow powder |
| | | MS (ESI) m/z: 304.2 (M+H)⁺ |
| 81 | 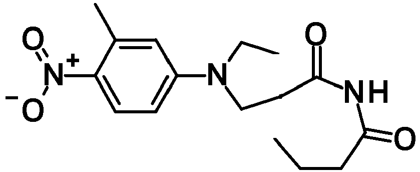 2-(3-Methyl-4-nitrophenyl)-2, 7-diazaspiro[4. 6]undecane-6, 8-dione | Yellow powder |
| | | MS (ESI) m/z: 318.2 (M+H)⁺ |
| 82 | 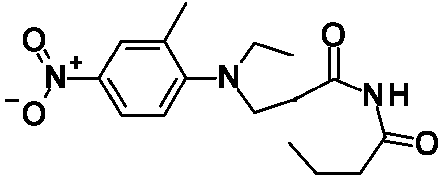 2-(2-Methyl-4-nitrophenyl)-2, 7-diazaspiro[4. 6]undecane-6, 8-dione | Yellow powder |
| | | MS (ESI) m/z: 318.2 (M+H)⁺ |
| 83 | 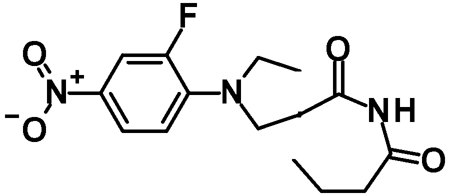 2-(2-Fluoro-4-nitrophenyl)-2, 7-diazaspiro[4. 6]undecane-6, 8-dione | Yellow powder |
| | | MS (ESI) m/z: 322.1 (M+H)⁺ |
| 84 | 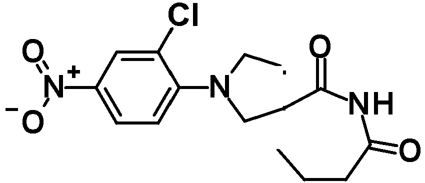 2-(2-Chloro-4-nitrophenyl)-2,7-diazaspiro[4. 6]undecane-6, 8-dione | Yellow powder |
| | | MS (ESI) m/z: 338.1/340.1 (M+H)⁺ |

### Examples 85 and 86:

The corresponding starting compounds were processed in the same manner as in Example 70 to obtain the compounds listed in Table 7 below.

**[Table 7]**

| Example | Structural formula | Physical properties etc. |
|---|---|---|
| 85 | 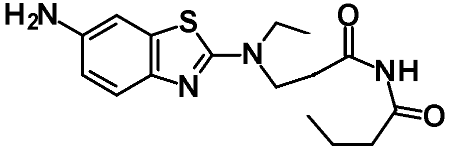 2-(6-Amino-1,3-benzothiazol-2-yl)-2,7-diazaspiro[4. 6]undecane-6, 8-dione | Brown powder |
| | | MS (ESI) m/z: 331.1 (M+H)⁺ |
| 86 | 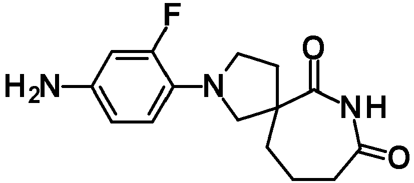 2-(4-Amino-2-fluorophenyl)-2,7-diazaspiro[4. 6]undecane-6, 8-dione | Gray powder |
| | | MS (ESI) m/z: 292.0 (M+H)⁺ |

### Examples 87 and 88:

The corresponding starting compounds were processed in the same manner as in Example 71 to obtain the compounds listed in Table 8 below.

**[Table 8]**

| Example | Structural formula | Physical properties etc. |
|---|---|---|
| 87 | 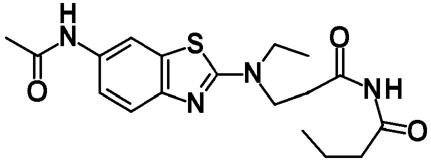 N-[2-(6, 8-dioxo-2, 7-diazaspiro[4.6]undecan-2-yl)-1, 3-benzothiazol-6-yl]acetamide | Gray powder |
| | | MS (ESI) m/z: 373.2 (M+H)⁺ |
| 88 | 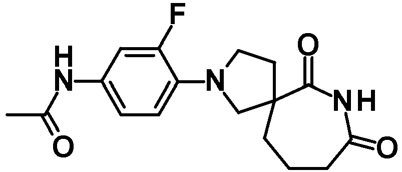 N-[4-(6, 8-dioxo-2, 7-diazaspiro[4.6]undecan-2-yl)-3-fluorophenyl]acetamide | Colorless powder |
| | | MS (ESI) m/z: 334.1 (M+H)⁺ |

### Example 89

### 2-[5-[(4-Acetylpiperazin-1-yl)methyl]pyridin-2-yl]-2,7-diazaspiro[4.6]undecane-6,8-dione

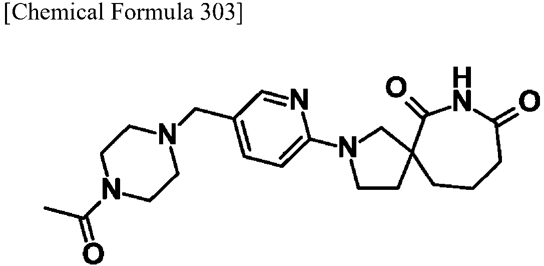

tert-Butyl 4-[[6-(6,8-dioxo-2,7-diazaspiro[4.6]undecan-2-yl)pyridin-3-yl]methyl]piperazine-1-carboxylate (11 mg) was dissolved in dichloromethane (0.5 mL), and trifluoroacetic acid (0.5 mL) was added. The mixture was stirred at room temperature for 0.5 hours. The reaction mixture was concentrated, and dried under reduced pressure. The residue was dissolved in dichloromethane (1 mL), and triethylamine (7 mg) and acetyl chloride (2 mg) were added, followed by stirring at room temperature for 0.5 hours. The reaction mixture was concentrated, and the residue was purified by silica gel column chromatography (chloroform:methanol = 100:0 to 92:8) to obtain the title compound (5 mg) as a gray powder. MS (ESI) m/z: 400.3 (M+H)⁺

### Reference Example and Example 90

### Reference Example 90

### tert-Butyl 6-(6-oxo-2,7-diazaspiro[4.6]undecan-2-yl)pyridine-3-carboxylate

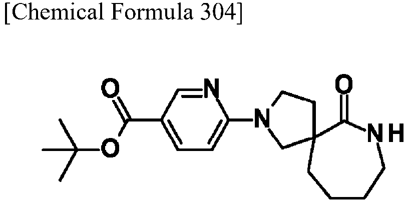

2,7-Diazaspiro[4.6]undecan-6-one hydrochloride (300 mg), tert-butyl 6-fluoropyridine-3-carboxylate (290 mg), and N,N-diisopropylethylamine (570 mg) were dissolved in N-methylpyrrolidone (3 mL) and stirred at 120 °C for 4 hours. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 70:30 to 0:100) to obtain the title compound (402 mg) as a colorless powder. MS (ESI) m/z: 346.2 (M+H)⁺.

### Example 90

### tert-Butyl 6-(6,8-dioxo-2,7-diazaspiro[4.6]undecan-2-yl)pyridine-3-carboxylate (Example Compound 90-1)

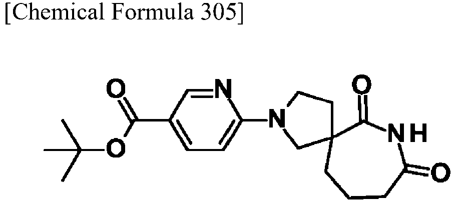

and

### tert-Butyl 6-(1,9,11-trioxo-2,10-diazaspiro[4.6]undecan-2-yl)pyridine-3-carboxylate (Example Compound 90-2)

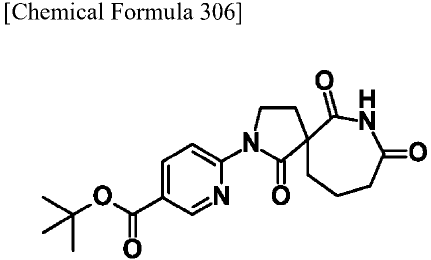

Using tert-butyl 6-(6-oxo-2,7-diazaspiro[4.6]undecan-2-yl)pyridine-3-carboxylate instead of tert-butyl 2-[2-fluoro-3-[(2-oxoazepan-3-yl)carbamoyl]phenoxy]acetate in Example 67, the same reaction and processing were performed to obtain tert-Butyl 6-(6,8-dioxo-2,7-diazaspiro[4.6]undecan-2-yl)pyridine-3-carboxylate (Example Compound 90-1: 93 mg) and tert-Butyl 6-(1,9,11-trioxo-2,10-diazaspiro[4.6]undecan-2-yl)pyridine-3-carboxylate (Example Compound 90-2: 52 mg) as colorless powders, respectively.
Example Compound 90-1: MS (ESI) m/z: 360.2 (M+H)⁺,
Example Compound 90-2: MS (ESI) m/z: 374.2 (M+H)⁺

### Reference Example and Example 91

### Reference Example 91

### tert-Butyl 2-(6-oxo-2,7-diazaspiro[4.6]undecan-2-yl)pyridine-4-carboxylate

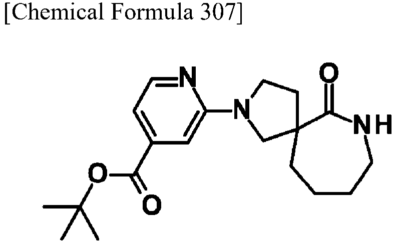

Using tert-butyl 2-fluoropyridine-4-carboxylate instead of tert-butyl 6-fluoropyridine-3-carboxylate in Reference Example 90, the same reaction and processing were performed to obtain the title compound (442 mg) as a pale yellow powder. MS (ESI) m/z: 346.2 (M+H)⁺.

### Example 91

### tert-Butyl 2-(6,8-dioxo-2,7-diazaspiro[4.6]undecan-2-yl)pyridine-4-carboxylate

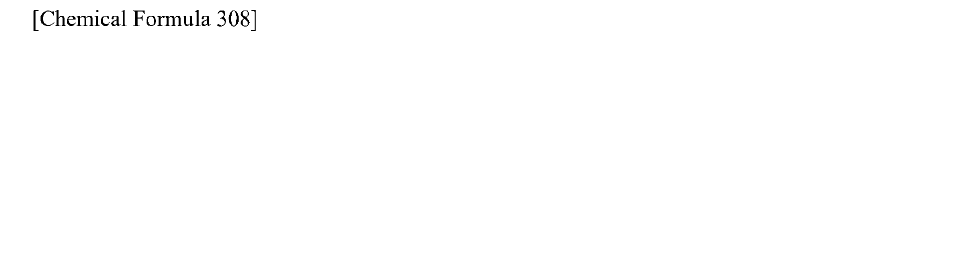

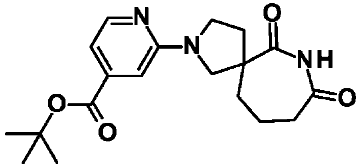

Using tert-butyl 2-(6-oxo-2,7-diazaspiro[4.6]undecan-2-yl)pyridine-4-carboxylate instead of tert-butyl 2-[2-fluoro-3-[(2-oxoazepan-3-yl)carbamoyl]phenoxy]acetate in Example 67, the same reaction and processing were performed to obtain the title compound as a colorless powder. MS (ESI) m/z: 360.2 (M+H)⁺.

### Reference Example and Example 92

### Reference Example 92

### tert-Butyl 6-(6-oxo-2,7-diazaspiro[4.6]undecan-2-yl)pyridine-2-carboxylate

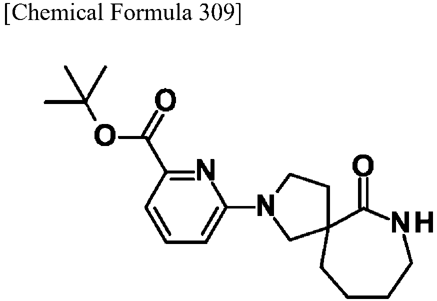

Using tert-butyl 6-chloropyridine-2-carboxylate instead of tert-butyl 6-fluoropyridine-3-carboxylate in Reference Example 90, the same reaction and processing were performed to obtain the title compound (102 mg) as a colorless powder. MS (ESI) m/z: 346.2 (M+H)⁺.

### Example 92

### tert-Butyl 6-(6,8-dioxo-2,7-diazaspiro[4.6]undecan-2-yl)pyridine-2-carboxylate

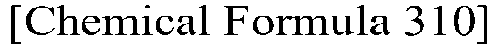

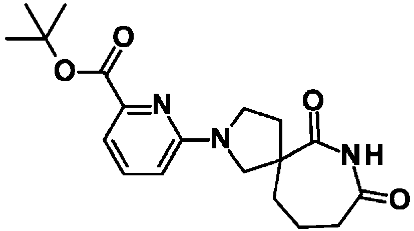

Using tert-butyl 6-(6-oxo-2,7-diazaspiro[4.6]undecan-2-yl)pyridine-2-carboxylate instead of tert-butyl 2-[2-fluoro-3-[(2-oxoazepan-3-yl)carbamoyl]phenoxy]acetate in Example 67, the same reaction and processing were performed to obtain the title compound (13 mg) as a colorless powder. MS (ESI) m/z: 360.2 (M+H)⁺.

### Reference Example and Example 93

### Reference Example 93

### tert-Butyl 6-(7-oxo-2,8-diazaspiro[5.6]dodecan-2-yl)pyridine-3-carboxylate

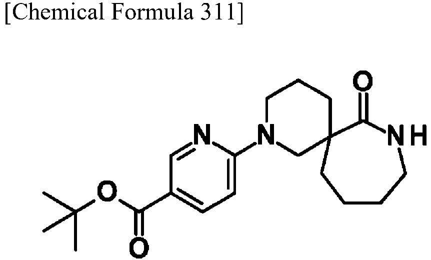

tert-Butyl 7-oxo-2,8-diazaspiro[5.6]dodecane-2-carboxylate (250 mg) was dissolved in dichloromethane (1 mL), and trifluoroacetic acid (1 mL) was added, followed by stirring at room temperature for 1 hour. The reaction mixture was concentrated, and toluene was added to the residue followed by further concentration. The resulting residue, tert-butyl 6-fluoropyridine-3-carboxylate (175 mg), and N,N-diisopropylethylamine (344 mg) were dissolved in N,N-dimethylformamide (3 mL) and stirred at 80 °C for 1 hour. The mixture was stirred at 110 °C for 7 hours. The mixture was stirred at 120 °C for 10 hours. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 70:30 to 0:100) to obtain the title compound (259 mg) as a colorless viscous material. MS (ESI) m/z: 360.3 (M+H)⁺.

### Example 93

### tert-Butyl 6-(7,9-dioxo-2,8-diazaspiro[5.6]dodecan-2-yl)pyridine-3-carboxylate

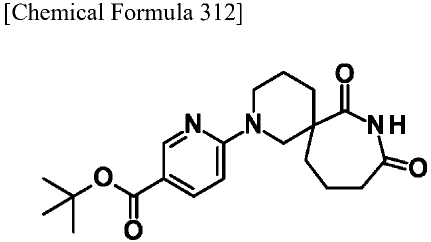

Using tert-butyl 6-(7-oxo-2,8-diazaspiro[5.6]dodecan-2-yl)pyridine-3-carboxylate instead of tert-butyl 2-[2-fluoro-3-[(2-oxoazepan-3-yl)carbamoyl]phenoxy]acetate in Example 67, the same reaction and processing were performed to obtain the title compound (36 mg) as a pale yellow powder. MS (ESI) m/z: 374.2 (M+H)⁺

### Reference Example and Example 94

### Reference Example 94

### tert-Butyl 6-(7-oxo-3,8-diazaspiro[5.6]dodecan-3-yl)pyridine-3-carboxylate

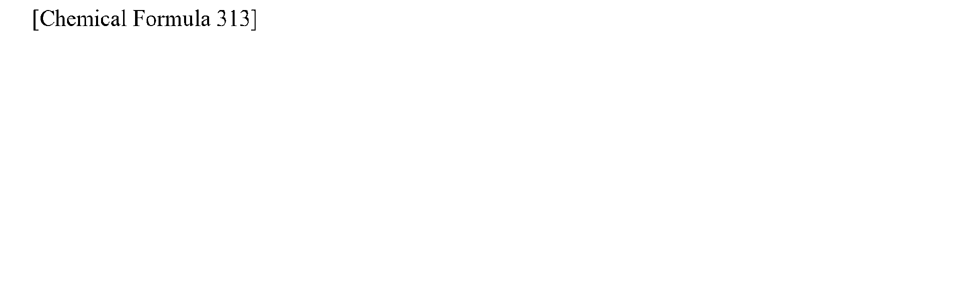

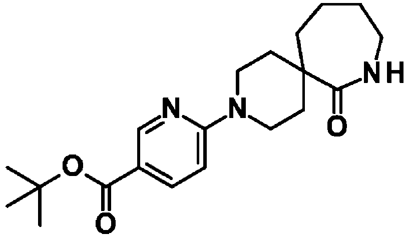

Using tert-butyl 7-oxo-3,8-diazaspiro[5.6]dodecane-3-carboxylate instead of tert-butyl 7-oxo-2,8-diazaspiro[5.6]dodecane-2-carboxylate in Reference Example 93, the same reaction and processing were performed to obtain the title compound (244 mg) as a colorless powder. MS (ESI) m/z: 360.3 (M+H)⁺.

### Example 94

### tert-Butyl 6-(7,9-dioxo-3,8-diazaspiro[5.6]dodecan-3-yl)pyridine-3-carboxylate

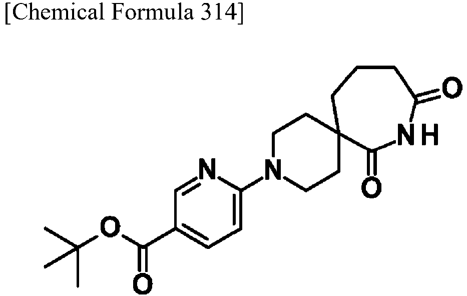

Using tert-butyl 6-(7-oxo-3,8-diazaspiro[5.6]dodecan-3-yl)pyridine-3-carboxylate instead of tert-butyl 2-[2-fluoro-3-[(2-oxoazepan-3-yl)carbamoyl]phenoxy]acetate in Example 67, the same reaction and processing were performed to obtain the title compound (67 mg) as a colorless powder. MS (ESI) m/z: 374.3 (M+H)⁺

### Reference Example and Example 95

### Reference Example 95 tert-Butyl 2-(6-oxo-2,7-diazaspiro[4.6]undecan-2-yl)thiazole-5-carboxylate

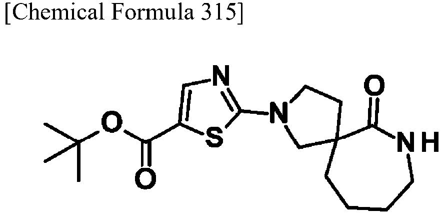

tert-Butyl 2-bromothiazole-5-carboxylate (320 mg), 2,7-diazaspiro[4.6]undecan-6-one (374 mg), tris(dibenzylideneacetone)dipalladium (56 mg), 2-dicyclohexylphosphino-2'-(N,N-dimethylamino)biphenyl (48 mg), and potassium phosphate (1286 mg) were suspended in N,N-dimethylformamide (4 mL) and stirred under microwave irradiation at 120 °C for 1 hour. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 70:30 to 0:100) to obtain the title compound (297 mg) as a yellow powder. MS (ESI) m/z: 296.1 (M-tBu+H)⁺

### Example 95

### tert-Butyl 2-(6,8-dioxo-2,7-diazaspiro[4.6]undecan-2-yl)-1,3-thiazole-5-carboxylate

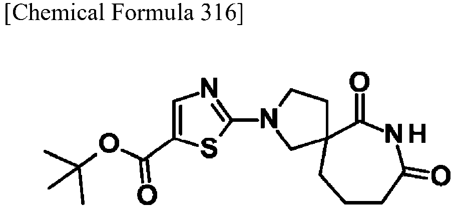

Using tert-butyl 2-(6-oxo-2,7-diazaspiro[4.6]undecan-2-yl)thiazole-5-carboxylate instead of tert-butyl 2-[2-fluoro-3-[(2-oxoazepan-3-yl)carbamoyl]phenoxy]acetate in Example 67, the same reaction and processing were performed to obtain the title compound (80 mg) as a colorless powder. MS (ESI) m/z: 366.2 (M+H)⁺.

### Example 96

### 6-(6,8-Dioxo-2,7-diazaspiro[4.6]undecan-2-yl)-N-methylpyridine-3-carboxamide

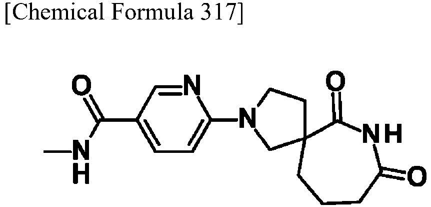

tert-Butyl 6-(6,8-dioxo-2,7-diazaspiro[4.6]undecan-2-yl)pyridine-3-carboxylate was dissolved in dichloromethane (0.5 mL), and trifluoroacetic acid (0.5 mL) was added. The mixture was stirred at room temperature for 1 hour. The reaction mixture was concentrated, and dried under reduced pressure. The resulting residue, methylamine hydrochloride (19 mg), O-(7-azabenzotriazol-1-yl)-N,N,N',N'-tetramethyluronium hexafluorophosphate (32 mg), and N,N-diisopropylethylamine (0.1 mL) were dissolved in N,N-dimethylformamide (1 mL) and stirred at room temperature for 1 hour. Water was added to the reaction mixture, and after extraction with ethyl acetate, washing with saturated brine was performed, followed by drying over anhydrous sodium sulfate. The aqueous layer was extracted twice more with chloroform, followed by washing with saturated brine and drying over anhydrous sodium sulfate. The combined extracts were concentrated under reduced pressure to remove the solvent. The residue was purified by silica gel column chromatography (chloroform:methanol = 100:0 to 90:10) to obtain the title compound (14 mg) as a colorless powder. MS (ESI) m/z: 317.2 (M+H)⁺.

### Example 97

### tert-Butyl 4-[6-(6,8-dioxo-2,7-diazaspiro[4.6]undecan-2-yl)pyridine-3-carbonyl]piperazine-1-carboxylate

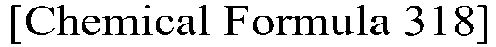

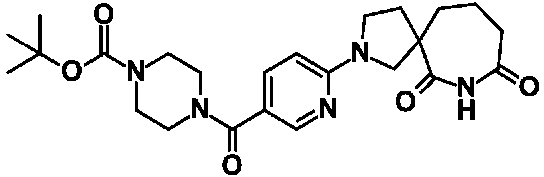

Using tert-butyl piperazine-1-carboxylate instead of methylamine hydrochloride in Example 96, the same reaction and processing were performed to obtain the title compound (40 mg) as a colorless powder. MS (ESI) m/z: 472.4 (M+H)⁺.

### Example 98

### tert-Butyl 4-[2-(4,7,9-trioxo-3,8-diazaspiro[5.6]dodecan-3-yl)acetyl]piperazine-1-carboxylate

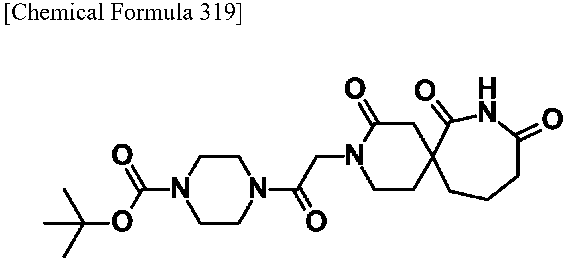

Using tert-butyl 2-(4,7,9-trioxo-3,8-diazaspiro[5.6]dodecan-3-yl)acetate instead of tert-butyl 6-(6,8-dioxo-2,7-diazaspiro[4.6]undecan-2-yl)pyridine-3-carboxylate in Example 97, the same reaction and processing were performed to obtain the title compound (18 mg) as a colorless powder. MS (ESI) m/z: 337.2 (M-Boc+H)⁺.

### Example 99

### tert-Butyl 4-[[6-(6,8-dioxo-2,7-diazaspiro[4.6]undecan-2-yl)pyridin-3-yl]methyl]piperazine-1-carboxylate

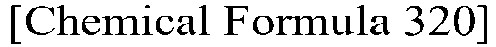

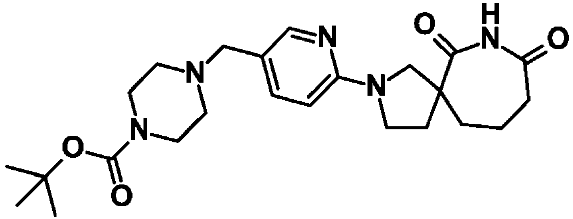

6-(6,8-Dioxo-2,7-diazaspiro[4.6]undecan-2-yl)pyridine-3-carbaldehyde (20 mg) and tert-butyl piperazine-1-carboxylate (16 mg) were dissolved in dichloromethane (1 mL), and sodium triacetoxyborohydride (18 mg) was added, followed by stirring at room temperature for 4 hours. Saturated aqueous sodium bicarbonate was added to the reaction mixture, and after extraction with chloroform, washing with water and saturated brine was performed, followed by drying over anhydrous sodium sulfate, and then the solvent was evaporated under reduced pressure. The residue was purified by silica gel column chromatography (chloroform:methanol = 100:0 to 95:5) to obtain the title compound (16 mg) as a colorless powder. MS (ESI) m/z: 458.4 (M+H)⁺.

### Example 100

### tert-Butyl 2-(6,8-dioxo-2,7-diazaspiro[4.6]undecan-2-yl)acetate

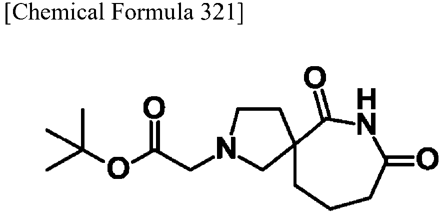

tert-Butyl 6,8-dioxo-2,7-diazaspiro[4.6]undecane-2-carboxylate (100 mg) was dissolved in dichloromethane (1 mL), and trifluoroacetic acid (1 mL) was added, followed by stirring at room temperature for 1 hour. The reaction mixture was concentrated, and toluene was added to the residue followed by further concentration. The resulting residue, tert-butyl 2-bromoacetate (83 mg), and N,N-diisopropylethylamine (138 mg) were dissolved in N,N-dimethylformamide (2 mL) and stirred at room temperature for 1 hour. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 70:30 to 20:80 to 0:100) to obtain the title compound (74 mg) as a colorless powder. MS (ESI) m/z: 297.2 (M+H)⁺.

### Example 101

### tert-Butyl 2-(7,9-dioxo-3,8-diazaspiro[5.6]dodecan-3-yl)acetate

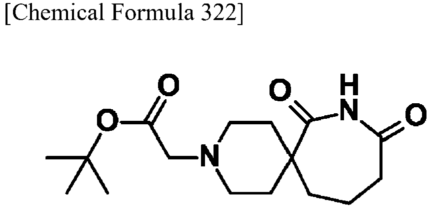

Using tert-butyl 7,9-dioxo-3,8-diazaspiro[5.6]dodecane-3-carboxylate instead of tert-butyl 6,8-dioxo-2,7-diazaspiro[4.6]undecane-2-carboxylate in Example 100, the same reaction and processing were performed to obtain the title compound (17 mg) as a colorless powder. MS (ESI) m/z: 311.2 (M+H)⁺.

### Example 102

### 2-[5-(Piperazine-1-carbonyl)pyridin-2-yl]-2,7-diazaspiro[4.6]undecane-6,8-dione

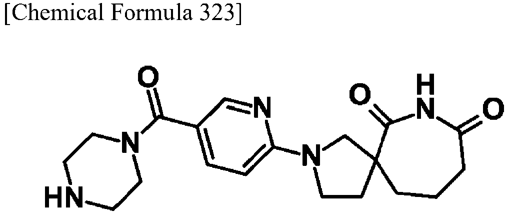

tert-Butyl 4-[6-(6,8-dioxo-2,7-diazaspiro[4.6]undecan-2-yl)pyridine-3-carbonyl]piperazine-1-carboxylate (35 mg) was dissolved in dichloromethane (1 mL), and trifluoroacetic acid (1 mL) was added. The mixture was stirred at room temperature for 1 hour. The reaction mixture was concentrated, and chloroform and saturated aqueous sodium bicarbonate were added to the residue, followed by extraction, and then the organic layer was dried over anhydrous sodium sulfate, and the solvent was evaporated under reduced pressure to obtain the title compound (9 mg) as a colorless powder. MS (ESI) m/z: 372.3 (M+H)⁺.

### Example 103

### tert-Butyl 7,9-dioxo-2,8-diazaspiro[5.6]dodecane-2-carboxylate

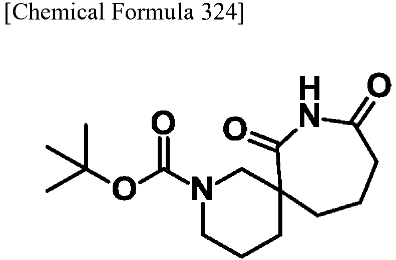

Using tert-butyl 7-oxo-2,8-diazaspiro[5.6]dodecane-2-carboxylate instead of tert-butyl 2-[2-fluoro-3-[(2-oxoazepan-3-yl)carbamoyl]phenoxy]acetate in Example 67, the same reaction and processing were performed to obtain the title compound (144 mg) as a colorless powder. MS (ESI) m/z: 197.2 (M-Boc+H)⁺.

### Example 104

### 2-Benzoyl-2,8-diazaspiro[5.6]dodecane-7,9-dione

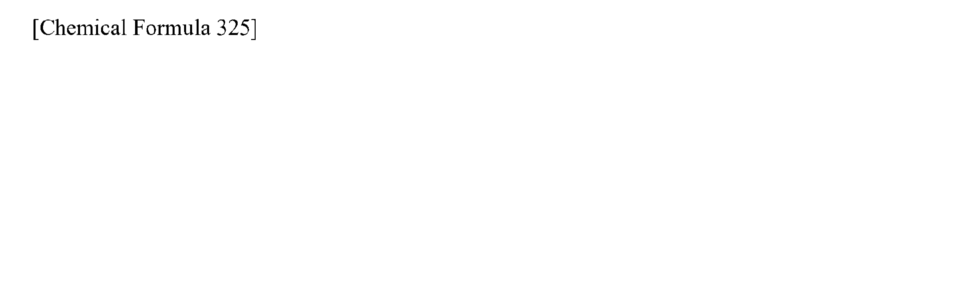

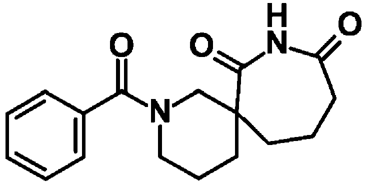

tert-Butyl 7,9-dioxo-2,8-diazaspiro[5.6]dodecane-2-carboxylate (30 mg) was dissolved in dichloromethane (1 mL), and trifluoroacetic acid (0.5 mL) was added. The mixture was stirred at room temperature for 3 hours. The reaction mixture was concentrated, and toluene was added to the resulting residue, followed by further concentration. The resulting residue and triethylamine (41 mg) were dissolved in chloroform (1 mL), benzoyl chloride (30 mg) was added, and the mixture was stirred at room temperature for 1 hour. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 40:60 to 0:100) to obtain the title compound (11 mg) as a colorless powder. MS (ESI) m/z: 301.2 (M+H)⁺.

### Example 105

### tert-Butyl 7,9-dioxo-3,8-diazaspiro[5.6]dodecane-3-carboxylate

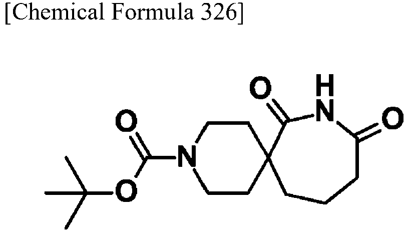

Using tert-butyl 7-oxo-3,8-diazaspiro[5.6]dodecane-3-carboxylate instead of tert-butyl 2-[2-fluoro-3-[(2-oxoazepan-3-yl)carbamoyl]phenoxy]acetate in Example 67, the same reaction and processing were performed to obtain the title compound (138 mg) as a colorless powder. MS (ESI) m/z: 197.2 (M-Boc+H)⁺.

### Example 106

### 3-Benzoyl-3,8-diazaspiro[5.6]dodecane-7,9-dione

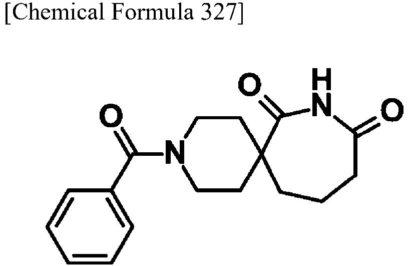

Using tert-butyl 7,9-dioxo-3,8-diazaspiro[5.6]dodecane-3-carboxylate instead of tert-butyl 7,9-dioxo-2,8-diazaspiro[5.6]dodecane-2-carboxylate in Example 104, the same reaction and processing were performed to obtain the title compound (11 mg) as a colorless powder. MS (ESI) m/z: 301.2 (M+H)⁺.

### Reference Example and Example 107

### Reference Example 107

### (Reference Example 107-1)

### Benzyl 2-oxopiperidine-4-carboxylate

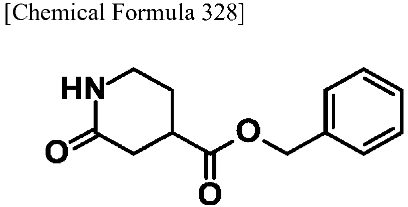

2-Oxopiperidine-4-carboxylic acid (7.00 g), benzyl bromide (10.0 g), and potassium carbonate (20.0 g) were added to acetonitrile and stirred at an external temperature of 80 °C for 21 hours. Water was added to the reaction mixture, followed by extraction with ethyl acetate and drying over anhydrous sodium sulfate, and the solvent was evaporated under reduced pressure. The residue was purified by silica gel column chromatography (chloroform:methanol = 100:0 to 92:8), washed by suspension with diisopropyl ether, filtered, and dried under reduced pressure to obtain the title compound (9.17 g) as a colorless powder. MS (ESI) m/z: 234.2 (M+H)⁺.

### (Reference Example 107-2)

### Benzyl 4-(4-chlorobutyl)-2-oxopiperidine-4-carboxylate

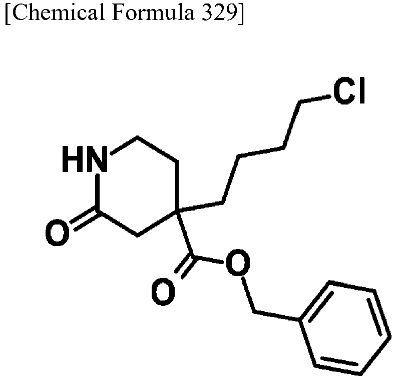

Benzyl 2-oxopiperidine-4-carboxylate (8.00 g) was dissolved in tetrahydrofuran (80 mL), and under a nitrogen atmosphere in a dry ice-acetone bath, lithium bis(trimethylsilyl)amide (about 26% tetrahydrofuran solution, 66 mL) was added dropwise over about 10 minutes. The dry ice-acetone bath was removed, and the mixture was stirred for 0.5 hours. The mixture was cooled again in a dry ice-acetone bath, and 1-bromo-4-chlorobutane (8.82 g) was added dropwise. The mixture was warmed to room temperature and stirred for 3 hours. 1N aqueous hydrochloric acid (about 200 mL) was added to the reaction mixture, followed by extraction with ethyl acetate. The organic layer was washed with saturated brine, and dried over anhydrous sodium sulfate, and then the solvent was evaporated under reduced pressure. The residue was purified by silica gel column chromatography (chloroform:methanol = 100:0 to 94:6) to obtain the title compound (9.76 g) as a light brown powder. MS (ESI) m/z: 324.2/326.1 (M+H)⁺.

### (Reference Example 107-3)

### Benzyl 4-(4-azidobutyl)-2-oxopiperidine-4-carboxylate

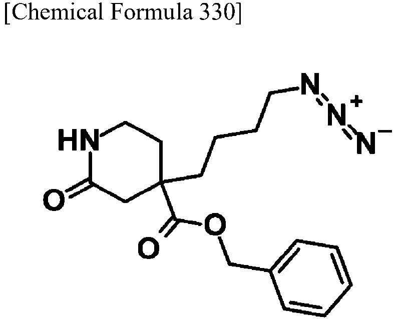

Benzyl 4-(4-chlorobutyl)-2-oxopiperidine-4-carboxylate (9.70 g) was dissolved in acetonitrile (100 mL), and tetrabutylammonium azide (10.8 g) was added. The mixture was stirred at an external temperature of 80 °C for 4 hours. Water was added to the reaction mixture, and the mixture was extracted with ethyl acetate. The organic layer was washed with saturated brine, and dried over anhydrous sodium sulfate, and then the solvent was evaporated under reduced pressure. The residue was purified by silica gel column chromatography (chloroform:methanol = 100:0 to 95:5) to obtain the title compound (9.24 g) as a brown viscous material. MS (ESI) m/z: 331.2 (M+H)⁺.

### (Reference Example 107-4)

### Benzyl 4-(4-azidobutyl)-1-[2-[(2-methylpropan-2-yl)oxy]-2-oxoethyl]-2-oxopiperidine-4-carboxylate

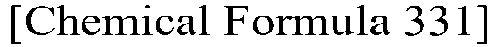

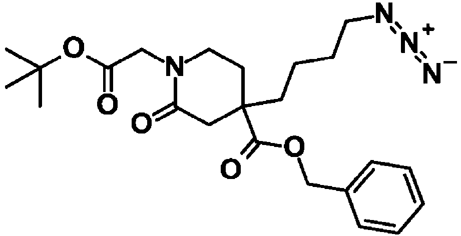

Benzyl 4-(4-azidobutyl)-2-oxopiperidine-4-carboxylate (3.00 g) and tert-butyl 2-bromoacetate (2.13 g) were dissolved in N,N-dimethylformamide (20 mL), and sodium hydride (545 mg) was added under ice-cooling. The mixture was warmed to room temperature and stirred for 3 hours. Additional sodium hydride (273 mg) was added to the reaction mixture, and the mixture was stirred at room temperature for 2 hours. Saturated aqueous ammonium chloride was added to the reaction mixture, and the mixture was extracted with ethyl acetate. The organic layer was washed with water and saturated brine, and dried over anhydrous sodium sulfate, and then the solvent was evaporated under reduced pressure. The residue was purified by silica gel column chromatography (hexane:ethyl acetate = 90:10 to 45:55) to obtain the title compound (3.10 g) as a brown viscous material. MS (ESI) m/z: 389.3 (M-tBu+H)⁺

### (Reference Example 107-5)

### Benzyl 4-(4-aminobutyl)-1-[2-[(2-methylpropan-2-yl)oxy]-2-oxoethyl]-2-oxopiperidine-4-carboxylate

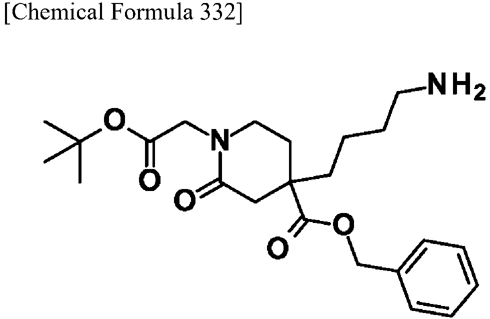

Benzyl 4-(4-azidobutyl)-1-[2-[(2-methylpropan-2-yl)oxy]-2-oxoethyl]-2-oxopiperidine-4-carboxylate (3.09 g) was dissolved in tetrahydrofuran (30 mL), and triphenylphosphine (2.74 g) and water (4 mL) were added, followed by stirring at room temperature for 17 hours. The reaction mixture was concentrated, and the residue was purified by silica gel column chromatography (chloroform:methanol = 100:0 to 90:10 to 65:35) to obtain the title compound (2.70 g) as a pale yellow viscous material. MS (ESI) m/z: 419.4 (M+H)⁺.

### (Reference Example 107-6)

### tert-Butyl 2-(4,7-dioxo-3,8-diazaspiro[5.6]dodecan-3-yl)acetate

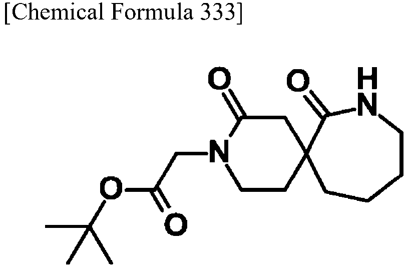

Benzyl 4-(4-aminobutyl)-1-[2-[(2-methylpropan-2-yl)oxy]-2-oxoethyl]-2-oxopiperidine-4-carboxylate (2.69 g) was dissolved in ethanol (25 mL), and palladium on carbon (300 mg) was added. The atmosphere was replaced with hydrogen, and the mixture was stirred at room temperature for 2 hours. The reaction mixture was filtered through Celite, and the filtrate was concentrated and dried under reduced pressure. The residue was suspended in acetonitrile (50 mL), and O-(7-azabenzotriazol-1-yl)-N,N,N',N'-tetramethyluronium hexafluorophosphate (3.67 g) and N,N-diisopropylethylamine (1.66 g) were added, followed by stirring at room temperature for 0.5 hours. Water was added to the reaction mixture, and the mixture was extracted with ethyl acetate. The organic layer was washed with water and saturated brine, and dried over anhydrous sodium sulfate, and then the solvent was evaporated under reduced pressure. The residue was purified by silica gel column chromatography (chloroform:methanol = 100:0 to 92:8) to obtain the title compound (1.01 g) as a pale yellow powder. MS (ESI) m/z: 255.2 (M+H)⁺.

### Example 107

### tert-Butyl 2-(4,7,9-trioxo-3,8-diazaspiro[5.6]dodecan-3-yl)acetate

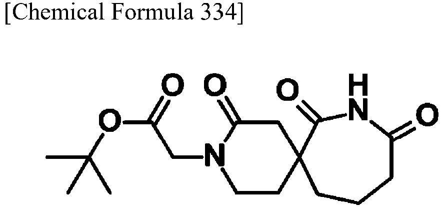

Using tert-butyl 2-(4,7-dioxo-3,8-diazaspiro[5.6]dodecan-3-yl)acetate instead of tert-butyl 2-[2-fluoro-3-[(2-oxoazepan-3-yl)carbamoyl]phenoxy]acetate in Example 67, the same reaction and processing were performed to obtain the title compound (237 mg) as a colorless powder. MS (ESI) m/z: 269.2 (M-tBu+H)⁺

### Reference Example and Example 108

### Reference Example 108

### (Reference Example 108-1)

### tert-Butyl 6-[4-(4-azidobutyl)-2-oxo-4-phenylmethoxycarbonylpiperidin-1-yl]pyridine-3-carboxylate

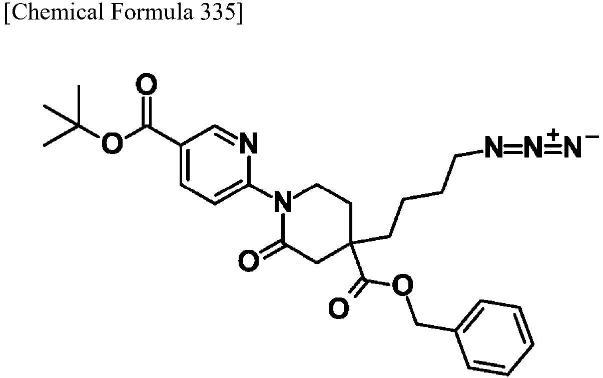

Benzyl 4-(4-azidobutyl)-2-oxopiperidine-4-carboxylate (1.27 g), tert-butyl 6-bromopyridine-3-carboxylate (1.19 g), tris(dibenzylideneacetone)dipalladium (352 mg), 4,5-bis(diphenylphosphino)-9,9-dimethylxanthene (445 mg), and cesium carbonate (3.76 g) were suspended in 1,2-dimethoxyethane (15 mL) and stirred at 100 °C for 2 hours. Water was added to the reaction mixture, and the mixture was extracted with ethyl acetate. The organic layer was washed with saturated brine, and dried over anhydrous sodium sulfate, and then the solvent was evaporated under reduced pressure. The residue was purified by silica gel column chromatography (hexane:ethyl acetate = 90:10 to 70:30) to obtain the title compound (680 mg) as a pale yellow powder. MS (ESI) m/z: 508.3 (M+H)⁺.

### (Reference Example 108-2)

### tert-Butyl 6-(4,7-dioxo-3,8-diazaspiro[5.6]dodecan-3-yl)pyridine-3-carboxylate

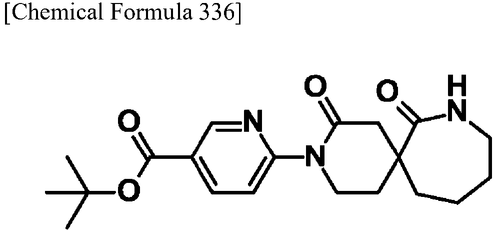

Using tert-butyl 6-[4-(4-azidobutyl)-2-oxo-4-phenylmethoxycarbonylpiperidin-1-yl]pyridine-3-carboxylate instead of benzyl 4-(4-azidobutyl)-1-[2-[(2-methylpropan-2-yl)oxy]-2-oxoethyl]-2-oxopiperidine-4-carboxylate in (Reference Example 107-5), the same reactions and processing as in (Reference Example 107-5) and (Reference Example 107-6) were performed to obtain the title compound (213 mg) as a colorless powder. MS (ESI) m/z: 374.2 (M+H)⁺

### Example 108

### tert-Butyl 6-(4,7,9-trioxo-3,8-diazaspiro[5.6]dodecan-3-yl)pyridine-3-carboxylate

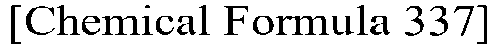

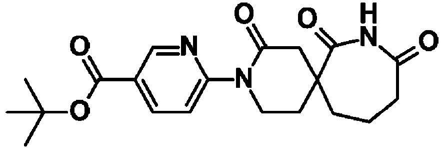

Using tert-butyl 6-(4,7-dioxo-3,8-diazaspiro[5.6]dodecan-3-yl)pyridine-3-carboxylate instead of tert-butyl 2-[2-fluoro-3-[(2-oxoazepan-3-yl)carbamoyl]phenoxy]acetate in Example 67, the same reaction and processing were performed to obtain the title compound (98 mg) as a colorless powder. MS (ESI) m/z: 388.2 (M+H)⁺.

### Reference Example and Example 109

### Reference Example 109

### tert-Butyl 4-(4,7-dioxo-3,8-diazaspiro[5.6]dodecan-3-yl)benzoate

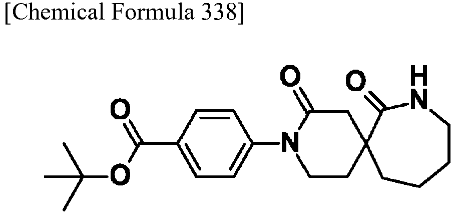

Using tert-butyl 4-bromobenzoate instead of tert-butyl 6-bromopyridine-3-carboxylate in (Reference Example 108-1), the same reactions and processing as in (Reference Example 108-1) and (Reference Example 108-2) were performed to obtain the title compound (161 mg) as a colorless powder. MS (ESI) m/z: 373.3 (M+H)⁺

### Example 109

### tert-Butyl 4-(4,7,9-trioxo-3,8-diazaspiro[5.6]dodecan-3-yl)benzoate

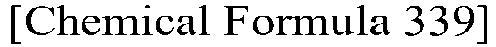

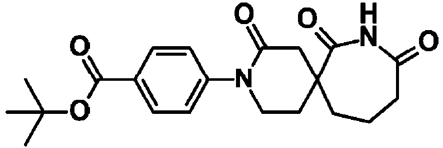

Using tert-butyl 4-(4,7-dioxo-3,8-diazaspiro[5.6]dodecan-3-yl)benzoate instead of tert-butyl 2-[2-fluoro-3-[(2-oxoazepan-3-yl)carbamoyl]phenoxy]acetate in Example 67, the same reaction and processing were performed to obtain the title compound (62 mg) as a colorless powder. MS (ESI) m/z: 387.2 (M+H)⁺

### Reference Example and Example 110

### Reference Example 110

### tert-Butyl 3-(4,7-dioxo-3,8-diazaspiro[5.6]dodecan-3-yl)benzoate

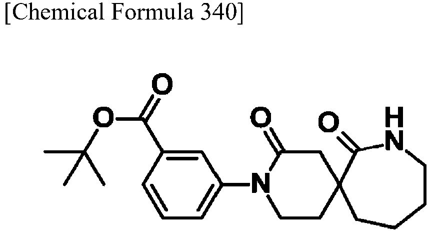

Using tert-butyl 3-bromobenzoate instead of tert-butyl 6-bromopyridine-3-carboxylate in (Reference Example 108-1), the same reactions and processing as in (Reference Example 108-1) and (Reference Example 108-2) were performed to obtain the title compound (185 mg) as a colorless powder. MS (ESI) m/z: 373.3 (M+H)⁺

### Example 110

### tert-Butyl 3-(4,7,9-trioxo-3,8-diazaspiro[5.6]dodecan-3-yl)benzoate

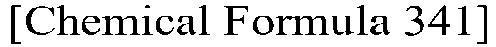

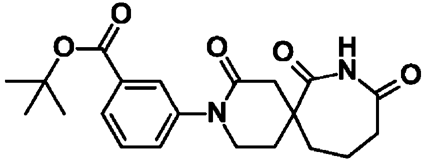

Using tert-butyl 3-(4,7-dioxo-3,8-diazaspiro[5.6]dodecan-3-yl)benzoate instead of tert-butyl 2-[2-fluoro-3-[(2-oxoazepan-3-yl)carbamoyl]phenoxy]acetate in Example 67, the same reaction and processing were performed to obtain the title compound (69 mg) as a colorless powder. MS (ESI) m/z: 387.3 (M+H)⁺

### Reference Example and Example 111

### Reference Example 111

### tert-Butyl 4-[(4,7-dioxo-3,8-diazaspiro[5.6]dodecan-3-yl)methyl]benzoate

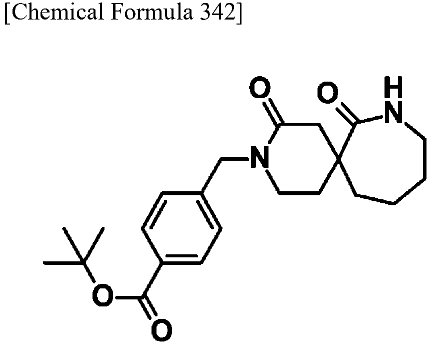

Using benzyl bromide instead of tert-butyl 2-bromoacetate in (Reference Example 107-4), the same reactions and processing as in (Reference Example 107-4), (Reference Example 107-5), and (Reference Example 107-6) were performed to obtain the title compound (215 mg) as a colorless powder. MS (ESI) m/z: 387.1 (M+H)⁺

### Example 111

### tert-Butyl 4-[(4,7,9-trioxo-3, 8-diazaspiro[5.6]dodecan-3-yl)methyl]benzoate

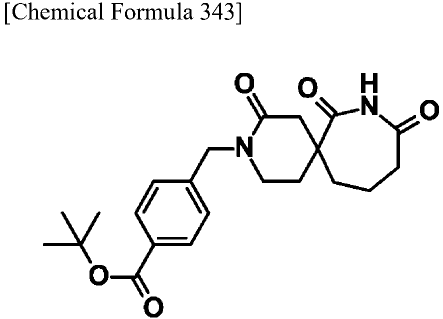

Using tert-butyl 4-[(4,7-dioxo-3,8-diazaspiro[5.6]dodecan-3-yl)methyl]benzoate instead of tert-butyl 2-[2-fluoro-3-[(2-oxoazepan-3-yl)carbamoyl]phenoxy]acetate in Example 67, the same reaction and processing were performed to obtain the title compound (46 mg) as a colorless powder. MS (ESI) m/z: 401.3 (M+H)⁺

### Reference Example and Example 112

### Reference Example 112

### tert-Butyl 2-(3,6-dioxo-2,7-diazaspiro[4.6]undecan-2-yl)acetate

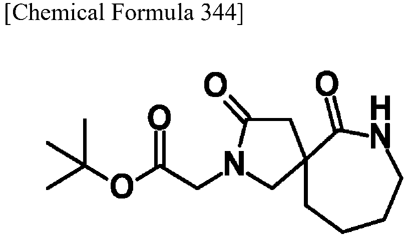

Using 5-oxopyrrolidine-3-carboxylic acid instead of 2-oxopiperidine-4-carboxylic acid in (Reference Example 107-1), the same reactions and processing as in (Reference Example 107-1), (Reference Example 107-2), (Reference Example 107-3), (Reference Example 107-4), (Reference Example 107-5), and (Reference Example 107-6) were performed to obtain the title compound (811 mg) as a colorless powder. MS (ESI) m/z: 241.2 (M-tBu+H)⁺

### Example 112

### tert-Butyl 2-(3,6,8-trioxo-2,7-diazaspiro[4.6]undecan-2-yl)acetate

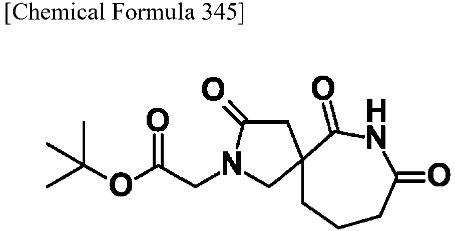

Using tert-butyl 2-(3,6-dioxo-2,7-diazaspiro[4.6]undecan-2-yl)acetate instead of tert-butyl 2-[2-fluoro-3-[(2-oxoazepan-3-yl)carbamoyl]phenoxy]acetate in Example 67, the same reaction and processing were performed to obtain the title compound (244 mg) as a colorless powder. MS (ESI) m/z: 255.2 (M-tBu+H)⁺

### Reference Example and Example 113

### Reference Example 113

### tert-Butyl 6-(3,6-dioxo-2,7-diazaspiro[4.6]undecan-2-yl)pyridine-3-carboxylate

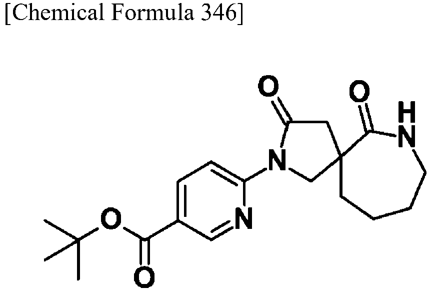

Using 5-oxopyrrolidine-3-carboxylic acid instead of 2-oxopiperidine-4-carboxylic acid in (Reference Example 107-1), the same reactions and processing as in (Reference Example 107-1), (Reference Example 107-2), (Reference Example 107-3), (Reference Example 108-1), and (Reference Example 108-2) were performed to obtain the title compound (270 mg) as a colorless powder. MS (ESI) m/z: 360.3 (M+H)⁺.

### Example 113

### tert-Butyl 6-(3,6,8-trioxo-2,7-diazaspiro[4.6]undecan-2-yl)pyridine-3-carboxylate

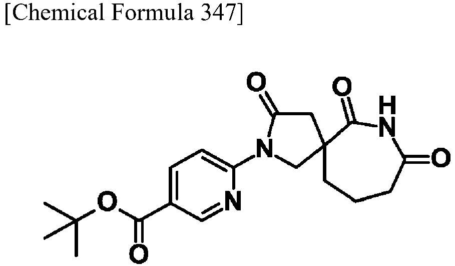

Using tert-butyl 6-(3,6-dioxo-2,7-diazaspiro[4.6]undecan-2-yl)pyridine-3-carboxylate instead of tert-butyl 2-[2-fluoro-3-[(2-oxoazepan-3-yl)carbamoyl]phenoxy]acetate in Example 67, the same reaction and processing were performed to obtain the title compound (66 mg) as a colorless powder. MS (ESI) m/z: 374.3 (M+H)⁺

### Reference Example and Example 114

### Reference Example 114

### (Reference Example 114-1)

### Ethyl 2-[(2,4-dimethoxyphenyl)methylcarbamoylamino]-1,3-benzothiazole-6-carboxylate

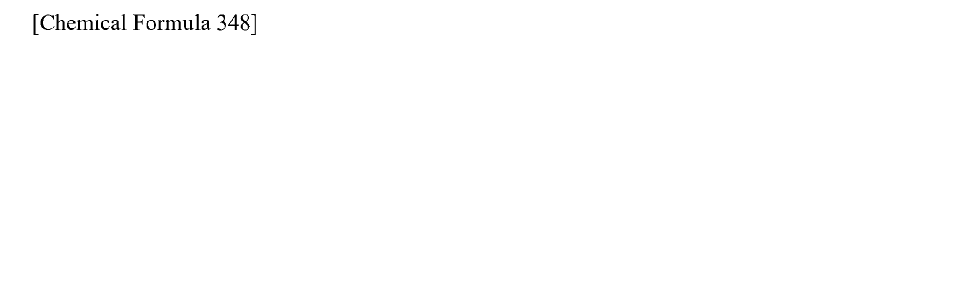

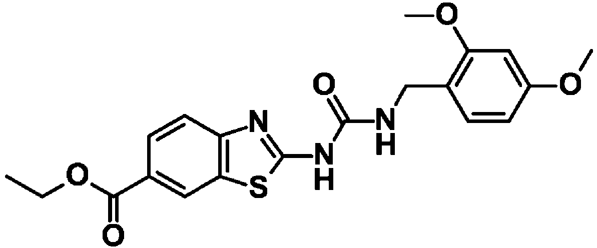

Ethyl 2-amino-1,3-benzothiazole-6-carboxylate (835 mg) and pyridine (365 µL) were dissolved in acetonitrile (8 mL), and (4-methoxyphenyl) chloroformate (770 mg) was added, followed by stirring at room temperature for 22 hours. N,N-Diisopropylethylamine (1.0 mL) and (2,4-dimethoxyphenyl)methylamine (691 mg) were added to the reaction mixture, and the mixture was stirred at an external temperature of 80 °C for 2 hours. The reaction mixture was concentrated, and the residue was purified by silica gel column chromatography (chloroform:methanol = 100:0 to 95:5) to obtain the title compound (852 mg) as a colorless powder. MS (ESI) m/z: 416.1 (M+H)⁺.

### (Reference Example 114-2)

### 2-[3-[(2,4-Dimethoxyphenyl)methyl]-2-oxo-1,3-diazepan-1-yl]-1,3-benzothiazole-6-carboxylic acid

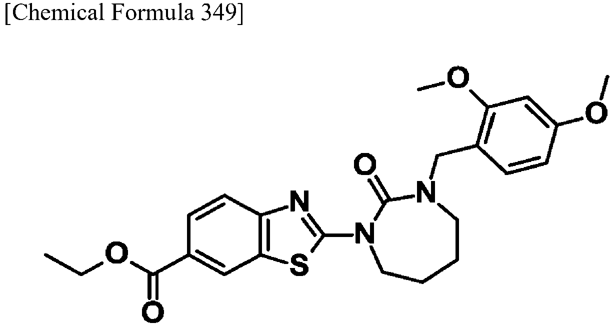

Ethyl 2-[(2,4-dimethoxyphenyl)methylcarbamoylamino]-1,3-benzothiazole-6-carboxylate (852 mg) was dissolved in N,N-dimethylformamide (10 mL), and 1,4-dibromobutane (1319 mg) and potassium carbonate (845 mg) were added, followed by stirring at an external temperature of 60 °C for 4 hours. Water was added to the reaction mixture, and the mixture was extracted with ethyl acetate. The organic layer was washed with water and saturated brine, and dried over anhydrous sodium sulfate, and then the solvent was evaporated under reduced pressure. The residue was purified by silica gel column chromatography (hexane:ethyl acetate = 80:20 to 60:40) to obtain ethyl 2-[4-bromobutyl-[(2,4-dimethoxyphenyl)methylcarbamoyl]amino]-1,3-benzothiazole-6-carboxylate as a brown viscous material containing impurities (736 mg). This was dissolved in N,N-dimethylformamide (10 mL), and sodium hydride (106 mg) was added, followed by stirring at room temperature for 4 hours. 1N hydrochloric acid was added to the reaction mixture, and the mixture was extracted with chloroform. The organic layer was washed with water and saturated brine, and dried over anhydrous sodium sulfate, and then the solvent was evaporated under reduced pressure. The residue was purified twice by silica gel column chromatography (chloroform:methanol = 100:0 to 90:10, followed by hexane:ethyl acetate = 80:20 to 20:80) to obtain the title compound (125 mg) as a colorless powder. MS (ESI) m/z: 442.2 (M+H)⁺.

### (Reference Example 114-3)

### tert-Butyl 2-(2-oxo-1,3-diazepan-1-yl)-1,3-benzothiazole-6-carboxylate

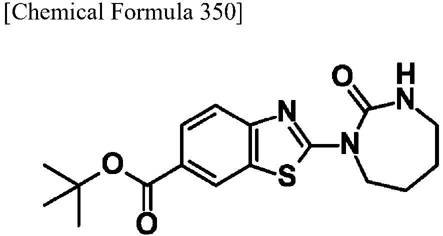

2-[3-[(2,4-Dimethoxyphenyl)methyl]-2-oxo-1,3-diazepan-1-yl]-1,3-benzothiazole-6-carboxylic acid (120 mg) was dissolved in dichloromethane (1 mL), and triisopropylsilane (52 mg) and trifluoroacetic acid (1 mL) were added, followed by stirring at room temperature for 18 hours and then at 60 °C for 8 hours. The reaction mixture was concentrated, and toluene was added to the residue followed by concentration. The residue was washed by suspension with ethyl acetate, filtered, and dried under reduced pressure to obtain 2-(2-oxo-1,3-diazepan-1-yl)-1,3-benzothiazole-6-carboxylic acid (72 mg). This was suspended in dichloromethane (1 mL), and 2-tert-butyl-1,3-diisopropylisourea (248 mg) was added, followed by stirring at room temperature for 46 hours. The reaction mixture was concentrated, and the residue was purified by silica gel column chromatography (hexane:ethyl acetate = 80:20 to 55:45) to obtain the title compound (72 mg) as a colorless powder. MS (ESI) m/z: 348.1 (M+H)⁺

### Example 114

### tert-Butyl 2-(2,4-dioxo-1,3-diazepan-1-yl)-1,3-benzothiazole-6-carboxylate

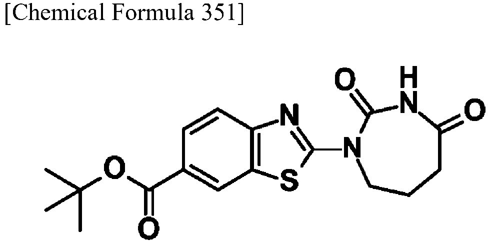

Using tert-butyl 2-(2-oxo-1,3-diazepan-1-yl)-1,3-benzothiazole-6-carboxylate instead of tert-butyl 2-[2-fluoro-3-[(2-oxoazepan-3-yl)carbamoyl]phenoxy]acetate in Example 67, the same reaction and processing were performed to obtain the title compound (18 mg) as a colorless powder. MS (ESI) m/z: 362.1 (M+H)⁺

### Reference Example and Example 115

### Reference Example 115

### tert-Butyl 2-(2-oxo-1,3-diazepan-1-yl)thiazole-5-carboxylate

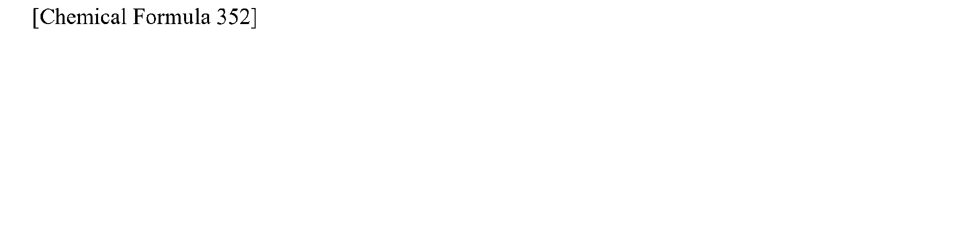

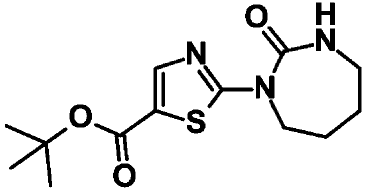

Using ethyl 2-aminothiazole-5-carboxylate instead of ethyl 2-amino-1,3-benzothiazole-6-carboxylate in (Reference Example 114-1), the same reactions and processing as in (Reference Example 114-1), (Reference Example 114-2), and (Reference Example 114-3) were performed to obtain the title compound (287 mg) as a colorless powder. MS (ESI) m/z: 298.1 (M+H)⁺

### Example 115

### tert-Butyl 2-(2,4-dioxo-1,3-diazepan-1-yl)thiazole-5-carboxylate

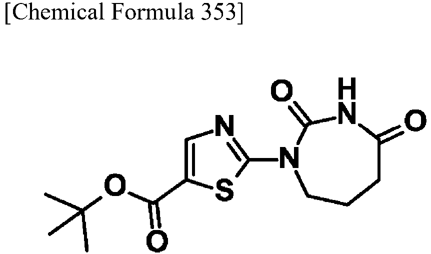

Using tert-butyl 2-(2-oxo-1,3-diazepan-1-yl)thiazole-5-carboxylate instead of tert-butyl 2-[2-fluoro-3-[(2-oxoazepan-3-yl)carbamoyl]phenoxy]acetate in Example 67, the same reaction and processing were performed to obtain the title compound (26 mg) as a colorless powder. MS (ESI) m/z: 256.1 (M-tBu+H)⁺

### Reference Example and Example 116

### Reference Example 116

### (Reference Example 116-1)

### tert-Butyl N-[4-[(6-nitro-1,3-benzothiazol-2-yl)amino]butyl]carbamate

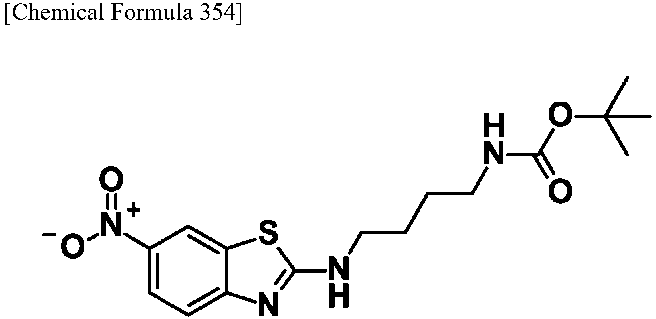

Using tert-butyl N-(4-aminobutyl)carbamate instead of 2,7-diazaspiro[4.6]undecan-6-one:2,2,2-trifluoroacetate in Example 67, the same reaction and processing were performed to obtain the title compound (3.01 g) as a pale yellow powder. MS (ESI) m/z: 367.1 (M+H)⁺

### (Reference Example 116-2)

### N'-(6-Nitro-1,3-benzothiazol-2-yl)butane-1,4-diamine

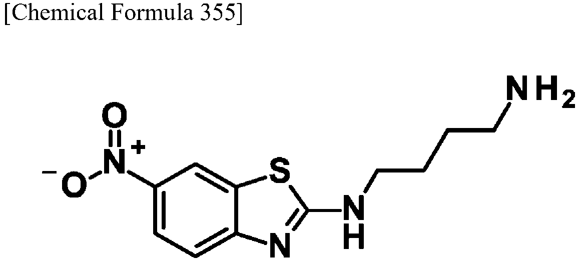

tert-Butyl N-[4-[(6-nitro-1,3-benzothiazol-2-yl)amino]butyl]carbamate (3.00 g) was suspended in dichloromethane (10 mL), and trifluoroacetic acid (5 mL) was added, followed by stirring at room temperature for 0.5 hours. The reaction mixture was concentrated, and then chloroform and 1N aqueous sodium hydroxide were added to the resulting residue, followed by extraction. The organic layer was dried over anhydrous sodium sulfate, and the solvent was evaporated under reduced pressure to obtain the title compound (1.77 g) as a yellow powder. MS (ESI) m/z: 267.1 (M+H)⁺

### (Reference Example 116-3)

### 1-(6-Nitro-1,3-benzothiazol-2-yl)-1,3-diazepan-2-one

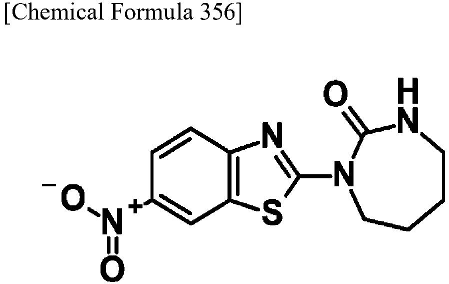

N'-(6-Nitro-1,3-benzothiazol-2-yl)butane-1,4-diamine (1.00 g) was suspended in tetrahydrofuran (30 mL), and N,N-diisopropylethylamine (1.95 mL) and triphosgene (446 mg) were added, followed by stirring at room temperature for 3 hours. The reaction mixture was concentrated, and then chloroform and 1N hydrochloric acid were added to the resulting residue, followed by extraction. The organic layer was dried over anhydrous sodium sulfate, and the solvent was evaporated under reduced pressure. The residue was washed by suspension with chloroform, filtered, and dried under reduced pressure to obtain 1-(6-nitro-1,3-benzothiazol-2-yl)-1,3-diazepan-2-one (420 mg) as a pale yellow powder. The previous filtrate was concentrated, and the residue was purified by silica gel column chromatography (hexane:ethyl acetate = 70:30 to 30:70) to obtain the title compound (216 mg) as a pale yellow powder. MS (ESI) m/z: 293.1 (M+H)⁺

### Example 116

### 1-(6-Nitro-1,3-benzothiazol-2-yl)-1,3-diazepane-2,4-dione

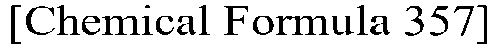

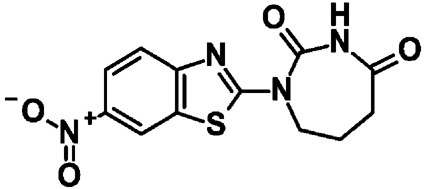

Using 1-(6-nitro-1,3-benzothiazol-2-yl)-1,3-diazepan-2-one instead of tert-butyl 2-[2-fluoro-3-[(2-oxoazepan-3-yl)carbamoyl]phenoxy]acetate in Example 67, the same reaction and processing were performed to obtain the title compound (64 mg) as a colorless powder. MS (ESI) m/z: 307.0 (M+H)⁺

### Example 117

### 1-Quinolin-6-yl-1,3-diazepane-2,4-dione

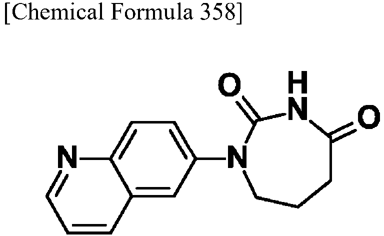

Using 1-quinolin-6-yl-1,3-diazepan-2-one instead of tert-butyl 2-[2-fluoro-3-[(2-oxoazepan-3-yl)carbamoyl]phenoxy]acetate in Example 67, the same reaction and processing were performed to obtain the title compound (14 mg) as a colorless powder. MS (ESI) m/z: 256.1 (M+H)⁺

### Example 118

### 1-(6-Amino-1,3-benzothiazol-2-yl)-1,3-diazepane-2,4-dione

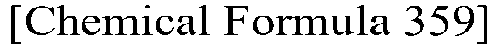

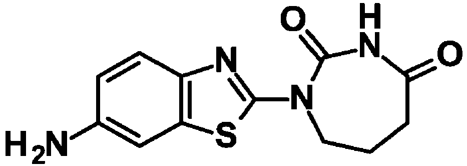

Using 1-(6-nitro-1,3-benzothiazol-2-yl)-1,3-diazepane-2,4-dione instead of 3-(3-nitrophenyl)azepane-2,7-dione in Example 70, the same reaction and processing were performed to obtain the title compound (21 mg) as a brown powder. MS (ESI) m/z: 277.0 (M+H)⁺

### Example 119

### N-[2-(2,4-Dioxo-1,3-diazepan-1-yl)-1,3-benzothiazol-6-yl]acetamide

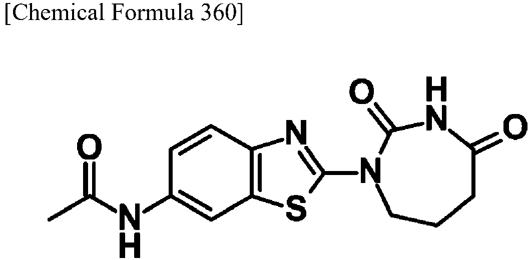

Using 1-(6-amino-1,3-benzothiazol-2-yl)-1,3-diazepane-2,4-dione instead of 3-(3-aminophenyl)azepane-2,7-dione in Example 71, the same reaction and processing were performed to obtain the title compound (5 mg) as a brown powder. MS (ESI) m/z: 277.0 (M+H)⁺

### Reference Example and Example 120

### Reference Example 120

### 3-(N-Methyl-4-nitro-anilino)azepan-2-one

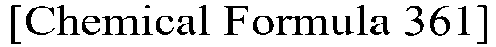

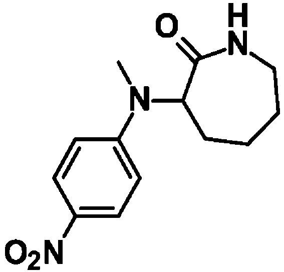

3-(4-Nitroanilino)azepan-2-one (300 mg) was dissolved in N,N-dimethylformamide (3 mL) with heating, and potassium carbonate (33 mg) and methyl iodide (257 mg) were added, followed by stirring at 100 °C for 3 hours. Additional methyl iodide (5.14 g) was added to the reaction mixture, and the mixture was stirred at 100 °C for 2 hours. Further methyl iodide (10.3 g) was added, and the mixture was stirred at 100 °C for 5 hours. Another portion of methyl iodide (10.3 g) was added, and the mixture was stirred at 100 °C for 2 hours. Water was added to the reaction mixture, and the mixture was extracted with ethyl acetate. The organic layer was washed with water and saturated brine, and dried over anhydrous sodium sulfate, and then the solvent was evaporated under reduced pressure. The residue was purified by silica gel column chromatography (chloroform:methanol = 100:0 to 94:6) to obtain the title compound (98 mg) as a yellow powder. MS (ESI) m/z: 264.3 (M+H)⁺

### Example 120

### 3-(N-Methyl-4-nitro-anilino)azepane-2,7-dione

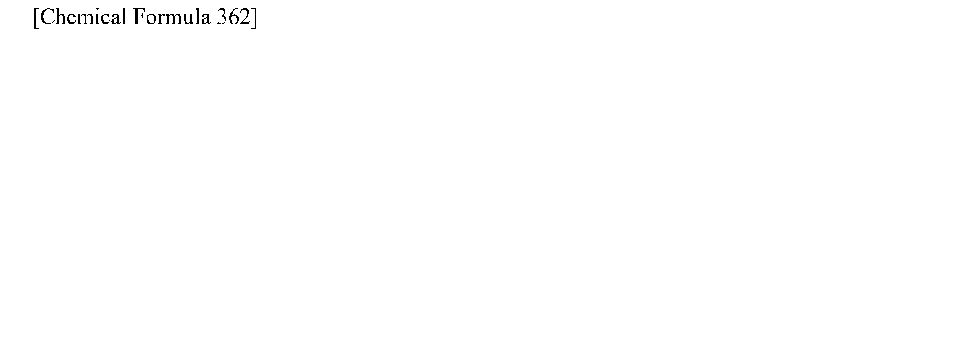

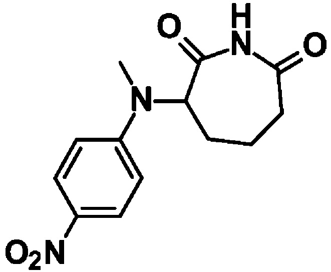

Using 3-(N-methyl-4-nitro-anilino)azepan-2-one instead of tert-butyl 2-[2-fluoro-3-[(2-oxoazepan-3-yl)carbamoyl]phenoxy]acetate in Example 67, the same reaction and processing were performed to obtain the title compound (72 mg) as a yellow powder. MS (ESI) m/z: 278.1 (M+H)⁺

### Example 121

### N-[4-[(2,7-Dioxoazepan-3-yl)-methyl-amino]phenyl]acetamide

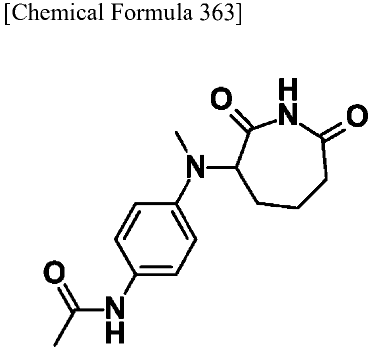

Using 3-(N-methyl-4-nitro-anilino)azepane-2,7-dione instead of 3-(3-nitrophenyl)azepane-2,7-dione in Example 70, the same reactions and processing as in Example 70 and Example 71 were performed to obtain the title compound (21 mg) as a brown powder. MS (ESI) m/z: 290.1 (M+H)⁺

### Example 122

### 2-Benzoyl-2,7-diazaspiro[4.6]undecane-6,8-dione

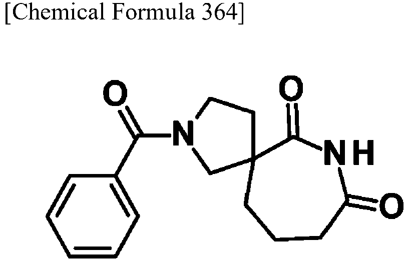

Using tert-butyl 6,8-dioxo-2,7-diazaspiro[4.6]undecane-2-carboxylate instead of tert-butyl 7,9-dioxo-2,8-diazaspiro[5.6]dodecane-2-carboxylate in Example 104, the same reaction and processing were performed to obtain the title compound (59 mg) as a colorless powder. MS (ESI) m/z: 287.2 (M+H)⁺

### Reference Example and Example 123

### Reference Example 123

### (Reference Example 123-1)

### O1-tert-Butyl O3-methyl 3-(4-bromobutyl)pyrrolidine-1,3-dicarboxylate

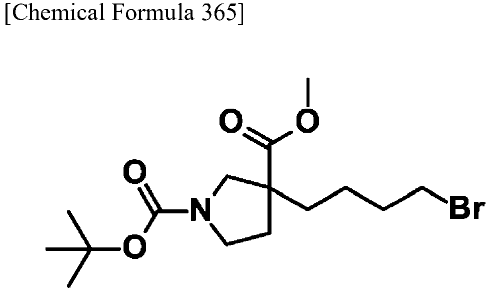

1-tert-Butyl 3-methyl pyrrolidine-1,3-dicarboxylate (22.0 g) was dissolved in tetrahydrofuran (220 mL), and under a nitrogen atmosphere in a dry ice-acetone bath, lithium bis(trimethylsilyl)amide (about 26% tetrahydrofuran solution, 95.0 mL) was added dropwise over about 15 minutes. The mixture was stirred at the same temperature for 1 hour. 1,4-Dibromobutane (30.0 g) was added dropwise over 5 minutes, the mixture was stirred at the same temperature for 0.5 hours, and then stirred while warming to -10 °C over 1 hour. Saturated aqueous ammonium chloride (40 mL) was added to the reaction mixture. After warming to room temperature, the mixture was diluted with water (30 mL) and extracted twice with ethyl acetate. The organic layer was washed with saturated brine, and dried over anhydrous sodium sulfate, and then the solvent was evaporated under reduced pressure. The residue was purified by silica gel column chromatography (hexane:ethyl acetate = 85:15 to 70:30) to obtain the title compound (20.4 g) as a colorless viscous material. MS (ESI) m/z: 307.8/309.8 (M-tBu+H)⁺

### (Reference Example 123-2)

### O1-tert-Butyl O3-methyl 3-(4-aminobutyl)pyrrolidine-1,3-dicarboxylate

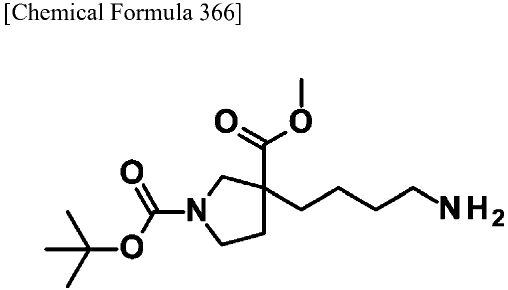

O1-tert-Butyl O3-methyl 3-(4-bromobutyl)pyrrolidine-1,3-dicarboxylate (20.4 g) was dissolved in N,N-dimethylformamide (200 mL), and tetrabutylammonium azide (21.5 g) was added, followed by stirring at 90 °C for 3 hours. After cooling to room temperature, saturated brine was added to the reaction mixture, and the mixture was extracted three times with hexane. The organic layer was washed with saturated brine, and dried over anhydrous sodium sulfate, and then the solvent was evaporated under reduced pressure. The residue was dissolved in tetrahydrofuran (200 mL), and triphenylphosphine (20.9 g) and water (20 mL) were added, followed by stirring at room temperature for 18 hours. The reaction mixture was concentrated and purified by silica gel column chromatography (chloroform:methanol = 100:0 to 80:20).

Residual chloroform was removed by azeotroping with ethyl acetate, to obtain the title compound (11.9 g) as a colorless viscous material. MS (ESI) m/z: 301.0 (M+H)⁺

### (Reference Example 123-3)

### tert-Butyl 6-oxo-2,7-diazaspiro[4.6]undecane-2-carboxylate

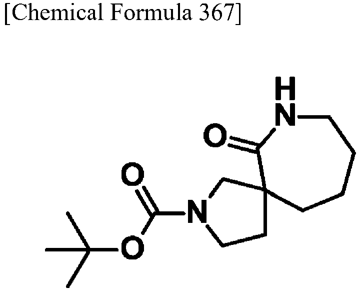

O1-tert-Butyl O3-methyl 3-(4-aminobutyl)pyrrolidine-1,3-dicarboxylate (3.30 g) was dissolved in methanol (30 mL), and 2N aqueous sodium hydroxide (11.0 mL) was added, followed by stirring at room temperature for 5 hours. The reaction mixture was neutralized by adding 1N hydrochloric acid, followed by concentration under reduced pressure. To the residue, acetonitrile (100 mL), N,N-diisopropylethylamine (5.70 mL), and O-(7-azabenzotriazol-1-yl)-N,N,N',N'-tetramethyluronium hexafluorophosphate (6.27 g) were added, and the mixture was stirred at room temperature for 2 hours. Water and chloroform were added to the reaction mixture, and the organic layer was concentrated. The residue was purified by silica gel column chromatography (hexane:ethyl acetate = 85:15 to 70:30) to obtain the title compound (1.61 g) as a pale yellow viscous material. MS (ESI) m/z: 269.2 (M+H)⁺

### (Reference Example 123-4)

### 2,7-Diazaspiro[4.6]undecan-6-one; hydrochloride

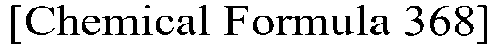

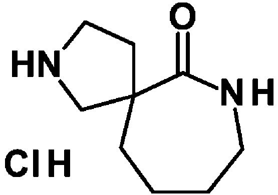

tert-Butyl 6-oxo-2,7-diazaspiro[4.6]undecane-2-carboxylate (1.37 g) was dissolved in chloroform (20 mL), and 4M hydrogen chloride (dioxane solution) (10 mL) was added, followed by stirring at room temperature for 26 hours. The reaction mixture was concentrated, and diethyl ether (30 mL) was added to the residue, followed by suspension washing, filtration, and drying to obtain the title compound (955 mg) as a colorless powder. MS (ESI) m/z: 169.1 (M+H)⁺

### (Reference Example 123-5)

### tert-Butyl 3-(6-oxo-2,7-diazaspiro[4.6]undecan-2-yl)benzoate

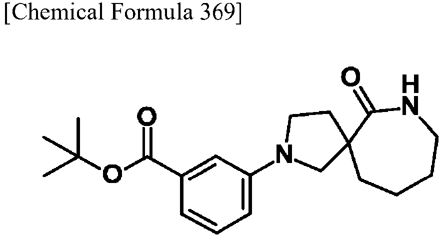

tert-Butyl 3-bromobenzoate (230 mg), 2,7-diazaspiro[4.6]undecan-6-one; hydrochloride (203 mg), tris(dibenzylideneacetone)dipalladium (42 mg), 2-dicyclohexylphosphino-2'-(N,N-dimethylamino)biphenyl (41 mg), and potassium phosphate (955 mg) were suspended in N,N-dimethylformamide (4 mL) and stirred under microwave irradiation at 130 °C for 1 hour. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 75:25 to 0:100) to obtain the title compound (180 mg) as a light brown powder. MS (ESI) m/z: 345.1 (M+H)⁺

### Example 123

### tert-Butyl 3-(6,8-dioxo-2,7-diazaspiro[4.6]undecan-2-yl)benzoate

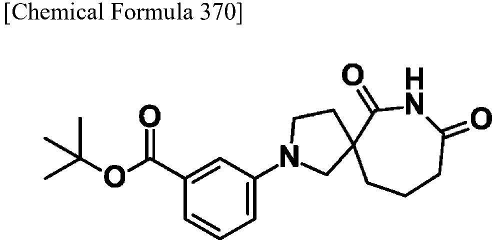

Using tert-butyl 3-(6-oxo-2,7-diazaspiro[4.6]undecan-2-yl)benzoate instead of tert-butyl 2-[2-fluoro-3-[(2-oxoazepan-3-yl)carbamoyl]phenoxy]acetate in Example 67, the same reaction and processing were performed to obtain the title compound (20 mg) as an orange powder. MS (ESI) m/z: 359.2 (M+H)⁺

### Reference Example and Example 124

### Reference Example 124

### 2-(3-Nitrophenyl)-2,7-diazaspiro[4.6]undecan-6-one

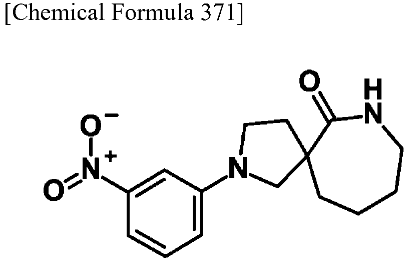

Using 1-bromo-3-nitrobenzene instead of tert-butyl 3-bromobenzoate in (Reference Example 123-5), the same reaction and processing were performed to obtain the title compound (166 mg) as an orange powder. MS (ESI) m/z: 290.0 (M+H)⁺

### Example 124

### 2-(3-Nitrophenyl)-2,7-diazaspiro[4.6]undecan-6,8-dione

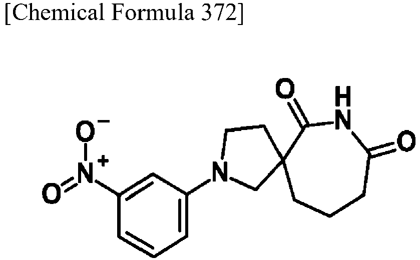

Using 2-(3-nitrophenyl)-2,7-diazaspiro[4.6]undecan-6-one instead of tert-butyl 2-[2-fluoro-3-[(2-oxoazepan-3-yl)carbamoyl]phenoxy]acetate in Example 67, the same reaction and processing were performed to obtain the title compound (20 mg) as an orange powder. MS (ESI) m/z: 303.9 (M+H)⁺

### Reference Example and Example 125

### Reference Example 125

### 2-(4-Methoxy-2-pyridyl)-2,7-diazaspiro[4.6]undecan-6-one

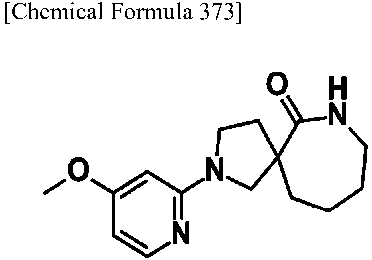

2-Chloro-4-methoxypyridine (89 mg), 2,7-diazaspiro[4.6]undecan-6-one; hydrochloride (102 mg), tris(dibenzylideneacetone)dipalladium (21 mg), dicyclohexyl-[2-(2,6-diisopropoxyphenyl)phenyl]phosphane (24 mg), sodium tert-butoxide (145 mg), and N,N-diisopropylethylamine (90 µL) were suspended in toluene (5 mL), purged with nitrogen, and stirred at 100 °C for 3 hours. Ethyl acetate and water were added to the reaction mixture, and the mixture was extracted twice with ethyl acetate. The organic layer was washed with water and saturated brine, and dried over anhydrous sodium sulfate, and then the solvent was evaporated under reduced pressure. The residue was purified by silica gel column chromatography (hexane:ethyl acetate = 60:40 to 0:100) to obtain the title compound (29 mg) as an orange powder. MS (ESI) m/z: 276.0 (M+H)⁺

### Example 125

### 2-(4-Methoxy-2-pyridyl)-2,7-diazaspiro[4.6]undecane-6,8-dione

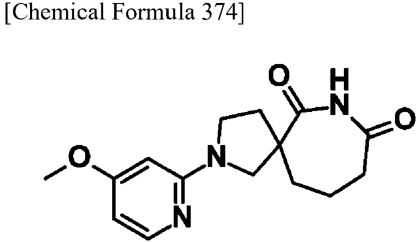

Using 2-(4-methoxy-2-pyridyl)-2,7-diazaspiro[4.6]undecan-6-one instead of tert-butyl 2-[2-fluoro-3-[(2-oxoazepan-3-yl)carbamoyl]phenoxy]acetate in Example 67, the same reaction and processing were performed to obtain the title compound (5.4 mg) as a light brown powder. MS (ESI) m/z: 290.0 (M+H)⁺

### Reference Example and Example 126

### Reference Example 126

### tert-Butyl 4-[2-(6-oxo-2,7-diazaspiro[4.6]undecan-2-yl)-4-pyridyl]piperazine-1-carboxylate

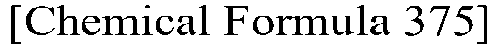

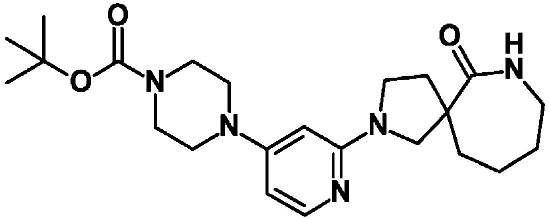

Using tert-butyl 4-(2-chloro-4-pyridyl)piperazine-1-carboxylate instead of 2-chloro-4-methoxypyridine in Reference Example 125, the same reaction and processing were performed to obtain the title compound (47 mg) as a pale yellow powder. MS (ESI) m/z: 430.3 (M+H)⁺

### Example 126

### tert-Butyl 4-[2-(6,8-dioxo-2,7-diazaspiro[4.6]undecan-2-yl)-4-pyridyl]piperazine-1-carboxylate

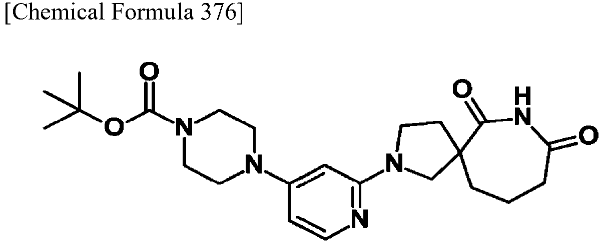

Using tert-butyl 4-[2-(6-oxo-2,7-diazaspiro[4.6]undecan-2-yl)-4-pyridyl]piperazine-1-carboxylate instead of tert-butyl 2-[2-fluoro-3-[(2-oxoazepan-3-yl)carbamoyl]phenoxy]acetate in Example 67, the same reaction and processing were performed to obtain the title compound (5.9 mg) as an orange powder. MS (ESI) m/z: 444.1 (M+H)⁺

### Reference Example and Example 127

### Reference Example 127

### 2-(5-Bromopyrimidin-2-yl)-2,7-diazaspiro[4.6]undecan-6-one

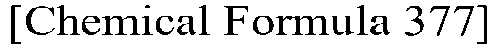

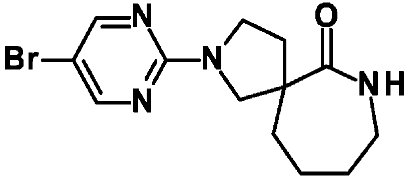

tert-Butyl 6-oxo-2,7-diazaspiro[4.6]undecane-2-carboxylate (614 mg) was dissolved in dichloromethane (5 mL), and trifluoroacetic acid (2.5 mL) was added, followed by stirring at room temperature for 2 hours. The reaction mixture was concentrated and azeotroped with toluene. The residue was dissolved in N,N-dimethylformamide (4 mL), and 5-bromo-2-fluoropyridine (380 mg) and N,N-diisopropylethylamine (1.50 mL) were added, followed by stirring at 80 °C for 1.5 hours. After cooling to room temperature, ice water (40 mL) was added, and the mixture was stirred at room temperature for 0.5 hours. The precipitate was filtered and dried to obtain the title compound (611 mg) as a light brown powder. MS (ESI) m/z: 324.9/326.9 (M+H)⁺

### Example 127

### 2-(5-Bromopyrimidin-2-yl)-2,7-diazaspiro[4.6]undecane-6,8-dione

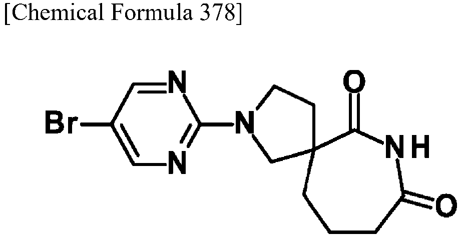

Using 2-(5-bromopyrimidin-2-yl)-2,7-diazaspiro[4.6]undecan-6-one instead of tert-butyl 2-[2-fluoro-3-[(2-oxoazepan-3-yl)carbamoyl]phenoxy]acetate in Example 67, the same reaction and processing were performed to obtain the title compound (88 mg) as a colorless powder. MS (ESI) m/z: 339.1/341.1 (M+H)⁺

### Reference Example and Example 128

### Reference Example 128

### (Reference Example 128-1)

### 1-(4-tert-Butoxycarbonylphenyl)-pyrrolidine-3-carboxylic acid

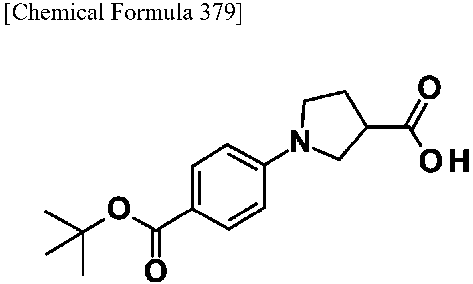

tert-Butyl 4-fluorobenzoate (7.85 g) and pyrrolidine-3-carboxylic acid (2.31 g) were suspended in dimethyl sulfoxide (40 mL), and potassium carbonate (6.95 g) was added. The mixture was stirred at 140 °C for 1.5 hours. After cooling to room temperature, the reaction mixture was poured into ice water (300 mL). After washing with ethyl acetate, the aqueous layer was acidified by adding 1N hydrochloric acid. The mixture was extracted twice with ethyl acetate, the organic layer was washed with water and saturated brine and dried over anhydrous sodium sulfate, and the solvent was evaporated under reduced pressure. The residue was purified by silica gel column chromatography (hexane:ethyl acetate = 80:20 to 20:80) to obtain the title compound (5.03 g) as a colorless powder. MS (ESI) m/z: 292.1 (M+H)⁺

### (Reference Example 128-2)

### Benzyl 1-(4-tert-butoxycarbonylphenyl)pyrrolidine-3-carboxylate

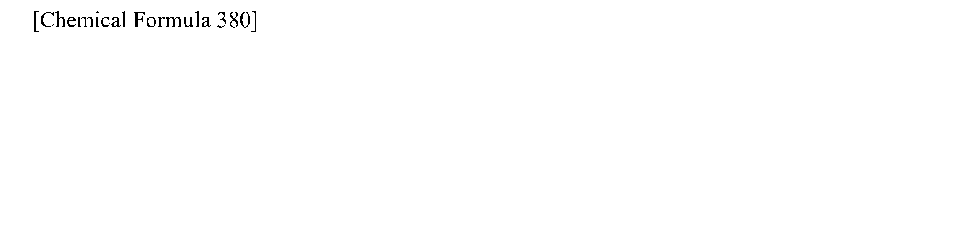

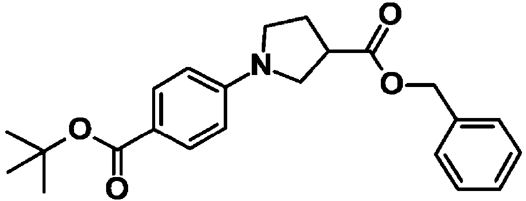

1-(4-tert-Butoxycarbonylphenyl)-pyrrolidine-3-carboxylic acid (2.97 g) was azeotroped with toluene, and then dissolved in N,N-dimethylformamide (35 mL), and sodium hydride (385 mg) was added under ice-cooling. The mixture was stirred at room temperature for 10 minutes, and then cooled with ice, and benzyl bromide (1.20 mL) was added, followed by stirring at the same temperature for 0.5 hours. The mixture was warmed to room temperature and stirred for 1 hour. The mixture was warmed to 80 °C, and stirred for 0.5 hours. The reaction mixture was cooled to room temperature, poured into ice water, and extracted twice with ethyl acetate. The organic layer was washed with water and saturated brine, and dried over anhydrous sodium sulfate, and then the solvent was evaporated under reduced pressure. The residue was purified by silica gel column chromatography (hexane:ethyl acetate = 95:5 to 80:20) to obtain the title compound (3.31 g) as a colorless powder. MS (ESI) m/z: 382.1 (M+H)⁺

### (Reference Example 128-3)

### Benzyl 3-(4-bromobutyl)-1-(4-tert-butoxycarbonylphenyl)pyrrolidine-3-carboxylate

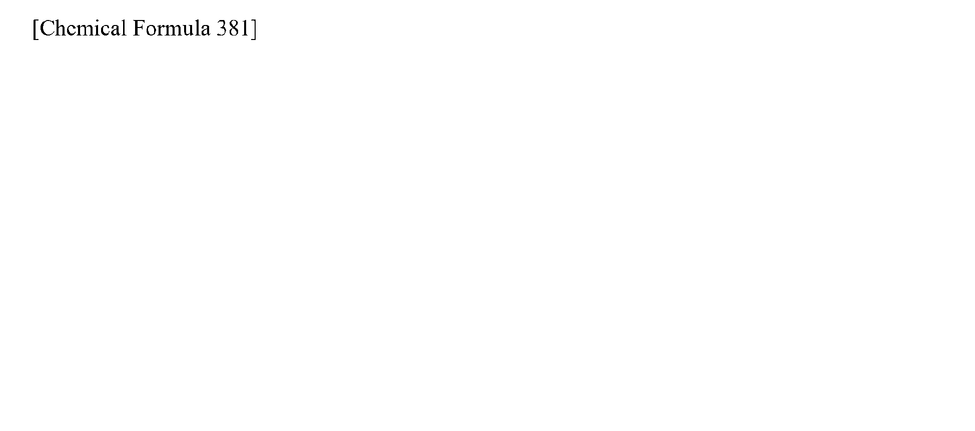

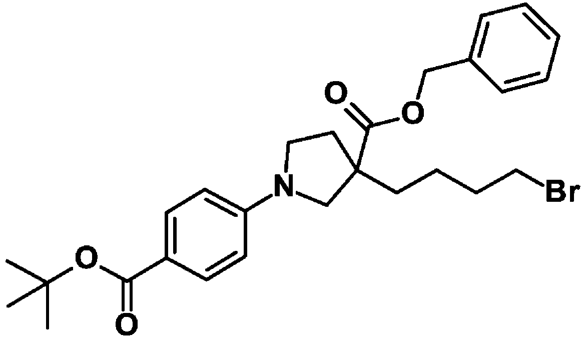

Using benzyl 1-(4-tert-butoxycarbonylphenyl)pyrrolidine-3-carboxylate instead of 1-tert-butyl 3-methyl pyrrolidine-1,3-dicarboxylate in (Reference Example 123-1), the same reaction and processing were performed to obtain the title compound (958 mg) as a colorless powder. MS (ESI) m/z: 516.0/518.0 (M+H)⁺

### (Reference Example 128-4)

### tert-Butyl 4-(6-oxo-2,7-diazaspiro[4.6]undecan-2-yl)benzoate

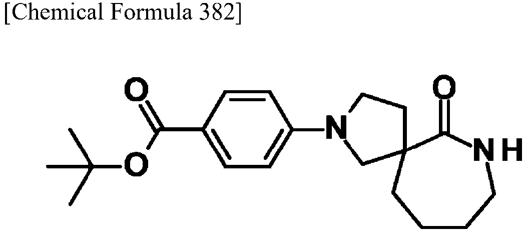

Using benzyl 3-(4-bromobutyl)-1-(4-tert-butoxycarbonylphenyl)pyrrolidine-3-carboxylate instead of benzyl 4-(4-chlorobutyl)-2-oxopiperidine-4-carboxylate in (Reference Example 107-3), the same reactions and processing as in (Reference Example 107-3), (Reference Example 107-5), and Reference Example (107-6) were performed to obtain the title compound (245 mg) as a colorless powder. MS (ESI) m/z: 345.3 (M+H)⁺

### Example 128

### tert-Butyl 4-(6,8-dioxo-2,7-diazaspiro[4.6]undecan-2-yl)benzoate

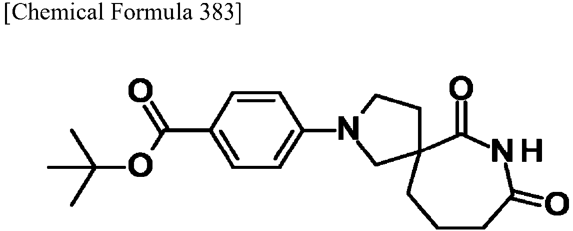

Using tert-butyl 4-(6-oxo-2,7-diazaspiro[4.6]undecan-2-yl)benzoate instead of tert-butyl 2-[2-fluoro-3-[(2-oxoazepan-3-yl)carbamoyl]phenoxy]acetate in Example 67, the same reaction and processing were performed to obtain the title compound (85 mg) as a colorless powder. MS (ESI) m/z: 359.3 (M+H)⁺

### Reference Example and Example 129

### Reference Example 129

### (Reference Example 129-1)

### tert-Butyl 3-allyl-3-(aminomethyl)pyrrolidine-1-carboxylate

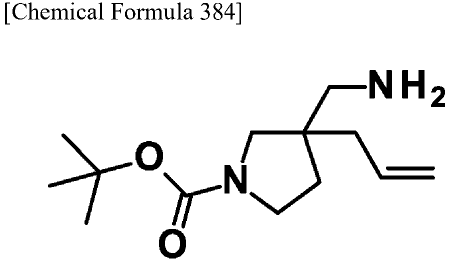

tert-Butyl 3-allyl-3-(hydroxymethyl)pyrrolidine-1-carboxylate (1.36 g) and triphenylphosphine (2.23 g) were dissolved in tetrahydrofuran (14 mL), and diphenylphosphoryl azide (1.82 mL) and diisopropyl azodicarboxylate (40% toluene solution, 4.50 mL) were added, followed by stirring at 50 °C for 10 minutes. Additional triphenylphosphine (758 mg) and diphenylphosphoryl azide (1.20 mL) were added, and the mixture was stirred at 50 °C for 1 hour. Triphenylphosphine (3.74 g), tetrahydrofuran (14 mL), and water (3 mL) were added to the reaction mixture, and the mixture was stirred at 50 °C for 1 hour. The reaction mixture was concentrated, and the residue was purified by silica gel column chromatography (chloroform:methanol = 100:0 to 90:10) to obtain the title compound (870 mg) as a pale yellow viscous material. MS (ESI) m/z: 185.0 (M-tBu+H)⁺

### (Reference Example 129-2)

### tert-Butyl 3-allyl-3-[(prop-2-enoylamino)methyl]pyrrolidine-1-carboxylate

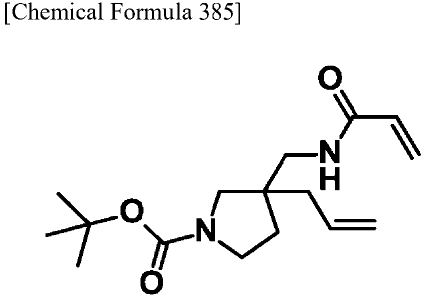

tert-Butyl 3-allyl-3-(aminomethyl)pyrrolidine-1-carboxylate (870 mg) was dissolved in tetrahydrofuran (9 mL) and saturated aqueous sodium bicarbonate (1 mL), and acryloyl chloride (344 mg) was added. Additional tetrahydrofuran (9 mL) and saturated aqueous sodium bicarbonate (4 mL) were added, and the mixture was stirred at room temperature for 0.5 hours. Additional acryloyl chloride (164 mg) was added, and the mixture was stirred at room temperature for 0.5 hours. The reaction mixture was diluted with saturated aqueous sodium bicarbonate and ethyl acetate, and then extracted three times with ethyl acetate. The organic layer was dried over anhydrous sodium sulfate, and the solvent was evaporated under reduced pressure. The residue was purified by silica gel column chromatography (hexane:ethyl acetate = 50:50 to 20:80) to obtain the title compound (975 mg) as a colorless viscous material. MS (ESI) m/z: 195.0 (M-Boc+H)⁺

### (Reference Example 129-3)

### tert-Butyl 9-oxo-2,10-diazaspiro[4.6]undec-7-ene-2-carboxylate

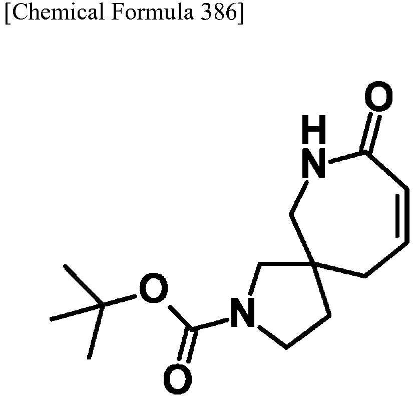

tert-Butyl 3-allyl-3-[(prop-2-enoylamino)methyl]pyrrolidine-1-carboxylate (200 mg) and second-generation Grubbs catalyst (15 mg) were dissolved in dichloromethane (34 mL), purged with nitrogen, and stirred at 40 °C for 1 hour. Dimethyl sulfoxide (60 µL) was added, and the mixture was stirred at room temperature overnight. The reaction mixture was concentrated, and the residue was purified by silica gel column chromatography (chloroform:methanol = 100:0 to 95:5) to obtain the title compound (173 mg) as a colorless powder. MS (ESI) m/z: 267.1 (M+H)⁺

### Example 129

### tert-Butyl 6,8-dioxo-2,7-diazaspiro[4.6]undec-9-ene-2-carboxylate

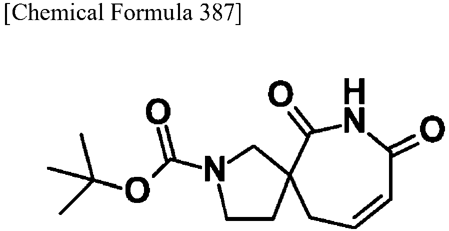

Using tert-butyl 9-oxo-2,10-diazaspiro[4.6]undec-7-ene-2-carboxylate instead of tert-butyl 2-[2-fluoro-3-[(2-oxoazepan-3-yl)carbamoyl]phenoxy]acetate in Example 67, the same reaction and processing were performed to obtain the title compound (88 mg) as a colorless viscous material. MS (ESI) m/z: 279.1 (M-H)⁻

### Example 130

### 2-Benzoyl-2,7-diazaspiro[4.6]undec-9-ene-6,8-dione

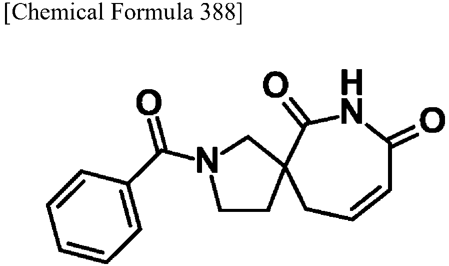

Using tert-butyl 6,8-dioxo-2,7-diazaspiro[4.6]undec-9-ene-2-carboxylate instead of tert-butyl 7,9-dioxo-2,8-diazaspiro[5.6]dodecane-2-carboxylate in Example 104, the same reaction and processing were performed to obtain the title compound (28 mg) as a colorless powder. MS (ESI) m/z: 285.0 (M+H)⁺

### Example 131

### tert-Butyl 6-(6,8-dioxo-2,7-diazaspiro[4.6]undec-9-en-2-yl)pyridine-3-carboxylate

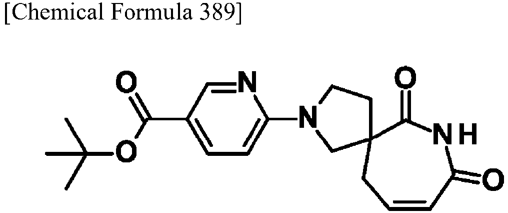

Using tert-butyl 6,8-dioxo-2,7-diazaspiro[4.6]undec-9-ene-2-carboxylate instead of tert-butyl 7-oxo-2,8-diazaspiro[5.6]dodecane-2-carboxylate in Reference Example 93, the same reaction and processing were performed to obtain the title compound (33 mg) as a white powder. MS (ESI) m/z: 358.3 (M+H)⁺

### Reference Example and Example 132

### Reference Example 132

### (Reference Example 132-1)

### Methyl 2-methyl-2-(3-nitrophenyl)-4-pentenoate

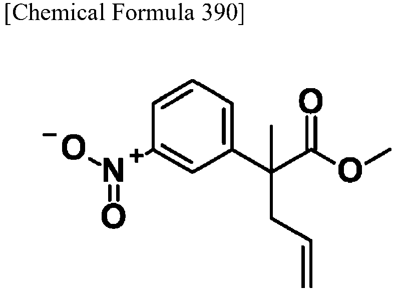

Methyl 2-(3-nitrophenyl)acetate (1.71 g) was dissolved in tetrahydrofuran (44 mL), and under a nitrogen atmosphere in a dry ice-acetone bath, lithium bis(trimethylsilyl)amide (about 26% tetrahydrofuran solution, 8.0 mL) was added dropwise. The mixture was stirred at the same temperature for 15 minutes. Allyl bromide (0.78 mL) was added dropwise, and the mixture was stirred under ice-cooling for 30 minutes. In the dry ice-acetone bath, lithium bis(trimethylsilyl)amide (about 26% tetrahydrofuran solution, 8.0 mL) was added dropwise, and the mixture was stirred at the same temperature for 30 minutes. Methyl iodide (1.70 mL) was added dropwise, and the mixture was stirred under ice-cooling for 45 minutes. Saturated aqueous ammonium chloride was added to the reaction mixture, and the mixture was extracted three times with ethyl acetate. The organic layer was dried over anhydrous sodium sulfate, and the solvent was evaporated under reduced pressure. The residue was purified by silica gel column chromatography (hexane:ethyl acetate = 100:0 to 80:20) to obtain the title compound (1.73 g) as an orange viscous material. MS (ESI) m/z: 250.0 (M+H)⁺

### (Reference Example 132-2)

### 2-Methyl-2-(3-nitrophenyl)-4-penten-1-ol

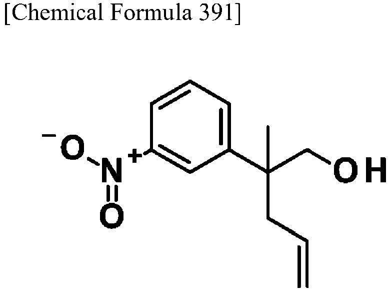

Methyl 2-methyl-2-(3-nitrophenyl)-4-pentenoate (1.72 g) was dissolved in dichloromethane (70 mL) and tetrahydrofuran (35 mL), and diisobutylaluminum hydride (1M toluene solution, 21 mL) was added at 0 °C. The mixture was stirred at the same temperature for 0.5 hours. Aqueous ammonium chloride was added to the reaction mixture, and the mixture was extracted three times with ethyl acetate. The organic layer was dried over anhydrous sodium sulfate, and the solvent was evaporated under reduced pressure. The residue was purified by silica gel column chromatography (hexane:ethyl acetate = 90:10 to 65:35) to obtain the title compound (641 mg) as a colorless powder. MS (ESI) not detected

### (Reference Example 132-3)

### 3-Methyl-3-(3-nitrophenyl)-2,4-dihydro-1H-azepin-7-one

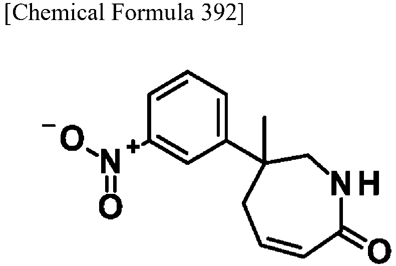

Using 2-methyl-2-(3-nitrophenyl)-4-penten-1-ol instead of tert-butyl 3-allyl-3-(hydroxymethyl)pyrrolidine-1-carboxylate in (Reference Example 129-1), the same reactions and processing as in (Reference Example 129-1), (Reference Example 129-2), and (Reference Example 129-3) were performed to obtain the title compound (260 mg) as a colorless viscous material. MS (ESI) m/z: 247.2 (M+H)⁺

### Example 132

### 3-Methyl-3-(3-nitrophenyl)-4H-azepine-2,7-dione

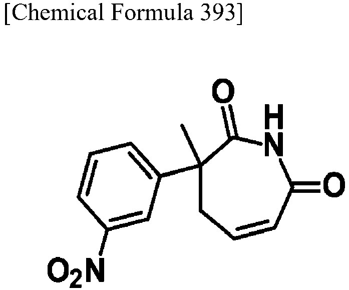

Using 3-methyl-3-(3-nitrophenyl)-2,4-dihydro-1H-azepin-7-one instead of tert-butyl 2-[2-fluoro-3-[(2-oxoazepan-3-yl)carbamoyl]phenoxy]acetate in Example 67, the same reaction and processing were performed to obtain the title compound (84 mg) as a white powder. MS (ESI) m/z: 260.9 (M+H)⁺

### Example 133

### 3-(3-Aminophenyl)-3-methyl-azepane-2,7-dione

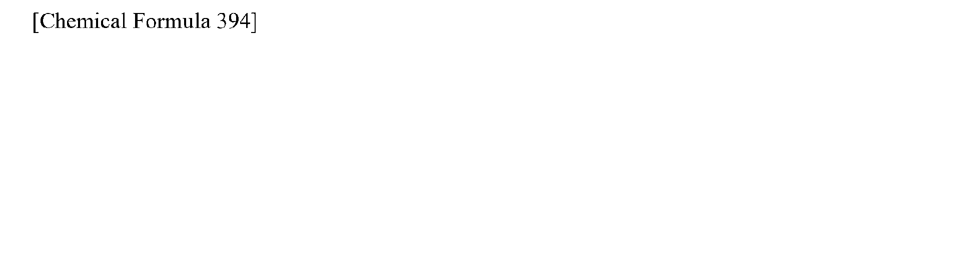

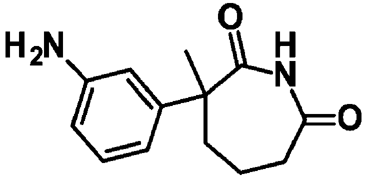

Using 3-methyl-3-(3-nitrophenyl)-4H-azepine-2,7-dione instead of 3-(3-nitrophenyl)azepane-2,7-dione in Example 70, the same reaction and processing were performed to obtain the title compound (32 mg) as a light brown viscous material. MS (ESI) m/z: 232.9 (M+H)⁺

### Example 134

### N-[3-(3-Methyl-2,7-dioxoazepan-3-yl)phenyl]acetamide

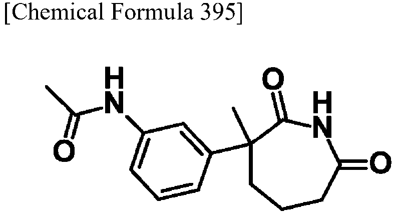

Using 3-(3-aminophenyl)-3-methyl-azepane-2,7-dione instead of 3-(3-aminophenyl)azepane-2,7-dione in Example 71, the same reaction and processing were performed to obtain the title compound (24 mg) as a white powder. MS (ESI) m/z: 275.0 (M+H)⁺

### Reference Example and Example 135

### Reference Example 135

### 3-(3-Iodoindazol-1-yl)azepan-2-one

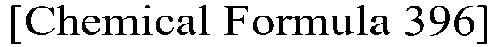

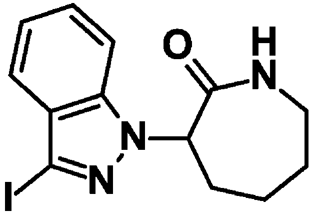

3-Iodoindazole (1.00 g), 3-bromoazepan-2-one (1.03 g), and potassium carbonate (850 mg) were suspended in N,N-dimethylformamide (10 mL) and stirred at 70 °C for 4 hours. Additional 3-bromoazepan-2-one (552 mg) and potassium carbonate (567 mg) were added, and the mixture was stirred at 70 °C for 4 hours. Water was added to the reaction mixture, and the mixture was extracted twice with ethyl acetate. The organic layer was washed with water and saturated brine, and dried over anhydrous sodium sulfate, and then the solvent was evaporated under reduced pressure. The residue was purified by silica gel column chromatography (hexane:ethyl acetate = 50:50 to 0:100) to obtain the title compound (373 mg) as a colorless powder. MS (ESI) m/z: 356.0 (M+H)⁺

### Example 135

### 3-(3-Iodoindazol-1-yl)azepane-2,7-dione

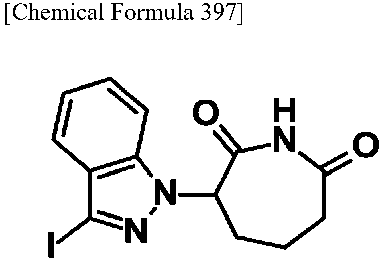

Using 3-(3-iodoindazol-1-yl)azepan-2-one instead of tert-butyl 2-[2-fluoro-3-[(2-oxoazepan-3-yl)carbamoyl]phenoxy]acetate in Example 67, the same reaction and processing were performed to obtain the title compound (224 mg) as a colorless powder. MS (ESI) m/z: 370.1 (M+H)⁺

### Example 136

### tert-Butyl (E)-3-[1-(2,7-oxazepan-3-yl)indazol-3-yl]-2-propenoate

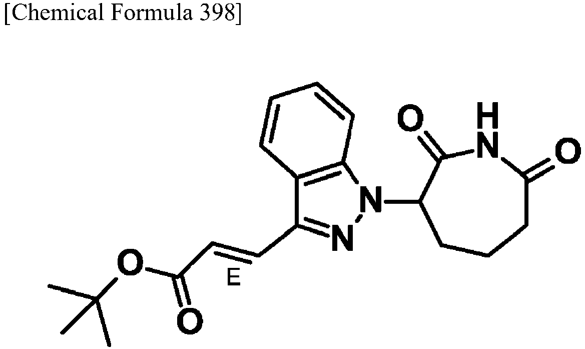

3-(3-Iodoindazol-1-yl)azepane-2,7-dione (50 mg), tert-butyl acrylate (53 mg), palladium acetate (3 mg), tri(o-tolyl)phosphine (9 mg), and triethylamine (42 mg) were dissolved in N,N-dimethylformamide (1 mL) and stirred under microwave irradiation at 120 °C for 1 hour. Water was added to the reaction mixture, and the mixture was extracted with ethyl acetate. The organic layer was washed with water and saturated brine, and dried over anhydrous sodium sulfate, and then the solvent was evaporated under reduced pressure. The residue was purified by silica gel column chromatography (hexane:ethyl acetate = 80:20 to 45:55) to obtain the title compound (23 mg) as a colorless powder. MS (ESI) m/z: 370.2 (M+H)⁺

### Example 137

### tert-Butyl 3-[1-(2,7-oxazepan-3-yl)indazol-3-yl]propanoate

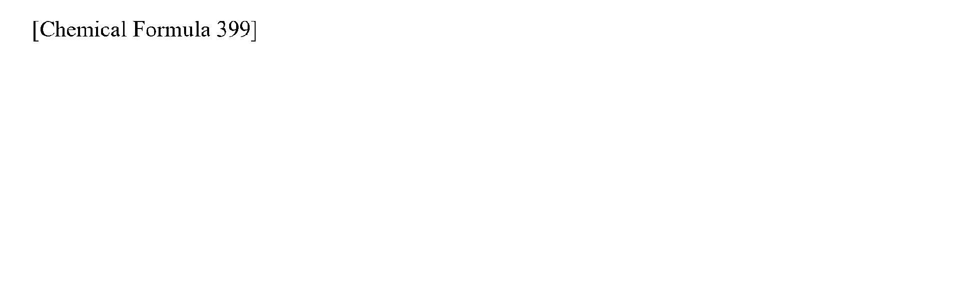

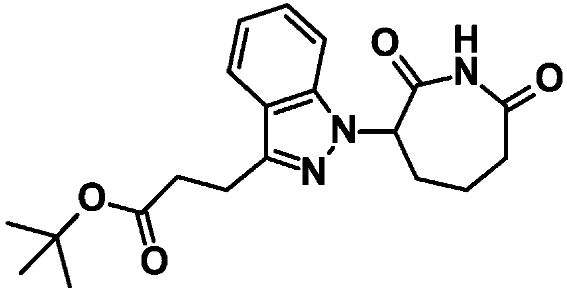

Using tert-butyl (E)-3-[1-(2,7-oxazepan-3-yl)indazol-3-yl]-2-propenoate instead of 3-(3-nitrophenyl)azepane-2,7-dione in Example 70, the same reaction and processing were performed to obtain the title compound (18 mg) as a brown powder. MS (ESI) m/z: 316.1 (M-tBu+H)⁺

### Reference Example and Example 138

### Reference Example 138

### (Reference Example 138-1)

### 3-(2-Nitroanilino)azepan-2-one

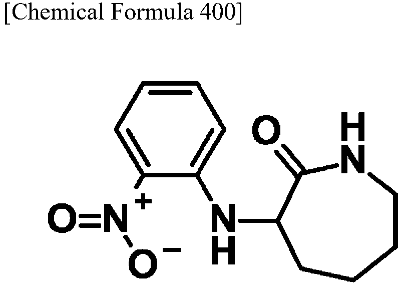

3-Aminoazepan-2-one (2.00 g), 1-fluoro-2-nitrobenzene (2.10 g), and N,N-diisopropylethylamine (2.90 g) were dissolved in acetonitrile (15 mL) and stirred under microwave irradiation at 120 °C for 1 hour. The reaction mixture was concentrated, and then chloroform and water were added to the residue, followed by extraction. The organic layer was dried over anhydrous sodium sulfate, and the solvent was evaporated under reduced pressure.

The residue was purified by silica gel column chromatography (hexane:ethyl acetate = 70:30 to 0:100) to obtain the title compound (3.28 g) as a yellow powder. MS (ESI) m/z: 250.1 (M+H)⁺

### (Reference Example 138-2)

### 3-(2-Aminoanilino)azepan-2-one

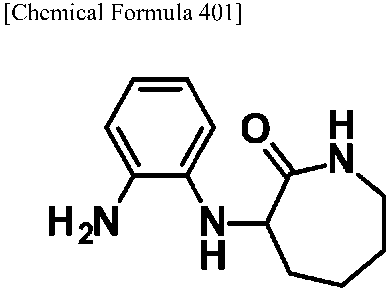

Sodium carbonate (12.1 g) was dissolved in water (150 mL), and sodium dithionite (12.6 g) was added slowly with attention to foaming. To this, a solution of 3-(2-nitroanilino)azepan-2-one (3.27 g) in ethanol (100 mL) and tetrahydrofuran (50 mL) was slowly added under ice-cooling, and then the mixture was warmed to room temperature and stirred for 0.5 hours. The reaction mixture was concentrated, and ethanol and tetrahydrofuran were removed by distillation. Chloroform was added, followed by extraction, and the organic layer was washed with water and saturated brine, and dried over anhydrous sodium sulfate, and the solvent was evaporated under reduced pressure. The residue was washed by suspension with ethyl acetate, filtered, and dried under reduced pressure to obtain the title compound (2.88 g) as a light brown powder. MS (ESI) m/z: 220.2 (M+H)⁺

### (Reference Example 138-3)

### tert-Butyl 2-[2-oxo-3 -(2-oxoazepan-3-yl)benzimidazol-1-yl] acetate

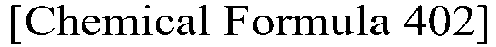

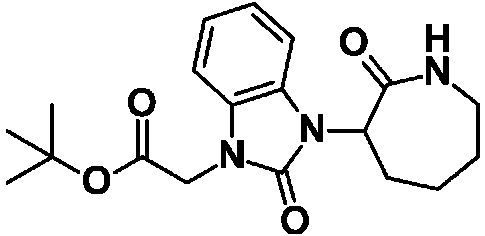

3-(2-Aminoanilino)azepan-2-one (505 mg) was dissolved in N,N-dimethylformamide (5 mL), and potassium carbonate (480 mg) and tert-butyl 2-bromoacetate (472 mg) were added, followed by stirring at room temperature for 16 hours. Water was added to the reaction mixture, and the mixture was extracted with ethyl acetate. The organic layer was washed with water and saturated brine, and dried over anhydrous sodium sulfate, and then the solvent was evaporated under reduced pressure. The residue was dissolved in tetrahydrofuran (10 mL), and N,N-diisopropylethylamine (2.0 mL) and triphosgene (342 mg) were added. The mixture was stirred at room temperature for 3 hours. Water was added to the reaction mixture, and the mixture was extracted with ethyl acetate. The organic layer was washed with water and saturated brine, and dried over anhydrous sodium sulfate, and then the solvent was evaporated under reduced pressure. The residue was purified by silica gel column chromatography (hexane:ethyl acetate = 70:30 to 0:100) to obtain the title compound (632 mg) as a yellow powder. MS (ESI) m/z: 360.1 (M+H)⁺

### Example 138

### tert-Butyl 2-[3-(2,7-dioxoazepan-3-yl)-2-oxo-benzimidazol-1-yl]acetate

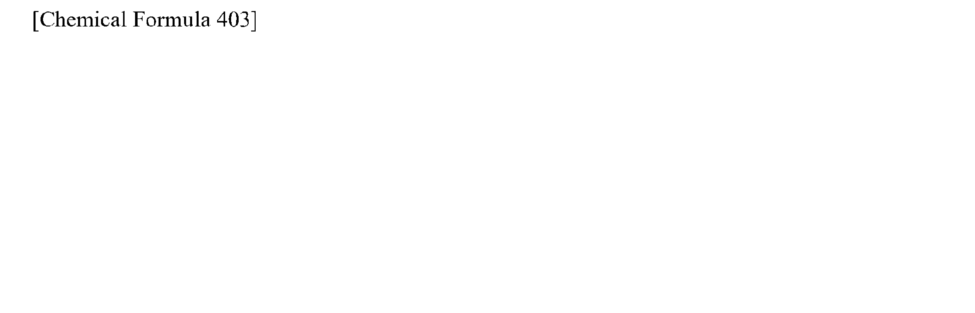

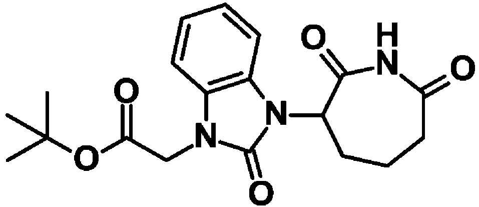

Using tert-butyl 2-[2-oxo-3-(2-oxoazepan-3-yl)benzimidazol-1-yl]acetate instead of tert-butyl 2-[2-fluoro-3-[(2-oxoazepan-3-yl)carbamoyl]phenoxy]acetate in Example 67, the same reaction and processing were performed to obtain the title compound (213 mg) as a colorless powder. MS (ESI) m/z: 318.1 (M-tBu+H)⁺

### Example and Reference Example 139

### Reference Example 139

### (Reference Example 139-1)

### 3-(2-Amino-4-bromoanilino)azepan-2-one

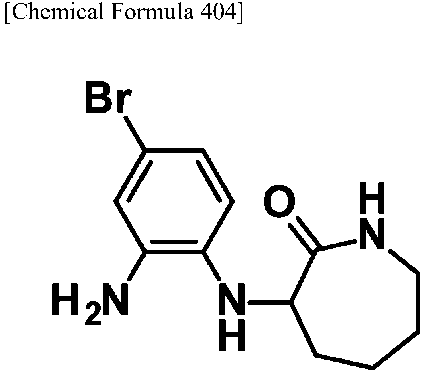

Using 4-bromo-1-fluoro-2-nitrobenzene instead of 1-fluoro-2-nitrobenzene in (Reference Example 138-1), the same reactions and processing as in (Reference Example 138-1 and (Reference Example 138-2) were performed to obtain the title compound (1.79 g) as a light brown powder. MS (ESI) m/z: 298.0/300.0 (M+H)⁺

### (Reference Example 139-2)

### 5-Bromo-3-methyl-1-(2-oxoazepan-3-yl)benzimidazol-2-one

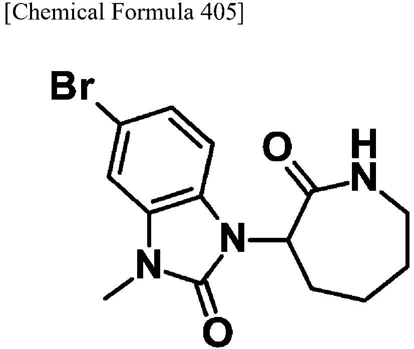

Using 3-(2-amino-4-bromoanilino)azepan-2-one instead of 3-(2-aminoanilino)azepan-2-one and methyl iodide instead of tert-butyl 2-bromoacetate in (Reference Example 138-3), the same reaction and processing were performed to obtain the title compound (859 mg) as a light brown powder. MS (ESI) m/z: 338.0/340.0 (M+H)⁺

### Example 139

### 3-(5-Bromo-3-methyl-2-oxobenzimidazol-1-yl)azepane-2,7-dione

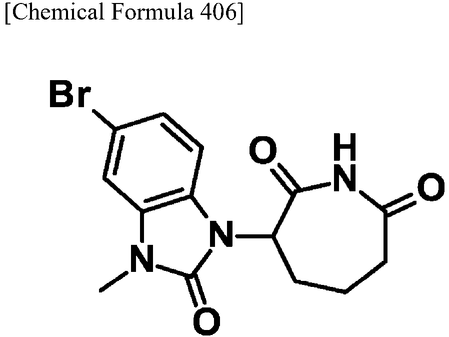

Using 5-bromo-3-methyl-1-(2-oxoazepan-3-yl)benzimidazol-2-one instead of tert-butyl 2-[2-fluoro-3-[(2-oxoazepan-3-yl)carbamoyl]phenoxy]acetate in Example 67, the same reaction and processing were performed to obtain the title compound (120 mg) as a colorless powder. MS (ESI) m/z: 352.0/354.0 (M+H)⁺

### Example 140

### tert-Butyl (E)-3-[1-(2,7-oxazepan-3-yl)-3-methyl-2-oxo-benzimidazol-5-yl]-2-propenoate

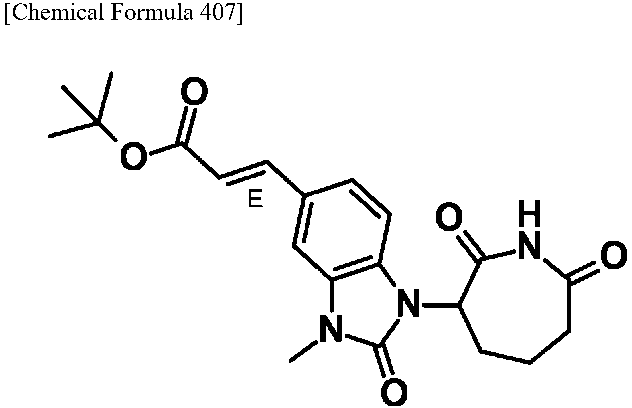

Using 5-bromo-3-methyl-1-(2-oxoazepan-3-yl)benzimidazol-2-one instead of 3-(3-iodoindazol-1-yl)azepane-2,7-dione in (Example 136), the same reaction and processing were performed to obtain the title compound (40 mg) as a colorless powder. MS (ESI) m/z: 400.2 (M+H)⁺

### Example 141

### tert-Butyl 3-[1-(2,7-oxazepan-3-yl)-3-methyl-2-oxo-benzimidazol-5-yl]propanoate

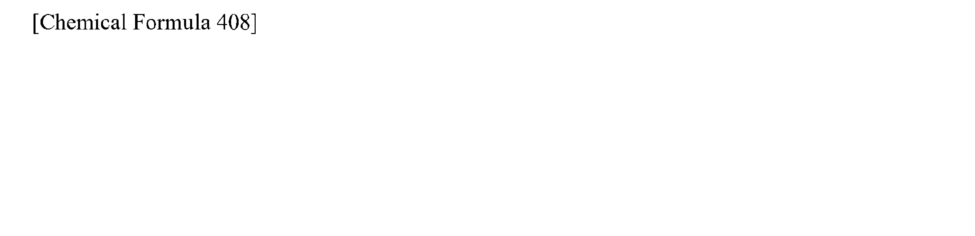

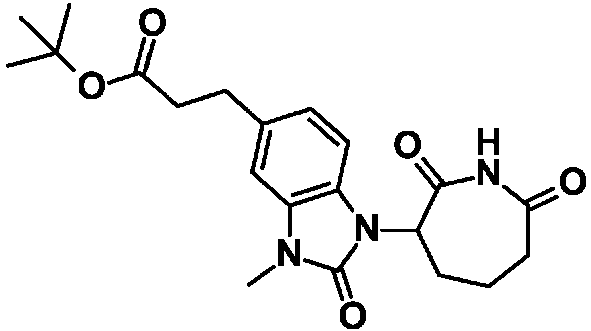

Using tert-butyl (E)-3-[1-(2,7-oxazepan-3-yl)-3-methyl-2-oxo-benzimidazol-5-yl]-2-propenoate instead of 3-(3-nitrophenyl)azepane-2,7-dione in Example 70, the same reaction and processing were performed to obtain the title compound (27 mg) as a colorless powder. MS (ESI) m/z: 402.2 (M+H)⁺

### Example 142

### N-[3-Carbamoyl-1-[4-[2-[2-[2-[[2-(6,8-dioxo-2,7-diazaspiro[4.6]undecan-2-yl)pyridine-4-carbonyl]amino]ethoxy]ethoxy]ethylcarbamoyl]phenyl]-pyrazol-4-yl]-2-[2-(cyclopropylmethylamino)-4-pyridyl]oxazole-4-carboxamide

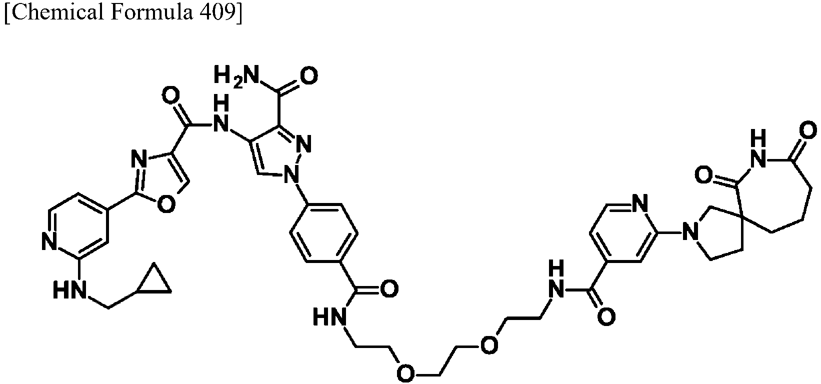

tert-Butyl 2-(6,8-dioxo-2,7-diazaspiro[4.6]undecan-2-yl)pyridine-4-carboxylate (20 mg) was dissolved in dichloromethane (1 mL), and trifluoroacetic acid (1 mL) was added, followed by stirring at room temperature for 3 hours. The reaction mixture was concentrated, and dried under reduced pressure. The residue was dissolved in N,N-dimethylformamide (1 mL), and N-[1-[4-[2-[2-(2-aminoethoxy)ethoxy]ethylcarbamoyl]phenyl]-3-carbamoyl-pyrazol-4-yl]-2-[2-(cyclopropylmethylamino)-4-pyridyl]oxazole-4-carboxamide; trifluoroacetate (40 mg), N,N-diisopropylethylamine (25 mg), and O-(7-azabenzotriazol-1-yl)-N,N,N',N'-tetramethyluronium hexafluorophosphate (27 mg) were added, followed by stirring at room temperature for 0.5 hours. Water was added to the reaction mixture, and the mixture was extracted with chloroform. The organic layer was dried over anhydrous sodium sulfate, and the solvent was evaporated under reduced pressure. The residue was purified by silica gel column chromatography (chloroform:methanol = 100:0 to 90:10). The residue was further purified by silica gel column chromatography (chloroform:methanol = 100:0 to 90:10) to obtain the title compound (11 mg) as a pale yellow powder. MS (ESI) m/z: 903.4 (M+H)⁺

### Example 143

### N-[3-Carbamoyl-1-[4-[2-[2-[2-[[6-(4,7,9-trioxo-3,8-diazaspiro[5.6]dodecan-3-yl)pyridine-3-carbonyl]amino]ethoxy]ethoxy]ethylcarbamoyl]phenyl]pyrazol-4-yl]-2-[2-(cyclopropylmethylamino)-4-pyridyl]oxazole-4-carboxamide

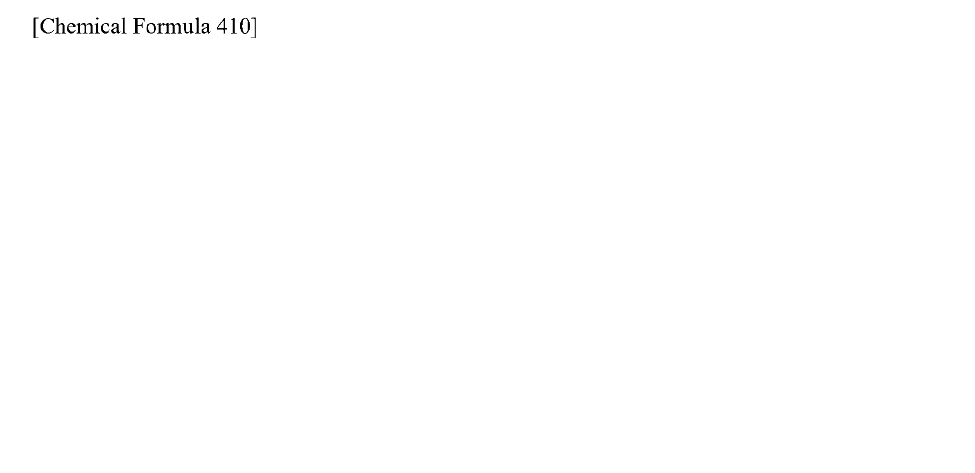

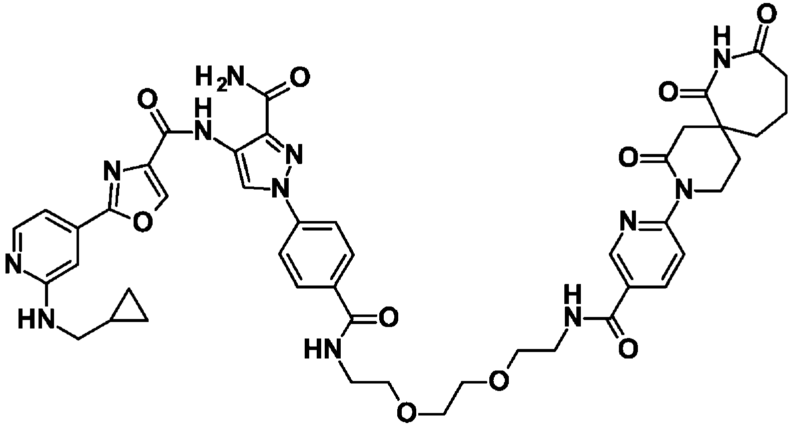

Using tert-butyl 6-(4,7,9-trioxo-3,8-diazaspiro[5.6]dodecan-3-yl)pyridine-3-carboxylate instead of tert-butyl 2-(6,8-dioxo-2,7-diazaspiro[4.6]undecan-2-yl)pyridine-4-carboxylate in Example 142, the same reaction and processing were performed to obtain the title compound (40 mg) as a white powder. MS (ESI) m/z: 931.6 (M+H)⁺

### Example 144

### N-[3-Carbamoyl-1-[4-[2-[2-[2-[ [2-(4,7,9-trioxo-3,8-diazaspiro[5.6]dodecan-3-yl)acetyl]amino]ethoxy]ethoxy]ethylcarbamoyl]phenyl]pyrazol-4-yl]-2-[2-(cyclopropylmethylamino)-4-pyridyl]oxazole-4-carboxamide

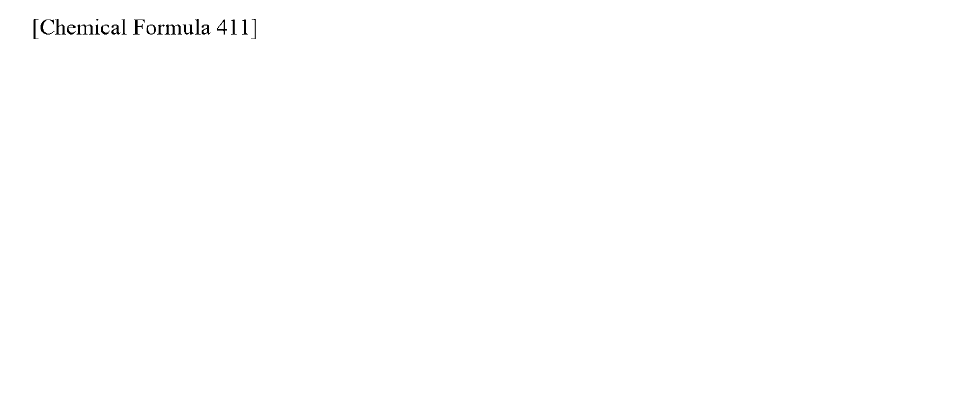

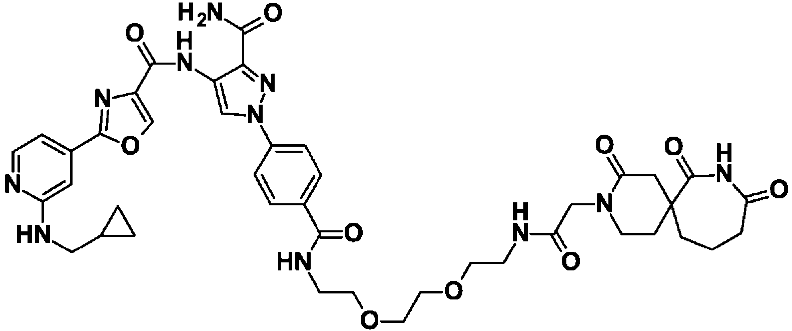

Using tert-butyl 2-(4,7,9-trioxo-3,8-diazaspiro[5.6]dodecan-3-yl)acetate instead of tert-butyl 2-(6,8-dioxo-2,7-diazaspiro[4.6]undecan-2-yl)pyridine-4-carboxylate in Example 142, the same reaction and processing were performed to obtain the title compound (28 mg) as a colorless powder. MS (ESI) m/z: 868.5 (M+H)⁺

The corresponding starting compounds were processed in the same manner as in Reference Example and Example 1 to obtain the compounds listed in the table below.

**[Table 9]**

| Example | Structural formula | Physical properties etc. |
|---|---|---|
| 145 | 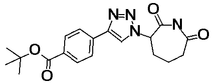 tert-Butyl 4-[1-(2,7-dioxoazepan-3-yl)triazol-4-yl]benzoate | White solid |
| | | MS (ESI) m/z: 371.3 [M+H]⁺ |
| 146 | 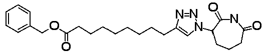 Benzyl 9-[1-(2,7-dioxoazepan-3-yl)triazol-4-yl]nonanoate | Pale yellow solid |
| | | MS (ESI) m/z: 441. 1 [M+H]⁺ |
| 147 | 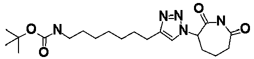 tert-Butyl N-[7-[1-(2,7-dioxoazepan-3-yl)triazol-4-yl]heptyl]carbamate | Pale yellow solid |
| | | MS (ESI) m/z: 408.1 [M+H]⁺ |
| 148 | 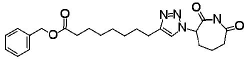 | White solid |
| | | MS (ESI) m/z: 427.1 [M+H]⁺ |
| | Benzyl 8-[1-(2,7-dioxoazepan-3-yl)triazol-4-yl]octanoate | |
| 149 | 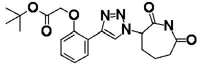 tert-Butyl 2-[2-[1-(2,7-dioxoazepan-3-yl)triazol-4-yl]phenoxy]acetate | White solid |
| | | MS (ESI) m/z: 401.0 [M+H]⁺ |

### Reference Example 148

### Benzyl dec-9-ynoate (Reference Example Compound 148)

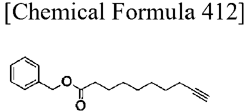

To a mixture of 9-decynoic acid (505 mg), potassium carbonate (822 mg), and N,N-dimethylformamide (10 mL), benzyl bromide (0.355 mL) was added, and the mixture was stirred at room temperature for 46 hours. Water was added to the reaction mixture, and the mixture was extracted with diethyl ether. The organic layer was dried over sodium sulfate, and then the solvent was evaporated under reduced pressure. The residue was purified by silica gel column chromatography (ethyl acetate:hexane = 0:99 to 7:93) to obtain the title compound (632 mg) as a colorless liquid. MS (ESI) m/z: 259.2 (M+H)⁺.

### Reference Example 149

### tert-Butyl 2-(2-ethynylphenoxy)acetate (Reference Example Compound 149)

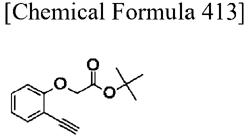

A methanol (5 mL) solution of tert-butyl 2-(2-formylphenoxy)acetate (300 mg) was cooled to 0 °C, and potassium carbonate (351 mg) and dimethyl (1-diazo-2-oxopropyl)phosphonate (0.210 mL) were added, followed by stirring at room temperature for 5 hours. The mixture was filtered through Celite using ethyl acetate, and the organic layer was concentrated under reduced pressure. The residue was purified by silica gel column chromatography (ethyl acetate:hexane = 1:99 to 35:65) to obtain the title compound (54.8 mg) as a colorless viscous material. MS (ESI) m/z: 233.1 (M+H)⁺.

### Reference Example and Example 150

### (150-1) 3-[4-(4-Iodophenyl)triazol-1-yl]azepan-2-one (Reference Example Compound 150-1)

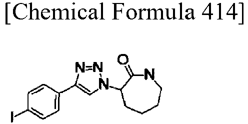

Using 1-ethynyl-4-iodobenzene instead of tert-butyl 2-propynylcarbamate in Reference Example 1 and Example 1, the same reaction and processing as in (1-1) were performed to obtain the title compound as a white solid. MS (ESI) m/z: 382.9 (M+H)⁺

### (150-2) tert-Butyl N-[6-[4-[1-(2-oxoazepan-3-yl)triazol-4-yl]phenyl]hexa-5-ynyl]carbamate (Reference Example Compound 150-2)

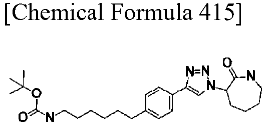

Reference Example Compound 150-1 (110 mg), tert-butyl N-hexa-5-ynylcarbamate (68.1 mg), bis(triphenylphosphine)palladium(II) dichloride (20.2 mg), copper(I) iodide (5.5 mg), and N,N-diisopropylethylamine (0.149 mL) were stirred in tetrahydrofuran (5 mL) at room temperature for 8 hours and at 50 °C for 7 hours. Water was added to the reaction mixture, followed by stirring, and the mixture was extracted twice with ethyl acetate. The organic layer was washed twice with water and then concentrated under reduced pressure. The residue was purified by silica gel column chromatography (ethyl acetate:hexane = 50:50 to 100:0) and silica gel column chromatography (methanol:chloroform = 0:100 to 5:95). To a tetrahydrofuran (2 mL) and ethanol (2 mL) solution of the obtained solid, palladium on carbon (20.0 mg) was added, and the atmosphere was replaced with hydrogen, followed by stirring at room temperature for 8 hours. The mixture was filtered through Celite using chloroform, and the filtrate was concentrated under reduced pressure. The residue was purified by NH silica gel column chromatography (methanol:chloroform = 0:100 to 5:95) to obtain the title compound (48.7 mg) as a white solid. MS (ESI) m/z: 456.1 (M+H)⁺.

### (150-3) tert-Butyl N-[6-[4-[1-(2,7-dioxoazepan-3-yl)triazol-4-yl]phenyl]hexyl]carbamate (Example Compound 150)

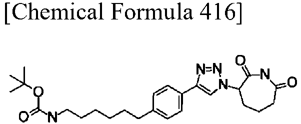

Using Reference Example Compound 150-2 instead of Reference Example Compound 1 in Reference Example and Example 1, the same reaction and processing as in (1-2) were performed to obtain the title compound as a white solid. MS (ESI) m/z: 470.1 (M+H)⁺.

### Example 151

### 2-(1-Methylindole-4-carbonyl)-2,7-diazaspiro[4.6]undecane-6,8-dione (Example Compound 151)

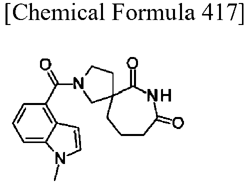

2,7-Diazaspiro[4.6]undecan-6-one trifluoroacetate (21 mg), 1-methylindole-4-carboxylic acid (12 mg), O-(7-azabenzotriazol-1-yl)-N,N,N',N'-tetramethyluronium hexafluorophosphate (34 mg), and N,N-diisopropylethylamine (60 µL) were dissolved in N,N-dimethylformamide (0.5 mL) and stirred at room temperature for 1 hour. Water was added to the reaction mixture, and after extraction with ethyl acetate, washing with saturated brine was performed, followed by drying over anhydrous sodium sulfate, and then the solvent was evaporated under reduced pressure. The residue was purified by silica gel column chromatography (chloroform:methanol = 100:0 to 95:5) to obtain the title compound (18 mg) as a colorless powder. MS (ESI) m/z: 340.1 (M+H)⁺

The corresponding starting compounds were processed in the same manner as in Example 151 to obtain the compounds listed in the table below.

**[Table 10]**

| Example | Structural formula | Physical properties etc. |
|---|---|---|
| 152 | 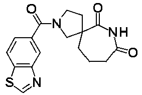 2-(1,3-Benzothiazole-5-carbonyl)-2,7-diazaspiro[4.6]undecane-6,8-dione | Colorless powder |
| | | MS (ESI) m/z: 344.1 (M+H)⁺ |
| 153 | 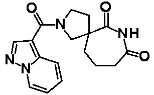 2-(Pyrazolo[1,5-a]pyridine-3-carbonyl)-2,7-diazaspiro[4.6]undecane-6,8-dione | Colorless powder |
| | | MS (ESI) m/z: 327.1 (M+H)⁺ |
| 154 | 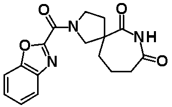 2-(1,3-Benzoxazole-2-carbonyl)-2,7-diazaspiro [4.6]undecane-6,8-dione | Colorless powder |
| | | MS (ESI) m/z: 328.1 (M+H)⁺ |
| 155 | 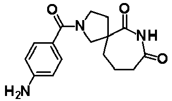 2-(4-Aminobenzoyl)-2,7-diazaspiro[4.6]undecane-6,8-dione | Colorless powder |
| | | MS (ESI) m/z: 302.1 (M+H)⁺ |
| 156 | 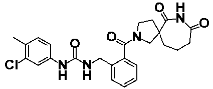 1-(3-Chloro-4-methylphenyl)-3-[[2-(6,8-dioxo-2,7-diazaspiro[4.6]undecane-2-carbonyl)phenyl]methyl]urea | Colorless powder |
| | | MS (ESI) m/z: 483.3/485.3 (M+H)⁺ |
| 157 | 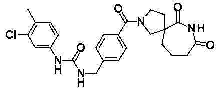 1-(3-Chloro-4-methylphenyl)-3-[[4-(6,8-dioxo-2,7-diazaspiro[4.6]undecane-2-carbonyl)phenyl]methyl]urea | Colorless powder |
| | | MS (ESI) m/z: 483.2/ 485.2 (M+H)⁺ |
| 158 | 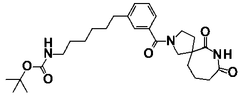 tert-Butyl N-[6-[3-(6,8-dioxo-2,7-diazaspiro[4.6]undecane-2-carbonyl)phenyl]hexyl]carbamate | Colorless semisolid |
| | | MS (ESI) m/z: 486.2 [M+H]⁺ |
| 159 | 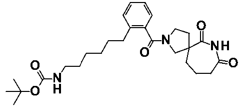 tert-Butyl N-[6-[2-(6,8-dioxo-2,7-diazaspiro[4.6]undecane-2-carbonyl)phenyl]hexyl]carbamate | Colorless solid |
| | | MS (ESI) m/z: 486.1 [M+H]⁺ |
| 160 | 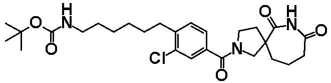 tert-Butyl N-[6-[2-chloro-4-(6, 8-dioxo-2,7-diazaspiro[4.6]undecane-2-carbonyl)phenyl]hexyl]carbamate | Colorless solid |
| | | MS (ESI) m/z: 420.3/422.3 [M-Boc+H]⁺ |
| 161 | 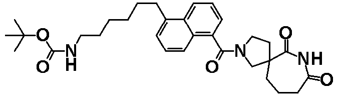 tert-Butyl N-[6-[5-(6,8-dioxo-2,7-diazaspiro[4.6]undecane-2-carbonyl)naphthalen-1-yl]hexyl]carbamate | Colorless solid |
| | | MS (ESI) m/z: 436.3 [M-Boc+H]⁺ |

### Reference Example 158

### (158-1) Methyl 3-[6-(tert-butoxycarbonylamino)hexa-1-ynyl]benzoate (Reference Example Compound 158-1)

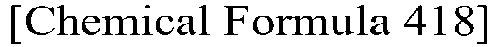

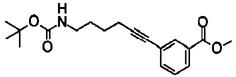

A mixture of methyl 3-iodobenzoate (1.99 g), tert-butyl N-hexa-5-ynylcarbamate (1 g), bis(triphenylphosphine)palladium(II) dichloride (356 mg), copper(I) iodide (193 mg), and triethylamine (705 µL) in tetrahydrofuran (30 mL) was stirred under a nitrogen atmosphere at 60 °C for 14 hours. The mixture was concentrated, and the residue was purified by silica gel column chromatography (petroleum ether/ethyl acetate = 100/0 to 7/1) to obtain the title compound (1.1 g) as a brown oil. MS (ESI) m/z: 354.1 [M+Na]⁺.

### (158-2) Methyl 3-[6-(tert-butoxycarbonylamino)hexyl]benzoate (Reference Example Compound 158-2)

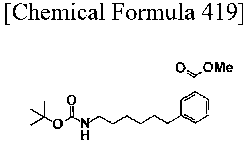

To a methanol (20 mL) solution of methyl 3-[6-(tert-butoxycarbonylamino)hexa-1-ynyl]benzoate (1.1 g), palladium on carbon (500 mg) was added, and the mixture was stirred under a hydrogen atmosphere at atmospheric pressure and room temperature for 16 hours. The reaction mixture was purged with nitrogen, followed by filtration, and the filtrate was concentrated. The residue was dissolved in methanol (20 mL), palladium on carbon (300 mg) was added, and the mixture was stirred under a hydrogen atmosphere at atmospheric pressure and room temperature for 16 hours. The reaction mixture was purged with nitrogen, followed by filtration, and the filtrate was concentrated to obtain the title compound (1.05 g) as a yellow oil.

### (158-3) 3-[6-(tert-Butoxycarbonylamino)hexyl]benzoic acid (Reference Example Compound 158-3)

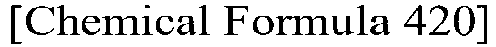

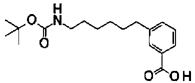

To a solution of methyl 3-[6-(tert-butoxycarbonylamino)hexyl]benzoate (910 mg) in tetrahydrofuran (6.5 mL) and methanol (6.5 mL), 1M aqueous sodium hydroxide (8.14 mL) was added, and the mixture was stirred at room temperature for 16 hours. The organic solvent was concentrated under reduced pressure, hydrochloric acid was added to the residue to adjust pH to 5, and the mixture was extracted with methylene chloride, dried over sodium sulfate, filtered, and concentrated to obtain the title compound (790 mg) as a colorless solid. MS (ESI) m/z: 344.1 [M+Na]⁺

### Reference Example 159

### 2-[6-(tert-Butoxycarbonylamino)hexyl]benzoic acid (Reference Example Compound 159)

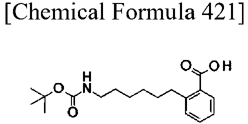

Using methyl 2-iodobenzoate instead of methyl 3-iodobenzoate in Reference Example 158, the same reaction and processing were performed to obtain the title compound as a colorless solid. MS (ESI) m/z: 322.0 (M+H)⁺

### Reference Example 160

### 4-[6-(tert-Butoxycarbonylamino)hexyl]-3-chlorobenzoic acid (Reference Example Compound 160)

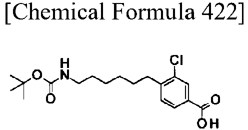

Using methyl 3-chloro-4-iodobenzoate instead of methyl 3-iodobenzoate in Reference Example 158, the same reaction and processing were performed to obtain the title compound as a colorless solid. MS (ESI) m/z: 378.1 [M+Na]⁺

### Reference Example 161

### 5-[6-(tert-Butoxycarbonylamino)hexyl]naphthalene-1-carboxylic acid (Reference Example Compound 161)

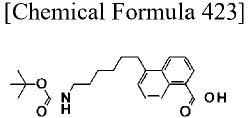

Using methyl 5-bromonaphthalene-1-carboxylate instead of methyl 3-iodobenzoate in Reference Example 158, the same reaction and processing were performed to obtain the title compound as a yellow solid. MS (ESI) m/z: 372.1 (M+H)⁺

### Reference Example and Example 162

### (162-1) Methyl 1-[6-(tert-butoxycarbonylamino)hexyl]indole-4-carboxylate (Reference Example Compound 162-1)

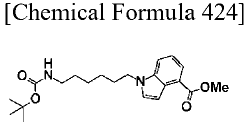

To a DMF (5 mL) solution of methyl 1H-indole-4-carboxylate (500 mg), sodium hydride (60% oil suspension, 125.6 mg) was added at 0 °C, and the mixture was stirred at 0 °C for 30 minutes. tert-Butyl N-(6-bromohexyl)carbamate (879.7 mg) was added, and the mixture was stirred at room temperature for 16 hours. Water was added to the reaction mixture, followed by extraction with ethyl acetate, washing with saturated brine, drying over sodium sulfate, filtration, and concentration to obtain the title compound (1.3 g) as a brown oil. MS (ESI) m/z: 397.1 [M+Na]⁺

### (Reference Example 162-2) 1-[6-(tert-Butoxycarbonylamino)hexyl]indole-4-carboxylic acid (Reference Example Compound 162-2)

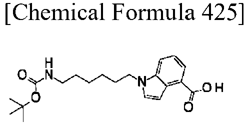

To a methanol (7 mL) solution of methyl 1-[6-(tert-butoxycarbonylamino)hexyl]indole-4-carboxylate (1.3 g), a water (3 mL) solution of lithium hydroxide monohydrate (437.0 mg) was added, and the mixture was stirred at room temperature for 24 hours. 0.5M hydrochloric acid was added to the reaction mixture to adjust pH to 4 to 5, and the mixture was extracted with ethyl acetate. The organic layer was washed with saturated brine, dried over sodium sulfate, filtered, and concentrated to obtain the title compound (800 mg) as a brown solid. MS (ESI) m/z: 261.1 (M-Boc+H)⁺.

### Example 162

### tert-Butyl N-[6-[4-(6,8-dioxo-2,7-diazaspiro[4.6]undecane-2-carbonyl)indol-1-yl]hexyl]carbamate (Example Compound 162)

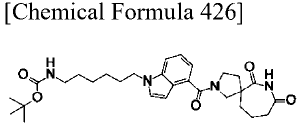

Using 1-[6-(tert-butoxycarbonylamino)hexyl]indole-4-carboxylic acid instead of 1-methylindole-4-carboxylic acid in Example 151, the same reaction and processing were performed to obtain the title compound as a colorless solid. MS (ESI) m/z: 525.2 (M+H)⁺.

The corresponding starting compounds were processed in the same manner as in Example 162 to obtain the compounds listed in the table below.

**[Table 11]**

| Example | Structural formula | Physical properties etc. |
|---|---|---|
| 163 | 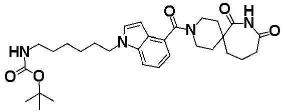 tert-Butyl N-[6-[4-(7,9-dioxo-3,8-diazaspiro[5.6]undecane-2-carbonyl)indol-1-yl]hexyl]carbamate | Colorless viscous material |
| | | MS (ESI) m/z: 539.3 (M+H)⁺ |
| 164 | 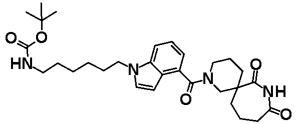 tert-Butyl N-[6-[4-(7,9-dioxo-2,8-diazaspiro[5.6]undecane-2-carbonyl) indol-1-yl]hexyl]carbamate | Colorless viscous material |
| | | MS (ESI) m/z: 539.3 (M+H)⁺ |

### Reference Example and Example 165

### (165-1) (Ir,4r)-Methyl 4-(3-bromoanilino)-4-cyano-cyclohexanecarboxylate (Reference Example Compound 165-1)

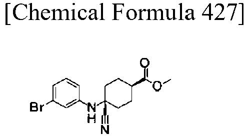

To a methanol (250 mL) solution of methyl 4-oxocyclohexanecarboxylate (9.0 g) and 3-bromoaniline (10.71 g), trimethylsilyl cyanide (28.01 g) was added, and the mixture was stirred under a nitrogen atmosphere at room temperature for 18 hours. The reaction mixture was concentrated, and the resulting residue was purified by silica gel column chromatography (ethyl acetate/petroleum ether = 0/1 to 1/3) to obtain the title compound (7.9 g) as a pale pink solid and its positional isomer, methyl (1s,4s)-4-(3-bromoanilino)-4-cyano-cyclohexanecarboxylate (7.28 g), as a pale red solid. MS (ESI) m/z: 338.9/340.9 (M+H)⁺.

### (165-2) Methyl (1r,4r)-4-(3-bromoanilino)-4-carbamoyl-cyclohexanecarboxylate (Reference Example Compound 165-2)

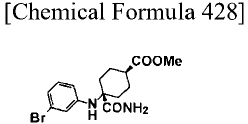

To a toluene (250 mL) solution of methyl (1r,4r)-4-(3-bromoanilino)-4-cyano-cyclohexanecarboxylate (7.89 g) and (1E)-acetaldehyde oxime (4.28 g), indium(III) chloride (258.75 mg) was added, and the mixture was stirred under a nitrogen atmosphere at 100 °C for 3 hours. The reaction mixture was concentrated to obtain the title compound (8.3 g) as a brown oil. MS (ESI) m/z: 356.9/358.9 (M+H)⁺

### (165-3) 1-(3-Bromoanilino)-3-azabicyclo[3.2.2]nonane-2,4-dione (Example Compound 165)

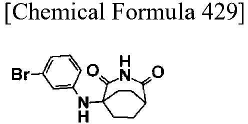

To a tetrahydrofuran (45 mL) solution of methyl (1r,4r)-4-(3-bromoanilino)-4-carbamoyl-cyclohexanecarboxylate (2.08 g), potassium tert-butoxide (1M/THF solution, 23.42 mL) was added at 0 °C, and the mixture was stirred under a nitrogen atmosphere at 0 °C for 1 hour. The reaction mixture was quenched with 1M aqueous citric acid and extracted three times with ethyl acetate. The organic layer was washed with saturated brine, and then dried over sodium sulfate, filtered, and concentrated. The crude product was triturated with methylene chloride to obtain the title compound (330 mg) as a pale yellow solid. MS (ESI) m/z: 323.0/325.0 (M+H)⁺.

### Example 166

### 1-(4-Bromoanilino)-3-azabicyclo[3.2.2]nonane-2,4-dione (Example Compound 166)

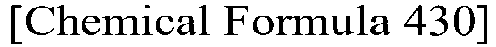

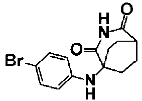

Using 4-bromoaniline instead of 3-bromoaniline, the same processing as in Example 165 was performed to obtain the title compound. MS (ESI) m/z: 323.0/325.0 (M+H)⁺

### Reference Example and Example 167

### (167-1) 4-(4-Bromophenyl)-4-cyanocyclohexane-1-carboxylic acid (Reference Example Compound 167-1)

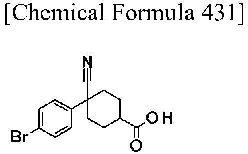

A mixture of 50% aqueous potassium hydroxide (2.0 g), benzyltriethylammonium chloride (28.7 mg), and tetrahydrofuran (3 mL) was cooled to 0 °C, and a tetrahydrofuran (3 mL) solution of 1-(4-bromophenyl)-4-oxocyclohexane-1-carbonitrile (700 mg) and 2-chloroacetonitrile (209 mg) was slowly added, followed by stirring at the same temperature for 1 hour. Water was added to the reaction mixture, and the mixture was stirred, and extracted three times with ethyl acetate, and the organic layer was concentrated under reduced pressure. The residue, lithium bromide (328 mg), and water (68 µL) were stirred in N,N-dimethylformamide (2 mL) and acetonitrile (2 mL) at 90 °C for 8 hours. The temperature was lowered to 60°C, N,N-dimethylformamide (2 mL) and 4M aqueous lithium hydroxide (1.26 mL) were added, and the mixture was stirred at the same temperature for 1 hour. Aqueous sodium hypochlorite was added, and stirred for 2 hours, and then the mixture was acidified with 1M hydrochloric acid. The mixture was diluted with water, and the resulting solid was filtered. The residue was purified by silica gel column chromatography (methanol:chloroform = 0:100 to 5:95) to obtain the title compound (642 mg) as a beige solid. MS (ESI) m/z: 306.0/308.0 (M-H)⁻.

### (167-2) Methyl 4-(4-bromophenyl)-4-carbamoylcyclohexane-1-carboxylate (Reference Example Compound 167-2)

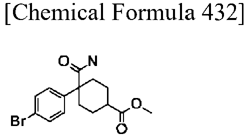

A tetrahydrofuran (2 mL) and methanol (2 mL) solution of Reference Example Compound 167-1 (642 mg) was cooled to 0 °C, and trimethylsilyldiazomethane (about 10% hexane solution, 5.2 mL) was slowly added dropwise, followed by stirring for 3 hours while warming to room temperature. The reaction mixture was concentrated under reduced pressure, and the residue was purified by silica gel column chromatography (ethyl acetate:hexane = 1:99 to 50:50) to obtain a white solid (458 mg). This solid, acetaldoxime (0.260 mL), and indium(III) chloride (15.7 mg) were stirred in toluene (5 mL) at 100 °C for 7 hours. The reaction mixture was concentrated under reduced pressure, and the residue was purified by silica gel column chromatography (ethyl acetate:hexane = 30:70 to 70:30) to obtain the title compound (215 mg) as a white solid. MS (ESI) m/z: 339.9/341.9 (M+H)⁺.

### (167-3) 1-(4-Bromophenyl)-3-azabicyclo[3.2.2]nonane-2,4-dione (Example Compound 167)

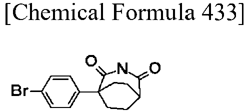

A tetrahydrofuran (5 mL) solution of Reference Example Compound 167-2 (102 mg) was cooled to 0 °C, and potassium tert-butoxide (1M, 0.6 mL) was added, followed by stirring for 6 hours while warming to room temperature. Water was added to the reaction mixture, followed by stirring, and extraction with ethyl acetate. The organic layer was washed twice with water and then concentrated under reduced pressure. The residue was purified by silica gel column chromatography (methanol:chloroform = 0:100 to 5:95) and re-purified by silica gel column chromatography (ethyl acetate:hexane = 10:90 to 70:30) to obtain the title compound (6.6 mg) as a white solid. MS (ESI) m/z: 308.1/310.1 (M+H)⁺.

### Example 168

### (168-1) tert-Butyl 4-[2-(6,8-dioxo-2,7-diazaspiro[4.6]undecan-2-yl)pyrimidin-5-yl]-3,6-dihydro-2H-pyridine-1-carboxylate (Example Compound 168-1)

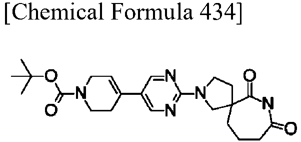

Example Compound 127 (49.7 mg), 1-BOC-1,2,3,6-tetrahydro-4-(4,4,5,5-tetramethyl-1,3,2-dioxaborolan-2-yl)pyridine (54.4 mg), [1,1'-bis(di-tert-butylphosphino)ferrocene]palladium(II) dichloride (9.6 mg), and cesium fluoride (44.5 mg) were stirred in N,N-dimethylformamide (2 mL) and water (0.5 mL) at 80 °C for 7 hours. The reaction mixture was loaded onto an inject column and purified by silica gel column chromatography (methanol:chloroform = 0:100 to 10:90) to obtain the title compound (27.8 mg) as a pale yellow solid. MS (ESI) m/z: 442.0 (M+H)⁺.

### (168-2) tert-Butyl 4-[2-(6,8-dioxo-2,7-diazaspiro[4.6]undecan-2-yl)pyrimidin-5-yl]piperidine-1-carboxylate (Example Compound 168-2)

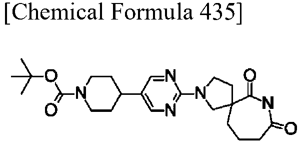

To a tetrahydrofuran (2 mL) and ethanol (2 mL) solution of Example Compound 168-1 (27.8 mg), palladium on carbon (10.0 mg) was added, and the mixture was stirred under a hydrogen atmosphere at room temperature for 7 hours. The mixture was filtered through Celite using chloroform, and the filtrate was concentrated under reduced pressure. The residue was purified by silica gel column chromatography (methanol:chloroform = 0:100 to 5:95) to obtain the title compound (22.9 mg) as a white solid. MS (ESI) m/z: 444.1 (M+H)⁺.

### Example 169

### tert-Butyl 4-[4-[(2,4-dioxo-3-azabicyclo[3.2.2]nonan-1-yl)amino]phenyl]piperidine-1-carboxylate (Example Compound 169)

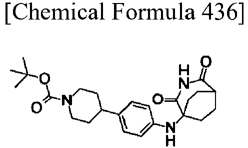

Using 1-(4-bromoanilino)-3-azabicyclo[3.2.2]nonane-2,4-dione (Example Compound 166) instead of Example Compound 127 in Example 168, the same reactions and processing as in (168-1) and (168-2) were performed to obtain the title compound as a white solid. MS (ESI) m/z: 428.1 (M+H)⁺.

### Example 170

### tert-Butyl 4-[3-[(2,4-dioxo-3-azabicyclo[3.2.2]nonan-1-yl)amino]phenyl]piperidine-1-carboxylate (Example Compound 170)

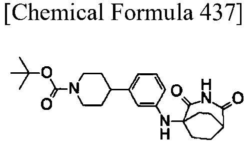

Using 1-(3-bromoanilino)-3-azabicyclo[3.2.2]nonane-2,4-dione (Example Compound 165) instead of Example Compound 127 in Example 168, the same reactions and processing as in (168-1) and (168-2) were performed to obtain the title compound as a white solid. MS (ESI) m/z: 426.3 [M-H]⁻.

### Example 171

### tert-Butyl 2-[4-[4-[(2,4-dioxo-3-azabicyclo[3.2.2]nonan-1-yl)amino]phenyl]piperidin-1-yl]acetate (Example Compound 171)

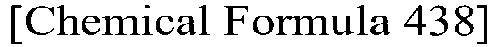

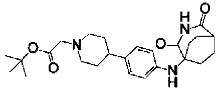

Using Example Compound 169 instead of tert-butyl 6,8-dioxo-2,7-diazaspiro[4.6]undecane-2-carboxylate in Example 100, the same reaction and processing were performed to obtain the title compound as a white solid. MS (ESI) m/z: 442.1 (M+H)⁺.

### Example 172

### tert-Butyl 2-[4-[3-[(2,4-dioxo-3-azabicyclo[3.2.2]nonan-1-yl)amino]phenyl]piperidin-1-yl]acetate (Example Compound 172)

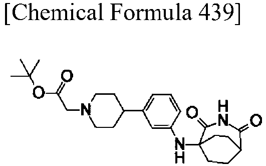

Using Example Compound 170 instead of tert-butyl 6,8-dioxo-2,7-diazaspiro[4.6]undecane-2-carboxylate in Example 100, the same reaction and processing were performed to obtain the title compound as a white solid. MS (ESI) m/z: 442.1 (M+H)⁺.

### Example and Reference Example 173

### (173-1) 2-(4-Bromophenyl)oxolane-2,5-dicarboxylic acid (Reference Example Compound 173-1)

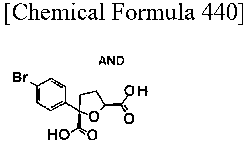

To a methanol (20 mL) solution of 2,3-dimethyl 1-(4-bromophenyl)-7-oxabicyclo[2.2.1]hepta-2,5-diene-2,3-dicarboxylate (2.09 g), platinum on carbon (2.40 g) was added, and the mixture was stirred under a hydrogen atmosphere at atmospheric pressure and room temperature for 2 hours. The reaction mixture was filtered, and the filtrate was concentrated under reduced pressure to obtain a brown viscous material (2.01 g). The obtained viscous material and sodium hydroxide (1.63 g) were mixed in tetrahydrofuran, and the mixture was stirred at room temperature under a nitrogen stream for 16 hours. Water (50 mL) was added, and 12M hydrochloric acid was added to adjust the pH to 2. The mixture was extracted with ethyl acetate, washed with saturated brine, dried over sodium sulfate, and the organic layer was concentrated under reduced pressure to obtain a yellow solid (1.92 g). A dichloromethane (20 mL) and methanol (20 mL) solution of this solid was cooled to -60 °C, and ozone was passed through the reaction mixture until it turned blue. Argon was bubbled through the reaction mixture until the blue color disappeared, dimethyl sulfide (454 mg) was added, and the mixture was stirred at - 10 °C for 1 hour and at room temperature for 1 hour. The reaction mixture was concentrated under reduced pressure, cooled with ice, and 40% sodium hydroxide (2.50 g) and 30% hydrogen peroxide (2.17 g) were added, followed by stirring at room temperature for 12 hours. The reaction mixture was cooled to 0 °C, and the pH was adjusted to 2 with 6M hydrochloric acid. The mixture was extracted with dichloromethane, the organic layer was washed with brine, dried over sodium sulfate, and concentrated under reduced pressure. The residue was purified by HPLC (Phenomenex Luna C18 (250*70 mm, 10 µm); mobile phase: [water (formic acid) - acetonitrile]; gradient: 22%-52% B over 23 min) to obtain the title compound (384 mg) as a yellow solid. MS (ESI) m/z: 268.9/270.9 [M-COOH]⁺.

### (173-2) Methyl 2-(4-bromophenyl)-5-carbamoyloxolane-2-carboxylate (Reference Example Compound 173-2)

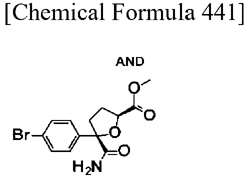

Reference Example Compound 173-1 (246 mg) was stirred in acetic anhydride (2 mL) at 120 °C for 7 hours. The reaction mixture was concentrated under reduced pressure, and 7M aqueous ammonia (3 mL) was added to the residue, followed by stirring at room temperature for 6 hours. The reaction mixture was concentrated under reduced pressure, and azeotropic distillation with toluene was performed twice. A tetrahydrofuran (2 mL) and methanol (2 mL) solution of the residue was cooled to 0 °C, trimethylsilyldiazomethane (about 10% hexane solution, 1.8 mL) was added, and the mixture was stirred for 5 hours while naturally warming to room temperature. The reaction mixture was concentrated under reduced pressure, and the residue was purified by silica gel column chromatography (methanol:chloroform = 0: 100 to 5:95) to obtain the title compound (143 mg) (mixture with positional isomer) as a white solid. MS (ESI) m/z: 328.1/330.1 (M+H)⁺.

### (173-3) 1-(4-Bromophenyl)-8-oxa-3-azabicyclo[3.2.1]octane-2,4-dione (Example Compound 173)

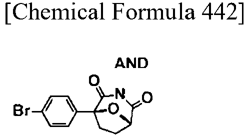

Using Reference Example Compound 173-2 instead of Reference Example Compound 167-2 in Example and Reference Example 167, the same reaction and processing as in (167-3) were performed to obtain the title compound as a white solid. MS (ESI) m/z: 296.1 / 298.1 (M+H)⁺.

### Example 174

### tert-Butyl 4-[4-(2,4-dioxo-8-oxa-3-azabicyclo[3.2.1]octan-1-yl)phenyl]piperidine-1-carboxylate (Example Compound 174)

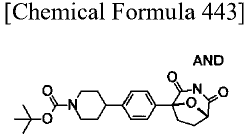

Using Example Compound 173 instead of Example Compound 127 in Example 168, the same reactions and processing as in (168-1) and (168-2) were performed to obtain the title compound as a white solid. MS (ESI) m/z: 345.0 (M-tBu+H)⁺.

### Example 175

### tert-Butyl 2-[4-[4-(2,4-dioxo-8-oxa-3-azabicyclo[3.2.1]octan-1-yl)phenyl]piperidin-1-yl]acetate (Example Compound 175)

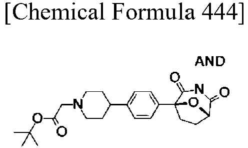

Using Example Compound 174 instead of tert-butyl 6,8-dioxo-2,7-diazaspiro[4.6]undecane-2-carboxylate in Example 100, the same reaction and processing were performed to obtain the title compound as a white solid. MS (ESI) m/z: 415.1 (M+H)⁺.

The corresponding starting compounds were processed in the same manner as in Example 32 to obtain the compounds listed in the table.

**[Table 12]**

| Example | Structural formula | Physical properties etc. |
|---|---|---|
| 176 | 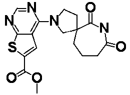 Methyl 4-(6,8-dioxo-2,7-diazaspiro[4.6]undecan-2-yl)thieno[2,3-d]pyrimidine-6-carboxylate | White solid |
| | | MS (ESI) m/z: 375.0 [M+H]⁺ |
| 177 | 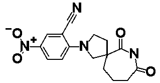 2-(6, 8-dioxo-2,7-diazaspiro[4.6]undecan-2-yl)-5-nitrobenzonitrile | Yellow viscous material |
| | | MS (ESI) m/z: 329.0 [M+H]⁺ |
| 178 | 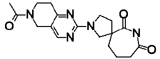 2-(6-Acetyl-7,8-dihydro-5H-pyrido[4,3-d]pyrimidin-2-yl)-2,7-diazaspiro[4.6]undecane-6,8-dione | Pale yellow solid |
| | | MS (ESI) m/z: 358.1 [M+H]⁺ |
| 179 | 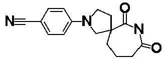 4-(6,8-Dioxo-2,7-diazaspiro[4.6]undecan-2-yl)benzonitrile | Pale yellow solid |
| | | MS (ESI) m/z: 284.0 [M+H]⁺ |
| 180 | 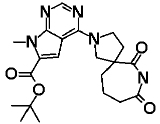 tert-Butyl 4-(6,8-dioxo-2,7-diazaspiro[4.6]undecan-2-yl)-7-methylpyrrolo[2,3-d]pyrimidine-6-carboxylate | Pale yellow solid |
| | | MS (ESI) m/z: 414.1 [M+H]⁺ |
| 181 | 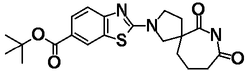 tert-Butyl 2-(6,8-dioxo-2,7-diazaspiro[4.6]undecane-2-yl)-1,3-benzothiazole-6-carboxylate | White solid |
| | | MS (ESI) m/z: 416.0 [M+H]⁺ |
| 182 | 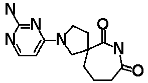 2-(2-Aminopyrimidin-4-yl)-2,7-diazaspiro[4.6]undecane-6,8-dione | White solid |
| | | MS (ESI) m/z: 276.2 [M+H]⁺ |
| 183 | 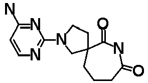 2-(4-Aminopyrimidin-2-yl)-2,7-diazaspiro[4.6]undecane-6,8-dione | White solid |
| | | MS (ESI) m/z: 276.2 [M+H]⁺ |
| 184 | 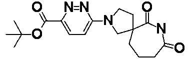 tert-Butyl 6-(6,8-dioxo-2,7-diazaspiro[4.6]undecan-2-yl)pyridazine-3-carboxylate | Pale yellow solid |
| | | MS (ESI) m/z: 361.0 [M+H]⁺ |
| 185 | 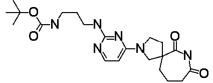 tert-Butyl 6-(6,8-dioxo-2,7-diazaspiro[4.6]undecan-2-yl)pyridazine-3-carboxylate | Pale yellow solid |
| | | MS (ESI) m/z: 433.0 [M+H]⁺ |
| 186 | 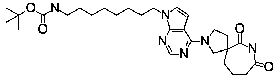 tert-Butyl N-[8-[4-(6,8-dioxo-2,7-diazaspiro[4.6]undecan-2-yl)pyrrolo[2,3-d]pyrimidin-7-yl]octyl]carbamate | Pale yellow solid |
| | | MS (ESI) m/z: 527.1 [M+H]⁺ |
| 187 | 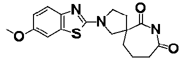 2-(6-Methoxy-1,3-benzothiazol-2-yl)-2,7-diazaspiro[4.6]undecane-6,8-dione | Pale yellow solid |
| | | MS (ESI) m/z: 346.3 [M+H]⁺ |
| 188 | 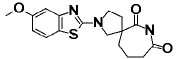 2-(5-Methoxy-1,3-benzothiazol-2-yl)-2,7-diazaspiro[4.6]undecane-6,8-dione | Pale yellow solid |
| | | MS (ESI) m/z: 346.3 [M+H]⁺ |
| 189 | 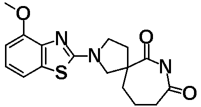 2-(4-Methoxy-1,3-benzothiazol-2-yl)-2,7-diazaspiro[4.6]undecane-6,8-dione | White solid |
| | | MS (ESI) m/z: 345.9 [M+H]⁺ |
| 190 | 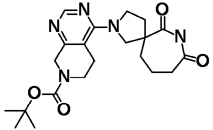 tert-Butyl 4-(6,8-dioxo-2,7-diazaspiro[4.6]undecan-2-yl)-6,8-dihydro-5H-pyrido[3,4-d]pyrimidine-7-carboxylate | White solid |
| | | MS (ESI) m/z: 416.0 [M+H]⁺ |

### Example and Reference Example 191

### (191-1) 2-(6-Oxo-2,7-diazaspiro[4.6]undecan-2-yl)benzonitrile (Reference Example Compound 191)

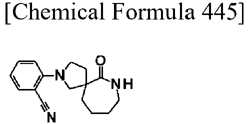

2,7-Diazaspiro[4.6]undecan-6-one (54.4 mg), 2-fluorobenzonitrile (43.1 mg), and potassium carbonate (89.4 mg) were stirred in dimethyl sulfoxide (3 mL) at 100 °C for 6 hours. Water and ethyl acetate were added to the reaction mixture for liquid-liquid separation, the organic layer was washed twice with water, and concentrated under reduced pressure. The residue was purified by silica gel column chromatography (methanol:chloroform = 0: 100 to 5:95) to obtain the title compound (55.2 mg) as a white solid. MS (ESI) m/z: 270.0 (M+H)⁺.

### (191-2) tert-Butyl 2-[2-[1-(2,7-dioxoazepan-3-yl)triazol-4-yl]phenoxy]acetate (Example Compound 191)

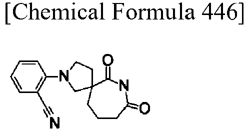

Using Reference Example Compound 191 instead of Reference Example Compound 1 in Reference Example and Example 1, the same reaction and processing as in (1-2) were performed to obtain the title compound as a yellow solid. MS (ESI) m/z: 284.0 (M+H)⁺.

The corresponding starting compounds were processed in the same manner as in Example 191 to obtain the compounds listed in the table below.

**[Table 13]**

| Example | Structural formula | Physical properties etc. |
|---|---|---|
| 192 | 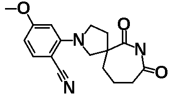 tert-Butyl 2-[2-[1-(2,7-dioxoazepan-3-yl)triazol-4-yl]phenoxy]acetate | Yellow solid |
| | | MS (ESI) m/z: 314.0 [M+H]⁺ |
| 193 | 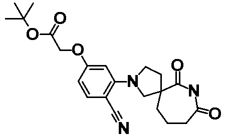 tert-Butyl 2-[4-cyano-3-(6,8-dioxo-2,7-diazaspiro[4.6]undecan-2-yl)phenoxy]acetate | Yellow solid |
| | | MS (ESI) m/z: 414.2 [M+H]⁺ |
| 194 | 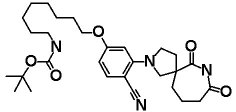 tert-Butyl 2-[4-cyano-3-(6,8-dioxo-2,7-diazaspiro[4.6]undecan-2-yl)phenoxy]acetate | Yellow solid |
| | | MS (ESI) m/z: 527.5 [M+H]⁺ |

### Reference Example 193

### tert-Butyl 2-(4-cyano-3-fluorophenoxy)acetate (Reference Example Compound 193)

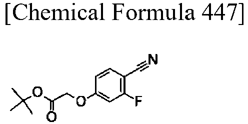

Using 2-fluoro-4-hydroxybenzonitrile instead of methyl 2-fluoro-3-hydroxybenzoate in Reference Example 67-1, the same reaction and processing were performed to obtain the title compound as a white solid. MS (ESI) m/z: 252.1 (M+H)⁺.

### Reference Example 194

### tert-Butyl N-[8-(4-cyano-3-fluorophenoxy)octyl]carbamate (Reference Example Compound 194)

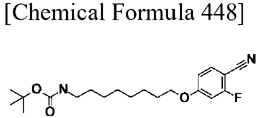

Using 2-fluoro-4-hydroxybenzonitrile instead of methyl 2-fluoro-3-hydroxybenzoate and tert-butyl N-(8-bromooctyl)carbamate instead of tert-butyl 2-bromoacetate in Reference Example 67-1, the same reaction and processing were performed to obtain the title compound as a white solid. MS (ESI) m/z: 309.0 (M-tBu+H)⁺.

### Reference Example and Example 195

### (195-1) 3-(2-Oxoazepan-3-yl)-1H-benzimidazol-2-one (Reference Example Compound 195-1)

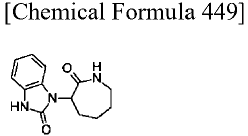

3-(2-Aminoanilino)azepan-2-one (2.10 g) was dissolved in tetrahydrofuran (30 mL), carbonyldiimidazole (1.86 g) was added, and the mixture was stirred at room temperature for 3 hours. Additional carbonyldiimidazole (466 mg) was added, and the mixture was stirred at room temperature for 14 hours. Water was added to the reaction mixture, THF was evaporated, and the mixture was extracted with chloroform. The organic layer was dried over sodium sulfate, filtered, and the filtrate was concentrated under reduced pressure. The residue was suspended and washed with ethyl acetate, filtered, and dried under reduced pressure to obtain the title compound (1.78 g) as a light brown powder. MS (ESI) m/z: 246.2 (M+H)⁺

### (195-2) tert-Butyl 8-[2-oxo-3-(2-oxoazepan-3-yl)benzimidazol-1-yl]octanoate (Reference Example Compound 195-2)

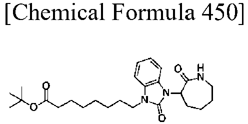

3-(2-Oxoazepan-3-yl)-1H-benzimidazol-2-one (150 mg), tert-butyl 8-bromooctanoate (205 mg), potassium iodide (11 mg), and potassium carbonate (127 mg) were added to N,N-dimethylformamide (2 mL) and stirred at room temperature for 65 hours. The mixture was warmed to 50 °C and stirred for 5 hours. Additional tert-butyl 8-bromooctanoate (104 mg) and potassium carbonate (85 mg) were added, and the mixture was stirred at 50 °C for 3 hours. Water was added to the reaction mixture, and the mixture was extracted with ethyl acetate. The organic layer was washed with water and saturated brine, dried over sodium sulfate, filtered, and the filtrate was concentrated under reduced pressure. The residue was purified by silica gel column chromatography (hexane:ethyl acetate = 70:30 to 20:80) to obtain the title compound (207 mg) as a colorless powder. MS (ESI) m/z: 444.3 (M+H)⁺

### (195-3) tert-Butyl 8-[3-(2,7-dioxoazepan-3-yl)-2-oxobenzimidazol-1-yl]octanoate (Example Compound 195)

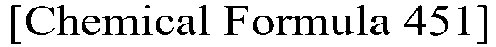

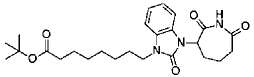

Using tert-butyl 8-[2-oxo-3-(2-oxoazepan-3-yl)benzimidazol-1-yl]octanoate instead of tert-butyl 2-[2-fluoro-3-[(2-oxoazepan-3-yl)carbamoyl]phenoxy]acetate in Example 67, the same reaction and processing were performed to obtain the title compound as a colorless powder. MS (ESI) m/z: 402.2 (M-tBu+H)⁺

The corresponding starting compounds were processed in the same manner as in (195-2) and (195-3) to obtain the compounds listed in the table below.

**[Table 14]**

| Example | Structural formula | Physical properties etc. |
|---|---|---|
| 196 | 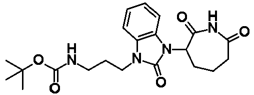 tert-Butyl N-[3-[3-(2,7-dioxoazepan-3-yl)-2-oxobenzimidazol-1-yl]propyl]carbamate | Colorless powder |
| | | MS (ESI) m/z: 317.1 (M-Boc+H)⁺ |
| 197 | 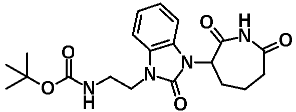 tert-Butyl N-[2-[3-(2,7-dioxoazepan-3-yl)-2-oxobenzimidazol-1-yl]ethyl]carbamate | Colorless powder |
| | | MS (ESI) m/z: 303.1 (M-Boc+H)⁺ |
| 198 | 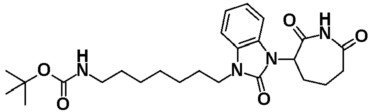 tert-Butyl N-[7-[3-(2,7-dioxoazepan-3-yl)-2-oxobenzimidazol-1-yl]heptyl]carbamate | Colorless powder |
| | | MS (ESI) m/z: 373.2 (M-Boc+H)⁺ |
| 199 | 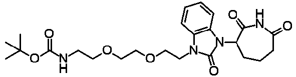 tert-Butyl N-[2-[2-[2-[3-(2, 7-dioxoazepan-3-yl)-2-oxobenzimidazol-1-yl]ethoxy]ethoxy]ethyl]carbamate | Colorless powder |
| | | MS (ESI) m/z: 391.2 (M-Boc+H)⁺ |
| 200 | 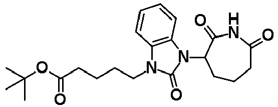 tert-Butyl 5-[3-(2,7-dioxoazepan-3-yl)-2-oxobenzimidazol-1-yl]pentanoate | Light brown powder |
| | | MS (ESI) m/z: 360.2 (M-tBu+H)⁺ |

### Example and Reference Example 201

### (201-1) 3-[(3-Amino-2-pyridyl)amino]azepan-2-one (Reference Example Compound 201-1)

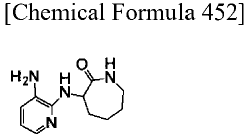

Using 2-fluoro-3-nitropyridine instead of 1-fluoro-2-nitrobenzene in Reference Example 138-1, the same reactions and processing as in (138-1) and (138-2) were performed to obtain the title compound as a yellow powder. MS (ESI) m/z: 221.2 (M+H)⁺

### (201-2) 3-(2-Oxoazepan-3-yl)-1H-imidazo[4,5-b]pyridin-2-one (Reference Example Compound 201-2)

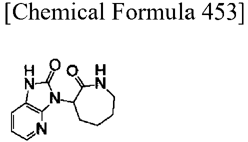

Using 3-[(3-amino-2-pyridyl)amino]azepan-2-one instead of tert-butyl 2-[2-fluoro-3-[(2-oxoazepan-3-yl)carbamoyl]phenoxy]acetate in Example 67, the same reaction and processing were performed to obtain the title compound as a yellow powder. MS (ESI) m/z: 247.2 (M+H)⁺

### (201-3) tert-Butyl 8-[2-oxo-3-(2-oxoazepan-3-yl)imidazo[4,5-b]pyridin-1-yl]octanoate (Reference Example Compound 201-3)

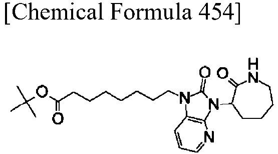

Using 3-(2-oxoazepan-3-yl)-1H-imidazo[4,5-b]pyridin-2-one instead of tert-butyl 2-[2-fluoro-3-[(2-oxoazepan-3-yl)carbamoyl]phenoxy]acetate in Reference Example 195-2, the same reaction and processing were performed to obtain the title compound as a yellow powder.

### (201-4) tert-Butyl 8-[3-(2,7-dioxoazepan-3-yl)-2-oxo-imidazo[4,5-b]pyridin-1-yl]octanoate (Example Compound 201-1)

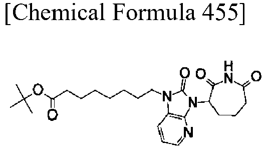

Using tert-butyl 8-[2-oxo-3-(2-oxoazepan-3-yl)imidazo[4,5-b]pyridin-1-yl]octanoate instead of tert-butyl 2-[2-fluoro-3-[(2-oxoazepan-3-yl)carbamoyl]phenoxy]acetate in Example 67, the same reaction and processing were performed to obtain the title compound as a yellow powder. MS (ESI) m/z: 459.3 (M+H)⁺.

### Example 201-2

### tert-Butyl N-[7-[3-(2,7-dioxoazepan-3-yl)-2-oxo-imidazo[4,5-b]pyridin-1-yl]heptyl]carbamate (Example Compound 201-2)

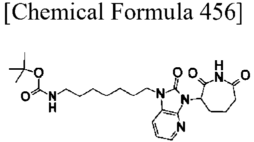

tert-Butyl 8-[3-(2,7-dioxoazepan-3-yl)-2-oxo-imidazo[4,5-b]pyridin-1-yl]octanoate was dissolved in methylene chloride (1 mL), trifluoroacetic acid (1 mL) was added, and the mixture was stirred at room temperature for 1 hour. After confirming the completion of the reaction, the reaction mixture was concentrated to obtain 8-[3-(2,7-dioxoazepan-3-yl)-2-oxo-imidazo[4,5-b]pyridin-1-yl]octanoic acid. This was dissolved in tert-butanol (1 mL), triethylamine (152 µL) and diphenylphosphoryl azide (90 mg) were added, and the mixture was stirred at 80 °C for 1.5 hours. The reaction mixture was concentrated, and the crude product was purified by silica gel column chromatography (chloroform/methanol = 100/0 to 90/10) to obtain the title compound (24 mg) as a colorless powder.

### Example 202

### 1-(3-Chloro-4-methylphenyl)-3-[3-[3-(2,7-dioxoazepan-3-yl)-2-oxobenzimidazol-1-yl]propyl]urea (Example Compound 202)

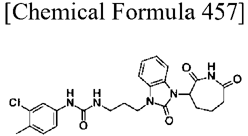

tert-Butyl N-[3-[3-(2,7-dioxoazepan-3-yl)-2-oxobenzimidazol-1-yl]propyl]carbamate (50 mg) was dissolved in dichloromethane (1 mL), trifluoroacetic acid (1 mL) was added, and the mixture was stirred at room temperature for 1 hour. The reaction mixture was concentrated and dried under reduced pressure. The residue, (4-methoxyphenyl) N-(3-chloro-4-methylphenyl)carbamate (36 mg), and N,N-diisopropylethylamine (63 mg) were dissolved in acetonitrile (1 mL) and stirred at 70 °C for 3 hours. Water was added to the reaction mixture, and the mixture was extracted with ethyl acetate. The organic layer was washed with saturated brine, dried over sodium sulfate, filtered, and the filtrate was concentrated under reduced pressure. The residue was purified by silica gel column chromatography (chloroform:methanol = 100:0 to 94:6) to obtain the title compound (36 mg) as a colorless powder. MS (ESI) m/z: 484.2/486.2 (M+H)⁺

### Example 203

### 1-(3-Chloro-4-methylphenyl)-3-[2-[3-(2,7-dioxoazepan-3-yl)-2-oxobenzimidazol-1-yl]ethyl]urea (Example Compound 203)

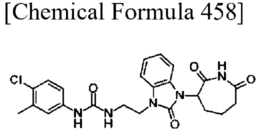

Using tert-butyl N-[2-[3-(2,7-dioxoazepan-3-yl)-2-oxobenzimidazol-1-yl]ethyl]carbamate instead of tert-butyl N-[3-[3-(2,7-dioxoazepan-3-yl)-2-oxobenzimidazol-1-yl]propyl]carbamate in Example 202, the same reaction and processing were performed to obtain the title compound (48 mg) as a colorless powder. MS (ESI) m/z: 470.2/472.2 (M+H)⁺

### Reference Example and Example 204

### Reference Example 204-1

### 3-(4-Iodophenyl)-4-methyl-1H-1,2,4-triazol-5-one (Reference Example Compound 204-1)

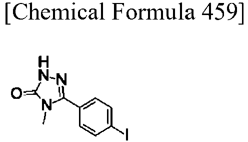

1-[(4-Iodobenzoyl)amino]-3-methylurea (1.93 g) was added to 4N aqueous sodium hydroxide (10 mL) and stirred at 100 °C for 9 hours. The reaction mixture was cooled to room temperature. Under ice-cooling, concentrated hydrochloric acid was added to adjust the pH to 8, and the precipitated solid was filtered, washed with 1N hydrochloric acid, and dried under reduced pressure to obtain the title compound (1.42 g) as a colorless powder. MS (ESI) m/z: 302.0 (M+H)⁺

### Reference Example 204-2

### 3-[3-(4-Iodophenyl)-4-methyl-5-oxo-1,2,4-triazol-1-yl]azepan-2-one (Reference Example Compound 204-2)

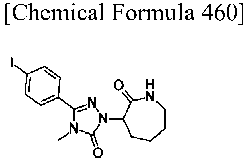

Using 3-(4-iodophenyl)-4-methyl-1H-1,2,4-triazol-5-one instead of 3-iodoindazole in Reference Example 135, the same reaction and processing were performed to obtain the title compound as a colorless powder. MS (ESI) m/z: 413.1 (M+H)⁺

### Example 204

### 3-[3-(4-Iodophenyl)-4-methyl-5-oxo-1,2,4-triazol-1-yl]azepane-2,7-dione (Example Compound 204)

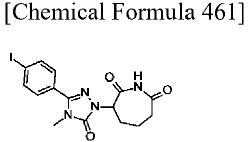

Using 3-[3-(4-iodophenyl)-4-methyl-5-oxo-1,2,4-triazol-1-yl]azepan-2-one instead of tert-butyl 2-[2-fluoro-3-[(2-oxoazepan-3-yl)carbamoyl]phenoxy]acetate in Example 67, the same reaction and processing were performed to obtain the title compound as a colorless powder. MS (ESI) m/z: 427.1 (M-tBu+H)⁺

### Example 205

### Benzyl 8-[1-(2,7-dioxoazepan-3-yl)indazol-3-yl]oct-7-ynoate (Example Compound 205)

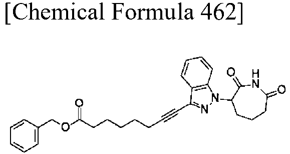

3-(3-Iodoindazol-1-yl)azepane-2,7-dione (50 mg), benzyl oct-7-ynoate (47 mg), bis(triphenylphosphine)palladium(II) dichloride (10 mg), copper(I) iodide (5.5 mg), and N,N-diisopropylethylamine (88 mg) were dissolved in tetrahydrofuran and stirred at an external temperature of 60 °C for 1.5 hours. Additional benzyl oct-7-ynoate (78 mg), bis(triphenylphosphine)palladium(II) dichloride (10 mg), copper(I) iodide (5.5 mg), and N,N-diisopropylethylamine (88 mg) were added to the reaction mixture, and the mixture was stirred at an external temperature of 60 °C for 3 hours. Water was added to the reaction mixture, and the mixture was extracted with ethyl acetate. The organic layer was washed with water and saturated brine, dried over sodium sulfate, filtered, and the filtrate was concentrated under reduced pressure. The residue was purified by silica gel column chromatography (hexane:ethyl acetate = 85:15 to 50:50) to obtain the title compound (51 mg) as a brown powder. MS (ESI) m/z: 472.3 (M+H)⁺

### Reference Example and Example 206

### (206-1) Benzyl 8-[3-methyl-2-oxo-1-(2-oxoazepan-3-yl)benzimidazol-4-yl]oct-7-ynoate (Reference Example Compound 206-1)

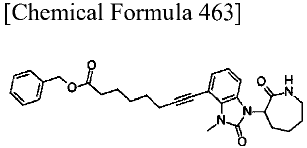

Using 4-iodo-3-methyl-1-(2-oxoazepan-3-yl)benzimidazol-2-one instead of 3-(3-iodoindazol-1-yl)azepane-2,7-dione in Example 205, the same reaction and processing were performed to obtain the title compound as a colorless powder. MS (ESI) m/z: 488.3 (M+H)⁺

### (206-2) tert-Butyl 8-[3-methyl-2-oxo-1-(2-oxoazepan-3-yl)benzimidazol-4-yl]oct-7-ynoate (Reference Example Compound 206-2)

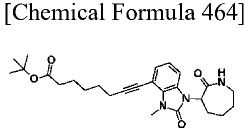

Benzyl 8-[3-methyl-2-oxo-1-(2-oxoazepan-3-yl)benzimidazol-4-yl]oct-7-ynoate (170 mg) was dissolved in tetrahydrofuran (1 mL) and methanol (1 mL), and 1N aqueous sodium hydroxide (2 mL) was added, followed by stirring at room temperature for 2 hours. The reaction mixture was concentrated, 1N hydrochloric acid was added, and the mixture was extracted with chloroform. The organic layer was dried over sodium sulfate, filtered, and the filtrate was concentrated under reduced pressure. The residue was suspended in dichloromethane (2 mL), 2-tert-butyl-1,3-diisopropylisourea (280 mg) was added, and the mixture was stirred at room temperature for 41 hours. Additional 2-tert-butyl-1,3-diisopropylisourea (280 mg) was added, and the mixture was stirred at room temperature for 95 hours. The reaction mixture was filtered through Celite, and the filtrate was concentrated under reduced pressure. The residue was purified by silica gel column chromatography (hexane:ethyl acetate = 50:50 to 0:100, ethyl acetate:methanol = 90:10) to obtain the title compound (127 mg) as a colorless powder. MS (ESI) m/z: 454.3 (M+H)⁺

### (206-3) tert-Butyl 8-[1-(2,7-dioxoazepan-3-yl)-3-methyl-2-oxobenzimidazol-4-yl]oct-7-ynoate (Example Compound 206)

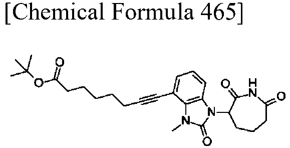

Using tert-butyl 8-[3-methyl-2-oxo-1-(2-oxoazepan-3-yl)benzimidazol-4-yl]oct-7-ynoate instead of tert-butyl 2-[2-fluoro-3-[(2-oxoazepan-3-yl)carbamoyl]phenoxy]acetate in Example 67, the same reaction and processing were performed to obtain the title compound as a light brown powder. MS (ESI) m/z: 468.2 (M+H)⁺

### Reference Example and Example 207

### Reference Example 207-1

### tert-Butyl 8-[3-methyl-2-oxo-1-(2-oxoazepan-3-yl)benzimidazol-4-yl]octanoate (Reference Example Compound 207-1)

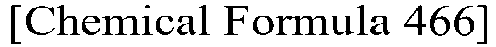

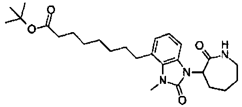

Using tert-butyl 8-[3-methyl-2-oxo-1-(2-oxoazepan-3-yl)benzimidazol-4-yl]oct-7-ynoate instead of 3-(3-nitrophenyl)azepane-2,7-dione in Example 70, the same reaction and processing were performed to obtain the title compound as a colorless powder. MS (ESI) m/z: 402.3 (M-tBu+H)⁺

### Example 207

### tert-Butyl 8-[1-(2,7-dioxoazepan-3-yl)-3-methyl-2-oxobenzimidazol-4-yl]octanoate (Example Compound 207)

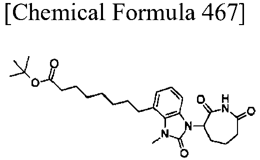

Using tert-butyl 8-[3-methyl-2-oxo-1-(2-oxoazepan-3-yl)benzimidazol-4-yl]octanoate instead of tert-butyl 2-[2-fluoro-3-[(2-oxoazepan-3-yl)carbamoyl]phenoxy]acetate in Example 67, the same reaction and processing were performed to obtain the title compound as a colorless powder. MS (ESI) m/z: 416.3 (M-tBu+H)⁺

### Example 208

### tert-Butyl 8-[4-[1-(2,7-dioxoazepan-3-yl)-4-methyl-5-oxo-1,2,4-triazol-3-yl]phenyl]octanoate (Example Compound 208)

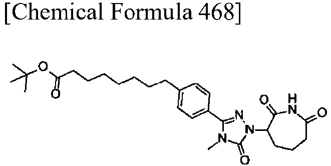

Using 3-[3-(4-iodophenyl)-4-methyl-5-oxo-1,2,4-triazol-1-yl]azepan-2-one instead of 4-iodo-3-methyl-1-(2-oxoazepan-3-yl)benzimidazol-2-one in (206-1), the same reactions and processing as in (206-1), (206-2), (207-1), and (207-2) were performed to obtain the title compound as a colorless powder. MS (ESI) m/z: 443.3 (M-tBu+H)⁺

### Example 209

### 8-[1-(2,7-Dioxoazepan-3-yl)indazol-3-yl]octanoic acid (Example Compound 209)

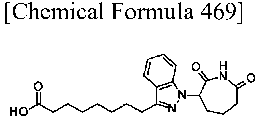

Benzyl 8-[1-(2,7-dioxoazepan-3-yl)indazol-3-yl]oct-7-ynoate (95 mg) was dissolved in tert-butanol (2 mL) and chloroform (1 mL), and palladium on carbon (20 mg) was added. The mixture was placed under a hydrogen atmosphere and stirred at room temperature for 30 hours. The reaction mixture was filtered through Celite, and the filtrate was concentrated under reduced pressure. The residue was purified by silica gel column chromatography (hexane:ethyl acetate = 80:20 to 50:50) to obtain benzyl 8-[1-(2,7-dioxoazepan-3-yl)indazol-3-yl]octanoate (35 mg) as a colorless viscous material. This was dissolved in tert-butanol (1 mL), and palladium on carbon (10 mg) was added. The mixture was placed under a hydrogen atmosphere and stirred at room temperature for 2 hours. The reaction mixture was filtered through Celite, and the filtrate was concentrated under reduced pressure. The residue was purified by silica gel column chromatography (chloroform:methanol = 100:0 to 95:5) to obtain the title compound (22 mg) as a colorless powder. MS (ESI) m/z: 386.2 (M+H)⁺

### Reference Example and Example 210

### Reference Example 210-1

### 3-(2-Amino-3-iodoanilino)azepan-2-one (Reference Example Compound 210-1)

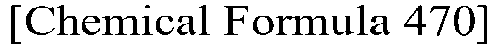

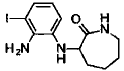

Using 3-iodo-1-fluoro-2-nitrobenzene instead of 1-fluoro-2-nitrobenzene in Reference Example 138-1, the same reactions and processing as in (Reference Example 138-1) and (Reference Example 138-2) were performed to obtain the title compound as a light brown powder. MS (ESI) m/z: 346.0 (M+H)⁺

### Reference Example 210-2

### 4-Iodo-3-methyl-1-(2-oxoazepan-3-yl)benzimidazol-2-one (Reference Example Compound 210-2)

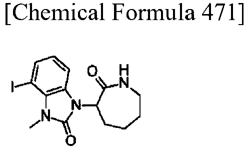

3-(2-Amino-3-iodoanilino)azepan-2-one (2.21 g) was dissolved in tetrahydrofuran (10 mL), and N,N-diisopropylethylamine (2.22 mL) and triphosgene (760 mg) were added. The mixture was stirred at room temperature for 0.5 hours. 1N hydrochloric acid was added to the reaction mixture, and the mixture was extracted with ethyl acetate. The organic layer was washed with saturated brine, and dried over anhydrous sodium sulfate, and then the solvent was evaporated under reduced pressure. The residue was dissolved in N,N-dimethylformamide (20 mL), and potassium carbonate (1.77 g) and iodomethane (1.10 g) were added, followed by stirring at room temperature for 14 hours. Water was added to the reaction mixture, and the precipitate was filtered and dried under reduced pressure to obtain the title compound (2.10 g) as a colorless powder. MS (ESI) m/z: 386.0 (M+H)⁺

### Example 210

### 3-(4-Iodo-3-methyl-2-oxobenzimidazol-1-yl)azepane-2,7-dione (Example Compound 210)

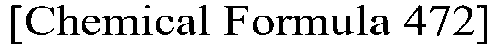

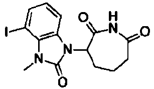

Using 4-iodo-3-methyl-1-(2-oxoazepan-3-yl)benzimidazol-2-one instead of tert-butyl 2-[2-fluoro-3-[(2-oxoazepan-3-yl)carbamoyl]phenoxy]acetate in Example 67, the same reaction and processing were performed to obtain the title compound as a colorless powder. MS (ESI) m/z: 400.1 (M+H)⁺

### Example 211

### 3-(4-Iodo-3-methyl-2-oxobenzimidazol-1-yl)azepane-2,7-dione (Optically Active A, Example Compound 211-1) and (Optically Active B, Example Compound 211-2)

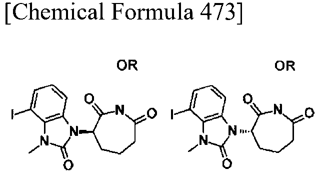

3-(4-Iodo-3-methyl-2-oxobenzimidazol-1-yl)azepane-2,7-dione (racemate, 30 mg) was optically resolved by chiral HPLC (CHIRALPACK IA (30×250 mm), mobile phase: ethanol:acetonitrile = 80:20). The earlier-eluting fraction was concentrated and dried under reduced pressure to obtain Optically Active A (6.5 mg) as a colorless powder. MS (ESI) m/z: 400.0 (M+H)⁺. The later-eluting fraction was concentrated and dried under reduced pressure to obtain Optically Active B (20 mg) as a colorless powder. MS (ESI) m/z: 400.0 (M+H)⁺.

### Example 212

### tert-Butyl (E)-3-[1-(2,7-dioxoazepan-3-yl)-3-methyl-2-oxobenzimidazol-4-yl]prop-2-enoate (Example Compound 212)

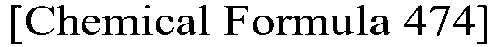

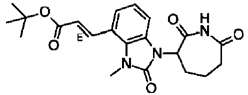

Using 3-(4-iodo-3-methyl-2-oxobenzimidazol-1-yl)azepane-2,7-dione instead of 3-(3-iodoindazol-1-yl)azepane-2,7-dione in Example 136, the same reaction and processing were performed to obtain the title compound as a pale yellow powder. MS (ESI) m/z: 400.2 (M+H)⁺.

### Example 213

### tert-Butyl 3-[1-(2,7-dioxoazepan-3-yl)-3-methyl-2-oxobenzimidazol-4-yl]propanoate (Example Compound 213)

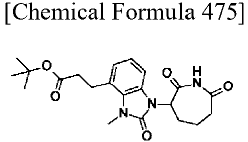

Using tert-butyl (E)-3-[1-(2,7-dioxoazepan-3-yl)-3-methyl-2-oxobenzimidazol-4-yl]prop-2-enoate instead of 3-(3-nitrophenyl)azepane-2,7-dione in Example 70, the same reaction and processing were performed to obtain the title compound as a colorless powder. MS (ESI) m/z: 346.1 (M-tBu+H)⁺.

### Example 214

### tert-Butyl 5-[1-(2,7-dioxoazepan-3-yl)-3-methyl-2-oxobenzimidazol-4-yl]pentanoate (Example Compound 214)

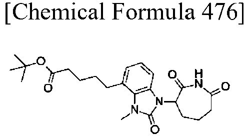

Using benzyl pent-5-ynoate instead of benzyl oct-7-ynoate in Reference Example 206-1, the same reactions and processing as in Reference Example 206-1, Reference Example 206-2, Reference Example 207-1, and Example 207 were performed to obtain the title compound as a colorless powder. MS (ESI) m/z: 374.2 (M-tBu+H)⁺

### Reference Example and Example 215

### Reference Example 215-1

### 5-Iodo-3-methyl-1-(2-oxoazepan-3-yl)benzimidazol-2-one (Reference Example Compound 215-1)

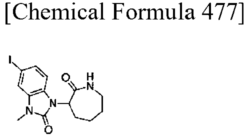

Using 4-iodo-1-fluoro-2-nitrobenzene instead of 3-iodo-1-fluoro-2-nitrobenzene in Reference Example 210-1, the same reactions and processing as in Reference Example 210-1 and Reference Example 210-2 were performed to obtain the title compound as a brown powder. MS (ESI) m/z: 386.0 (M+H)⁺

### Reference Example 215-2

### tert-Butyl 8-[3-methyl-2-oxo-1-(2-oxoazepan-3-yl)benzimidazol-5-yl]oct-7-ynoate (Reference Example Compound 215-2)

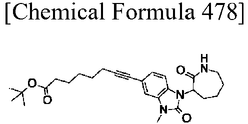

Using 5-iodo-3-methyl-1-(2-oxoazepan-3-yl)benzimidazol-2-one instead of 4-iodo-3-methyl-1-(2-oxoazepan-3-yl)benzimidazol-2-one in Reference Example 206-1, the same reactions and processing as in Reference Example 206-1 and Reference Example 206-2 were performed to obtain the title compound as a pale yellow powder. MS (ESI) m/z: 398.3 (M-tBu+H)⁺

### Example 215

### tert-Butyl 8-[1-(2,7-dioxoazepan-3-yl)-3-methyl-2-oxobenzimidazol-5-yl]oct-7-ynoate (Example Compound 215)

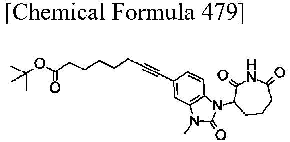

Using tert-butyl 8-[3-methyl-2-oxo-1-(2-oxoazepan-3-yl)benzimidazol-5-yl]oct-7-ynoate instead of tert-butyl 2-[2-fluoro-3-[(2-oxoazepan-3-yl)carbamoyl]phenoxy]acetate in Example 67, the same reaction and processing were performed to obtain the title compound as a colorless powder. MS (ESI) m/z: 412.2 (M-tBu+H)⁺

### Example 216

### tert-Butyl 8-[1-(2,7-dioxoazepan-3-yl)-3-methyl-2-oxobenzimidazol-5-yl]octanoate (Example Compound 216)

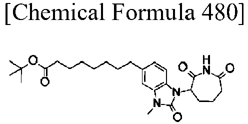

Using tert-butyl 8-[3-methyl-2-oxo-1-(2-oxoazepan-3-yl)benzimidazol-5-yl]oct-7-ynoate instead of tert-butyl 8-[3-methyl-2-oxo-1-(2-oxoazepan-3-yl)benzimidazol-4-yl]oct-7-ynoate in Reference Example 207-1, the same reactions and processing as in Reference Example 207-1 and Example 207 were performed to obtain the title compound as a colorless powder. MS (ESI) m/z: 416.3 (M-tBu+H)⁺

### Example 217

### tert-Butyl 5-[1-(2,7-dioxoazepan-3-yl)-3-methyl-2-oxobenzimidazol-5-yl]pentanoate (Example Compound 217)

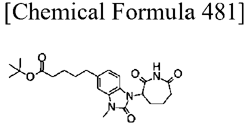

Using benzyl pent-4-ynoate instead of benzyl oct-7-ynoate in Reference Example 215-2, the same reactions and processing as in Reference Example 215-2, Reference Example 206-2, and Example 216 were performed to obtain the title compound as a colorless powder. MS (ESI) m/z: 374.2 (M-tBu+H)⁺

### Reference Example and Example 218

### Reference Example 218-1

### tert-Butyl 4-[3-methyl-2-oxo-1-(2-oxoazepan-3-yl)benzimidazol-5-yl]-3,6-dihydro-2H-pyridine-1-carboxylate (Reference Example Compound 218-1)

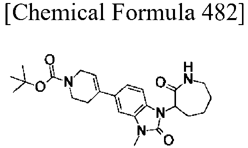

5-iodo-3-methyl-1-(2-oxoazepan-3-yl) benzimidazol-2-one (500mg), 1-tert-butoxycarbonyl-1,2,3,6-tetrahydro-4-(4,4,5,5-tetramethyl-1,3,2-dioxaborolan-2-yl)pyridine (482mg), tetrakis(triphenylphosphine)palladium (150mg), and potassium carbonate (540mg) were dissolved in water (1mL) and 1,4-dioxane (5mL), and the mixture was stirred at 100 °C for 2 hours. Water was added to the reaction mixture, and the mixture was extracted with ethyl acetate. The organic layer was washed with water and saturated brine, and dried over anhydrous sodium sulfate, and then the solvent was evaporated under reduced pressure. The residue was purified by silica gel column chromatography (ethyl acetate:methanol = 100:0 to 90:10) to obtain the title compound (342 mg) as a light brown powder. MS (ESI) m/z: 441.3 (M+H)⁺

### Reference Example 218-2

### tert-Butyl 4-[3-methyl-2-oxo-1-(2-oxoazepan-3-yl)benzimidazol-5-yl]piperidine-1-carboxylate (Reference Example Compound 218-2)

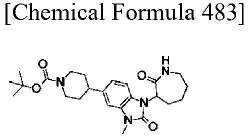

tert-Butyl 4-[3-methyl-2-oxo-1-(2-oxoazepan-3-yl)benzimidazol-5-yl]-3,6-dihydro-2H-pyridine-1-carboxylate (255 mg) was dissolved in methanol (3 mL), and palladium hydroxide (200 mg) was added. The mixture was placed under a hydrogen atmosphere and stirred at room temperature for 17 hours. The reaction mixture was filtered through Celite, the filtrate was concentrated, and the resulting residue was purified by silica gel column chromatography (ethyl acetate:methanol = 100:0 to 95:5 to 90:10) to obtain the title compound (192 mg) as a colorless powder. MS (ESI) m/z: 387.2 (M-tBu+H)⁺

### Example 218

### tert-Butyl 4-[1-(2,7-dioxoazepan-3-yl)-3-methyl-2-oxobenzimidazol-5-yl]piperidine-1-carboxylate (Example Compound 218)

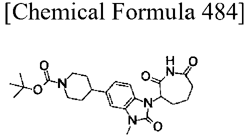

Using tert-butyl 4-[3-methyl-2-oxo-1-(2-oxoazepan-3-yl)benzimidazol-5-yl]piperidine-1-carboxylate instead of 3-(3-nitrophenyl)azepane-2,7-dione in Example 70, the same reaction and processing were performed to obtain the title compound as a pale yellow powder. MS (ESI) m/z: 401.2 (M-tBu+H)⁺

### Example 219

### tert-Butyl 2-[4-[1-(2,7-dioxoazepan-3-yl)-3-methyl-2-oxobenzimidazol-5-yl]piperidin-1-yl]acetate (Example Compound 219)

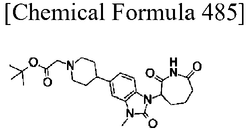

Using tert-butyl 4-[1-(2,7-dioxoazepan-3-yl)-3-methyl-2-oxobenzimidazol-5-yl]piperidine-1-carboxylate instead of tert-butyl 6,8-dioxo-2,7-diazaspiro[4.6]undecane-2-carboxylate in Example 100, the same reaction and processing were performed to obtain the title compound as a colorless powder. MS (ESI) m/z: 471.4 (M+H)⁺

### Example 220 and Example 221

### tert-Butyl 4-[1-(2,7-dioxoazepan-3-yl)-3-methyl-2-oxobenzimidazol-4-yl]-3,6-dihydro-2H-pyridine-1-carboxylate (Example Compound 220)

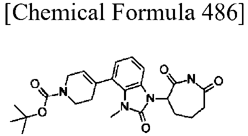

### 3-[3-Methyl-2-oxo-4-(4-pyridyl)benzimidazol-1-yl]azepane-2,7-dione (Example Compound 221)

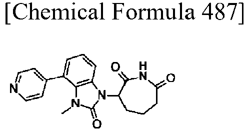

Using 5-bromo-3-methyl-1-(2-oxoazepan-3-yl)benzimidazol-2-one instead of 5-iodo-3-methyl-1-(2-oxoazepan-3-yl)benzimidazol-2-one in Reference Example 218-1, the same reactions and processing as in Reference Example 218-1 and Example 218 were performed to obtain Example Compound 220 (70 mg) as a colorless powder. MS (ESI) m/z: 455.2 (M+H)⁺. At the same time, Example Compound 221 (71 mg) was obtained as a colorless powder. MS (ESI) m/z: 351.1 (M+H)⁺

### Example 222

### tert-Butyl 2-[4-[1-(2,7-dioxoazepan-3-yl)-3-methyl-2-oxobenzimidazol-4-yl]-3,6-dihydro-2H-pyridin-1-yl]acetate (Example Compound 222)

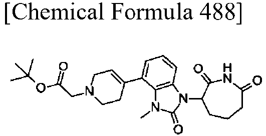

Using tert-butyl 4-[1-(2,7-dioxoazepan-3-yl)-3-methyl-2-oxobenzimidazol-4-yl]-3,6-dihydro-2H-pyridine-1-carboxylate instead of tert-butyl 6,8-dioxo-2,7-diazaspiro[4.6]undecane-2-carboxylate in Example 100, the same reaction and processing were performed to obtain the title compound as a colorless powder. MS (ESI) m/z: 469.2 (M+H)⁺

### Example 223 and Example 224

### tert-Butyl 4-(4,7,9-trioxo-3,8-diazaspiro[5.6]dodecan-3-yl)benzoate (Optically Active A, Example Compound 223) and (Optically Active B, Example Compound 224)

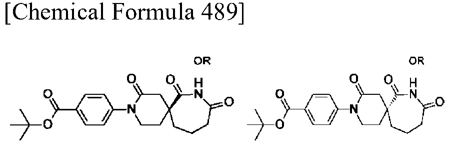

tert-Butyl 4-(4,7,9-trioxo-3,8-diazaspiro[5.6]dodecan-3-yl)benzoate (28 mg) was optically resolved by chiral HPLC (CHIRALPACK IE (30×250 mm), mobile phase: ethanol:acetonitrile = 70:30).
The earlier-eluting fraction was concentrated and dried under reduced pressure to obtain Example Compound 223 (11.2 mg) as a colorless powder. MS (ESI) m/z: 387.3 (M+H)⁺
The later-eluting fraction was concentrated and dried under reduced pressure to obtain Example Compound 224 (11.2 mg) as a colorless powder. MS (ESI) m/z: 387.3 (M+H)⁺

### Reference Example and Example 225

### Reference Example 225-1

### Methyl 4-(4-azidobutyl)-2-oxopiperidine-4-carboxylate (Reference Example Compound 225-1)

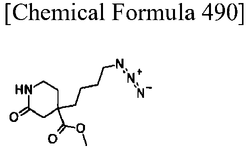

Using methyl 2-oxopiperidine-4-carboxylate instead of benzyl 2-oxopiperidine-4-carboxylate in Reference Example 107-2, the same reactions and processing as in Reference Example 107-2 and Reference Example 107-3 were performed to obtain the title compound as a light brown powder. MS (ESI) m/z: 255.2 (M+H)⁺.

### Reference Example 225-2

### Naphthalen-1-ylmethyl 4-(4-azidobutyl)-2-oxopiperidine-4-carboxylate (Reference Example Compound 225-2)

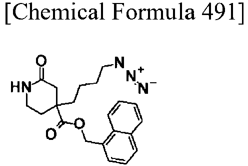

Methyl 4-(4-azidobutyl)-2-oxopiperidine-4-carboxylate (1.00 g) was dissolved in methanol (9 mL), 1N aqueous sodium hydroxide (3 mL) was added, and the mixture was stirred at room temperature for 27 hours. The reaction mixture was concentrated, neutralized by adding 1N hydrochloric acid, concentrated under reduced pressure, and azeotroped with toluene. The residue was suspended in N,N-dimethylformamide (10 mL), 1-(chloromethyl)naphthalene (903 mg) and potassium carbonate (1.09 g) were added, and the mixture was stirred at room temperature for 1 hour. The mixture was then stirred at 60 °C for 3 hours. Water was added to the reaction mixture, and after extraction with ethyl acetate, washing with water and saturated brine was performed, followed by drying over anhydrous sodium sulfate, and then the solvent was evaporated under reduced pressure. The residue was purified by silica gel column chromatography (hexane:ethyl acetate = 50:50 to 0:100, ethyl acetate:methanol = 90:10) to obtain the title compound (1.29 g) as a colorless powder. MS (ESI) m/z: 381.2 (M+H)⁺

### Reference Example 225-3

### Naphthalen-1-ylmethyl 4-(4-azidobutyl)-1-(4-nitrophenyl)-2-oxopiperidine-4-carboxylate (Reference Example Compound 225-3)

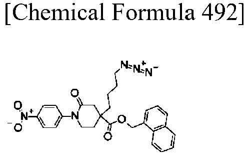

Using naphthalen-1-ylmethyl 4-(4-azidobutyl)-2-oxopiperidine-4-carboxylate instead of benzyl 4-(4-azidobutyl)-2-oxopiperidine-4-carboxylate and 1-iodo-4-nitrobenzene instead of tert-butyl 6-bromopyridine-3-carboxylate in Reference Example 108-1, the same reaction and processing were performed to obtain the title compound as a brown powder. MS (ESI) m/z: 502.2 (M+H)⁺

### Reference Example 225-4

### Naphthalen-1-ylmethyl 4-(4-aminobutyl)-1-(4-nitrophenyl)-2-oxopiperidine-4-carboxylate (Reference Example Compound 225-4)

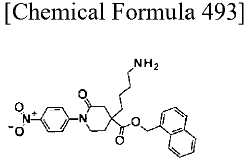

Using naphthalen-1-ylmethyl 4-(4-azidobutyl)-1-(4-nitrophenyl)-2-oxopiperidine-4-carboxylate instead of benzyl 4-(4-azidobutyl)-1-[2-[(2-methylpropan-2-yl)oxy]-2-oxoethyl]-2-oxopiperidine-4-carboxylate in Reference Example 107-5, the same reaction and processing were performed to obtain the title compound as a light brown powder. MS (ESI) m/z: 476.3 (M+H)⁺

### Reference Example 225-5

### 3-(4-Nitrophenyl)-3,8-diazaspiro[5.6]dodecane-4,7-dione (Reference Example Compound 225-5)

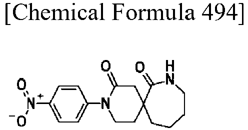

To naphthalen-1-ylmethyl 4-(4-aminobutyl)-1-(4-nitrophenyl)-2-oxopiperidine-4-carboxylate (1.53 g), anisole (1.05 g) and trifluoroacetic acid (15 mL) were added, and the mixture was stirred at room temperature for 17 hours, and then at 50 °C for 2 hours. The reaction mixture was concentrated and azeotroped with toluene. The residue was dissolved in N,N-dimethylformamide (30 mL), and N,N-diisopropylethylamine (1.25 g) and O-(7-azabenzotriazol-1-yl)-N,N,N',N'-tetramethyluronium hexafluorophosphate (1.47 g) were added. The mixture was stirred at room temperature for 2 hours. Water was added to the reaction mixture, and the mixture was extracted with ethyl acetate. The aqueous layer was extracted with chloroform. The organic layers were combined and dried over anhydrous sodium sulfate, and the solvent was evaporated under reduced pressure. The residue was purified by silica gel column chromatography (chloroform:methanol = 100:0 to 94:6) to obtain the title compound (640 mg) as a colorless powder. MS (ESI) m/z: 318.1 (M+H)⁺

### Example 225

### 3-(4-Nitrophenyl)-3,8-diazaspiro[5.6]dodecane-4,7,9-trione (Example Compound 225)

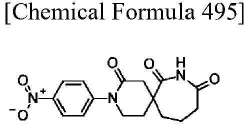

Using 3-(4-nitrophenyl)-3,8-diazaspiro[5.6]dodecane-4,7-dione instead of 3-(3-nitrophenyl)azepane-2,7-dione in Example 70, the same reaction and processing were performed to obtain the title compound as a pale yellow powder. MS (ESI) m/z: 332.1 (M+H)⁺

The corresponding starting compounds were processed in the same manner as in Example 70 to obtain the compounds listed in the table below.

**[Table 15]**

| Example | Structural formula | Physical properties etc. |
|---|---|---|
| 226 | 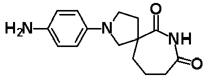 2-(4-Aminophenyl)-2, 7-diazaspiro[4.6]undecane-6, 8-dione | Green powder |
| | | MS (ESI) m/z: 274.2 (M+H)⁺ |
| 227 | 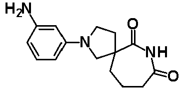 2-(3-Aminophenyl)-2, 7-diazaspiro[4.6]undecane-6, 8-dione | Brown powder |
| | | MS (ESI) m/z: 274.2 (M+H)⁺ |
| 228 | 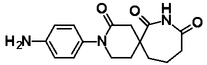 2-(4-Aminophenyl)-3,8-diazaspiro[5.6]dodecane-4,7,9-trione | Light brown powder |
| | | MS (ESI) m/z: 302.2 (M+H)⁺ |

The corresponding starting compounds were processed in the same manner as in Example 45 to obtain the compounds listed in the table.

**[Table 16]**

| Example | Structural formula | Physical properties etc. |
|---|---|---|
| 229 | 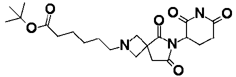 tert-Butyl 6-[6-(2,6-dioxopiperidin-3-yl)-5,7-dioxo-2, 6-diazaspiro[3. 4]octan-2-yl]hexanoate | White-brown viscous material |
| | | MS (ESI) m/z: 422.3 [M+H]⁺ |
| 230 | 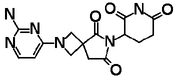 2-(2-Aminopyrimidin-4-yl)-6-(2,6-dioxopiperidin-3-yl)-2, 6-diazaspiro[3. 4]octane-5, 7-dione | White solid |
| | | MS (ESI) m/z: 345.0 [M+H]⁺ |
| 231 | 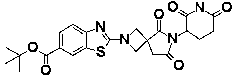 tert-Butyl 2-[6-(2,6-dioxopiperidin-3-yl)-5,7-dioxo-2,6-diazaspiro[3.4]octan-2-yl]-1,3-benzothiazole-6-carboxylate | White solid |
| | | MS (ESI) m/z: 485.3 [M+H]⁺ |
| 232 | 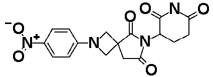 6-(2,6-Dioxopiperidin-3-yl)-2-(4-nitrophenyl)-2,6-diazaspiro[3. 4]octane-5, 7-dione | Yellow solid |
| | | MS (ESI) m/z: 373.0 [M+H]⁺ |
| 233 | 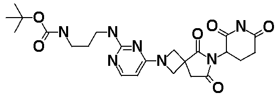 tert-Butyl N-[3-[4-(2, 6-dioxopiperidin-3-yl)-5,7-dioxo-2,6-diazaspiro[3.4]octan-2-yl]pyrimidin-2-yl]amino]propyl]carbamate | White-brown viscous material |
| | | MS (ESI) m/z: 502. 1 [M+H]⁺ |
| 234 | 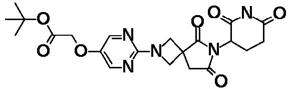 tert-Butyl 2-[2-(2,6-dioxopiperidin-3-yl)-5,7-dioxo-2, 6-diazaspiro[3.4]octan-2-yl]pyrimidin-5-yl]oxyacetate | White solid |
| | | MS (ESI) m/z: 460.0 [M+H]⁺ |
| 235 | 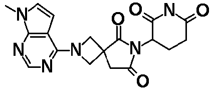 6-(2,6-Dioxopiperidin-3-yl)-2-(7-methylpyrrolo[2,3-d]pyrimidin-4-yl)-2,6-diazaspiro[3.4]octane-5,7-dione | Yellow solid |
| | | MS (ESI) m/z: 383.3 [M+H]⁺ |
| 236 | 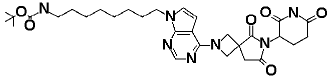 tert-Butyl N-[8-[4-[6-(2,6-dioxopiperidin-3-yl)-5,7-dioxo-2,6-diazaspiro[3.4]octan-2-yl]pyrrolo[2,3-d]pyrimidin-7-yl]octyl]carbamate | Yellow solid |
| | | MS (ESI) m/z: 596.1 [M+H]⁺ |
| 237 | 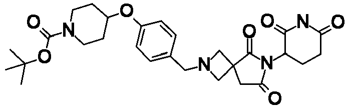 tert-Butyl 4-[4-[[6-(2,6-dioxopiperidin-3-yl)-5,7-dioxo-2,6-diazaspiro[3.4]octan-2-yl]methyl]phenoxy]piperidine-1-carboxylate | White solid |
| | | MS (ESI) m/z: 541. 0 [M+H]⁺ |
| 238 | 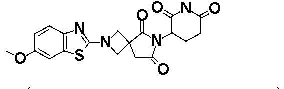 6-(2,6-Dioxopiperidin-3-yl)-2-(6-methoxy-1,3-benzothiazol-2-yl)-2,6-diazaspiro[3.4]octane-5,7-dione | White-brown solid |
| | | MS (ESI) m/z: 414.9 [M+H]⁺ |
| 239 | 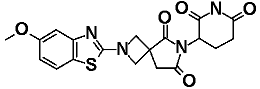 6-(2,6-Dioxopiperidin-3-yl)-2-(5-methoxy-1,3-benzothiazol-2-yl)-2,6-diazaspiro[3.4]octane-5,7-dione | White solid |
| | | MS (ESI) m/z: 414.9 [M+H]⁺ |
| 240 | 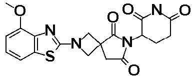 6-(2,6-Dioxopiperidin-3-yl)-2-(4-methoxy-1,3-benzothiazol-2-yl)-2,6-diazaspiro[3.4]octane-5,7-dione | White solid |
| | | MS (ESI) m/z: 414.9 [M+H]⁺ |
| 241 | 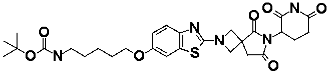 tert-Butyl N-[5-[[2-[6-(2,6-dioxopiperidin-3-yl)-5,7-dioxo-2,6-diazaspiro[3.4]octan-2-yl]-1,3-benzothiazol-6-yl]oxy]pentyl]carbamate | White-brown solid |
| | | MS (ESI) m/z: 586.5 [M+H]⁺ |
| 242 | 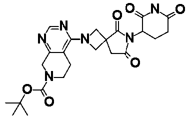 tert-Butyl 4-[6-(2, 6-dioxo-3-piperidyl)-5, 7-dioxo-2, 6-diazaspiro[3.4]octan-2-yl]-6,8-dihydro-5H-pyrido[3,4-d]pyrimidine-7-carboxylate | White solid |
| | | MS (ESI) m/z: 485.4 [M+H]⁺ |

### Example and Reference Example 243

### tert-Butyl 4-(3-hydroxy-2-oxoazepan-3-yl)benzoate (Reference Example Compound 243-1)

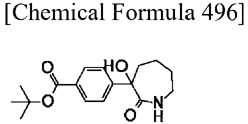

(243-1) Under a nitrogen stream, a tetrahydrofuran (3 mL) solution of tert-butyl 4-iodobenzoate (598 mg) was cooled to -20 °C, and isopropylmagnesium chloride-lithium chloride complex solution (1.3M, 1.51 mL) was added, followed by stirring for 30 minutes. A tetrahydrofuran (2 mL) solution of azepane-2,3-dione (100 mg) was added, and the mixture was stirred for 3 hours while warming to room temperature. 0.5M hydrochloric acid and chloroform were added to the reaction mixture, followed by stirring. The aqueous layer was removed using a phase separator, and the organic layer was concentrated under reduced pressure. The residue was purified by silica gel column chromatography (ethyl acetate:hexane = 50:50 to 100:0) to obtain the title compound (144 mg) as a white solid. MS (ESI) m/z: 306.2 (M+H)⁺.

### (243-2) tert-Butyl 4-(3-fluoro-2-oxoazepan-3-yl)benzoate (Reference Example Compound 243-2)

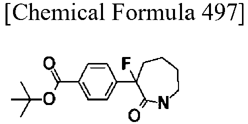

A dichloromethane (5 mL) solution of Reference Example Compound 243-1 (144 mg) was cooled to 0 °C, diethylaminosulfur trifluoride (0.103 mL) was added, and the mixture was stirred for 5 hours while warming to room temperature. Saturated aqueous sodium bicarbonate and chloroform were added to the reaction mixture, followed by stirring. The aqueous layer was removed using a phase separator, and the organic layer was concentrated under reduced pressure. The residue was purified by silica gel column chromatography (ethyl acetate:hexane = 30:70 to 70:30) to obtain the title compound (79.4 mg) as a pale yellow solid. MS (ESI) m/z: 308.0 (M+H)⁺.

### (243-3) tert-Butyl 4-(3-fluoro-2,7-dioxoazepan-3-yl)benzoate (Example Compound 243)

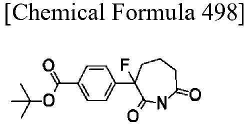

Using Reference Example Compound 243-2 instead of Reference Example Compound 1 in Reference Example and Example 1, the same reaction and processing as in (1-2) were performed to obtain the title compound as a white solid. MS (ESI) m/z: 320.1 [M-H]⁻.

### Example 244

### tert-Butyl 3-(3-fluoro-2,7-dioxoazepan-3-yl)benzoate (Example Compound 244)

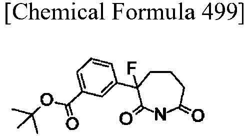

Using tert-butyl 3-iodobenzoate instead of tert-butyl 4-iodobenzoate in Example and Reference Example 243, the same reactions and processing as in (243-1) to (243-3) were performed to obtain the title compound as a pale yellow solid. MS (ESI) m/z: 320.1 [M-H]⁻.

### Example and Reference Example 245

### (245-1) N-[3-(3-Hydroxy-2-oxoazepan-3-yl)phenyl]acetamide (Reference Example Compound 245)

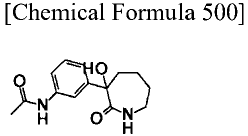

Under a nitrogen stream, a tetrahydrofuran (3 mL) solution of 3-[bis(trimethylsilyl)amino]phenylmagnesium chloride (1.0M, 2.36 mL) was cooled to 0 °C, and a tetrahydrofuran (2 mL) solution of azepane-2,3-dione (100 mg) was added dropwise, followed by stirring at room temperature for 2 hours. The mixture was treated with 0.5M hydrochloric acid, concentrated under reduced pressure, and azeotroped with toluene. To a tetrahydrofuran (5 mL) suspension of the residue, triethylamine (0.548 mL) and acetyl chloride (0.195 mL) were added, and the mixture was stirred at room temperature for 2 hours. Water and chloroform were added to the reaction mixture, followed by stirring. The aqueous layer was removed using a phase separator, and the organic layer was concentrated under reduced pressure. The residue was purified by silica gel column chromatography (methanol:chloroform = 0:100 to 5:95) and further purified by silica gel column chromatography (methanol:ethyl acetate = 0:100 to 5:95) to obtain the title compound (79.7 mg) as a white solid. MS (ESI) m/z: 263.0 (M+H)⁺.

### (245-2) N-[3-(3-Fluoro-2,7-dioxoazepan-3-yl)phenyl]acetamide (Example Compound 245)

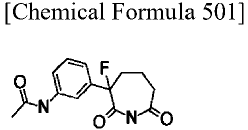

Using Reference Example Compound 245 instead of Reference Example Compound 243-1 in Example and Reference Example 243, the same reactions and processing as in (243-2) and (243-3) were performed to obtain the title compound as a pale yellow solid. MS (ESI) m/z: 278.9 (M+H)⁺.

### Example and Reference Example 246

### (246-1) 2-(6-Oxo-2,7-diazaspiro[4.6]undecan-2-yl)benzonitrile (Reference Example Compound 246)

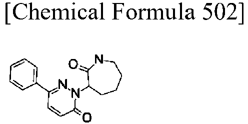

3-Phenyl-1H-pyridazin-6-one (200 mg), 3-bromoazepan-2-one (268 mg), and cesium carbonate (568 mg) were stirred in N,N-dimethylformamide (5 mL) at 80 °C for 8 hours. The reaction mixture was concentrated under reduced pressure, and the residue was purified by silica gel column chromatography (methanol:ethyl acetate = 0:100 to 10:90) to obtain the title compound (108 mg) as a white solid. MS (ESI) m/z: 284.0 (M+H)⁺.

### (246-2) 3-(6-Oxo-3-phenylpyridazin-1-yl)azepane-2,7-dione (Example Compound 246)

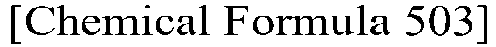

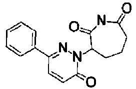

Using Reference Example Compound 246 instead of Reference Example Compound 1 in Reference Example and Example 1, the same reaction and processing as in (1-2) were performed to obtain the title compound as a white solid. MS (ESI) m/z: 298.0 (M+H)⁺.

The corresponding starting compounds were processed in the same manner as in Example 246 to obtain the compounds listed in the table below.

**[Table 17]**

| Example | Structural formula | Physical properties etc. |
|---|---|---|
| 247 | 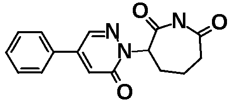 3-(6-Oxo-4-phenylpyridazin-1-yl)azepane-2,7-dione | White solid |
| | | MS (ESI) m/z: 298.1 [M+H]⁺ |
| 248 | 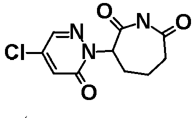 3-(4-Chloro-6-oxopyridazin-1-yl)azepane-2,7-dione | White solid |
| | | MS (ESI) m/z: 255.9/257.8 [M+H]⁺ |
| 249 | 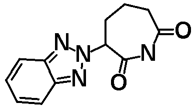 3-(Benzotriazol-2-yl)azepine 2,7-dione | White solid |
| | | MS (ESI) m/z: 244.9 [M+H]⁺ |
| 250 | 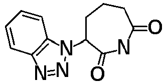 3-(Benzotriazol-1-yl)azepine 2,7-dione | White solid |
| | | MS (ESI) m/z: 244.9 [M+H]⁺ |
| 251 | 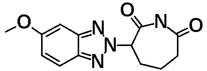 3-(5-Methoxybenzotriazol-2-yl)azepane-2,7-dione | White solid |
| | | MS (ESI) m/z: 274.9 [M+H]⁺ |
| 252 | 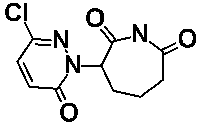 3-(3-Chloro-6-oxopyridazin-1-yl)azepane-2, 7-dione | White solid |
| | | MS (ESI) m/z: 255.9/257.8 [M+H]⁺ |
| 253 | 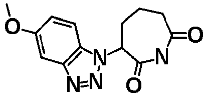 3-(5-Methoxybenzotriazol-1-yl)azepane-2,7-dione | White solid |
| | | MS (ESI) m/z: 275.1 [M+H]⁺ |
| 254 | 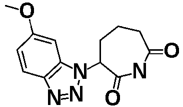 3-(6-Methoxybenzotriazol-1-yl)azepane-2,7-dione | White solid |
| | | MS (ESI) m/z: 274.9 [M+H]⁺ |
| 255 | 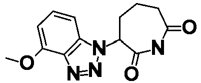 3-(4-Methoxybenzotriazol-1-yl)azepane-2,7-dione | White solid |
| | | MS (ESI) m/z: 275.1 [M+H]⁺ |
| 256 | 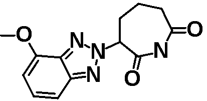 3-(4-Methoxybenzotriazol-2-yl)azepane-2,7-dione | White solid |
| | | MS (ESI) m/z: 274.9 [M+H]⁺ |
| 257 | 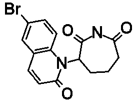 3-(6-Bromo-2-oxoquinolin-1-yl)azepane-2,7-dione | White solid |
| | | MS (ESI) m/z: 348.9/350.9 [M+H]⁺ |
| 258 | 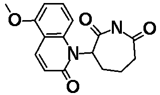 3-(5-Methoxy-2-oxoquinolin-1-yl)azepane-2,7-dione | White solid |
| | | MS (ESI) m/z: 300.9 [M+H]⁺ |
| 259 | 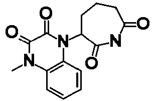 1-(2,7-Dioxoazepan-3-yl)-4-methylquinoxaline-2,3-dione | White solid |
| | | MS (ESI) m/z: 302.3 [M+H]⁺ |

### Reference Example and Example 260

### Reference Example 260-1

### 6-Bromo-3-(2-oxoazepan-3-yl)-1,3-benzoxazol-2-one (Reference Example Compound 260-1)

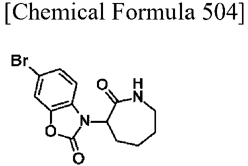

Using 6-bromo-3H-1,3-benzoxazol-2-one instead of 3-iodoindazole in Reference Example 135, the same reaction and processing were performed to obtain the title compound (643 mg) as a brown powder. MS (ESI) m/z: 325.0/327.0 (M+H)⁺

### Reference Example 260-2

### tert-Butyl (E)-3-[2-oxo-3-(2-oxoazepan-3-yl)-1,3-benzoxazol-6-yl]prop-2-enoate (Reference Example Compound 260-2)

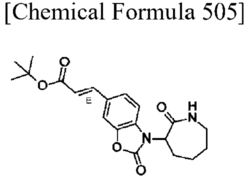

Using 6-bromo-3-(2-oxoazepan-3-yl)-1,3-benzoxazol-2-one instead of 3-(3-iodoindazol-1-yl)azepane-2,7-dione in Reference Example 136, the same reaction and processing were performed to obtain the title compound (200 mg) as a light brown powder. MS (ESI) m/z: 373.2 (M+H)⁺

### Reference Example 260-3

### tert-Butyl 3-[2-oxo-3-(2-oxoazepan-3-yl)-1,3-benzoxazol-6-yl]propanoate (Reference Example Compound 260-3)

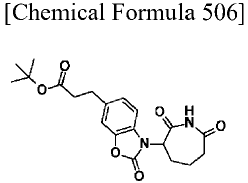

Using tert-butyl (E)-3-[2-oxo-3-(2-oxoazepan-3-yl)-1,3-benzoxazol-6-yl]prop-2-enoate instead of 3-(3-nitrophenyl)azepane-2,7-dione in Example 70, the same reaction and processing were performed to obtain the title compound (166 mg) as a light brown powder. MS (ESI) m/z: 319.1 (M-tBu+H)⁺

### Example 260

### tert-Butyl 3-[3-(2,7-dioxoazepan-3-yl)-2-oxo-1,3-benzoxazol-6-yl]propanoate (Example Compound 260)

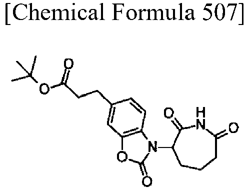

Using tert-butyl 3-[2-oxo-3-(2-oxoazepan-3-yl)-1,3-benzoxazol-6-yl]propanoate instead of tert-butyl 2-[2-fluoro-3-[(2-oxoazepan-3-yl)carbamoyl]phenoxy]acetate in Example 67, the same reaction and processing were performed to obtain the title compound (134 mg) as a colorless powder. MS (ESI) m/z: 333.1 (M-tBu+H)⁺

### Reference Example and Example 261

### Reference Example 261-1 3-[(4-Bromo-2-nitrophenyl)methylamino]azepan-2-one (Reference Example Compound 261-1)

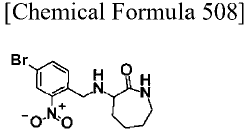

3-Aminoazepan-2-one (470 mg), 4-bromo-2-nitrobenzaldehyde (700 mg), and sodium triacetoxyborohydride (970 mg) were added to dichloromethane (10 mL), followed by stirring at room temperature for 18 hours. Saturated aqueous sodium bicarbonate was added to the reaction mixture, followed by extraction with chloroform and drying over anhydrous sodium sulfate, and the solvent was evaporated under reduced pressure. The residue was purified by silica gel column chromatography (ethyl acetate:methanol = 100:0 to 90:10) to obtain the title compound (795 mg) as a pale yellow powder. MS (ESI) m/z: 342.0/344.0 (M+H)⁺

### Reference Example 261-2

### 7-Bromo-3-(2-oxoazepan-3-yl)-1,4-dihydroquinazolin-2-one (Reference Example Compound 261-2)

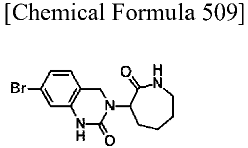

3-[(4-Bromo-2-nitrophenyl)methylamino]azepan-2-one (790 mg) was dissolved in ethanol (8 mL), tin(II) chloride (1.76 g) was added, and the mixture was stirred at 70 °C for 4 hours. Saturated aqueous potassium carbonate was added to the reaction mixture, followed by extraction with chloroform, and then drying over anhydrous sodium sulfate, and the solvent was evaporated under reduced pressure. The residue and N,N-diisopropylethylamine (0.40 mL) were dissolved in tetrahydrofuran (20 mL), and triphosgene (227 mg) was added under ice-cooling. The mixture was stirred for 30 minutes. Water was added to the reaction mixture, followed by extraction with chloroform, and then drying over anhydrous sodium sulfate, and the solvent was evaporated under reduced pressure. The residue was purified by silica gel column chromatography (ethyl acetate:methanol = 100:0 to 95:5) to obtain the title compound (290 mg) as a pale yellow powder. MS (ESI) m/z: 338.1/340.1 (M+H)⁺

### Reference Example 261-3

### 7-Bromo-1-methyl-3-(2-oxoazepan-3-yl)-4H-quinazolin-2-one (Reference Example Compound 261-3)

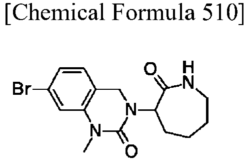

7-Bromo-3-(2-oxoazepan-3-yl)-1,4-dihydroquinazolin-2-one (289 mg) was suspended in N,N-dimethylformamide (3 mL), and cesium carbonate (420 mg) and iodomethane (158 mg) were added, followed by stirring at room temperature for 16 hours. Iodomethane (85 mg) was added, and the mixture was stirred at room temperature for 7 hours. Cesium carbonate (200 mg) was added, and the mixture was stirred at room temperature for 24 hours. Iodomethane (122 mg) was added, and the mixture was stirred at room temperature for 91 hours. Water was added to the reaction mixture, followed by extraction with ethyl acetate, and washing with water and saturated brine. The organic layer was dried over anhydrous sodium sulfate, and the solvent was evaporated under reduced pressure. The residue was purified by silica gel column chromatography (ethyl acetate:methanol = 100:0 to 95:5) to obtain the title compound (220 mg) as a colorless powder. MS (ESI) m/z: 352.1/354.1 (M+H)⁺

### Example 261

### 7-Bromo-3-(2,7-dioxoazepan-3-yl)-1-methylquinazoline-2,4-dione (Example Compound 261)

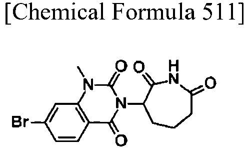

Using 7-bromo-1-methyl-3-(2-oxoazepan-3-yl)-4H-quinazolin-2-one instead of tert-butyl 2-[2-fluoro-3-[(2-oxoazepan-3-yl)carbamoyl]phenoxy]acetate in Example 67, the same reaction and processing were performed to obtain the title compound (8 mg) as a colorless powder. MS (ESI) m/z: 380.1/382.1 (M+H)⁺

The corresponding starting compounds were processed in the same manner as in Reference Example and Example 135 to obtain the compounds listed in the table below.

**[Table 18]**

| Example | Structural formula | Physical properties etc. |
|---|---|---|
| 262 | 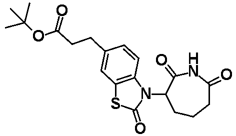 tert-Butyl 3-[3-(2,7-dioxoazepan-3-yl)-2-oxo-1,3-benzothiazol-6-yl]propanoate | Colorless powder |
| | | MS (ESI) m/z: 349.1 (M-tBu+H)⁺ |
| 263 | 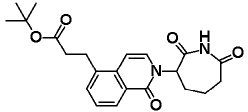 tert-Butyl 3-[2-(2,7-dioxoazepan-3-yl)-1-oxoisoquinolin-5-yl]propanoate | Colorless powder |
| | | MS (ESI) m/z: 399.3 (M+H)⁺ |
| 264 | 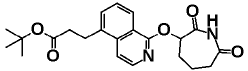 tert-Butyl 3-[1-(2,7-dioxoazepan-3-yl)oxyisoquinolin-5-yl]propanoate | Colorless powder |
| | | MS (ESI) m/z: 343.1 (M-tBu+H)⁺ |
| 265 | 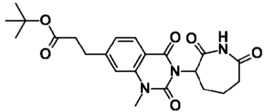 tert-Butyl 3-[3-(2,7-dioxoazepan-3-yl)-1-methyl-2,4-dioxoquinazolin-7-yl]propanoate | Colorless powder |
| | | MS (ESI) m/z: 430.3 (M+H)⁺ |
| 266 | 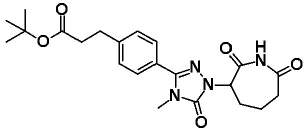 tert-Butyl 3-[4-[1-(2,7-dioxoazepan-3-yl)-4-methyl-5-oxo-1,2,4-triazol-3-yl]phenyl]propanoate | Colorless powder |
| | | MS (ESI) m/z: 429.3 (M+H)⁺ |

### Example 267

### 3-(4-Bromophenoxy)azepane-2,7-dione (Example Compound 267)

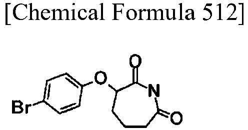

Using 3-(4-bromophenoxy)azepan-2-one instead of Reference Example Compound 1 in Reference Example and Example 1, the same reaction and processing as in (1-2) were performed to obtain the title compound as a white solid. MS (ESI) m/z: 297.9/299.8 (M+H)⁺.

### Example and Reference Example 268

### tert-Butyl 6-(7-oxoazepan-3-yl)oxypyridine-3-carboxylate (Reference Example Compound 268)

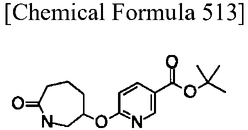

To an N,N-dimethylformamide (2 mL) solution of 6-hydroxyazepan-2-one (50.0 mg) and tert-butyl 6-fluoropyridine-3-carboxylate (84.0 mg), potassium tert-butoxide (0.770 mL, 1.0M tetrahydrofuran solution) was added, and the mixture was stirred at room temperature for 6 hours. Saturated aqueous ammonium chloride was added to the reaction mixture, followed by stirring, and the mixture was extracted twice with ethyl acetate. The organic layer was washed twice with water and then concentrated under reduced pressure. The residue was purified by silica gel column chromatography (methanol:chloroform = 0:100 to 5:95) to obtain the title compound (54.2 mg) as a pale yellow solid. MS (ESI) m/z: 307.0 (M+H)⁺.

### tert-Butyl 6-(2,7-dioxoazepan-3-yl)oxypyridine-3-carboxylate (Example Compound 268)

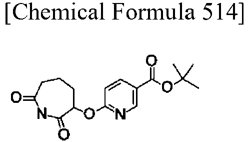

Using Reference Example Compound 268 instead of Reference Example Compound 1 in Reference Example and Example 1, the same reaction and processing as in (1-2) were performed to obtain the title compound as a white solid. MS (ESI) m/z: 321.3 (M+H)⁺.

### Example 269

### 2-(1-Pyrimidin-2-ylpiperidine-4-carbonyl)-2,7-diazaspiro[4.6]undecane-6,8-dione (Example Compound 269)

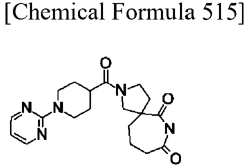

To a chloroform (1 mL) solution of tert-butyl 6,8-dioxo-2,7-diazaspiro[4.6]undecane-2-carboxylate (30.0 mg), trifluoroacetic acid (1 mL) was added, and the mixture was stirred at room temperature for 1 hour. The reaction mixture was concentrated under reduced pressure, and azeotropic distillation with toluene was performed twice. The residue, 1-(2-pyrimidinyl)-4-piperidinecarboxylic acid (24.2 mg), HATU (60.6 mg), and N,N-diisopropylethylamine (0.919 mL) were stirred in N,N-dimethylformamide (3 mL) at room temperature for 3 hours. The reaction mixture was concentrated under reduced pressure, and the residue was purified by silica gel column chromatography (methanol:chloroform = 0:100 to 5:95) to obtain the title compound (33.2 mg) as a white solid. MS (ESI) m/z: 372.1 (M+H)⁺.

### Example 270

### tert-Butyl N-[6-[2-chloro-4-[4-(2,7-dioxoazepan-3-yl)-3-oxopiperazine-1-carbonyl]phenyl]hexyl]carbamate (Example Compound 270)

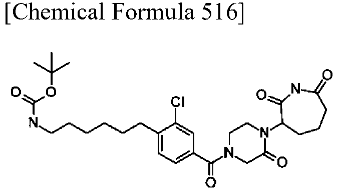

Using 1-[6-(tert-butoxycarbonylamino)hexyl]-3-chlorobenzoic acid instead of 1-(2-pyrimidinyl)-4-piperidinecarboxylic acid and Example Compound 29-1 instead of tert-butyl 6,8-dioxo-2,7-diazaspiro[4.6]undecane-2-carboxylate in Example 269, the same reaction and processing were performed to obtain the title compound as a colorless solid. MS (ESI) m/z: 463.2/465.2 (M-Boc+H)⁺.

### Example 271

### tert-Butyl N-[6-[4-[(2,7-dioxoazepan-3-yl)-methylsulfamoyl]phenyl]hexyl]carbamate (Example Compound 271)

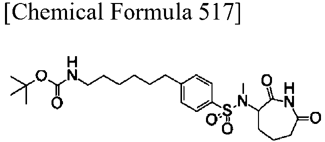

Using 3-(methylamino)azepan-2-one instead of DL-α-amino-ε-caprolactam and tert-butyl N-[6-(4-chlorosulfonylphenyl)hexyl]carbamate instead of 4-chlorobenzenesulfonyl chloride in Reference Example and Example 7, the same reactions and processing as in (7-1) and (7-3) were performed to obtain the title compound as a white solid. MS (ESI) m/z: 494.2 [M-H]⁻.

### Example 272

### 3-[4-(4-Methylpiperazin-1-yl)-6-oxopyridazin-1-yl]azepane-2,7-dione (Example Compound 272)

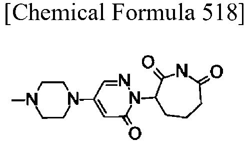

Example Compound 248 (43.5 mg) and 1-methylpiperazine (0.0225 mL) were stirred in N,N-dimethylformamide (2 mL) at room temperature for 7 hours. The reaction mixture was concentrated under reduced pressure, the residue was suspended in chloroform, saturated aqueous sodium bicarbonate was added, and the mixture was stirred at room temperature. The chloroform layer was separated, concentrated, and then the crude product was purified by silica gel column chromatography (methanol:chloroform = 0:100 to 10:90) to obtain the title compound (21.3 mg) as a white solid. MS (ESI) m/z: 320.0 (M+H)⁺.

### Example 273

### (273-1) tert-Butyl 6-(2,7-dioxoazepan-3-yl)-5,7-dioxo-2,6-diazaspiro[3.4]octane-2-carboxylate (Example Compound 273-1)

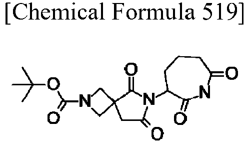

A solution of 1-tert-butoxycarbonyl-3-(2-methoxy-2-oxoethyl)azetidine-3-carboxylic acid (9 g) and lithium hydroxide hydrate (3.45 g) in tetrahydrofuran (30 mL) and water (60 mL) was stirred at room temperature for 16 hours. 1M hydrochloric acid was added to adjust the pH to 4, and the mixture was extracted twice with ethyl acetate. The organic layer was concentrated under reduced pressure to obtain a white solid (7 g). The resulting white solid (1.0 g) was stirred in acetic anhydride (10 mL) at 120 °C for 30 minutes. The reaction mixture was concentrated under reduced pressure, pyridine (20 mL) and 3-aminoazepane-2,7-dione hydrochloride (1.8 g) were added, and the mixture was stirred at 140 °C for 2 hours, and then concentrated under reduced pressure. To the tetrahydrofuran solution of the residue, 1,1'-carbonyldiimidazole (0.75 g) and 4-dimethylaminopyridine (0.05 g) were added, and the mixture was stirred at 60 °C for 2 hours. The reaction mixture was concentrated under reduced pressure, and the residue was purified by reverse-phase chromatography to obtain the title compound (278 mg) as a white solid. MS (ESI) m/z: 309.8 (M+H-Boc)⁺.

### (273-2) Ethyl 2-[6-(2,7-dioxoazepan-3-yl)-5,7-dioxo-2,6-diazaspiro[3.4]octan-2-yl]-1,3-benzothiazole-6-carboxylate (Example Compound 273-2)

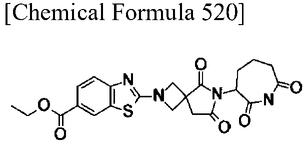

Using Example Compound 273-1 in Example 52, the same reaction and processing were performed to obtain the title compound as a white solid. MS (ESI) m/z: 471.0 (M+H)⁺.

### Example and Reference Example 274

### (274-1) 3-[2-(Hydroxymethyl)-4-nitroanilino]azepan-2-one (Reference Example Compound 274-1)

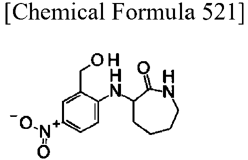

(2-Fluoro-5-nitrophenyl)methanol (1 g), 3-aminoazepan-2-one (824 mg), and potassium carbonate (1.62 g) were stirred in dimethyl sulfoxide (5 mL) at 100 °C for 8 hours. Water was added to the reaction mixture, followed by stirring, and the mixture was extracted twice with ethyl acetate. The organic layer was washed twice with water and then concentrated under reduced pressure. The residue was purified by silica gel column chromatography (methanol:ethyl acetate = 0:100 to 5:95) to obtain the title compound (1.03 g) as a yellow solid. MS (ESI) m/z: 280.0 (M+H)⁺.

### (274-2) 6-Nitro-1-(2-oxoazepan-3-yl)-4H-3,1-benzoxazin-2-one (Reference Example Compound 274-2)

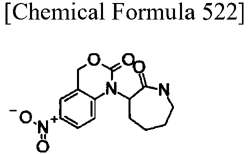

Reference Example Compound 274-1 (300 mg) and 1,1'-carbonyldiimidazole (174 mg) were heated under reflux in acetonitrile (5 mL) for 6 hours. The reaction mixture was concentrated under reduced pressure, and the residue was purified by silica gel column chromatography (ethyl acetate:hexane = 50:50 to 100:0) to obtain the title compound (152 mg) as a yellow solid. MS (ESI) m/z: 305.9 (M+H)⁺.

### (274-3) 3-(6-Nitro-2-oxo-4H-3,1-benzoxazin-1-yl)azepane-2,7-dione (Example Compound 274)

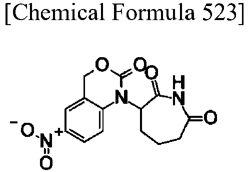

Using Reference Example Compound 274-2 instead of Reference Example Compound 1 in Reference Example and Example 1, the same reaction and processing as in (1-2) were performed to obtain the title compound as a pale yellow solid. MS (ESI) m/z: 319.9 (M+H)⁺.

### Example and Reference Example 275

### (275-1) tert-Butyl 2-[2-[(2-oxoazepan-3-yl)amino]anilino]acetate (Reference Example Compound 275-1)

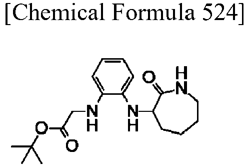

3-(2-Aminoanilino)azepan-2-one (300 mg), tert-butyl bromoacetate (0.211 mL), and potassium carbonate (284 mg) were stirred in N,N-dimethylformamide (5 mL) at room temperature for 2 hours. Water was added to the reaction mixture, followed by stirring, and the mixture was extracted twice with ethyl acetate. The organic layer was washed twice with water and then concentrated under reduced pressure. The residue was purified by silica gel column chromatography (ethyl acetate:hexane = 30:70 to 70:30) to obtain the title compound (332 mg) as a yellow solid. MS (ESI) m/z: 334.0 (M+H)⁺.

### (275-2) tert-Butyl 2-[2,3-dioxo-4-(2-oxoazepan-3-yl)quinoxalin-1-yl]acetate (Reference Example Compound 275-2)

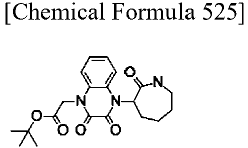

A tetrahydrofuran (10 mL) solution of Reference Example Compound 275-1 (332 mg) was cooled to 0 °C, and N,N-diisopropylethylamine (0.517 mL) and ethyl chloroglyoxylate (0.122 mL) were added. The mixture was stirred at room temperature for 2 hours and then heated under reflux for 12 hours. The reaction mixture was concentrated under reduced pressure, and the residue was purified by silica gel column chromatography (methanol:chloroform = 0:100 to 5:95) to obtain the title compound (159 mg) as a white solid.

MS (ESI) m/z: 388.0 (M+H)⁺.

### (275-3) tert-Butyl 2-[4-(2,7-dioxoazepan-3-yl)-2,3-dioxoquinoxalin-1-yl]acetate (Example Compound 275)

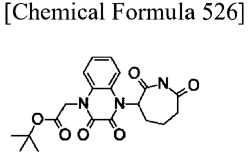

Using Reference Example Compound 275-2 instead of Reference Example Compound 1 in Reference Example and Example 1, the same reaction and processing as in (1-2) were performed to obtain the title compound as a white solid. MS (ESI) m/z: 402.0 (M+H)⁺.

### Example and Reference Example 276

### (276-1) tert-Butyl 4-[4-(2-oxoazepan-3-yl)oxyphenyl]piperidine-1-carboxylate (Reference Example Compound 276)

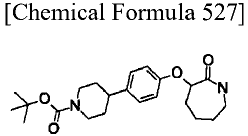

Using 3-(4-bromophenoxy)azepan-2-one instead of Example Compound 127 in Example 168, the same reactions and processing as in (168-1) and (168-2) were performed to obtain the title compound as a pale yellow solid. MS (ESI) m/z: 333.0 (M-tBu+H)⁺.

### (276-2) tert-Butyl 4-[4-(2-oxoazepan-3-yl)oxyphenyl]piperidine-1-carboxylate (Example compound 276)

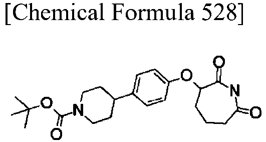

Using Reference Example Compound 276 instead of Reference Example Compound 1 in Reference Example and Example 1, the same reaction and processing as in (1-2) were performed to obtain the title compound as a white solid. MS (ESI) m/z: 347.3 (M-tBu+H)⁺.

### Example 277

### Benzyl 2-[4-[6-(2,6-dioxopiperidin-3-yl)-5,7-dioxo-2,6-diazaspiro[3.4]octan-2-yl]piperidin-1-yl]acetate (Example Compound 277)

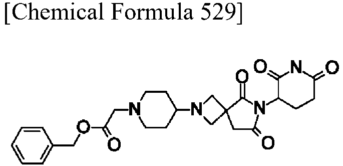

To a chloroform (1 mL) solution of Example Compound 44-1 (50.0 mg), trifluoroacetic acid (1 mL) was added, and the mixture was stirred at room temperature for 1 hour. The reaction mixture was concentrated under reduced pressure, and azeotropic distillation with toluene was performed twice. To a tetrahydrofuran (3 mL) solution of the residue and benzyl 2-(4-oxo-1-piperidyl)acetate (34.8 mg), sodium triacetoxyborohydride (54.3 mg) was added, and the mixture was stirred at room temperature for 3 hours. Saturated aqueous sodium bicarbonate and chloroform were added to the reaction mixture, followed by stirring. The aqueous layer was removed using a phase separator, and the organic layer was concentrated under reduced pressure. The residue was purified by silica gel column chromatography (methanol:chloroform = 0:100 to 5:95) to obtain the title compound (39.8 mg) as a white solid. MS (ESI) m/z: 483.0 (M+H)⁺.

### Example 278

### tert-Butyl N-[2-[4-[[6-(2,6-dioxopiperidin-3-yl)-5,7-dioxo-2,6-diazaspiro[3.4]octan-2-yl]sulfonyl]phenyl]ethyl]carbamate (Example Compound 278)

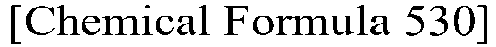

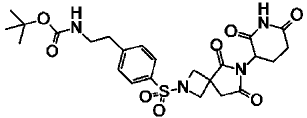

To a chloroform (1 mL) solution of Example Compound 44-1 (30.0 mg), trifluoroacetic acid (1 mL) was added, and the mixture was stirred at room temperature for 2 hours. The reaction mixture was concentrated under reduced pressure, and azeotropic distillation with toluene was performed twice. The residue, tert-butyl N-[2-(4-chlorosulfonylphenyl)ethyl]carbamate (27.0 mg), and pyridine (0.0310 mL) were stirred in tetrahydrofuran (3 mL) at 50 °C for 6 hours. 1M hydrochloric acid was added to the reaction mixture, and the mixture was stirred, and extracted twice with ethyl acetate. The organic layer was washed twice with water and then concentrated under reduced pressure. The residue was purified by silica gel column chromatography (methanol:chloroform = 0:100 to 5:95) to obtain the title compound (12.5 mg) as a white solid. MS (ESI) m/z: 533.2 [M-H]⁻.

### Example and Reference Example 279

### (279-1) Methyl 5-[4-[methyl-(2-oxoazepan-3-yl)sulfamoyl]phenyl]pentanoate (Reference Example Compound 279-1)

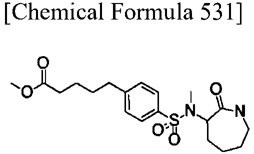

Using 3-(methylamino)azepan-2-one instead of DL-α-amino-ε-caprolactam and methyl 5-(4-chlorosulfonylphenyl)pentanoate instead of 4-chlorobenzenesulfonyl chloride in Reference Example and Example 7, the same reaction and processing as in (7-1) were performed to obtain the title compound as a colorless viscous material. MS (ESI) m/z: 397.0 (M+H)⁺.

### (279-2) tert-Butyl 5-[4-[methyl-(2-oxoazepan-3-yl)sulfamoyl]phenyl]pentanoate (Reference Example Compound 279-2)

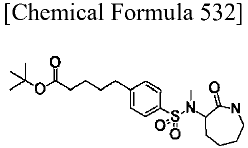

To a tetrahydrofuran (2 mL) and methanol (2 mL) solution of Reference Example Compound 279-1 (99.5 mg), 1M aqueous sodium hydroxide (0.753 mL) was added, and the mixture was stirred at room temperature for 4 hours. The reaction mixture was neutralized with 1M hydrochloric acid and concentrated under reduced pressure. To a dichloromethane (5 mL) solution of the residue, 2-tert-butyl-1,3-diisopropylisourea (0.299 mL) was added, and the mixture was stirred at room temperature for 3 hours. Additional 2-tert-butyl-1,3-diisopropylisourea (0.299 mL) was added, and stirring was continued for 6 hours. The reaction mixture was concentrated under reduced pressure, and the residue was purified by silica gel column chromatography (methanol:chloroform = 0:100 to 5:95) to obtain the title compound (76.9 mg) as a colorless viscous material. MS (ESI) m/z: 439.0 (M+H)⁺.

### (279-3) tert-Butyl 5-[4-[(2,7-dioxoazepan-3-yl)-methylsulfamoyl]phenyl]pentanoate (Example Compound 279)

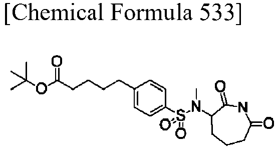

Using Reference Example Compound 279-2 instead of Reference Example Compound 1 in Reference Example and Example 1, the same reaction and processing as in (1-2) were performed to obtain the title compound as a colorless viscous material. MS (ESI) m/z: 451.2 [M-H]⁻.

### Reference Example and Example 280

### Reference Example 280-1

### 1-(4-Nitrophenyl)-3-(prop-2-enylamino)pyrrolidin-2-one (Reference Example Compound 280-1)

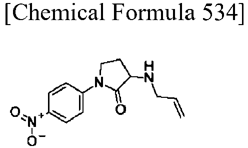

4-Nitroaniline (2.00 g) and potassium phosphate (1.54 g) were suspended in acetonitrile (40 mL), and 2,4-dibromobutanoyl chloride (4.25 g) was added under ice-cooling. The mixture was stirred at room temperature for 1 hour. Potassium carbonate (4.00 g) was added to the reaction mixture, followed by stirring at room temperature for 1 hour. Water was added to the reaction mixture, and the mixture was extracted with ethyl acetate. The organic layer was washed with water and saturated brine, dried over sodium sulfate, filtered, and the filtrate was concentrated under reduced pressure. The residue was suspended and washed with diisopropyl ether, filtered, and dried under reduced pressure. The residue was dissolved in N,N-dimethylformamide (30 mL), and N,N-diisopropylethylamine (6.75 g) and allylamine hydrochloride (3.05 g) were added, followed by stirring at 90 °C for 7 hours. Water was added to the reaction mixture, and the mixture was extracted with ethyl acetate three times. The organic layer was washed with water and saturated brine, dried over sodium sulfate, filtered, and the filtrate was concentrated under reduced pressure. The residue was purified by silica gel column chromatography (chloroform:methanol = 100:0 to 94:6). The residue was further purified by silica gel column chromatography (hexane:ethyl acetate = 50:50 to 0:100, ethyl acetate:methanol = 95:5) to obtain the title compound (2.38 g) as a yellow powder. MS (ESI) m/z: 262.2 (M+H)⁺

### Reference Example 280-2

### N-[[1-(4-Nitrophenyl)-2-oxopyrrolidin-3-yl]-prop-2-enylcarbamoyl]prop-2-enamide (Reference Example Compound 280-2)

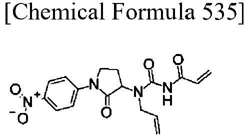

Acryloyl chloride (642 µL) and silver cyanate (1.15 g) were added to diethyl ether (10 mL) and the mixture was stirred at 40 °C for 1.5 hours. The reaction mixture was passed through a phase separator to remove insoluble matter while being added to a dichloromethane (10 mL) solution of 1-(4-nitrophenyl)-3-(prop-2-enylamino)pyrrolidin-2-one (1.00 g). The mixture was stirred at room temperature for 1 hour. The reaction mixture was concentrated, and the residue was purified by silica gel column chromatography (hexane:ethyl acetate = 50:50 to 0:100) to obtain the title compound (1.22 g) as a colorless powder. MS (ESI) m/z: 359.1 (M+H)⁺

### Example 280

### 3-[1-(4-Nitrophenyl)-2-oxopyrrolidin-3-yl]-4H-1,3-diazepine-2,7-dione (Example Compound 280)

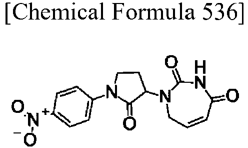

N-[[1-(4-Nitrophenyl)-2-oxopyrrolidin-3-yl]-prop-2-enylcarbamoyl]prop-2-enamide (300 mg) was dissolved in dichloroethane (150 mL), and second-generation Grubbs catalyst (72 mg) was added, followed by stirring at 80 °C for 2 hours. Additional second-generation Grubbs catalyst (18 mg) was added, and the mixture was stirred at 80 °C for 5 hours. Dimethyl sulfoxide (300 µL) was added to the reaction mixture, followed by strring at room temperature for 6 hours. The reaction mixture was concentrated, and ethyl acetate was added thereto, followed by suspension washing, filtering, and drying under reduced pressure to obtain the title compound (155 mg) as a brown powder. MS (ESI) m/z: 331.1 (M+H)⁺

### Reference Example and Example 281

### Reference Example 281-1

### 4-Bromo-2-fluoro-N-prop-2-enylbenzenecarbothioamide (Reference Example Compound 281-1)

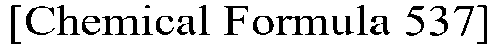

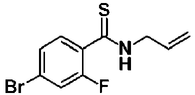

Allylamine hydrochloride (2.15 g), 4-bromo-2-fluorobenzoic acid (5.00 g), and O-(7-azabenzotriazol-1-yl)-N,N,N',N'-tetramethyluronium hexafluorophosphate (8.70 g) were dissolved in N,N-dimethylformamide (40 mL), and N,N-diisopropylethylamine (7.9 mL) was added, followed by stirring at room temperature for 1 hour. Water (about 50 mL) was added to the reaction mixture, and the precipitate was filtered and dried under reduced pressure. The residue was dissolved in toluene (50 mL), Lawesson's reagent (4.30 g) was added, and the mixture was stirred at 60 °C for 3 hours. The reaction mixture was concentrated, and the residue was purified by silica gel column chromatography (hexane:ethyl acetate = 90:10 to 75:25) to obtain the title compound (3.94 g) as a yellow viscous material. MS (ESI) m/z: 274.0/276.0 (M+H)⁺

### Reference Example 281-2

### 6-Bromo-1-methyl-N-prop-2-enylindazol-3-amine (Reference Example Compound 281-2)

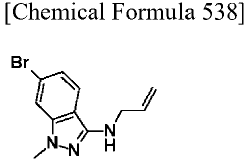

4-Bromo-2-fluoro-N-prop-2-enylbenzenecarbothioamide (3.94 g) was dissolved in ethanol (30 mL), and methylhydrazine (8.80 g) was added. The mixture was stirred at 80 °C for 22 hours. The reaction mixture was concentrated, and the residue was purified by silica gel column chromatography (hexane:ethyl acetate = 95:5 to 75:25) to obtain the title compound (1.68 g) as a pale yellow powder. MS (ESI) m/z: 266.1/268.1 (M+H)⁺

### Reference Example 281-3

### N-[(6-Bromo-1-methylindazol-3-yl)-prop-2-enylcarbamoyl]prop-2-enamide (Reference Example Compound 281-3)

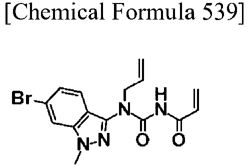

Using 6-bromo-1-methyl-N-prop-2-enylindazol-3-amine instead of 1-(4-nitrophenyl)-3-(prop-2-enylamino)pyrrolidin-2-one in Reference Example 280-2, the same reaction and processing were performed to obtain the title compound as a colorless powder. MS (ESI) m/z: 363.1/365.1 (M+H)⁺

### Reference Example 281-4

### N-[(6-Bromo-1-methylindazol-3-yl)-prop-2-enylcarbamoyl]-N-(2-trimethylsilylethoxymethyl)prop-2-enamide (Reference Example Compound 281-4)

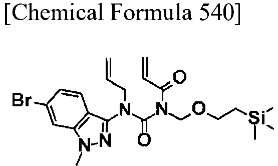

N-[(6-Bromo-1-methylindazol-3-yl)-prop-2-enylcarbamoyl]prop-2-enamide (390 mg) was dissolved in dichloromethane (4 mL), and N,N-diisopropylethylamine (0.89 mL) and 2-(chloromethoxy)ethyl-trimethylsilane (0.72 mL) were added, followed by stirring at room temperature for 16 hours. Water was added to the reaction mixture, and the mixture was extracted with chloroform. The organic layer was dried over sodium sulfate, filtered, and the filtrate was concentrated under reduced pressure. The residue was purified by silica gel column chromatography (hexane:ethyl acetate = 95:5 to 80:20) to obtain the title compound (299 mg) as a pale yellow powder. MS (ESI) m/z: 493.2/495.2 (M+H)⁺

### Reference Example 281-5

### 3-(6-Bromo-1-methylindazol-3-yl)-1-(2-trimethylsilylethoxymethyl)-4H-1,3-diazepine-2,7-dione (Reference Example Compound 281-5)

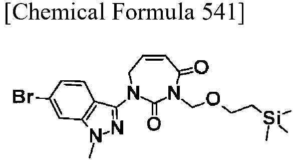

N-[(6-Bromo-1-methylindazol-3-yl)-prop-2-enylcarbamoyl]-N-(2-trimethylsilylethoxymethyl)prop-2-enamide (300 mg) was dissolved in dichloroethane (60 mL), and second-generation Hoveyda-Grubbs catalyst (77 mg) was added, followed by stirring at 60 °C for 4 hours. Additional second-generation Hoveyda-Grubbs catalyst (77 mg) was added, and the mixture was stirred at 60 °C for 5 hours. The reaction mixture was concentrated, and the residue was purified by silica gel column chromatography (hexane:ethyl acetate = 95:5 to 65:35) to obtain the title compound (146 mg) as a brown viscous material. MS (ESI) m/z: 465.2/467.2 (M+H)⁺

### Example 281

### 3-(6-Bromo-1-methylindazol-3-yl)-4H-1,3-diazepine-2,7-dione (Example Compound 281)

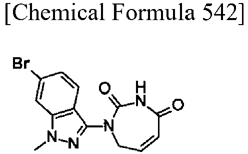

3-(6-Bromo-1-methylindazol-3-yl)-1-(2-trimethylsilylethoxymethyl)-4H-1,3-diazepine-2,7-dione (200 mg) was dissolved in dichloromethane (1 mL), and trifluoroacetic acid (1 mL) was added, followed by stirring at room temperature for 0.5 hours. The reaction mixture was concentrated and azeotroped with toluene. The residue was dissolved in methanol (1 mL), and N,N-diisopropylethylamine (372 µL) was added, followed by stirring at room temperature for 0.5 hours. Water was added to the reaction mixture, and the mixture was extracted with ethyl acetate. The organic layer was washed with water and saturated brine, dried over sodium sulfate, filtered, and the filtrate was concentrated under reduced pressure. The residue was purified by silica gel column chromatography (hexane:ethyl acetate = 70:30 to 20:80) to obtain the title compound (97 mg) as a colorless powder. MS (ESI) m/z: 335.0/337.0 (M+H)⁺

### Reference Example 282

### Reference Example 282-1

### 4-(1-tert-Butoxycarbonyl-4-piperidyl)-2-fluorobenzoic acid (Reference Example Compound 282-1)

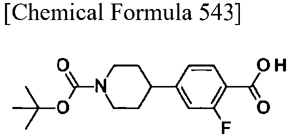

Using methyl 4-bromo-2-fluorobenzoate instead of 5-iodo-3-methyl-1-(2-oxoazepan-3-yl)benzimidazol-2-one in Reference Example 218-1, the same reactions and processing as in (Reference Example 218-1), (Reference Example 218-2), and (Reference Example 66-2) were performed to obtain the title compound as a colorless powder. MS (ESI) m/z: 244.1 (M-Boc+H)⁺

### Reference Example 282-2

### tert-Butyl 4-[3-(allylamino)-1-indazol-6-yl]piperidine-1-carboxylate (Reference Example Compound 282-2)

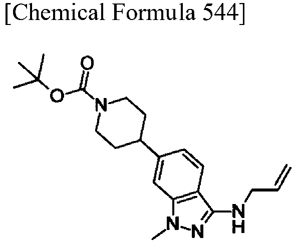

Using 4-(1-tert-butoxycarbonyl-4-piperidyl)-2-fluorobenzoic acid instead of 4-bromo-2-fluorobenzoic acid in Reference Example 281-1, the same reactions and processing as in (Reference Example 281-1) and (Reference Example 281-1) were performed to obtain the title compound as a pale yellow powder. MS (ESI) m/z: 371.2 (M+H)⁺

### Reference Example 283

### Reference Example 283-1

### 3-[(2,4-Dimethoxyphenyl)methyl-prop-2-enylamino]-3-methyl-1H-indol-2-one (Reference Example Compound 283-1)

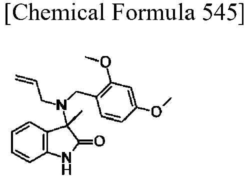

3-Bromo-3-methyl-1H-indol-2-one (1.50 g), N-[(2,4-dimethoxyphenyl)methyl]prop-2-en-1-amine (1.66 g), and cesium carbonate (6.50 g) were added to dichloromethane (20 mL) and the mixture was stirred at room temperature for 22 hours. Water was added to the reaction mixture, and the mixture was extracted with chloroform. The organic layer was dried over sodium sulfate, filtered, and the filtrate was concentrated under reduced pressure. The residue was purified by silica gel column chromatography (hexane:ethyl acetate = 90:10 to 80:20 to 70:30) to obtain the title compound (2.20 g) as a colorless powder. MS (ESI) m/z: 351.3 (M-H)⁻

### Reference Example 283-2

### Benzyl 2-[3-methyl-2-oxo-3-(prop-2-enylamino)indol-1-yl]acetate (Reference Example Compound 283-2)

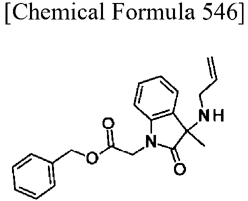

3-[(2,4-Dimethoxyphenyl)methyl-prop-2-enylamino]-3-methyl-1H-indol-2-one (500 mg) was dissolved in N,N-dimethylformamide (5 mL), and potassium tert-butoxide (200 mg) and benzyl 2-bromoacetate (340 mg) were added. The mixture was stirred at room temperature for 1 hour. Water was added to the reaction mixture, and the mixture was extracted with ethyl acetate. The organic layer was washed with water and saturated brine, dried over sodium sulfate, filtered, and the filtrate was concentrated under reduced pressure. The residue was purified by silica gel column chromatography (hexane:ethyl acetate = 70:30 to 20:80) to obtain benzyl 2-[3-[(2,4-dimethoxyphenyl)methyl-prop-2-enylamino]-3-methyl-2-oxoindol-1-yl]acetate (555 mg) as a colorless viscous material. Anisole (360 mg) and trifluoroacetic acid (5 mL) were added, and the mixture was stirred at room temperature for 0.5 hours, and then at 50 °C for 2 hours. The reaction mixture was concentrated, and the residue was purified by silica gel column chromatography (hexane:ethyl acetate = 80:20 to 0:100) to obtain the title compound (341 mg) as a colorless viscous material. MS (ESI) m/z: 351.2 (M+H)⁺

The corresponding starting compounds were processed in the same manner as in Reference Example 283-1 to obtain the compounds shown in the table below.

**[Table 19]**

| Reference Example | Structural formula | Physical properties etc. |
|---|---|---|
| 284-1 | 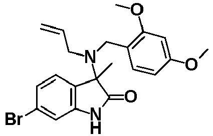 3-[(2,4-Dimethoxyphenyl)methyl-prop-2-enylamino]-6-bromo-3-methyl-1H-indol-2-one | Colorless viscous material |
| | | MS (ESI) m/z: 429. 3/431. 3 (M-H)⁻ |
| 285-1 | 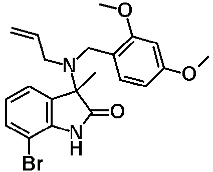 | Colorless viscous material |
| | | MS (ESI) m/z: 429. 3/431. 3 (M-H)⁻ |
| | 3-[(2,4-Dimethoxyphenyl)methyl-prop-2-enylamino]-7-bromo-3-methyl-1H-indol-2-one | |

The corresponding starting compounds were processed in the same manner as in Reference Example 283-2, using iodomethane instead of benzyl 2-bromoacetate, to obtain the compounds listed in the table below.

**[Table 20]**

| Reference Example | Structural formula | Physical properties etc. |
|---|---|---|
| 284-2 | 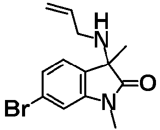 3-(Allylamino)-6-bromo-1,3-dimethylindolin-2-one | Colorless powder |
| | | MS (ESI) m/z: 295.0/297.0 (M+H)⁺ |
| 285-2 | 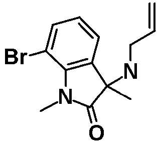 3-(Allylamino)-7-bromo-1,3-dimethylindolin-2-one | Colorless powder |
| | | MS (ESI) m/z: 295. 1/297. 1 (M+H)⁺ |

The corresponding starting compounds were processed in the same manner as in Reference Example 281-3, Reference Example 281-4, Reference Example 281-5, and Example 281, to obtain the compounds listed in the table below.

**[Table 21]**

| Example | Structural formula | Physical properties etc. |
|---|---|---|
| 282 | 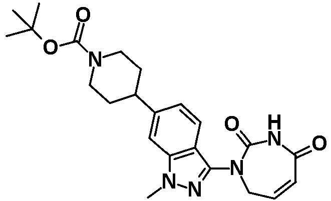 tert-Butyl 4-[3-(2,7-dioxo-4H-1,3-diazepin-3-yl)-1-methyl-indazol-6-yl]piperidine-1-carboxylate | Colorless powder |
| | | MS (ESI) m/z: 384.1 (M-tBu+H)⁺ |
| 283 | 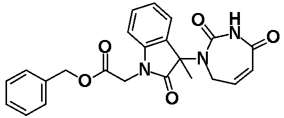 Benzyl 2-[3-(2,7-dioxo-4H-1,3-diazepin-3-yl)-3-methyl-2-oxoindol-1-yl]acetate | Colorless powder |
| | | MS (ESI) m/z: 420.2 (M+H)⁺ |
| 284 | 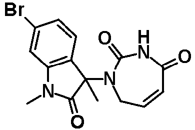 3-(6-bromo-1,3-dimethyl-2-oxoindolin-3-yl)-4H-1,3-diazepine-2,7-dione | Colorless powder |
| | | MS (ESI) m/z: 364.0/366.0 (M+H)⁺ |
| 285 | 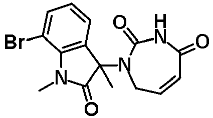 3-(7-bromo-1,3-dimethyl-2-oxoindolin-3-yl)-4H-1,3-diazepine-2,7-dione | Colorless powder |
| | | MS (ESI) m/z: 364.0/366.0 (M+H)⁺ |
| 286 | 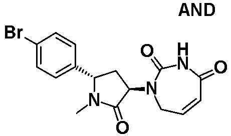 3-[(3R*,5S*)-5-(4-bromophenyl)-1-methyl-2-oxopyrrolidin-3-yl]-4H-1,3-diazepine-2,7-dione | Colorless powder |
| | | MS (ESI) m/z: 378.0/380.0 (M+H)⁺ |

The corresponding starting compounds were processed in the same manner as in Example 136 to obtain the compounds listed in the table below.

**[Table 22]**

| Example | Structural formula | Physical properties etc. |
|---|---|---|
| 287 | 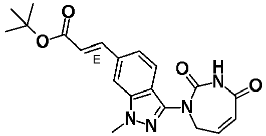 tert-Butyl (E)-3-[3-(2,7-dioxo-4H-1,3-diazepin-3-yl)-1-methylindazol-6-yl]prop-2-enoate | Pale yellow powder |
| | | MS (ESI) m/z: 383.3 (M+H)⁺ |
| 288 | 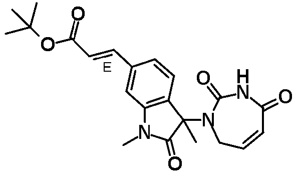 tert-Butyl (E)-3-[3-(2,7-dioxo-4H-1,3-diazepin-3-yl)-1,3-dimethyl-2-oxoindolin-6-yl]prop-2-enoate | Colorless powder |
| | | MS (ESI) m/z: 412.2 (M+H)⁺ |
| 289 | 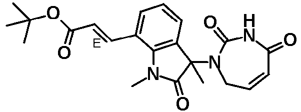 tert-Butyl (E)-3-[3-(2,7-dioxo-4H-1,3-diazepin-3-yl)-1,3-dimethyl-2-oxoindolin-7-yl]prop-2-enoate | Colorless powder |
| | | MS (ESI) m/z: 412.2 (M+H)⁺ |
| 290 | 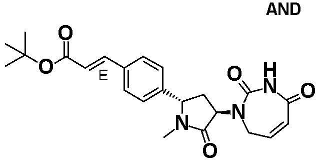 tert-Butyl (E)-3-[4-[(2S*, 4R*)-4-(2,7-dioxo-4H-1,3-diazepin-3-yl)-1-methyl-5-oxo-pyrrolidin-2-yl]phenyl]prop-2-enoate | Colorless powder |
| | | MS (ESI) m/z: 426.2 (M-tBu+H)⁺ |

The corresponding starting compounds were processed in the same manner as in Example 137 to obtain the compounds listed in the table below.

**[Table 23]**

| Example | Structural formula | Physical properties etc. |
|---|---|---|
| 291 | 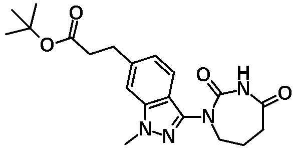 tert-Butyl 3-[3-(2,4-dioxo-1,3-diazepan-1-yl)-1-methylindazol-6-yl]propanoate | Colorless powder |
| | | MS (ESI) m/z: 387.2 (M+H)⁺ |
| 292 | 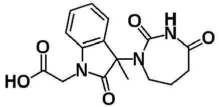 2-[3-(2,4-dioxo-1,3-diazepan-1-yl)-3-methyl-2-oxoindol-1-yl]acetic acid | Colorless powder |
| | | MS (ESI) m/z: 332.1 (M+H)⁺ |

### Example 293

### tert-Butyl 2-[4-[3-(2,7-dioxo-4H-1,3-diazepin-3-yl)-1-methyl-indazol-6-yl]-1-piperidyl]acetate (Example Compound 293)

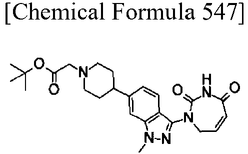

Using tert-butyl 4-[3-(2,7-dioxo-4H-1,3-diazepin-3-yl)-1-methyl-indazol-6-yl]piperidine-1-carboxylate instead of tert-butyl 6,8-dioxo-2,7-diazaspiro[4.6]undecane-2-carboxylate in Example 100, the same reaction and processing were performed to obtain the title compound as a colorless powder. MS (ESI) m/z: 454.2 (M+H)⁺

### Reference Example 286

### (286-1) (3S*,5S*)-3-Bromo-5-(4-bromophenyl)-1-methyl-pyrrolidin-2-one (Reference Example Compound 286-1)

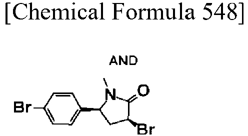

To a THF (210 mL) solution of 5-(4-bromophenyl)-1-methyl-pyrrolidin-2-one (21 g), lithium diisopropylamide (2M/THF, 103 mL) was added dropwise at -78 °C, and the mixture was stirred at -78 °C for 30 minutes. A THF (21 mL) solution of chlorotrimethylsilane (22.44 g) was added, and the mixture was stirred at -78 °C for 30 minutes. A THF (210 mL) solution of N-bromosuccinimide (36.8 g) was added to the reaction mixture, and the mixture was stirred at - 78 °C for 2 hours. The reaction mixture was quenched with MeOH, concentrated, and then, water was added, followed by extraction with ethyl acetate. The organic layer was concentrated, and the crude product was purified by silica gel column chromatography (petroleum ether/ethyl acetate = 4/1 to 0/1) to obtain the title compound (3.9 g) as a yellow solid. MS (ESI) m/z: 331.9/333.9/335.9 (M+H)⁺.

### (286-2) (3R*,5S*)-3-(Allylamino)-5-(4-bromophenyl)-1-methyl-pyrrolidin-2-one (Reference Example Compound 286-2)

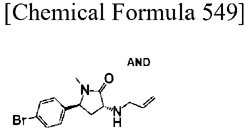

To a DMF (20 mL) solution of (3S*,5S*)-3-bromo-5-(4-bromophenyl)-1-methyl-pyrrolidin-2-one (3.9 g), cesium carbonate (7.63 g) and allylamine (1.34 g) were added, and the mixture was stirred at room temperature for 2 hours. Water was added to the reaction mixture, and the mixture was extracted with ethyl acetate. The organic layer was concentrated to half its original volume and then extracted with 1M hydrochloric acid. The aqueous layer was adjusted to pH 9 with potassium carbonate, and extracted with ethyl acetate, and then the organic layer was concentrated to obtain the title compound (1.17 g) as a yellow oil. MS (ESI) m/z: 309.1/311.1 (M+H)⁺.

### Example 294

### tert-Butyl 6-[[2-(6,8-dioxo-2,7-diazaspiro[4.6]undecan-2-yl)-1,3-benzothiazol-6-yl]amino]hexanoate (Example Compound 294)

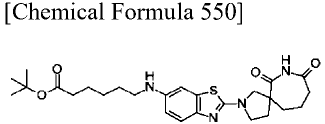

2-(6-Amino-1,3-benzothiazol-2-yl)-2,7-diazaspiro[4.6]undecane-6,8-dione (50 mg), tert-butyl 6-bromohexanoate (42 mg), and N,N-diisopropylethylamine (40 mg) were dissolved in N,N-dimethylformamide (1 mL) and stirred at 60 °C for 4 hours. The mixture was warmed to 90 °C, and stirred for 9 hours. Water was added to the reaction mixture, and the mixture was extracted with ethyl acetate. The organic layer was washed with water and saturated brine, dried over sodium sulfate, filtered, and the filtrate was concentrated under reduced pressure. The residue was purified by silica gel column chromatography (chloroform:methanol = 100:0 to 92:8) to obtain the title compound (22 mg) as a yellow powder. MS (ESI) m/z: 501.3 (M+H)⁺

### Example 295

### tert-Butyl 6-[3-(6,8-dioxo-2,7-diazaspiro[4.6]undecan-2-yl)anilino]hexanoate (Example Compound 295)

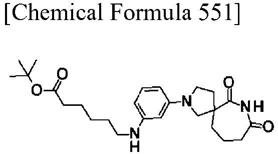

Using 2-(3-aminophenyl)-2,7-diazaspiro[4.6]undecane-6,8-dione instead of 2-(6-amino-1,3-benzothiazol-2-yl)-2,7-diazaspiro[4.6]undecane-6,8-dione in Example 294, the same reaction and processing were performed to obtain the title compound (17 mg) as a colorless viscous material. MS (ESI) m/z: 444.3 (M+H)⁺

### Example 296

### 1-(3-Chloro-4-methylphenyl)-3-[2-(6,8-dioxo-2,7-diazaspiro[4.6]undecan-2-yl)-1,3-benzothiazol-6-yl]urea (Example Compound 296)

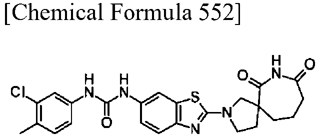

2-(6-Amino-1,3-benzothiazol-2-yl)-2,7-diazaspiro[4.6]undecane-6,8-dione (25 mg) was suspended in chloroform (1 mL), and N,N-diisopropylethylamine (15 mg) and 2-chloro-4-isocyanato-1-methylbenzene (14 mg) were added, followed by stirring at room temperature for 1 hour. Water was added to the reaction mixture, and the mixture was extracted with chloroform. The insoluble matter was collected and dissolved in a chloroform-methanol mixed solvent. This was combined with the organic layer, dried over sodium sulfate, filtered, and the filtrate was concentrated under reduced pressure. The residue was purified by silica gel column chromatography (chloroform:methanol = 100:0 to 94:6) to obtain the title compound (17 mg) as a colorless powder. MS (ESI) m/z: 498.1/500.2 (M+H)⁺

### Example 297

### 1-[1-(4-Aminophenyl)-2-oxopyrrolidin-3-yl]-1,3-diazepane-2,4-dione (Example Compound 297)

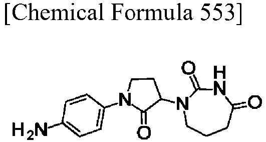

3-[1-(4-Nitrophenyl)-2-oxopyrrolidin-3-yl]-4H-1,3-diazepine-2,7-dione (102 mg) was suspended in methanol (4 mL) and tetrahydrofuran (4 mL), palladium on carbon (20 mg) was added, and the mixture was placed under a hydrogen atmosphere. The mixture was stirred at room temperature for 2 hours. The mixture was then stirred at 50 °C for 3 hours. The reaction mixture was filtered through Celite, followed by washing with a chloroform/methanol mixed solvent. The filtrate was concentrated, and the residue was purified by silica gel column chromatography (chloroform:methanol = 100:0 to 94:6) to obtain the title compound (30 mg) as a colorless powder. MS (ESI) m/z: 303.1 (M+H)⁺

### Example 298

### tert-Butyl 6-[3-(2,7-dioxoazepan-3-yl)anilino]hexanoate (Example Compound 298)

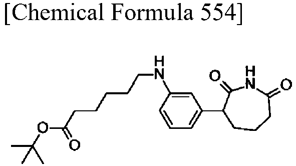

3-(3-Aminophenyl)azepane-2,7-dione (30 mg) and tert-butyl 6-oxohexanoate (52 mg) were suspended in dichloromethane (1 mL), and sodium triacetoxyborohydride (44 mg) was added. The mixture was stirred at room temperature for 3 hours. Saturated aqueous sodium bicarbonate was added to the reaction mixture, followed by extraction with chloroform and drying over anhydrous sodium sulfate, and the solvent was evaporated under reduced pressure. The residue was purified by silica gel column chromatography (hexane:ethyl acetate = 80:20 to 55:45 to 40:60) to obtain the title compound (40 mg) as a colorless viscous material. MS (ESI) m/z: 389.9 (M+H)⁺

The corresponding starting compounds were processed in the same manner as in Example 298 to obtain the compounds listed in the table below.

**[Table 24]**

| Example | Structural formula | Physical properties etc. |
|---|---|---|
| 299 | 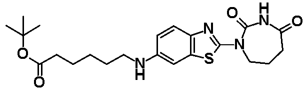 tert-Butyl 6-[[2-(2,4-dioxo-1,3-diazepan-1-yl)-1,3-benzothiazol-6-yl]amino]hexanoate | Colorless powder |
| | | MS (ESI) m/z: 447.2 (M+H)⁺ |
| 300 | 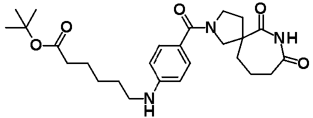 tert-Butyl 6-[4-(6,8-dioxo-2,7-diazaspiro[4. 6]undecane-2-carbonyl)anilino]hexanoate | Colorless viscous material |
| | | MS (ESI) m/z: 472.3 (M+H)⁺ |
| 301 | 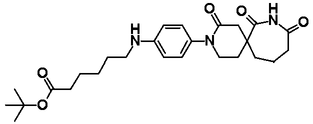 tert-Butyl 6-[4-(4,7,9-trioxo-3,8-diazaspiro[5. 6]dodecan-3-yl) anilino]hexanoate | Colorless powder |
| | | MS (ESI) m/z: 416.3 (M-tBu+H)⁺ |
| 302 | 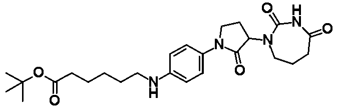 tert-Butyl 6-[4-[3-(2,4-dioxo-1,3-diazepan-1-yl)-2-oxopyrrolidin-1-yl]anilino]hexanoate | Colorless powder |
| | | MS (ESI) m/z: 417.3 (M-tBu+H)⁺ |

### Example 303

### tert-Butyl N-[7-[[2-(2,4-dioxo-1,3-diazepan-1-yl)-1,3-benzothiazol-6-yl]amino]heptyl]carbamate (Example Compound 303)

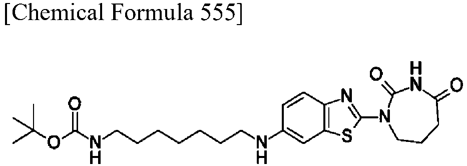

1-(6-Amino-1,3-benzothiazol-2-yl)-1,3-diazepane-2,4-dione (20 mg), tert-butyl N-(7-oxoheptyl)carbamate (22 mg), and sodium triacetoxyborohydride (31 mg) were added to dichloromethane (1 mL), followed by stirring at room temperature for 16 hours. Saturated aqueous sodium bicarbonate was added to the reaction mixture, followed by extraction with chloroform and drying over anhydrous sodium sulfate, and the solvent was evaporated under reduced pressure. The residue was purified by silica gel column chromatography (hexane:ethyl acetate = 80:20 to 50:50) to obtain the title compound (20 mg) as a colorless powder. MS (ESI) m/z: 490.3 (M+H)⁺

### Examples 304 and 305

The corresponding starting compounds were processed in the same manner as in Example 303 to obtain the compounds listed in the table below.

**[Table 25]**

| Example | Structural formula | Physical properties etc. |
|---|---|---|
| 304 | 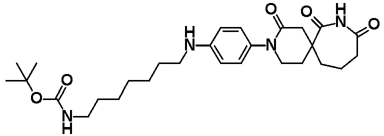 tert-Butyl N-[7-[4-(4,7,9-trioxo-3,8-diazaspiro[5.6]dodecan-3-yl)anilino]heptyl]carbamate | Colorless powder |
| | | MS (ESI) m/z: 415.3 (M-Boc+H)⁺ |
| 305 | 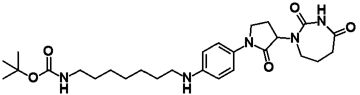 tert-Butyl N-[7-[4-[3-(2,4-dioxo-1,3-diazepan-1-yl)-2-oxopyrrolidin-1-yl]anilino]heptyl]carbamate | Colorless powder |
| | | MS (ESI) m/z: 416.3 (M-Boc+H)⁺ |

### Reference Example and Example 306

### (306-1) tert-Butyl N-[5-[(2-chloro-1,3-benzothiazol-6-yl)oxy]pentyl]carbamate (Reference Example Compound 306-1)

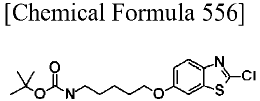

Using 2-chloro-6-hydroxy-1,3-benzothiazole instead of methyl 2-fluoro-3-hydroxybenzoate and tert-butyl N-(5-bromopentyl)carbamate instead of tert-butyl 2-bromoacetate in Reference Example 67-1, the same reaction and processing were performed to obtain the title compound as a colorless viscous material. MS (ESI) m/z: 314.9/316.9 (M-tBu+H)⁺.

### (306-2) tert-Butyl N-[5-[[2-(6,8-dioxo-2,7-diazaspiro[4.6]undecan-2-yl)-1,3-benzothiazol-6-yl]oxy]pentyl]carbamate (Example Compound 306)

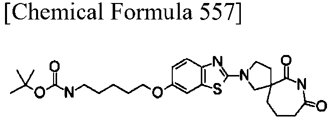

Using Reference Example Compound 306-1 instead of tert-butyl 2-chloropyrimidine-5-carboxylate in Example 32, the same reaction and processing as in (32-1) were performed to obtain the title compound as a white solid. MS (ESI) m/z: 517.4 (M+H)⁺.

### Example and Reference Example 307

### (307-1) N-(4-Bromo-1-hydroxy-indan-2-yl)-4-nitro-benzenesulfonamide (Reference Example Compound 307-1)

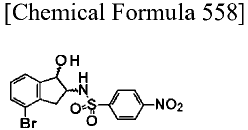

Using 2-amino-4-bromo-indan-1-ol instead of DL-α-amino-ε-caprolactam and 4-nitrobenzenesulfonyl chloride instead of 4-chlorobenzenesulfonyl chloride in Reference Example and Example 7, the same reaction and processing as in (7-1) were performed to obtain the title compound as a white solid. MS (ESI) m/z: 411.1 / 413.1 (M+H)⁺.

### (307-2) 2-(Allylamino)-4-bromo-indan-1-ol (Reference Example Compound S307-2)

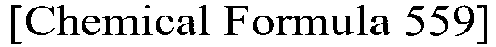

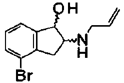

Reference Example Compound 307-1 (2.73 g) was dissolved in N,N-dimethylformamide (25 mL), and potassium carbonate (1.83 g) and allyl bromide (0.86 mL) were added, followed by stirring at room temperature overnight. Water was added to the reaction mixture, followed by extraction with ethyl acetate, and concentration. The residue was purified by silica gel column chromatography (ethyl acetate:hexane = 20:80 to 30:70). This was dissolved in methanol (40 mL), and potassium carbonate (2.73 g) and thioacetic acid (0.96 mL) were added, followed by stirring at 60 °C for 4 hours. The reaction mixture was concentrated, and then, saturated aqueous sodium bicarbonate and chloroform were added to the residue, followed by stirring. After that, the chloroform layer was separated and concentrated. The crude product was purified by silica gel column chromatography (methanol:chloroform = 0:100 to 15:85) to obtain the title compound as a pale yellow powder. MS (ESI) m/z: 268.1/270.1 (M+H)⁺.

### (307-3) N-Allyl-4-bromo-1-[tert-butyl(dimethyl)silyl]oxy-indan-2-amine (Reference Example Compound 307-3)

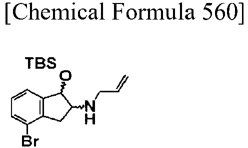

Reference Example Compound 307-2 (1.5 g) was dissolved in N,N-dimethylformamide (40 mL), and imidazole (1.5 g) and tert-butylchlorodimethylsilane (2.5 g) were added, followed by stirring at 60 °C for 5 hours. Water was added to the reaction mixture, followed by extraction with diethyl ether, and concentration. The residue was purified by silica gel column chromatography (ethyl acetate:hexane = 10:90 to 25:75) to obtain the title compound as a yellow oil. MS (ESI) 382.0/384.0 [M+H]⁺.

### (307-4) N-Allyl-4-bromo-1-[tert-butyl(dimethyl)silyl]oxy-indan-2-yl]carbamoyl]prop-2-enamide (Reference Example Compound 307-4)

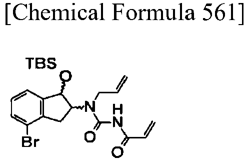

Using (Reference Example Compound 307-3) instead of 1-(4-nitrophenyl)-3-(prop-2-enylamino)pyrrolidin-2-one in Reference Example 280-2, the same reaction and processing were performed to obtain the title compound as a colorless powder. MS (ESI) m/z: 479.1/481.1 (M+H)⁺

### (307-5) N-Allyl-4-bromo-1-[tert-butyl(dimethyl)silyl]oxy-indan-2-yl]carbamoyl]-N-(2-trimethylsilylethoxymethyl)prop-2-enamide (Reference Example Compound 307-5)

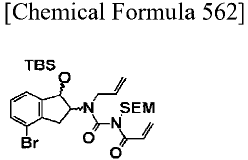

Using (Reference Example Compound 307-4) instead of N-[(6-bromo-1-methylindazol-3-yl)-prop-2-enylcarbamoyl]prop-2-enamide in Reference Example 281-4, the same reaction and processing were performed to obtain the title compound as a colorless oil. MS (ESI) m/z: 551.1/553.1 [M+H-tBu]⁺

### (307-6) 3-[4-Bromo-1-[tert-butyl(dimethyl)silyl]oxy-indan-2-yl]-1-(2-trimethylsilylethoxymethyl)-4H-1,3-diazepine-2,7-dione (Reference Example Compound 307-6)

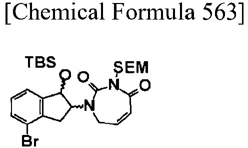

Using (Reference Example Compound 307-5) instead of N-[(6-bromo-1-methylindazol-3-yl)-prop-2-enylcarbamoyl]-N-(2-trimethylsilylethoxymethyl)prop-2-enamide in Reference Example 281-5, the same reaction and processing were performed to obtain the title compound as a colorless powder. MS (ESI) m/z: 523.1/525.1 [M-tBu]⁺

### (307-7) 3-[4-Bromo-1-[tert-butyl(dimethyl)silyl]oxy-indan-2-yl]-4H-1,3-diazepine-2,7-dione (Example Compound 307-1)

### 3-(4-Bromo-1-hydroxy-indan-2-yl)-4H-1,3-diazepine-2,7-dione (Example Compound 307-2)

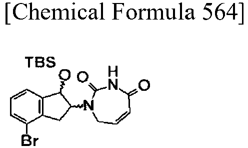

### (Example Compound 307-1)

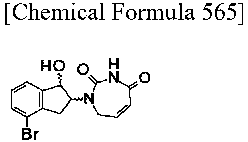

### (Example Compound 307-2)

Using (Reference Example Compound 307-6) instead of 3-(6-bromo-1-methylindazol-3-yl)-1-(2-trimethylsilylethoxymethyl)-4H-1,3-diazepine-2,7-dione in Example 281, the same reaction and processing were performed to obtain Example Compound 307-1 as a colorless oil. MS (ESI) m/z: 449.2/451.2 (M-H)⁻. At the same time, Example Compound 307-2 was obtained as a colorless oil. MS (ESI) m/z: 335.1/337.1 (M-H)⁻.

### Example 308

### 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]-N-[6-[3-(6,8-dioxo-2,7-diazaspiro[4.6]undecane-2-carbonyl)phenyl]hexyl]acetamide (Example compound 308)

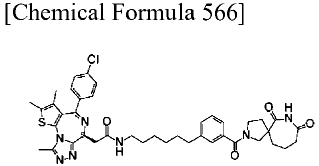

tert-Butyl N-[6-[3-(6,8-dioxo-2,7-diazaspiro[4.6]undecan-2-carbonyl)phenyl]hexyl]carbamate (25 mg) was dissolved in dichloromethane (1 mL), and trifluoroacetic acid (0.2 mL) was added, followed by stirring at room temperature for 1 hour. The reaction mixture was concentrated, and dried under reduced pressure. The residue was dissolved in N,N-dimethylformamide (0.5 mL), and 2-[(9S)-7-(4-chlorophenyl)-4,5,13-trimethyl-3-thia-1,8,11,12-tetraazatricyclo[8.3.0.02.6]trideca-2(6),4,7,10,12-pentaen-9-yl]acetic acid (27 mg), N,N-diisopropylethylamine (20 mg), and O-(7-azabenzotriazol-1-yl)-N,N,N',N'-tetramethyluronium hexafluorophosphate (25 mg) were added, followed by stirring at room temperature for 1 hour. Water was added to the reaction mixture, and the mixture was extracted with chloroform. The organic layer was dried over anhydrous sodium sulfate, and the solvent was evaporated under reduced pressure. The residue was purified by silica gel column chromatography (chloroform:methanol = 100:0 to 95:5) to obtain the title compound (22 mg) as a colorless powder. MS (ESI) m/z: 768.4 / 770.3 (M+H)⁺

The corresponding starting compounds were processed in the same manner as in Example 308 to obtain the compounds listed in the table below.

**[Table 26]**

| Example | Starting compound | Structural formula | Physical properties etc. |
|---|---|---|---|
| 309 | Example 51 | 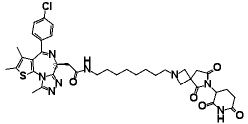 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[8-[6-(2,6-dioxopiperidin-3-yl)-5,7-dioxo-2,6-diazaspiro[3.4]octan-2-yl]octyl]acetamide | White powder |
| | | | MS (ESI) m/z: 761. 2/763. 2 [M+H]⁺ |
| 310 | Example 147 | 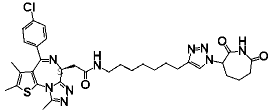 2-[(9S)-7-(4-chlorophenyl)-4,5,13-trimethyl-3-thia-1,8, 11, 12-tetraazatricyclo[8.3.0.02,6]trideca-2(6), 4, 7, 10, 12-pentaen-9-yl]-N-[7-[1-(2, 7-dioxoazepan-3-yl)triazol-4-yl]heptyl]acetamide | White powder |
| | | | MS (ESI) m/z: 690. 4/692. 5 [M+H]⁺ |
| 311 | Example 271 | 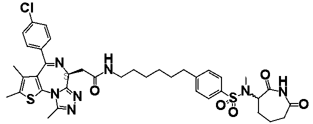 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[6-[4-[(2,7-dioxoazepan-3-yl)-methylsulfamoyl]phenyl]hexyl]acetamide | White powder |
| | | | MS (ESI) m/z: 778. 1/780. 1 [M+H]⁺ |
| 312 | Example 302 | 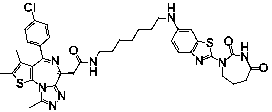 2-[(9S)-7-(4-chlorophenyl)-4, 5, 13-trimethyl-3-thia-1,8, 11, 12-tetraazatricyclo[8.3.0.02,6]trideca-2(6),4,7,10,12-pentaen-9-yl]-N-[7-[[2-(2,4-dioxo-1,3-diazepan-1-yl)-1,3-benzothiazol-6-yl]amino]heptyl]acetamide | White powder |
| | | | MS (ESI) m/z: 772.3/774.3 [M+H]⁺ |
| 313 | Example 305 | 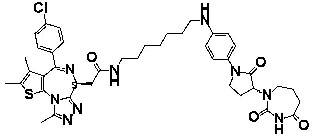 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]-N-[7-[4-[3-(2, 4-dioxo-1, 3-diazepan-1-yl)-2-oxopyrrolidin-1-yl]anilino]heptyl]acetamide | White powder |
| | | | MS (ESI) m/z: 798. 4/800. 3 [M+H]⁺ |
| 314 | Example 304 | 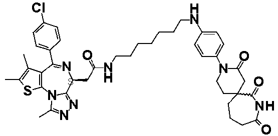 2-[(9S)-7-(4-chlorophenyl)-4, 5, 13-trimethyl-3-thia-1,8, 11, 12-tetraazatricyclo[8.3.0.02,6]trideca-2(6), 4, 7, 10, 12-pentaen-9-yl]-N-[7-[4-(4, 7,9-trioxo-3,8-diazaspiro[5.6]dodecan-3-yl)anilino]heptyl]acetamide | White powder |
| | | | MS (ESI) m/z: 797. 4/799. 3 [M+H]⁺ |
| 315 | Example 233 | 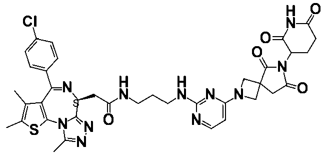 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[3-[[4-[6-(2, 6-dioxopiperidin-3-yl)-5, 7-dioxo-2, 6-diazaspiro[3. 4]octan-2-yl]pyrimidin-2-yl]amino]propyl]acetamide | White powder |
| | | | MS (ESI) m/z: 784. 1/786. 1 [M+H]⁺ |
| 316 | Example 150 | 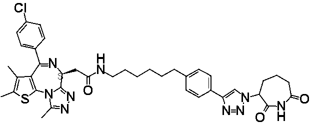 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[6-[4-[1-(2,7-dioxoazepan-3-yl)triazol-4-yl]phenyl]hexyl]acetamide | White powder |
| | | | MS (ESI) m/z: 752. 1/754. 1 [M+H]⁺ |
| 317 | Example 278 | 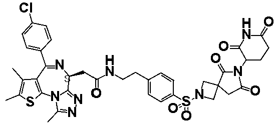 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[2-[4-[[6-(2, 6-dioxopiperidin-3-yl)-5, 7-dioxo-2, 6-diazaspiro[3.4]octan-2-yl]sulfonyl]phenyl]ethyl]acetamide | White powder |
| | | | MS (ESI) m/z: 817.1/819.1 [M+H]⁺ |
| 318 | Example 236 | 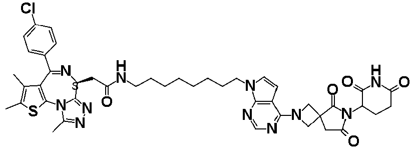 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[8-[4-[6-(2, 6-dioxopiperidin-3-yl)-5, 7-dioxo-2, 6-diazaspiro[3. 4]octan-2-yl]pyrrolo[2, 3-d]pyrimidin-7-yl]octyl]acetamide | White powder |
| | | | MS (ESI) m/z: 878. 1/880. 1 [M+H]⁺ |
| 319 | Example 186 | 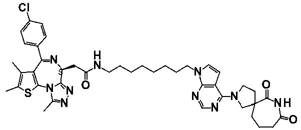 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[8-[4-(6,8-dioxo-2,7-diazaspiro[4.6]undecan-2-yl)pyrrolo[2, 3-d]pyrimidin-7-yl]octyl]acetamide | White powder |
| | | | MS (ESI) m/z: 809. 6/811. 6 M+H]⁺ |
| 320 | Example 194 | 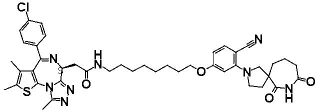 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[8-[4-cyano-3-(6,8-dioxo-2,7-diazaspiro[4.6]undecan-2-yl)phenoxy]octyl]acetamide | White powder |
| | | | MS (ESI) m/z: 809. 1/811. 1 M+H]⁺ |
| 321 | Example 241 | 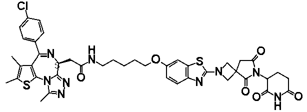 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[5-[[2-[6-(2, 6-dioxopiperidin-3-yl)-5, 7-dioxo-2, 6-diazaspiro[3.4]octan-2-yl]-1,3-benzothiazol-6-yl]oxy]pentyl]acetamide | White powder |
| | | | MS (ESI) m/z: 868. 6/870. 6 M+H]⁺ |
| 322 | Example 306 | 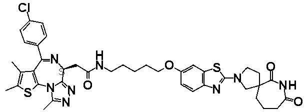 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[5-[[2-(6,8-dioxo-2,7-diazaspiro[4.6]undecan-2-yl)-1,3-benzothiazol-6-yl]oxy]pentyl]acetamide | White powder |
| | | | MS (ESI) m/z: 799. 5/801. 5 M+H]⁺ |
| 323 | Example 160 | 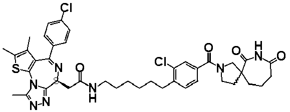 N-[6-[2-chloro-4-(6, 8-dioxo-2, 7-diazaspiro[4. 6]undecane-2-carbonyl)phenyl]hexyl]-2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]acetamide | White powder |
| | | | MS (ESI) m/z: 802. 3/804. 3 M+H]⁺ |
| 324 | Example 161 | 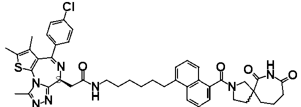 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]-N-[6-[5-(6,8-dioxo-2,7-diazaspiro[4.6]undecane-2-carbonyl)naphthalen-1-yl]hexyl]acetamide | White powder |
| | | | MS (ESI) m/z: 818.4/820. 3 M+H]⁺ |
| 325 | Example 201 | 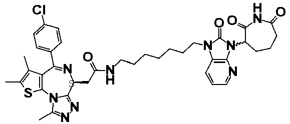 2-[(9S)-7-(4-chlorophenyl)-4,5,13-trimethyl-3-thia-1,8, 11, 12-tetraazatricyclo[8.3.0.02,6]trideca-2(6),4,7,10,12-pentaen-9-yl]-N-[7-[3-(2,7-dioxoazepan-3-yl)-2-oxoimidazo[4,5-b]pyridin-1-yl]heptyl]acetamide | White powder |
| | | | MS (ESI) m/z: 756. 4/758. 4 M+H]⁺ |
| 326 | Example 163 | 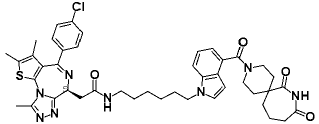 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]-N-[6-[4-(7,9-dioxo-3,8-diazaspiro[5.6]undecane-3-carbonyl)indol-1-yl]hexyl]acetamide | Colorless powder |
| | | | MS (ESI) m/z: 821. 3/823. 3 (M+H)⁺ |
| 327 | Example 270 | 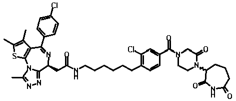 N-[6-[2-chloro-4-[4-(2,7-dioxoazepan-3-yl)-3-oxopiperazine-1-carbonyl]phenyl]hexyl]-2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetamide | Colorless powder |
| | | | MS (ESI) m/z: 845. 3/847. 3 M+H]⁺ |

### Reference Example and Example 328

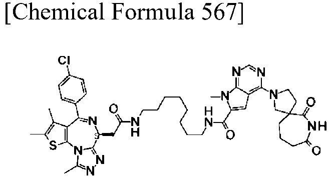

### (Example Compound 328)

### (328-1) tert-Butyl N-[8-[[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]octyl]carbamate (Reference Example Compound 328-1)

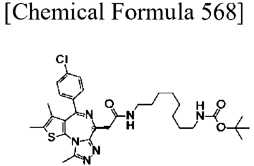

tert-Butyl N-(8-aminooctyl)carbamate (671 mg) was dissolved in N,N-dimethylformamide (15 mL), and 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetic acid (1 g), N,N-diisopropylethylamine (1.29 mL), and O-(7-azabenzotriazol-1-yl)-N,N,N',N'-tetramethyluronium hexafluorophosphate (1.14 g) were added, followed by stirring at room temperature for 3 hours. Water was added to the reaction mixture, and the mixture was extracted with ethyl acetate. The organic layer was dried over anhydrous sodium sulfate, and the solvent was evaporated under reduced pressure. The residue was purified by silica gel column chromatography (chloroform:methanol = 100:0 to 92:8) to obtain the title compound (1.704 g) as an orange oil. MS (m/z): 627.5/629.5 [M+H]⁺

### (328-2) N-(8-Aminooctyl)-2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetamide hydrochloride (Reference Example Compound 328)

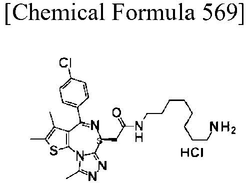

To a methanol (1 mL) solution of Reference Example Compound 328-1 (397 mg), 4N hydrochloric acid/dioxane solution (3 mL) was added, and the mixture was stirred at room temperature for 2 hours. The reaction mixture was concentrated under reduced pressure and dried under reduced pressure to obtain the title compound (362.9 mg) as a yellow solid. MS (ESI) m/z: 527.4/529.3 (M+H)⁺

### Example 328

### N-[8-[[2-[(9S)-7-(4-Chlorophenyl)-4,5,13-trimethyl-3-thia-1,8,11,12-tetraazatricyclo[8.3.0.02,6]trideca-2(6),4,7,10,12-pentaen-9-yl]acetyl]amino]octyl]-4-(6,8-dioxo-2,7-diazaspiro[4.6]undecan-2-yl)-7-methylpyrrolo[2,3-d]pyrimidine-6-carboxamide (Example Compound 328)

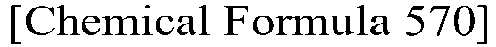

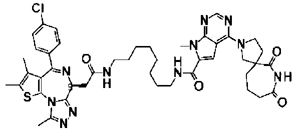

Example Compound 180 was dissolved in chloroform (1 mL), and trifluoroacetic acid (1 mL) was added, followed by stirring at room temperature for 3 hours. The reaction mixture was concentrated and azeotroped with toluene to obtain 4-(6,8-dioxo-2,7-diazaspiro[4.6]undecan-2-yl)-7-methyl-pyrrolo[2,3-d]pyrimidine-6-carboxylic acid.

The above compound and Reference Example Compound 328 (30 mg) were dissolved in N,N-dimethylformamide (3 mL), and N,N-diisopropylethylamine (43.3 µL) and O-(7-azabenzotriazol-1-yl)-N,N,N',N'-tetramethyluronium hexafluorophosphate (28.5 mg) were added, followed by stirring at room temperature for 3 hours. The reaction mixture was evaporated under reduced pressure, and the residue was purified by silica gel column chromatography (chloroform:methanol = 100:0 to 95:5) to obtain the title compound (41.3 mg) as a colorless powder. MS (ESI) m/z: 866.2/868.2 (M+H)⁺

### Reference Example 351

### N-(7-Aminoheptyl)-2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetamide hydrochloride (Compound of Reference Example 351)

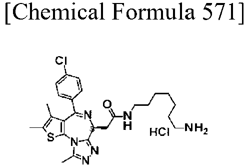

Using tert-butyl N-(7-aminoheptyl)carbamate instead of tert-butyl N-(8-aminooctyl)carbamate in (328-1), the same processing as in (328-1) and (328-2) was performed to obtain the title compound as a yellow powder. MS (ESI) m/z: 513.2/515.2 (M+H)⁺

The corresponding starting compounds were processed in the same manner as in Example 328 to obtain the compounds listed in the table below.

**[Table 27]**

| Example | Starting compound | Structural formula | Physical properties etc. |
|---|---|---|---|
| 329 | Example 181 | 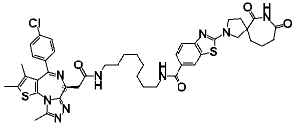 N-[8-[[2-[(9S)-7-(4-chlorophenyl)-4, 5,13-trimethyl-3-thia-1,8,11,12-tetraazatricyclo[8.3.0.02,6]trideca-2(6),4,7,10,12-pentaen-9-yl]acetyl]amino]octyl]-2-(6,8-dioxo-2,7-diazaspiro[4. 6]undecan-2-yl)-1, 3-benzothiazole-6-carboxamide | White powder |
| | | | MS (ESI) m/z: 868. 1/870. 1 [M+H]⁺ |
| 330 | Example 45 | 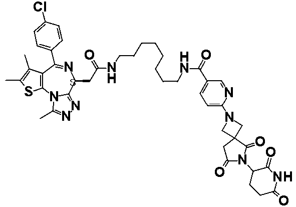 N-[8-[[2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]acetyl]amino]octyl]-6-[6-(2, 6-dioxopiperidin-3-yl)-5,7-dioxo-2,6-diazaspiro[3.4]octan-2-yl]pyridine-3-carboxamide | White powder |
| | | | MS (ESI) m/z: 881. 2/883. 2 [M+H]⁺ |
| 331 | Example 48 | 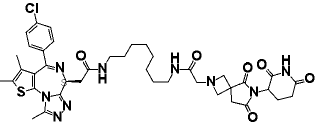 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]-N-[8-[[2-[6-(2,6-dioxopiperidin-3-yl)-5,7-dioxo-2,6-diazaspiro[3.4]octan-2-yl]acetyl]amino]octyl]acetamide | White powder |
| | | | MS (ESI) m/z: 818.2/820.1 [M+H]⁺ |
| 332 | Example 138 | 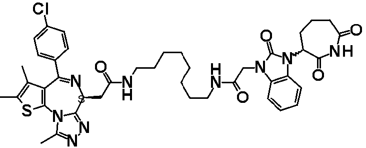 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]-N-[8-[[2-[3-(2,7-dioxoazepan-3-yl)-2-oxobenzimidazol-1-yl]acetyl]amino]octyl]acetamide | White powder |
| | | | MS (ESI) m/z: 826. 3/828. 3 [M+H]⁺ |
| 333 | Example 213 | 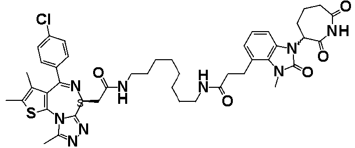 N-[8-[[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]octyl]-3-[1-(2,7-dioxoazepan-3-yl)-3-methyl-2-oxobenzimidazol-4-yl]propanamide | White powder |
| | | | MS (ESI) m/z: 854. 4/856. 3 [M+H]⁺ |
| 334 | Example 137 | 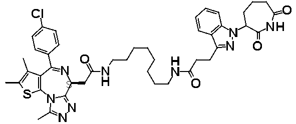 N-[8-[[2-[(9S)-7-(4-chlorophenyl)-4, 5,13-trimethyl-3-thia-1,8,11,12-tetraazatricyclo[8.3.0.02,6]trideca-2 (6),4,7,10,12-pentaen-9-yl]acetyl]amino]octyl]-3-[1-(2,7-dioxoazepan-3-yl)indazol-3-yl]propanamide | White powder |
| | | | MS (ESI) m/z: 824.3/826.3 [M+H]⁺ |
| 335 | Example 108 | 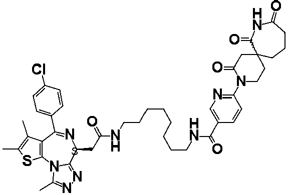 N-[8-[[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]octyl]-6-(4,7,9-trioxo-3,8-diazaspiro[5.6]dodecan-3-yl)pyridine-3-carboxamide | White powder |
| | | | MS (ESI) m/z: 840. 2/842. 2 [M+H]⁺ |
| 336 | Example 266 | 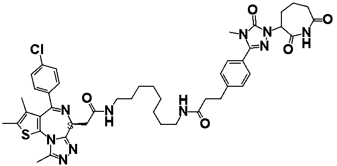 N-[8-[[2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]acetyl]amino]octyl]-3-[4-[1-(2,7-dioxoazepan-3-yl)-4-methyl-5-oxo-1,2,4-triazol-3-yl]phenyl]propanamide | White powder |
| | | | MS (ESI) m/z: 881.4/883. 3 [M+H]⁺ |
| 337 | Example 231 | 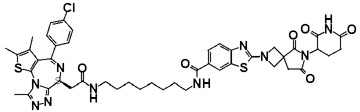 N-[8-[[2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]acetyl]amino]octyl]-2-[6-(2,6-dioxopiperidin-3-yl)-5,7-dioxo-2,6-diazaspiro[3.4]octan-2-yl]-1, 3-benzothiazole-6-carboxamide | White powder |
| | | | MS (ESI) m/z: 937.5/939.5 [M+H]⁺ |
| 338 | Example 268 | 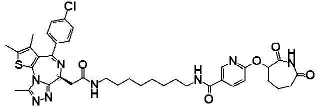 N-[8-[[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]octyl]-6-(2,7-dioxoazepan-3-yl)oxypyridine-3-carboxamide | White powder |
| | | | MS (ESI) m/z: 773. 1/775. 1 [M+H]⁺ |
| 339 | Example 193 | 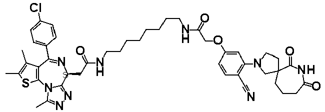 2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]-N-[8-[[2-[4-cyano-3-(6, 8-dioxo-2, 7-diazaspiro[4. 6]undecan-2-yl)phenoxy]acetyl]amino]octyl]acetamide | White powder |
| | | | MS (ESI) m/z: 866. 2/868. 2 [M+H]⁺ |
| 340 | Example 149 | 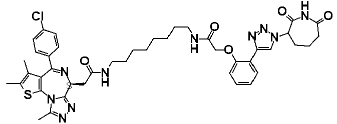 2-[(9S)-7-(4-chlorophenyl)-4,5, 13-trimethyl-3-thia-1,8,11,12-tetraazatricyclo[8.3.0.02,6]trideca-2(6),4,7,10,12-pentaen-9-yl]-N-[8-[[2-[2-[1-(2,7-dioxoazepan-3-yl)triazol-4-yl]phenoxy]acetyl]amino]octyl]acetamide | White powder |
| | | | MS (ESI) m/z: 853. 3/855. 2 [M+H]⁺ |
| 341 | Example 260 | 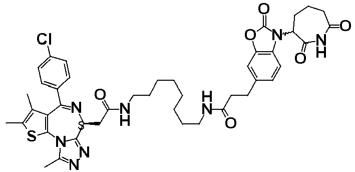 N-[8-[[2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]acetyl]amino]octyl]-3-[3-(2,7-dioxoazepan-3-yl)-2-oxo-1,3-benzoxazol-6-yl]propanamide | White powder |
| | | | MS (ESI) m/z: 841. 4/843. 4 [M+H]⁺ |
| 343 | Example 262 | 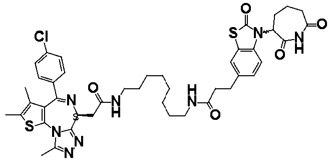 N-[8-[[2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]acetyl]amino]octyl]-3-[3-(2,7-dioxoazepan-3-yl)-2-oxo-1,3-benzothiazol-6-yl]propanamide | White powder |
| | | | MS (ESI) m/z: 857. 3/859. 3 [M+H]⁺ |
| 343 | Example 263 | 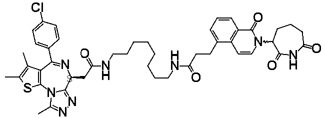 N-[8-[[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]octyl]-3-[2-(2,7-dioxoazepan-3-yl)-1-oxoisoquinolin-5-yl]propanamide | White powder |
| | | | MS (ESI) m/z: 851.4/853. 3 [M+H]⁺ |
| 344 | Example 265 | 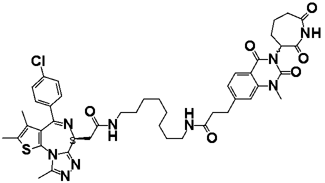 N-[8-[[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]octyl]-3-[3-(2,7-dioxoazepan-3-yl)-1-methyl-2,4-dioxo-quinazolin-7-yl]propanamide | White powder |
| | | | MS (ESI) m/z: 882. 3/884. 2 [M+H]⁺ |
| 345 | Example 107 | 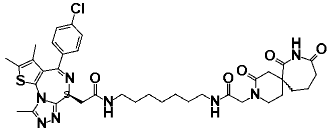 2-[(9S)-7-(4-chlorophenyl)-4,5, 13-trimethyl-3-thia-1,8,11,12-tetraazatricyclo[8.3.0.02,6]trideca-2(6),4,7,10,12-pentaen-9-yl]-N-[7-[[2-(4,7, 9-trioxo-3,8-diazaspiro[5.6]dodecan-3-yl)acetyl]amino]heptyl]acetamide | White powder |
| | | | MS (ESI) m/z: 763. 3/765. 3 [M+H]⁺ |
| 346 | Example 291 | 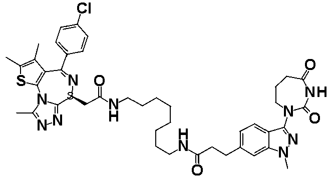 N-[8-[[2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]acetyl]amino]octyl]-3-[3-(2,4-dioxo-1,3-diazepan-1-yl)-1-methyl-indazol-6-yl]propanamide | White powder |
| | | | MS (ESI) m/z: 839.9/841. 3 [M+H]⁺ |
| 347 | Example 69 | 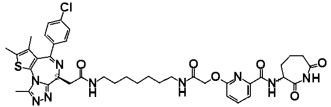 6-[2-[7-[[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]heptylamino]-2-oxo-ethoxy]-N-(2,7-dioxoazepan-3-yl)pyridine-2-carboxamide | White powder |
| | | | MS (ESI) m/z: 816.2/818.2 [M+H]⁺ |
| 348 | Example 289 | 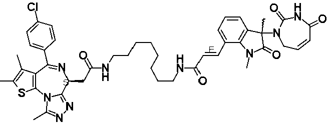 (E)-N-[8-[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]octyl]-3-[3-(2,7-dioxo-4H-1,3-diazepin-3-yl)-1,3-dimethyl-2-oxoindolin-7-yl] prop-2-enamide | Colorless powder |
| | | | MS (ESI) m/z: 864. 3/866. 2 (M+H)⁺ |
| 349 | Example 290 | 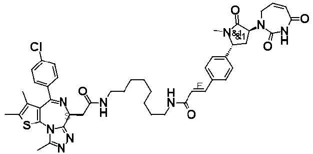 (E)-N-[8-[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]octyl]-3-[4-[(2R*,4S*)-4-(2,7-dioxo-4H-1,3-diazepin-3-yl)-1-methyl-5-oxoindolin-2-yl]phenyl]prop-2-enamide | Colorless powder |
| | | | MS (ESI) m/z: 878. 5/880. 6 (M+H)⁺ |
| 350 | Example 275 | 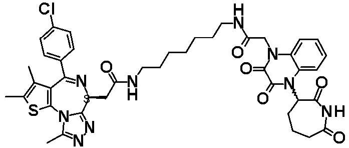 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]-N-[7-[[2-[4-(2,7-dioxoazepan-3-yl)-2,3-dioxo-quinoxalin-1-yl]acetyl]amino]heptyl]acetamide | Pale yellow solid |
| | | | MS (ESI) m/z: 840.5/842.5 [M+H]⁺ |
| 351 | Example 91 | 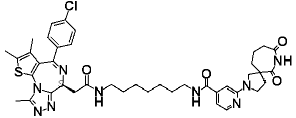 N-[7-[[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]heptyl]-2-(6,8-dioxo-2,7-diazaspiro[4.6]undecan-2-yl)pyridine-4-carboxamide | White powder |
| | | | MS (ESI) m/z: 798.3 [M+H]⁺ |

### Example 352

### 2-[(9S)-7-(4-Chlorophenyl)-4,5,13-trimethyl-3-thia-1,8,11,12-tetraazatricyclo[8.3.0.02,6]trideca-2(6),4,7,10,12-pentaen-9-yl]-N-[8-[[2-[3-(2,4-dioxo-1,3-diazepan-1-yl)-3-methyl-2-oxoindol-1-yl]acetyl]amino]octyl]acetamide (Example Compound 352)

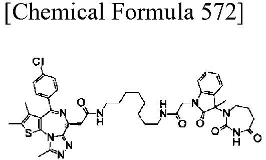

Example 292 (13 mg) and Reference Example Compound 328 (22 mg) were dissolved in N,N-dimethylformamide (0.5 mL), and N,N-diisopropylethylamine (32 µL) and O-(7-azabenzotriazol-1-yl)-N,N,N',N'-tetramethyluronium hexafluorophosphate (18 mg) were added, followed by stirring at room temperature for 0.5 hours. Water was added to the reaction mixture, and the mixture was extracted with chloroform. The organic layer was dried over sodium sulfate, filtered, and the filtrate was concentrated under reduced pressure. The residue was purified by silica gel column chromatography (chloroform:methanol = 100:0 to 95:5) to obtain the title compound (17 mg) as a colorless powder. MS (ESI) m/z: 840.4/842.3 (M+H)⁺

### Reference Example and Example 353

### (353-1) 2-[3-(2,7-Dioxo-4H-1,3-diazepin-3-yl)-3-methyl-2-oxo-indolin-1-yl]acetic acid (Reference Example Compound 353-1)

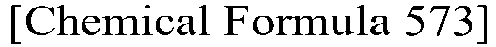

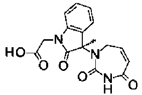

Benzyl 2-[3-(2,7-dioxo-4H-1,3-diazepin-3-yl)-3-methyl-2-oxo-indolin-1-yl]acetate (33 mg) was dissolved in methylene chloride (0.5 mL), and under ice-cooling, anisole (43 mg) and trifluoromethanesulfonic acid (35 µL) were added, followed by stirring at the same temperature for 30 minutes and then at room temperature for 30 minutes. The reaction mixture was concentrated and dried under reduced pressure to obtain the title compound. The crude product was used as is in the next reaction.

### (353-2) 2-[(9S)-7-(4-Chlorophenyl)-4,5,13-trimethyl-3-thia-1,8,11,12-tetraazatricyclo[8.3.0.02,6]trideca-2(6),4,7,10,12-pentaen-9-yl]-N-[8-[[2-[3-(2,7-dioxo-4H-1,3-diazepin-3-yl)-3-methyl-2-oxoindol-1-yl]acetyl]amino]octyl]acetamide (Example Compound 353)

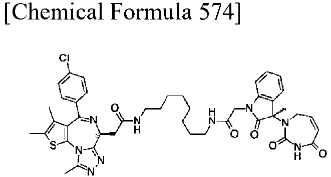

Using Reference Example Compound 353-1 instead of 4-(6,8-dioxo-2,7-diazaspiro[4.6]undecan-2-yl)-7-methyl-pyrrolo[2,3-d]pyrimidine-6-carboxylic acid in Example 328, the same reaction and processing were performed to obtain the title compound as a colorless powder. MS (ESI) m/z: 838.3/840.3 (M+H)⁺

### Example and Reference Example 354

### Reference Example 354

### 11-[[2-[(9S)-7-(4-Chlorophenyl)-4,5,13-trimethyl-3-thia-1,8,11,12-tetraazatricyclo[8.3.0.02,6]trideca-2(6),4,7,10,12-pentaen-9-yl]acetyl]amino]undecanoic acid (Reference Example Compound 354)

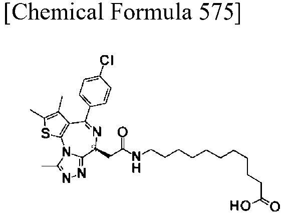

Using methyl 11-aminoundecanoate instead of tert-butyl N-(8-aminooctyl)carbamate in (328-1), the same reaction and processing were performed to obtain methyl 11-[[2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]acetyl]amino]undecanoate as an orange oil. MS (ESI) m/z: 598.5/600.5 (M+H)⁺

Subsequently, using the above compound instead of methyl 1-[6-(tert-butoxycarbonylamino)hexyl]indole-4-carboxylate in (162-2), the same reaction and processing were performed to obtain the title compound as a yellow powder. MS (ESI) m/z: 584.5/586.5 (M+H)⁺

### Example 354

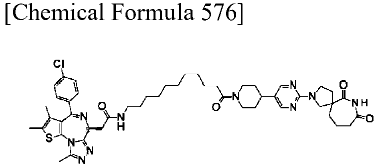

### 2-[(9S)-7-(4-Chlorophenyl)-4,5,13-trimethyl-3-thia-1,8,11,12-tetraazatricyclo[8.3.0.02,6]trideca-2(6),4,7,10,12-pentaen-9-yl]-N-[11-[4-[2-(6,8-dioxo-2,7-diazaspiro[4.6]undecan-2-yl)pyrimidin-5-yl]piperidin-1-yl]-11-oxoundecyl] acetamide

Example Compound 168-2 (19.2 mg) was dissolved in chloroform (1mL), and trifluoroacetic acid (1 mL) was added, followed by stirring at room temperature for 1 hour. The reaction mixture was concentrated and azeotroped with toluene to obtain 2-[5-(4-piperidyl)pyrimidin-2-yl]-2,7-diazaspiro[4.6]undecane-6,8-dione trifluoroacetate.

The above compound and Reference Example Compound 354 (25 mg) were dissolved in N,N-dimethylformamide (3 mL), and N,N-diisopropylethylamine (37.5 µL) and O-(7-azabenzotriazol-1-yl)-N,N,N',N'-tetramethyluronium hexafluorophosphate (24.7 mg) were added, followed by stirring at room temperature for 5 hours. The reaction mixture was evaporated under reduced pressure, and the residue was purified by silica gel column chromatography (chloroform:methanol = 100:0 to 90:10) to obtain the title compound (27.3 mg) as a pale yellow solid. MS (ESI) m/z: 909.7/911.8 (M+H)⁺

The corresponding starting compounds were processed in the same manner as in Example 354 to obtain the compounds listed in the table below.

**[Table 28]**

| Example | Starting compound | Structural formula | Physical properties etc. |
|---|---|---|---|
| 355 | Example 170 | 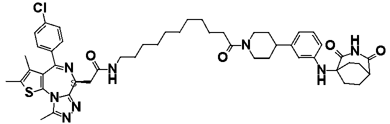 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]-N-[11-[4-[3-[(2, 4-dioxo-3-azabicyclo[3.2.2]nonan-1-yl)amino]phenyl]piperidin-1-yl]-11-oxoundecyl]acetamide | White powder |
| | | | MS (ESI) m/z: 893. 2/895. 2 [M+H]⁺ |
| 356 | Example 276 | 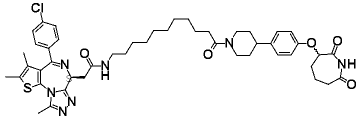 2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]-N-[11-[4-[4-[(2,7-dioxoazepan-3-yl)oxyphenyl]-1-piperidyl]-11-oxo-undecyl]acetamide | White powder |
| | | | MS (ESI) m/z: 868. 2/870. 2 [M+H]⁺ |
| 357 | Example 242 | 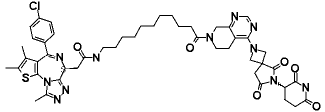 2-[(9S)-7-(4-chlorophenyl)-4,5,13-trimethyl-3-thia-1,8,11,12-tetraazatricyclo[8.3.0.02,6]trideca-2(6),4,7,10,12-pentaen-9-yl]-N-[11-[4-[6-(2,6-dioxo-3-piperidyl)-5,7-dioxo-2,6-diazaspiro[3.4]octan-2-yl]-6, 8-dihydro-5H-pyrido[3, 4-d]pyrimidin-7-yl]-11-oxo-undecyl]acetamide | Pale yellow solid |
| | | | MS (ESI) m/z: 950. 7/952. 6 [M+H]⁺ |
| 358 | Example 174 | 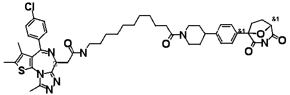 2-[(9S)-7-(4-chlorophenyl)-4, 5, 13-trimethyl-3-thia-1,8,11,12-tetraazatricyclo[8.3.0.02,6]trideca-2(6),4,7,10,12-pentaen-9-yl]-N-[11-[4-[4-[(1R*,5R*)-2, 4-dioxo-8-oxa-3-azabicyclo[3.2.1]octan-1-yl]phenyl]-1-piperidyl]-11-oxo-undecyl]acetamide | Pale yellow powder |
| | | | MS (ESI) m/z: 866. 2/868. 2 [M+H]⁺ |

### Example 359

### N-[3-Carbamoyl-1-[4-[2-[2-[2-[3-(2,7-dioxoazepan-3-yl)-2-oxobenzimidazol-1-yl]ethoxy]ethoxy]ethylcarbamoyl]phenyl]pyrazol-4-yl]-2-[2-(cyclopropylmethylamino)-4-pyridinyl]-1,3-oxazole-4-carboxamide (Example Compound 359)

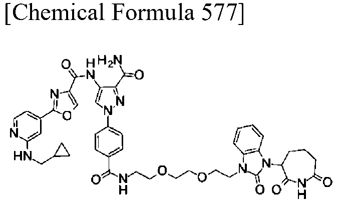

Using tert-butyl N-[2-[2-[2-[3-(2,7-dioxoazepan-3-yl)-2-oxobenzimidazol-1-yl]ethoxy]ethoxy]ethyl]carbamate instead of tert-butyl 2-(6,8-dioxo-2,7-diazaspiro[4.6]undecan-2-yl)pyridine-4-carboxylate in Example 142, the same reaction and processing were performed to obtain the title compound (60 mg) as a colorless powder. MS (ESI) m/z: 860.6 (M+H)⁺

### Example 360

### (360-1) tert-Butyl N-[7-[1-(2,7-dioxoazepan-3-yl)-3-methyl-2-oxo-benzimidazol-4-yl]hept-6-ynyl]-N-methyl-carbamate (Example Compound 360-1)

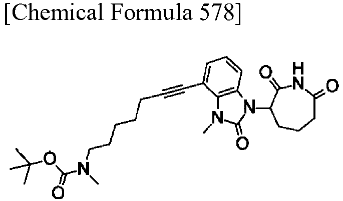

Using 3-(4-iodo-3-methyl-2-oxo-benzimidazol-1-yl)azepane-2,7-dione instead of 3-(3-iodoindazol-1-yl)azepane-2,7-dione and tert-butyl N-methyl-N-hept-6-ynyl-carbamate instead of benzyl oct-7-ynoate in Example 205, the same reaction and processing were performed to obtain the title compound as a yellow powder.

### (360-2) tert-Butyl N-[7-[1-(2,7-dioxoazepan-3-yl)-3-methyl-2-oxo-benzimidazol-4-yl]heptyl]-N-methyl-carbamate (Example Compound 360-2)

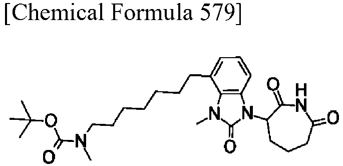

Using tert-butyl N-[7-[1-(2,7-dioxoazepan-3-yl)-3-methyl-2-oxo-benzimidazol-4-yl]hept-6-ynyl]-N-methyl-carbamate instead of 3-(3-nitrophenyl)azepane-2,7-dione in Example 70, the same reaction and processing were performed to obtain the title compound as a colorless powder.

### (360-3) 3-[4-[7-[[1-[5-[(1R,3R)-2-(2-fluoro-2-methyl-propyl)-3-methyl-1,3,4,9-tetrahydropyrido[3,4-b]indol-1-yl]pyrimidin-2-yl]-4-piperidyl]methyl-methyl-amino]heptyl]-3-methyl-2-oxo-benzimidazol-1-yl]azepane-2,7-dione (Example Compound 360-3)

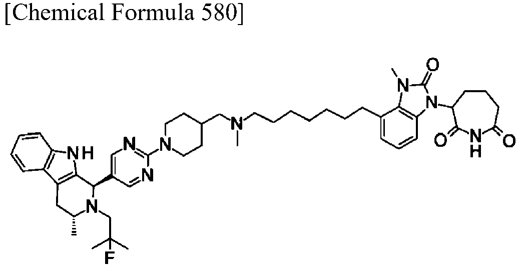

Using tert-butyl N-[7-[1-(2,7-dioxoazepan-3-yl)-3-methyl-2-oxo-benzimidazol-4-yl]heptyl]-N-methyl-carbamate instead of Example Compound 44-1 and 1-[5-[(1R,3R)-2-(2-fluoro-2-methylpropyl)-3-methyl-1,3,4,9-tetrahydropyrido[3,4-b]indol-1-yl]pyrimidin-2-yl]piperidine-4-carbaldehyde instead of benzyl 2-(4-oxo-1-piperidyl)acetate in Example 277, the same reaction and processing were performed to obtain the title compound as a yellow powder. MS (ESI) m/z: 834 (M+H)+

### Reference Example and Example 361

### (361-1) Ethyl 4-[7-[tert-butoxycarbonyl(methyl)amino]hept-1-ynyl]benzoate (Reference Example Compound 361-1)

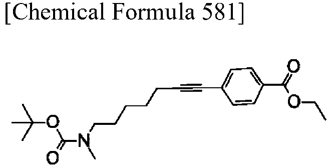

Using ethyl 4-iodobenzoate instead of 3-(3-iodoindazol-1-yl)azepane-2,7-dione and tert-butyl N-methyl-N-hept-6-ynyl-carbamate instead of benzyl oct-7-ynoate in Example 205, the same reaction and processing were performed to obtain the title compound as a yellow powder.

### (361-2) Ethyl 4-[7-[tert-butoxycarbonyl(methyl)amino]heptyl]benzoate (Reference Example Compound 361-2)

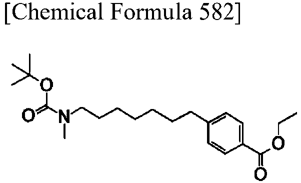

Using ethyl 4-[7-[tert-butoxycarbonyl(methyl)amino]hept-1-ynyl]benzoate instead of 3-(3-nitrophenyl)azepane-2,7-dione in Example 70, the same reaction and processing were performed to obtain the title compound as a colorless powder.

### (361-3) 4-[7-[tert-Butoxycarbonyl(methyl)amino]heptyl]benzoic acid (Reference Example Compound 361-3)

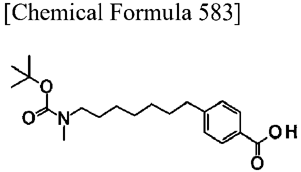

### (361-4) tert-Butyl N-[7-[4-(6,8-dioxo-2,7-diazaspiro[4.6]undecane-2-carbonyl)phenyl]heptyl]-N-methyl-carbamate (Example Compound 361-1)

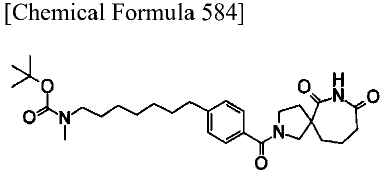

Using 4-[7-[tert-butoxycarbonyl(methyl)amino]heptyl]benzoic acid instead of 1-methylindole-4-carboxylic acid in Example 151, the same reaction and processing were performed to obtain the title compound as a colorless powder.

### (361-5) 2-[4-[7-[[1-[5-[(1R,3R)-2-(2-fluoro-2-methyl-propyl)-3-methyl-1,3,4,9-tetrahydropyrido[3,4-b]indol-1-yl]pyrimidin-2-yl]-4-piperidyl]methyl-methyl-amino]heptyl]benzoyl]-2,7-diazaspiro[4.6]undecane-6,8-dione (Example Compound 361-2)

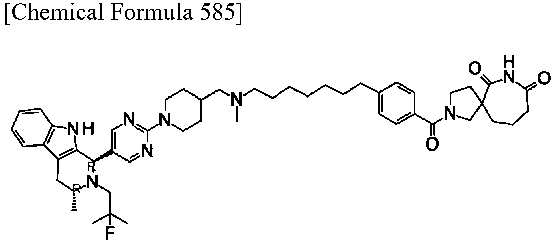

Using tert-butyl N-[7-[4-(6,8-dioxo-2,7-diazaspiro[4.6]undecane-2-carbonyl)phenyl]heptyl]-N-methyl-carbamate instead of Example Compound 44-1 and 1-[5-[(1R,3R)-2-(2-fluoro-2-methylpropyl)-3-methyl-1,3,4,9-tetrahydropyrido[3,4-b]indol-1-yl]pyrimidin-2-yl]piperidine-4-carbaldehyde instead of benzyl 2-(4-oxo-1-piperidyl)acetate in Example 277, the same reaction and processing were performed to obtain the title compound as a yellow powder. MS (ESI) m/z: 847 (M+H)+

### Test Example 1: Measurement of molecular interactions using surface plasmon resonance (SPR)

The instrument used was the Biacore T200 (manufactured by Cytiva). The CRBN truncate (318-426) was obtained by expressing a construct with an Avi-tag added at the C-terminus. The sensor chip used was Sensor Chip SA (manufactured by Cytiva). The CRBN truncate was immobilized using the affinity between SA (streptavidin) on the sensor chip and the Avi-tag.

The running buffer was a mixture of 10 mM HEPES pH 7.5, 150 mM NaCl, 1 mM TCEP, 0.005% Tween 20, and 2% DMSO. Under 25°C conditions, solutions of various concentrations of test compounds (compounds of the examples above) were flowed at 30 µL/min over the sensor chip with the immobilized CRBN truncate, and the mass changes on the sensor chip were observed. Contact time and dissociation time were set at 60 seconds and 120 seconds, respectively. The observed data were analyzed using Biacore T200 Evaluation Software Version 2.0 (manufactured by Cytiva), and the Kd values of the compounds for the CRBN truncate were calculated. In this test, the upper limit for the Kd value measurement, based on the lower quantification limit, was 200 µM.

The test results of this test are shown in Table 29 below. In the table, "***" indicates that the Kd value is less than 50µM; "**" indicates that the Kd value is 50µM or more and less than 100µM; and "*" indicates that the Kd value is 100µM or more and binding affinity was confirmed.

**[Table 29-1]**

| Example | Kd | Example | Kd | Example | Kd | Example | Kd | Example | Kd |
|---|---|---|---|---|---|---|---|---|---|
| 1 | * | 33 | * | 65 | * | 93 | * | 134 | ** |
| 2 | * | 34 | * | 66-1 | * | 94 | ** | 135 | *** |
| 3 | * | 35 | * | 66-2 | * | 95 | * | 136 | ** |
| 5 | * | 36 | *** | 67 | * | 96 | * | 137 | *** |
| 6 | * | 37 | * | 68 | * | 97 | * | 138 | ** |
| 7 | * | 38 | * | 69 | ** | 98 | * | 139 | ** |
| 8 | * | 39 | * | 70 | * | 99 | * | 140 | * |
| 9 | * | 40 | * | 71 | * | 104 | * | 141 | ** |
| 10 | * | 41 | * | 72 | *** | 106 | * | 142 | *** |
| 11 | * | 42 | ** | 73 | *** | 107 | * | 143 | *** |
| 12-2 | * | 43 | ** | 74 | * | 108 | ** | 144 | *** |
| 13-2 | * | 44-2 | * | 75 | *** | 109 | *** | | |
| 14 | ** | 45 | ** | 76 | * | 110 | * | | |
| 15 | * | 46 | ** | 77 | *** | 111 | ** | | |
| 16 | * | 47 | ** | 78 | * | 113 | * | | |
| 18 | * | 48 | * | 79 | * | 115 | ** | | |
| 19 | * | 49 | * | 80 | *** | 116 | *** | | |
| 20 | * | 50 | * | 81 | * | 117 | * | | |
| 21 | * | 51 | * | 82 | ** | 118 | * | | |
| 22 | * | 52 | *** | 83 | *** | 119 | *** | | |
| 24 | * | 53 | ** | 84 | *** | 120 | * | | |
| 25 | * | 54 | ** | 85 | ** | 121 | * | | |
| 26 | * | 55 | * | 86 | * | 122 | * | | |
| 27 | * | 56 | ** | 87 | * | 123 | *** | | |
| 28 | * | 57 | * | 88 | * | 125 | * | | |
| 29-1 | * | 59 | *** | 89 | * | 126 | * | | |
| 29-2 | * | 60 | ** | 90-1 | *** | 127 | * | | |
| 30 | * | 61 | * | 90-2 | * | 128 | *** | | |
| 31 | ** | 62 | * | 91 | ** | 131 | * | | |
| 32 | * | 63 | * | 92 | * | 133 | * | | |

**[Table 29-2]**

| Example | Kd | Example | Kd | Example | Kd | Example | Kd |
|---|---|---|---|---|---|---|---|
| 147 | * | 187 | ** | 222 | ** | 256 | * |
| 148 | ** | 188 | * | 223 | *** | 257 | * |
| 149 | * | 189 | * | 224 | * | 258 | * |
| 150 | * | 190 | * | 225 | * | 259 | * |
| 151 | * | 191 | * | 228 | * | 260 | ** |
| 152 | * | 192 | *** | 229 | * | 261 | ** |
| 153 | * | 193 | *** | 230 | * | 262 | ** |
| 154 | * | 194 | * | 231 | *** | 263 | *** |
| 155 | * | 195 | *** | 232 | ** | 265 | * |
| 156 | * | 196 | *** | 233 | * | 266 | * |
| 157 | *** | 197 | * | 234 | * | 267 | * |
| 158 | ** | 198 | *** | 235 | * | 268 | * |
| 159 | * | 199 | * | 236 | *** | 269 | * |
| 160 | ** | 201-2 | * | 237 | * | 271 | * |
| 161 | ** | 202 | * | 238 | *** | 272 | * |
| 162 | ** | 203 | * | 239 | *** | 273-2 | * |
| 165 | * | 206 | *** | 240 | * | 274 | * |
| 167 | * | 207 | * | 241 | * | 275 | * |
| 168-2 | *** | 209 | * | 242 | * | 276 | * |
| 173 | * | 210 | ** | 243 | * | 277 | *** |
| 176 | *** | 211 | ** | 245 | * | 278 | * |
| 177 | ** | 213 | *** | 246 | ** | 279 | * |
| 178 | *** | 214 | ** | 247 | * | 281 | * |
| 179 | *** | 215 | * | 248 | * | 282 | * |
| 180 | ** | 216 | * | 250 | * | 283 | ** |
| 182 | * | 217 | *** | 251 | * | 287 | *** |
| 183 | * | 218 | ** | 252 | * | 288 | *** |
| 184 | * | 219 | *** | 253 | * | 289 | *** |
| 185 | * | 220 | ** | 254 | * | 290 | * |
| 186 | ** | 221 | ** | 255 | * | 291 | ** |

**[Table 29-3]**

| Example | Kd | Example | Kd |
|---|---|---|---|
| 292 | * | 329 | *** |
| 296 | * | 330 | *** |
| 297 | * | 331 | *** |
| 298 | * | 332 | *** |
| 300 | *** | 333 | *** |
| 301 | * | 334 | *** |
| 302 | * | 335 | * |
| 303 | * | 336 | *** |
| 304 | * | 337 | *** |
| 307 | * | 338 | *** |
| 308 | * | 339 | *** |
| 309 | * | 340 | *** |
| 310 | * | 341 | *** |
| 311 | *** | 342 | *** |
| 312 | *** | 343 | *** |
| 313 | *** | 344 | *** |
| 314 | *** | 345 | ** |
| 315 | *** | 346 | *** |
| 316 | *** | 347 | *** |
| 317 | *** | 348 | *** |
| 318 | *** | 349 | *** |
| 319 | *** | 350 | *** |
| 320 | *** | 352 | *** |
| 321 | *** | 354 | *** |
| 322 | *** | 355 | ** |
| 323 | *** | 356 | *** |
| 324 | *** | 357 | *** |
| 326 | *** | 358 | *** |
| 327 | *** | 360-3 | *** |
| 328 | *** | 361-2 | *** |

### Test Example 2: Protein degradation-inducing effect in cancer cells (IRAK4 protein)

The degradation-inducing effect of the example compounds on IRAK4 protein in cancer cells was evaluated using a CCRF-CEM cell line, which is a human acute lymphoblastic leukemia cell line. GAPDH was used as a control.

A CCRF-CEM cell line cultured in RPMI-1640 culture medium containing 10% fetal bovine serum (FBS) was seeded onto an evaluation plate. A test substance (an example compound described above) was dissolved in DMSO, a dilution series was prepared, mixed with culture medium, and added to an evaluation plate. The cells were cultured for 24 hours to perform compound exposure. After the culture, the cells were collected and lysed using a cell lysis solution (RIPA buffer, manufactured by Thermo Fisher Scientific) supplemented with a protease inhibitor (Halt Protease & Phosphatase Inhibitor Cocktail, manufactured by Thermo Fisher Scientific). This cell lysate was dissolved in an electrophoresis buffer (NuPAGE LDS Sample Buffer, manufactured by Thermo Fischer Scientific) mixed with a reducing agent (NuPAGE Sample Reducing Agent, manufactured by Thermo Fischer Scientific). The lysate was heat-treated (95 °C, 5 minutes) and then subjected to NuPAGE electrophoresis. Proteins in the NuPAGE gel were transferred to a PVDF membrane using a semi-dry transfer device (iBlot2 system, manufactured by Thermo Fisher Scientific). After the transfer, blocking was performed using a blocking solution (Blocking One, manufactured by Nacalai Tesque), and then antibody reactions were carried out by shaking at room temperature for about 1 hour each with a primary antibody solution (anti-IRAK4 antibody, manufactured by Abcam) and a secondary antibody solution (HRP-labeled anti-rabbit antibody, manufactured by Cell Signaling). The membrane was washed with Tris-buffered saline (Tris-Buffered Saline with Tween 20, liquid, manufactured by Santa Cruz Biotechnology), and then antibody-dependent chemiluminescence was performed using a coloer-developing solution for HRP (SuperSignal West Femto, manufactured by Cell Signaling, or Immobilon Forte Western HRP Substrate, manufactured by Merck Millipore), and a band corresponding to IRAK4 protein on the membrane was detected using an image analysis device (Amersham Imager 600, manufactured by Amersham). As a control for each sample, GAPDH detection was performed in the same manner as estrogen receptor protein detection, using a GAPDH antibody (manufactured by Cell Signaling) to detect a band corresponding to GAPDH protein.

The test results of this test are shown in Table 30 below. In the table, "*" indicates that degradation induction of IRAK4 protein was confirmed at a compound concentration of 10 µM.

**[Table 30]**

| Example | IRAK4 degradation-inducing effect |
|---|---|
| 142 | * |
| 359 | * |

### Test Example 3: Protein degradation-inducing effect in cancer cells (Estrogen Receptor protein)

The degradation-inducing effect of the example compounds on Estrogen Receptor protein in cancer cells was evaluated using a T-47D cell line, which is a breast cancer cell line. GAPDH was used as a control.

A T-47D cell line cultured in RPMI-1640 culture medium containing 10% fetal bovine serum (FBS) and 0.2 Units/mL insulin was seeded into an evaluation plate. A test substance (an example compound described above) was dissolved in DMSO, a dilution series was prepared, mixed with culture medium, and added to an evaluation plate. The cells were cultured for 24 hours to perform compound exposure. After the culture, the cells were collected and lysed using a cell lysis solution (RIPA buffer, manufactured by FUJIFILM Wako Pure Chemical Corporation) supplemented with a protease and phosphatase inhibitor (Halt Protease & Phosphatase Inhibitor Cocktail, manufactured by Thermo Fisher Scientific). This cell lysate was dissolved in an electrophoresis buffer (NuPAGE LDS Sample Buffer, manufactured by Thermo Fischer Scientific) mixed with a reducing agent (NuPAGE Sample Reducing Agent, manufactured by Thermo Fischer Scientific). The lysate was heat-treated (95 °C, 5 minutes) and then subjected to NuPAGE electrophoresis. Proteins in the NuPAGE gel were transferred to a PVDF membrane using a semi-dry transfer device (iBlot2 system, manufactured by Thermo Fisher Scientific). After the transfer, blocking was performed using a blocking solution (Blocking One, manufactured by Nacalai Tesque), and then antibody reactions were carried out by shaking at room temperature for about 1 hour each with a primary antibody solution (anti-Estrogen Receptor antibody, manufactured by Cell Signaling Technology) and a secondary antibody solution (HRP-labeled anti-rabbit antibody, manufactured by Cell Signaling Technology). The membrane was washed with Tris-buffered saline (Tris-Buffered Saline with Tween 20, liquid, manufactured by Santa Cruz Biotechnology) and then antibody-dependent chemiluminescence was performed using a coloer-developing solution for HRP (SuperSignal West Femto, manufactured by Thermo Fisher Scientific, or Immobilon Forte Western HRP Substrate, manufactured by Merck Millipore), and a band corresponding to Estrogen Receptor protein on the membrane was detected using an image analysis device (Amersham Imager 600, manufactured by Amersham). As a control for each sample, GAPDH detection was performed in the same manner as estrogen receptor protein detection, using a GAPDH antibody (manufactured by Cell Signaling) to detect a band corresponding to GAPDH protein.

The test results of this test are shown in Table 31 below. In the table, "*" indicates that degradation induction of Estrogen Receptor (ER) protein was confirmed at a compound concentration of 40 µM.

**[Table 31]**

| Example | ER degradation-inducing effect |
|---|---|
| 360-3 | * |
| 361-2 | * |

### Test Example 4: Protein degradation-inducing effect in cancer cells (BRD4 protein)

The degradation-inducing effect of the example compounds on BRD4 protein in cancer cells was evaluated using an MV-4-11 cell line, which is an acute myeloid leukemia cell line. β-Actin was used as a control.

An MV-4-11 cell line cultured in IMDM culture medium containing 10% fetal bovine serum (FBS) was seeded onto an evaluation plate. A test substance (an example compound described above) was dissolved in DMSO, a dilution series was prepared, mixed with culture medium, and added to an evaluation plate. The cells were cultured for 24 hours to perform compound exposure. After the culture, the cells were collected and lysed using a cell lysis solution (RIPA buffer, manufactured by Thermo Fisher Scientific) supplemented with a protease and phosphatase inhibitor (Halt Protease & Phosphatase Inhibitor Cocktail, manufactured by Thermo Fisher Scientific). This cell lysate was dissolved in an electrophoresis buffer (NuPAGE LDS Sample Buffer, manufactured by Thermo Fischer Scientific) mixed with a reducing agent (NuPAGE Sample Reducing Agent, manufactured by Thermo Fischer Scientific). The lysate was heat-treated (95 °C, 5 minutes) and then subjected to NuPAGE electrophoresis. Proteins in the NuPAGE gel were transferred to a PVDF membrane using a semi-dry transfer device (iBlot2 system, manufactured by Thermo Fisher Scientific). After the transfer, blocking was performed using a blocking solution (Blocking One, manufactured by Nacalai Tesque), and then an antibody reaction was carried out by shaking at room temperature for about 1 hour with a primary antibody solution (anti-BRD4 antibody, manufactured by Abcam). The membrane was washed with Tris-buffered saline (Tris-Buffered Saline with Tween 20, liquid, manufactured by Santa Cruz Biotechnology) and then antibody-dependent chemiluminescence was performed using a coloer-developing solution for HRP (SuperSignal West Femto, manufactured by Thermo Fisher Scientific, or Immobilon Forte Western HRP Substrate, manufactured by Merck Millipore), and a band corresponding to BRD4 protein on the membrane was detected using an image analysis device (Amersham Imager 600, manufactured by Amersham).

As a control for each sample, β-Actin detection was performed in the same manner as BRD4 protein detection, using a primary antibody (anti-β-Actin antibody, manufactured by Cell Signaling) to detect a band corresponding to β-Actin protein.

The test results of this test are shown in Table 32 below. In the table, "***" indicates that degradation induction of BRD4 protein was confirmed at a compound concentration of 0.1 µM; "**" indicates that degradation induction of BRD4 protein was confirmed at a compound concentration of 1 µM; and "*" indicates that degradation induction of BRD4 protein was confirmed at a compound concentration of 10 µM.

**[Table 32]**

| Example | BRD4 degradation-inducing effect | Example | BRD4 degradation-inducing effect |
|---|---|---|---|
| 308 | ** | 329 | * |
| 309 | * | 332 | *** |
| 310 | * | 333 | *** |
| 311 | * | 334 | *** |
| 312 | * | 339 | * |
| 313 | * | 341 | * |
| 318 | * | 342 | ** |
| 319 | * | 345 | * |
| 320 | * | 348 | * |
| 322 | * | 352 | * |
| 323 | * | 353 | ** |
| 324 | * | 354 | * |
| 326 | * | 355 | ** |
| 327 | * | 356 | * |
| 328 | * | | |

### Test Example 5: Protein degradation-inducing effect in cancer cells (IKZF1 protein)

The degradation-inducing effect of the example compounds on IKZF1 protein in cancer cells was evaluated using an MM.1S cell line, which is a B-lymphoblastoid cell line. GAPDH was used as a control.

An MM.1S cell line cultured in RPMI-1640 culture medium containing 10% fetal bovine serum (FBS) was seeded onto an evaluation plate. A test substance (an example compound described above) was dissolved in DMSO, a dilution series was prepared(10 µM, 100 µM), mixed with culture medium, and added to an evaluation plate. The cells were cultured for 24 hours to perform compound exposure. After the culture, the cells were collected and lysed using a cell lysis solution (RIPA buffer, manufactured by FUJIFILM Wako Pure Chemical Corporation) supplemented with a protease and phosphatase inhibitor (Halt Protease & Phosphatase Inhibitor Cocktail, manufactured by Thermo Fisher Scientific). This cell lysate was dissolved in an electrophoresis buffer (NuPAGE LDS Sample Buffer, manufactured by Thermo Fischer Scientific) mixed with a reducing agent (NuPAGE Sample Reducing Agent, manufactured by Thermo Fischer Scientific). The lysate was heat-treated (95 °C, 5 minutes) and then subjected to NuPAGE electrophoresis. Proteins in the NuPAGE gel were transferred to a PVDF membrane using a semi-dry transfer device (iBlot2 system, manufactured by Thermo Fisher Scientific). After the transfer, blocking was performed using a blocking solution (Blocking One, manufactured by Nacalai Tesque), and then antibody reactions were carried out by shaking at room temperature for about 1 hour each with a primary antibody solution (anti-IKZF1 antibody, manufactured by Thermo Fisher Scientific) and a secondary antibody solution (HRP-labeled anti-rabbit antibody, manufactured by Cell Signaling Technology). The membrane was washed with Tris-buffered saline (Tris-Buffered Saline with Tween 20, liquid, manufactured by Santa Cruz Biotechnology) and then antibody-dependent chemiluminescence was performed using a coloer-developing solution for HRP (SuperSignal West Femto, manufactured by Thermo Fisher Scientific, or Immobilon Forte Western HRP Substrate, manufactured by Merck Millipore), and a band corresponding to IKZF1 protein on the membrane was detected using an image analysis device (Amersham Imager 600, manufactured by Amersham). As a control for each sample, GAPDH detection was performed in the same manner as IKZF1 protein detection, using a GAPDH antibody (manufactured by Cell Signaling) to detect a band corresponding to GAPDH protein.

The test results of this test are shown in Table 33 below. In the table, "n" indicates that there was no degradation induction of IKZF1 protein; and "*" indicates that there was degradation induction of IKZF1 protein.

**[Table 33]**

| Example | IKZF1 degradation-inducing effect (10 µM) | IKZF1 degradation-inducing effect (100 µM) |
|---|---|---|
| 36 | n | n |
| 73 | n | n |
| 90-1 | n | n |
| 94 | n | * |
| 115 | n | n |
| 116 | n | n |
| 128 | n | n |
| 134 | n | n |
| 135 | n | n |
| 138 | n | n |
| 139 | n | n |
| 149 | n | n |
| 176 | n | n |
| 197 | n | n |
| 210 | n | n |
| 213 | n | n |
| 223 | n | n |
| 235 | n | n |
| 246 | n | n |

### Test Example 6: Protein degradation-inducing effect in cancer cells (SALL4 protein)

The degradation-inducing effect of the example compounds on SALL4 protein in cancer cells was evaluated using a KELLY cell line, which is a human neuroblastoma cell line. β-Actin was used as a control.

A KELLY cell line cultured in RPMI-1640 culture medium containing 10% fetal bovine serum (FBS) was seeded onto an evaluation plate. A test substance (an example compound described above) was dissolved in DMSO, a dilution series was prepared(10 µM, 100 µM), mixed with culture medium, and added to an evaluation plate. The cells were cultured for 24 hours to perform compound exposure. After the culture, the cells were collected and lysed using a cell lysis solution (RIPA buffer, manufactured by Thermo Fisher Scientific) supplemented with a protease and phosphatase inhibitor (Halt Protease & Phosphatase Inhibitor Cocktail, manufactured by Thermo Fisher Scientific). This cell lysate was dissolved in an electrophoresis buffer (NuPAGE LDS Sample Buffer, manufactured by Thermo Fischer Scientific) mixed with a reducing agent (NuPAGE Sample Reducing Agent, manufactured by Thermo Fischer Scientific). The lysate was heat-treated (95 °C, 5 minutes) and then subjected to NuPAGE electrophoresis. Proteins in the NuPAGE gel were transferred to a PVDF membrane using a semi-dry transfer device (iBlot2 system, manufactured by Thermo Fisher Scientific). After the transfer, blocking was performed using a blocking solution (Blocking One, manufactured by Nacalai Tesque), and then antibody reactions were carried out by shaking at room temperature for about 1 hour each with a primary antibody solution (anti-SALL4 antibody, manufactured by Proteintech Group Inc.) and a secondary antibody solution (HRP-labeled anti-rabbit antibody, manufactured by Cell Signaling Technology). The membrane was washed with Tris-buffered saline (Tris-Buffered Saline with Tween 20, liquid, manufactured by Santa Cruz Biotechnology) and then antibody-dependent chemiluminescence was performed using a coloer-developing solution for HRP (SuperSignal West Femto, manufactured by Thermo Fisher Scientific, or Immobilon Forte Western HRP Substrate, manufactured by Merck Millipore), and a band corresponding to SALL4 protein on the membrane was detected using an image analysis device (Amersham Imager 600, manufactured by Amersham). As a control for each sample, β-Actin detection was performed in the same manner as SALL4 protein detection, using a primary antibody (anti-β-Actin antibody, manufactured by Cell Signaling) to detect a band corresponding to β-Actin protein.

The test results of this test are shown in Table 34 below. In the table, "n" indicates that there was no degradation induction of SALL4 protein; and "*" indicates that there was degradation induction of SALL4 protein.

**[Table 34]**

| Example | SALL4 degradation-inducing effect (10 µM) | SALL4 degradation-inducing effect (100 µM) |
|---|---|---|
| 36 | n | n |
| 73 | n | n |
| 90-1 | n | n |
| 94 | n | n |
| 115 | n | n |
| 116 | n | n |
| 128 | n | n |
| 134 | n | n |
| 135 | n | n |
| 138 | n | n |
| 139 | n | n |
| 149 | n | n |
| 176 | n | n |
| 197 | n | n |
| 210 | n | n |
| 213 | n | n |
| 223 | n | n |
| 235 | n | n |
| 238 | n | * |
| 246 | n | n |

### Test Example 7: Stability against racemization

Each test sample (1 mg) was dissolved in CH₃CN (1 mL), and 0.1 M N-methylmorpholine aqueous solution (adjusted to pH 7.4 with 1N HCl) (1 mL) was added to dilute the solution twofold, thereby preparing a solution with a final concentration of 0.5 mg/mL. The sample was divided into nine portions of 150 µL each, and each portion was placed in a sampler set to 37°C. The enantiomers in the sample were quantified at 0, 1, 3, 6, 24, 48, 72, and 167 hours under the following HPLC conditions, and the enantiomeric excess (ee) at each time point was calculated. The results are shown in Table 35.

As test samples, Example Compound 211-1 was used as an example, while (S)-thalidomide represented by the following formula 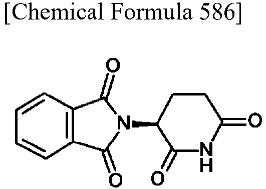 and a compound (Compound A') represented by the following formula 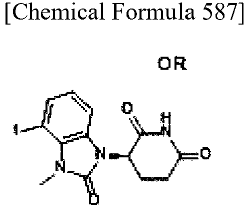 were used as comparative examples. Compound A' was synthesized with reference to the method described in WO2019/060693.

### HPLC Conditions

### [Common]

- Equipment: ACQUITY UPLC H-Class Waters
- Column: CHIRAKPAK IE-3 (3µm, 4.6 × 150 mm) Daicel Corporation
- Flow rate: 0.5mL/min
- Column temperature: 25°C

### [Thalidomide]

- Mobile phase: 100% MeOH
- Injection volume: 0.7 µL
- Detection wavelength: 217.0 nm

### [Compound A']

- Mobile phase: 100% MeOH
- Injection volume: 1.2 µL
- Detection wavelength: 221.0 nm

### [Example Compound 211-1]

- Mobile phase: EtOH/CH₃CN = 90/10
- Injection volume: 1.5 µL
- Detection wavelength: 221.0 nm

**[Table 35]**

| | Example 211-1 | | (S) Thalidomide | | Compound A' | |
|---|---|---|---|---|---|---|
| Time (h) | ee | Normalized to 100 at 0 min | ee | Normalized to 100 at 0 min | ee | Normalized to 100 at 0 min |
| Initial | 99.44 | | 92.16 | | 95.39 | |
| 0 | 99.43 | 100 | 92.11 | 100 | 96.08 | 100 |
| 24 | 87.71 | 88.21 | 73.31 | 79.59 | 77.77 | 80.94 |
| 48 | 77.71 | 78.16 | 58.25 | 63.24 | 62.84 | 65.4 |
| 72 | 70.06 | 70.46 | 46.27 | 50.23 | 50.69 | 52.76 |
| 167 | 41.83 | 42.07 | 18.57 | 20.16 | 21.53 | 22.41 |

## Claims

1. A compound represented by the following Formula (I): 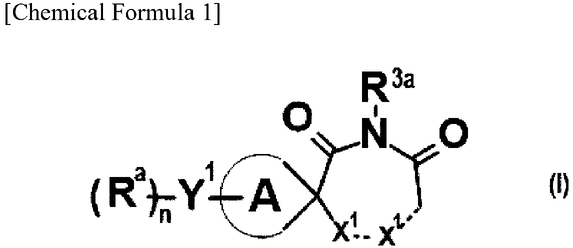 [wherein
a dotted line represents a single bond or a double bond,
two X¹ groups are independently selected from CH and CH₂, or one X¹ is CH₂ and the other X¹ is O, NH, NCH₃, or S,
Ring A is a saturated or partially unsaturated 3- to 8-membered ring that may contain 1 to 3 atoms independently selected from nitrogen, oxygen, and sulfur atoms, and the ring may be substituted with one or more substituents independently selected from a group consisting of a fluorine atom, a methyl group, an ethyl group, and an oxo group,
R^{3a} is a hydrogen atom, a methyl group, -OH, -CH₂OC(O)R'^{a}, -CH₂OP(O)OHOR'^{a}, - CH₂OP(O)R'^{a}₂, or -CH₂OP(O)(OR'^{a})₂,
R'^{a} is a C₁₋₄ alkyl group,
n is 0 or 1,
when n is 0,
Y¹ is an optionally substituted C₁₋₁₀ alkyl group, an optionally substituted C₁₋₁₀ alkoxy group, an optionally substituted C₁₋₁₀ alkylcarbonyl group, or an optionally substituted C₁₋₁₀ alkoxycarbonyl group,
when n is 1,
Y¹ is a single bond, CO, SO₂, CH₂CO, or a C₁₋₄ alkylene group, and
R^{a} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or the following Formula (II): 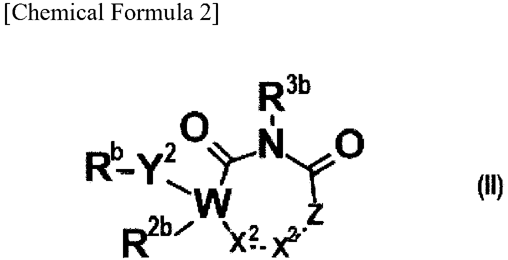
[wherein
a dotted line represents a single bond or a double bond,
two X² groups are independently selected from CH and CH₂, or one X² is CH₂ and the other X² is O, NH, NCH₃, or S,
Z is a single bond or CH₂,
R^{2b} is a hydrogen atom, a fluorine atom, or a methyl group,
R^{3b} is a hydrogen atom, a methyl group, -OH, -CH₂OC(O)R'^{b}, -CH₂OP(O)OHOR'^{b}, - CH₂OP(O)R'^{b}₂, or -CH₂OP(O)(OR'^{b})₂,
R'^{b} is a C₁₋₄ alkyl group,
when Z is CH₂,
W is C or N, when W is N, R^{2b} is absent,
Y² is a single bond, -CR"R"'-, -O-, -NR"-, -NR"C(O)-, -C(O)NR"-, -NR"SO₂-, -SO₂NR"-, - CH₂NR"-, -NR"CH₂-, -C(O)NR"CH₂-, -CH₂NR"C(O)-, -CH₂N(COR")-, -N(COR")CH₂-, - CH₂N(COOR")-, or -N(COOR")CH₂-,
R" and R‴ are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms,
R^{b} is an optionally substituted 6- to 10-membered aryl group (provided that when W is N, a 6-membered aryl group is excluded), an optionally substituted 5- to 7-membered monocyclic heteroaryl group, an optionally substituted 5- to 7-membered monocyclic heterosaturated ring group (provided that when W is N, a tetrahydrofuranyl group is excluded), an optionally substituted and optionally partially saturated 8- to 10-membered bicyclic heteroaryl group (provided that when W is C, the following structures: 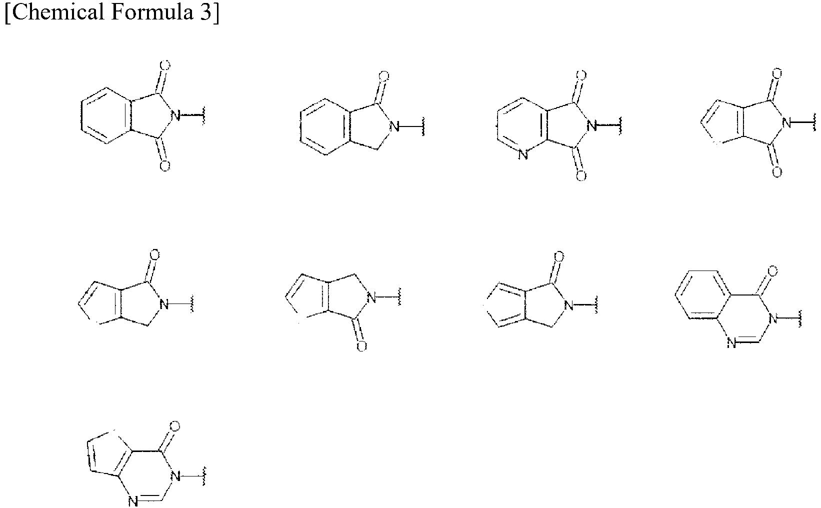
(wherein the ring structures are unsubstituted or substituted with 1 to 5 arbitrary substituents) are excluded), or an optionally substituted 5-4 spiro ring group which may contain 1 to 3 atoms independently selected from nitrogen, oxygen, and sulfur atoms,
when Z is a single bond,
W is C,
Y² is an optionally substituted 5-4 spiro ring divalent group which may contain 1 to 3 atoms independently selected from nitrogen, oxygen, and sulfur atoms, wherein the 5-4 spiro ring is bonded to W at any position of its 5-membered ring, and
R^{b} is an optionally substituted C₁₋₁₀ alkyl group, an optionally substituted C₁₋₁₀ alkylcarbonyl group, an optionally substituted C₁₋₁₀ alkoxy group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or the following Formula (III): 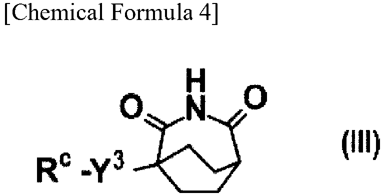
[wherein
Y³ is a single bond or -NH-, and
R^{c} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or the following Formula (IV): 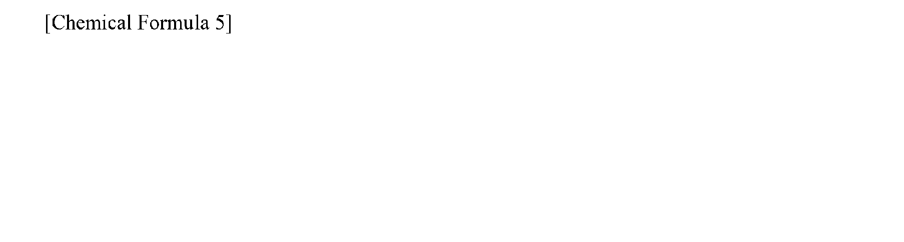 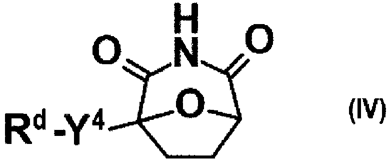
[wherein
Y⁴ is a single bond, and
R^{d} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group],
or a pharmacologically acceptable salt thereof (provided that the following compounds: 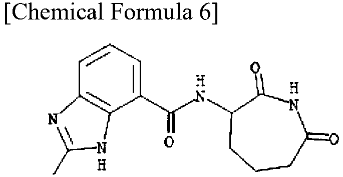 and 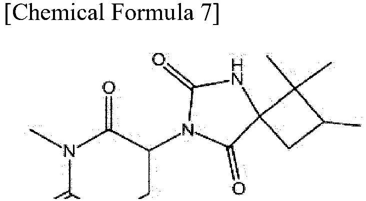
are excluded).

2. The compound according to Claim 1 or a pharmacologically acceptable salt thereof, wherein the compound represented by the above Formula (I) is a compound represented by the following Formula (IA): 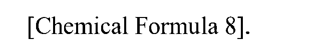 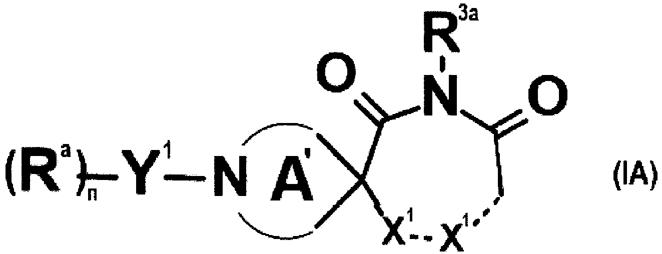 [wherein
a dotted line represents a single bond or a double bond,
two X¹ groups are independently selected from CH and CH₂, or one X¹ is CH₂ and the other X¹ is O, NH, NCH₃, or S,
Ring A' is a 3- to 8-membered monocyclic heterosaturated ring containing a nitrogen atom bonded to Y¹ and optionally containing 1 to 2 atoms independently selected from nitrogen, oxygen, and sulfur atoms, and the ring may be substituted with 1 to 2 substituents independently selected from a group consisting of a fluorine atom, a methyl group, an ethyl group, and an oxo group,
R^{3a} is a hydrogen atom, a methyl group, or -OH,
n is 0 or 1,
when n is 0,
Y¹ is an optionally substituted C₁₋₁₀ alkyl group, an optionally substituted C₁₋₁₀ alkoxy group, an optionally substituted C₁₋₁₀ alkylcarbonyl group, or an optionally substituted C₁₋₁₀ alkoxycarbonyl group,
when n is 1,
Y¹ is a single bond, CO, SO₂, -CH₂CO-, or a C₁₋₄ alkylene group, and
R^{a} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group].

3. The compound according to Claim 2 or a pharmacologically acceptable salt thereof, wherein
in the above Formula (IA),
a dotted line represents a single bond or a double bond,
two X¹ groups are independently selected from CH and CH₂, or one X¹ is CH₂ and the other X¹ is O,
Ring A' is a 3- to 8-membered monocyclic heterosaturated ring containing a nitrogen atom bonded to Y¹ and optionally containing 1 to 2 atoms independently selected from nitrogen, oxygen, and sulfur atoms, and the ring may be substituted with 1 to 2 substituents independently selected from a group consisting of a fluorine atom, a methyl group, an ethyl group, and an oxo group,
R^{3a} is a hydrogen atom,
n is 1,
Y¹ is a single bond, CO, SO₂, -CH₂CO-, or a C₁₋₄ alkylene group, and
R^{a} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

4. The compound according to Claim 3 or a pharmacologically acceptable salt thereof, wherein
in the above Formula (IA),
a dotted line represents a single bond,
two X¹ groups are CH₂,
Ring A' is a 4- to 6-membered monocyclic heterosaturated ring containing a nitrogen atom bonded to Y¹ and optionally containing 1 to 2 atoms independently selected from nitrogen and oxygen atoms, and the ring may be substituted with 1 to 2 substituents independently selected from a group consisting of a fluorine atom, a methyl group, an ethyl group, and an oxo group,
R^{3a} is a hydrogen atom,
n is 1,
Y¹ is a single bond, CO, SO₂, -CH₂CO-, or a C₁₋₄ alkylene group, and
R^{a} is an optionally substituted phenyl group, an optionally substituted naphthyl group, an optionally substituted dihydroindenyl group, an optionally substituted 5- to 6-membered monocyclic heteroaryl group, an optionally substituted 5- to 6-membered monocyclic heterosaturated ring group, or a bicyclic heteroaryl group selected from a group consisting of the following: 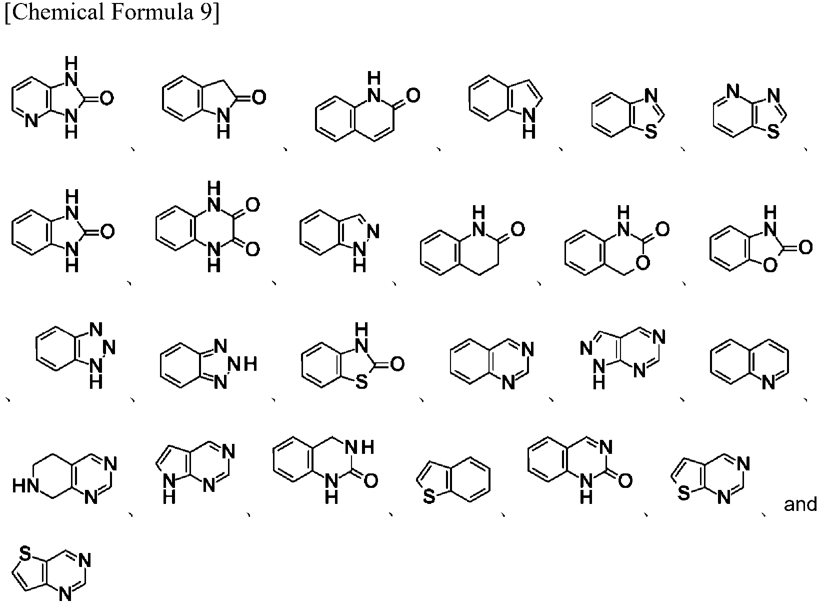
(wherein the bicyclic heteroaryl group may be substituted).

5. The compound according to any one of Claims 2 to 4 or a pharmacologically acceptable salt thereof, wherein
in the above Formula (IA),
Ring A' is a 5- to 6-membered monocyclic heterosaturated ring containing a nitrogen atom bonded to Y¹, and the ring may be substituted with 1 to 2 oxo groups.

6. The compound according to Claim 5 or a pharmacologically acceptable salt thereof, wherein
in the above Formula (IA),
Ring A' is a 5-membered monocyclic heterosaturated ring containing a nitrogen atom bonded to Y¹, and the ring may be substituted with 1 to 2 oxo groups.

7. The compound according to any one of Claims 2 to 6 or a pharmacologically acceptable salt thereof, wherein
in the above Formula (IA),
n is 1, and
Y¹ is a single bond or CO.

8. The compound according to any one of Claims 3 to 7 or a pharmacologically acceptable salt thereof, wherein
in the above Formula (IA),
n is 1, and
R^{a} is an optionally substituted phenyl group, an optionally substituted naphthyl group, an optionally substituted pyridinyl group, an optionally substituted pyridazinyl group, an optionally substituted pyrimidinyl group, an optionally substituted pyrazinyl group, an optionally substituted thiazolyl group, an optionally substituted piperidinyl group, an optionally substituted piperazinyl group, or a bicyclic heteroaryl group selected from a group consisting of the following: 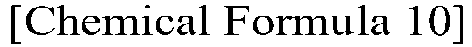 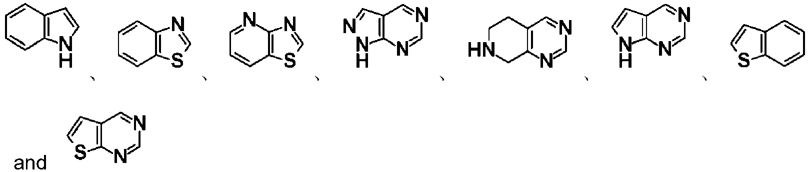
(wherein the bicyclic heteroaryl group may be substituted).

9. The compound according to any one of Claims 3 to 8 or a pharmacologically acceptable salt thereof, wherein
in the above Formula (IA),
n is 1, and
R^{a} is an optionally substituted nitrogen-containing heterocycle, and Ra has a bond to Y¹ on a nitrogen atom that is a member of the ring.

10. The compound according to Claim 1 or a pharmacologically acceptable salt thereof, wherein
the compound is a compound represented by the above Formula (II), and in the above Formula (II),
a dotted line represents a single bond or a double bond,
two X² groups are independently selected from CH and CH₂, or one X² is CH₂ and the other X² is O, NH, NCH₃, or S,
Z is a single bond,
R^{2b} is a hydrogen atom, a fluorine atom, or a methyl group,
R^{3b} is a hydrogen atom, a methyl group, or -OH,
W is C,
Y² is an optionally substituted 5-4 spiro ring divalent group which may contain 1 to 3 atoms independently selected from nitrogen, oxygen, and sulfur atoms, wherein the 5-4 spiro ring is bonded to W at any position of its 5-membered ring, and
R^{b} is an optionally substituted C₁₋₁₀ alkyl group, an optionally substituted C₁₋₁₀ alkylcarbonyl group, an optionally substituted C₁₋₁₀ alkoxy group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

11. The compound according to Claim 1 or a pharmacologically acceptable salt thereof, wherein
the compound is a compound represented by the above Formula (II), and in the above Formula (II),
a dotted line represents a single bond or a double bond,
two X² groups are independently selected from CH and CH₂, or one X² is CH₂ and the other X² is O, NH, NCH₃, or S,
Z is CH₂,
R^{2b} is a hydrogen atom, a fluorine atom, or a methyl group,
R^{3b} is a hydrogen atom, a methyl group, or -OH,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R"' are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted 5-4 spiro ring group which may contain 1 to 3 atoms independently selected from nitrogen, oxygen, and sulfur atoms.

12. The compound according to Claim 1 or a pharmacologically acceptable salt thereof, wherein
the compound is a compound represented by the above Formula (II), and in the above Formula (II),
a dotted line represents a single bond or a double bond,
two X² groups are independently selected from CH and CH₂, or one X² is CH₂ and the other X² is O, NH, NCH₃, or S,
Z is CH₂,
R^{2b} is a hydrogen atom, a fluorine atom, or a methyl group,
R^{3b} is a hydrogen atom, a methyl group, or -OH,
W is N, R^{2b} is absent,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R"' are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted naphthyl group, an optionally substituted 5- to 7-membered monocyclic heteroaryl group, an optionally substituted 5- to 7-membered monocyclic heterosaturated ring group (provided that a tetrahydrofuranyl group is excluded), an optionally substituted and optionally partially saturated 8- to 10-membered bicyclic heteroaryl group, or an optionally substituted 5-4 spiro ring group which may contain 1 to 3 atoms independently selected from nitrogen, oxygen, and sulfur atoms.

13. The compound according to Claim 10 or a pharmacologically acceptable salt thereof, wherein
in the above Formula (II),
a dotted line represents a single bond or a double bond,
two X² groups are independently selected from CH and CH₂, or one X² is CH₂ and the other X² is O,
Z is a single bond,
R^{2b} is a hydrogen atom, a fluorine atom, or a methyl group,
R^{3b} is a hydrogen atom,
W is C,
Y² is an optionally substituted 5-4 spiro ring divalent group which may contain 1 to 3 atoms independently selected from nitrogen, oxygen, and sulfur atoms, wherein the 5-4 spiro ring is bonded to W at any position of its 5-membered ring, and
R^{b} is an optionally substituted C₁₋₁₀ alkyl group, an optionally substituted C₁₋₁₀ alkylcarbonyl group, an optionally substituted C₁₋₁₀ alkoxy group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

14. The compound according to Claim 13 or a pharmacologically acceptable salt thereof, wherein
in the above Formula (II),
a dotted line represents a single bond,
two X² groups are CH₂,
Z is a single bond,
R^{2b} is a hydrogen atom, or a methyl group,
R^{3b} is a hydrogen atom,
W is C,
Y² is selected from a group consisting of the following: 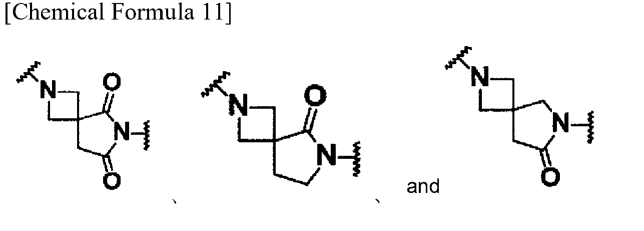
wherein a wavy line represents a point of attachment to W and R^{b}, and
R^{b} is an optionally substituted C₁₋₁₀ alkyl group, an optionally substituted C₁₋₁₀ alkylcarbonyl group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

15. The compound according to Claim 14 or a pharmacologically acceptable salt thereof, wherein
the compound represented by the above Formula (II) is selected from a group consisting of the following: 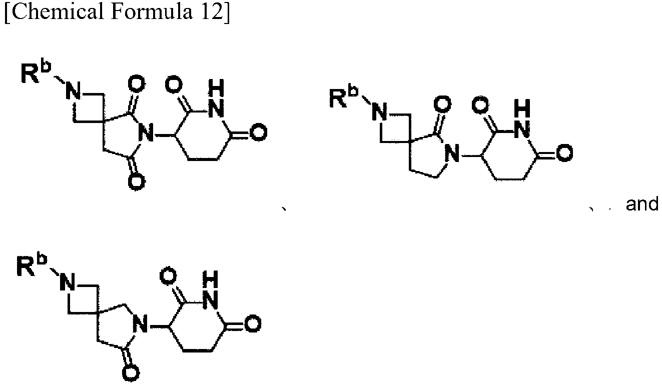
wherein R^{b} is an optionally substituted C₁₋₁₀ alkyl group, an optionally substituted C₁₋₁₀ alkylcarbonyl group, an optionally substituted benzoyl group, an optionally substituted 6- to 10-membered aryl group, an optionally substituted 6- to 10-membered arylsulfonyl group, an optionally substituted 5- to 7-membered monocyclic heteroaryl group, an optionally substituted 5- to 7-membered monocyclic heterosaturated ring group, or an optionally substituted and optionally partially saturated 8- to 10-membered bicyclic heteroaryl group.

16. The compound according to any one of Claims 13 to 15 or a pharmacologically acceptable salt thereof, wherein
in the above Formula (II),
R^{b} is an optionally substituted C₁₋₈ alkyl group, a tert-butoxycarbonyl group, an optionally substituted benzoyl group, an optionally substituted phenyl group, an optionally substituted phenylsulfonyl group, an optionally substituted pyridinyl group, an optionally substituted pyrimidinyl group, an optionally substituted piperidinyl group, or a bicyclic heteroaryl group of the following: 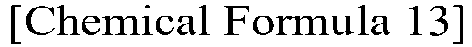 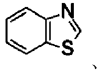 or 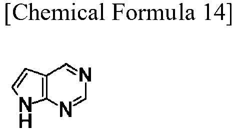
(wherein the bicyclic heteroaryl groups may be substituted).

17. The compound according to any one of Claims 13 to 16 or a pharmacologically acceptable salt thereof, wherein
R^{b} is an optionally substituted nitrogen-containing heterocycle, and R^{b} has a bond to Y² on a nitrogen atom that is a member of the ring.

18. The compound according to Claim 11 or a pharmacologically acceptable salt thereof, wherein
in the above Formula (II),
a dotted line represents a single bond or a double bond,
two X² groups are independently selected from CH and CH₂, or one X² is CH₂ and the other X² is O,
Z is CH₂,
R^{2b} is a hydrogen atom, a fluorine atom, or a methyl group,
R^{3b} is a hydrogen atom,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R‴ are each independently a hydrogen atom or a methyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted 6- to 10-membered aryl group, an optionally substituted 5- to 7-membered monocyclic heteroaryl group, an optionally substituted 5- to 7-membered monocyclic heterosaturated ring group, or an optionally substituted 5-4 spiro ring group which may contain 1 to 3 atoms independently selected from nitrogen, oxygen, and sulfur atoms.

19. The compound according to Claim 18 or a pharmacologically acceptable salt thereof, wherein
in the above Formula (II),
a dotted line represents a single bond or a double bond,
two X² groups are CH₂,
Z is CH₂,
R^{2b} is a hydrogen atom, a fluorine atom, or a methyl group,
R^{3b} is a hydrogen atom,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R"' are each independently a hydrogen atom or a methyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted phenyl group, an optionally substituted naphthyl group, an optionally substituted dihydroindenyl group, an optionally substituted 5- to 6-membered monocyclic heteroaryl group, an optionally substituted 5- to 6-membered monocyclic heterosaturated ring group, or a 5-4 spiro ring group of the following: 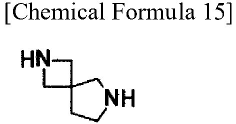
(wherein the 5-4 spiro ring group may be substituted).

20. The compound according to Claim 19 or a pharmacologically acceptable salt thereof, wherein
in the above Formula (II),
R^{2b} is a hydrogen atom or a fluorine atom,
Y² is a single bond, and
R^{b} is an optionally substituted phenyl group, an optionally substituted dihydroindenyl group, an optionally substituted 5- to 6-membered monocyclic heteroaryl group, an optionally substituted 5- to 6-membered monocyclic heterosaturated ring group, or a 5-4 spiro ring group of the following: 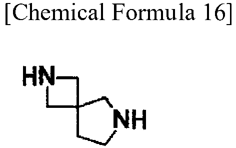
(wherein the 5-4 spiro ring group may be substituted).

21. The compound according to Claim 20 or a pharmacologically acceptable salt thereof, wherein
in the above Formula (II),
R^{b} is an optionally substituted phenyl group, a 5- to 6-membered monocyclic heteroaryl group optionally substituted with an oxo group (wherein the monocyclic heteroaryl group may be further substituted with a substituent other than an oxo group), a 5- to 6-membered monocyclic heterosaturated ring group optionally substituted with an oxo group (wherein the monocyclic heterosaturated ring group may be further substituted with a substituent other than an oxo group), or a 5-4 spiro ring group selected from the following: 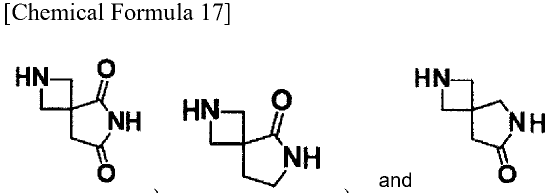
(wherein the 5-4 spiro ring group may be further substituted with a substituent other than an oxo group).

22. The compound according to any one of Claims 18 to 21 or a pharmacologically acceptable salt thereof, wherein
in the above Formula (II), R^{b} is an optionally substituted phenyl group, an optionally substituted pyridinyl group, an optionally substituted pyridazinyl group, an optionally substituted triazolyl group, an optionally substituted pyrrolidinyl group, an optionally substituted piperazinyl group, or a 5-4 spiro ring group of the following: 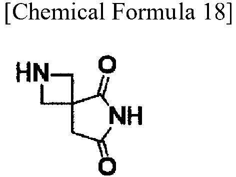
(wherein the 5-4 spiro ring group may be further substituted).

23. The compound according to any one of Claims 18 to 22 or a pharmacologically acceptable salt thereof, wherein
R^{b} is an optionally substituted nitrogen-containing heterocycle, and R^{b} has a bond to Y² on a nitrogen atom that is a member of the ring.

24. The compound according to Claim 12 or a pharmacologically acceptable salt thereof, wherein
in the above Formula (II),
a dotted line represents a single bond or a double bond,
two X² groups are independently selected from CH and CH₂, or one X² is CH₂ and the other X² is O,
Z is CH₂,
R^{3b} is a hydrogen atom,
W is N, R^{2b} is absent,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R"' are each independently a hydrogen atom or a methyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted naphthyl group, an optionally substituted dihydroindenyl group, an optionally substituted 5- to 7-membered monocyclic heteroaryl group, an optionally substituted 5- to 7-membered monocyclic heterosaturated ring group (provided that a tetrahydrofuranyl group is excluded), an optionally substituted and optionally partially saturated 8- to 10-membered bicyclic heteroaryl group, or an optionally substituted 5-4 spiro ring group which may contain 1 to 3 atoms independently selected from nitrogen, oxygen, and sulfur atoms.

25. The compound according to Claim 24 or a pharmacologically acceptable salt thereof, wherein
in the above Formula (II),
a dotted line represents a single bond or a double bond,
two X² groups are CH₂,
Z is CH₂,
R^{3b} is a hydrogen atom,
W is N, R^{2b} is absent,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R"' are each independently a hydrogen atom or a methyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is an optionally substituted naphthyl group, an optionally substituted dihydroindenyl group, an optionally substituted 5- to 6-membered monocyclic heteroaryl group, an optionally substituted 5- to 6-membered monocyclic heterosaturated ring group (provided that a tetrahydrofuranyl group is excluded), a bicyclic heteroaryl group selected from a group consisting of the following: 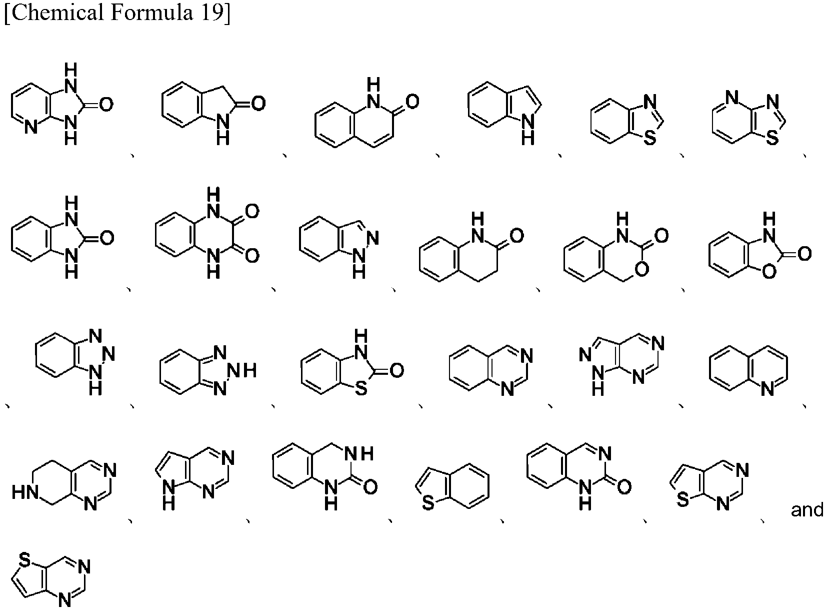
(wherein the bicyclic heteroaryl group may be substituted), or a 5-4 spiro ring group of the following: 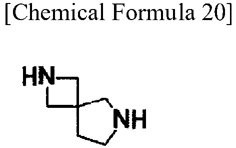
(wherein the 5-4 spiro ring group may be substituted).

26. The compound according to Claim 24 or 25 or a pharmacologically acceptable salt thereof, wherein
R^{b} is an optionally substituted nitrogen-containing heterocycle, and R^{b} has a bond to Y¹ on a nitrogen atom that is a member of the ring.

27. The compound according to Claim 1 or a pharmacologically acceptable salt thereof, wherein
the compound is a compound represented by the above Formula (II), and in the above Formula (II),
a dotted line represents a single bond or a double bond,
two X² groups are independently selected from CH and CH₂, or one X² is CH₂ and the other X² is O, NH, NCH₃, or S,
R^{2b} is a hydrogen atom, a fluorine atom, or a methyl group,
R^{3b} is a hydrogen atom, a methyl group, or -OH,
Z is CH₂,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R‴ are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is a bicyclic group selected from a group consisting of the following: 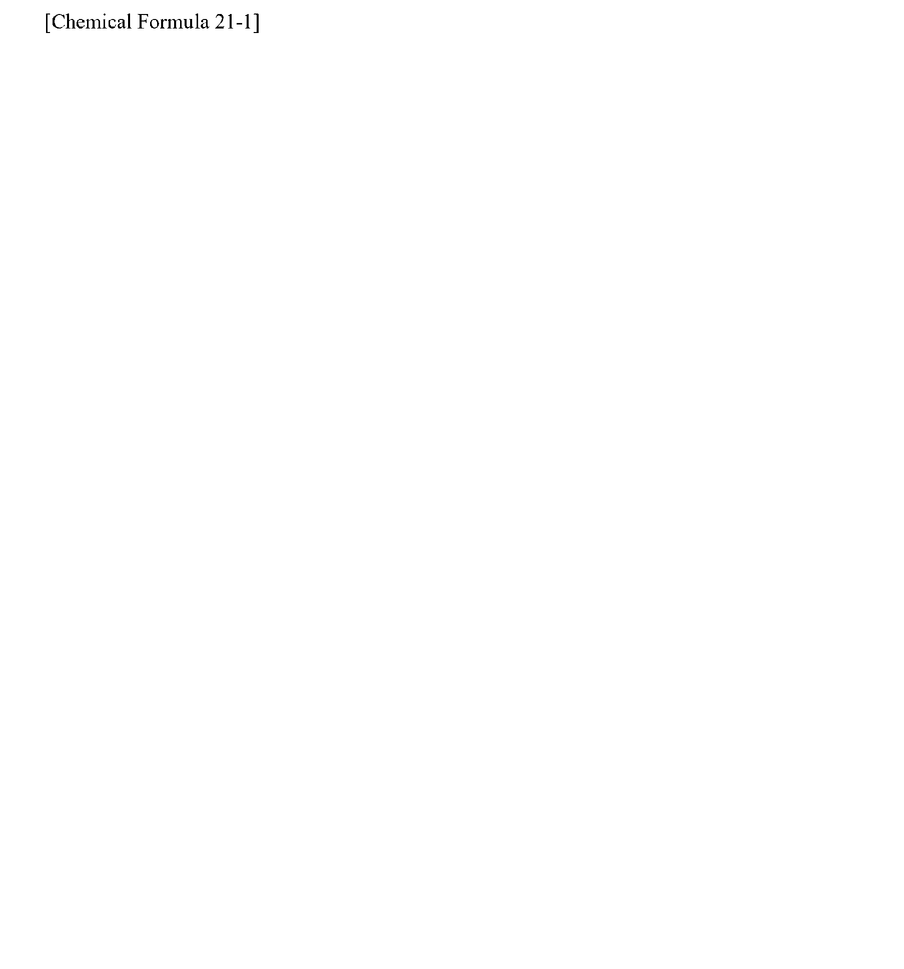 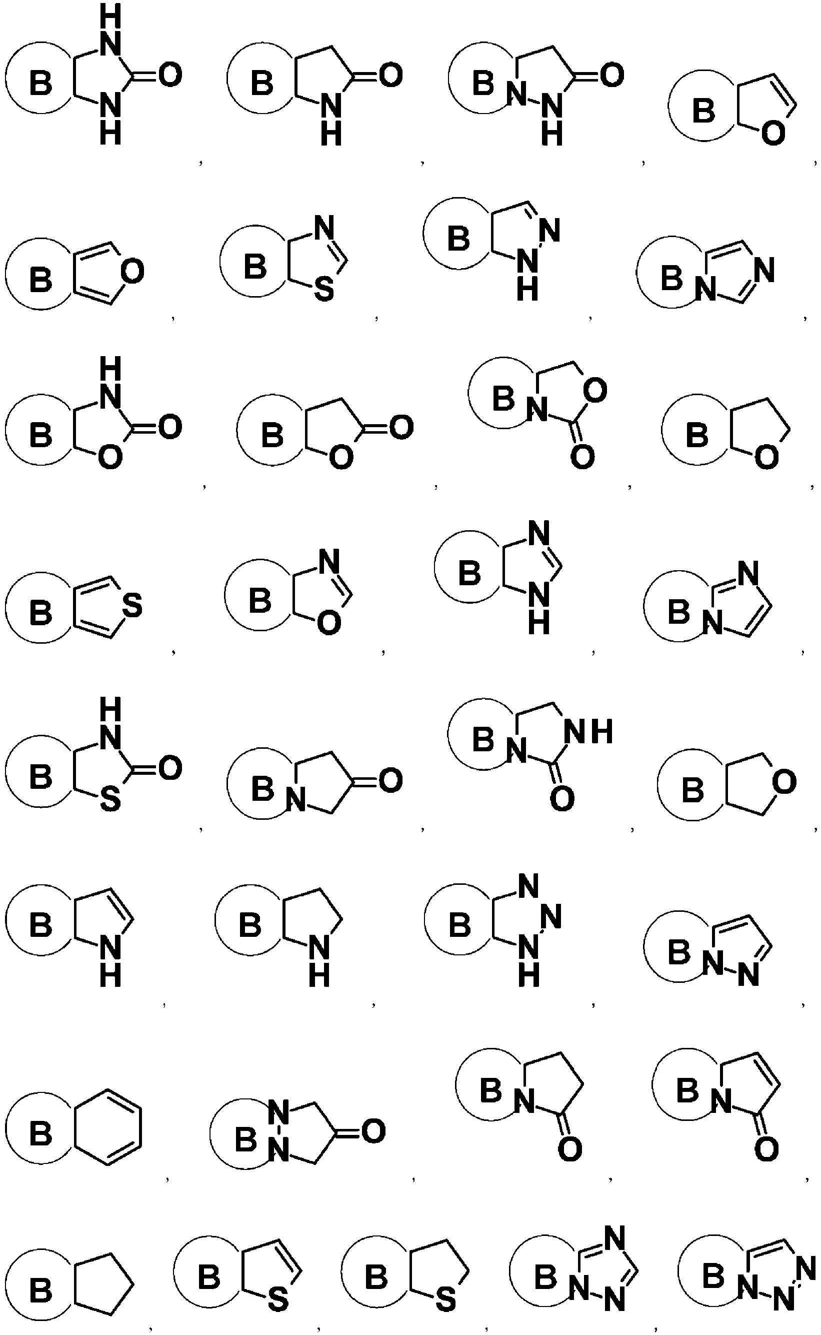 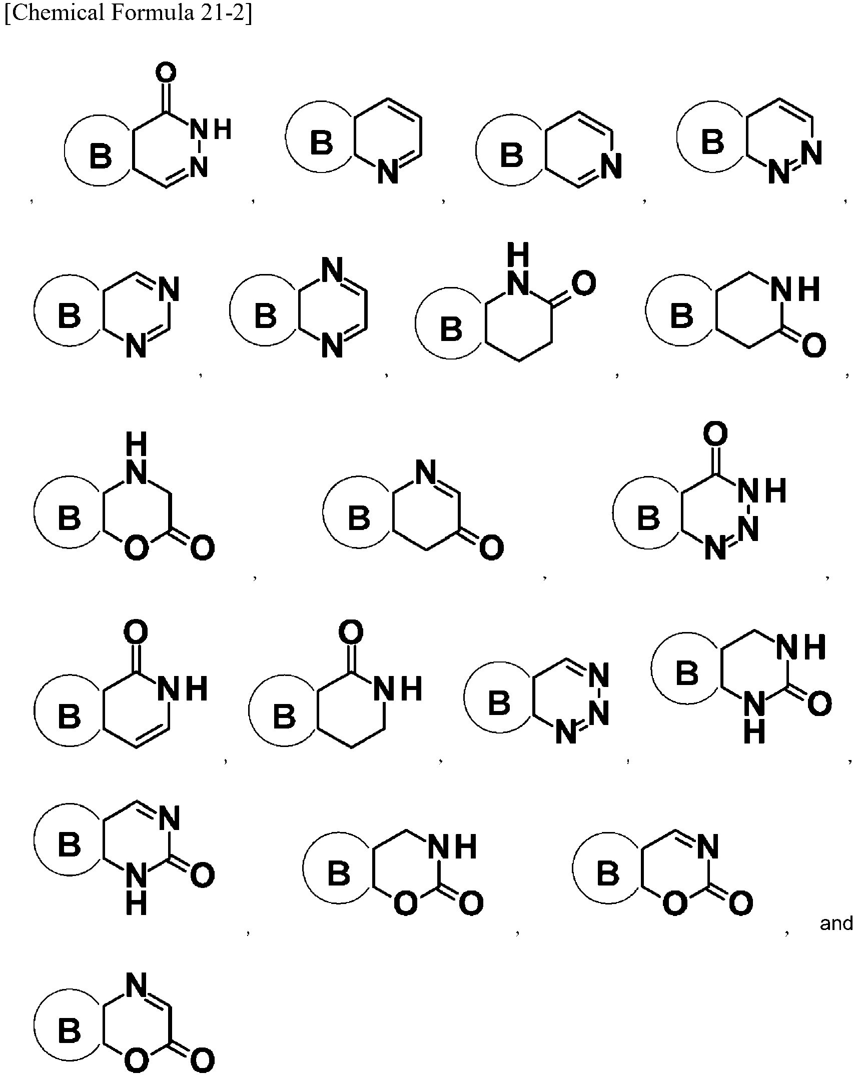
wherein Ring B is a 4- to 6-membered ring that may contain 1 to 3 atoms independently selected from nitrogen, oxygen, and sulfur atoms, and is saturated, partially unsaturated, or aromatic, and the bicyclic group may be substituted.

28. The compound according to Claim 27 or a pharmacologically acceptable salt thereof, wherein
in the above Formula (II),
a dotted line represents a single bond,
two X² groups are CH₂,
R^{2b} is a hydrogen atom, a fluorine atom, or a methyl group,
R^{3b} is a hydrogen atom,
Z is CH₂,
W is C,
Y² is a single bond, -CR"R"'-, -O-, -NH-, -N(CH₃)-, -NHC(O)-, -C(O)NH-, -NHSO₂-, - N(CH₃)SO₂-, -SO₂NH-, -SO₂N(CH₃)-, -CH₂NH-, -NHCH₂-, -C(O)NHCH₂-, -CH₂NHC(O)-, - CH₂N(COCH₃)-, -N(COCH₃)CH₂-, -CH₂N(COO-tBu)-, or -N(COO-tBu)CH₂-,
R" and R"' are each independently a hydrogen atom or an optionally substituted C₁₋₄ alkyl group, or R" and R‴ together form an optionally substituted saturated or partially unsaturated 3- to 8-membered ring which may contain 1 to 3 atoms independently selected from nitrogen, oxygen, and sulfur atoms, and
R^{b} is a bicyclic group selected from a group consisting of the following: 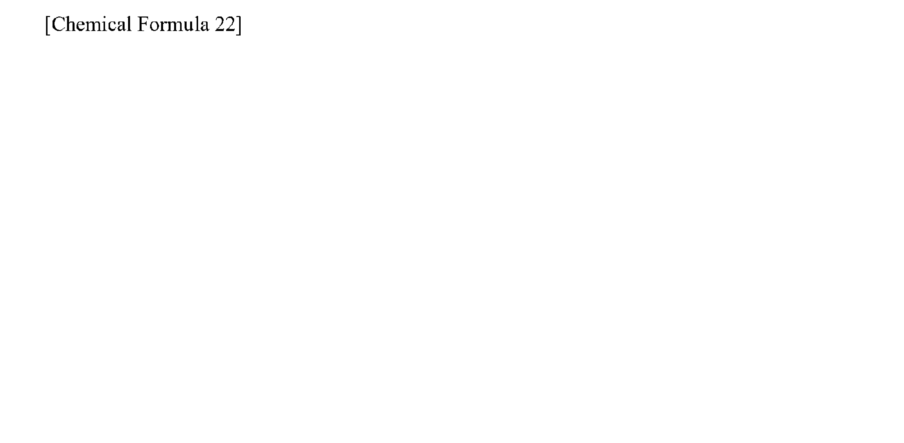 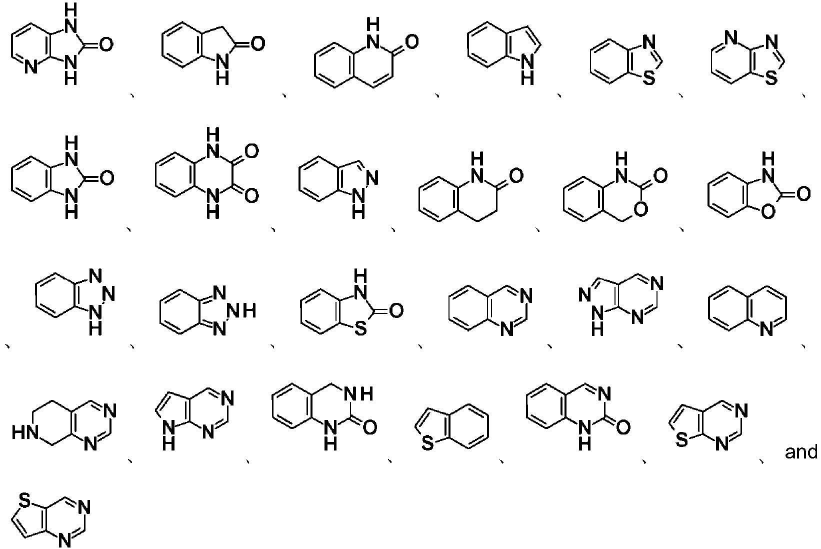
(wherein the bicyclic group may be substituted).

29. The compound according to Claim 28 or a pharmacologically acceptable salt thereof, wherein
in the above Formula (II), Y² is a single bond, and R^{2b} is a hydrogen atom.

30. The compound according to any one of Claims 27 to 29 or a pharmacologically acceptable salt thereof, wherein
R^{b} is an optionally substituted nitrogen-containing heterocycle, and R^{b} has a bond to Y² on a nitrogen atom that is a member of the ring.

31. The compound according to Claim 1 or a pharmacologically acceptable salt thereof, wherein
the compound is a compound represented by the above Formula (III), and in the above Formula (III), R^{c} is an optionally substituted 6- to 10-membered aryl group.

32. The compound according to Claim 31 or a pharmacologically acceptable salt thereof, wherein
in the above Formula (III), R^{c} is an optionally substituted phenyl group.

33. The compound according to Claim 1 or a pharmacologically acceptable salt thereof, wherein
the compound is a compound represented by the above Formula (IV), and in the above Formula (IV), R^{d} is an optionally substituted 6- to 10-membered aryl group.

34. The compound according to Claim 33 or a pharmacologically acceptable salt thereof, wherein
in the above Formula (IV), R^{d} is an optionally substituted phenyl group.

35. The compound according to any one of Claims 1 to 34 or a pharmacologically acceptable salt thereof, wherein
when n is 0 in Formula (I), each optionally substituted group represented by Y¹ is substituted with L having a first terminal group or with substituted L,
when n is 1 in Formula (I), each optionally substituted group represented by R^{a} is substituted with L having a first terminal group or with substituted L,
in Formula (II), each optionally substituted group represented by R^{b} is substituted with L having a first terminal group or with substituted L,
in Formula (III), each optionally substituted group represented by R^{c} is substituted with L having a first terminal group or with substituted L,
in Formula (IV), each optionally substituted group represented by R^{d} is substituted with L having a first terminal group or with substituted L, and
L is a bond or a chemical linker.

36. The compound according to Claim 35 or a pharmacologically acceptable salt thereof, wherein
L is a group represented by -L₁-L₂-L₃-
[wherein
L₁ and L₃ each independently represent
(1) a bond,
(2) CR^{L1}R^{L1}',
(3) O,
(4) S,
(5) SO,
(6) SO₂,
(7) NR^{L1}
(8) SO₂NR^{L1}
(9) NR^{L1}SO₂,
(10) SONR^{L1},
(11) NR^{L1}SO,
(12) CONR^{L1},
(13) NR^{L1}CO,
(14) NR^{L1}CONR^{L1}',
(15) NR^{L1}SO₂NR^{L1}', or
(16) CO,
in the above formulas, R^{L1} and R^{L1}' each independently represent
1) a hydrogen atom,
2) a halogen atom,
3) -CN,
4) -NO₂,
5) -SF₅,
6) -COOH,
7) -N(R^{L2}R^{L2}'),
8) -A^{L2}R^{L2},
9) an optionally substituted alkyl group,
10) an optionally substituted cycloalkyl group,
11) an optionally substituted heterocyclic saturated group,
12) an optionally substituted aryl group,
13) an optionally substituted heteroaryl group,
14) -SO₂R^{L2},
15) -P(O)(OR^{L2})OR^{L2}',
16) -C≡CR^{L2},
17) -C(R^{L2})=C(R^{L21}R^{L2}"),
18) -COR^{L2},
19) -CON(R^{L2}R^{L21}'),
20) -SO₂N(R^{L2}R^{L21}'),
21) -N(R^{L2})CON(R^{L2}'R^{L2}"), and
22) -N(R^{L2})SO₂N(R^{L21}R^{L2}"),
A^{L2} represents an oxygen atom or a sulfur atom,
R^{L2}, R^{L2}', and R^{L2}" each independently represent
a) a hydrogen atom,
b) an optionally substituted C₁₋₈ alkyl group, or
c) an optionally substituted C₃₋₈ cycloalkyl group,
L₂ represents
(CH₂)ₚ₁ₐ-O-(CH₂-CH₂-O)ₚ₂ₐ-(CH₂)ₚ₃ₐ
wherein
p1a and p3a represent an integer from 0 to 10 when an atom directly bonded to adjacent L₁ or L₃ is a carbon atom or a bond, and an integer from 2 to 10 when the atom is other than a carbon atom or a bond,
p2a represents an integer from 0 to 10, and
some -CH₂-CH₂- groups in (CH₂)ₚ₁ₐ or (CH₂)ₚ₃ₐ of L₂ may be replaced with -CH=CH- or -C≡ C-].

37. The compound according to Claim 35 or a pharmacologically acceptable salt thereof, wherein
L is a group represented by -L_{b1}-L_{b2}-L_{b3}-
[wherein
L_{b1} and L_{b3} each independently represent
(1) a bond,
(2) CR^{L1}R^{L1}',
(3) O,
(4) S,
(5) SO,
(6) SO₂,
(7) NR^{L1},
(8) SO₂NR^{L1}
(9) NR^{L1}SO₂,
(10) SONR^{L1},
(11) NR^{L1}SO,
(12) CONR^{L1},
(13) NR^{L1}CO,
(14) NR^{L1}CONR^{L1}',
(15) NR^{L1}SO₂NR^{L1}', or
(16) CO,
in the above formulas, R^{L1} and R^{L1}' each independently represent
1) a hydrogen atom,
2) a halogen atom,
3) -CN,
4) -NO₂,
5) -SF₅,
6) COOH,
7) -N(R^{L2}R^{L2}'),
8) -A^{L2}R^{L2},
9) an optionally substituted alkyl group,
10) an optionally substituted cycloalkyl group,
11) an optionally substituted heterocyclic saturated group,
12) an optionally substituted aryl group,
13) an optionally substituted heteroaryl group,
14) -SO₂R^{L2},
15) -P(O)(OR^{L2})OR^{L21},
16) -C≡CR^{L2},
17) -C(R^{L2})=C(R^{L2}'R^{L2}"),
18) -COR^{L2},
19) -CON(R^{L2}R^{L2}'),
20) -SO₂N (R^{L2}R^{L2}'),
21) -N(R^{L2})CON(R^{L2}'R^{L2}"), and
22) -N(R^{L2})SO₂N(R^{L2}'R^{L2}"),
A^{L2} represents an oxygen atom or a sulfur atom,
R^{L2}, R^{L2}', and R^{L2}" each independently represent
a) a hydrogen atom,
b) an optionally substituted C₁₋₈ alkyl group, or
c) an optionally substituted C₃₋₈ cycloalkyl group,
L_{b2} represents
(1) a bond,
(2) (CH₂)₁₋₁₀,
(3) (CH₂)₀₋₆-O-(CH₂)₀₋₆,
(4) (CH₂)₀₋₆-CONH-(CH₂)₀₋₆,
(5) (CH₂)₀₋₆-NHCO-(CH₂)₀₋₆,
(6) (CH₂)₀₋₆-NH-(CH₂)₀₋₆,
(7) (CH₂)₀₋₆-NHSO₂-(CH₂)₀₋₆, or
(8) (CH₂)₀₋₆-SO₂NH-(CH₂)₀₋₆,
wherein some -CH₂-CH₂- groups in (CH₂)₁₋₁₀ or (CH₂)₀₋₆ in L_{b2} may be replaced with -CH=CH- or -C≡C-,
provided that a case where L_{b1}, L_{b2}, and L_{b3} are all bonds is excluded].

38. The compound according to Claim 35 or a pharmacologically acceptable salt thereof, wherein
L is a group represented by the following Formula (V): 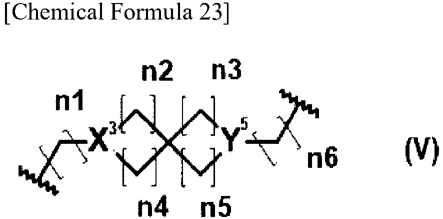
[wherein
n1 and n6 are each independently 0, 1, 2, 3, 4, 5, 6, 7, 8, 9, or 10,
n2, n3, n4, and n5 are each independently 1, 2, 3, or 4,
X³ and Y⁵ are each independently CH or N].

39. The compound according to Claim 35 or a pharmacologically acceptable salt thereof, wherein
L is a group selected from a group consisting of:
-N(R^{L3})-(CH₂)ₘ₁-O(CH₂)ₘ₂-O(CH₂)ₘ₃-O(CH₂)ₘ₄-O(CH₂)ₘ₅-O(CH₂)ₘ₆-OCH₂-,
-O-(CH₂)ₘ₁-O(CH₂)ₘ₂-O(CH₂)ₘ₃-O(CH₂)ₘ₄-O(CH₂)ₘ₅-O(CH₂)ₘ₆-OCH₂-,
-O-(CH₂)ₘ₁-O(CH₂)ₘ₂-O(CH₂)ₘ₃-O(CH₂)ₘ₄-O(CH₂)ₘ₅-O(CH₂)ₘ₆-O-,
-N(R)-(CH₂)ₘ₁-O(CH₂)ₘ₂-O(CH₂)ₘ₃-O(CH₂)ₘ₄-O(CH₂)ₘ₅-O(CH₂)ₘ₆-O-,
-(CH₂)ₘ₁-O(CH₂)ₘ₂-O(CH₂)ₘ₃-O(CH₂)ₘ₄-O(CH₂)ₘ₅-O(CH₂)ₘ₆-O-,
-(CH₂)ₘ₁-O(CH₂)ₘ₂-O(CH₂)ₘ₃-O(CH₂)ₘ₄-O(CH₂)ₘ₅-O(CH₂)ₘ₆-OCH₂-, 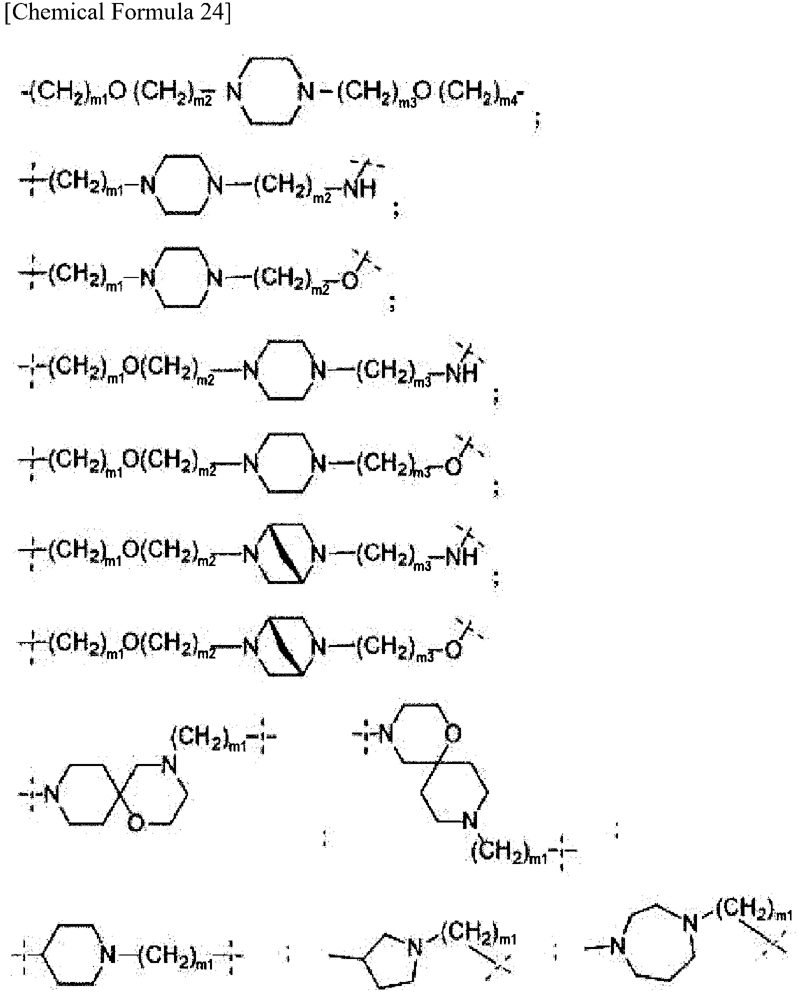 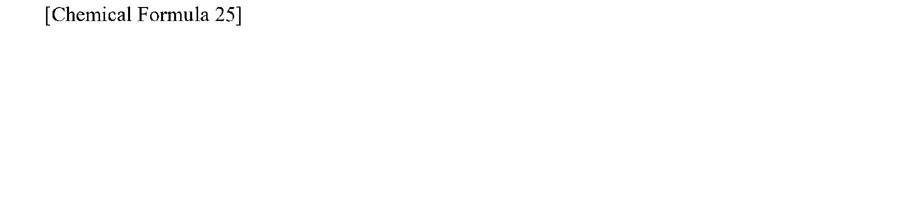 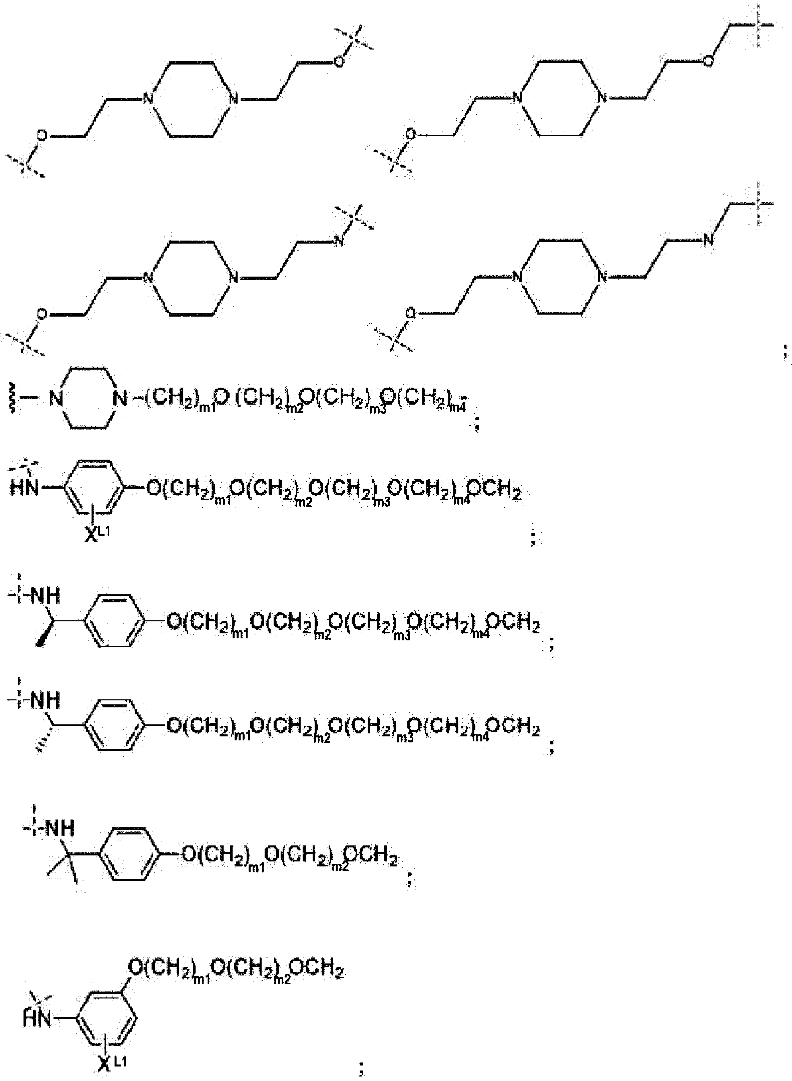 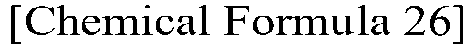 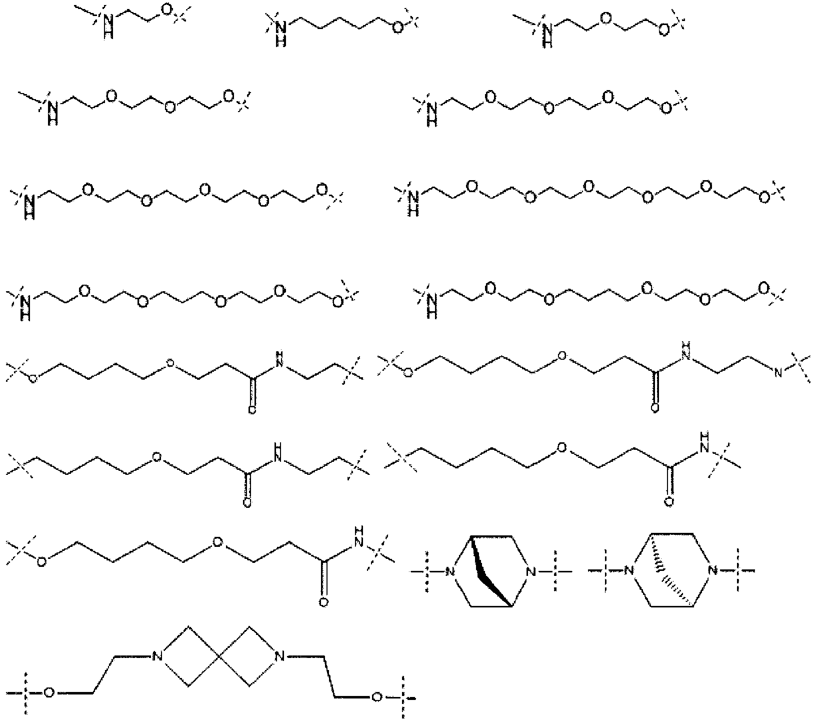 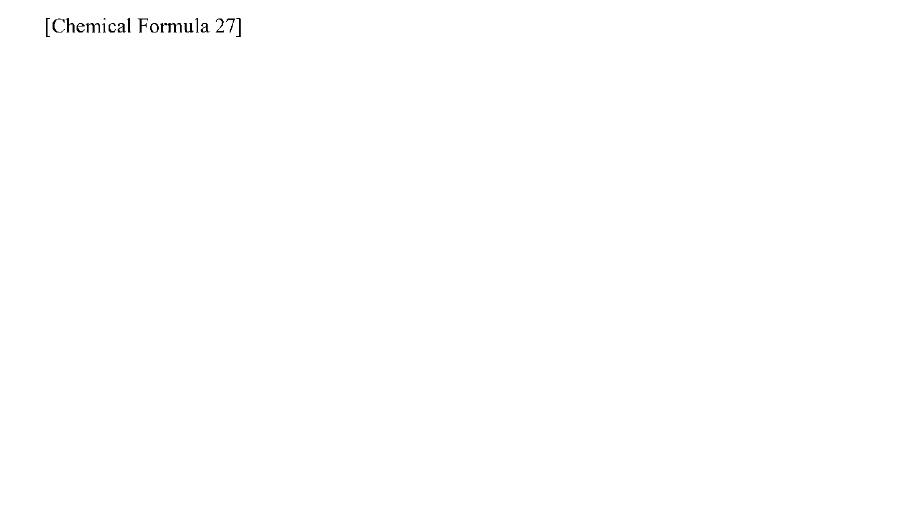 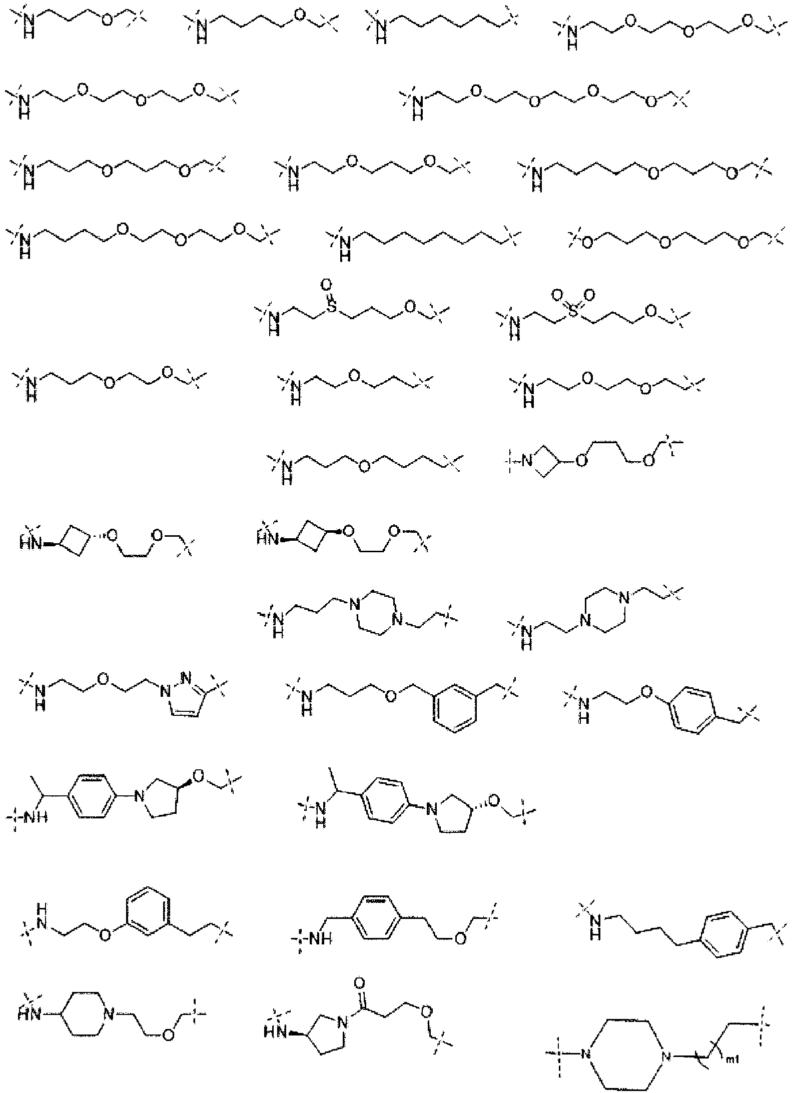 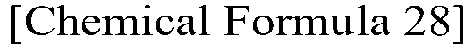 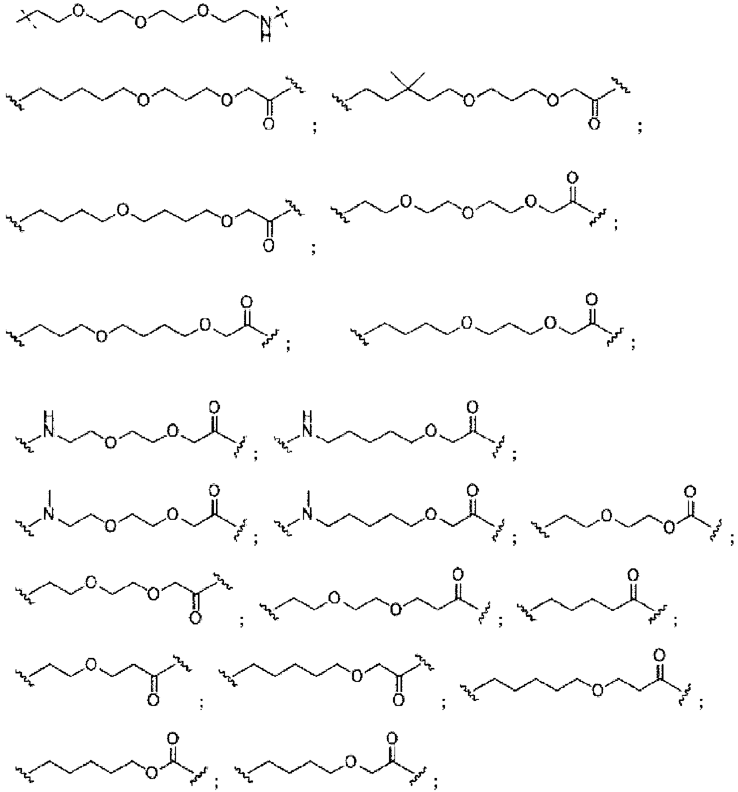 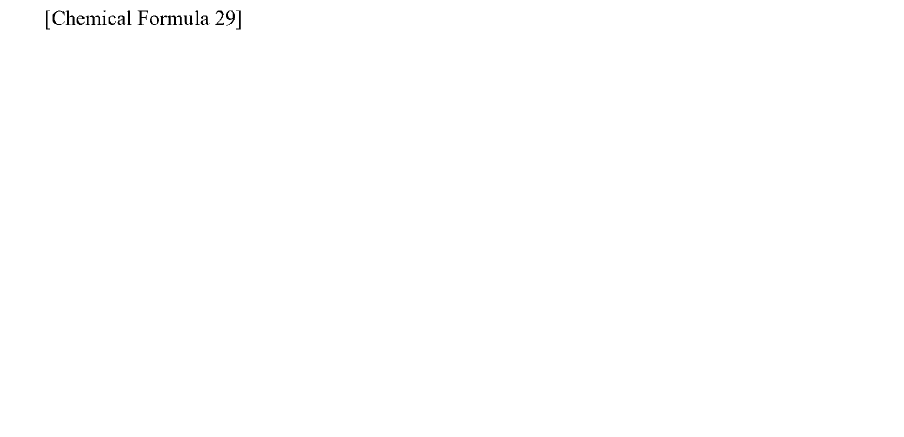 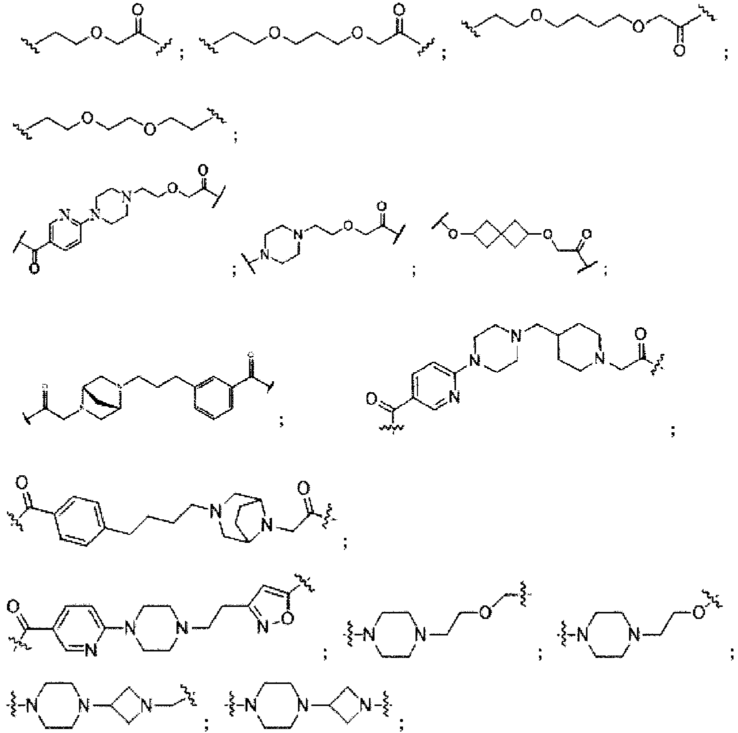 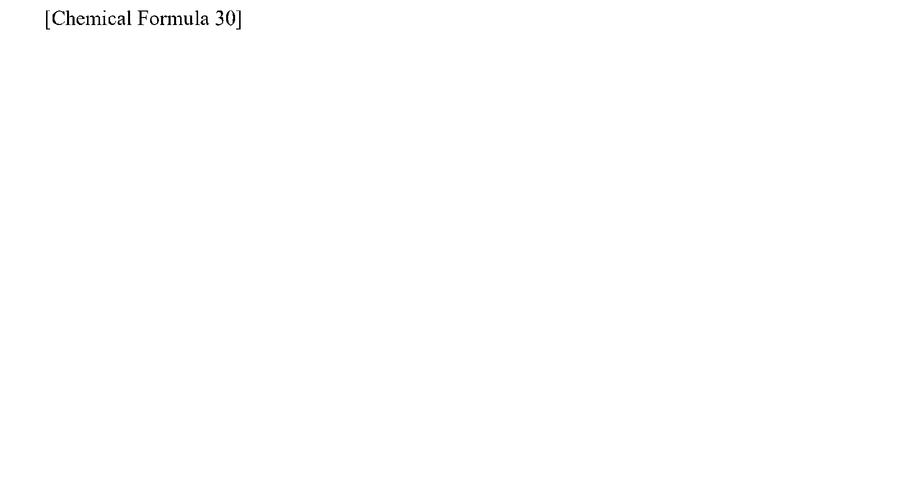 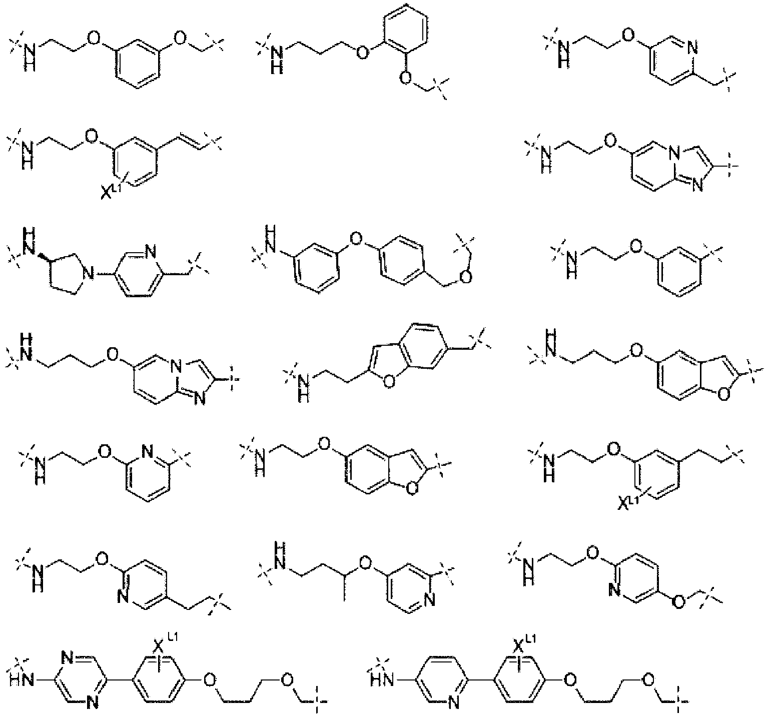 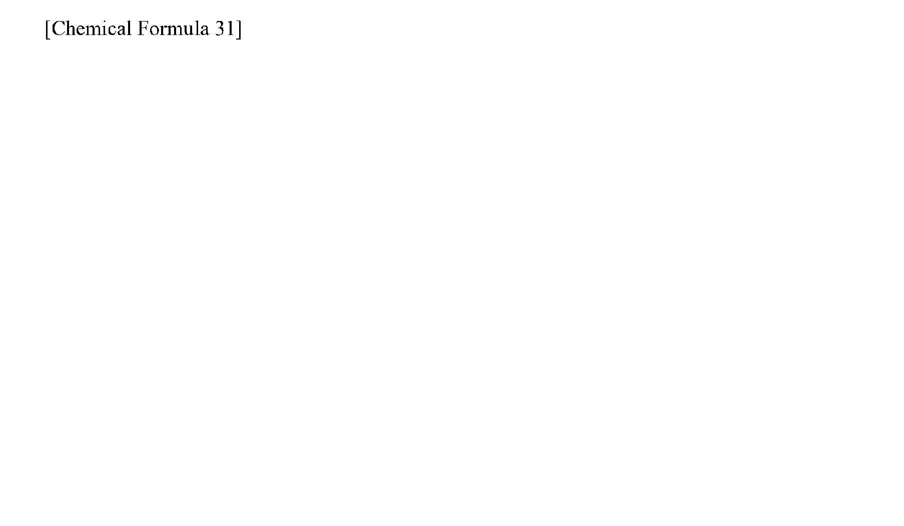 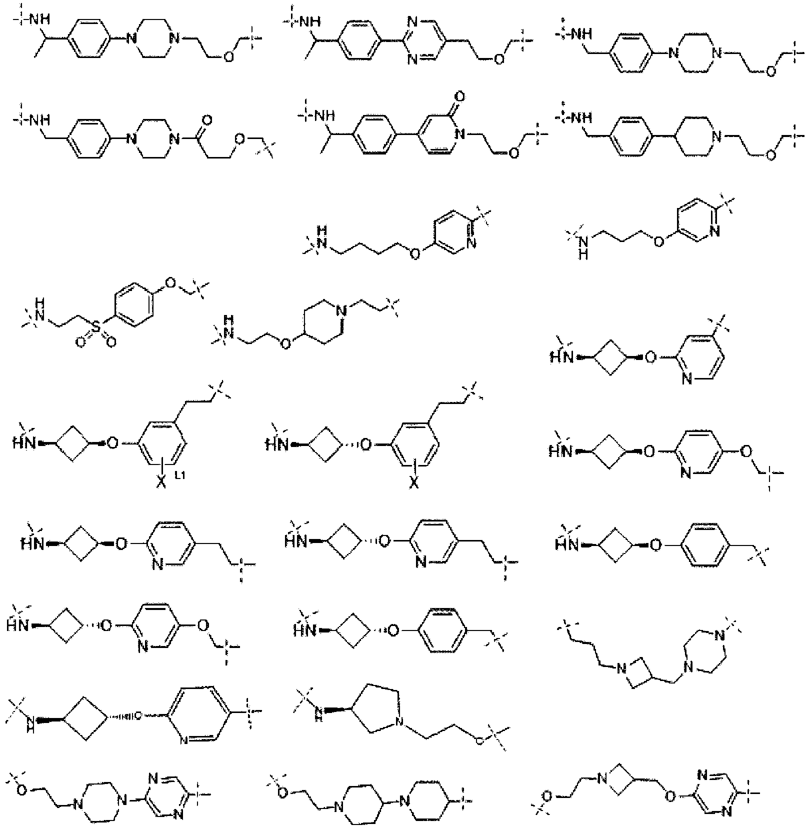 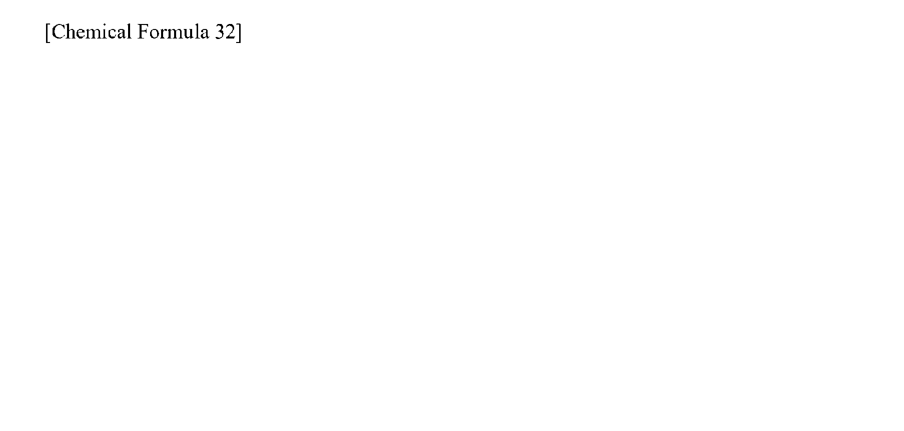 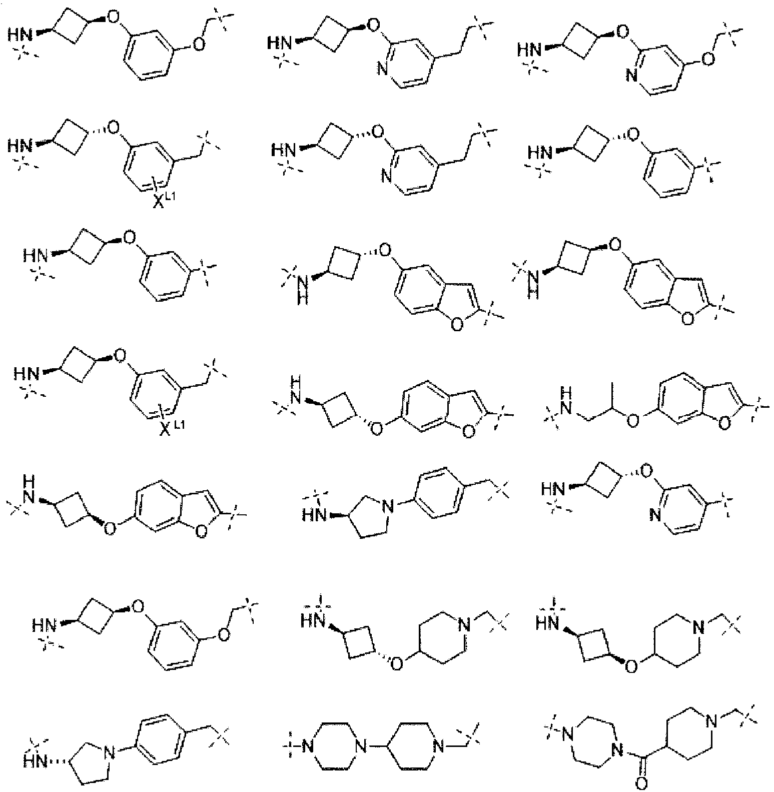 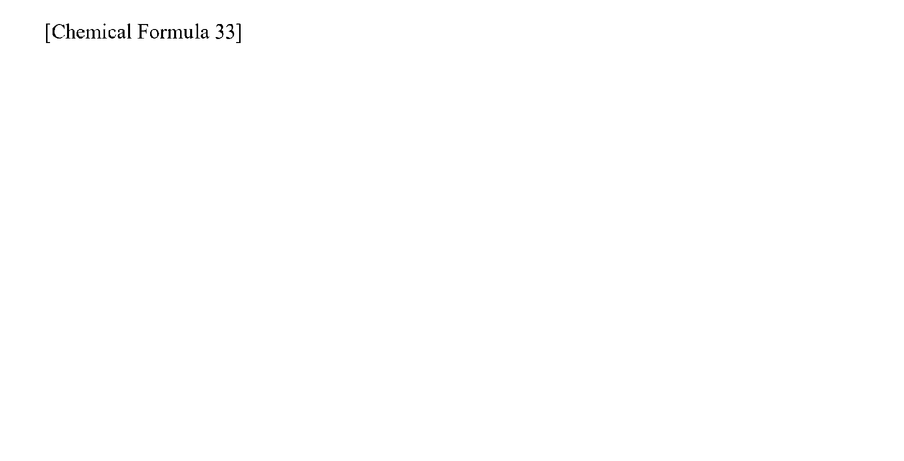 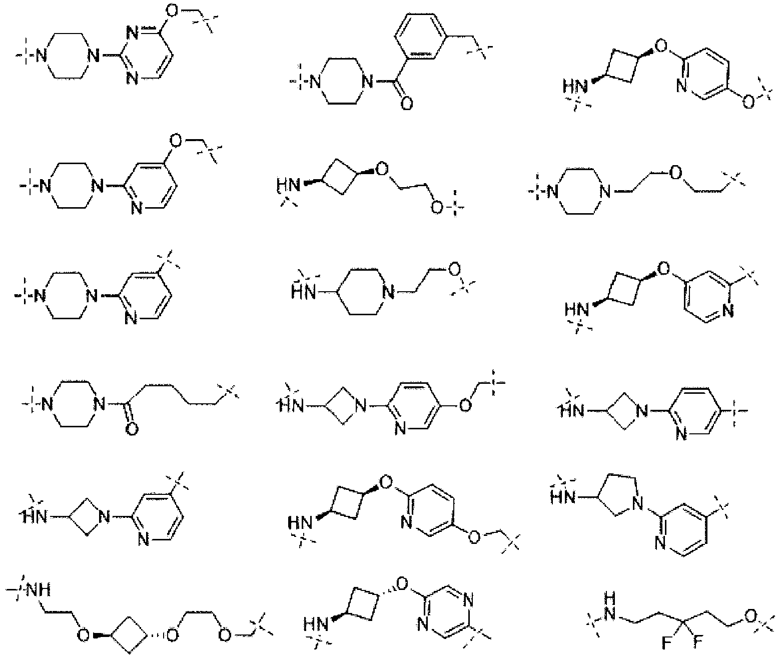
[wherein
m1, m2, m3, m4, m5, and m6 are each independently 0, 1, 2, 3, 4, 5, 6, 7, 8, 9, 10, 11, 12, 13, 14, 15, 16, 17, 18, 19, or 20, provided that when the number is 0, no N-O bond or O-O bond is present,
R^{L3} is H, methyl, or ethyl; and
X^{L1} is H or F].

40. The compound according to Claim 35 or a pharmacologically acceptable salt thereof, wherein
L is selected from a group consisting of: 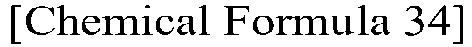 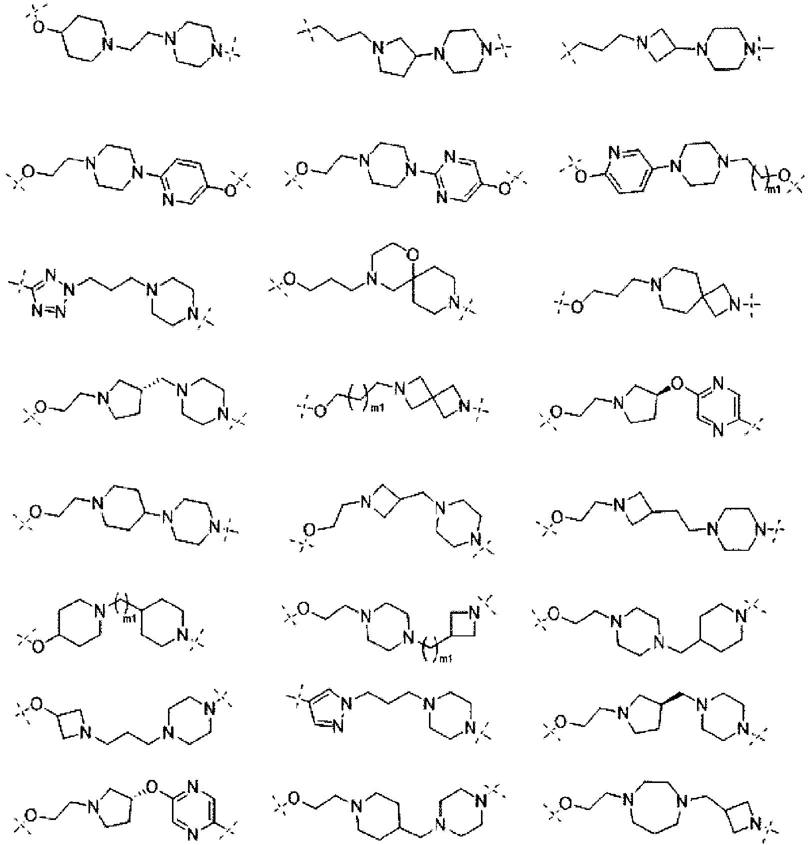 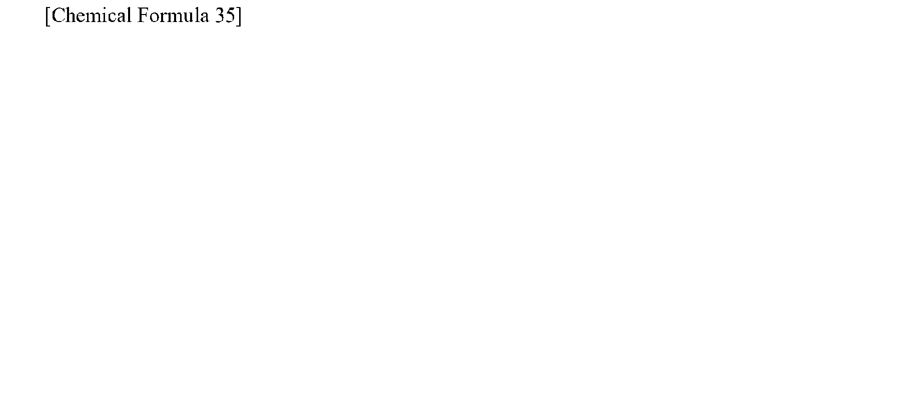 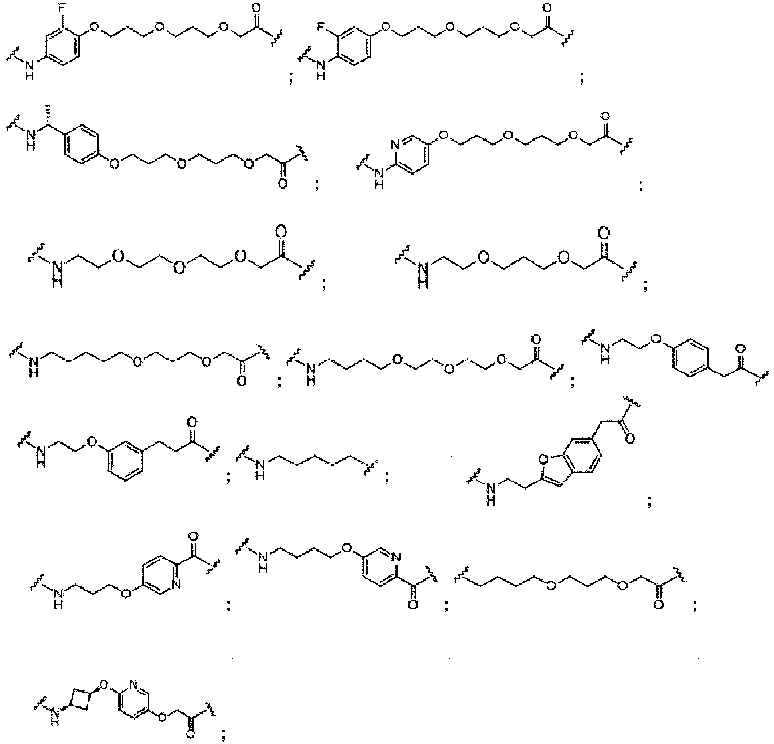 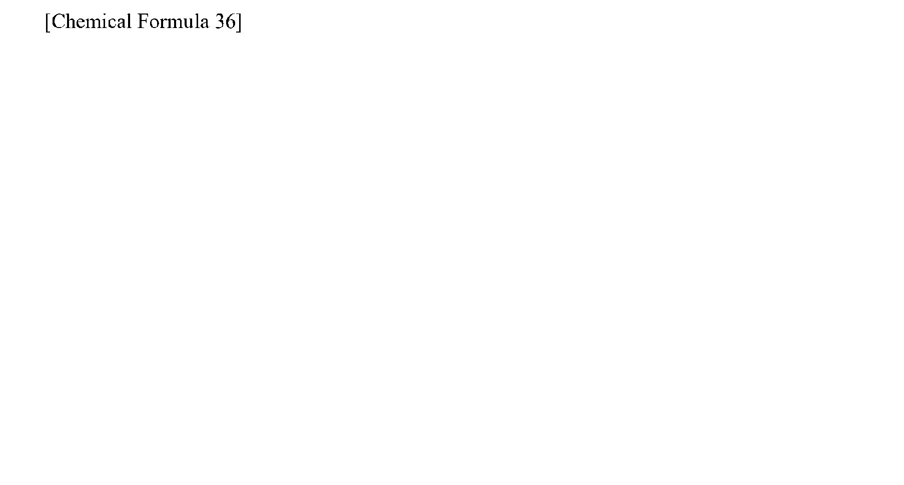 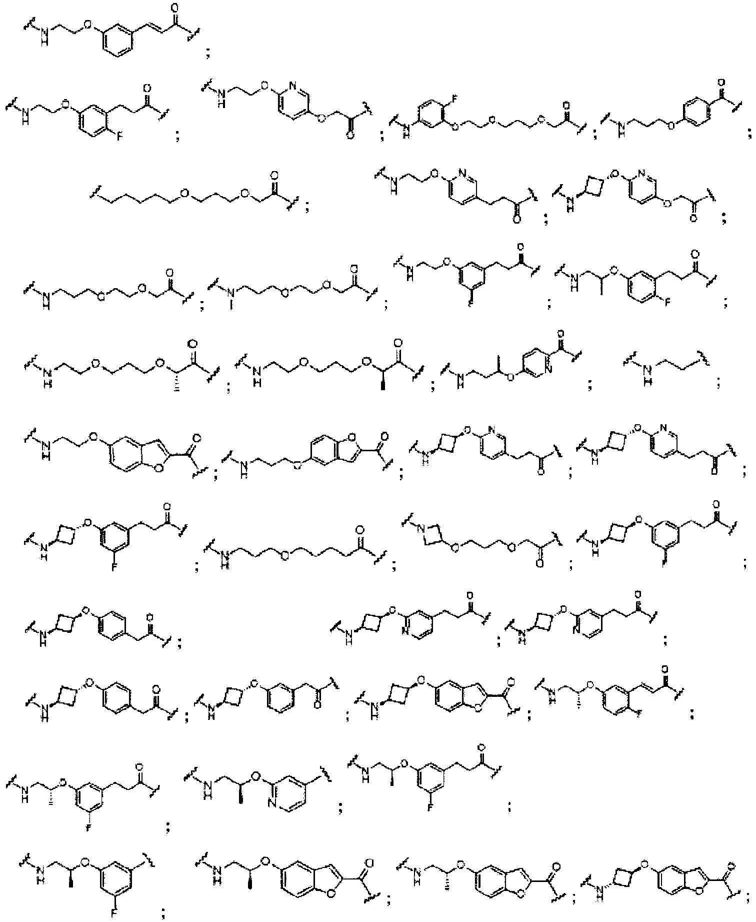 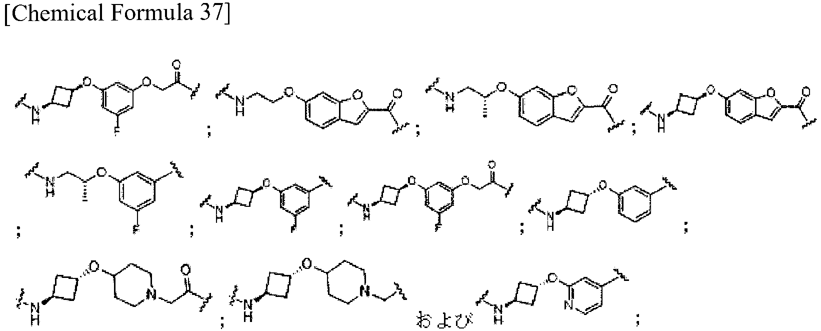
[wherein
each m1 is independently 0, 1, 2, 3, 4, 5, 6, 7, 8, 9, 10, 11, 12, 13, 14, 15, 16, 17, 18, 19, or 20].

41. The compound according to Claim 35 or a pharmacologically acceptable salt thereof, wherein
L is a group selected from a group consisting of: 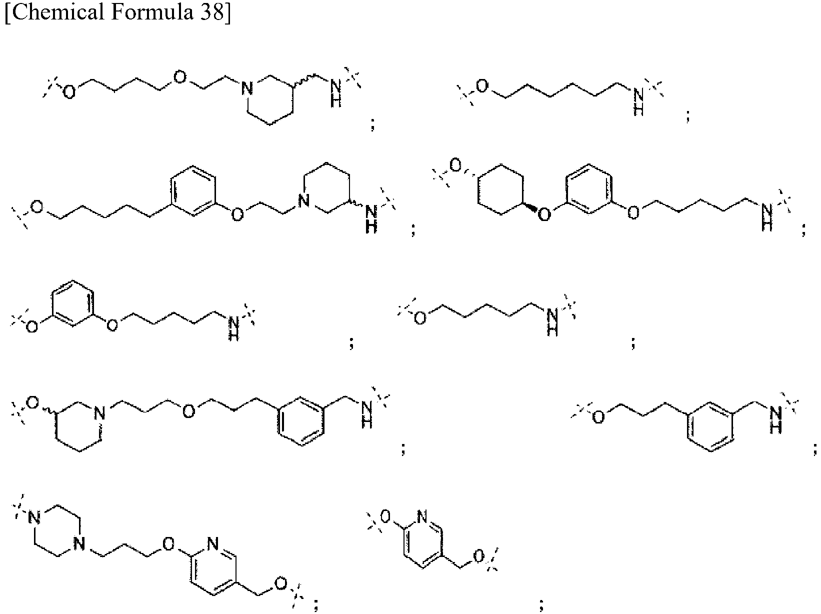 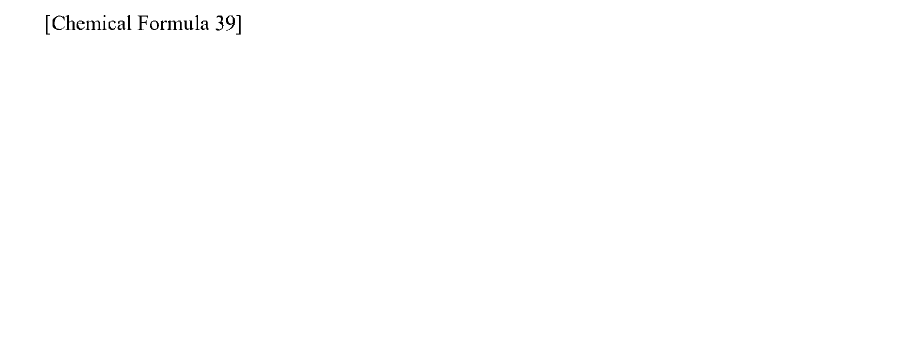 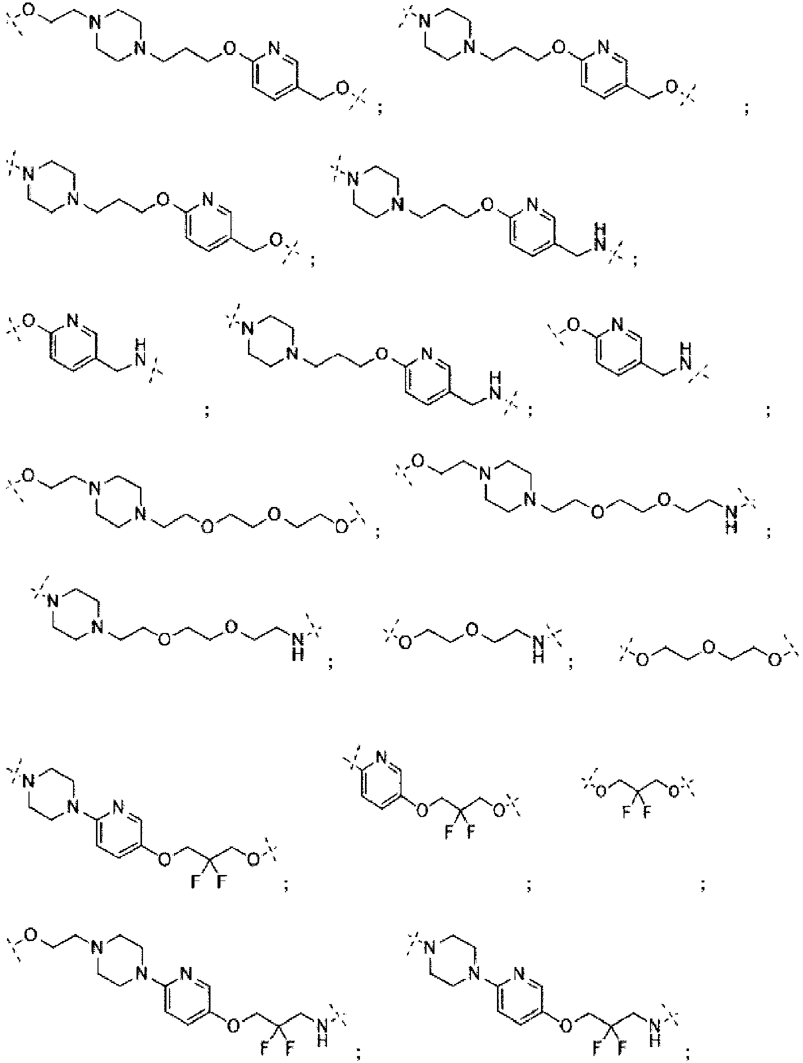 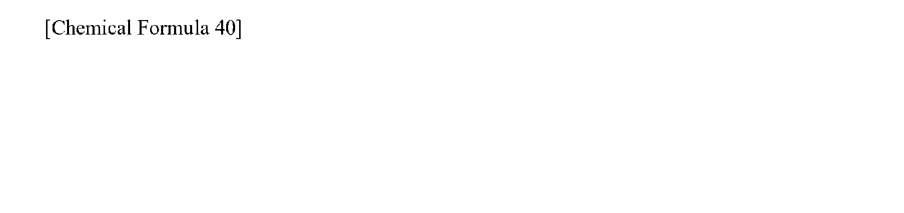 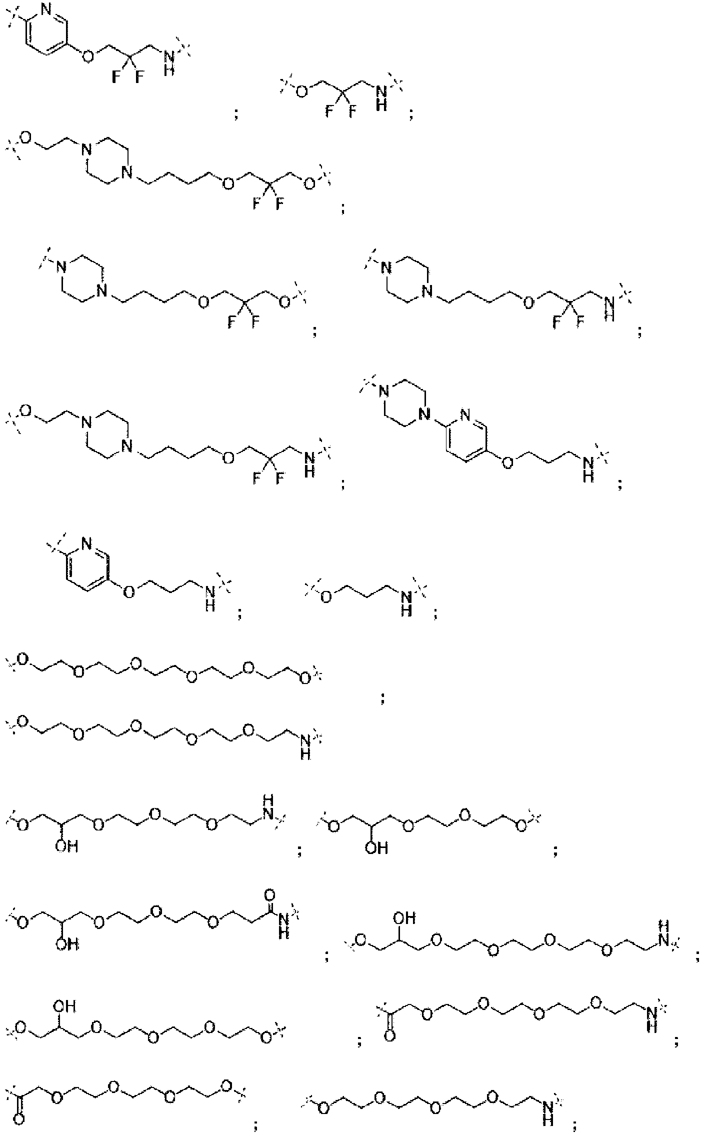 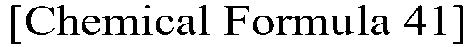 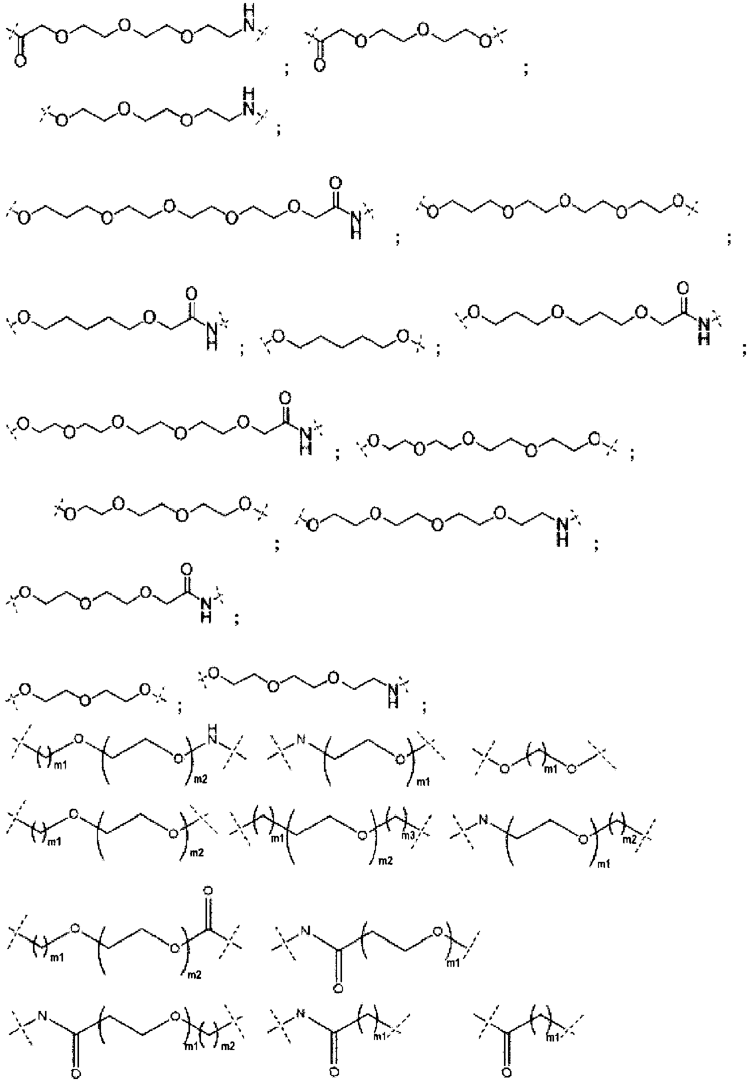 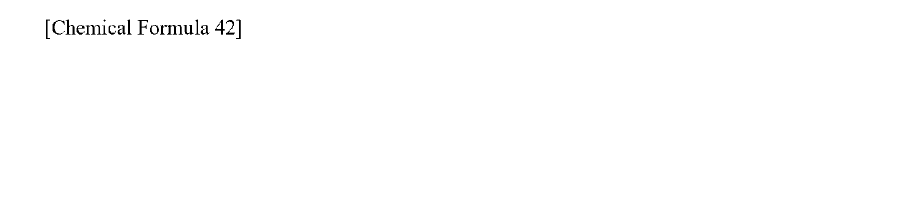 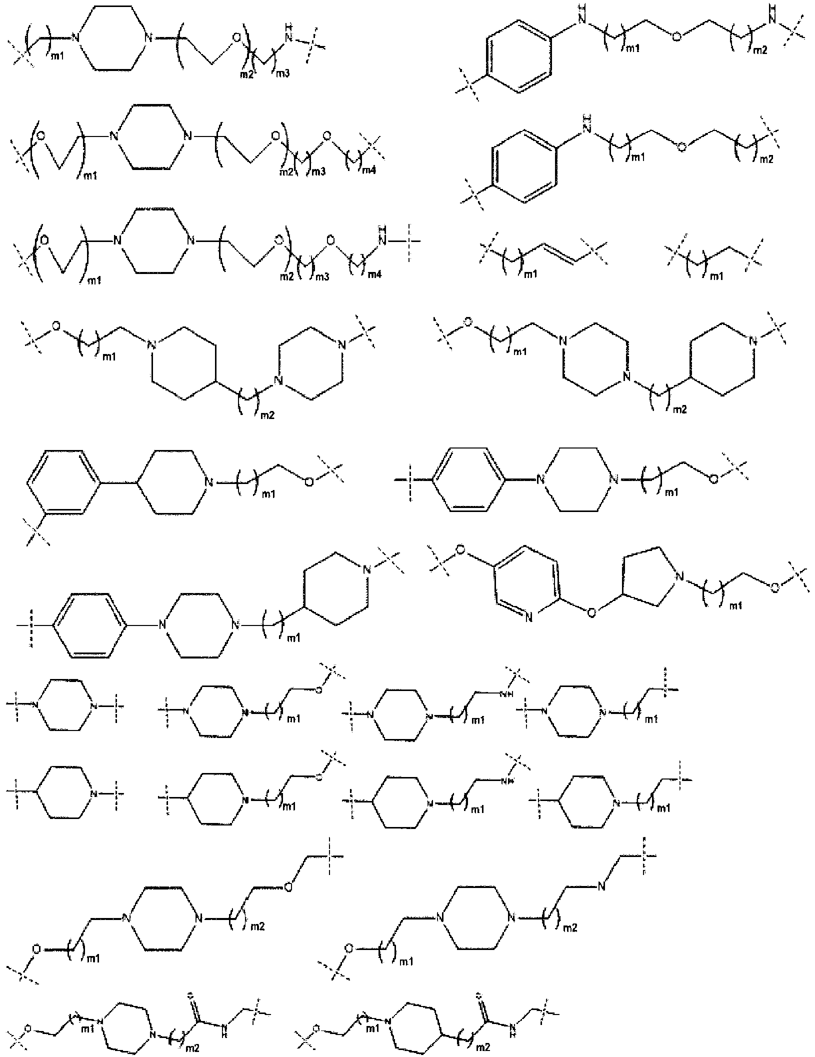 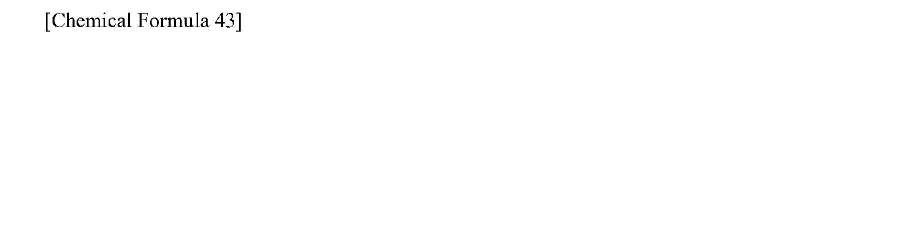 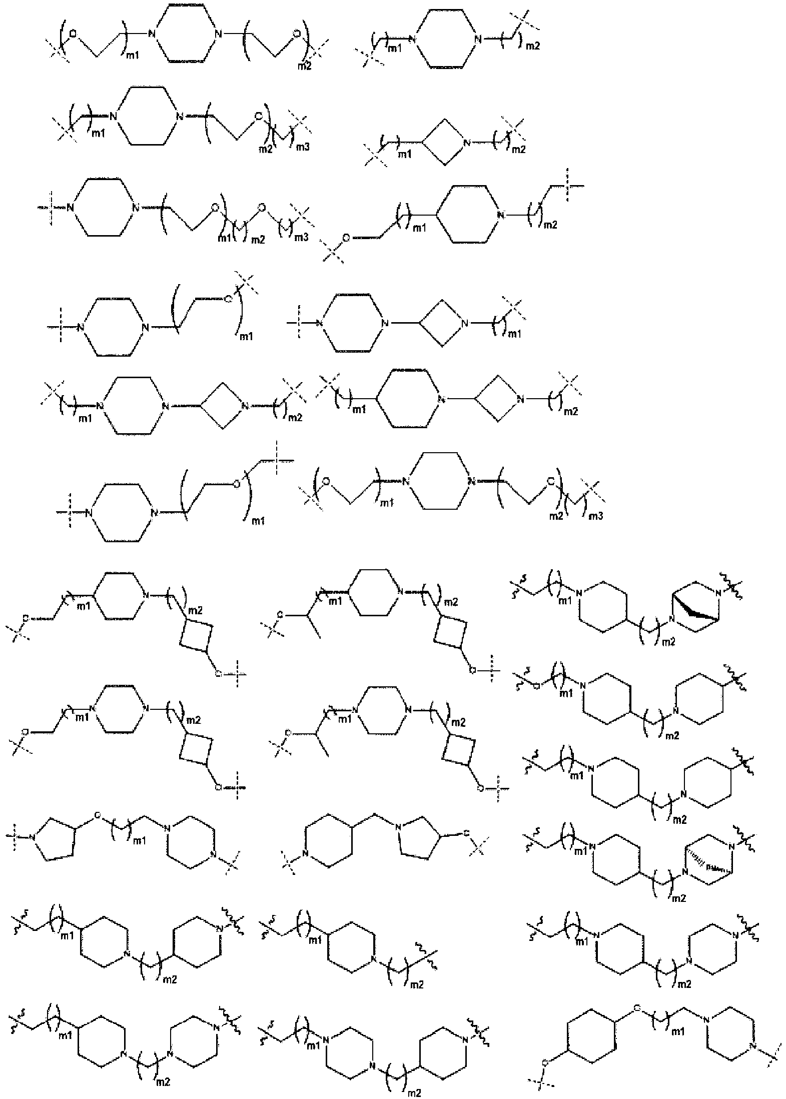 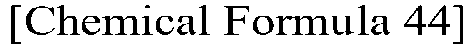 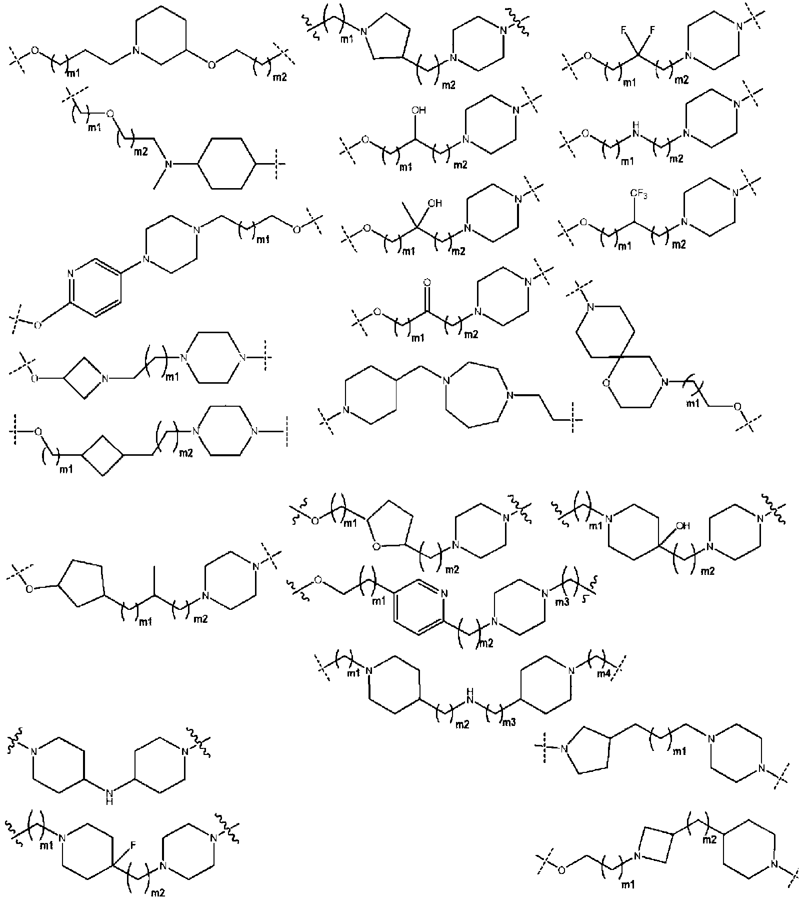 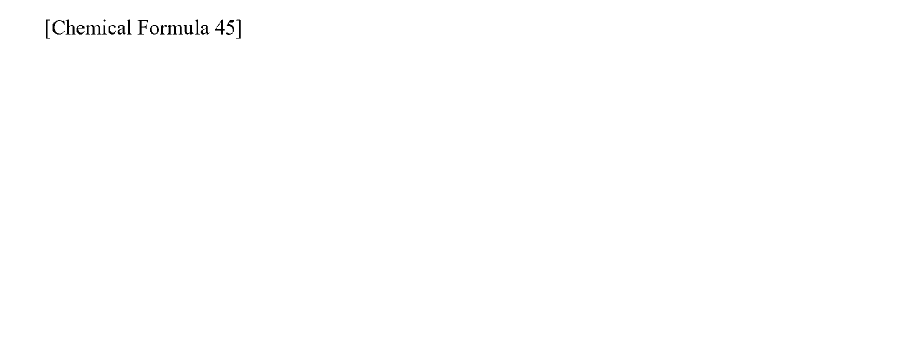 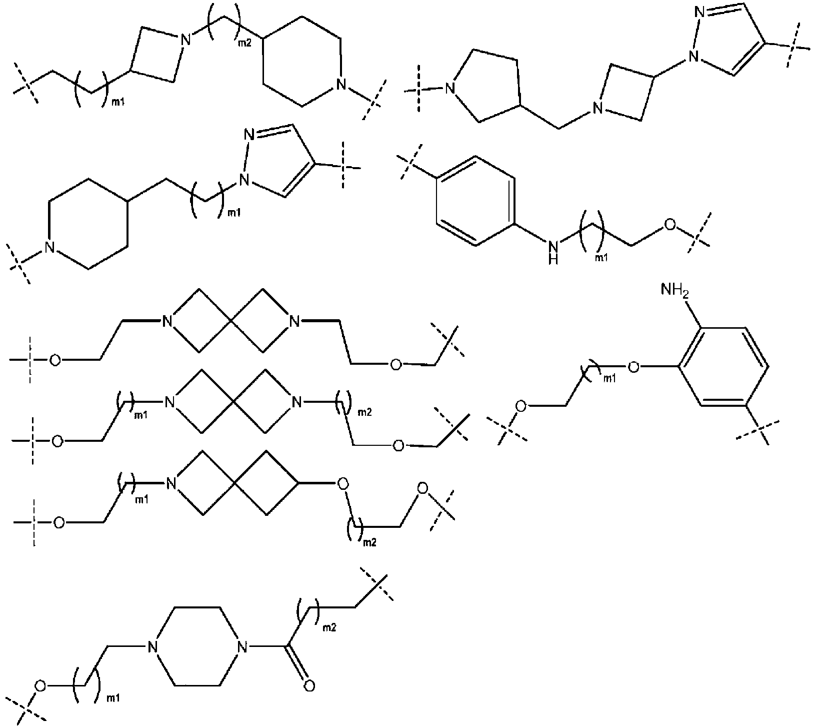 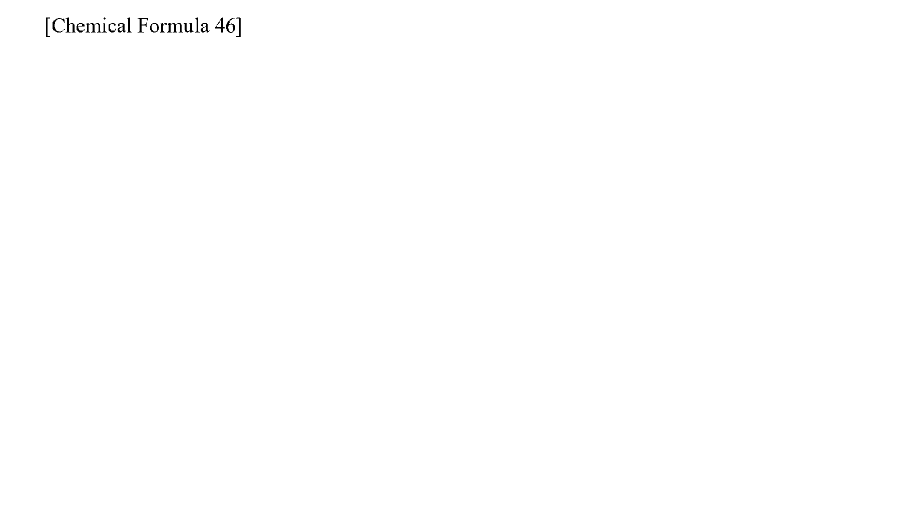 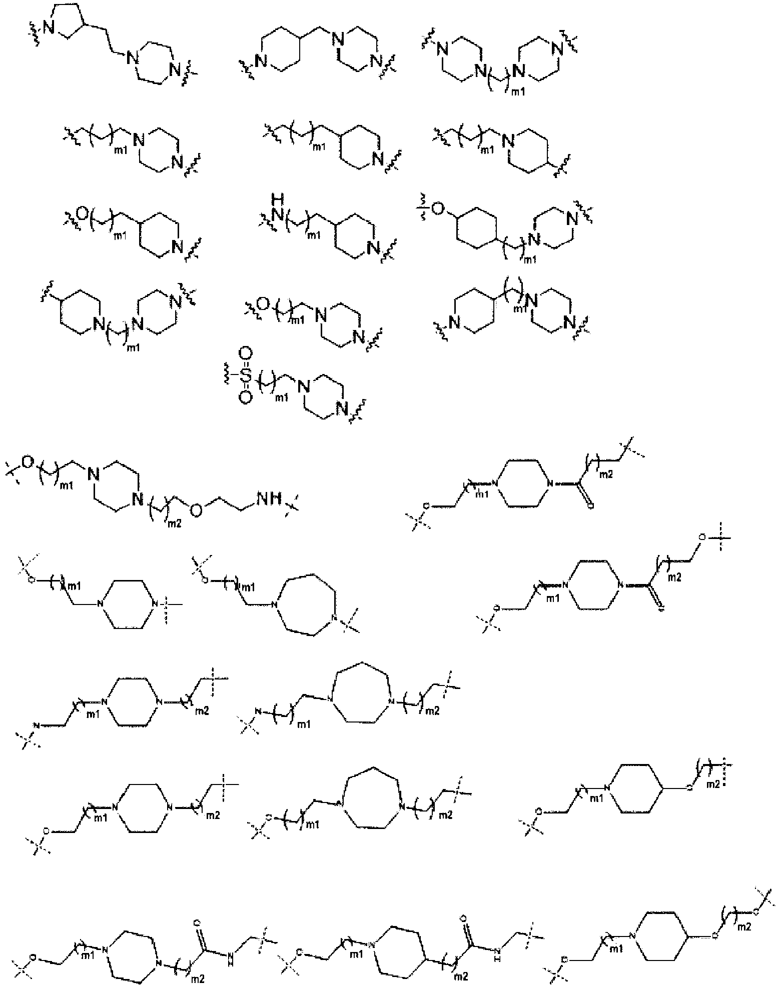 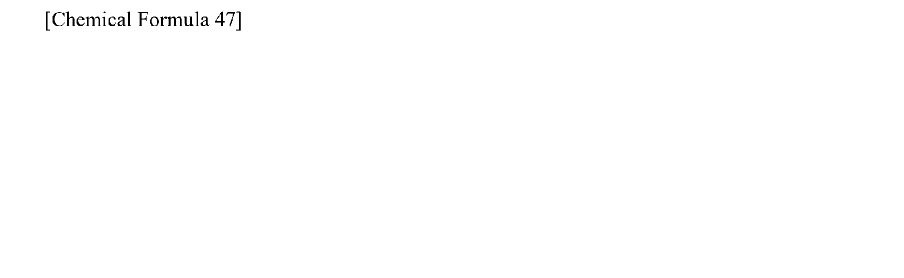 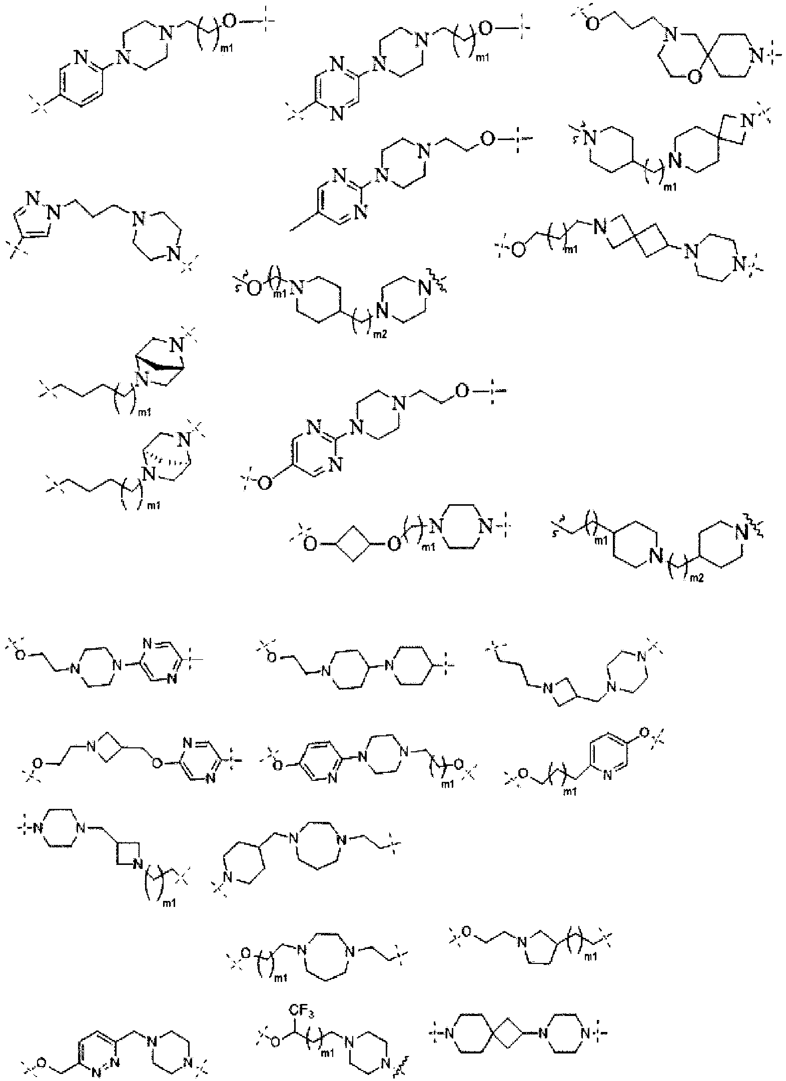 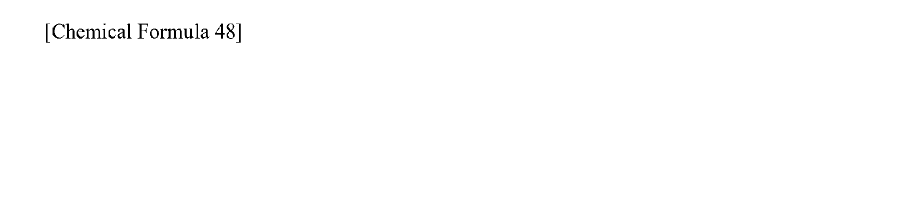 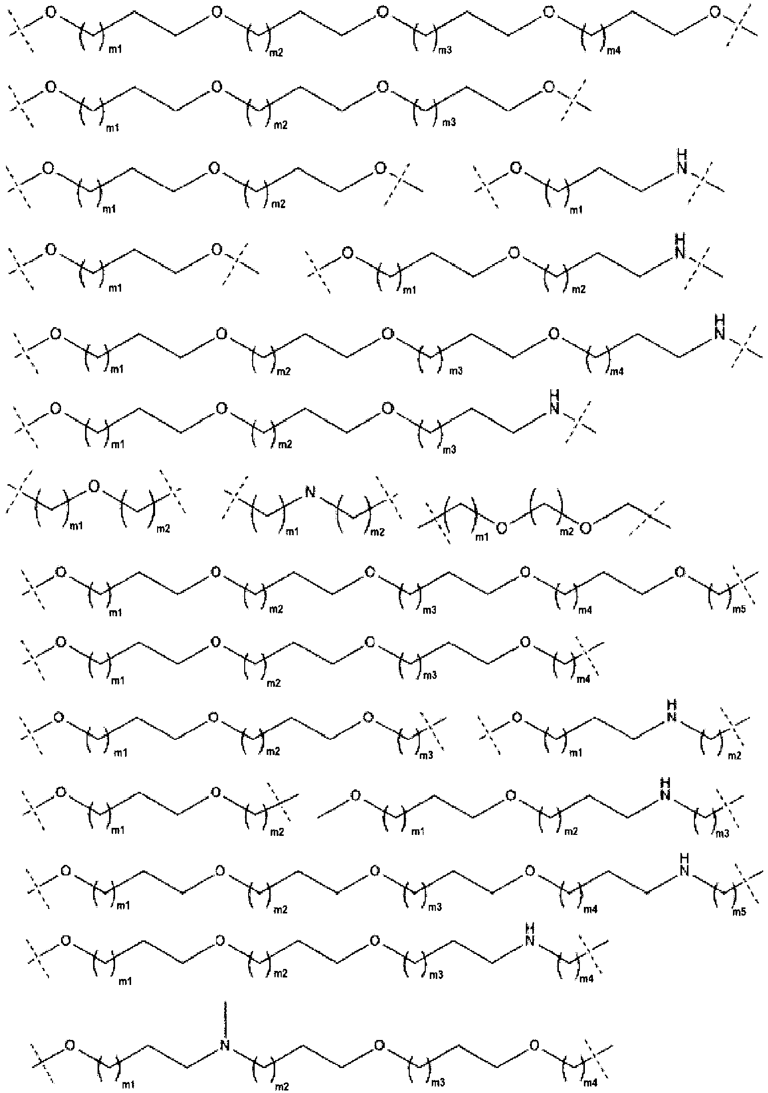 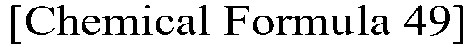 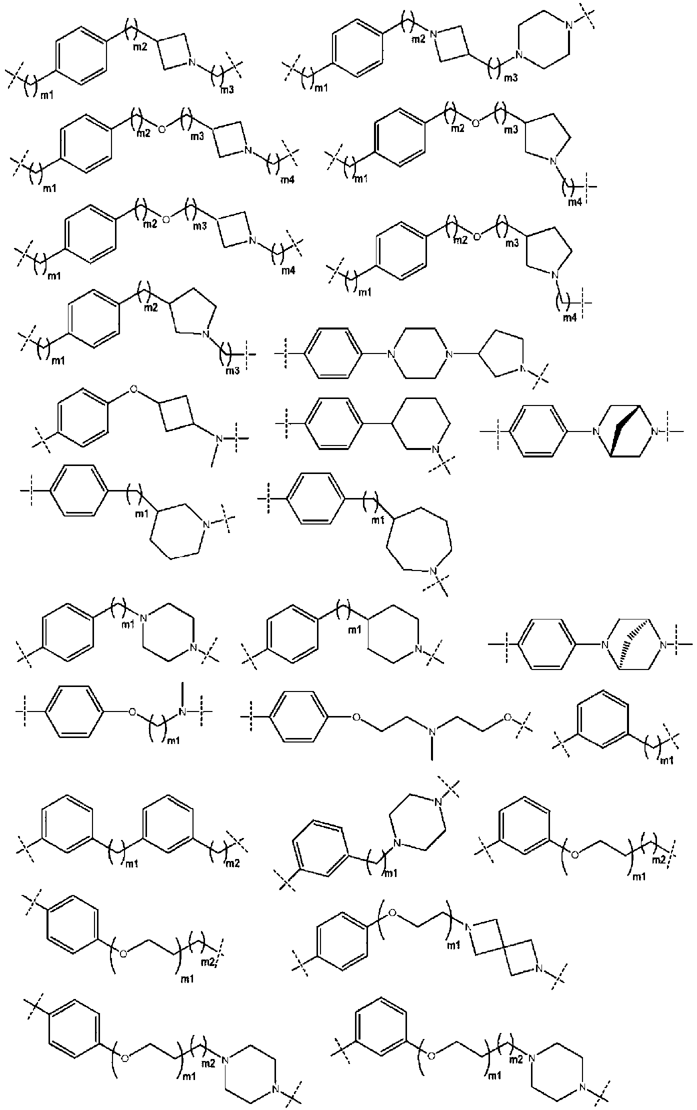 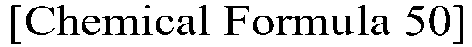 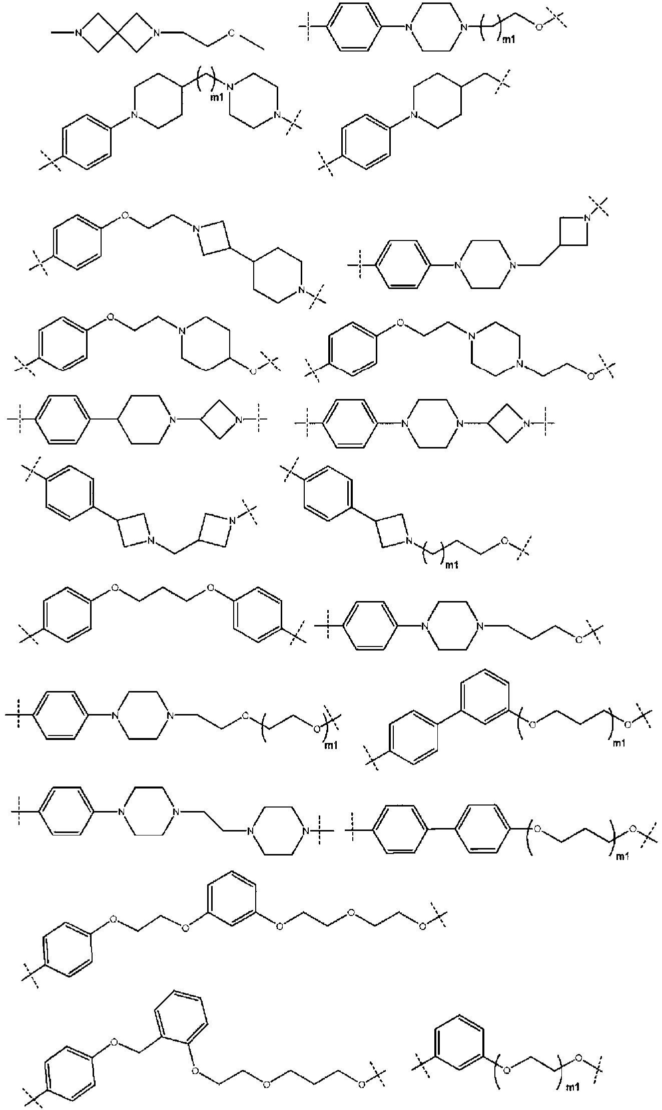 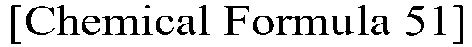 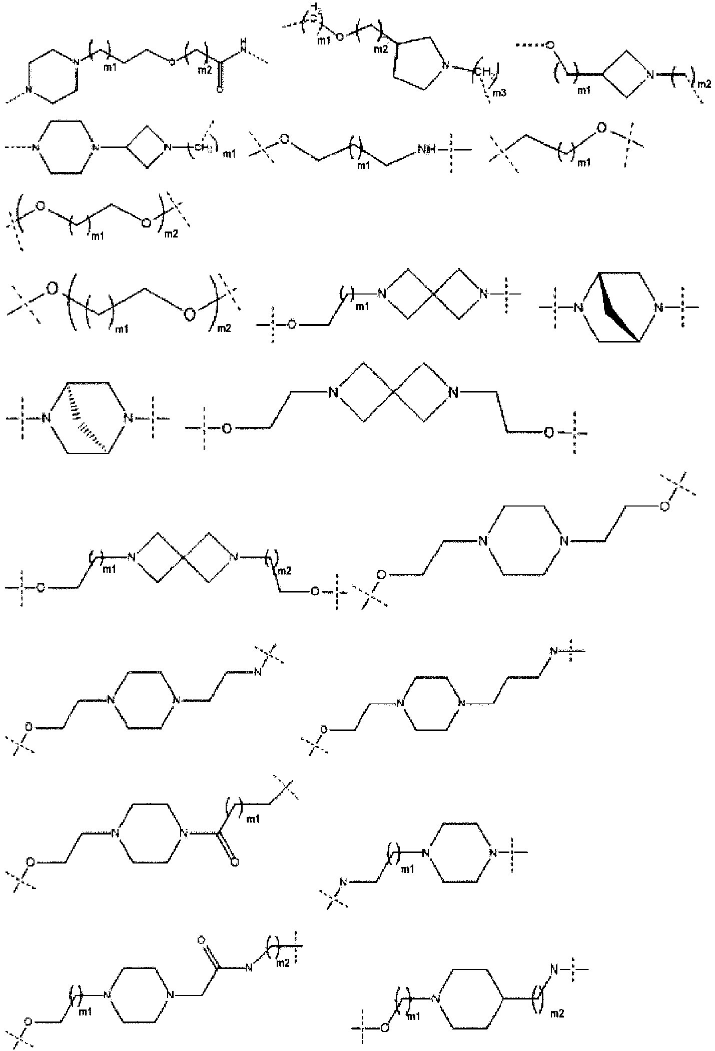 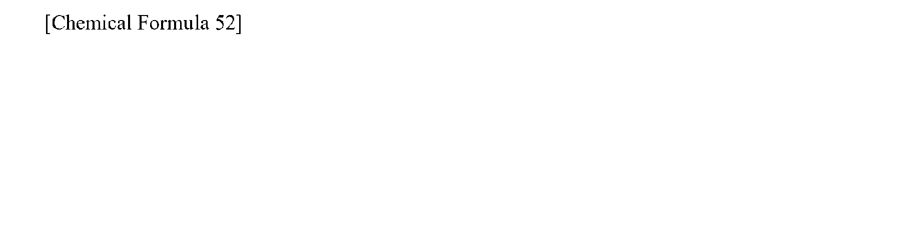 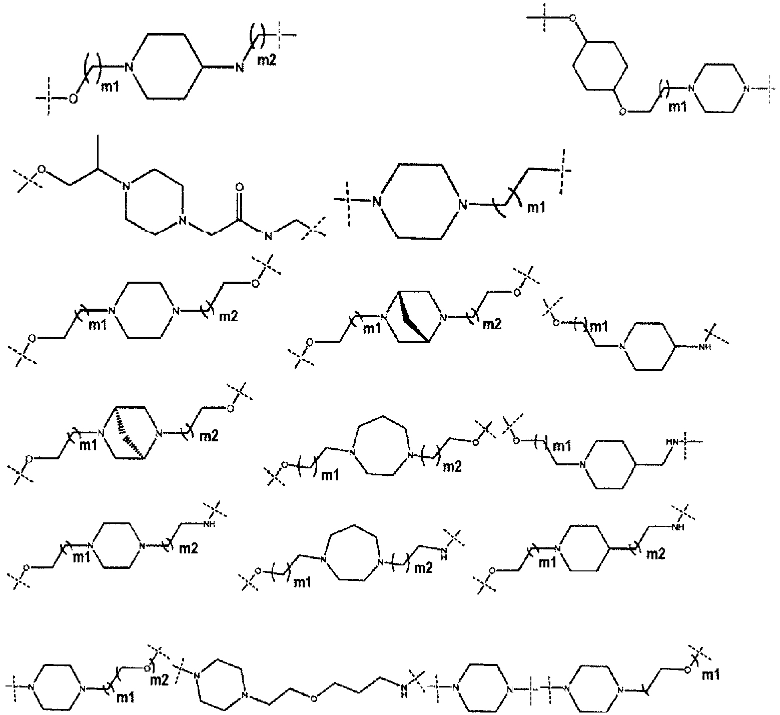 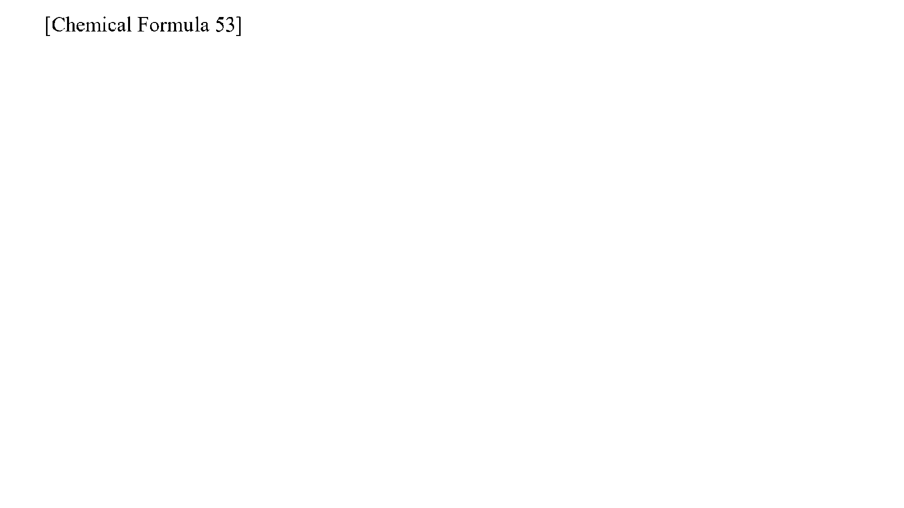 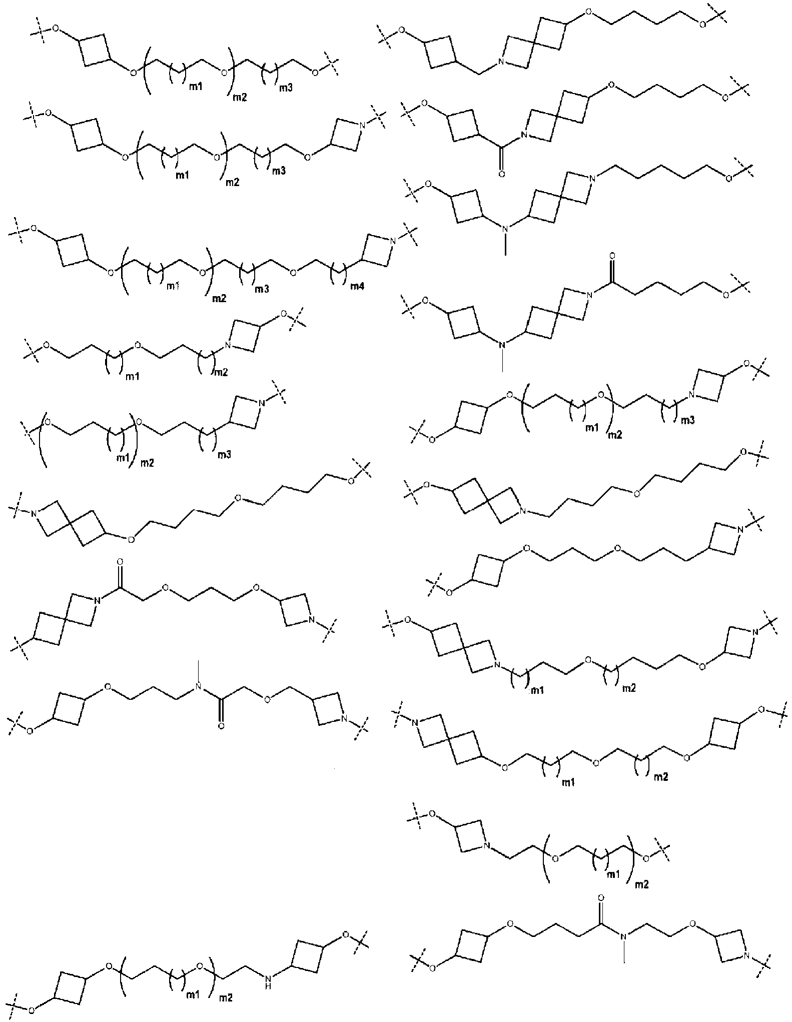 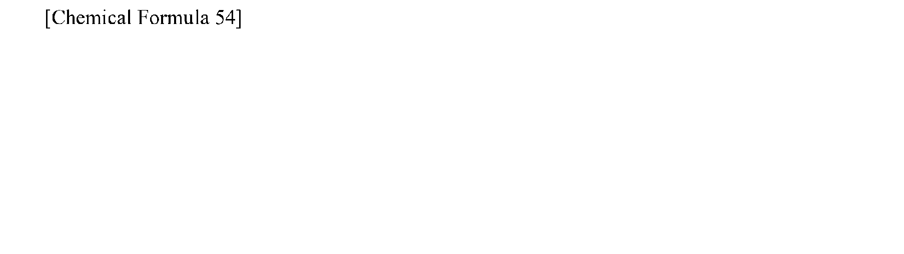 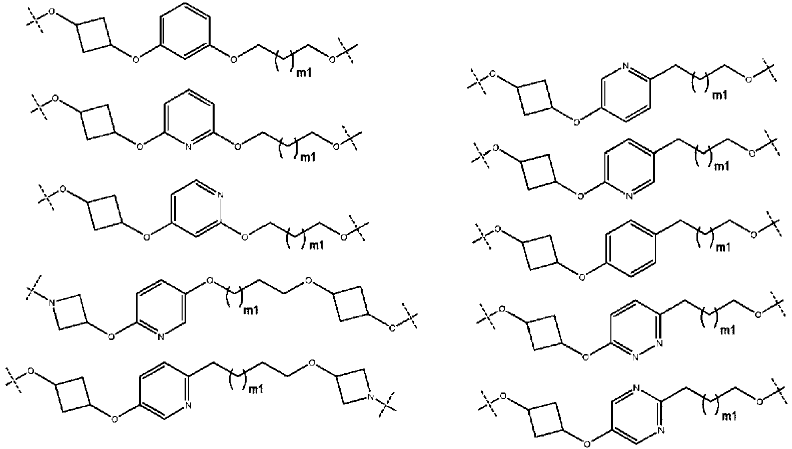 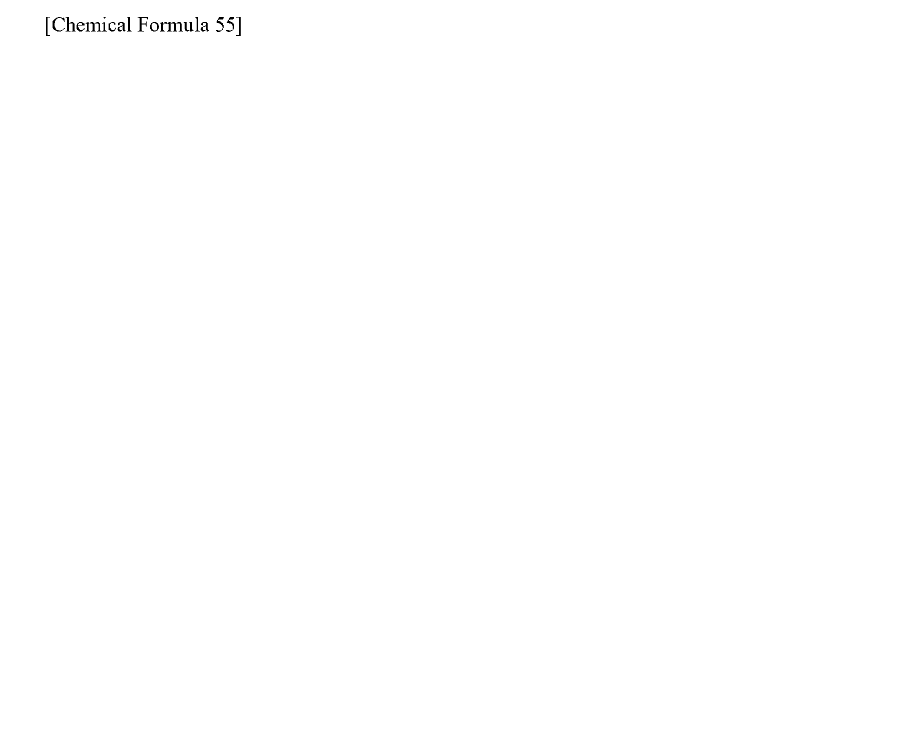 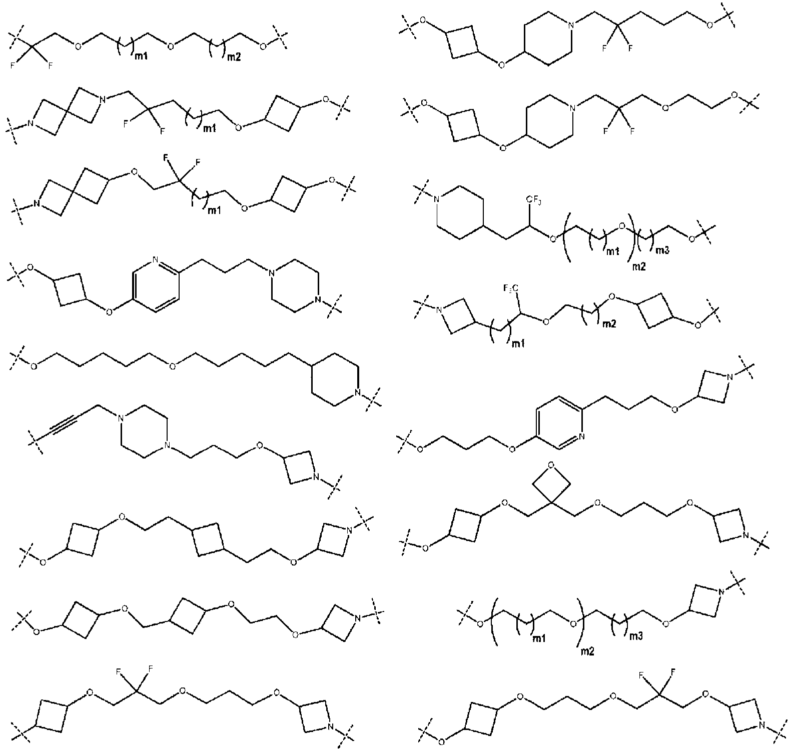 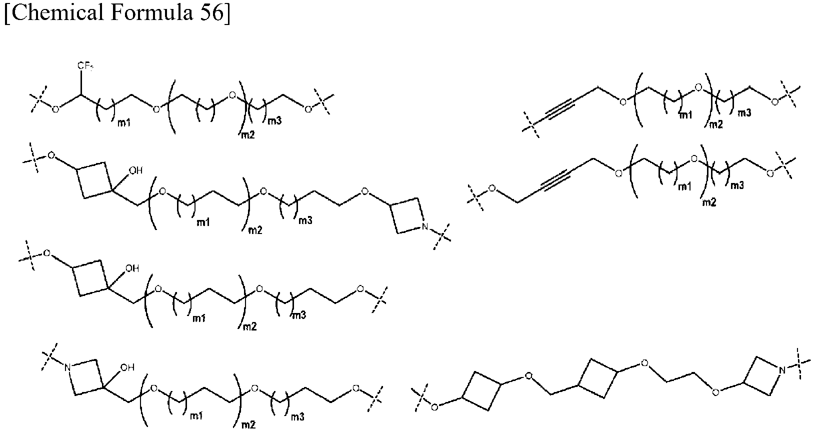 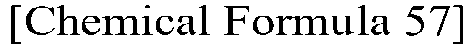 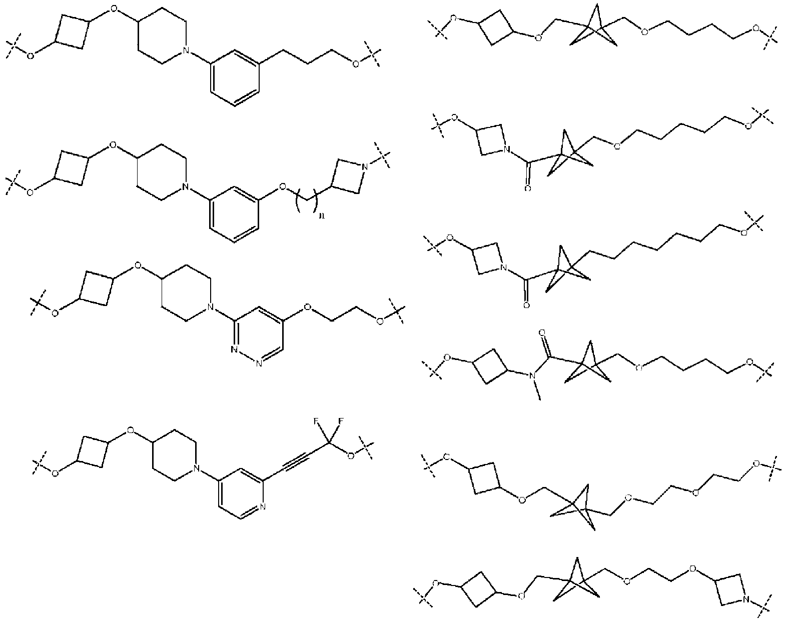 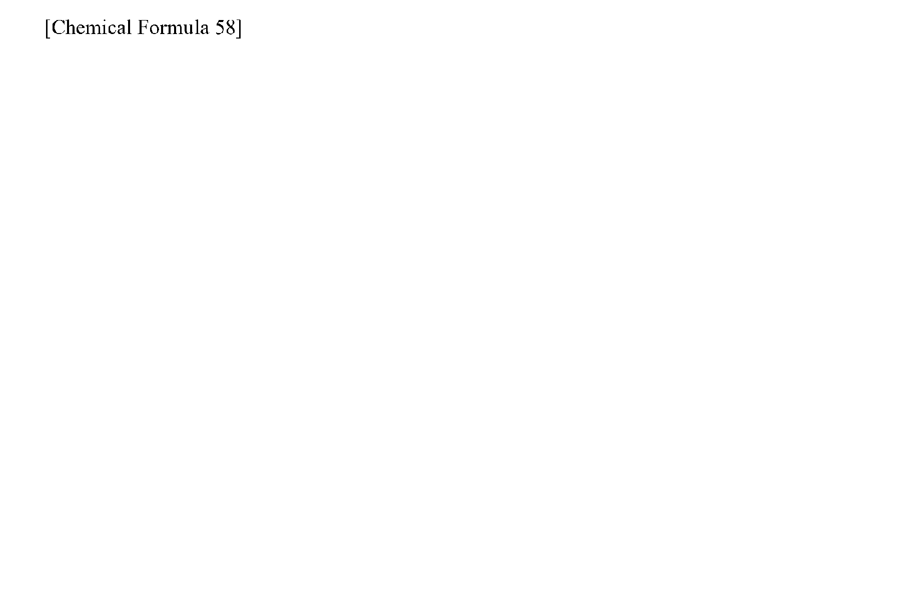 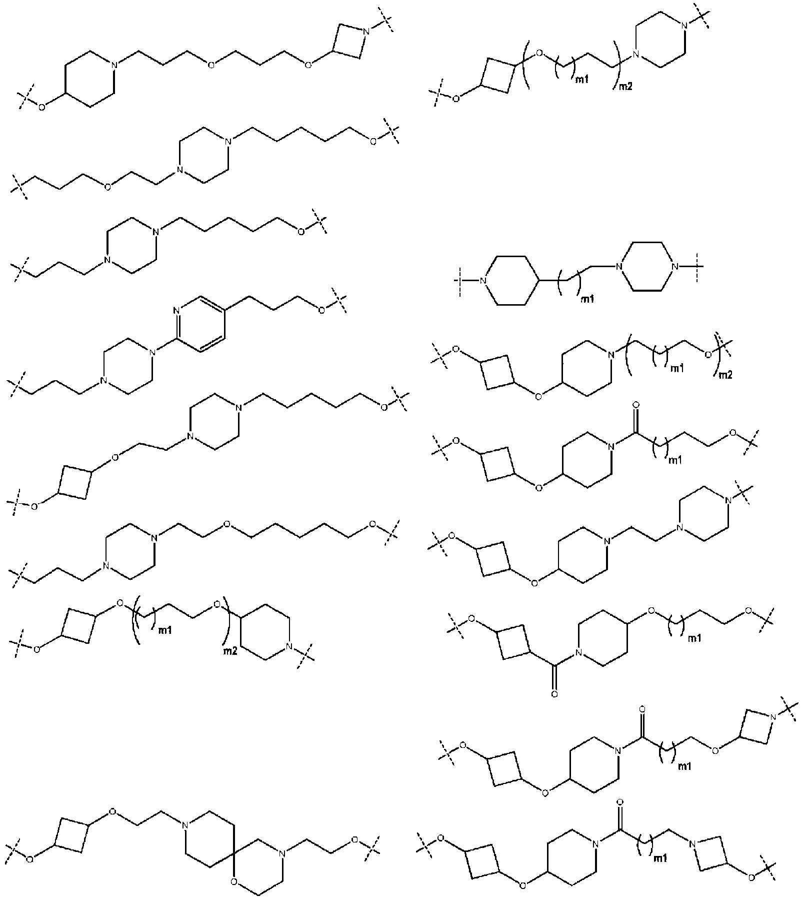 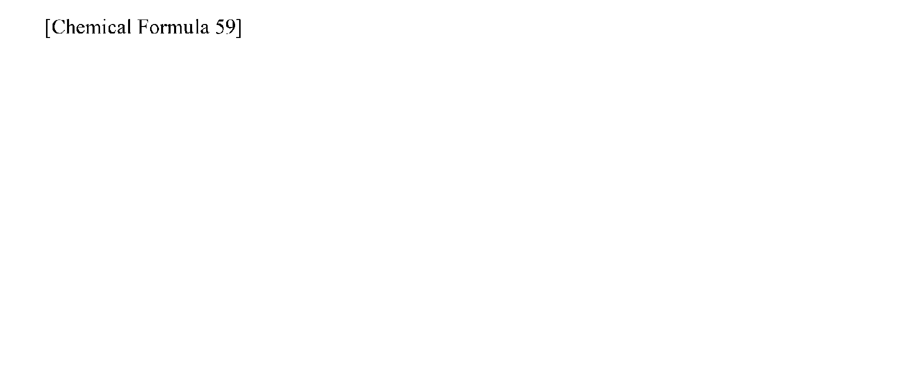 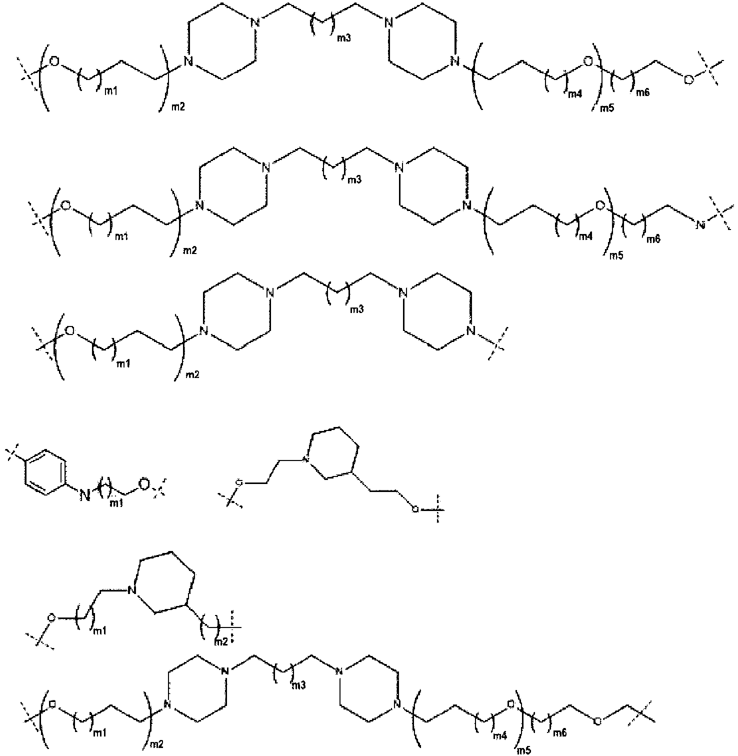 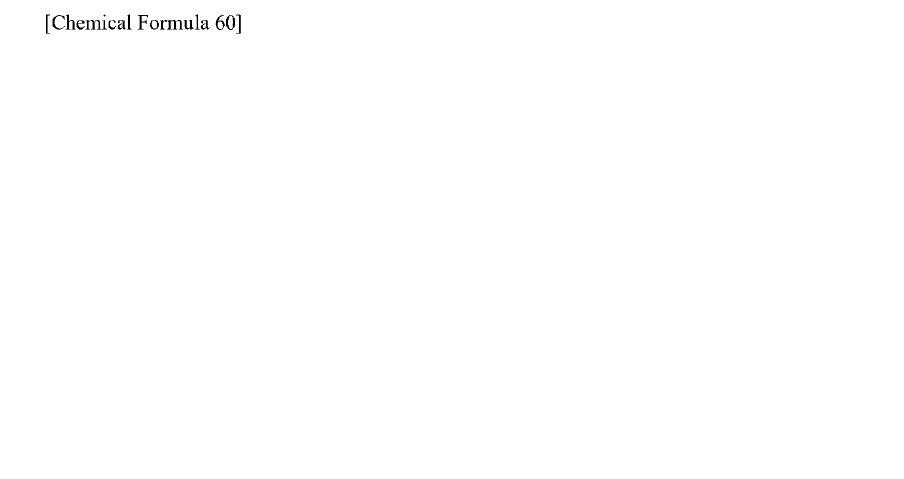 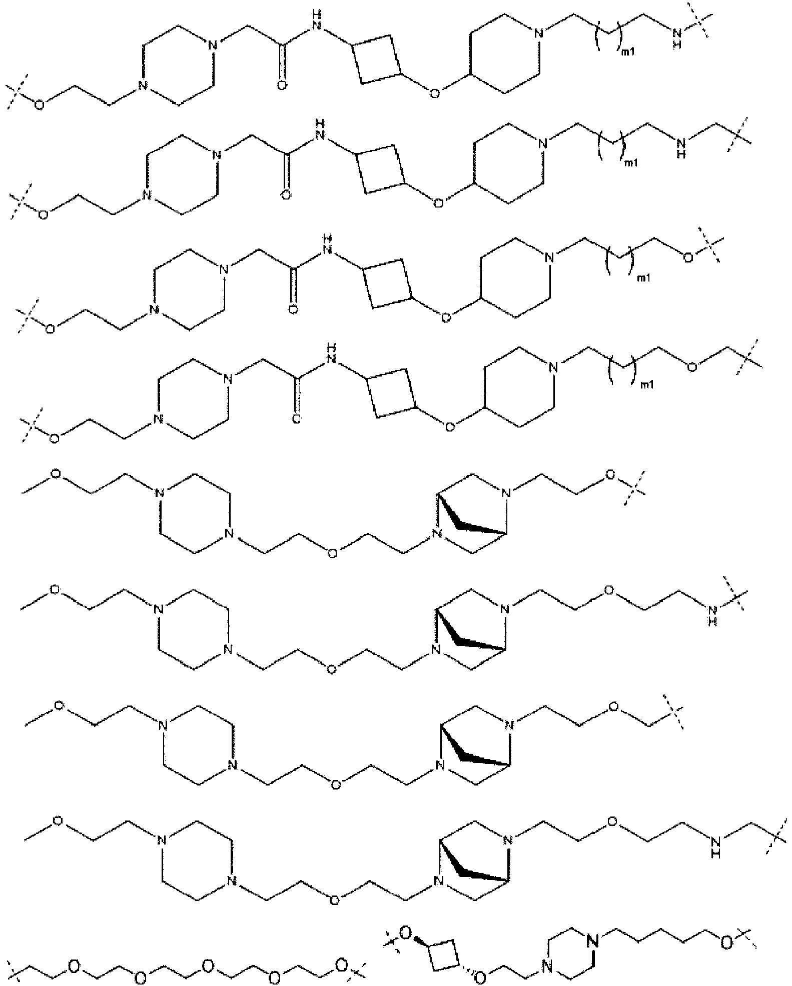 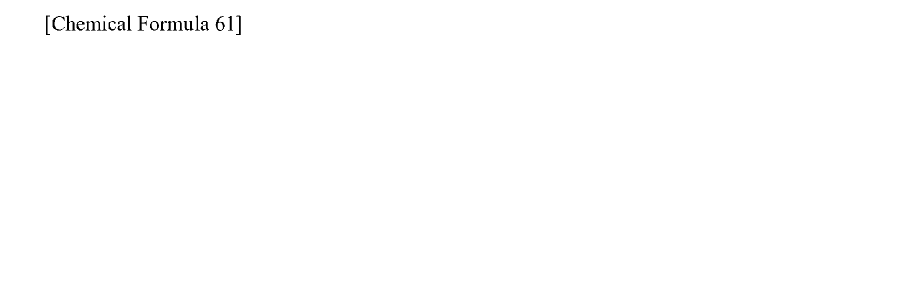 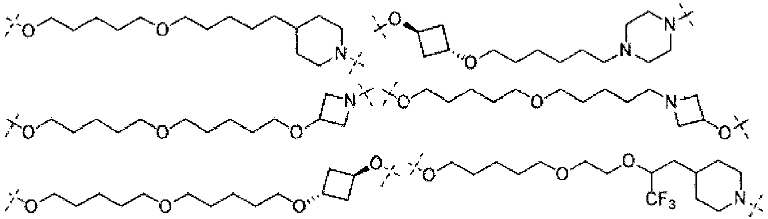
[wherein
m1, m2, m3, m4, m5, and m6 are each independently 0, 1, 2, 3, 4, 5, 6, 7, 8, 9, 10, 11, 12, 13, 14, 15, 16, 17, 18, 19, or 20].

42. The compound according to Claim 35 or a pharmacologically acceptable salt thereof, wherein
the linker is a group selected from a group consisting of:

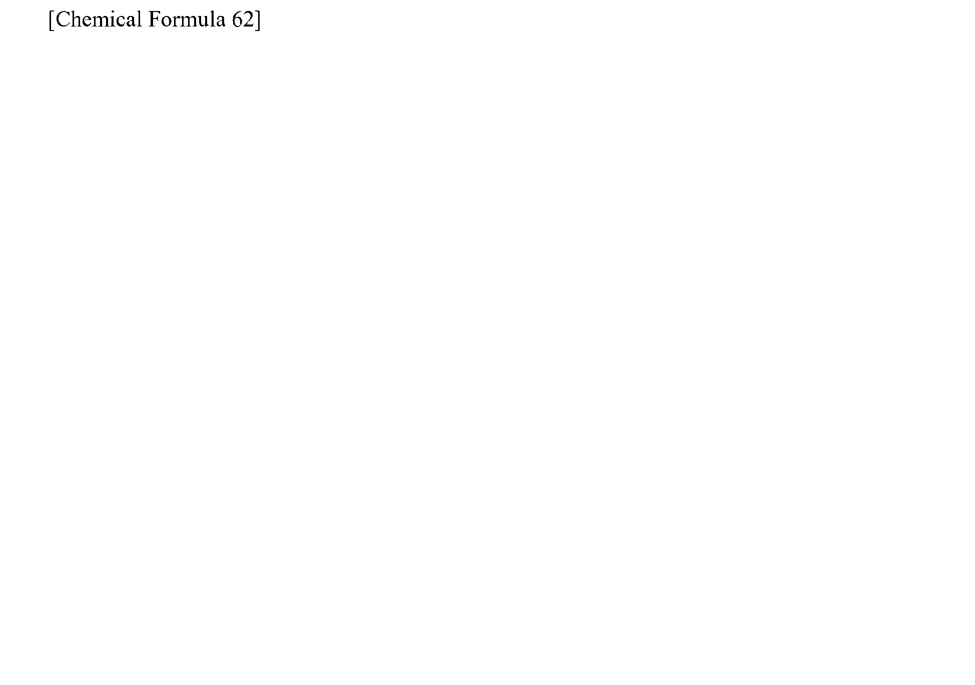

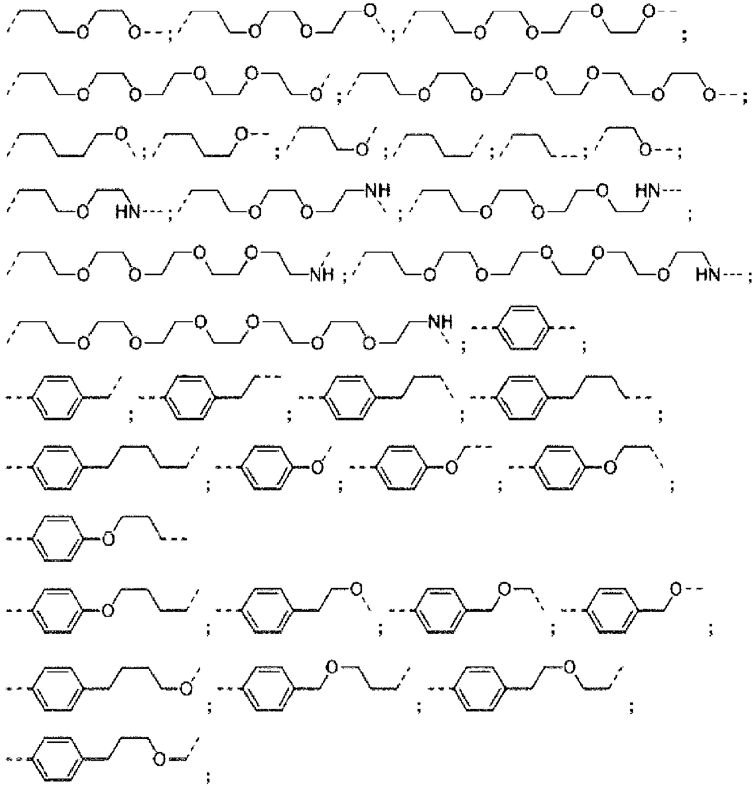

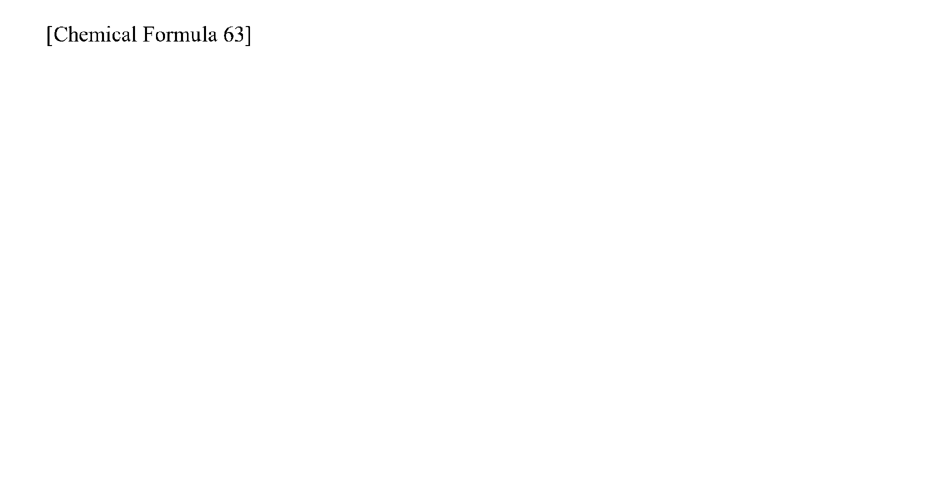

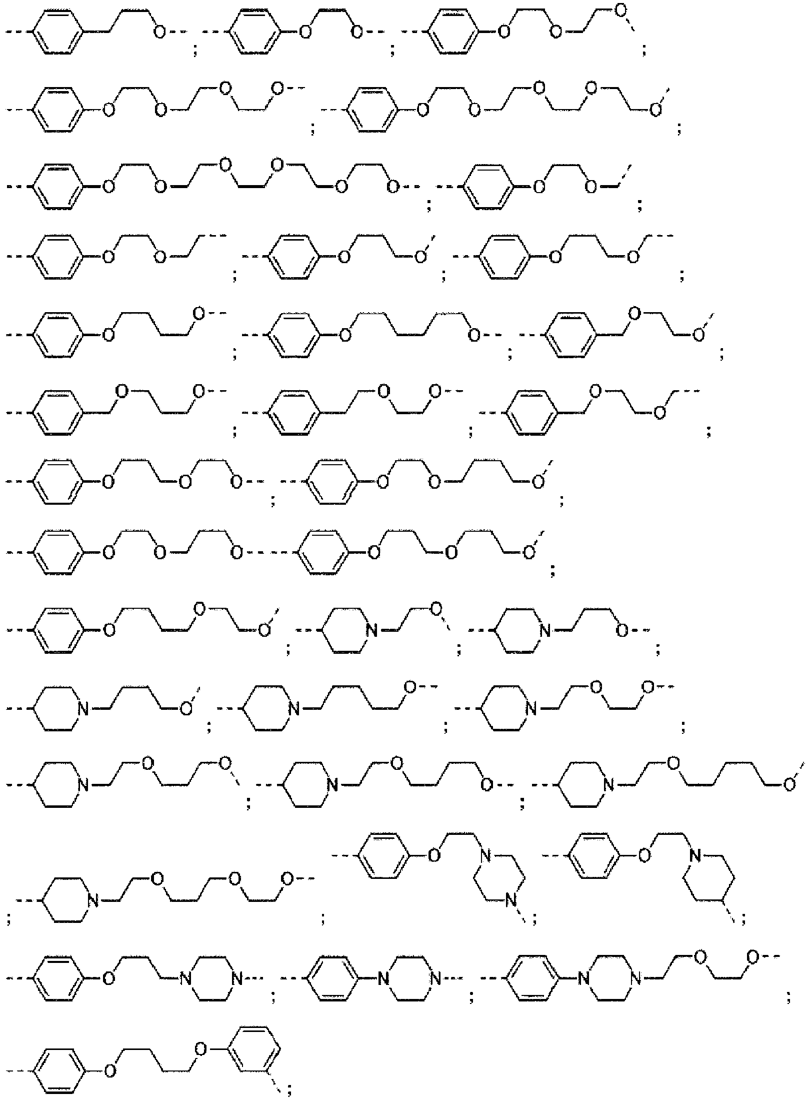

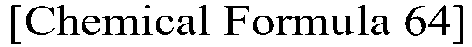

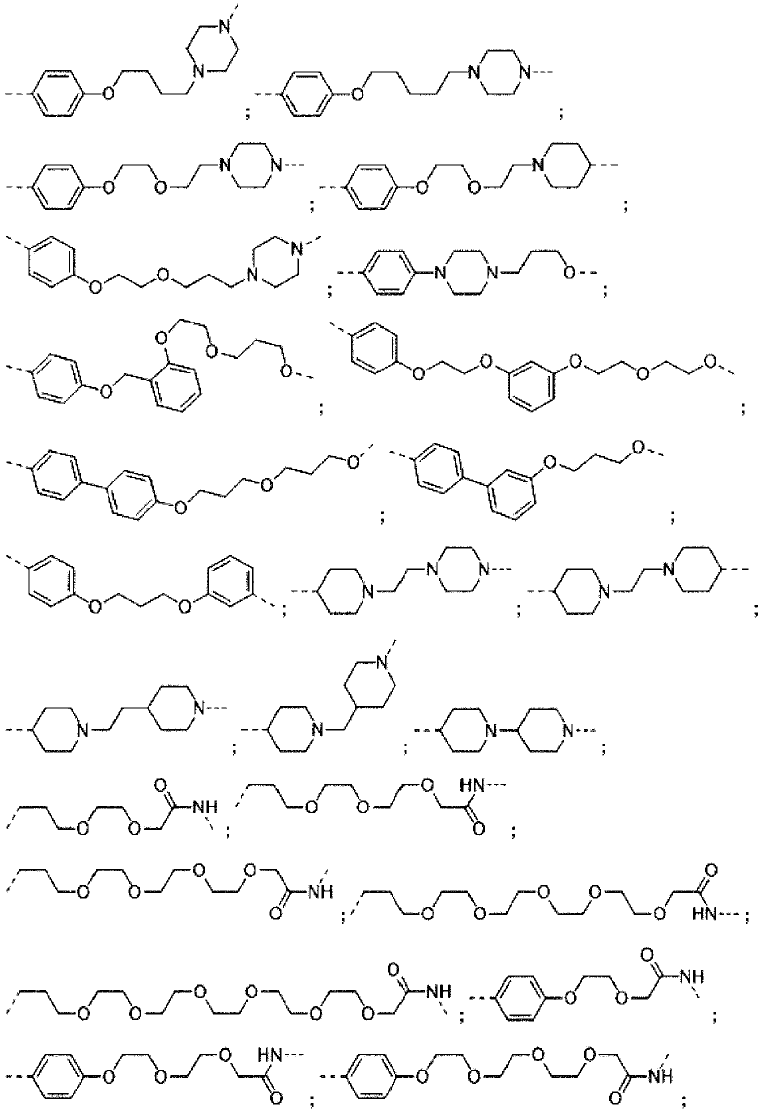

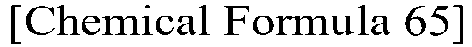

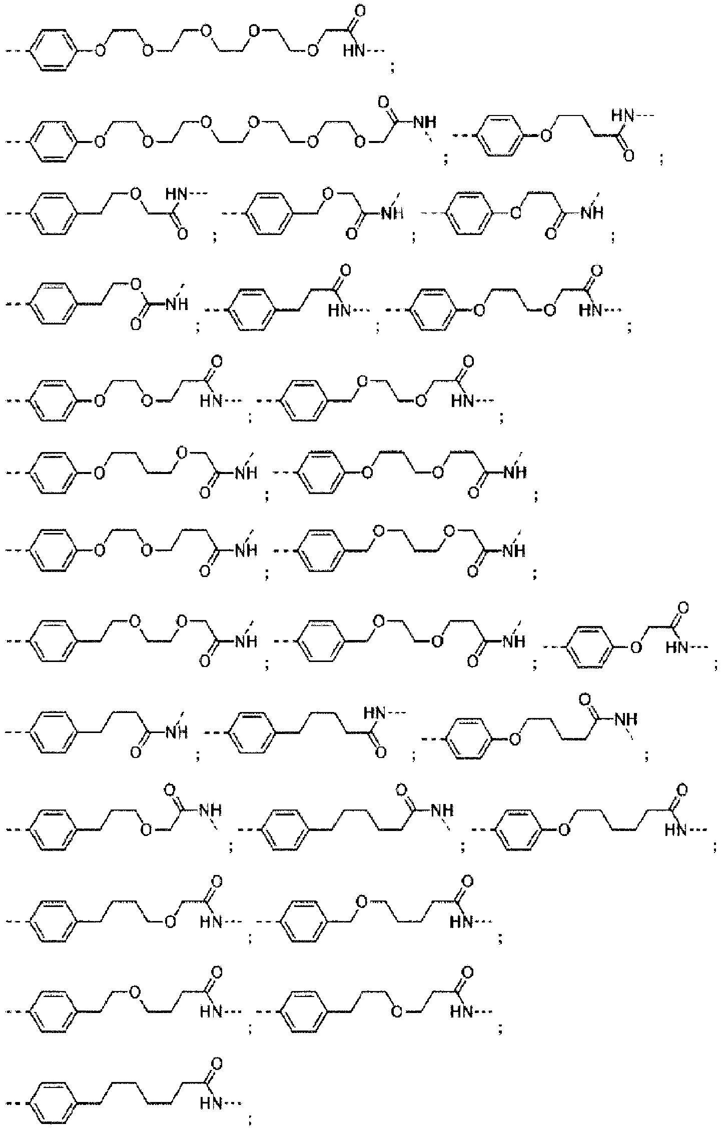

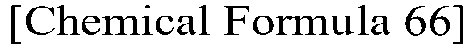

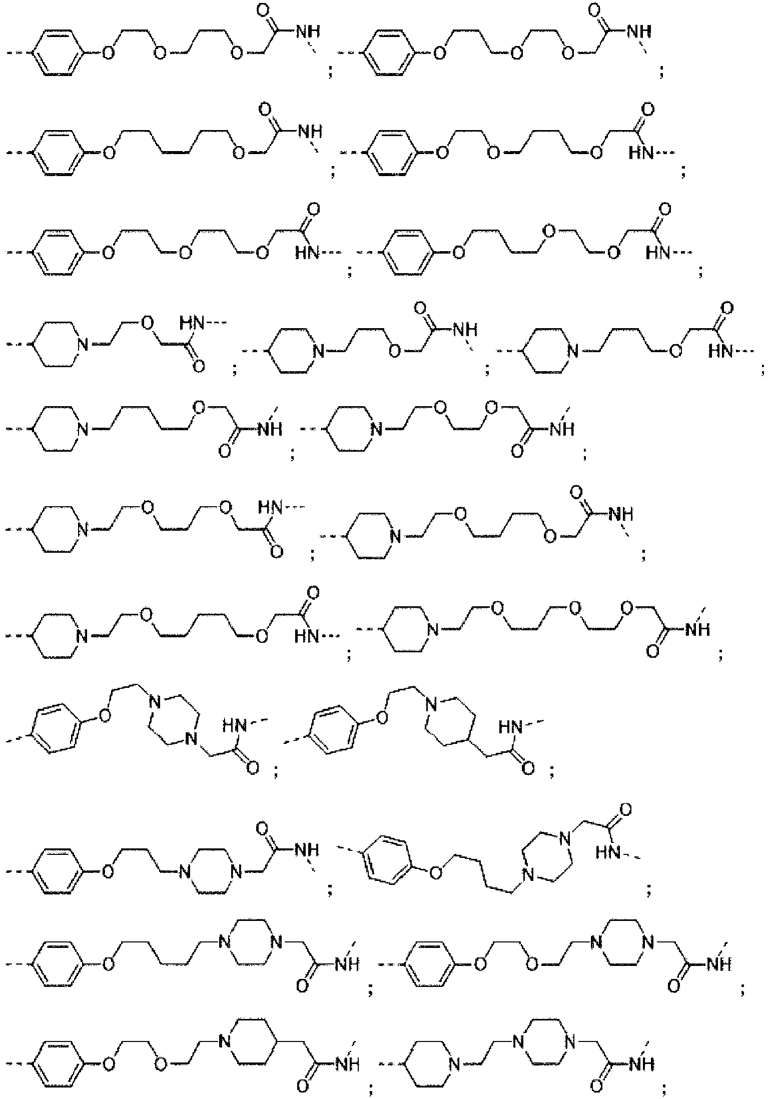

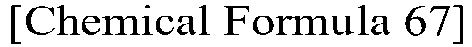

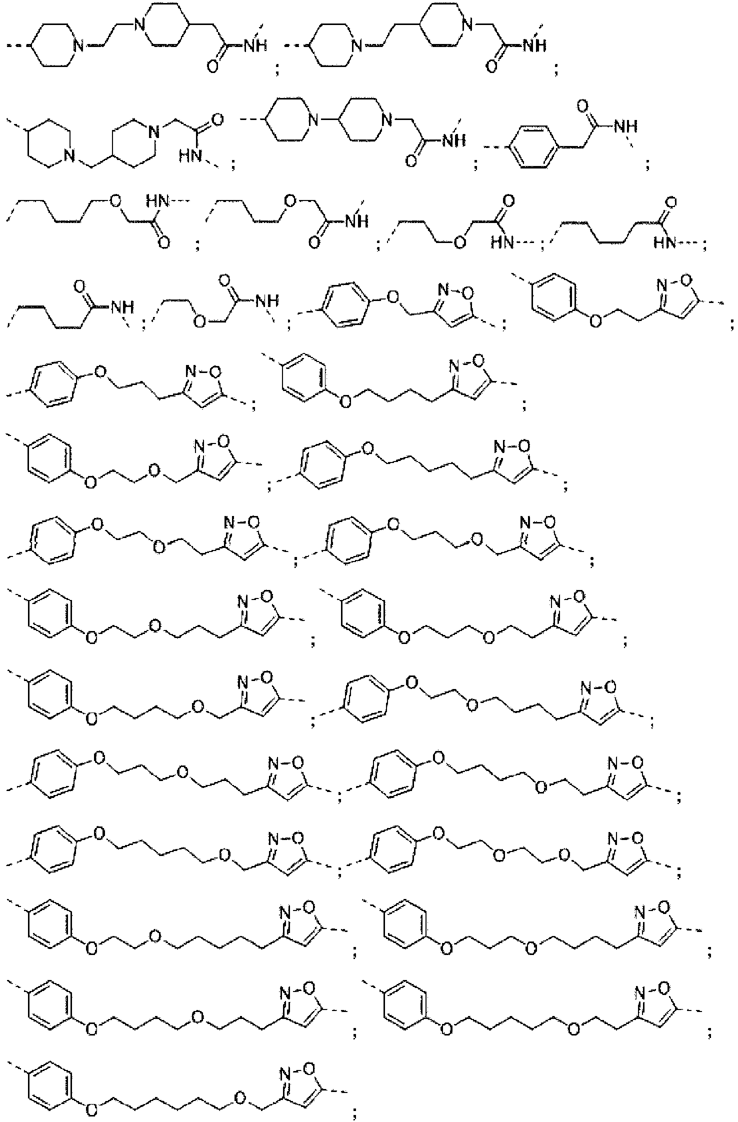

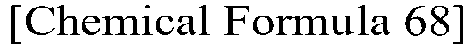

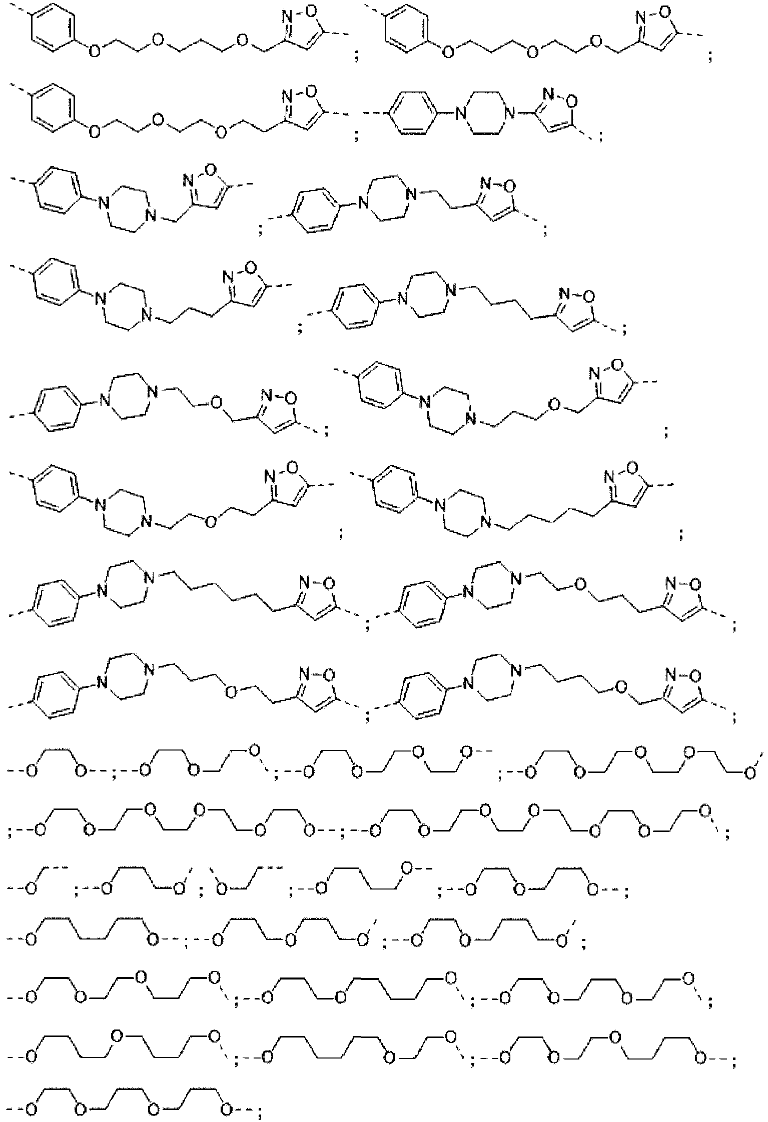

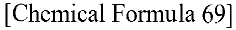

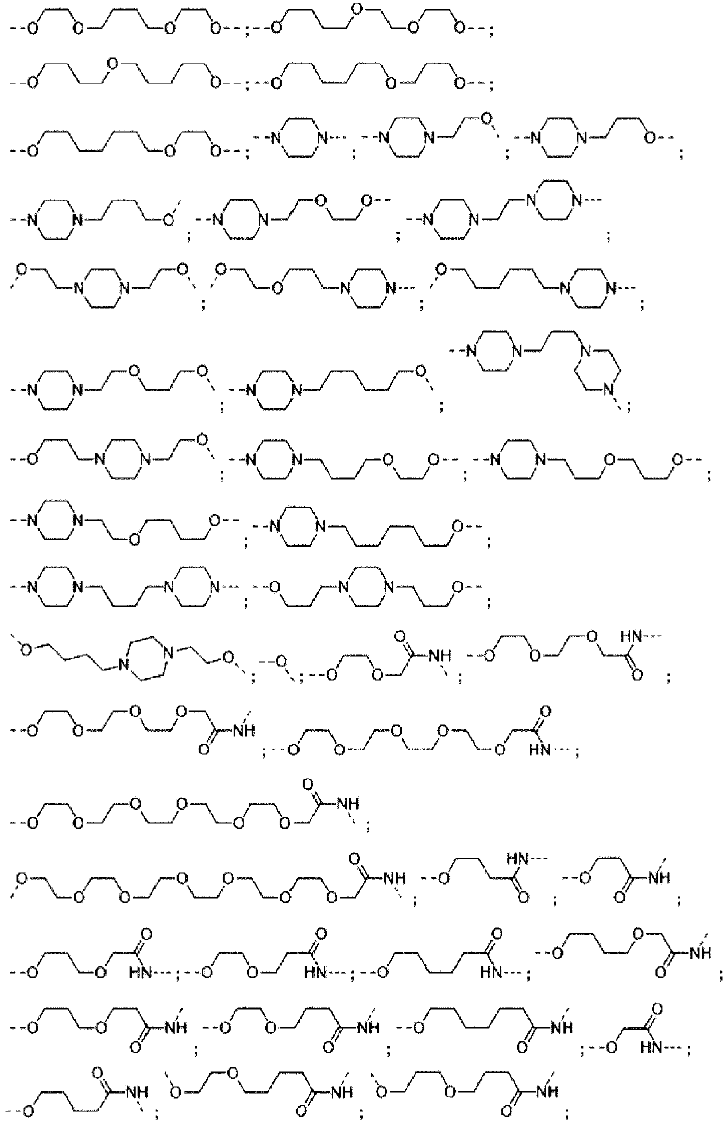

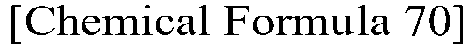

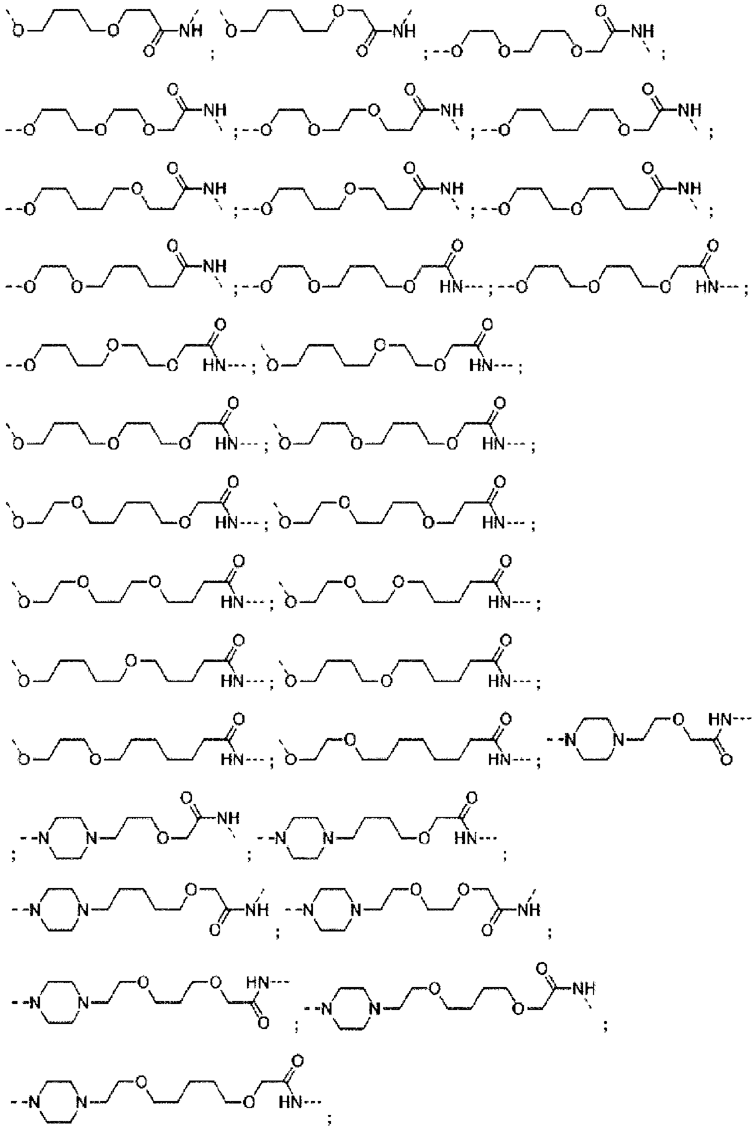

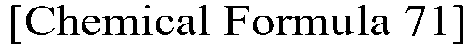

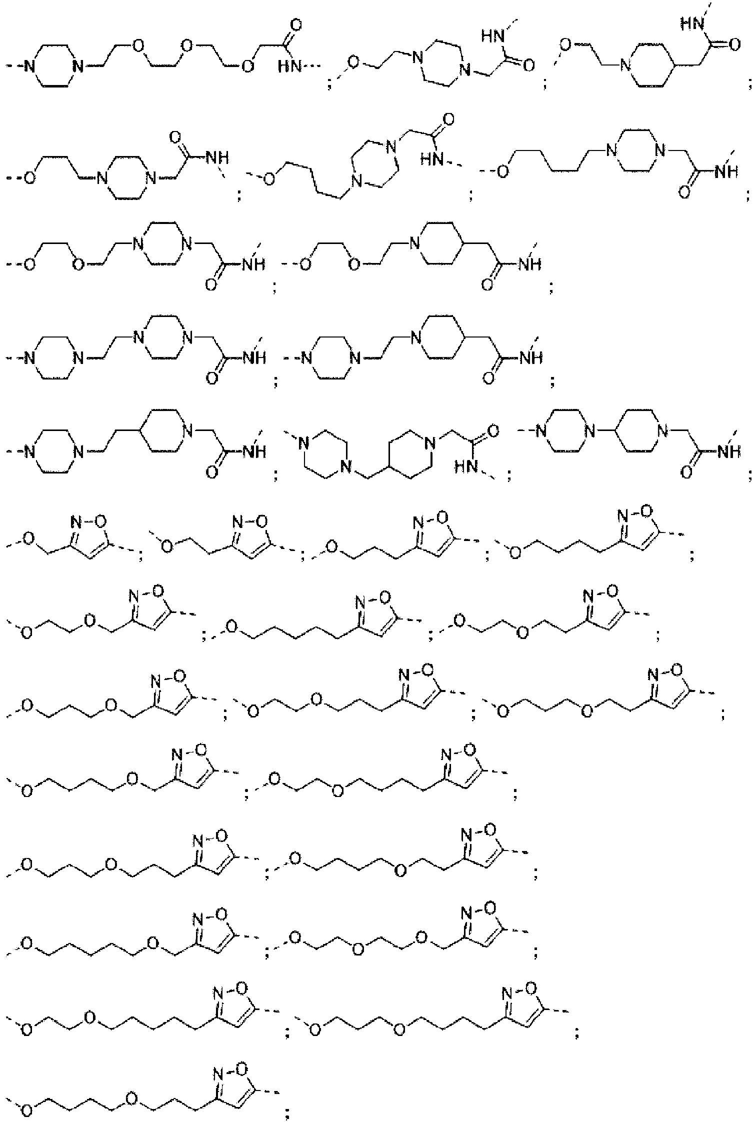

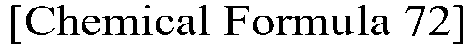

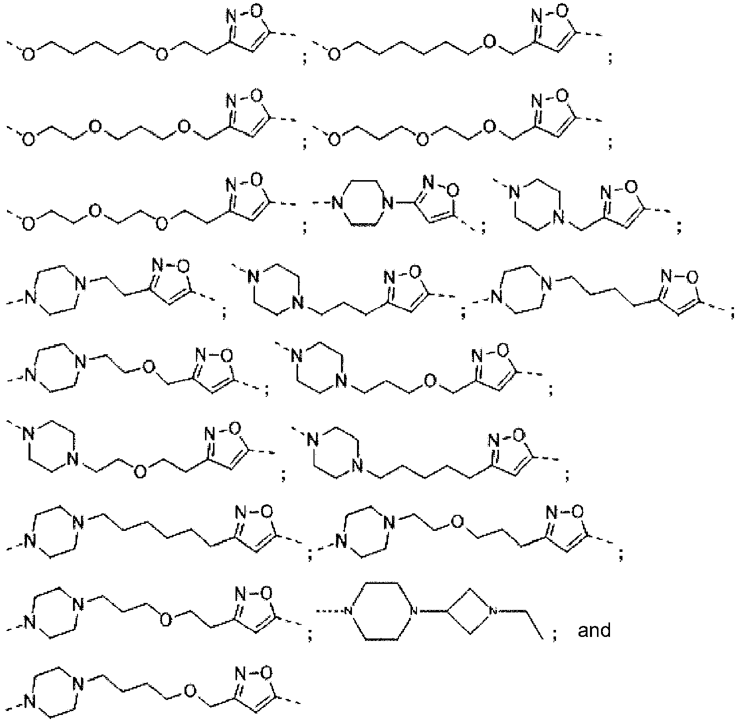

43. The compound according to Claim 35 or a pharmacologically acceptable salt thereof, wherein
L is a group selected from a group consisting of: 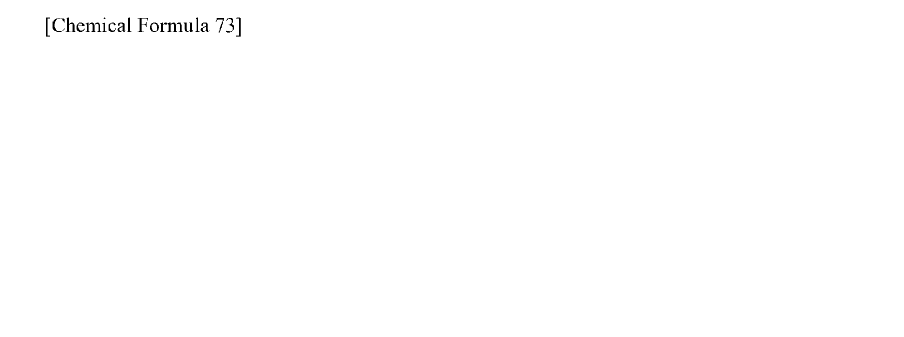 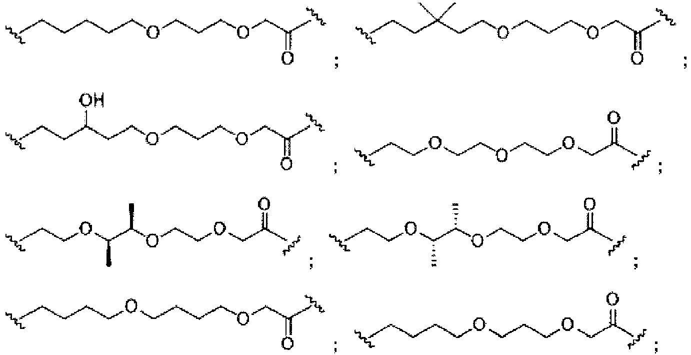 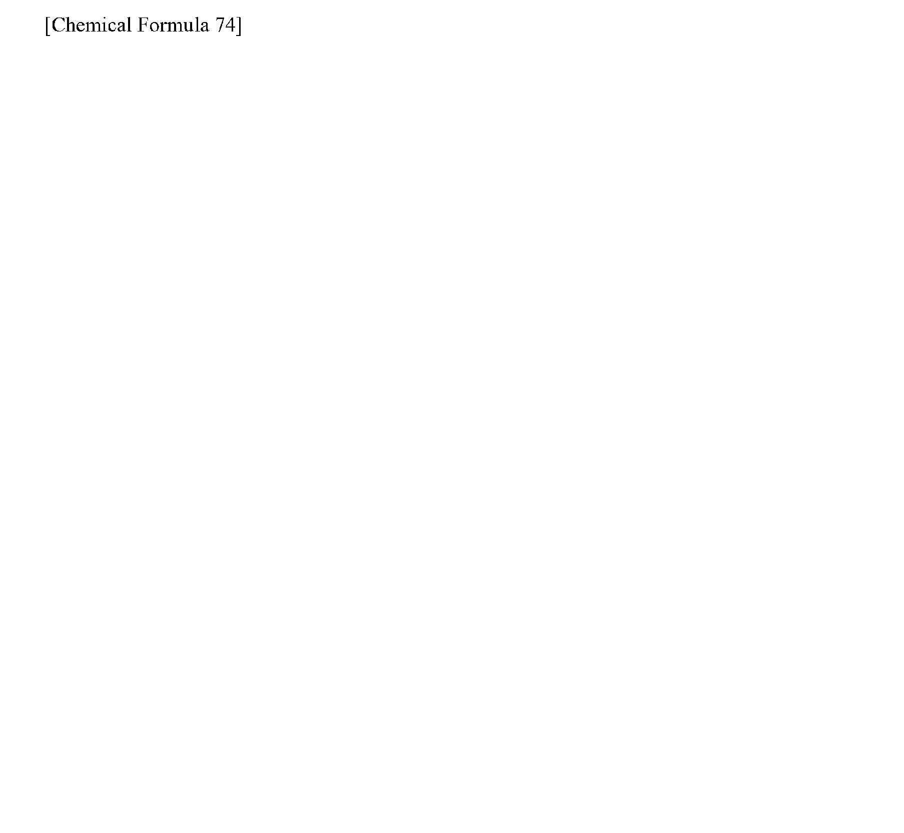 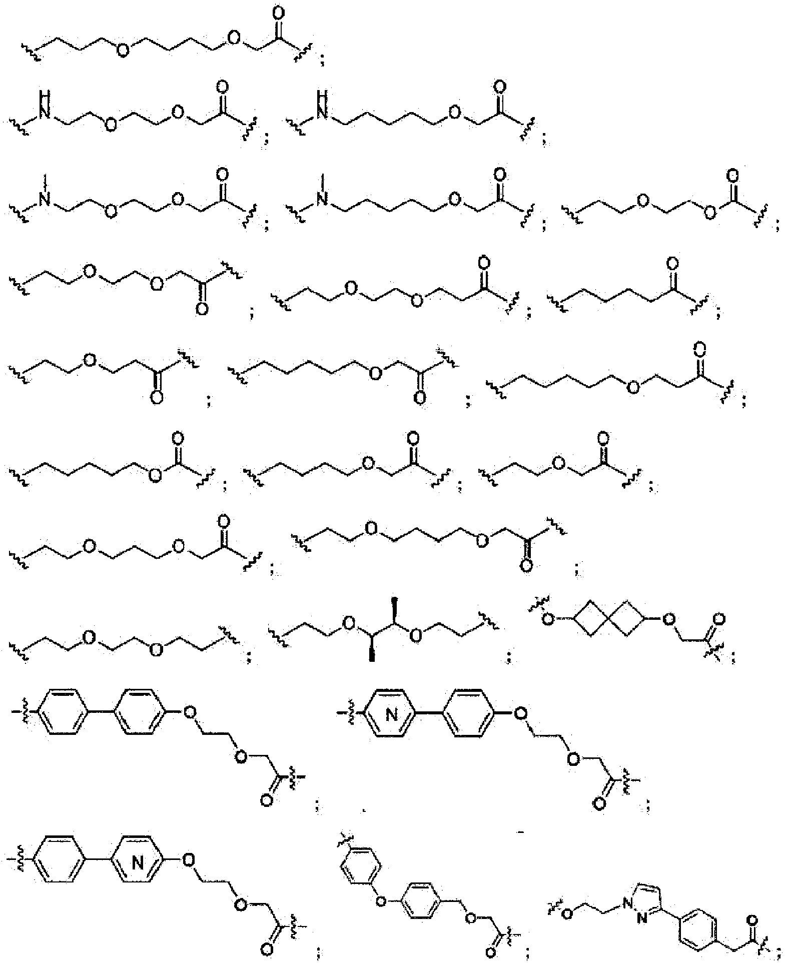 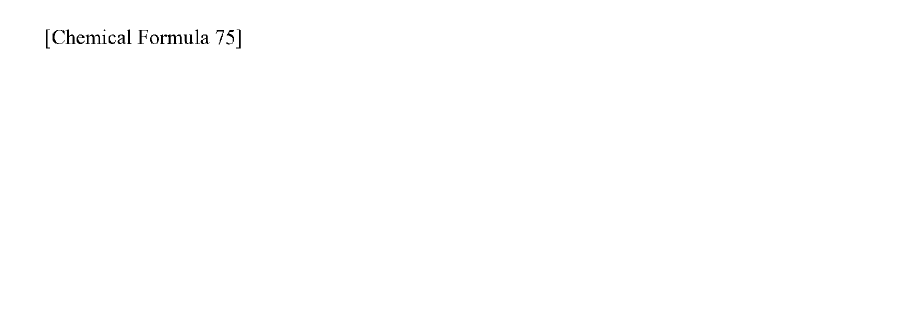 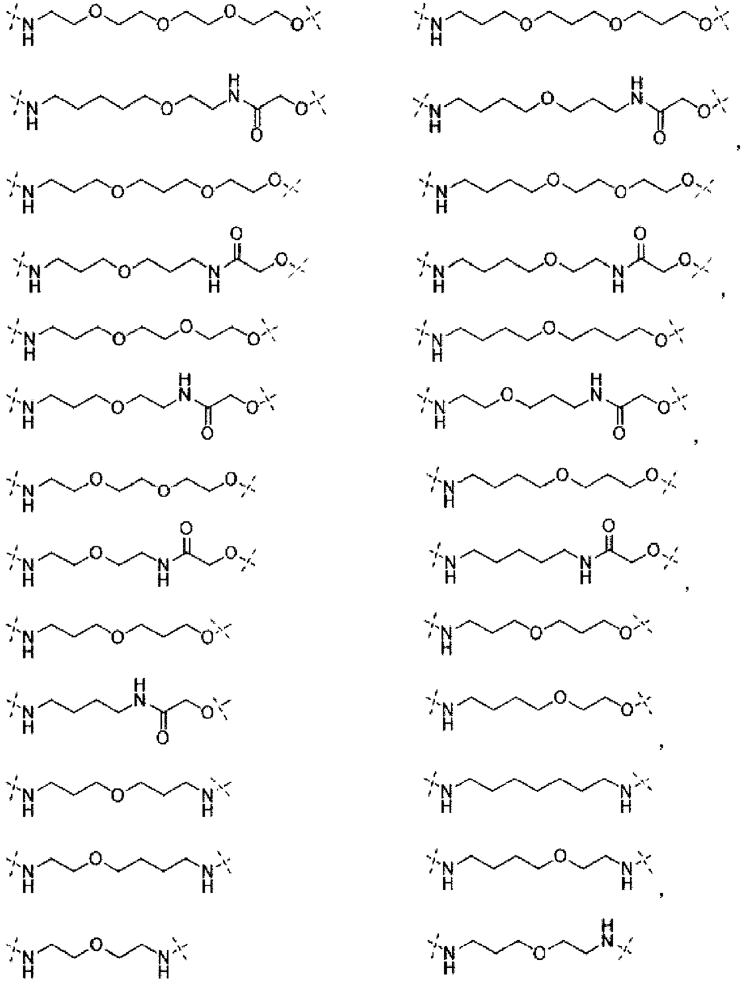 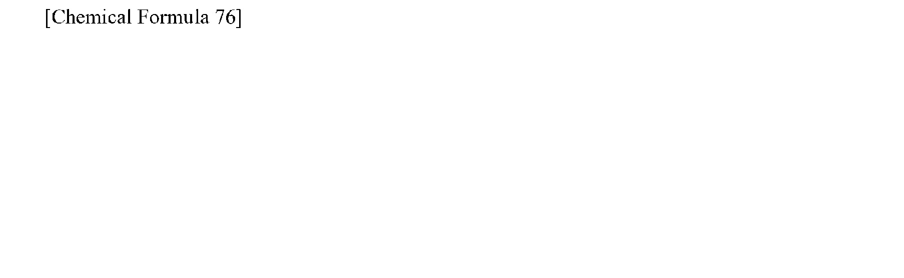 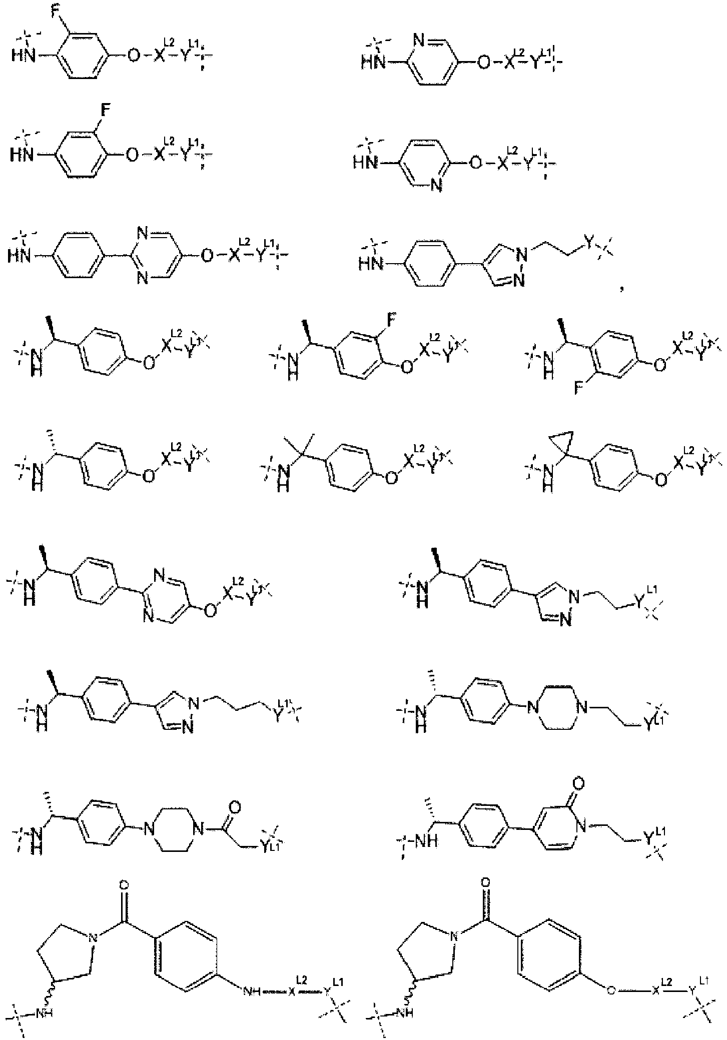
[wherein
X^{L2} is a linear chain containing 2 to 14 atoms, and may contain an oxygen atom, and Y^{L1} is O, N, S, SO, or SO₂].

44. The compound according to any one of Claims 35 to 43 or a pharmacologically acceptable salt thereof, wherein
the substituted L is substituted with TBL, wherein TBL is a group having a moiety capable of binding to a target protein or a moiety that binds to a target protein.

45. The compound according to Claim 44 or a pharmacologically acceptable salt thereof, wherein
the target protein is selected from a group consisting of proteins related to cancer-related proteins, autoimmune disease-related proteins, inflammatory disease-related proteins, neurodegenerative disease-related proteins, and genetic disease-related proteins.

46. The compound according to Claim 45 or a pharmacologically acceptable salt thereof, wherein
the target protein is a cancer-related protein.

47. The compound according to Claim 46 or a pharmacologically acceptable salt thereof, wherein
the cancer-related protein is selected from a group consisting of ABL, AKT, ALK, AR, ARG1, AR-V7, ASH1L, ATM, AURKA, AURORA-A, AuroraA, Bcl2, Bcl-6, BCL9 (β-catenin PPI), Bcl-XL, BCR-ABL, BRAF, BRDs, BRD4, BET (Bromodomain and extraterminal domain) proteins, BRG1/BRM, BRPF1, BTK, CBFβ, CBP, CBP/p300, CDK2, CDK2/5, CDK2/9, CDK4/6, CDK8 (or CDK19), CDK12 (or CDK9), c-KIT, CK1α, CK1α/CDK, CK2, cMet, CRBN, CREBBP, CSF-1R kinase, cyclosporin, DOT1L, EED, EGFR, EGFR/PARP, ENL, EP300 (HAT), ER, ErBb, ERK1/2, ERK1, ERK2, EZH2, FGFR, FGFR2, FGFR3, FGFR4, FKBP, FLT3, FLT3-ITD, Gli1, GSK3β, HDAC, HDAC3, HER3, HMGCR, HPK1, HSP90, IRAK, IRAK4 BTK, ITK, JAK, JAK1,2, JAK2, JAK3, KEAP1, KRas, KRASG12D, LRRK2, LZK, MALT1, MEK, MDM2, mHTT, mTOR, MYB, NF-kB, NR4A1, NTRK1, p38a/d, PARP, PARP1, PBRM1, PD-L1, PDE4, PDGFRa, PIK3CA, PI3K/mTOR, PKCBβ1, PLK1/BRD4, PPAR, PPARγ, PRC2, PTK6, PTPN1/2, RAF, Ras, RET, SHP2, smad3, SMARCA, SMARCA2, SMARCA2/4, SOS1, STAT, STAT3, STK4, Tau, TEAD, TERT, TOP1, TDP1, TRIM24, VEGFR-2, α-tubulin, AURKB, AXL, BRD3, BRD7, BUB1B, CDK12, CDK12 C1039F, CDK17, CHEK1, c-Met, CSNK1A1, DAPK1, DDR2, eIF4E, EPHA1, EPHA2, EPHA3, EPHB2, EPHB3, EPHB4, EPHB6, FKBP12, FLT1, FYN, GCN5, HDAC2, IGF-1R, KRASG12C, LATS1, LCK, LXRA, LYN, MAP3K1, MAP3K11, MAP3K7, MAP4K1, MAP4K3, MAPK10, MAPK9, MAPKAPK2, MCL1, MerTK, MLLT1, MYC, NUAK1, PAK1, PARP2, PARP3, PCAF, PDK1, PRKAA1, PRKAA2, PRKCI, RPS6KA3, RPS6KA4, RPS6KA6, SF3B1, SIRT2, SLC9A2, Src, STK10, STK33, STK40, TAOK2, TAOK3, TGFBR1, TNK1, TTK, TYK2, and YES1.

48. A pharmaceutical composition comprising: as an active ingredient, the compound according to any one of Claims 44 to 47 or a pharmacologically acceptable salt thereof; and a pharmaceutically acceptable carrier.

49. The pharmaceutical composition according to Claim 48 for use in treating a disease caused by dysregulation of protein activity selected from cancer, autoimmune diseases, inflammatory diseases, neurodegenerative diseases, and genetic diseases,

50. The pharmaceutical composition according to Claim 49 for use in treating cancer.

51. The pharmaceutical composition according to Claim 50, wherein
the cancer is related to a cancer-related protein selected from a group consisting of ABL, AKT, ALK, AR, ARG1, AR-V7, ASH1L, ATM, AURKA, AURORA-A, AuroraA, Bcl2, Bcl-6, BCL9 (β-catenin PPI), Bcl-XL, BCR-ABL, BRAF, BRDs, BRD4, BET (Bromodomain and extraterminal domain) proteins, BRG1/BRM, BRPF1, BTK, CBFβ, CBP, CBP/p300, CDK2, CDK2/5, CDK2/9, CDK4/6, CDK8 (or CDK19), CDK12 (or CDK9), c-KIT, CK1α, CK1α/CDK, CK2, cMet, CRBN, CREBBP, CSF-1R kinase, cyclosporin, DOT1L, EED, EGFR, EGFR/PARP, ENL, EP300 (HAT), ER, ErBb, ERK1/2, ERK1, ERK2, EZH2, FGFR, FGFR2, FGFR3, FGFR4, FKBP, FLT3, FLT3-ITD, Gli1, GSK3β, HDAC, HDAC3, HER3, HMGCR, HPK1, HSP90, IRAK, IRAK4 BTK, ITK, JAK, JAK1,2, JAK2, JAK3, KEAP1, KRas, KRASG12D, LRRK2, LZK, MALT1, MEK, MDM2, mHTT, mTOR, MYB, NF-kB, NR4A1, NTRK1, p38a/d, PARP, PARP1, PBRM1, PD-L1, PDE4, PDGFRa, PIK3CA, PI3K/mTOR, PKCBβ1, PLK1/BRD4, PPAR, PPARγ, PRC2, PTK6, PTPN1/2, RAF, Ras, RET, SHP2, smad3, SMARCA, SMARCA2, SMARCA2/4, SOS1, STAT, STAT3, STK4, Tau, TEAD, TERT, TOP1, TDP1, TRIM24, VEGFR-2, α-tubulin, AURKB, AXL, BRD3, BRD7, BUB1B, CDK12, CDK12 C1039F, CDK17, CHEK1, c-Met, CSNK1A1, DAPK1, DDR2, eIF4E, EPHA1, EPHA2, EPHA3, EPHB2, EPHB3, EPHB4, EPHB6, FKBP12, FLT1, FYN, GCN5, HDAC2, IGF-1R, KRASG12C, LATS1, LCK, LXRA, LYN, MAP3K1, MAP3K11, MAP3K7, MAP4K1, MAP4K3, MAPK10, MAPK9, MAPKAPK2, MCL1, MerTK, MLLT1, MYC, NUAK1, PAK1, PARP2, PARP3, PCAF, PDK1, PRKAA1, PRKAA2, PRKCI, RPS6KA3, RPS6KA4, RPS6KA6, SF3B1, SIRT2, SLC9A2, Src, STK10, STK33, STK40, TAOK2, TAOK3, TGFBR1, TNK1, TTK, TYK2, and YES1.

52. A method for producing a compound represented by
Formula (I-1): 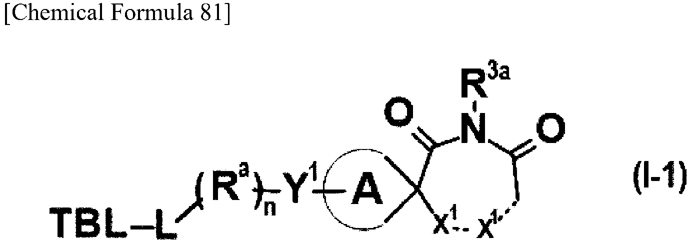
[wherein the symbols have the same meanings as described above, and Y¹ or R^{a} is substituted with L substituted with TBL],
or Formula (II-1): 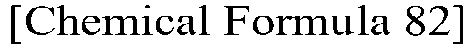 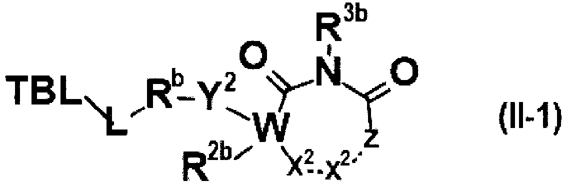
[wherein the symbols have the same meanings as described above, and R^{b} is substituted with L substituted with TBL],
or Formula (III-1): 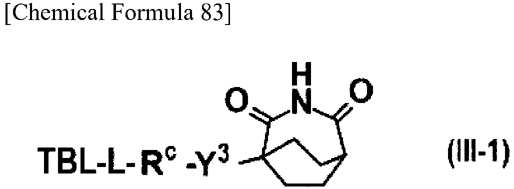
[wherein the symbols have the same meanings as described above, and R^{c} is substituted with L substituted with TBL],
or Formula (IV-1): 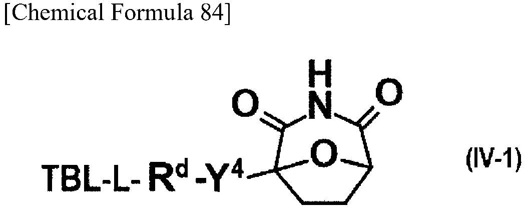
[wherein the symbols have the same meanings as described above, and R^{d} is substituted with L substituted with TBL]
or a pharmacologically acceptable salt thereof, the method comprising:
reacting a compound represented by
the following Formula (I'): 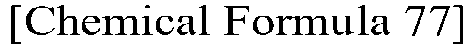 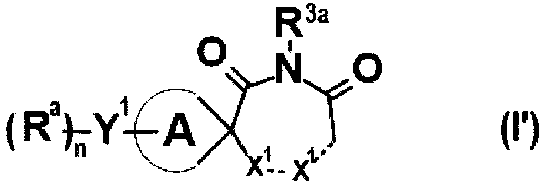
[wherein dotted lines, Ring A, X¹, Y¹, R^{3a}, R^{a}, and n have the same meanings as defined in Formula (I) in Claim 1, provided that Y¹ or R^{a} is not substituted with L having a first terminal group or with substituted L],
or the following Formula (II'): 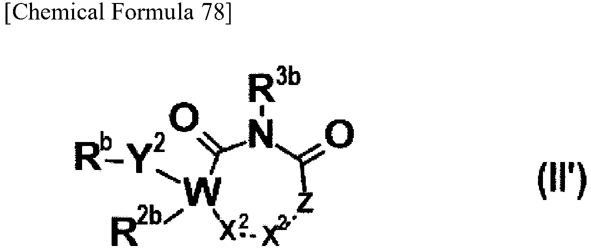
[wherein dotted lines, X², Y², Z, W, R^{b}, R^{2b} and R^{3b} have the same meanings as defined in Formula (II) in Claim 1, provided that R^{b} is not substituted with L having a first terminal group or with substituted L],
or the following Formula (III'): 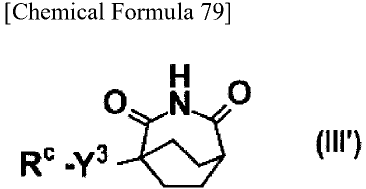
[wherein
Y³ and R^{c} have the same meanings as defined in Formula (III) in Claim 1, provided that R^{c} is not substituted with L having a first terminal group or with substituted L],
or the following Formula (IV'): 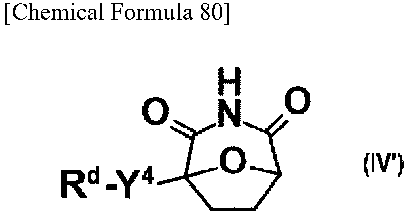
[wherein Y⁴ and R^{d} have the same meanings as defined in Formula (IV) in Claim 1, provided that R^{d} is not substituted with L having a first terminal group or with substituted L],
with a TBL-L compound [wherein the TBL-L compound is a compound in which L having a second terminal group is bonded to TBL, L has the same meaning as defined in Claim 35, and TBL has the same meaning as defined in Claim 44] in a solvent, in the presence or absence of a base.

53. A method for producing a compound represented by
Formula (I-1): 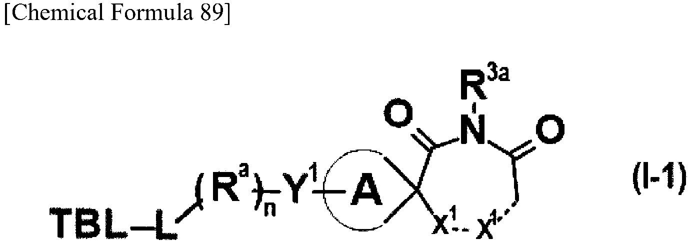
[wherein the symbols have the same meanings as described above, and Y¹ or R^{a} is substituted with L substituted with TBL],
or Formula (II-1): 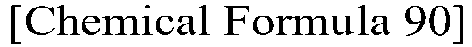 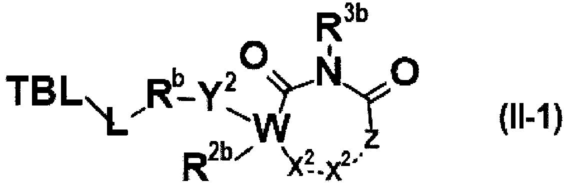
[wherein the symbols have the same meanings as described above, and R^{b} is substituted with L substituted with TBL],
or Formula (III-1): 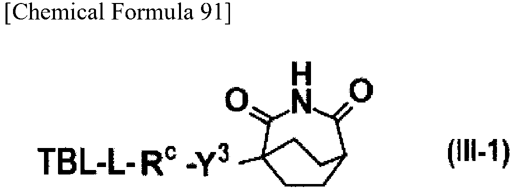
[wherein the symbols have the same meanings as described above, and R^{c} is substituted with L substituted with TBL],
or Formula (IV-1): 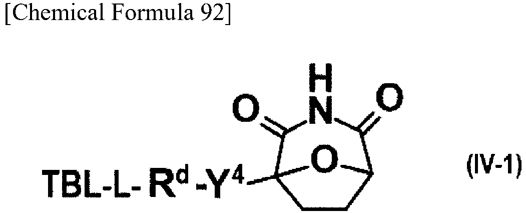
[wherein the symbols have the same meanings as described above, and R^{d} is substituted with L substituted with TBL]
or a pharmacologically acceptable salt thereof, the method comprising:
reacting a compound represented by
the following Formula (I"): 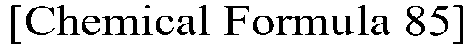 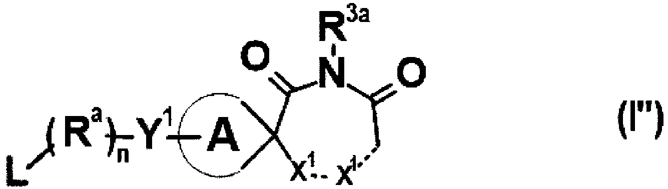
[wherein dotted lines, Ring A, X¹, Y¹, R^{3a}, R^{a} and n have the same meanings as defined in Formula (I) in Claim 1, Y¹ or R^{a} is substituted with L having a first terminal group, and L has the same meaning as defined in Claim 35]
or the following Formula (II"): 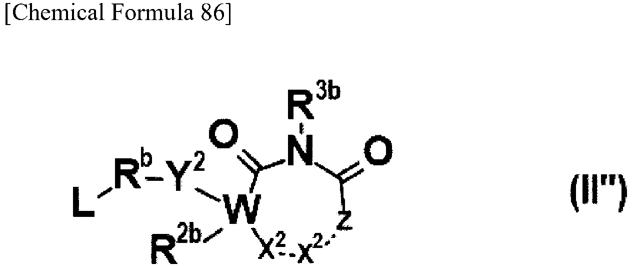
[wherein dotted lines, X², Y², Z, W, R^{b}, R^{2b} and R^{3b} have the same meanings as defined in Formula (II) in Claim 1, R^{b} is substituted with L having a first terminal group, and L has the same meaning as defined in Claim 35]
or the following Formula (III"): 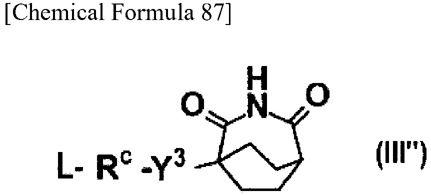
[wherein Y³ and R^{c} have the same meanings as defined as in Formula (III) in Claim 1, R^{c} is substituted with L having a first terminal group, and L has the same meaning as defined in Claim 35]
or the following Formula (IV"): 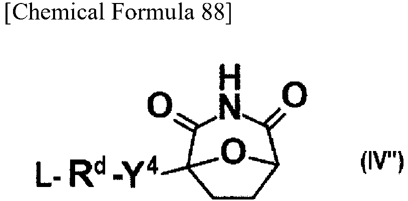
[wherein Y⁴ and R^{d} have the same meanings as defined as in Formula (IV) in Claim 1, R^{d} is substituted with L having a first terminal group, and L has the same meaning as defined in Claim 35]
with a TBL compound [wherein, the TBL compound is a compound in which TBL is bonded to a monovalent chemical group, and TBL has the same meaning as defined in Claim 44]
in a solvent, in the presence or absence of a base.
